# EUROPEAN PATENT APPLICATION

(11) **EP 1 439 189 A2**
(43) Date of publication of application: **21.07.2004**
(21) Application number: 04008119.2
(22) Date of filing: 11.06.1998
(51) Int. Cl.: C07K 14/47, C12N 15/12, C12N 5/10, C07K 16/18, C07K 16/30, G01N 33/68, A61K 38/17

(54) **86 Human Secreted Proteins**

(30) Priority: 13.06.1997 US 49547 P; 13.06.1997 US 49548 P; 13.06.1997 US 49549 P; 13.06.1997 US 49550 P; 13.06.1997 US 49566 P; 13.06.1997 US 49606 P; 13.06.1997 US 49607 P; 13.06.1997 US 49608 P; 13.06.1997 US 49609 P; 13.06.1997 US 49610 P; 13.06.1997 US 49611 P; 13.06.1997 US 50901 P; 13.06.1997 US 52989; 08.07.1997 US 51919 P; 18.08.1997 US 55984; 12.09.1997 US 58665; 12.09.1997 US 58668 P; 12.09.1997 US 58669 P; 12.09.1997 US 58750; 12.09.1997 US 58971 P; 12.09.1997 US 58972; 12.09.1997 US 58975; 02.10.1997 US 60834 P; 02.10.1997 US 60841 P; 02.10.1997 US 60844 P; 02.10.1997 US 60865 P; 02.10.1997 US 61059 P; 02.10.1997 US 61060 P
(62) Divisional of application: 98929001.0
(71) Applicant: Human Genome Sciences, Inc., Rockville, MD 20850 (US)
(72) Inventor: Ruben, Steven M., Brookeville MD 20833 (US); Ni, Jian, Germantown Maryland 20874 (US); Rosen, Craig A:, Laytonsville Maryland 20882 (US); Ebner, Reinhard, Gaithersburg Maryland 20878 (US); Young, Paul, Gaithersburg Maryland 20878 (US); Moore, Paul A, Germantown Maryland 20874 (US); Feng, Ping, Germantown Maryland 20874 (US); LaFleur, David W., Washington D.C. 20015 (US); Olsen, Henrik S., Gaithersburg Maryland 20878 (US); Yanggu, Shi, Gaithersburg Maryland 20878 (US); Brewer, Laurie A., Poolesville MD 20837 (US); Greene, John M., Gaithersburg MD 20878 (US); Ferrie, Ann M., Germantown MD 20874 (US); Yu, Guo-Liang, Darnestown MD 20878 (US)
(74) Representative: VOSSIUS & PARTNER

(57) **Abstract**

The present invention relates to 86 novel human secreted proteins and isolated nucleic acids containing the coding regions of the genes encoding such proteins. Also provided are vectors, host cells, antibodies, and recombinant methods for producing human secreted proteins. The invention further relates to diagnostic and therapeutic methods useful for diagnosing and treating disorders related to these novel human secreted proteins.

Sequence Listing

(1) GENERAL INFORMATION:

(i) APPLICANT: Rosen et al.

(ii) TITLE OF INVENTION: 86 Human Secreted Proteins

(iii) NUMBER OF SEQUENCES: 318

(iv) CORRESPONDENCE ADDRESS:

(A) ADDRESSEE: Human Genome Sciences, Inc.

(B) STREET: 9410 Key West Avenue

(C) CITY: Rockville

(D) STATE: Maryland

(E) COUNTRY: USA

(F) ZIP: 20850

(v) COMPUTER READABLE FORM:

(A) MEDIUM TYPE: Diskette, 3.50 inch, 1.4Mb storage

(B) COMPUTER: HP Vectra 486/33

(C) OPERATING SYSTEM: MSDOS version 6.2

(D) SOFTWARE: ASCII Text

(vi) CURRENT APPLICATION DATA:

(A) APPLICATION NUMBER:

(B) FILING DATE: June 11, 1998

(C) CLASSIFICATION:

(vii) PRIOR APPLICATION DATA:

(A) APPLICATION NUMBER:

(B) FILING DATE:

(viii) ATTORNEY/AGENT INFORMATION:

(A) NAME: A. Anders Brookes

(B) REGISTRATION NUMBER: 36,373

(C) REFERENCE/DOCKET NUMBER: PZ008PCT

(vi) TELECOMMUNICATION INFORMATION:

(A) TELEPHONE: (301) 309-8504

(B) TELEFAX: (301) 309-8439

(2) INFORMATION FOR SEQ ID NO: 1:

(i) SEQUENCE CHARACTERISTICS:
(A) LENGTH: 733 base pairs
(B) TYPE: nucleic acid
(C) STRANDEDNESS: double
(D) TOPOLOGY: linear

(xi) SEQUENCE DESCRIPTION: SEQ ID NO: 1:

GGGATCCGGA GCCCAAATCT TCTGACAAAA CTCACACATG CCCACCGTGC CCAGCACCTG 60

AATTCGAGGG TGCACCGTCA GTCTTCCTCT TCCCCCCAAA ACCCAAGGAC ACCCTCATGA 120

TCTCCCGGAC TCCTGAGGTC ACATGCGTGG TGGTGGACGT AAGCCACGAA GACCCTGAGG 180

TCAAGTTCAA CTGGTACGTG GACGGCGTGG AGGTGCATAA TGCCAAGACA AAGCCGCGGG 240

AGGAGCAGTA CAACAGCACG TACCGTGTGG TCAGCGTCCT CACCGTCCTG CACCAGGACT 300

GGCTGAATGG CAAGGAGTAC AAGTGCAAGG TCTCCAACAA AGCCCTCCCA ACCCCCATCG 360

AGAAAACCAT CTCCAAAGCC AAAGGGCAGC CCCGAGAACC ACAGGTGTAC ACCCTGCCCC 420

CATCCCGGGA TGAGCTGACC AAGAACCAGG TCAGCCTGAC CTGCCTGGTC AAAGGCTTCT 480

ATCCAAGCGA CATCGCCGTG GAGTGGGAGA GCAATGGGCA GCCGGAGAAC AACTACAAGA 540

CCACGCCTCC CGTGCTGGAC TCCGACGGCT CCTTCTTCCT CTACAGCAAG CTCACCGTGG 600

ACAAGAGCAG GTGGCAGCAG GGGAACGTCT TCTCATGCTC CGTGATGCAT GAGGCTCTGC 660

ACAACCACTA CACGCAGAAG AGCCTCTCCC TGTCTCCGGG TAAATGAGTG CGACGGCCGC 720

GACTCTAGAG GAT 733

(2) INFORMATION FOR SEQ ID NO: 2:

(i) SEQUENCE CHARACTERISTICS:
(A) LENGTH: 5 amino acids
(B) TYPE: amino acid
(D) TOPOLOGY: linear

(xi) SEQUENCE DESCRIPTION: SEQ ID NO: 2:

Trp Ser Xaa Trp Ser
1               5

(2) INFORMATION FOR SEQ ID NO: 3:

(i) SEQUENCE CHARACTERISTICS:
(A) LENGTH: 86 base pairs
(B) TYPE: nucleic acid
(C) STRANDEDNESS: double
(D) TOPOLOGY: linear

(xi) SEQUENCE DESCRIPTION: SEQ ID NO: 3:

GCGCCTCGAG ATTTCCCCGA AATCTAGATT TCCCCGAAAT GATTTCCCCG AAATGATTTC 60

CCCGAAATAT CTGCCATCTC AATTAG 86

(2) INFORMATION FOR SEQ ID NO: 4:

(i) SEQUENCE CHARACTERISTICS:
(A) LENGTH: 27 base pairs
(B) TYPE: nucleic acid
(C) STRANDEDNESS: double
(D) TOPOLOGY: linear

(xi) SEQUENCE DESCRIPTION: SEQ ID NO: 4:

GCGGCAAGCT TTTTGCAAAG CCTAGGC 27

(2) INFORMATION FOR SEQ ID NO: 5:

(i) SEQUENCE CHARACTERISTICS:
(A) LENGTH: 271 base pairs
(B) TYPE: nucleic acid
(C) STRANDEDNESS: double
(D) TOPOLOGY: linear

(xi) SEQUENCE DESCRIPTION: SEQ ID NO: 5:

CTCGAGATTT CCCCGAAATC TAGATTTCCC CGAAATGATT TCCCCGAAAT GATTTCCCCG 60

AAATATCTGC CATCTCAATT AGTCAGCAAC CATAGTCCCG CCCCTAACTC CGCCCATCCC 120

GCCCCTAACT CCGCCCAGTT CCGCCCATTC TCCGCCCCAT GGCTGACTAA TTTTTTTTAT 180

TTATGCAGAG GCCGAGGCCG CCTCGGCCTC TGAGCTATTC CAGAAGTAGT GAGGAGGCTT 240

TTTTGGAGGC CTAGGCTTTT GCAAAAAGCT T 271

(2) INFORMATION FOR SEQ ID NO: 6:

(i) SEQUENCE CHARACTERISTICS:
(A) LENGTH: 32 base pairs
(B) TYPE: nucleic acid
(C) STRANDEDNESS: double
(D) TOPOLOGY: linear

(xi) SEQUENCE DESCRIPTION: SEQ ID NO: 6:

GCGCTCGAGG GATGACAGCG ATAGAACCCC GG 32

(2) INFORMATION FOR SEQ ID NO: 7:

(i) SEQUENCE CHARACTERISTICS:
(A) LENGTH: 31 base pairs
(B) TYPE: nucleic acid
(C) STRANDEDNESS: double
(D) TOPOLOGY: linear

(xi) SEQUENCE DESCRIPTION: SEQ ID NO: 7:

GCGAAGCTTC GCGACTCCCC GGATCCGCCT C 31

(2) INFORMATION FOR SEQ ID NO: 8:

(i) SEQUENCE CHARACTERISTICS:
(A) LENGTH: 12 base pairs
(B) TYPE: nucleic acid
(C) STRANDEDNESS: double
(D) TOPOLOGY: linear

(xi) SEQUENCE DESCRIPTION: SEQ ID NO: 8:

GGGGACTTTC CC 12

(2) INFORMATION FOR SEQ ID NO: 9:

(i) SEQUENCE CHARACTERISTICS:
(A) LENGTH: 73 base pairs
(B) TYPE: nucleic acid
(C) STRANDEDNESS: double
(D) TOPOLOGY: linear

(xi) SEQUENCE DESCRIPTION: SEQ ID NO: 9:

GCGGCCTCGA GGGGACTTTC CCGGGGACTT TCCGGGGACT TTCCGGGACT TTCCATCCTG 60

CCATCTCAAT TAG 73

(2) INFORMATION FOR SEQ ID NO: 10:

(i) SEQUENCE CHARACTERISTICS:
(A) LENGTH: 256 base pairs
(B) TYPE: nucleic acid
(C) STRANDEDNESS: double
(D) TOPOLOGY: linear

(xi) SEQUENCE DESCRIPTION: SEQ ID NO: 10:

CTCGAGGGGA CTTTCCCGGG GACTTTCCGG GGACTTTCCG GGACTTTCCA TCTGCCATCT 60

CAATTAGTCA GCAACCATAG TCCCGCCCCT AACTCCGCCC ATCCCGCCCC TAACTCCGCC 120

CAGTTCCGCC CATTCTCCGC CCCATGGCTG ACTAATTTTT TTTATTTATG CAGAGGCCGA 180

GGCCGCCTCG GCCTCTGAGC TATTCCAGAA GTAGTGAGGA GGCTTTTTTG GAGGCCTAGG 240

CTTTTGCAAA AAGCTT 256

(2) INFORMATION FOR SEQ ID NO: 11:

(i) SEQUENCE CHARACTERISTICS:
(A) LENGTH: 1220 base pairs
(B) TYPE: nucleic acid
(C) STRANDEDNESS: double
(D) TOPOLOGY: linear

(xi) SEQUENCE DESCRIPTION: SEQ ID NO: 11:

CATGAATGGC TCGCACAAGG ACCCCCTCCT CCCCTTTCCT GCTTCTGCGA GAACTCCCTC 60

CCTCCCTCCA GCTCCGCCAG CCCAGGCGCC CCTTCCCTGG AAGCCGAGCG GCTTCGCTCG 120

CATTTCACCG CCGCCGCCTC TCGCAATATT GCAATATAGG GGAAAAGCAG ACCATGGTGA 180

ATCCGGGCAG CAGCTCGCAG CCGCCCCCGG TGACGGCCGG CTCCCTCTCC TGGAAGCGGT 240

GCGCAGGCTG CGGGGGCAAG ATTGCGGACC GCTTTCTGCT CTATGCCATG GACAGCTATT 300

GGCACAGCCG GTGCCTCAAG TGCTCCTGCT GCCAGGCGCA NTGGGCGACA TCGGCACGTC 360

CTGTTACACC AAAAGTGGCA TGATCCTTTG CAGAAATGAC TACATTAGGT TATTTGGAAA 420

TAGCGGTGCT TGCAGCGCTT GCGGACAGTC GATTCCTGCG AGTGAACTCG TCATGAGGGC 480

GCAAGGCAAT GTGTATCATC TTAAGTGTTT TACATGCTCT ACCTGCCGGA ATCGCCTGGT 540

CCCGGGAGAT CGGTTTCACT ACATCAATGG CAGTTTATTT TGTGAACATG ATAGACCTAC 600

AGCTCTCATC AATGGCCATT TGAATTCACT TCARAGCAAT CCACTACTGC CAGACCAGAA 660

GGTCTGCTAA AAGGTCAGAG TAATGCAGAA TGCGTGCCTT CATCTCAGAT TTGTTCATCA 720

CAGGTGGATC CCATGTKTCT TCAGTAGACA AGTCACCTTT GTAGCTAGCA CCAGTGCCAG 780

CTCCATGCCA TTGCACCTTC TTTAGTCTTG ATTGCCCTTC CCGCATTTWT TGGTGTATTA 840

AAATGACTRA TKAAGCTAAT TAAAAGAAGC ATTCAAATCT GCTTTCTACC CTCATTAACA 900

ATTAGCAGGG CACTGGCCAG AGTTTGTACC CTGTGTTTTA CCTTAACAAC ATTCTATTTG 960

CTCTTTGTAT ATTTAAGTGT TGTAAGGAAA CGTGTTTCAA TCAAAACTGA CCATGAGATA 1020

AAGGAAAGAG ATGTGGCTTT TGTGATATTC TATCACAAAC ACTTATTGTA TCTCTGTAAA 1080

ATACAATGTA TGTATGCATG TAAGTGTTTT TGTCCTAATG TTGCTACTCC CATGGCAAAG 1140

AAAAAAAAAA GAATGAAAAA AARAAAAAAA AAAAAAAAAA AAAAAAAAAA CTCGAGGGGG 1200

GGCCCGTACC CAATCGCCCT 1220

(2) INFORMATION FOR SEQ ID NO: 12:

(i) SEQUENCE CHARACTERISTICS:
(A) LENGTH: 1939 base pairs
(B) TYPE: nucleic acid
(C) STRANDEDNESS: double
(D) TOPOLOGY: linear

(xi) SEQUENCE DESCRIPTION: SEQ ID NO: 12:

GAACACAAAC ATGCAGTCTG TAGCAGATGG TAATAGGCTG AYATATTACA CTTGTTGATG 60

TAAATCTGAT AGGTTTCTTT CTCTCCAAGG ACAGCTTTTT AAATATTTAA CAGTATCAAT 120

AATTTTTCAG TTTCTGTGAG AATTTTATAA TTTATAATTT GCAGACTTAA TGTATAATCT 180

ATTTTGTCCT AACAATTACA AATATATTTT TTATTTCAGA TTRTATATAT TCCTACCAGA 240

TGGAGATAAT TACAGCTTTA AAAATTTTTA TTTTTTCATT TTATTTCACA CATTGACATT 300

AAATTTTTAT GGACACATAA TAACTGTACA TATATATGGG GTAGAATGTG ATGTTTTAAT 360

ACATGTACTC AATGTGTAAT GATCAAATCA GGGTAATTTG CATAATGATT TTTCTGTAGG 420

GAGAAAATTC AAAATCTACT CTTCTGGCTA TTTTCAAATA TATAATATGT TATTGTTAAC 480

TATACTCATC CTACTATGCA ATAGGACACC AGAACTTATT CCTGGGTTCT ACATCCGTTA 540

AGGCAACCAA GGATTGGAAA TATTGGAAAA AAAAATTGCG TCTGTACTGA ACATGTACAG 600

ACTTTTTTCT TGTCCTTATT CCTTACACAA TATAGTACAA TAACTATTTG CATGACATTT 660

ACATCGGATA TTATGAGTGA TCTAGAGTTG ATATGAAGTA TATGGGAGGA TGTGCAAAGG 720

TGATGTGCAA ATACTATGTC ATTTTATATC AGGGACTTGA GTATCCTTTG TTAYCCTCAG 780

GAGATCCTGA AACYAGTCCC CCATGGATAC TGAGGGCTGA CTGTATAGTC CTATCCTCAC 840

GGAACTTTCA TTCTAATGRG GGAAGACTGA CTATAAACAA AATATATGTA ATAGGTGGTG 900

GTAAGTACCG TGGAGAAGTA ACAAATGGGG CAAAGTGAGT TATACAGCTC CATYCTTAGA 960

AACCTTGGAG TACTTTTCTT AGTTTATACT CGTGGTGGTT TCCTTTTGTC TCCTTTATTA 1020

CATGGGACTC TGACATGTGC CCATAGCTAG GGTGGCAGTA GGATCTACCC GAAAAGCGTC 1080

CTGCTGATAC AGGACCAAAG CATCCTGTTG TTCTCGAGCC TATAAAAAGA GCTAATGGTC 1140

TTGCTTCTCT TAACTGTGGC CTCCTACACT GTGTTTTGGA TGATTGGTGA TGTCTTGGAT 1200

ATTCTGTTTC TTTGGAACTT TGAATATACA ACACTTTACT AGGGAATTAG CAATGGAAGC 1260

AGAGCAAAGA TGTACAGAGG AAACAATGCR TAACTCTGAT GGAATTGAAG TCATGAGGCA 1320

GCAGAGAGCT TAAATTASAG CTTTAAAAAT TTTTATTTTT TAGAGGGAAT TTAMTTGGGA 1380

GTAACAGCAG TAATAGTTAA CGGAGCCAGA ATGCTTGAGT CATATAATTG CAAAGCAGAG 1440

TTGGGAGCAA CAGATGCTAA AGAGTAGTTG CTGTAGTTCC TCTTTGGGTC GTAGGAGCAG 1500

TTGTCATRTT MCTATAYAGC TACTGCATGA AGAAGAGTTC TTAGTGAGGC CTGGGTGAAC 1560

AGCTCTTCTT AGTATTCTGT GTGACCCCAT TYGACCTTTT AACAAATCCC TAAGTAAATA 1620

AATAGCCCCT MAGGWAAACT AAGTTTTTCT CTGCTGTTTT TTTGCTTGAG AGAGCTATAA 1680

CTGTAATAGA CTTATATTTC TGAACATTTT AGTGCTTGCC AATATTTGGT AATATTTATG 1740

TTTCCTATAT TTGTAATGAA CATTCTTCTT CMGGTACATT TYTTGTTAAA TTATTGTTTS 1800

ATGSATAAAA GTTCACCTTT TATTGTATAA AATTGACTCA GATTAATTTA TACACATTGA 1860

CAATGGGTAA ATAGAGTTTT TCAGATTATT AAAAGCTGAA GGATGCCCAT GTAAGCAAAA 1920

AAAAAAAAAA AAAACTCGA 1939

(2) INFORMATION FOR SEQ ID NO: 13:

(i) SEQUENCE CHARACTERISTICS:
(A) LENGTH: 2602 base pairs
(B) TYPE: nucleic acid
(C) STRANDEDNESS: double
(D) TOPOLOGY: linear

(xi) SEQUENCE DESCRIPTION: SEQ ID NO: 13:

GGGTTCTTCG GGCAACTTTC CTTTCCGGGT GTTCTGAAGC GGTTTTCCTG TAATCGTCAG 60

TGAGGAAACC CACCGTGAAT CGGATTGCCG TTCAGTCCCA CGGAAGCCTG GCTCGTTGGC 120

CATGTNGGGG ACGCATGTTC ATTAAGTTCA TTAAAATAAT TTCATTTGTC TTGGTTTGAA 180

GACTGCTTCA TTCTGCCTCT AGTACCAGCG GTTTCTCTGT TCTGTGATCA ATGTGATTCA 240

CAGGAACTCC TTAAGTAACA AACGAAATGA GCCAGGGGCG TGGAAAATAT GACTTCTATA 300

TTGGTCTGGG ATTGGCTATG AGCTCCAGCA TTTTCATTGG AGGAAGTTTC ATTTTGAAAA 360

AAAAGGGCCT CCTTCGACTT GCCAGGAAAG GCTCTATGAG AGCAGGTCAA GGTGGCCATG 420

CATATCTTAA GGAATGGTTG TGGTGGGCTG GACTGCTGTC AATGGGAGCT GGTGAGGTGG 480

CCAACTTCGC TGCGTATGCG TTTGCACCAG CCACTCTAGT GACTCCACTA GGAGCTCTCA 540

GCGTGCTAGT AAGTGCCATT CTTTCTTCAT ACTTTCTCAA TGAAAGACTT AATCTTCATG 600

GGAAAATTGG GTGTTTGCTA AGTATTCTAG GATCTACAGT TATGGTCATT CATGCTCCAA 660

AGGAAGAGGA GATTGAGACT TTAAATGAAA TGTCTCACAA GCTAGGTGAT CCAGGTTTTG 720

TGGTCTTTGC AACCCTTGTG GTCATTGTGG CCTTGATATT AATCTTCGTG GTGGGTCCTC 780

GCCATGGACA GACAAACATT CTTGTGTACA TAACAATCTG CTCTGTAATC GGCGCGTTTT 840

CAGTCTCCTG TGTGAAGGGC CTGGGCATTG CTATCAAGGA GCTGTTTGCA GGGAAGCCTG 900

TGCTGCGGCA TCCCCTGGCT TGGATTCTGC TGCTGAGCCT CATCGTCTGT GTGAGCACAC 960

AGATTAATTA CCTAAATAGG GCCCTGGATA TATTCAACAC TTCCATTGTG ACTCCAATAT 1020

ATTATGTATT CTTTACAACA TCAGTTTTAA CTTGTTCAGC TATTCTTTTT AAGGAGTGGC 1080

AAGATATGCC TGTTGACGAT GTCATTGGTA CTTTGAGTGG CTTCTTTACA ATCATTGTGG 1140

GGATATTCTT GTTGCATGCC TTTAAAGACG TCAGCTTTAG TCTAGCAAGT CTGCCTGTGT 1200

CTTTTCGAAA AGACGAGAAA GCAATGAATG GCAATCTCTC TAATATGTAT GAAGTTCTTA 1260

ATAATAATGA AGAAAGCTTA ACCTGTGGAA TCGAACAACA CACTGGTGAA AATGTCTCCC 1320

GAAGAAATGG AAATCTGACA GCTTTTTAAG AAAGGTGTAA TTAAAGGTTA ATCTGTGATT 1380

GTTATGAAGT GAATTTGAAT ATCATCAGAA TGTGTCTGAA AAAACATTGT CCTCAAATAA 1440

TGTTCTTTAA AGGCAATCTT TTTAAAGATT TCACTAATTT GGACCAAGAA ATTACTTTTC 1500

TTGTATTTAA ACAAACAATG GTAGCTCACT AAAATGACCT CAGCACATGA CGATTTCTAT 1560

TAACATTTTA TTGTTGTAGA AGTATTTTAC ATTTTCATCC CTTCTCCAAA AGCCGAATGC 1620

ACTAATGACA GTTTTAAGTC TATGAAAATG CTTTATTTTT TCATTGGTGA TGAAAGTCTG 1680

AAATGTGCAT TTGTCATCCC CACTCCATCA ATCCCTGACC ATGTAAGGCT TTTTTATTTT 1740

AAAAAAACAG AGTTATCCCA ATACATTATC CTGTGATTTA CCTTACCTAC AAAAGTGGCT 1800

CCTGTTTGTT TGATGATGAT TGGTTTTATT TTTGAAATAT TTATTAAGGG AAAACTAAGT 1860

TACTGAATGA AGGAACCTCT TTCTTACAAA ACAAAAAAAA GGGCAGAAAT CACCCCAAGG 1920

AACGATTTCT CAGGTTGAGA TGATCACCGT GAATCCGGCT TCCTCTGAGC ATTCGATGGC 1980

CTTAGCACCT CATCAAGCCA GCACATCCTG CCTGCTGTTG CAGCCTGGCT GGGTTTATTC 2040

TTCAGTTACC CTAATCCCAT GATGCCTGGA ACCTTGATTA CCGTTTTACA TCAGCTCTTG 2100

TACTTTTCAG TATATTTTCA TAATGAGTTA TATTGTCATT TAGACTTTGA ACAGCTCTGG 2160

GAAATAGAAG ACTAGGGTTG TTTCTTAAAT TTAGCTCATG TTATAATAAA AAGTTGAAAT 2220

GAAGTTCTTA TTCTAAAAGT CTGAATGCTT AGAACAAACT TAACATGTTT ATAGAATATG 2280

GTCTCTTTGT ACCAAGTACT TTGCTTAAGA GCTCCTTTGG GCCACTACAT ATTTTGGTTT 2340

CTAGAAAATG TTTGTTTATG AAGAAGTCGA TGGAAAACTG CAAACATATG CAGAAAAGGT 2400

AGAATAATAA AAAAGGTCTA ATGAACTCCA TTCAGCTTTG AACCTATCCA CTCATAACCA 2460

TTGACTGGCC TTTTAAAAAA AAGTATTGGG CAGAATTAAA TTTCCACCTA GGTGATGGGG 2520

AAGGAAAGTG TTCGCCTGTN CCAGCCTGTG GTTCCTGCCT GGGNGGTTTA CCCAGTGGTG 2580

GCGCCAGGCC AAGGTCCATT CA 2602

(2) INFORMATION FOR SEQ ID NO: 14:

(i) SEQUENCE CHARACTERISTICS:
(A) LENGTH: 808 base pairs
(B) TYPE: nucleic acid
(C) STRANDEDNESS: double
(D) TOPOLOGY: linear

(xi) SEQUENCE DESCRIPTION: SEQ ID NO: 14:

ACCCACGCGT CCGGTTAAAC AAAGGGAATG ACGATATGGG AAAGAAAATA CATTTGGATG 60

TTACAGATAT GTGTGTTCCT GGAGCCCAGG GCCAAGCCCT CCCTGGGGGA CTTGGATTGG 120

TGATCTCTCT CCTTGGCCCC AACCTGACAT CTTTTCTTGT CCTTTTAGGA ATGTCTGATG 180

GAAATTCCTC CTAACCTGGG GTCATACTCC ATTTCATTCT CTGGGCTCAN TGAGAAGGAA 240

AATTTTTTTT TAAGTAATTT ACTGAAAACC CAGATCACAC CATCATAAAT TCAGATAGGT 300

GCAATTCTGC CCACAATGAA GGCAAAGTGT TACACTAATT TGAAAACAGT TTAGCCTCTT 360

ATTCCCCCAA ACTTCATTCT TGAATTTTGT CATTTTTTGT GGGCAAGCTG TGGGAAAGGG 420

GCACAAAAGT ATCACTGAAG TATTTTTTCA AAAAAGAAAA AAGGCAGTCT TCCTCTACTA 480

ATGAGAATGC AAAATGTTGA ACAACTGTAA AATGTTTTCA CCCTGCTTTT AGACATAAAG 540

CTTTAAAAAA CTGTGAGGTC TTTTATCACT TCCCCATTGT ATATGTAATA TGGCTCCAGA 600

TAATTACTCT GCCACGGGGA GAAAATCTTC CATAACTCTC CCCTATATAT ATGTATACTC 660

CACCACCTTA TCTTGTTATG TCATGGTGGT GGGAGTATTT ATMCCACAGA AACAGGCAAA 720

TGATACAAAC CTGGGCGACA GAGCAAGACT CCACTTCAAA AAAAAAAAAA AAAAAAAAAA 780

AAAAAAAAAA AAAAAAAAAA GGGCGGCC 808

(2) INFORMATION FOR SEQ ID NO: 15:

(i) SEQUENCE CHARACTERISTICS:
(A) LENGTH: 864 base pairs
(B) TYPE: nucleic acid
(C) STRANDEDNESS: double
(D) TOPOLOGY: linear

(xi) SEQUENCE DESCRIPTION: SEQ ID NO: 15:

GGGTTTTTTG TTTTTGTTTT TTNAGGGGGG AGGGGGGGTT TCCCCTCCTT TGCCCCAGAC 60

TTCTCTTTGA ACACAAATGC ATTAGCCTTG TGGCTAGAAM ACCCTCTTCC TACCTCTGTC 120

TCCCCTCACT TGTCATATGC TCTGACATGC TAACATTTCT TTTGTTCATC CCTGTTGCCC 180

CCACAGAAAC ATCCCAGAAA AACCGGTCAG TGTTCCTTCC TCCCTGATCC TTAGGTTTCT 240

GAAATAGGGT TCTGTTACAT CCTCTTCGAT AGCCTGTTTA AAATGTTTAG AAGGTCTGGA 300

GCTCAAAAAT GCGTTCTTCC ACATTGATAA TTTAGTAAAC TGAGAACATT GACATCACTA 360

CAGGGCAGCA TAAGAGGTTG CTTACATGTG GTAGCAGCTC TGGTTTGATT CAAGTTGCTA 420

CCATGTACAT TGACAGCACA TATACCATAA CCAGCGTGTT GGGTTGAATT GCACTTTCTA 480

CCTTTGTATG AGATTTACAG ACTTTCCTTC TGGGTTTGTA TCATGACCAG AGGGGTACTA 540

TAGGGTTGGT TTATACTGCA ATATAGAGGA TCAGAAGCCA TTTGATTTGG TAGGTGTGTC 600

AGAAGGGAGA ATGATGGCAG ACGAACTGCT GGAAGAGGTC AGAAGATAGC CATGCTAAAA 660

TGCAATTATA TCCTCATGTT TATCCCAAAC TAATCTTGGA CTTTTCCACT CATTAGCTTT 720

GTTTTGCCCT TGTTTCCCTT GAAGGTTTAA GTTCAACCAT ATTCTGTCAA CTGTTCAGTT 780

TCAGTGGAAT CTTGTATTTC TGGTTCATTA TAACAAATTG TTCGCTTAAA AAAAAAAAAA 840

AAAAGGGGCG GCCGCTCTAG AGGG 864

(2) INFORMATION FOR SEQ ID NO: 16:

(i) SEQUENCE CHARACTERISTICS:

(A) LENGTH: 2361 base pairs
(B) TYPE: nucleic acid
(C) STRANDEDNESS: double
(D) TOPOLOGY: linear

(xi) SEQUENCE DESCRIPTION: SEQ ID NO: 16:

GGCACGAGCT CGAGTTTTTT TTTTTTTTTT TTTCTATTTT TGCCAGACTC TTGATACTCT 60

TAAAACTTGT TTGTGGTCAG CACAACAAGG AACAAAACAA AGCTTTGAAA AAACTTTAAC 120

ATGAAAAAAC GCACTGACAT TTTTTTTTAT TTAATATAGC CTGGACTTTA CCTGCGTATG 180

CACATGCTCA GAATTGTCTA CTAGGCTGAC TATGTATCAC CTCTTCAGCT TGGATCCAAT 240

TGTGGATTTA TTTACAAACA TCAAATGCCT TCAAGCCAAT CCTTTTTGCT GTATGTTTTG 300

CAGCCTACTG TAGTAGATAC GCAACAGATA WTGTGGGAAA AAAAGAGATA AGAGGAGGAA 360

GCTAATAAGA GACTGTCAAG ATTGTATACC TTCTTGGTTT CTTTTAAGAA TTTGTTGCCT 420

TTCTACTATT ACAGCAAAGC AGCATTTTGT TACTGACTGC CTAAAATCAC TTAATCTCAG 480

GTGAACGCAT CACTTGCCAA ACTGTTGGAA TGCTATTTGT GTTTTGTTGC ACTGTTTTTT 540

TCGTTTGTTT GTTTGTTTAT TTGGTTGGCT TTTTGGAGAG GGAAATTTGG AAACGGGACA 600

TACACAAAAG TTACACACCC ACATTCCCTT TTTATCATGA CATACAAGAA GAAACTAGCA 660

GAGCTAAGAA TGGAGTGAAG AAAGGCAGTA TGGCAGGCAC CAGCAAAGAG TTGAGGGCTG 720

TTGCTCTTAA AAATTATTTT TTTTATTATT ATTTTGAAAG TATGGAAGTT TTCCATTCAC 780

TGGGGAAAGG AGGGAAAAGT GCATTTATTT TTATACAGAG TTACTTAATT ACCTCCAAAA 840

CACATATGTT GGAAATCGCT TTTGCTGGTG CAAAGTATAT TAATGAGCAG GAATACATAC 900

ATTGAGGTTA TGAATAGAGA GCTCAATTTG TACCTTTGCT GTCTTGCTCA AGCTTGGTAT 960

GGCATGAAAA CTCGACTTTA TTCCAAAAGT AACTTCAAAA TTTAAAATAC TAGAACGTTT 1020

GCTGCGATAA ATCTTTTGGA TTTTTGTGTT TTTCTAATGA GAATACTGTT TTTCATTACC 1080

TAAAGAACAA TTTGCTAAAC ATGAGAAATC ACTCACTTTG ATTATGTATA GATTACATAG 1140

GAAGAACAAT CACATCAGTA AGTTATAGTT TATATTAAAG GTAATTTTCT GTTGGCTCAT 1200

AACAAATATA CCAGCATTCA TGATAGCATT TCAGCATTTT CCAAGGTACC AAGTGTACTT 1260

ATTTTGTTGT TGTTGTTGTT GTTGTATTTT AGAAGGAATT CAGCTCTGAT GTTTTTAAAG 1320

AAAACCAGCA TCTCTGATGT TGCAACATAC GTGTAAAATG GGTGTTACAT CTATCCTGCC 1380

ATTTAACCCC ACAGTTAATA AAGTGGCTGA AAATAATAGT AGCTCTGGCT TGGTGCTTGA 1440

CCTGGTTAAA TACTGTCTTA AAGCTCATAC AAAACAAATA GGCTTTTCCA TAAGTGGCCT 1500

TTAAGAAAAC ATGGAAGACA ATTCATGTTT GACAAATGCT GACAGGGTGA AGAAAGCCCA 1560

GTGTAAAAAT GAATCGCGTT TTAAGTGATT CGGTTAAAGA GTTTGGGCTC CCGTAGCAAA 1620

CTAATACTAG ATAATAAGGA AATGGGGGTG AAATATTTTT TTATTGTTGA ATCATTTTGT 1680

GAATGTCCCC CTCAAAAAAA GCTAATGGAA TATTTGGCAT AAAGGGCATT TGGTGGTTTT 1740

ATTTTTGTTT GAGGGGGWTT GTCAGAAAAT CCCTTTTCTC TCTTACGYCT AACTGACTAG 1800

GGAACAATTG TTGATATGCA TAGCATTGGG AATACTTGTC ATTATATACT CTTACAAATA 1860

ACACATGAAG CAAGAATGAC CAATATTCTG NATAATTGGG CACTGGGATC ACAAAATGTG 1920

ATAAAACTTT AAATGTATAA AACTTTATCA AATAAAGTTT TATTTTCCCC TTTAAAATGT 1980

ATTTCTTTAG AGGCATTACT TTTTTAAAAA TATTGGTCAA TTCCTGACAT AAGATGTGAG 2040

GTTCACAGTT GTATTCCAGT ATTCAAGATA GATTCCTGAT TTTTCAATTA GGAAAAGTAA 2100

AATCCAAAAT GTTAGCAAAA CAAAGTGCAA TATTAAATGT TTGCTTTATA GATTATATTC 2160

TATGGCTGTT TGTAATTTCT CTTTTTTTCC TTTTTTATTT GGTGCTGAAT ATGTCCTTGT 2220

AGGCTCTGTT TTAAGAAAAC AATATGTGGG AAATGATTTA ATTTTTCCTA TTGCTCTTCC 2280

TTGTGGAAAA TAAAGTGTTT TGTTTTTTTC TGTTTTGTAA AAAAAAAAAA AAAAAAAAAA 2340

AAAAAAAAAA AAGAANGAGA A 2361

(2) INFORMATION FOR SEQ ID NO: 17:

(i) SEQUENCE CHARACTERISTICS:
(A) LENGTH: 803 base pairs
(B) TYPE: nucleic acid
(C) STRANDEDNESS: double
(D) TOPOLOGY: linear

(xi) SEQUENCE DESCRIPTION: SEQ ID NO: 17:

CAGCTGCCCA CAAGGTGGGC TCCTGGGGGA GGGTCATCCC TCTGAGAAGA GGGCGGCACC 60

AAGACCCACA CACCTGAAAA ATGTGGTACT TCATGTCGCT GATCTCGATG GTCTTGCTGC 120

TGTCCCCATC CTGTTCTGAT TTATTGGTCA TTAGTGTCTT GAACCTGGAG CAAAGGAGAC 180

AAAGCAAGGT GGGTTTTGAA CCTTTTACTT CACCACTGTG TGGCGNATGG CACCATCTGT 240

CACCTGACCG GCTACCACAA GACGGAACAT TTTAAAAATT ACTGCTGTGC TCCTAAAATA 300

ATTTTCAGCA AGTGCCATTT TACACCATCT TAGGAAGACA TCTGAGCTGA GCCCAATTCT 360

GTCCCCACCA CCCACCCTAC AAGCGACCTG ACGCCTGTGG CCAGAATGCT GACTCTTCAT 420

TCCAGGATAT TTATGTTTTC TAATAATAAA AGCAATAACT AGGCCAGAAA GAACACCACC 480

TCAGAGCCCC CCTTTCCTGC TGCCCTGGGT CCACCCCGTC TCATCCCGCT GTGGGGCGAG 540

TGGGGCTCTG CTGCAATGTG ACTGCAGTCT GAGGGGCAGA RGCTGCAGGK TACAGCCCCA 600

GCGAKTCACT CTCTGTCACC TGGAATCTGA AACAAGGTGC TTCTGTGCCC CTGGGCTGGG 660

AGTTTGTTAT CTGAGGCTGC CTACCTGTTA GAACNTGTCA CCAGCAGGAC TTTATGTGCA 720

TAAAACAGCT TTCCTTCCAC CAAAAAAAAA AAAAAAAAAC TCGAGGGGGG GCCCGGTACC 780

CAATTCGCCC TATAGTGAGC GAT 803

(2) INFORMATION FOR SEQ ID NO: 18:

(i) SEQUENCE CHARACTERISTICS:
(A) LENGTH: 1794 base pairs
(B) TYPE: nucleic acid
(C) STRANDEDNESS: double
(D) TOPOLOGY: linear

(xi) SEQUENCE DESCRIPTION: SEQ ID NO: 18:

TTCTTTTTTG TTCATGGGAC ATGGTACCTA AGCAAATAGG AGTTGGGTTT GGTTTTTCTC 60

CTAAAATAAT GCTCAATACT TACCTAATCA AATGGCATCC ATTTGAATAA AATGACAATA 120

ACTAAAGCTA GTTAATGTCA GTGACATTAA ACTAACTCCA GGATTCAGGA GTTTTAATGT 180

TAGAATTTAG ATTTAACAGA TAGAGTGTGG CTTCATTTGT CCATGGTAGC CCATCTCTCC 240

TAAGACCTTT TCTAGTCTGT CTTCCTGCCT TCGAACTTGA TGACAGTAAA ACCCTGTTTA 300

GTATTCTCTT GTGCATTTGG TTTGTTGGTT AGCCGACTGT CTTGAAACTA TTCATTTTGC 360

TTCTAGTTTT ATTTTACAGA GGTAGCATTG GTGGGTTTTT TTTTTTTTTT CTGTCTCTGT 420

GTTTGAAGTT TCAGTTTCTG TTTTCTAGGT AAGGCTTATT TTTGATTAGC AGTCAATGGC 480

AAAGAAAAAG TAAATCAAAG ATGACTTCTT TTCAAAATGT ATTGTTTAGC ACTTAACTCA 540

GATGAATTTA TAAATTATTA ATCTTGATAC TAAGGATTTG TTACTTTTTT GCATATTAGG 600

TTAATTTTTA CCTTACATGT GAGAGTCTTA CCACTAAGCC ATTCTGTCTC TGTACTGTTG 660

GGAAGTTTTG GAAACCCCTG CCAGTGATCT GGTGATGATC TGATGATTTA TTTAAAGAGC 720

CGTTGATGCC TCCAGGAAAC TTAAGTATTT TATTAATATA TATATAGGAA TTTTTTTTTA 780

TTTTGCTTTG TCTTTCTCTC CCTTCTTTTA TCCTCATGTT CATTCTTCAA ACCAGTGTTT 840

TGGAAGTATG CATGCAGGCC TATAAATGAA AAACACAATT CTTTATGTGT ATAGCATGTG 900

TATTAATGTC TAACTACATA CGCAAAAACT TCCTTTACAG AGGTTCGGAC TAACATTTCA 960

CATGCACATT TCAAAACAAG ATGTGTCATG AAAACAGCCC CTTTACCTGC CAAGACAAGC 1020

AGGGCTATAT TTCAGTGACA GCTGATATTT GTTTTGAAAG TGAATCTCAT AATATATATA 1080

TGTATTACAC ATTATTATGA CTAGAAGTAT GTAAGAAATG ATCAGAACAA AAGAAAATTT 1140

CTATTTTCAT GCAAATATTT TTCATCAGTC ATCACTCTCA AATATAAATT AAAATATAAC 1200

ACTCCTGAAT GCCTGAGGCA CGATCTGGAT TTTAAATGTG TGGTATTCAT TGAAAAGAAG 1260

CTCTCCACCC ACTTGGTATT TCAAGAAAAT TTAAAACGAT CCCAAGGAAA GATGATTTGT 1320

ATGTTAAAGT GACTGCACAA GTAAAAGTCC AATGTTGTGT GCATGAAAAG GATTCCTTGG 1380

TTATGTGCAG GGAATCATCT CACATGCTGT TTTTCCTATT TGGTTTGAGA AACAGGCTGA 1440

CACTATTCTC TTTGATTAGA AAATAAACTC ATAAAACTCA TAATGTTGAT ATAATCAAGA 1500

TGTAACCACT ATAAATATGT AGAAGAGGAA GTTTTAAAAG ACCTTAAGCT GGCATTGTGA 1560

AGGAACACCA TGGTAGACTC TTTTTGTAAA TGTATTTTGT ATTTAATGAA ATGCAGTATA 1620

AAGGTTGGTG AAGTGTAATA TAATTGTGTA AACAAATCCT GTTAATAGAG AGATGTACAG 1680

AATCGTTTTG TACTGTATCT TGAAACTTGT GAAATAAAGA TTCCACCTCT GGTTAAAAAA 1740

AAAAAAAAAA AAYTCGGGGC CAGTTCCCCC CCGGCTATTT TAAAAGGNAA AAAG 1794

(2) INFORMATION FOR SEQ ID NO: 19:

(i) SEQUENCE CHARACTERISTICS:
(A) LENGTH: 1037 base pairs
(B) TYPE: nucleic acid
(C) STRANDEDNESS: double
(D) TOPOLOGY: linear

(xi) SEQUENCE DESCRIPTION: SEQ ID NO: 19:

TCGAGTTTTT TTTTTTTTTT TGACAGAGTC TTGCTATGTT GCCCAGGCTG GAGTGCAGTG 60

GCAATCTTGG CTCAYTGCAA CCTYTGCYTC CTGGGTTCAA GCAATTYTCC TGCYTCAGCY 120

TCCYTAGTAG CTGGGACTAC AGGCACCTGC CACCATGCCA GGTTAACTTT TTGTATTTTA 180

GTAGAGACAG AGTTTCACCA TGTTGCCCAC GCTGGTGTCG AACTCCTGAG CTCAGGCAAT 240

CTGCCCACCT TGGCCTCCGA AAGTGCTAGG ATTACAGGCT TGAGCCACTG CACCCAGCCA 300

AGCTGTACTT TTTTTTTTTT TTTTAAAGCT TCAAACCTTC AATATTTCAT TAAGAGTTAC 360

AGTTTGGTTT CAGTCATTCK GAGGRAAATT AAGGAAGGGG CTTGGCCCAW ACCTGGTAAA 420

AGAATGGAAG GAACCAATTT TTAACCATTT GGACCAGTGA TTYTCAATGG GAGTGCTTTT 480

TGTCCCCCAG GAAACATCTR GAAAGGTATA WKGAGATATT TSTGGSTTGT CACAATTTGT 540

GATGGGGGAA AAAAGAACTA CCAGTATCAG GGGGATACAG GCCCGGTATC AGGTGGATAG 600

AGGCCTGGAA TATTGCTAAA CATTCTACAG TGCAAAGACA SCCTTTMACA WACAGAACTA 660

TYTGGTCCAA AATGTCAATA GTGCTGAGGT TGAAGAACTC AATATTTTAT ATGTTTTCAG 720

GGAATTTCTA TGTGGGCTTG GGAAAGTTTG AAGTCAATTG TCATTTGTAT ATTTAAAGGG 780

ATATATTTTA TCATTAGTCT ATAAATTCCA GTTGCAAAGT AGAGGCCCTG CACATTTGTG 840

CACATATACA CACACCAGAA ATAAAYTMTC TKGCAATTAT CTTCTCTATC ATTGACAGGG 900

CAATGACCTA TGAAAATTAT GTTATGTCTA ATAGTCCCTC ATTGTTATGT GCAAAACACC 960

CAGCAAAGCT CAAGTTAAGR TTGTGGTCAC AAAGAAAAGA GCTATCATTG CTTTATGATG 1020

TTGTCTGAAG TTAATGA 1037

(2) INFORMATION FOR SEQ ID NO: 20:

(i) SEQUENCE CHARACTERISTICS:
(A) LENGTH: 1309 base pairs
(B) TYPE: nucleic acid
(C) STRANDEDNESS: double
(D) TOPOLOGY: linear

(xi) SEQUENCE DESCRIPTION: SEQ ID NO: 20:

GGCACAGACT TTAAGAAATG CCAAATGCAA GGACCATTAA GAAAATTCTC CCCGAAATGA 60

GGCTCCTCTA ACAAATGATG ATTANAACGC TCTCTCCTTG AGCAGTCACA TTCTAGAAAC 120

ACGACATTCC ATGAGGCAGG AAGAGTTCAG TTAATTTGCT CCKGAAAAAG TGTGGTTCAG 180

TGTTTGTGTG GCAATGTACG TGGGCAGAAG AGGCCGCTCA AGCTGTGTCC CCCCTGAGCA 240

GGATTCAGGA AAGGGAAAAG AAGTTCTCTT CAACTCAGCC AAGGGGCCGT ACGATGGCCG 300

ATGAGATTAT GTATTTAAAA GTTCTTTGTA AAGTGTAAAC TAAAAACCTT AAATGTAAGA 360

TGCTGTTGTT ATTATTACTG TTGTTGTTGC TGTTATGGAC ATGCCAAAAG GCCCTTGTTA 420

GAAGACAGTT TTGCCTTTTC AATCTCATAG CAAGGAACTC AAGTCTGATG CTTCAAAAAG 480

ATGAGAAGAA GGGCAAGAAG AGGGATAACT CCCAAGCTCA GAGGGAAAAA AAAGGTGGGG 540

GAAAAGAGCC CCAGGGTGAC CTTCAGGAAA GGCCAGGACC AGGATGATCT AACCTTTCCC 600

TTCACCAGAA ACAAAGCTAT TGCCAGACTG AACCCTAAAG TCAAGCAGTC ACCCACTGCC 660

TTTGCTGGGA GCAGAAGCCC ATAGCAACAA GTGACCTGCC CCTCAGACTC AAGATCCCAG 720

ATACCAGAGC TGGAGGAGTC ATAGGGCATT ACTGGTAGGC AGGAAAACTG AGGGTCGAAC 780

AAATGGAAGA ATGCGGTGAT CATAGACCAA AGACACACAG ATAATTAACC CCATGTGTCC 840

ACCCAGGCCA AAGTTCTTCC TGCTACCCCA CAGTGGATGT CCAGGCAGAT GGTCCCCACA 900

TGATGGGGAA GCAGAGGGCA TAGTGTGGTT TTGTGGACTT TGTTCATGTT TTGTAGTGTG 960

GGCTCAACAG TGCCAAAGGA AACACTAGGG AAAAGTTGGT GAAACATGCC AGCTAGCAGG 1020

ACCAGTAAAG GCATAATCAG GCATTTGGCA AAGCTTGCTT TTCTAATTCA ATGATAGGTT 1080

CTAATAGGAA ATTTTTGAAG ATTTTTTAAA ACAATGTTAT AGTGGCACTT CCCCAGTATG 1140

GAATAAATAA CATGCATTCT TTTTTCAATA TACTGTCATA TTCAGATGTC ATTAAAATAA 1200

ATGGATGAGT CACAGAGGAG CTATCAGATG CTCTCATGAC TACCATAACT CAAAAAAAAA 1260

AAAAAAAAWA AAAGGGGGGC CCGTACCCAT TTGCCCTAAA GGGATCGTA 1309

(2) INFORMATION FOR SEQ ID NO: 21:

(i) SEQUENCE CHARACTERISTICS:
(A) LENGTH: 1081 base pairs
(B) TYPE: nucleic acid
(C) STRANDEDNESS: double
(D) TOPOLOGY: linear

(xi) SEQUENCE DESCRIPTION: SEQ ID NO: 21:

ACANATNTTT TACTTAAATT TTATTTTATC TTATTTTTAG GTGCTTTTAA TCTCAAAATT 60

CTGAAAAGCG AATAGCACGT GTTTTCAGAA ACAAATGTGA AAGCAGTCAA ATTAAGTAGA 120

TACTATTTAG AAATGTAAAA TACTCTCCAG ATCTACCATT AATAGAAAAT AAACTAAACC 180

TTATATTTTA TTTTTGCCAA AATATTTTAT TATAAAATAT GACCAAAATA TTTAAAATGC 240

ACAATGCTTT TAACTTAAAT GTGCTAACCC TGTTTCTGTC TGTTTTGTGC TGTACCTTTT 300

CTGATTCMGA ATTATAGAAA ACTTGATAAA TACTTGATTT TAACCAATGA GACTACAGGC 360

AGATGGGACT AAGTGTTTAT GGGACAATTA TGTACTATTT AACTTAAATA TATTTTGTTT 420

AATAGGAAAT ATATAATAAT AGCATTTTAT GTAATAAAAT ATGGGCAACG ATTATCTTGG 480

AAATTAAAGA GTCAAAGCAA AGAAATGAAG GGCTGGTAAA ATGAATTTTG TAATATCCTC 540

AGGATACTTT TATCTTAAAA GTATGTTGTT AAAGATTTTG TAAATTGTAT TTCAACAATT 600

TTAAATGTGT TGAGCAAGTT GCAGTGCAAA CACTGTCATT ATGTAGAGAG TTTATATGCA 660

CATAATAACC TGTACCTATA AATCGTGCAA TAACCATATG CGACTATTTT GCCATGGAGA 720

AATCTGACAG CATTGCAAAC AATAGTATTG TTTGATGTAG TTAACCTTAA GTTATTTTTC 780

AGTAATTTCT TCACAAATCA AGATTCAAAC AGCTTTAAAC ACTTCCAATG AGATAAAATA 840

TTTACTATTA TGCTTATTAG AACAAAAGGT GTTTAAGGAT GAACTAAATA TTTTAATTGA 900

GCATTTATAT GGATAATCAT ACATTATGTA AGCCCATATG TATTTACATC CAGAGTCATA 960

ATATTTTAAA TAAACAATCA TGCAGAAACT TTTTTAGGGG GTATACTATT GTTTTAATAT 1020

CGTTGCCAAT TTNGCTGACT TAAAATATGT GACATTTTAA AATCAGGATT TTCCATATTN 1080

G 1081

(2) INFORMATION FOR SEQ ID NO: 22:

(i) SEQUENCE CHARACTERISTICS:
(A) LENGTH: 807 base pairs
(B) TYPE: nucleic acid
(C) STRANDEDNESS: double
(D) TOPOLOGY: linear

(xi) SEQUENCE DESCRIPTION: SEQ ID NO: 22:

GAATTCGGCA CGAGCTCCTT CAGAAATGTC TTGGCTATTC TTGCTCTTTG CTCTTCTCTG 60

TAAATTTCAG CATAAACTTA RTTTCCATAA TATATGACTG GAAATTTTAC AGAAGAGTTA 120

ATGTGTCTAA CTAGCAAACA CGAAGAAAAG CTCAGTGTTA GCAGTTAACT GAGGGAATGC 180

AAATCAAGAC CACAAGGAGA TAACAATTTG AGCCTATTGA CAAAAGTTCA GAAGTCTAAT 240

AATACTAAGT GTTGGAGAGG ATATGGCCCA GTATGATCTT ATCCACTGTT GGTGGGAGTA 300

TCAATTAGTA CAAACACTTT GAAAAATAAG ARGGAATTCT ATAATATCTA ACATTTGCAT 360

ATATCCATTT ATCTCTCTAG ATCTAGATCT TAGCCCTCTC CACCCTGCAC TGTGTTCTTG 420

GAAGGGGATC ATGAATGGTT TCCTTGCATT CTGCCTTCTG ATTTGGTTCA GCCAATGAGA 480

GACCATGGCA AGACATTTGT GAGAAGGGTA GAGAGTCAGG TCAAGGTTCT TAGTGAGATC 540

AACTCTTTCT CTGCCAGTTT GTTAACTGAA TTCTACTGAA AGCTAGAGCT CTGTTGAGTA 600

ATCTTTTAAA GCTGCAGCTA CCCTTTTGAG ATTAAGTAAT AGCTCCCTGT TTGTGCCTTG 660

TTAGGGCTAG GGATGTTTAA GGATCCTTGC CCTTGCTAGT CCTAGCATGT TTTGTTGTCC 720

CATAATAGTT CTTTTTTTAA ACTTTCCTCA ATTACACAAT TTGATCTTGT TCCTACCAGT 780

ACCNTTGCTG GTACAACCTT AAACTGG 807

(2) INFORMATION FOR SEQ ID NO: 23:

(i) SEQUENCE CHARACTERISTICS:
(A) LENGTH: 632 base pairs
(B) TYPE: nucleic acid
(C) STRANDEDNESS: double
(D) TOPOLOGY: linear

(xi) SEQUENCE DESCRIPTION: SEQ ID NO: 23:

GAATTCGGCA CGAGTCTAAC AGCATAAAGA AATAACAGCT GCATTCAAGA CCAGGATATG 60

TAAAATAATT TGTTTAGTTT CAGCCACTTT TTAAAGTCAA TTTTACACCC TGAAAGAAAG 120

GCAATCCTGA CTCCATTGTT CTTTCGCCAA TAAGGAGATC GGGAATTACA ATAATAAATA 180

GAAGAAAGAA TGTTGCTTTT CCTCACTGTA ATTAATTTTA TGGCTCTTGC GAAGATGAAT 240

TTTTGTGGTG ATTAAAATAG TCCCTTGCAC ATATTAGGTA CTCAGTAAGC ATTTGTGAAA 300

TAGGGACTTT CTAGCCTTTA TTTGTGTTTA AGGAATCAGG GAATAAGTTC AAAATTGCCT 360

TTCAAGAAAT TTTTGGAACT CTCTTCTCAC TAAGAAACTG TAAAGTCTTA TAAAAGAGAC 420

ATTATTTATT TTCTCCAAGT ATTGCTTGCG AGGTGAATTG AAGGTTTTTT TTTTATCAAC 480

AGTTGTTTTA TAAGATCGTT TGAGGACTAA AAGGGCTGAT TGTAATCACC TGTAACATGT 540

TACCCAGCAA GACATTCCTC ACCAGGTTGA AGTAAAAAAA ARAAATGAAG TGAGAATATC 600

AAGCTTATGC AAGTTTGAAA TTNCAAACAA GA 632

(2) INFORMATION FOR SEQ ID NO: 24:

(i) SEQUENCE CHARACTERISTICS:
(A) LENGTH: 1358 base pairs
(B) TYPE: nucleic acid
(C) STRANDEDNESS: double
(D) TOPOLOGY: linear

(xi) SEQUENCE DESCRIPTION: SEQ ID NO: 24:

GGCACGAGGA TAAATTGCAA GTATTAATCG GTCCCAACTT TAATATGGGA TAAAAATAAC 60

AGTCAGTATG TGACCTCCTA AACAATCCCT CTACTGAGCT GTGGAGGGGA GAAGGGAGGT 120

CCTGGGGCCA GGACAGACAG GGCTATTTTC AGTAGTACAA CTTATATGCT ACTCTAAGAA 180

AAGTCCAGAA AATGCRATTC TCTTCATACG AAGTCTTARA TACCCTCATK ATTTRGATAA 240

ATACATTTTC ARRTCTAATA TGGAGACAGA AAGCTGCCTA GATTTATACC CACAAGTATT 300

ATAAATTTAG AGAGTCTGAC CAGCCTCAAT TATTTCTCTT CGAAGTGGGA GAGAGAAATC 360

AAAAGTCAGA AATGGTGGRT AATCTCCAAG TCATATCCAT TTGGSTTTGR TCTACTACTT 420

GTTTTTATGC TTGTATTTGG RGRCAAGGRT GCCTGATGTT AAGGGRATTT CMTACMTTGA 480

ATAATGTGAC CAGACTGCCA TCTAGTCAAA AACCTATAAA ATGTTATTTA CTTTAATTCT 540

GGGCTAATTC AACAGAAGTY YYSGATAAAA RCTCTCCAAA CAATAATTAT GARCCTTAGT 600

TTTTTGTTTT GTTTTGGATA CAAAACAAAA CAGCTCTGTA GTTGTTCTGT GAGGTTTATA 660

AATAGATTTT TTTAACTACT TAATTTTCYG GTTTCYGCCY CTGKGTTTYC TGTACCTATA 720

GAGGTAGCTC TTTTCAGTTA AGTAGAGAAA AGCTCTTCCC CTGGGTTGAA AATAATGCAG 780

TCCCGAGAGG CTACTTAACT CTACCTTTCT GGAGGTCATG GTAGCAATTG GAGATCTCCC 840

AGGCATTCTA AGGGGAGCTA CTAAAGAGCC CCAGATACTC AATTTACCAC TAGAAATTCG 900

CTTCATCTAC TCTCTGTCAT CTGGGGAGRA AAGTATTATA ACTGACATTC AGTATGCACA 960

CAATAAGTGC ATAATAAAGA GCTATTGAGG GGATCCAAGG GAGTAAAATG GGTTTGCCCA 1020

TAGGACTCCA TCAGGGTCCA CCAACACAGA CTTACAGCAA AAATTGGAAG GCTCTTTTCT 1080

GCTGGATTCT GGGAATCTGT GTTCTCTAGT GTGCCAGGGA GAGTTGGAAT CAAAACACGT 1140

AATATAATGT TTCTATTCAG AGCCCCATTT TTTTGCCAAA TAAAGTAGCA CTGTCAAATA 1200

ATAAATCTTG TATTCACTTG GGCATGTATG TTTATTATTG GATCTCTAAA ATATGCTTCA 1260

AATAATGCAC TGAAATAAGT GAGGTGATGA ATTTTGAAAT AATAACAGTT TATGATGGGT 1320

AGCTCCAAAA TTTTTAAAAA AAAAAAAAAA AAACTCGA 1358

(2) INFORMATION FOR SEQ ID NO: 25:

(i) SEQUENCE CHARACTERISTICS:
(A) LENGTH: 1376 base pairs
(B) TYPE: nucleic acid
(C) STRANDEDNESS: double
(D) TOPOLOGY: linear

(xi) SEQUENCE DESCRIPTION: SEQ ID NO: 25:

CCCACCTTTA GCGAGCCAAC GAGAGAACAC CGCCTGCAGC TAGAACAGCC TGGTCAGGAG 60

CGTAACGGAG TGGTGCGCCA ACGTGAGAGG AAACCCGTGC GCGGCTGCGC TTTCCTGTCC 120

CCAAGCCGTT CTAGACGCGG GAAAAATGCT TTCTGAAAGC AGCTCCTTTT TGAAGGGTGT 180

GATGCTTGGA AGCATTTTCT GTGCTTTGAT CACTATGCTA GGACACATTA GGATTGGTCA 240

TGGAAATAGA ATGCACCACC ATGAGCATCA TCACCTACAA GCTCCTAACA AAGAAGATAT 300

CTTGAAAATT TCAGAGGATG AGCGCATGGA GCTCAGTAAG AGCTTTCGAG TATACTGTAT 360

TATCCTTGTA AAACCCAAAG ATGTGAGTCT TTGGGCTGCA GTAAAGGAGA CTTGGACCAA 420

ACACTGTGAC AAAGCAGAGT TCTTCAGTTC TGAAAATGTT AAAGTGTTTG AGTCAATTAA 480

TATGGACACA AATGACATGT GGTTAATGAT GAGAAAAGCT TACAAATACG CCTTTGAWAA 540

GTATAGAGAC CAATACAACT GGTTCTTCCT TGCACGCCCC ACTACGTTTG CTATCATTGA 600

AAACCTAAAG TATTTTTTGT TAAAAAAGGA TCCATCACAG CCTTTCTATC TAGGCCACAC 660

TATAAAATCT GGAGACCTTG AATATGTGGG TATGGAAGGA GGAATTGTCT TAAGTGTAGA 720

ATCAATGAAA AGACTTAACA GCCTTCTCAA TATCCCAGAA AAGTGTCCTG AACAGGGAGG 780

GATGATTTGG AAGATATCTG AAGATAAACA GCTAGCAGTT TGCCTGAAAT ATGCTGGAGT 840

ATTTGCAGAA AATGCAGAAG ATGCTGATGG AAAAGATGTA TTTAATACCA AATCTGTTGG 900

GCTTTCTATT AAAGAGGCAA TGACTTATCA CCCCAACCAG GTAGTAGAAG GCTGTTGTTC 960

AGATATGGCT GTTACTTTTA ATGGACTGAC TCCAAATCAG ATGCATGTGA TGATGTATGG 1020

GGTATACCGC CTTAGGGCAT TTGGGCATAT TTTCAATGAT GCATTGGTTT TCTTACCTCC 1080

AAATGGTTCT GACAATGACT GAGAAGTGGT AGAAAAGCGT GAATATGATC TTTGTATAGG 1140

ACGTGTGTTG TCATTATTTG TAGTAGTAAC TACATATCCA ATACAGCTGT ATGTTTCTTT 1200

TTCTTTTCTA ATTTGGTGGC ACTGGTATAA CCACACATTA AAGTCAGTAG TACATTTTTA 1260

AAAAAAAAAA AAAAAAAAAA AAAAAAAAAA AAAAAAAAAA AAAAAAAAAA AAAAAAAAAA 1320

AAAAAAAAAA AAAAAAAAAA AAAAAAAAAA AAAAAAAAAA AAAAAAAAAA AAAAAA 1376

(2) INFORMATION FOR SEQ ID NO: 26:

(i) SEQUENCE CHARACTERISTICS:
(A) LENGTH: 2923 base pairs
(B) TYPE: nucleic acid
(C) STRANDEDNESS: double
(D) TOPOLOGY: linear

(xi) SEQUENCE DESCRIPTION: SEQ ID NO: 26:

CTCCTCCTCC GGGGCCCCCT CCTCCCCCTT TMACTGGTGC AGATGGCCAG CCTGCTATAC 60

CACCACCGCT TTCTGATACC ACCAAGCCCA AGTCCTCCTT GCCTGCCGTG AGCGATGCCC 120

GTAGCGACCT GCTTTCAGCC ATCCGTCAAG GTTTTCAGCT GCGCAGGGTT GAKGAGCAGC 180

GGGAACAAGA GAAGCGGGAT GTTGTGGGCA ATGACGTGGC CACCATCTTG TCTCGTCGCA 240

TTGCTGTTGA GTACAGTGAC TCAGAAGATG ACTCCTCTGA ATTTGATGAG GACGACTGGT 300

CCGATTAACT CTTTCTGCCT GCTGCCCACC TTCTTTTTCT TTCCTTCCTA CCTGCCTTCT 360

TTGATGCCAA CCCCAACAGA CCCGTAGGGG AGGAAAAGGG AGGAAAAAAG TAATTTTAAG 420

GGGCCAAAGC TTTCCCTGAA GCAACCAAAG ATATATCCAA GTGCTTCCTC CAAGTCAACA 480

TGTATTTCCT CTCCCCATTT TCAGGCCCTG TGGGGCTCCT GAGGTTCAGT AGCTGGGATG 540

TTCCCTCTTT CCTTCAAGTG CCTGTTGCAT ATTGAAAGGA AGGAGAAATC CCAAAGCAGA 600

TTCCTTTGAT CGGGTTTCTG TTGGAGATGG GGCTTCCCTT AGGAGCCATA TTCAACTACA 660

GCCTTCTAAA ACCTGTGCCC TCAGCCACTT CGAATGCCAG CCACCTTCTG GTTCTAAAAC 720

GGGGAGTGGT CTGAATGAAC ACAGCTGACC CCTTTCCCGC GCACTGAAAG GGCAGAGTAG 780

GCCGAAGGTC CAAGGGCCAG ACTGCCTCAC CCTCTGCCCT AATCAGCAGG GTGGGCCTGC 840

CTTTTGCTAA GCGATCTCTA TGCCTGGGAT GCCCTTTATT CCAGGAGGCA TCAAGCCTCT 900

AAAGAATGTC TCACCTCCTC TGCCCAAAAA TGATGCCTTT CTGTAGGCTG GTGTTGTTGC 960

CTCCCTCCCA GGATCCCTTT GGTGAGTATG GTGTTCAGGA TGCACCACCA CCACCTCTAG 1020

ATACCTTCAG GCAACACAGC CCAGTTTTAA CCTCTAGTAT CCATGACCAA ACTATCCCTG 1080

ACACATGAGG ACAGGGGCCT CTTCTGGCTG TCAGGAGCAA AGCCTGAAGA CTTGGAGCTG 1140
CAGGACTGGA AGAACAGTGG AGCCCCGTGG GTCTCACCCT TTAAGGATGC TGAGGCCTAG 1200
AGATGGGAAG TGACTTGCTC AAGGTCACAC AATTGGATAG TGACATAGCT AGAGCGCAGA 1260
GTTCCTGATT CCAAGTCACC TGTGCTTTCT GGGACCAAAG AATGGGCACC TGCTGGAGTC 1320
CGGGCAGAGC TTTCTCAGTT GTATTGCTAC TCCAGACCTC ACCATAGGTT GGGGTCCCAG 1380
TAGGAAGGCT CAGGGTCTGT GCCAGCCCTG TCGGTGCTGC TCAGACCTTC ATAGCCTCTC 1440
TTGTCATTCT TTGTTGCCCC TTTTCTGTCA CCAGCCAACC ACATAGCCTT GGGACCAGCC 1500
TCTCTGGGGG ACCAGAAGTA GTGAGAGAAG GAAGGGGATA GGCAGCTTTG ACAGGTGCTG 1560
CTTTCAATTC CTCTGCAACT CCTCCCCCTT TTATTTCCCC AATTTAAACA AAGATTCTGC 1620
CAACTGTGGA AACTTCAGTC CCTCAGGCTG GCAGCCATGC CAGTACCTGC CTGGGGGTGG 1680
GGGGTGCCTG GCAGCCATGA AGCAGGCTGA AAGGCAGAGG GGCTCCAGGT CCTGTTTCCA 1740
GCTCCCCTCA CTGCACATGG TGAAGCTCGC TCCCTCCCTC CCTCCCTTCC CGCTTTTCCC 1800
AGAGCTAATA CACAGGTGCT ATTATTCAGA AAAAAACTGG TCAGCTCTAG CCAACAGTGA 1860
AGGTTTCTTT TCTTCTGCCC TNAACTATTG TGTAGCCTCT TATGCTGAAA TCGGCTTCTG 1920
CTGGCTTCTC CGGCTTTCAG AGCCCTGAAA CAAAGAGAAA CAGGATCTGT CCCTACCCAG 1980
CACAGCAAAT GGTTGTAGTA ATTGCCAAAG CCCTCATAAA GCCCTCCGGC TTGAGGAGAG 2040
AGTGTATAGT CATGGGTTCT GCCTCTGTGC CCTTGCTGGC CGCTTCTCCT CTGCCTTCTT 2100
TCCTGGAACT CAGGGTGTGG GGACTGAGCC TGTAGGGGAC AGCATGCCGT CTTGCTGTGG 2160
CCACTCCCAA GTGTGCCCTC TTCCCTCTTT ACACATCAGG TGTCTCTGGC ACAGGACTTG 2220
GCACTAAGCT CCATGCTGAG ACACCAGGCT ATGTGGGCCC CCACCTTGTT TCCCAGCCTG 2280
CACCTTAGAA GCCGAAGTGC TTTCATCAGA ACCCTAAAAT GGTCGTTGAA GGCGCCTGGG 2340
CCGCAGCCAG CAGTAGTTGG AGAGGCAGGC AGAGGGCAGT GGTTCTCCCA AATAGGAGAC 2400
CTGGGGCCTG GCCAGGCAGG GTTTGGGCCT AATGGCTTTG ACTAAATTAC CCCCATCCTC 2460
CTTGCCCGGA AAAGGGAGAG CTAGAGCCAC TCACTGTCAT TCTGCTCTGA CCTTGAAGGG 2520
GGCGGTGTTG GCCTGGCTTC TGGAATGGAC TGAGTCCATC GTGGAAAGGG CTGGGGGCAG 2580
GAGGAGGTGG GGAGGGGCAC TGCCTGCGGA AGGTAGGATT AGATCATTAG CTCAGTGACC 2640
TCCTAGGGTT TCGATGTGCT ATGTTCTCAT CCTACAGTTG GTTTGGTAAT GATCTGCAAG 2700
TCCCGGAGAG CAACAGCACA GCTCTGCCTG ACGCTCTCAT TAAAATCTAT GCAGCCAAGC 2760
TCGGCACTTT GTAGCAGCCG GCCTTGCGAA GCCTCCTCAG CTCGGGGGGC CGGGGACCCA 2820
GTGAGCCGNA GAKCSTCTGG GCTCCACTTA TGCATATGCA CCAAAAAAAA AAAAAAAAAA 2880

AAAAGGGGGG CCGCTCTANA AGGATTCCTC NAAGGGGCCC AAG 2923

(2) INFORMATION FOR SEQ ID NO: 27:

(i) SEQUENCE CHARACTERISTICS:
(A) LENGTH: 775 base pairs
(B) TYPE: nucleic acid
(C) STRANDEDNESS: double
(D) TOPOLOGY: linear

(xi) SEQUENCE DESCRIPTION: SEQ ID NO: 27:

GAACTAGTGN ATCCCCCGGG CTGCAGGAAT TCGGCACGAG CCCRACCCSC ACCACCACCA 60

GAATGCAGTT CCAGCTTAGG AAGCCACAAA CAAGCCACCC AGGAGGAACA AAACACCGCC 120

AGCGTGGATT TTCCCAAATT TCCCTGGAAA GTAAGTCTCG CTCTTGCCAA AGAAAAGTCT 180

GGCTTGGAGA GTCTCTGGAG CCCAGGATGC CAGCATGTGC CAATGACTGT CACCTTCATC 240

TCTTCAAAAG AAAAGCCATA GCCGAGGACT GTCCCGCGAC CCCCGTGGAC TGCGTCTAGG 300

TCATGTGATT CTGTTTTCAT TTCTCATCCC ATCCAATTTG TCCTTTTCTC CTGTCATTTT 360

CTTCCTCTGT GGTCCCTTCA AAGTTGTTAT AATTTGTACT GAACTTCAAA ATGTGTCCCG 420

TTCTCCCCAG ACCACTCTAG CCACAGTATA TTGCAATAAA ATTACTTCTT ATATTTGCAG 480

AAATTCTTTT GGTGTAATTT TATTTTTTCC TCTCAATATA TATAATTGGA CAAACGCTGG 540

CAAAAAGAAA AAAATGGTAA GCAAAAAACC CAAGATAAAG TTTCGAGGAC ATCAGGCCTT 600

TTGAAATACA ATGTCAAATG ACACATTGTA CGKTTTCAAA AAATCCGCTA GACATGTCAT 660

AAGTTTTAAC TGTAATGCCC AGGAAAGGAT ATCTTAAAAT ATTCTAAACT TGTGTAACAA 720

AGGAATAATT AACTGTAATA GTTTTTCAAT AAATCGAGTT GGGTGTTTCC ACCGT 775

(2) INFORMATION FOR SEQ ID NO: 28:

(i) SEQUENCE CHARACTERISTICS:
(A) LENGTH: 534 base pairs
(B) TYPE: nucleic acid
(C) STRANDEDNESS: double
(D) TOPOLOGY: linear

(xi) SEQUENCE DESCRIPTION: SEQ ID NO: 28:

GAATTCGGCA CGAGCAAGGG TGGAACCTGA GTCTGCTTGT CTGTTTGCCC CATGACAGCC 60

CAGGGGTGGT GGSCTCACCC CACCTCCAGG CAMCCACAAG AATATAAAAT CTTGTACAAR 120

GATGTCGATA TTACTATTGS CATTCCCAAG TGCACCTGCA CCTGTAGTAT CAGGTGGTTT 180

GCAGCCTTGG CTGCATAGCT GCATATGAGA ATCACCTGGG AAGCTTTTAA AAATCCCAGT 240

ATCCCCACCT CTTCCCCAGT TACAGTGGAG TCTTGCGGGT GGTGGGGGAC ATCAATTATT 300

TTTGAAAGCT CCMAAGTAAT TCTGGTGTGC AGTGGGGTGA CCAGCTGTCC CAGGGAMCTC 360

CTTTAAAAAA TAATATCCCG GGCACATGAC AGGCCAATTG CCCTAATGCA ACCAAGGTTA 420

AGAACTACTG GTTTAATGGG AAAATATTTT TTTCCNGTGC TTGAATAATA CTGGTTTTAT 480

TAAACTCCNG AATCCCATTT CTTTCCTTGC CAAATTTTTT AAAGGCNAAA AAAA 534

(2) INFORMATION FOR SEQ ID NO: 29:

(i) SEQUENCE CHARACTERISTICS:
(A) LENGTH: 1827 base pairs
(B) TYPE: nucleic acid
(C) STRANDEDNESS: double
(D) TOPOLOGY: linear

(xi) SEQUENCE DESCRIPTION: SEQ ID NO: 29:

NNCNGCACGA GCNCGGTCCT GTCCCGTCAG CGTCCCGCCA GCCAGCTCCT TGCACCCTTC 60

GCGGCCGAGG CGCTCCCTGG TGCTCCCCGC GCAGCCATGG CTCAGCACTT CTCCCTGGCC 120

GCCTGCGACG TGGTCGGATT CGACCTGGAC CACACTCTGT GTCGCTACAA CCTGCCCGAG 180

AGCGCCCCGC TCATTTATAA TAGCTTTGCC CAGTTCCTAG TTAAGGAGAA AGGGTACGAT 240

AAGGAATTGC TCAATGTGAC CCCAGAGGAT TGGGATTTCT GTTGCAAAGG TTTGGCATTG 300

GATCTAGAAG ATGGGAACTT CCTTAAACTT GCAAATAATG GCACTGTTCT CAGGGCAAGC 360

CATGGCACCA AGATGATGAC TCCAGAGGTG CTGGCAGAGG CATATGGCAA GAAAGAGTGG 420

AAGCACTTCT TGTCGGACAC TGGAATGGCT TGCCGCTCAG GAAAGTATTA CTTTTACGAC 480

AACTACTTTG ACCTGCCAGG AGCTCTTCTG TGTGCCAGGG TGGTGGACTA TTTAACAAAA 540

CTGAACAATG GTCAAAAAAC ATTTGATTTT TGGAAGGATA TAGTTGCTGC TATACAACAC 600

AATTATAAAA TGTCAGCTTT TAAGGAAAAC TGTGGAATAT ATTTTCCAGA AATAAAAAGA 660

GATCCAGGCA GATATTTACA TAGTTGTCCT GAATCTGTGA AAAAATGGCT TCGACAGCTA 720

AAGAATGCTG GGAAAATTCT TCTGTTAATT ACCAGTTCTC ACAGTGATTA CTGTAGACTT 780

CTCTGCGAAT ATATTCTTGG GAATGATTTT ACAGACCTTT TTGACATTGT GATTACAAAT 840

GCATTGAAGC CTGGTTTCTT CTCCCACTTA CCAAGTCAGA GACCTTTCCG GACACTCGAG 900

AATGATGAGG AGCAGGAGGC ACTGCCATCT CTGGATAAAC CTGGCTGGTA CTCCCAAGGG 960

AACGCTGTCC ACCTCTATGA ACTTCTGAAG AAAATGACTG GCAAACCTGA ACCCAAGGTT 1020

GTTTATTTTG GTGACAGCAT GCATTCAGAT ATTTTCCCAG CTCGTCACTA TAGTAATTGG 1080

GAGACAGTCC TCATCCTGGA AGAACTCAGA GGGGATGAAG GCACGAGGAG TCAGAGGCCT 1140

GAGGAGTCAG AGCCTCTAGA GAAGAAAGGA AAATATGAGG GACCAAAAGC AAAACCTTTA 1200

AATACTTCAT CTAAAAAATG GGGCTCTTTT TTTATTGATT CAGTTTTGGG ACTGGAAAAT 1260

ACAGAAGACT CCTTGGTTTA TACATGGTCT TGTAAGAGAA TCAGTACTTA CAGCACTATT 1320

GCAATTCCAA GTATTGAAGC AATCGCAGAA TTACCTCTGG ACTACAAATT TACAAGATTC 1380

TCTTCAAGCA ATTCAAAAAC AGCTGGCTAC TATCCAAATC CTCCACTGGT CTTATCAAGT 1440

GATGAGACAC TGATATCCAA ATAAGTTGTC TTTACTGAAA AATGAAGTGA AGACCCATAT 1500

ATGCAGTTAA AAAAAAGTTA ATTTTCAAAA AATACTGTAA AAGACTTTAA GGAACAAGTT 1560

TTATTGACCA ATAAGTTGAT ATTTGTCCAT AGGTCTCCTT TCTATAAATC ATCTTGATGT 1620

TTAACAACTC TTATTATATT AAAATCTCAG TATCCTAAAA CTTAGGAACC TTATTGGATA 1680

TTTTCTATTA CAGTAGTTTT GTGGTTGGGA TTCACCCGGG GGGGCCACAC ACTCACACGG 1740

CACAGTTCAC TCTTTACACA TATGGCCNCG GTCCCGTGGG GTTCTCNAAG GTGTGGTTCC 1800

CTTGGGGCCT NTTGGGCTTG GGCCTTT 1827

(2) INFORMATION FOR SEQ ID NO: 30:

(i) SEQUENCE CHARACTERISTICS:
(A) LENGTH: 1479 base pairs
(B) TYPE: nucleic acid
(C) STRANDEDNESS: double
(D) TOPOLOGY: linear

(xi) SEQUENCE DESCRIPTION: SEQ ID NO: 30:

GGCACGAGGG CGGGTGGCAT CAGCAGAGGG GCACCAGCCA AAGGGTGTGG CTACCTCACT 60

GCTGGTCCCC AGGCCCGGGA GGTGGGGAGC ACACACAGTG CCTTGGGTAC CCAGNTGGGT 120

GTTCTCCCGC TGCAGAGGAG ACRGCAGCCT GGGTCCTGCC CTTCACCTCT GGCGGCTTTC 180

TCTACATCGC CTTGGTGAAC GTGCTCCCTG ACCTCTTGGA AGAAGAGGAC CCGTGGCGCT 240

CCCTGCAGCA GCTGCTTCTG CTCTGTGCGG GCATCGTGGT AATGGTGCTG TTCTCGCTCT 300

TCGTGGATTA ACTTTCCCTG ATGCCGACGC CCCTGCCCCC TGCAGCAATA AGATGCTCGG 360

ATTCACTCTG TGACCGCATA TGTGAGAGGC AGAGAGGGCG AGTGGCTGCG AGAGAGAATG 420

AGCCTCCCGC CAGACAGGAG GGAGGTGCGT GTGGATGTAT GTGGTGTGCA CATGTGGCCA 480

GAGGTGTGTG CGCGAGACCG ACACTGTGAT CCCTGTGCTG GGTCCGGGGC CCAGTGTAGC 540

GCCTGTCCCC AGCCATGCTG TGGTTACCTC TCCTTGCCGC CCTGTCACCT TCACCTCCTG 600

GAGTAAGCAG CGAGGAAGAG CAGCACTGGT CCCAAGCAGA GGCCTTGCCC TGCTGGGACC 660

CCGGGAGTGA GAGCAGCCCA AGGATCCCAG GGTGCAGGGA ACTCCAGAGC TGCCCACCTC 720

CCACTGCCCC CTCAGCACAC ACACAGTCCC CAGGCGGCCT AGGGGCCAAG GCTGGGGCGG 780

CTTTGGTCCC TTTTCCTGGC CCTTCCTTCC CCACTTCTAA GCCAAAGAAA GGAGAGGCAG 840

GTGCTCCTGT ACCCCAGCCC CACTCAGCAC TGACAGTCCC CAGCTCCTAG TAGTGAGCTG 900

GGAGGCGCTT CCTAAGACCC TTTCCTCAGG GCTGCCCTGG GAGCTCATTC CTGGCCAACA 960

CGCCCTGGCA GCACCAGCAG CTCTTGCCAC CTCCAGCTGC CAAACAGCAG CCTGCCGGGC 1020

AGGGAGCAGC CCCAGGCCAG AGAGGCCTCC CGGTCCAGCT CAGGGATGCT CCTGCCAGCA 1080

CAGGGGCCAG GGACTCCTGG AGCAGGCACA TAGTGAGCCC GGGCAGCCCT GCCCAGCTCA 1140

GGCCCCTTTC CTTCCCCATT GAGGTTGGGG TAGGTGGGGG CGGTGAGGGC TCCACGTTGT 1200

CAGCGCTCAG GAATGTGCTC CGGCAGAGTG CTGAAGCCAT AATCCCCAAC CATTTCCCTT 1260

GGCTGACGCC CAGGTACTCA GCTGGCCCAC TCCACAGCCA GGCCTGCCCT GCCCTTCACC 1320

GTGGATGTTT TCAGAAGTGG CCATCGAGAG GTCTGGATGG TTTTATAGCA ACTTTGCTGT 1380

GATTCCGTTT GTATCTGTAA ATATTTGTTC TATAGATAAG ATACAAATAA ATATTATCCA 1440

CATAAAAAAA AAAAAAAAAA AACTTGGGGG GGGGNCCCG 1479

(2) INFORMATION FOR SEQ ID NO: 31:

(i) SEQUENCE CHARACTERISTICS:
(A) LENGTH: 987 base pairs
(B) TYPE: nucleic acid
(C) STRANDEDNESS: double
(D) TOPOLOGY: linear

(xi) SEQUENCE DESCRIPTION: SEQ ID NO: 31:

GGCACGAGCG CAATCGCGTT TCCGGAGAGA CCTGGCTGCT GTGTCCCGCG GCTTGCGCTC 60

CGTAGTGGAC TCCGCGGGCC TTCGGCAGAT GCAGGCCTGG GGTAGTCTCC TTTCTGGACT 120

GAGAAGAGAA GAATGGAGAA GCCCCTCTTC CCATTAGTGC CTTTGCATTG GTTTGGCTTT 180

GGCTACACAG CACTGGTTGT TTCTGGTGGG ATCGTTGGCT ATGTAAAAAC AGGCAGCGTG 240

CCGTCCCTGG CTGCAGGGCT GCTCTTCGGC AGTCTAGCCG GCCTGGGTGC TTACCAGCTG 300

TATCAGGATC CAAGGAACGT TTGGGGTTTC CTAGCCGCTA CATCTGTTAC TTTTGTTGGT 360

GTTATGGGAA TGAGATCCTA CTACTATGGA AAATTCATGC CTGTAGGTTT AATTGCAGGT 420

GCCAGTTTGC TGATGGCCGC CAAAGTTGGA GTTCGTATGT TGATGACATC TGATTAGCAG 480

AAGTCATGTT CCAGCTTGGA CTCATGAAGG ATTAAAAATC TGCATCTTCC ACTATTTTCA 540

ATGTATTAAG AGAAATAAGT GCAGCATTTT TGCATCTGAC ATTTTACCTA AAAAAAAAAA 600

GACACCAAAT TTGGCGGAGG GGTGGAAAAT CAGTTGTTAC CATTATAACC CTACAGAGGT 660

GGTGAGCATG TAACATGAGC TTATTGAGAC CATCATAGAG ATCGATTCTT GTATATTGAT 720

TTTATCTCTT TCTGTATCTA TAGGTAAATC TCAAGGGTAA AATGTTAGGT GTTGACATTG 780

AGAACCCTGA AACCCCATTC CCTGCTCAGA GGAACAGTGT GAAAAAAAAT CTCTTGAGAG 840

ATTTAGAATA TCTTTTCTTT TGCTCATCTT AGACCACAGA CTGACTTTGA AATTATGTTA 900

AGTGAAATAT CAATGAAAAT AAAGTTTACT ATAAATAAWA AAAAAAAAAA AAAAAAAAAA 960

AAAAAAAAAA AAAAAAAAAA ANANAAA 987

(2) INFORMATION FOR SEQ ID NO: 32:

(i) SEQUENCE CHARACTERISTICS:
(A) LENGTH: 2933 base pairs
(B) TYPE: nucleic acid
(C) STRANDEDNESS: double
(D) TOPOLOGY: linear

(xi) SEQUENCE DESCRIPTION: SEQ ID NO: 32:

TCTACCTCCG AGTAGTATTA GACTGTAAAC ACAGTAATAT AGNCGCCATC ATTCGTGAAG 60

GGGTTTCTTT TGCGGGACAG AGGATCAGAT GTTGAGAGTT TGGACAAACT CATGAAAACC 120

AAAAATATAC CTGAAGCTCA CCAAGATGCA TTTAAAACTG GTTTTGCGGA AGGTTTTCTG 180

AAAGCTCAAG CACTCACACA AAAAACCAAT GATTCCCTAA GGCGAACCCG TCTGATTCTC 240

TTCGTTCTGC TGCTATTCGG CATTTATGGA CTTCTAAAAA ACCCATTTTT ATCTGTCCGC 300

TTCCGGACAA CAACAGGGCT TGATTCTGCA GTAGATCCTG TCCAGATGAA AAATGTCACC 360

TTTGAACATG TTAAAGGGGT GGAGGAAGCT AAACAAGAAT TACAGGAAGT TGTTGAATTC 420

TTGAAAAATC CACAAAAATT TACTATTCTT GGAGGTAAAC TTCCAAAAGG AATTCTTTTA 480

GTTGGACCCC CAGGGACTGG AAAGACACTT CTTGCCCGAG CTGTGGCGGG AGAAGCTGAT 540

GTTCCTTTTT ATTATGCTTC TGGATCCGAA TTTGATGAGA TGTTTGTGGG TGTGGGAGCC 600

AGCCGTATCA GAAATCTTTT TAGGGAAGCA AAGGCGAATG CTCCTTGTGT TATATTTATT 660

GATGAATTAG ATTCTGTTGG TGGGAAGAGA ATTGAATCTC CAATGCATCC ATATTCAAGG 720

CAGACCATAA ATCAACTTCT TGCTGAAATG GATGGTTTTA AACCCAATGA AGGAGTTATC 780

ATAATAGGAG CCACAAACTT CCCAGAGGCA TTAGATAATG CCTTAATACG TCCTGGTCGT 840

TTTGACATGC AAGTTACAGT TCCAAGGCCA GATGTAAAAG GTCGAACAGA AATTTTGAAA 900

TGGTATCTCA ATAAAATAAA GTTTGATCAW TCCGTTGATC CAGAAATTAT AGCTCGAGGT 960

ACTGTTGGCT TTTCCGGAGC AGAGTTGGAG AATCTTGTGA ACCAGGCTGC ATTAAAAGCA 1020

GCTGTTGATG GAAAAGAAAT GGTTACCATG AAGGAGCTGG GAGTTTTCCA AAGACAAAAT 1080

TCTAATGGGG CCTGAAAGAA GAAGTGTGGA AATTGATAAC AAAAACAAAA CCATCACAGC 1140

ATATCATGAA TCTGGTCATG CCATTATTGC ATATTACACA AAAGATGCAA TGCCTATCAA 1200

CAAAGCTACA ATCATGCCAC GGGGGCCAAC ACTTGGNACA TGTGTCCCTG TTACCTGAGA 1260

ATGACAGATG GAATGAAACT AGAGCCCAGC TGCTTGCACA AATGGATGTT AGTATGGGAG 1320

GAAGAGTGGC AGAGGAGCTT ATATTTGGAA CCGACCATAT TACAACAGGT GCTTCCAGTG 1380

ATTTTGATAA TGCCACTAAA ATAGCAAAGS GGATGGTTAC CAAATTTGGA ATGAGTGAAA 1440

AGCTTGGAGT TATGACCTAC AGTGATACAG GGAAACTAAG TCCAGAAACC CAATCTGCCA 1500

TCGAACAAGA AATAAGAATC CTTCTAAGGG ACTCATATGA ACGAGCAAAA CATATCTTGA 1560

AAACTCATGC AAAGGAGCAT AAGAATCTCG CAGAAGCTTT ATTGACCTAT GAGACTTTGG 1620

ATGCCAAAGA GATTCAAATT GTTCTTGAGG GGAAAAAGTT GGAAGTGAGA TGATAACTCT 1680

CTTGATATGG ATGCTTGCTG GTTTTATTGC AAGAATAYAA GTAGCATTGC AGTAGTCTAC 1740

TTTTACAACG CTTTCCCCTC ATTCTTGATG TGGTGTAATT GAAGGGTGTG AAATGCTTTG 1800

TCAATCATTT GTCACATTTA TCCAGTTTGG GTTATTCTCA TTATGACACC TATTGCAAAT 1860

TAGCATCCCA TGGCAAATAT ATTTTGAAAA AATAAAGAAC TATCAGGATT GAAAACAGCT 1920

CTTTTGAGGA ATGTCAATTA GTTATTAAGT TGAAAGTAAT TAATGATTTT ATGTTTGGTT 1980

ACTCTACTAG ATTTGATAAA AATTGTGCCT TTAGCCTTCT ATATACATCA GTGGAAACTT 2040

AAGATGCAGT AATTATGTTC CAGATTGACC ATGAATAAAA TATTTTTTAA TCTAAATGTA 2100

GAGAAGTTGG GATTAAAAGC AGTCTCGGAA ACACAGAGCC AGGGAATATA GCCTTTTGGC 2160

ATGGTGCCAT GGCTCACATC TGTAATCCCA GCACTTTTGG AGGCTGAGGC GGGTGGATTG 2220

CTTGAGGCCA GGAGTTCGAG ACCAGCCTGG CCAACGTGGT GAAACGCTGT YTCTACTAAA 2280

ATACAAAAAA ATAGGGCTGG GCGCGGTTGC TCACGCCTGT AATCCCAGCA CTTTTCAGAG 2340

GCCAAGGCGG GCAAATCACC TGAGGTCAAG AGTTTGAGAC CAGCCTGGCC AACATGGTGA 2400

AACCCCATCT CTACTAAACA TGCAAAAATT ACCTGGGCAT GGTGGCAGGT GCTTATAATC 2460

CCAGCTACTC TGGGGGCCAA GGCAGGAGAA TTGCTTGAGC CTGGGAGATG GAGGTTGCAG 2520

TGAGCTGAGA TCATGCCACT GCACTCCAGC CTGGGCAACA GAGCAAGACT CTGCCTCAAA 2580

AAAAAATTAA AATAAATTTA AATACAAAAA AAAATAGCCA GGTGTGGGGT GCATGCCTGG 2640

AATCCCAGCT ACTTGAGAGG CTGAGGCACG AGAATTGCTT GAACCCAGGA GGTGGAGGTT 2700

GCAGTGAGCC AAGATCACAG GAGCCACTGC ACTCCAGCCT GGGTGACAGA GTGAGACTCT 2760

GTCTCAAAAM AAAATTAAAT AAATTATTAT AACCTTTCAG AAATGCTGTG TGCATTTTCA 2820

TGTTCTTTTT TTTAGCATTA CTGTCACTCT CCCTAATGAA ATGTACTTCA GAGAAGCAGT 2880

ATTTTGTTAA ATAAATACAT AACCTCAAAA AAAAAAAAAA AAAAAAAACT CGA 2933

(2) INFORMATION FOR SEQ ID NO: 33:

(i) SEQUENCE CHARACTERISTICS:
(A) LENGTH: 1366 base pairs
(B) TYPE: nucleic acid
(C) STRANDEDNESS: double
(D) TOPOLOGY: linear

(xi) SEQUENCE DESCRIPTION: SEQ ID NO: 33:

GGGAATACCT ATTCTCCTTT ACCGTGTGTC TTTTCCCCCT GGAATTGAGC CAGCAAGTTC 60

TTGGCATGGC AGGTGTTTCT GAAATATCAG TGTGTTTTTY TTTGCTTTCT TTGTTTTCCT 120

TGTTTTGCTC TTTCTATTTT CCTAAGCAGG CAACTCCAAA AAGAGATTTG TTTGTGCAGG 180

AGTCAGGAAA AGGGAAGAGG AATACTGAAA GCTGGGAGTA GGGCAGGACA GAAGAGGGGG 240

AGGAGTCTAT TTTCATTGTG TAAGTKTTGA ACTTCCACCA ATGCCAAAGT CACGGACATG 300

TGTGCAGTTG GATGTKCGAG TTAGAGCAGC CCCAAGGGCC TGTAACCTGA ATAGCAGGCA 360

CTCACCCAGC TGATAACTCA AGTTCCAAAT GGACCACAGC TGAGTTGTAG GGGATGTGTG 420

TGTGTGTGTA CGCGTGCGTT TGAGATTCCT GGAACAGATT TCCTCTGAGA TCTCAACAGG 480

CTTTTTCATT ATCATTGGGG AGCTATGGTT TCTCTTATTT CACAAGGCCC ATTTCTTCCT 540

TTTGAGATGT GCAAGGAGAT GACTCCATCC ATGACTTGGC TTTACACTCT CCCTCCTTGG 600

CTTTTTATCA TCAGTGCAGR AGARATTCTT GCTCGTTCTT CAAACAATCT CATTCGAGCT 660

TTATAAAGAT TATTGGARTT TAAATAATAT TCATATCTAT GGCCTAGAAC AATGTTCCTC 720

AAGTATGCGT CAGAATCATG AGTGGTAGAG GGAGGATTAT AATGTAGTTT CCTACATTTC 780

TACCTCCCAC CACCCTGGAG TCTGCATTTT AACGTACTTC TGTYTGAGGA TCAGAYTTTG 840

GGAAGCGTTG GGCTTGAGAT GTTTTCTKGA CATTGATTTA TGTTGAGACC AGACCAAGAA 900

GCAGATGGAT GGACATGATC AGTTCATAAA CATGTTCCTT TCTTAGGGTC AAATTGGAGG 960

AGGCTCTAGA GAAGCACTGT CCAATAGAAA TATAATGCCA ACAATATATG TWATTTTAAG 1020

TCTTCTATTG GTGCATTTAA AAAGTAAAAG AAGGCTGAGT GGCTGGGCAT GGCTCCTCGT 1080

GCCTGTAATC CCAGCACTTT GGGAGGCCGG GGTGGGCAGA TCACCTGAGG TCAGGAGTTC 1140

GAGACCAGCC TGCCCAACAT GGTGAAACCC CATATNTACT AAAAATACAA AAAATTAACC 1200

GGGCATAGTG GCAGGTGCCT GTAATCCCAG CTACTCGGGA GGCTGAGGCA GGAGAATCGC 1260

TTGAACCTGG GAGGCAGAGA CTGCAGTGAG CTGAGATCGT GCCACTACAC TCCAGCCTGG 1320

GTGATGAGCG AAACTCCGTC TCAAAAAAAA AAAAAAAAAA ACTCGA 1366

(2) INFORMATION FOR SEQ ID NO: 34:

(i) SEQUENCE CHARACTERISTICS:
(A) LENGTH: 667 base pairs
(B) TYPE: nucleic acid
(C) STRANDEDNESS: double
(D) TOPOLOGY: linear

(xi) SEQUENCE DESCRIPTION: SEQ ID NO: 34:

ATTTTCGGCA CAGGCCGGAA GCTACCTATC TGGTAGGGAG CTCCCCCAGC ACCGAAGACT 60

GCGATGACTT CTGCRCTGAC CCAGGGGCTG GAGCGAATCC CAGACCAGCT CGGCTACCTG 120

GTACTGAGTG AAGGTGCAGT GCTGGCGTCA TCTGGGGACC TGGAGAATGA TGAGCAGGCA 180

GCCAGTGCCA TCTCTGAGCT GGTCAGCACA GCCTGCGGTT TCCGGCTGCA CCGCGGCATG 240

AATGTGCCCT TCAAGCGCCT GTCTGTGGTC TTTGGAGAAC ACACACTGCT GGTGACGGTG 300

TCAGGACAGA GGGTGTTTGT GGTGAAGAGG CAGAACCGAG GTCGGGAGCC CATTGATGTC 360

TGAGCCTGCC GGAGGGCGAG GGTCGGAGAA GCGGATTGGG TCCTGGGCCT CTGTGATGAG 420

GCAGGCACAN CTGTCGGTCT TGGCTTGCTG CTAGAACTAG GGCCTTCTGC TCGCCCACCT 480

CCCACCCCTA CCTGGACGGG CCCAGGCTTG GGGACTCTGA GCTGTGTTAA GGAGAACAAG 540

GGCAAGGAGA CCTCCCTTTG TGCTCCCTCA CTCCCTAATA AACATGAGTC TGATGTTCTC 600

CARMMMAAAA AAAAAAAAAA AAAAAAAAAA AAAAAAAAAA AAAAAAAAAA AAAAAAAAAA 660

AAAAANN 667

(2) INFORMATION FOR SEQ ID NO: 35:

(i) SEQUENCE CHARACTERISTICS:
(A) LENGTH: 1710 base pairs
(B) TYPE: nucleic acid
(C) STRANDEDNESS: double
(D) TOPOLOGY: linear

(xi) SEQUENCE DESCRIPTION: SEQ ID NO: 35:

GGCACGAGCC AGAGCAGGCT GCTAGGCCTG GGGCCACCAC TGCCCCTGGG TGCTACACCC 60

AGTGTGCTGG GTCACTGGGA ACTTCCTGAA GTGGTGTCAC CTGAACTGGG CCCCCAAGGA 120

TGGGGTGCGG GCAGTACCGC AGGAAGAGGA GCAGCCCCTG TGAAGATTGA GAGCTGCCAG 180

AGGCTCTGTG ATTGGCTGCG GCACGATGAC CCGCGCACGG ATTGGCTGCT TCGGGCCGGG 240

GGGCCGGGCC CGGGGGACAG AATCCGCCCC CGAACCTTCA AAGAGGGTAC CCCCCGGCAG 300

GAGNTGGCAG ACCTTAGGAG GTGCGACAGA CCCGCGGGGC AAACGGACTG GGGCCAAGAG 360

CCGGGAGCGC GGGCGCAAAG GCACCAGGGC CCGCCCAGGG CGCCGCGCAG CACGGCCTTG 420

GGGGTTCTGC GGGCCTTCGG GTGCGCGTCT CGCCTCTAGC CATGGGGTCC GCAGCGTTGG 480

AGATCCTGGG CCTGGTGCTG TGCCTGGTGG GCTGGGGGGG TCTGATCCTG GCGTGCGGGC 540

TGCCCATGTG GCAGGTGACC GCCTTCCTGG ACCACAACAT CGTGACGGCG CAGACCACCT 600

GGAAGGGGCT GTGGATGTCG TGCGTGGTGC AGAGCACNGG GCACATGCAG TGCAAAGTGT 660

ACGACTCGGT GCTGGCTCTG AGCACCGAGG TGCAGGCGGC GCGGGCGCTC ACCGTGAGCG 720

CCGTGCTGCT GGCGTTCGTT GCGCTCTTCG TGACCCTGGC GGGCGCGCAG TGCACCACCT 780

GCGTGGCCCC GGGCCCGGCC AAGGCGCGTG TGGCCCTCAC GGGAGGCGTG CTCTACCTGT 840

TTTGCGGGCT GCTGGCGCTC GTGCCACTCT GCTGGTTCGC CAACATTGTC GTCCGCGAGT 900

TTTACGACCC GTCTGTGCCC GTGTCGCAGA AGTACGAGCT GGGCGCANGC TGTACATCGG 960

CTGGGCGGCC ACCGCGCTGC TCATGGTAGG CGGCTGCCTC TTGTGCTGCG GCGCCTGGGT 1020

CTGCACCGGC CGTCCCGACC TCAGCTTCCC CGTGAAGTAC TCAGCGCCGC GGCGGCCCAC 1080

GGCCACCGGC GACTACGACA AGAAGAACTA CGTCTGAGGG CGCTGGGCAC GGCCGGGCCC 1140

CTCCTGCCAG CCACGCCTGC GAGGCGTTGG ATAAGCCTGG GGAKCCCCGC ATGGACCGCG 1200

GCTTCCGCCG GGTAGCGCGG CGCGCAGGCT CCTCGGAACG TCCGGCTCTG CGCCCCGACG 1260

CGGCTCCTGG ATCCGCTCCT GCCTGCGCCC GCAGCTGACC TTCTCCTGCC ACTAGCCCGG 1320

CCCTGCCCTT AACAGACGGA ATGAAGTTTC CTTTTCTGTG CGCGGCGCTG TTTCCATAGG 1380

CAGAGCGGGT GTCAGACTGA GGATTTCGCT TCCCCTCCAA GACGCTGGGG GTCTTGGCTG 1440

CTGCCTTACT TCCCAGAGGC TCCTGCTGAC TTCGGAGGGG CGGATGCAGA GCCCAGGGCC 1500

CCCACCGGAA GATGTGTACA GCTGGTCTTT ACTCCATCGG CAGGCCCGAG CCCAGGGACC 1560

AGTGACTTGG CCTGGACCTC CCGGTCTCAC TCCAGCATCT CCCCAGGCAA GGCTTGTGGG 1620

CACCGGAGCT TGAGAGAGGG CGGGAGTGGG AAGGCTAAGA ATCTGCTTAG TAAATGGTTT 1680

GAACTCTCAA AAAAAAAAAA AAAAAAAAAA 1710

(2) INFORMATION FOR SEQ ID NO: 36:

(i) SEQUENCE CHARACTERISTICS:
(A) LENGTH: 1096 base pairs
(B) TYPE: nucleic acid
(C) STRANDEDNESS: double
(D) TOPOLOGY: linear

(xi) SEQUENCE DESCRIPTION: SEQ ID NO: 36:

GGCCAGTGGG CAGGGTCACA GGGCAAGGTC CCGCGGGCCG CTGGGTGCGG CGACTTCCGT 60

GCTCCCGGCG AGCGGGCGGA GAGCGGGGGC CGCACTGGGG AGTGTGGGCT GGGCCGCAGA 120

TGTCATGTGG CCTGTKTTTT GGACCGTGGT TCGTACCTAT GCTCCTTATG TCACATTCCC 180

TGTTGCCTTC GTGGTCGGGG CTGTGGGTTA CCACCTGGAA TGGTTCATCA GGGGAAAGGA 240

CCCCCAGCCC GTGGAGGAGG AAAAGAGCAT CTCAGAGCGC CGGGAGGATC GCAAGCTGGA 300

TGAGCTTCTA GGCAAGGACC ACACGCAGGT GGTGAGCCTT AAGGACAAGC TAGAATTTGC 360

CCCGAAAGCT GTGCTGAACA GAAACCGCCC AGAGAAGAAT TAATGGAGGA CACAGGGCCC 420

TATGGTCCTA CTGTGGGTGG TGACTTGTCC TGCTACCATG TTGACAGAGC CCCAGAACCC 480

ACATCTAATT GGCTTTGTTG CTTATTCTGG CCCTTCCCAC ACCACACAGC CACACAAATA 540

CTGGCTGCTC CTTGATGGCC AGGCAGACCC AGCAGCAGCC GAGGGGCCAG TGAAGAGGAA 600

GGCCGCATCT GTTGTGTGGT GGCCACAAGC ACTCAGGCAT CTGAGTTTAC TGGTGCACTG 660

CTGGGAGGAG AGTTATGAGA TGAACATTGG CTGTCAATCT CTGTGGGCAG GCGGTTTGGC 720

CTCTAGTGGG AATGGCTGGG ATTTGGGCGT TGCCTTTAGG AGGGATACCT GCATGTCTAG 780

TTCCAGTCTG CACTGGAAAG AATTCAAATA TGCACCTGGC TCCCTTCACT ATTTTGCCCT 840

ATCCTTTGTG CTCATTCTTA CTGAAATCTG TCTTGTCAGC TCAGGAATGG GATTCCCCCA 900

GGAAGGAAAG CACTTTTCTG TTCTGGGAAG CCCAGACTGT TCACTTTGGG GCAGGGACGA 960

ACATGTGCCT CGTGAATTTG CTTGAAAACA GTCACCATCT TCTACCCCCA TCACTGTATA 1020

GTGAAAAACC TGATTAAAGT GGTATCTGAG AACCAWAAAA AAAAAAAAAA AAAAAAAAAA 1080

AAAAANGGGG GGNCCC 1096

(2) INFORMATION FOR SEQ ID NO: 37:

(i) SEQUENCE CHARACTERISTICS:
(A) LENGTH: 2279 base pairs
(B) TYPE: nucleic acid
(C) STRANDEDNESS: double
(D) TOPOLOGY: linear

(xi) SEQUENCE DESCRIPTION: SEQ ID NO: 37:

GGTGGGCAAG GGGCTCAGCT CGCAGCGCAT GCCCGCGCAC AGGTTCGTGC TGGCCGTGGG 60
CAGCGCCGTC TTTAATGCCA TGTTCAACGG GGGMATGGCC ACAACATCCA CGGAGATTGA 120
GCTGCCCGAC GTRGAACCCG CCGCCTTCCT CGCACTGCTC AAGTTTCTCT ACTCGGACGA 180
GGTGCAGATT GGCCCGGAGA CGGTGATGAC CACGSTATAC ACCGCCAAGA AGTACGCGGT 240
GCCAGCGCTC GAGGCCCATT GCGTGGAGTT CCTGAAGAAG AACCTGCGAG CCGACAACGC 300
CTTCATGCTG CTCACGCAGG CGCGACTCTT CGATGAACCG CAGCTGGCCA GCCTGTGCCT 360
GGAGAACATC GACAAAAACA CTGCAGACGC CATCACCGCG GAGGGCTTCA CCGACATTGA 420
CCTGGACACG CTGGTGGCTG TCCTGGAGCG CGACACACTG GGCATCCGTG AGGTGCGGCT 480
GTTCAATGCC GTTGTCCGCT GGTCCGAGGC CGAGTGTCAG CGGCAGCAGC TGCAGGTGAC 540
GCCAGAGAAC AGGCGGAAGG TTCTGGGCAA GGCCCTGGGC CTCATTCGCT TCCCGCTCAT 600
GACCATCGAG GAGTTCGCTG CAGGTCCCGC ACAGTCGGGC ATCCTGGTGG ACCGCGAGGT 660
GGTCAGCCTC TTCTGCACTT CACCGTCAAC CCCAAGCCAC GAGTGGAGTT CATTGACCGG 720
CCCCGCTGCT GCCTGCGTGG GAAGGAGTGC AGCATCAACC GCTTCCAGCA GGTGGAGAGT 780
CGCTGGGGCT ACAGSGGGAC CAGTGACCGC ATCAGGTTCT CAGTCAACAA GCGCATCTTC 840
GTGGTGGGAT TTGGGCTGTA TGGATCCATC CACGGGCCCA CCGACTACCA AGTGAACATC 900
CAGATTATTC ACACCGATAG CAACACCGTC TTGGGCCAGA ACGACACGGG CTTCAGCTGC 960
GACGGCTCAG CCAGCACCTT CCGCGTCATG TTCAAGGAGC CGGTGGAGGT GCTGCCCAAC 1020
GTCAACTACA CGGCCTGTGC CACGCTCAAG GGCCCAGACT CCCACTACGG CACCAAAGGC 1080
CTGCGCAAGG TGACACACGA GTCGCCCACC ACGGGCGCCA AGACCTGCTT CACCTTTTGC 1140
TACGCGGCCG GGAACAACAA TGGCACATCC GTGGAGGACG GCCAGATCCC CGAGGTCATC 1200
TTCTACACCT AGGCTGCCCG ACACCGACAC CGCCCTCCCT CCGTGGGGAT AGCCGCAGCC 1260
CCAGGCCATC ATCTGCTGCT GGGGYCCCCC CACCACGCGG TGCCAGGCCC AGTGTCCCCC 1320
AGGCCGTCTG TCCACTCCAT GCCACCTTTC TCAGCATCAG GACGGGGTTG CCCTGTGTTC 1380
ACCACGAGTK TGGCTGCTGG ATCAGGGCAG CCGGGGAGGT GGCCAGGCCA GTGGCCAGGC 1440
CCTGTGGAGA CAATCCCTCA GGACTAGGGA CAGGGCTGTG CCGGCCTGGG CCAGGGCCCA 1500
CGGACCCGCA GCTCAGGGCG CCTGCCCACG TCGTCTGCCG GCGGTGCGCC GCGGGCGTCC 1560
CTCGCGTCTC TTCACTGCAC ATTGCAATGC ATTTGCGATT CCCATTTCTC TGCTAGGAGC 1620
CAGCCTGGGT GGCGCTGCTC CCAGAGCCGT GGGTCCCAGA CCTTGCGTTC CTTTTGTTCC 1680
TGTCCGTTTA TCAGGACACG GGCCCCACCT GTCACGTGCC CGAGGCCACC CAAGCCCAGC 1740
CTGCGGGGCG TTCCCACTGC CTGGATGCCG GCTTGAGTTC TGCGCACGCA GGATTCAGTG 1800

TGGGGACGGC CCCTGCCGGA TAGGCCTAGC CCTGGCCCAG GTGGTGAGCG GTTTGCAGTG 1860

TCCGTTCTCA TCCACCTGAT GGGCCCAGAT AAAGGCCCCC GCTGTCCAGC CTCCCTGGAC 1920

GGCCCTCGCG GTCCCTGCAG CCCAAGATGG GACTCAGACC CTGTGCCCCA GAGCTCCCCT 1980

GCCGCAGAAT GGGGCCCCAG CCGGCCCCGA CCGGGTCCAG GAGCACTGCT CGCCTGTACA 2040

TACTGTTGCC CTAGCCCACC TGGTGCCGTG GGAGCCACCC CCAGGTGCTG GGGCACAGCC 2100

CCTCCCCACT CCGGCCACGC CCCCACCCAC CCCGCGTGTT TCTGCCCTGT GACTCCTGGA 2160

ACCTGCGTCC TCCCCAAAGC CATGGGAGGG GTGTCCTCCT CAGACCATGC CCCCAGATGA 2220

TTTTTTTAAA TAAAGAAACA AATGCACCTG CAAAACAAAA AAAAAAAAAA AAAACTCGA 2279

(2) INFORMATION FOR SEQ ID NO: 38:

(i) SEQUENCE CHARACTERISTICS:
(A) LENGTH: 745 base pairs
(B) TYPE: nucleic acid
(C) STRANDEDNESS: double
(D) TOPOLOGY: linear

(xi) SEQUENCE DESCRIPTION: SEQ ID NO: 38:

GTACAGGACT GAGAAGCAGA TAACAAGAGT GACGCTCACA GGGCTGGGCT GACGCTAACA 60

GGAGGCAGTG TGTGGCTCGA AGATTCTTGA ACCCACAGCA GCAGCTGCGG CCACCCCATC 120

CTGCCCACAG CTCCAGCCCT GAGACGACGA GGAGGAGAGT CGACTTTGCC TCTTGCCCAA 180

GGGACCATGC CCAGGTGCCG GTGGCTCTCC CTGATCCTCC TCACCATTCC CCTGGCCCTG 240

GTGGCCAGGA AAGACCCAAA AAAGAATGAG ACGGGGGTGC TGAGGAAATT AAAACCCGTC 300

AATGCCTTCA ANTGCCAACG TGGAAGCAGT GTYYGTGGTT TTGCCATGCA AGAATACAAC 360

AAAGAGAGCG AGGACAAGTA TGTCTTCCTG GTGGTCAAGA CACTGCAAGC CCAGCTTCAG 420

GTCACAAATC TTCTGGAATA CCTTATTGAT GTAGAAATTG CCCGCAGCGA TTGCAGAAAG 480

CCTTTAAGCA CTAATGAAAT CGCGCCATTC AAGARAACTC CAAGCTGAAA AGGAAATTAA 540

GCTGCAGCTT TTTGGTAGGA GCACTTCCCT GGAATGGTGA ATTCACTGTG ATGGAGAAAA 600

AGTGTGAAGA TGCTTAATGG TGTTTTGAGG CATCCCTCCA ACCTCTGTGA CTACTTTATC 660

CATGAAAATG AAGCAATGGT CAGGTGGGAG GCTCTTCCCA ATGTGCTTTC TTCAAAAAAA 720

AAAAAAAAAA AAAAAAAAAA CTCGA 745

(2) INFORMATION FOR SEQ ID NO: 39:

(i) SEQUENCE CHARACTERISTICS:
(A) LENGTH: 1718 base pairs
(B) TYPE: nucleic acid
(C) STRANDEDNESS: double
(D) TOPOLOGY: linear

(xi) SEQUENCE DESCRIPTION: SEQ ID NO: 39:

CCCCATAGGC AGGAGGCCCC CGGGCAGCAC ATCCTGTCTG CTTGTGTCTG CTGCAGAGTT 60

CTGTCCTTGC ATTGGTGCGC CTCAGGCCAG GCTGCACTGC TGGGACCTGG GCCATGTCTC 120

CCCACCCCAC CGCCCTCCTG GGCCTAGTGC TCTGCCTGGC CCAGACCATC CACACGCAGG 180

AGGAAGATCT GCCCAGACCC TCCATCTCGG CTGAGCCAGG CACCGTGATC CCCCTGGGGA 240

GCCATGTGAC TTTCGTGTGC CGGGGCCCGG TTGGGGTTCA AACATTCCGC CTGGAGAGGG 300

AGAGTAGATC CACATACAAT GATACTGAAG ATGTGTCTCA AGCTAGTCCA TCTGAGTCAG 360

AGGCCAGATT CCGCATTGAC TCAGTAAGTG AAGGAAATGC CGGGCCTTAT CGCTGCATCT 420

ATTATAAGCC CCCTAAATGG TCTGAGCAGA GTGACTACTG GAGCTGCTGG TGAAAGAAAC 480

CTCTGGAGGC CSGGACTCCC CGGACACAGA GCCCGGCTCC TCAGCTGGAC CCACGCAGAG 540

GCCGTCGGAC AACAGTCACA ATGAGCATGC ACCTGCTTCC CAAGGCCTGA AAGCTGAGCA 600

TCTGTATATT CTCATCGGGG TCTCAGTGGT CTTCCTCTTC TGTCTCCTCC TCCTGGTCCT 660

CTTCTGCCTC CATCGCCAGA ATCAGATAAA GCAGGGGCCC CCCAGAAGCA AGGACGAGGA 720

GCAGAAGCCA CAGCAGAGGC CTGACCTGGC TGTTGATGTT CTAGAGAGGA CAGCAGACAA 780

GGCCACAGTC AATGGACTTC CTGAGAAGGA CAGAGAGACG GACACCTCGG CCCTGGCTGC 840

AGGGAGTTCC CAGGAGGTGA CGTATGCTCA GCTGGACCAC TGGGCCCTCA CACAGAGGAC 900

AGCCCGGGCT GTGTCCCCAC AGTCCACAAA GCCCATGGCC GAGTCCATCA CGTATGCAGC 960

CGTTGCCAGA CACTGACCCC ATACCCACCT GGCCTCTGCA CCTGAGGGTA GAAAGTCACT 1020

CTAGGAAAAG CCTGAAGCAG CCATTTGGAA GGCTTCCTGT TGGATTCCTC TTCATCTAGA 1080

AAGCCAGCCA GGCAGCTGTC CTGGAGACAA GAGCTGGAGA CTGGAGGTTT CTAACCAGCA 1140

TCCAGAAGGT TCGTTAGCCA GGTGGTCCCT TCTACAATCG AGCAGCTCCT TGGACAGACT 1200

GTTTCTCAGT TATTTCCAGA GACCCAGCTA CAGTTCCCTG GCTGTTTCTA GAGACCCAGC 1260

TTTATTCACC TGACTGTTTC CAGAGACCCA GCTAAAGTCA CCTGCCTGTT CTAAAGGCCC 1320

AGCTACAGCC AATCAGCCGA TTTCCTGAGC AGTGATGCCA CCTCCAAGCT TGTCCTAGGT 1380

GTCTGCTGTG AACCTCCAGT GACCCCAGAG ACTTTGCTGT AATTATCTGC CCTGCTGACC 1440

CTAAAGACCT TCCTAGAAGT CAAGAGCTAG CCTTGAGACT GTGCTATACA CACACAGCTG 1500

AGAGCCAAGC CCAGTTCTCT GGGTTGTGCT TTACTCCACG CATCAATAAA TAATTTTGAA 1560

GGCCTCACAT CTGGCAGCCC CAGGCCTGGT CCTGGGTGCA TAGGTCTCTC GGACCCACTC 1620

TCTGCCTTCA CAGTTGTTCA AAGCTGAGTG AGGGAAACAG GACCTACGAA AAAAAAAAAA 1680

AAAAAAATCG AGGGGGGGCC CGTACCCAAT CGCCTGTA 1718

(2) INFORMATION FOR SEQ ID NO: 40:

(i) SEQUENCE CHARACTERISTICS:
(A) LENGTH: 1966 base pairs
(B) TYPE: nucleic acid
(C) STRANDEDNESS: double
(D) TOPOLOGY: linear

(xi) SEQUENCE DESCRIPTION: SEQ ID NO: 40:

GTCGCGCCTG CAGGTCGACA CTAGTGGATC CAAAGAATTC GGCACGAGCT GGGGAGCGGG 60

ACTSGAGAAT ACTGCCCAGT TACTCTAGCG CGCCAGGCCG AACCGCAGCT TCTTGGCTTA 120

GGTACTTCTA CTCACAGCGG CCGATTCCGA GGCCAACTCC AGCAATGGCT TTTGCAAATC 180

TGCGGAAAGT GCTCATCAGT GACAGCCTGG ACCCTTGCTG CCGGAAGATC TTGCAAGATG 240

GAGGGCTGCA GGTGGTGGAA AAGCAGAACC TTAGCAAAGA GGAGCTGATA GCGGACTGCA 300

GGACTGTGAA GGCCTTATTG TTCGCTCTGC CACCAAGGTG ACCGCTGATG TCATCAACGC 360

AGCTGAGAAA CTCCAGGTGG TGGGCAGGGC TGGCACAGGT GTGGACAATG TGGATCTGGA 420

GGCCGCAACA AGGAAGGGCA TCTTGGTTAT GAACACCCCC AATGGGAACA GCCTCAGTGC 480

CGCAGAACTC ACTTGTGGAA TGATCATGTG CCTGGCCAGG CAGATTCCCC AGGCGACGGC 540

TTCGATGAAG GACGGCAAAT GGGAGCGGAA GAAGTTCATG GGAACAGAGC TGAATGGAAA 600

GACCCTGGGA ATTCTTGGCC TGGGCAGGAT TGGGAGAGAG GTAGCTACCC GGATGCAGTC 660

CTTTGGGATG AAGACTATAG GGTATGACCC CATCATTTCC CCAGAGGTCT CGGCCTCCTT 720

TGGTGTTCAG CAGCTGCCCC TGGAGGAGAT CTGGCCTCTC TGTGATTTCA TCACTGTGCA 780

CACTCCTCTC CTGCCCTCCA CGACAGGCTT GCTGAATGAC AACACCTTTG CCCAGTGCAA 840

GAAGGGGGTG CGTGTGGTGA ACTGTGCCCG TGGAGGGATC GTGGACGAAG GCGCCCTGCT 900

CCGGGCCCTG CAGTCTGGCC AGTGTGCCGG GGCTGCACTG GACGTGTTTA CGGAAGAGCC 960

GCCACGGGAC CGGGCCTTGG TGGACCATGA GAATGTCATC AGCTGTCCCC ACCTGGGTGC 1020

CAGCACCAAG GAGGCTCAGA GCCGCTGTGG GGAGGAAATT GCTGTTCAGT TCGTGGACAT 1080

GGTGAAGGGG AAATCTCTCA CGGGGGTTGT GAATGCCCAG GCCCTTACCA GTGCCTTCTC 1140

TCCACACACC AAGCCTTGGA TTGGTCTGGC AGAAGCTCTG GGGACACTGA TGCGAGCCTG 1200

GGCTGGGTCC CCCAAAGGGA CCATCCAGGT GATAACACAG GGAACATCCC TGAAGAATGC 1260

TGGGAACTGC CTAAGCCCCG CAGTCATTGT CGGCCTCCTG AAAGAGGCTT CCAAGCAGGC 1320

GGATGTGAAC TTGGTGAACG CTAAGCTGCT GGTGAAAGAG GCTGGCCTCA ATGTCACCAC 1380

CTCCCACAGC CCTGCTGCAC CAGGGGAGCA AGGCTTCGGG GAATGCCTCC TGGCCGTGGC 1440

CCTGGCAGGC GCCCCTTACC AGGCTGTGGG CTTGGTCCAA GGCACTACRC CTGTACTGCA 1500

GGGGCTCAAT GGAGCTGTCT TCAGGCCAGA AGTGCCTCTC CGCAGGGACC TGCCCCTGCT 1560

CCTATTCCGG ACTCAGACCT CTGACCCTGC AATGCTGCCT ACCATGATTG GCCTCCTGGC 1620

AGAGGCAGGC GTGCGGCTGC TGTCCTACCA GACTTCACTG GTGTCAGATG GGGAGACCTG 1680

GCACGTCATG GGCATCTCCT CCTTGCTGCC CAGCCTGGAA GCGTGGAAGC AGCATGTGAC 1740

TGAAGCCTTC CAGTTCCACT TCTAACCTTG GAGCTCACTG GTCCCTGCCT CTGGGGCTTT 1800

TCTGAAGAAA CCCACCCACT GTGATCAATA GGGAGAGAAA ATCCACATTC TTGGGCTGAA 1860

CGCGGGCCTC TGACACTGCT TACACTGCAC TCTGACCCTG TAGTACAGCA ATAACCGTCT 1920

AATAAAGAGC CTACCCCCAA AAAAAAAAAA AAAAAAAAAA ACTCGA 1966

(2) INFORMATION FOR SEQ ID NO: 41:

(i) SEQUENCE CHARACTERISTICS:
(A) LENGTH: 972 base pairs
(B) TYPE: nucleic acid
(C) STRANDEDNESS: double
(D) TOPOLOGY: linear

(xi) SEQUENCE DESCRIPTION: SEQ ID NO: 41:

GGCACGAGCC AAGTGGTCCC CCAGACAAGG CTCAGGATGT CCACATCCAC TGCATCCTGG 60

ACCCTGTGCA GGTGAAGATG TCCCGACCCA CGCATACTCC TCTTTCGCCT GCCACCATTT 120

CTCCAACCAT CACAGTAGCA GTCTTCTTCG CTGTGTTCGT CGCCGCCGCC GCCGCCACCG 180

CCGTTGTCGC CGTCGCTGCT GCAACCACCA GCAGCGGSCG CAGAACTASA GACAAATCCC 240

CCATAGCCAC TCAGTCTTCC GTAACCCACA TCGCAGCCAA AAGATGTCAC AACTACACCG 300

AGTGCCTTTC TTTGATCAGG ARGACCCGGA TTCCTACCTG GARGARGARG ACAACCTGCC 360

CTTCCCGTAT CCCAAGTACC CACGTCGCGG CTGGGGCGGG TTTTATCAGA GAGCGGGCCT 420

GCCTCCAATG TGGGGCTGTG GGGCCACCAG GGTGTATCCT GGCCAGTCTG CCACCACCCT 480

CTCTCTACCT GTCACCTGAG CTGCGCTGCA TGCCCAAGCG TGTAGAGGCC AGGTCTGAGC 540

TGAGGCTCTG CCCGCCTGGC GTCNTCTGAC TACCTCTGCC TCCCTCACGG TGTTGGACGA 600

GGCCTCCCAT CAACGGACCC CAGCTCCAAG CTCAGTGCTG GTCCCCCATT CCTCCCAGCC 660

CTGGCCCAAA GTCCAGGCTG CGGACCCTGC CCCTCCCCCG ACCATGTTTG TCCCACTCAG 720

CCGGAATCCA GGGGGCAATG CCAACTACCA GGTGTACGAC AGCCTGGAGC TGAAGCGGCA 780

GGTGCAGAAG AGCAGAGCCA GGTCCAGCTC ACTGCCACCG GCTTCCACCT CCACCTTGAG 840

GCCCTYTCTG CACAGGAGCC AGACCGAGAA ACTCAACTGA CCAGCAGGCG GATGTGGGGT 900

GTGGGGCAGG GCATGGAGGG AGAGGAATAA AGAGAAACAG AGTCCAGGAA AAAAAAAAAA 960

AAAAAAACTC GA 972

(2) INFORMATION FOR SEQ ID NO: 42:

(i) SEQUENCE CHARACTERISTICS:
(A) LENGTH: 1536 base pairs
(B) TYPE: nucleic acid
(C) STRANDEDNESS: double
(D) TOPOLOGY: linear

(xi) SEQUENCE DESCRIPTION: SEQ ID NO: 42:

GGCACAGGCC AACTTAGTTT GAGTTCTTCT TCTGGACTCT GTATGTCCTT GTGTGTACCC 60

TATGCCGTTC ACAGTCCGTA CTCTCTCTGT GARATTGGCT GTCTAATCCA GGTGGATCAG 120

GAGGTGCTTT GTGGTTTTTT TGCAAAGAAA TGAAGTCTGG CAAGCAAACA ATGATTAAAC 180

ATGTTTCGAT TCGTGACTTG TCTTTTGGCG AAATGCAAAG GTGGGTGTGC ATTCTTGAAT 240

TCAAAGAAAA TCTCTTTCAA ATCCCCTCAT CCCTTGTTGC TCTTCTAAAT ACTCTCTTTC 300

TAGATATCTT GCACCCCCAA AACTCCCTCA GCCCCCATGG CAGCTTTTCT CTCTCCTCTC 360

TCTCTTTCCC GCCTCTCCCT GTCTCCTCAC TTCAGCCTTT CCTCTTTCTT AGATCTTTAT 420

TATGTAGATA AAAACCCCTC CAACCTCCTT AGCCTTCTCT CCATTGCATC CCCTACCCGA 480

ATTATCCTCA AGAAAGAGGC CAGGATCCGA CACAGCGATC AGAAATCCTC CTCCCTTASA 540

AGCSCAGGGG TGAGGGAGTT CAGGAATATT CATACACTGG TAATCCTTGT CCCTGTTACA 600

GTCACTTCCT TGTATCAGGA CCCTTGTTAC TATTTACAGA CTATTTTCCA TCTCTCCTAA 660

TGCAATTGCT CAAAGGGCAC TTTAAGNATA ATCATTATCC ATTGATGTTT TTTGGAGGCT 720

TTTATTCCCT CCAATAAGTT CTGCCGAATA CTGGCCGCTG GCTCTATTTG TTAAACAATG 780

GAGGGCTTTG TTCCGCTTTT TTTTTTTTTT TTWTTCWTAA CCTGAGCTTT CTGCCCACCC 840

TTAGTATGGG GCCAAAGGGA AGATTTTTAT GCCACCCCTT TTGGTGAGAA GAGTCACTTC 900

CTGATTAGTG TTTGGGCTGA AAATGGGTCC CCCTTTGGGA AGAAACATGG GTGCAGTGTA 960

CTTCCTGTGT CACAGGATTA ACAGCTCCTG CCCCACTCCC AAGGAGGCAG CTCYTCGGGG 1020

CAGTTCYTCT TTGAGAATTT CATGGTCATT AAGAAGCAGG YTCCCAGGGA CCCCAGAGTG 1080

GGAACCTTTG ACTGAAGTCA CCACAGTGGG TGTAAGATAA ACATAAGAGA CTTTTCTCAG 1140

GGAAGATTTG GAACGAAGAA AAAGAGTAAA AAGTTCACAT GGACCATGGA GTGTTNTGGA 1200

AAAGGGCCCA GAAAGGGAAG CTGTGGCTAA GAAGATAAAC TGCCTGATTG CAGAGACCCA 1260

GGAGAGGGGA TGAAATCTCT TTGTCTGGTC ACATTTCTCW WTAATGATKY TCCACATGTA 1320

CAAAGCTAGC CAGTTTACCA AGTGCTTCCA CACACATTGC TTCATTCTGT GTCTCTTAAG 1380

CAGATTGACT CCTTGGAAAA GCCTCACGTC TGGCATTCTG CACCTGCCCA TCACCAGTTT 1440

GGCCTTGGTC TGCTTGGCTG GTTGGGTCTC CCCATGGTGA GCTCCCATGG TATCTCCTCT 1500

TCACCTTTAT ATCACTCATT AGACACCGGT GACAAC 1536

(2) INFORMATION FOR SEQ ID NO: 43:

(i) SEQUENCE CHARACTERISTICS:
(A) LENGTH: 2541 base pairs
(B) TYPE: nucleic acid
(C) STRANDEDNESS: double
(D) TOPOLOGY: linear

(xi) SEQUENCE DESCRIPTION: SEQ ID NO: 43:

AATTCGGCAC GAGGTTCCTG GCCAACCTGC TGCTGGAGGA GGATAACAAG TTTTGTGCAG 60

ATTGCCAGTC TAAAGGGCCG CGATGGGCCT CTTGGAACAT TGGTGTGTTC ATCTGCATTC 120

GATGTGCTSG AATCCACAGG AATCTGGGGG TGCACATATC CAGGGTAAAG TCAGTTAACC 180

TCGACCAGTG GACTCAAGTA CAGATTCAGT GCATGCAAGW GATGGGAAAT GGAAAGGCAA 240

ACCGACTTTA TGAAGCCTAT CTTCCTGAGA CCTTTCGGCG ACCTCAGATA GACCCAGCTG 300

TTGAAGGATT TATTCGAGAC AAWTATGAGA AGAAGAAATA CATGGACCGA AGTCTGGGAC 360

ATCAATGCCT TTAGGAAAGA AAAAGATGAC AAGTGGAAAA GAGGGAGCGA ACCAGTTCCA 420

GAAAAAAAAT TGGAACCTGT TGTTTTTGAG AAGGTGAAAA TGCCACAGAA AAAAGAAGAC 480

CCACAGCTAC CTCGGAAAAG CTCCCCGAAA TCCACAGCGC CTGTCATGGA TTTGTTGGGC 540

CTTGATGCTC CTGTGGCCTG CTCCATTGCA AATAGTAAGA CCAGCAATAC CCTAGAGAAG 600

GATTTAGATC TGTTGGCCTC TGTTCCATCC CCTTCTTCTT CGGGTTCCAG AAAGGTTGTA 660

GGTTCCATGC CAACTGCAGG GAGTGCCGGC TCTGTTCCTG AAAATCTGAA CCTGTTTCCG 720

GAGCCAGGGA GCAAATCAGA AGAAATAGGC AAGAAACAGC TCTCTAAAGA CTCCATTCTT 780

TCACTGTATG GATCCCAGAC GCYTCAAATG CCTACTCAAG CAATGTTCAT GGCTCCCGCT 840

CAGATGGCAT ATCCCACAGC CTACCCCAGC TTCCCCGGGG TTACACCTCC TAACAGCATA 900

ATGGGGAGCA TGATGCCTCC ACCAGTAGGC ATGGTTGCTC AGCCAGGAGC TTCTGGGATG 960

GTTGCCCCCA TGGCCATGCC TGCAGGCTAT ATGGGTGGCA TGCAGGCATC AATGATGGGT 1020

GTGCCGAATG GAATGATGAC CACCCAGCAG GCTGGCTACA TGGCAGGCAT GGCAGCTATG 1080

CCCCAGACTG TGTATGGGGT CCAGCCAGCT CAGCAGCTGC AATGGAACCT TACTCAGATG 1140

ACCCAGCAGA TGGCTGGGAT GAACTTCTAT GGAGCCAATG GCATGATGAA CTATGGACAG 1200

TCAATGAGTG GCGGAAATGG ACAGGCAGCA AATCAGACTC TCAGTCCTCA GATGTGGAAA 1260

TAAAAACAAA ACACCTGTAT GGCTGCCATT CTCTTCAGCC CTCGCTCTCC CCTTTCCACA 1320

GCCTCCACCC CTGACCCCCA TCCTCTTTTC CTACCTCTCT GTTTGGTTTA GAAATTGCTC 1380

AATAAGTCAT TTGGGGTTTG GCATCCTGCC CAGCCACTTC CCAAACATGA AGACCTCTCT 1440

GTTGCTTTAT GTTGTACATG CCCCATAGCC ATCCCAACGT CCTCCCCAGT CCTCTCCTGG 1500

CACCAGCACC TTAGAAGTTG TTGGCAGAAG GCACTTAAAC TGTGGGAGAA GTGTGCACAC 1560

CTTTGAGTCC CTTCCCTCAA GGTTAAAGCT CCTGTCAGAC TCTCAGAAGG GTCTGTGGGT 1620

GTTGTATATT AGGCAAACAG GGGAAAGCTT AGAGGTCCTT CTATATGTGT TAATAAGCTG 1680

TTTCTAAGTG TTTAAATTTG AAAAGCATCA TGTTCTCATG ATTTATGGGA ATGAAGCAAG 1740

TACTGAAATC AAATTAAATA CTCCCTGGGT CCTGGGTCAG TTTGACCCTA GCCCTGGGGT 1800

GAGGCAAGCC CCCTCCTATG AGGATGAGCA AAAATACTAC TCTCTTCGCC CTGAGTTGCT 1860

TTCTGGATCT GGGGCTTCAG GACTTGCTGC TTCAGTCAGC CTTTATTAGC ACCAAAGACT 1920

TTATGAAGAT CCCACACACA GACACACATC CCTTCCCGCC TCCCCCCTGC CTTCAGTAGG 1980

ATCTGGCTCC GTGGCTGGAG GACCAACCCC TATAGTGGGA ATGCAGAGCT TAACGTGTAC 2040

TGCTTGTGTG TGTGCGTGAG TGTGTGTGTG TGTATGAGTG TGTGTTCCGC CTCCCACCCT 2100

CTCCCCATCT GCTCTGGGTA TTTTTGTTTT TGTTTAGTTT TAGGTTTACA ACAGAGAGGA 2160

ATTAATTTAT CAGCAGCCTA AAACTGTTGT GTTTTTCTTA TGGTTTAAAA AACGCCATGT 2220

CATTGATAAC TCCCTTTCTC CCTTCCCTTC TCCCGGTCTG CTGATCACTC TTTCATGCCT 2280

GTGTATCCAG GGTGCTCTGT TTCCCCACCG TTCCCAGGTG TACGAGGCAG AGGGCCGGGA 2340

CAGCTTTCCT CTCAGTCATT GTTCACCCCA CTTGAAAATT CAGACAAGAA AACTTTGCTT 2400

AAAAGATTTC ATGTGTGGGA ACCACAGTTC CTGGCTGCCT TTCTCCTGTG TATGTGTAAA 2460

TTCCTTAATA AATATTGCAG GGAAGGACAA AAAAAAAAAA AAAAAAAAAA AAAAAAAAAA 2520

AAAAAAAAAA AAAAAACTCG A 2541

(2) INFORMATION FOR SEQ ID NO: 44:

(i) SEQUENCE CHARACTERISTICS:
(A) LENGTH: 2418 base pairs
(B) TYPE: nucleic acid
(C) STRANDEDNESS: double
(D) TOPOLOGY: linear

(xi) SEQUENCE DESCRIPTION: SEQ ID NO: 44:

CCCACGCGTC CGCCCACGCG TCCGCCCACG CGTCCGCCCA CGCGTCCGGG ACTCAGCGAA 60

GGGTGGGCGC CGCCGAGGCC TCCTGCCGCT GGCGGGTTTC CGCGGAGTGC CGCCCGGCTC 120

CGCTCTGCCG CCGGCGCGGC TCATGGGCAG AGTCGGCCGG GCGGGCCGGC ATTAAACTGA 180

AGAAAAGATG TCCCTGTACG ATGACCTAGG AGTGGAGACC AGTGACTCAA AAACAGAAGG 240

CTGGTCCAAA AACTTCAAAC TTCTGCAGTC TCAGCTTCAG GTGAAGAAGG CAGCTCTCAC 300

TCAGGCAAAG AGCCAAAGGA CGAAACAAAG TACAGTCCTC GCCCCAGTCA TTGACCTGAA 360

GCGAGGTGGC TCCTCAGATG ACCGGCAAAT TGTGGACACT CCACCGCATG TAGCAGCTGG 420

GCTGAAGGAT CCTGTTCCCA GTGGGTTTTC TGCAGGGGAA GTTCTGATTC CCTTAGCTGA 480

CGAATATGAC CCTATGTTTC CTAATGATTA TGAGAAAGTA GTGAAGCGCG CAAAGAGAGG 540

AACGACAGAG ACAGCGGGAG TGGANAAGAC AAAAGGAAAT AGAAGAAAGG GAAAAAAGGC 600

GTAAAGACAG ACATGAAGCA AGTGGGTTTG CAAGGAGACC AGATCCAGAT TCTGATGAAG 660

ATGAAGATTA TGAGCGAGAG AGGAGGAAAA GAAGTATGGG CGGACTGCCA TTGCCCCACC 720

CACTTCTCTG GTAGAGAAAG ACAAAGAGTT ACCCCGAGAT TTTCCTTATG AAGAGGACTC 780

AAGACCTCGA TCACAGTCTT CCAAAGCAGC CATTCCTCCC CCAGTGTACG AGGAACAAGA 840

CAGACCGAGA TCTCCAACCG GACCTAGCAA CTCCTTCCTC GCTAACATGG GGGGCACGGT 900

GGCGCACAAG ATCATGCAGA AGTACGGCTT CCGGGAGGGC CAGGGTCTGG GGAAGCATGA 960

GCAGGGCCTG AGCACTGCCT TGTCAGTGGA GAAGACCAGC AAGCGTGGCG GCAAGATCAT 1020

CGTGGGCGAC GCCACAGAGA AAGGTGTGTC CCCAGGGAAG CGTGTGACTA GAGGGAAAGG 1080

ACTGGCCCCA TCCATATCAG ACATGGCCAG TCTTGATCCT CATGTGTCAG CAGGGGGACA 1140

ATGAGGCGTG TGGCCAGAGG GAGAGGGCTG GCCCTGCCAT CACTAGAACA CAGGCCGTCC 1200

TGTTCATATG ATGCACTGCC ACTTCCGTTT TGTGAAACCA GGAATCCTGA GGCTCATCTT 1260

TATTTTTTCA GAACAGACGT AGAGAGATGA AGGCTTGTGG AGGAAAAGAT GGTGAGAGAC 1320

TTGGGCAGAA AATGAGTAGT CCTCAGGAAG AAATCTTGGT TATGTGTTTA GAGCATGAAG 1380

GACAGAGCCA TATAGTGTGG CAGTGAATAT ACCTGCTATC TCCATCTCAG AGGTCGTCTC 1440

TACTTTTCCC TTTTGCCCTT TCAGTATAGA TGTGATTTCT GATTCTCTTA CAGATTGTTT 1500

GCTTTGCGAG ATCTGATGTT ATGTTGCAGT CTCTTGGTAA ATGATGCCTA GTTGGTGTTT 1560

TATTTTCATT TAATTTTTAC AGTCTGTTCT GTGTTGAGGG AATTCAGGAA AGAGACAAAC 1620

ATATGTTAGC ATTTTAATCA GGGAATTAAG TTTGAGTCAG CCTAGCTGAA CTTCCTTTGC 1680

TAAAGAAAGA AGAAAACTTT TCTGGCAGCC CCGTTCATGC ACAGCTTAGG GATACATCAC 1740

GAGCCTGACA GATGCATCCA AGAAGTCAGA TTCAAATCCG CTGACTGAAA TACTTAAGTG 1800

TCCTACTAAA GTGGTCTTAC TAAGGAACAT GGTTGGTGCG GGAGAGGTGG ATGAAGACTT 1860

GGNAAGTTGA AACCAAGGAA GAATGTGAAA AATATGGCAA AGTTGGAAAA TGTGTGATAT 1920

TTGAAATTCC TGGTGCCCCT GATGATGAAG CAGTACGGAT ATTTTTAGAA TTTGAGAGAG 1980

TTGAATCAGC AATTAAAGCG GTTGTTGACT TGAATGGGAG GTATTTTGGT GGACGGGTGG 2040

TAAAAGCATG TTTCTACAAT TTGGACAAAT TCAGGGTCTT GGATTTGGCA GAACAAGTTT 2100

GATTTTAAGA ACTAGAGCAC GAGTCATCTC CGGTGATCCT TAAATGAACT GCAGGCTGAG 2160

AAAAGAAGGA AAAAGGTCAC AGCCTCCATG GCTGTTGCAT ACCAAGACTC TTGGAAGGAC 2220

TTCTAAGATA TATGTTGATT GATCCCTTTT TTATTTTGTG GTTTTTTAAT ATAGTATAAA 2280

AATCCTTTTA AAAAAACAAC AATCTGTGTG CCTCTCTGGT TGTTTCTCTT TTTTATTATT 2340

ACTCCTGAGT TGATGACATT TTTTGTTAGA TTTCATGGTA ATTCTCAAGT GCTTCAATGA 2400

TGCAGCATTT CTTGCACT 2418

(2) INFORMATION FOR SEQ ID NO: 45:

(i) SEQUENCE CHARACTERISTICS:
(A) LENGTH: 1337 base pairs
(B) TYPE: nucleic acid
(C) STRANDEDNESS: double
(D) TOPOLOGY: linear

(xi) SEQUENCE DESCRIPTION: SEQ ID NO: 45:

TCGACCCACG CGTCCGGAGC GACCTCTCTG CTCCGCTCGT CTCGTTGGTT CCGGAGGTCG 60

CTGCGGCGGT GGGAAATGCT GGCGCGCGCG GCGCGGGGCA CTGGGGCCCT TTTGCTGAGG 120

GGCTCTCTAC TGGCTTCTGG CCGCGCTCCG CGCCGCGCCT CCTCTGGATT GCCCCGAAAC 180

ACCGTGGTAC TGTTCGTGCC GCAGCAGGAG GCCTGGGTGG TGGAGCGAAT GGGCCGATTC 240

CACCGGATCC TGGAGCCTGG TTTGAACATC CTCATCCCTG TGTTAGACCG GATCCGATAT 300

GTGCAGAGTC TCAAGGAAAT TGTCATCAAC GTGCCTGAGC AGTCGGCTGT GACTCTCGAC 360

AATGTAACTC TGCAAATCGA TGGAGTCCTT TACCTGCGCA TCATGGACCC TTACAAGGCA 420

AGCTACGGTG TGGAGGACCC TGAGTATGCC GTCACCCAGC TAGCTCAAAC AACCATGAGA 480

TCAGAGCTCG GCAAACTCTC TCTGGACAAA GTCTTCCGGG AACGGGAGTC CCTGAATGCC 540

AGCATTGTGG ATGCCATCAA CCAAGCTGCT GACTGCTGGG GTATCCGCTG CCTCCGTTAT 600

GAGATCAAGG ATATCCATGT GCCACCCCGG GTGAAAGAGT CTATGCAGAT GCAGGTGGAG 660

GCAGAGCGGC GGAAACGGGC CACAGTTCTA GAGTCTGAGG GGACCCGAGA GTCGGCCATC 720

AATGTGGCAG AAGGGAAGAA ACAGGCCCAG ATCCTGGCCT CCGAAGCAGA AAAGGCTGAA 780

CAGATAAATC AGGCAGCAGG AGAGGCCAGT GCAGTTCTGG CGAAGGCCAA GGCTAAAGCT 840

GAAGCTATTC GAATCCTGGC TGCAGCTCTG ACACAACATA ATGGAGATGC AGCAGCTTCA 900

CTGACTGTGG CCGAGCAGTA TGTCAGCGCG TTCTCCAAAC TGGCCAAGGA CTCCAACACT 960

ATCCTACTGC CCTCCAACCC TGGCGATGTC ACCAGCATGG TGGCTCAGGC CATGGGTGTA 1020

TATGGAGCCC TCACCAAAGC CCCAGTGCCA GGGACTCCAG ACTCACTCTC CAGTGGGAGC 1080

AGCAGAGATG TCCAGGGTAC AGATGCAAGT CTTGATGAGG AACTTGATCG AGTCAAGATG 1140

AGTTAGTGGA GCTGGGCTTG GCCAGGGAGT CTGGGGACAA GGAAGCAGAT TTTCCTGATT 1200

CTGGCTCTAG CTTCCCTGCC AAGATTTTGG TTTTTATTTT TTTATTTGAA CTTTAGTCGT 1260

GTAATAAACT CACCAGTGGC AAACCAAAAA AAAAAAAAAA AAAAAAAAAA AAAAAAAAAA 1320

AAAAAAAAAA AAAANNN 1337

(2) INFORMATION FOR SEQ ID NO: 46:

(i) SEQUENCE CHARACTERISTICS:
(A) LENGTH: 1276 base pairs
(B) TYPE: nucleic acid
(C) STRANDEDNESS: double
(D) TOPOLOGY: linear

(xi) SEQUENCE DESCRIPTION: SEQ ID NO: 46:

CTCACGCGTC CGGGACGGCN GGACGCGTGG GTGCATTTGC TGAGTGTTTT ACTTCCAATT 60

ATGTGATTCN ATATTACAGG NGCTGCCATG TGGTAATGAG AAGAATGTAT ATTCTGTTGT 120

TTTGGGGTGG ARTGTTCCAT AGATGTCTAT CARGTCTGTT TGATCCAGAR CTGARTTCAR 180

GTCCTGGTAT CTCARTCTTT ACTGTGARTC TTCAAATGAC ATAAGAATGA CAGAAMTTGT 240

AGTTAAGGAC AACAGRGCAW TSCAAGGCAG CAGCATAGTC CAAAATAGAC GTGTCTTCTT 300

CCCGAAGTCA CTGTAGTGGG GGACATAAAA TTTAAGGAAC CTCTGGGTCT TACTACCTGA 360

TGTGGCCAAT TGGACTAAAA CCAATAACCA TTAAGGAAWA AATSSACTWA ACCACAAGCA 420

ACTCAATTAA MAAATAGGCA AAGAACTTGA AGAGGCATTT TCCCAAAGAA GCCAACAAGC 480

ATGTGAAAAG ATGCTCAACA TCATTAGACA TCAGGGAAAT ACAGATCAAA ATCAAAATGA 540

GATACCAGTT TATACTAAGG TGGCTATAAT AAACATCATA ATAATGAAGG ACATTAACAT 600

GTATTAGTGA GGATGTGGAG AAATGGAACC CATTTCTGGT AGGAATGTAA AATAGTGCAG 660

CCACTGTGGA AAACAGTTTG GTGGTTCCCC AGAAAGCTAA GCATAGAGTT ACCAGAGAAC 720

CTAGCAATTT AACTTATAGG TACATACTTC AAAGGAATTG AAAACATAGA TYCTAACAGA 780

TACTKGTACA GCAATATYCA TKGTGGCWTT ATTCACGATA GCCAAAAGGT AAAACAACTC 840

AAGTGTCCAT CAAAATATAA ATGTGTAAAC AATGTGGTAT ATTCCTAGAG GGGAATATTA 900

TTCAGCTTTA AAAAGGAATG AAGTACTGGT ACATGCTACA AAGGTGGATG AGCCTCAGAA 960

ACATGCTGAG TGAAAGAAGC CAATGATAAA AGACCATATA TTGTATGATT CCATTATATG 1020

AAATKTCCAG RACATTCAAG TCTATAGAGA CAGAAAGTAG ATTAGTGAYT GCTTAGGGCT 1080

GGCAGGGATA AGGGGKTCAT GGCTAAAGGG TATGGGTTTT TGTTTGTGGA GGTGAAAAAT 1140

TTTAAAACTT GKGSTGATGG TTGCACAAGC CTGTGAAGAT ACTGAAAACC ATTGAATTGT 1200

GTGCTTTAAA TGGATGAATT GTATGGTGTT TGAACTATAT CCCAATAAAG CTGTTTTTTA 1260

AAAAAGAAAA AAAAAA 1276

(2) INFORMATION FOR SEQ ID NO: 47:

(i) SEQUENCE CHARACTERISTICS:
(A) LENGTH: 1282 base pairs
(B) TYPE: nucleic acid
(C) STRANDEDNESS: double
(D) TOPOLOGY: linear

(xi) SEQUENCE DESCRIPTION: SEQ ID NO: 47:

GGCACGAGAG AAAGGCCAGT TTGTGGGGCA AATTAGACTA AACTCTGTGC TGGTAGAACT 60

GCTTTCCAAG AATGCTGTCA CTGCTATAGT TTTTAATGCT TCAAATCTCA ACTCNCTCCC 120

TCCATTCGCC ATAGCTCAAC CATGTTCCAG GAGTGTATTC CAATCAGCTT GTTTTYTCTT 180

AACTGGTCAA AGGAATGTTG CTCATTCACC TGCCCCAACT CACATATTAA CAATTGTTTA 240

ACTGGGATTA GATAAAAGGA AAGCTGACTT ACAGATGAAC CAAGAGGGAG CTATTTATGC 300

CACAGCCCCC AGCCCAGTAA CTTTATGTTT CTGATCTCCT GCAAAATTTT TTTATAAAAA 360

AAGCTTAGCC AGGAACTAGT AGAAAGAATA AAGTAAAGAT GGTGTAAGAA ATATATGGAT 420

AGGCAAGTTC CWNYGYTGAG ACCTTAYGAA GAATGGTGAG GTGTGGTTAA ATGGAGGAGA 480

TAATCAGCAG ATAAWAGCTC AGATGGTCMS AAACATWTAG AACTATAATG CCATCTCCAA 540

AGTATTGCAT GCATACAAAT GACGTTCAAT CCGTTGAATA TAATGGAGAC ACACTATTTC 600

AAAAATTAAG TTCTTCTWTC TTGAGCTTTA AAAGTATACA CATTTACCCM AATGAATTWA 660

AAACATGCMC ACMAATATTT ATATCAAAAG TGTACATGAT TTCCAAAACT TGGAAGTWAC 720

CAAGATTTAC TTCCWTGGGT TAGTGCATAA ATTAACTGTG ATACATATAT ACTATGGAAT 780

WTTAYTCAGC AACAGAAATA AATGAGHTAT CAAACCACAG AAAGACATGG AGGAAACTTA 840

AATCCAGGTG GMTAAGTGAW AGAAGCCAAT ATGAAAAGGC TACATTSTAT ATGATTTCAA 900

ATATATGACA TTCAGGAAAA GGCAAGGCTG CAGAGACAGT AAARAGATCA GCTAGGTGCA 960

TGKGGSTCAC GCCACTTTGG GAGGCTTGAG GCAGGKGGAT TATMTTGAAG TCAGGAGTTC 1020

NAGACCAGCN TGGGCAACAT GNTGANACCC CATATNTCCT AAAAGNACNA AAATTTAACT 1080

GGGCGTGGTG GCACGTGCCT GTANTCCCAN CNACTCTGGT GGCTNAGACN GGNGAATTGC 1140

TTGAACCCAG GAGGCAGAGG TTGCGGTGAG CCAATGATTG CACCACTGCA NTCCAGCCTG 1200

GGTGGTAGAG CGAGACTCAG TCTCAACNTT NATCAAGATA GGANNGAAAT AGAANGGAAG 1260

AAAGAGAAAA AATAAAAATA NA 1282

(2) INFORMATION FOR SEQ ID NO: 48:

(i) SEQUENCE CHARACTERISTICS:
(A) LENGTH: 645 base pairs
(B) TYPE: nucleic acid
(C) STRANDEDNESS: double
(D) TOPOLOGY: linear

(xi) SEQUENCE DESCRIPTION: SEQ ID NO: 48:

AAGGTAGAAA AGTACAGAAA ACACTAAATT TTCATTGTGC TGTTTCAATG TGGCAGATTC 60

TTTAAAATAC TTCGACACGC TACAATAATT AAAGGTTTTA AGAACATTAA GATACTTAAA 120

AAATAAAAGC CCACAATTGA ATAACAAAAA TGAACTTTGT TTTATTTTTT ATTGGCATTA 180

ATGTAGGTTG CCGTGGTGAA AATAGTTTGA AATACTTCAC AGTAACAGTT TTGTGCAGCC 240

CTAGAGATTA AAAACAGCAA AGTAAATAAG CAGGACTCTC AACGACTCAT ACTCACAGAC 300

ATGTTTAATG TAATCCTAGC ACTTCGGGAG GCTGAGGCGG GAGGATTACT TGAGCCTAGG 360

AGTTTGAGAC CAGCCTGGGC AACATAGCAA GATCCCATCT CTACAAAAAA GTGAAAAAGT 420

TAGCTGAACA AGGCGGCATG CACATGCTAC TCCAGACGCT GAAGTGGGAA GATCACTTAA 480

GTCCGAGAGA TCGAGGCTTC AGTGAGATAT GGCTGAGACA CTGCTCTCAG CCTGGATGAC 540

AGAGTGAGAA CCTGTCTCAA ACAAGAGAAA AAAATAAATC AAATGCTATT CAAAATTCTA 600

AAAAAAAAAA AAAAAAAAAA AAAAAAAAAA AAAAAAAAAA AAAAA 645

(2) INFORMATION FOR SEQ ID NO: 49:

(i) SEQUENCE CHARACTERISTICS:
(A) LENGTH: 1495 base pairs
(B) TYPE: nucleic acid
(C) STRANDEDNESS: double
(D) TOPOLOGY: linear

(xi) SEQUENCE DESCRIPTION: SEQ ID NO: 49:

TGTGGAAAAC AGTAGGAAAG CAATGAAAGA AGCTGGTAAG GGAGGCGTCG CTGATTCCAG 60

AGAGCTAAAG CCGATGGTAG GTGGAGATGA GGAGGTGGCC GCCCTCCAAG AATTTCACTT 120

TCACTTCCTC TCTCTCTCTG TCTTCACTGA CTGCACTTCT TCAGGAGAAG CTTTTGTTAT 180

CTGTATCACG CAGACATGCT GCTCTTTCTG TTTGTGTGCT TACCCATCAC TTGGATGGCA 240

GAATTCTTGT CACAACTGAG ACCACCTTCT ATAAAAGTAA GCTGAAAGGA ACAGCATCCT 300

CGTCAGTGCT CGGCAGGGGC GGGTAGGGGA TGATGGTTTT TTCCCTAAGG TAAAACTGCT 360

GTTGCTCTTG TTTCCTTTTT AACTGTCAGT GTTTGGCTTT CATCAGAMTG AACATTTTGG 420

TGTTCCACTT GAACTGACGG TTTGATTTTT ATCATTTTGG AAAGGTGATC ATAGCAATTC 480

CTTTCCAACT TGCTAAAATT CCATACTCCC CCCTTTTAAA ARWATKGTTS TGCTTMCATT 540

GCTKTMCWTT TSCCTTGKCT SMCTTTTTCY TCCTGTKGSC TGAARTTKTW CYTTCYTTKT 600

TTCTTAAGST WTTTTTCAGT AGCAAACAAG GCTGTTTTCA TCAATACCCA CATTCCCAYT 660

CRGKRRGRMM ATYTAGTYTT YTCCCAGKTT AAKTGKGRGR KGGRKGAAAA TRATKTCKGG 720

KANGKGGAWA TKAWAWAKGK KWWATGKAAA CACAAATATA TYTYTYTAMA TTCCACTTTA 780

ATTKGGGAAA AAAGGCAGCT KAAGTGGAGT GTWAAGRARR ACCTKGRRST GCTTTTCAAC 840

ATGGGATATG GTCACTATRG CATRGGAAAC ANGATGCCTT CTATCAWAKA TGGGTCTAAT 900

TACTYCCTAA TTTAAAACAC GTATTTTTTT AAATAGCATG TTTATTTTCA AATATDATAT 960

AATGGTCGSG CRTCCTTAAA TAATTTTAAA CAANGTGTCC CCGRGACNGC ATATAATGTT 1020

CAAAWGTKAG AGGTAAGGAC TTYCCTTTCT GTCTYCTTAA CACTTWAGTA AATRATTNGA 1080

WTTAWAGCAA GTTTGTCCAA CTKGCNNCCT GNGGNCCGCA NANGGMWGRG GAAGGGCTTT 1140

TCMAACACAA ATTCGTAAAC TTTATTAAAA CATGAGATTT TTTGCCTTTT TTTTTTTAAG 1200

CCCATCAGCT ATCCTTAATG TATTTTANAT GTGGCCCAAG ACAATTCTTC TTCCAGGATG 1260

GCCTGGGGAA GCCAAAAGAT TGGANACCCC TGATTTGTAG GTTTTCAACT TTAAAATATA 1320

TGCTATAAAA TAAGTTCATT TAAGTAGGCT AGGCATGGTG GCTCATGTNT GTAATCCTAG 1380

CACTTAGGGG GCCCGAGGCA GAAAGATTRM CTGAGCTCAG CAGTTTGAGA CCAGCCTGGG 1440

CCAAACGGTG NAACCCTGTT TTTACTNAAA TACCCAAAAA AAAAAAAAAA AAAAA 1495

(2) INFORMATION FOR SEQ ID NO: 50:

(i) SEQUENCE CHARACTERISTICS:
(A) LENGTH: 1630 base pairs
(B) TYPE: nucleic acid
(C) STRANDEDNESS: double
(D) TOPOLOGY: linear

(xi) SEQUENCE DESCRIPTION: SEQ ID NO: 50:

GAATTCGGCA CGAGATTATC TGTCTTCTTC TTACCAATTT ATAGAACTTT TTAGTATTGC 60

AGATAAAGTT CCTCATCGGA TATCTTCTCT CCTTCTATTG GGTACCTTTT TATTGTCTTA 120

ATGGGGGTCT TTTAATGACC AGAAGTTCTT AGTTTTAAAA TAGTCCAGTT TATCCATTTT 180

TAAATTGTTA GTGCTATTTG TGTCCTGCTT GAGAGATTTT TGCCTACTGC AAGGTCACAA 240

AGATGTTTTC CTCTAAAAGC CTTTTGGTTT TGCCCTTTTG TTTTAGATCT GCAGCTCATC 300

TGGAATTGAG TGTGTGGTGT GTGTGTGGTG TGAGGTAGGG GTCCTTTTTT TCATATGGAT 360

ATCCAATTGA CCCAGAACAG TGTATTGAAA AAAAAAATCT GTCTTAGTCA ATTTGGACTG 420

CCGTAACAAA ATACCATAAC CTGGGTGGCT TAGACTACAG AAATGTAGCG CTCACAGYTC 480

TGGAGGCTGG AAGGCCAGGA TCAAGACACC AGCAGATTCG GTGTCTNGTG AGGACCCACT 540

TTGTGNTTCA TAGATGTCAC CTTCTTGCTG TGTCCCAGTG GTGRAAGGGG CAAACTAGCT 600

CCCTTAAACC TCTTTTTATA AGATCCCTAA AACCTTTAAT GAGGGCTCCA CCCTAATGAT 660

CTAATCACCT CTCAATACCT TATCTTGGGG GTTAAGATTT GAACAGAGGA ATTTGGGGGA 720

GACATAGACA TTTGGAGCAT AGCATCTTCT TTTCCTCAGT GCACAGCAGT GCTGCCTTCA 780

TCATCAGTCA GGTGTCTGTA GGTGTGTGGC TATTTCTGGA CTTGGCACTC TGTCCTACTT 840

GTTGATTTCT CTGCCTTATA CCAATGCCAC ACCATCTTAA TTATTGTAAC CATCTTAATT 900

ATTTATAAAA AGTCTTTTTT TTTTTTTTGA TACAGTCTCA CTCTGTCCCC CAGGCTGGAG 960

TGCAGAGGTA CAGTATTGGC TCACTGCAAC CTCTGTCCCC AGGCTTAAGC AATTCTCATG 1020

CCTCAGCCTC CTGAGTAGCT GGGATTACAT GTGCACCACC ACACTTGGCC TTCTTTCTTT 1080

TCTTTCCAAY CCATTKGTTT TTTATTTCTT TCCCTKGCTT TATKGCACTG GCTAAGATTT 1140

CCAGTGCTGA ATAGGAGTGA TGACAGTGGG CACCCTTGTC TTTCTCCCAA CCTCAGAGGG 1200

AAAAGTATCC AATGCATTTG TAGATATTCT TTATCAGATT AGCTTCCTTT CTAGCGGCTT 1260

GTGTCTTTGC ATTGTTTTTC ATGAGCAAGT GTTGAACTTT TTCACTGAGT TTTCCAAATA 1320

CTTTTTCCAT TGAGTTTTTT TACTTTAACC GTCATATTGC CAAAAGTCTG CATTTGTTAT 1380

TTCCTCCCAA ATTGCTGGGA TTATAGGCAT TAGCCACTGC ACCCAGCCAG ACTTTATAGA 1440

AAATCTTGAT ATCTGGTCAT GGAAGTCCCC TAGCTTGGTT ATTTTTTTTT GGTACCGCTT 1500

TGTCTATTTT CGGCCCTTTC CATTTCCATG TAACTTTTAG GATCAGCTTG TCAGTTCCTA 1560

CCAAAAAAAA AAAAAAAAAA ACTCGAGGGG GGCCCGGTAC CCAAATCGCC GGGTAGTGAT 1620

CGTAACAATC 1630

(2) INFORMATION FOR SEQ ID NO: 51:

(i) SEQUENCE CHARACTERISTICS:
(A) LENGTH: 2420 base pairs
(B) TYPE: nucleic acid
(C) STRANDEDNESS: double
(D) TOPOLOGY: linear

(xi) SEQUENCE DESCRIPTION: SEQ ID NO: 51:

GCCAACAGTG CTCCCTCATA GATGGACGAA GTGTGACCCC CCTTCAGGCT TCAGGGGGAC 60

TGGTCCTCCT GGAGGGAGAT GCTCGCCTTG GGGAATAATC ACTTTATTGG TTTTGTGAAT 120

GATTCTGTGA CTAAGTCTAT TGTGGCTTTG CGCTTAACTC TGGTGGTGAA GGTCAGCACG 180

WGGCCGGGGG AGAGTCACGC AAATGACTTG GAGTGTTCAG GAAAAGGAAA ATGCACCACG 240

AAGCCGTCAG AGGCAACTTT TTCCTGTACC TGTGAGGAGC AGTACGTGGG TACTTTCTGT 300

GAAGAATACG ATGCTTGCCA GAGGAAACCT TGCCAAAACA ACGCGAGCTG TATTGATGCA 360

AATGAAAAGC AAGATGGGAG CAATTTCACC TGTGTTTGCC TTCCTGGTTA TACTGGAGAG 420

CTTTGCCAGT CCAAGATTGA TTACTGCATC CTAGACCCAT GCAGAAATGG AGCAACATGC 480

ATTTCCAGTC TCAGTGGATT CACCTGCCAG TGTCCAGAAG GATACTTCGG ATCTGCTTGT 540

GAAGAAAAGG TGGACCCCTG CGCCTCGTCT CCGTGCCAGA ACAACGGCAC CTGCTATGTG 600

GACGGGGTAC ACTTTACCTG CAACTGCAGC CCGGGCTTCA CAGGGCCGAC CTGTGCCCAG 660

CTTATTGACT TCTGTGCCCT CAGCCCCTGT GCTCATGGCA CGTGCCGCAG CGTGGGCACC 720

AGCTACAAAT GCCTCTGTGA TCCAGGTTAC CATGGCCTCT ACTGTGAGGA GGAATATAAT 780

GAGTGCCTCT CCGCTCCATG CCTGAATGCA GCCACCTGCA GGGACCTCGT TAATGGCTAT 840

GAGTGTGTGT GCCTGGCAGA ATACAAAGGA ACACACTGTG AATTGTACAA GGATCCCTGC 900

GCTAACGTCA GCTGTCTGAA CGGAGCCACC TGTGACAGCG ACGGCCTGAA TGGCACGTGC 960

ATCTGTGCAC CCGGGTTTAC AGGTGAAGAG TGCGACATTG ACATAAATGA ATGTGACAGT 1020

AACCCCTGCC ACCATGGTGG GAGCTGCCTG GACCAGCCCA ATGGTTATAA CTSCCACTGC 1080

CCGCATGGTT GGGTGGGAGC AAACTGTGAG ATCCACCTCC AATGGAAGTC CGGGCACATG 1140

GCGGAGAGCC TCACCAACAT GCCACGGCAC TCCCTCTACA TCATCATTGG AGCCCTCTGC 1200

GTGGCCTTCA TCCTTATGCT GATCATCCTG ATCGTGGGGA TTTGCCGCAT CAGCCGCATT 1260

GAATACCAGG GTTCTTCCAG GCCAGCCTAT RAGGAGTTCT ACAACTGCCG CAGCATCGAC 1320

AGCGAGTTCA GCAATGCCAT TGCATCCATC CGGCATGCCA GGTTTGGAAA GAAATCCCGG 1380

CCTGCAATGT ATGATGTGAG CCCCATCGCC TATGAAGATT ACAGTCCTGA TGACAAACCC 1440

TTGGTCACAC TGATTAAAAC TAAAGATTTG TAATCTTTTT TTGGATTATT TTTCAAAAAG 1500

ATGAGATACT ACACTCATTT AAATATTTTT AAGAAAWTAA AAAGCTTAAG AAATTTAAAA 1560

TGCTAGCTGC TCAAGAGTTT TCAGTAGAAT ATTTAAGAAC TAATTTTCTG CAGCTTTTAG 1620

TTTGGAAAAA ATATTTTAAA AACAAAATTT GTGNAACCTA TAGACGATGT TTTAATGTAC 1680

CTTCAGCTCT CTAAACTGTG TGCTTCTACT AGTGTGTGCT CTTTTCACTG TAGACACTAT 1740

CACGAGACCC AGATTAATTT CTGTGGTTGT TACAGAATAA GTCTAATCAA GGAGAAGTTT 1800

CTGTTTGACG TTTGAGTGCC GGCTTTCTGA GTAGAGTTAG GAAAACCACG TAACGTAGCA 1860

TATGATGTAT AATAGAGTAT ACCCGTTACT TAAAAAGAAG TCTGAAATGT TCGTTTTGTG 1920

GAAAAGAAAC TAGTTAAATT TACTATTCCT AACCCGAATG AAATTAGCCT TTGCCTTATT 1980

CTGTGCATGG GTAAGTAACT TATTTCTGCA CTGTTTTGTT GAACTTTGTG GAAACATTCT 2040

TTCGAGTTTG TTTTTGTCAT TTTCGTAACA GTCGTCGAAC TAGGCCTCAA AAACATACGT 2100

AACGAAAAGG CCTAGCGAGG CAAATTCTGA TTGATTTGAA TCTATATTTT TCTTTAAAAA 2160

GTCAAGGGTT CTATATTGTR AGTAAATTAA ATTTACATTT GAGTTGTTTG TTGCTAAGAG 2220

GTAGTAAATG TAAGAGAGTA CTGGTTCCTT CAGTAGTGAG TATTTCTCAT AGTGCAGCTT 2280

TATTTATCTC CAGGATGTTT TTGTGGCTGT ATTTGATTGA TATGTGCTTC TTCTGATTCT 2340

TGCTAATTTC CAACCATATT GAATAAATGT GATCAAGTCA AAAAAAAAAA AAAAAAAAAA 2400

AACTCGAGGG GGGGTCCCGT 2420

(2) INFORMATION FOR SEQ ID NO: 52:

(i) SEQUENCE CHARACTERISTICS:
(A) LENGTH: 1172 base pairs
(B) TYPE: nucleic acid
(C) STRANDEDNESS: double
(D) TOPOLOGY: linear

(xi) SEQUENCE DESCRIPTION: SEQ ID NO: 52:

AAAATTATTC TGTACCATCA CAGCTTTTCA CAACGATGGC AAGCCTTATG TCTTGGGAGC 60

CTGTTTTGCT AGGCAAAGTT ACAAGTGACC TAATGGGAGC TCAAATGTGT GTGTGTCTCT 120

CTGTGTGTTT GTGTGTGTGT GTGCACTCAA GACCTCTAAC AGCCTCGAAG CCTGGGGTGG 180

CATCCCGGCC TTGCCATTAG CATGCCTCAT GCATCATCAG ATGACAAGGA CAACCCTCAT 240

GACGAAGCAA CATGAATTAG GGGGCCTCTT GGCCTTGGTC CAAAATTGTC AATCAGAAAT 300

GAACATAAAG GACTCCAGAG CAGTGGGACT GTCTGTCAAA AGACTCTGTA TATCTTTTGT 360

GGATGAGTTT TGTGAGAGAA CAGAGAGACC ATTGTACCTG GCACAAGGGC TSTTCATGAA 420

AAGGGAGACT TACTGGGAGG TGCAAGACAG TGGCATTTCT CCTCTCCTCT TGCTGCTCAG 480

CACAGCCCTG GATTGCAGCC CCGAGGCTGA GACCAGACAA AGCCCGGGAG GCAGAAAGAT 540

GCTCCAAGAA CCAACACTAT CAATGTCTTT GCAAATCCTC ACAGGATTCC TGTGGGTCCA 600

GCTTTGGAAC TGGGAAACCT TTCTTCGGAT CCGCACTCAT TCCACTGATG CCAGCTGCCC 660

CTGAAGGATG CCAGTACTGT GGTGTGTGAG TCTCAGCAGC CGCCCACACG CTCCTAACTC 720

TGCTGCATGG CAGATGCCTA GGTGGAAATA GCAAAAACAA GGCCCAGGCT GGGGCCAGGG 780

CCAGAGGGGA AGGCCCTGGA TTCTCACTCA TGTGAGATCT TGAATCTCTT TCTTTGTTCT 840

GTTTGTTTAG TTAGTATCAT CTGGTAAAAT AGTTAAAAAA CAACAAAAAA CTCTGTATCT 900

GTTTCTAGCA TGTGCTGCAT TGACTCTATT AATCACATTT CAAATTCACC CTACATTCCT 960

CTCCTCTTCA CTAGCCTCTC TGAAGGTGTC CTGGCCAGCC CTGGAGAAGC ACTGGTGTCT 1020

GCAGCACCCC TCAGTTCCTG TGCCTCAGCC CACAGGCCAC TGTGATAATG GTCTGTTTAG 1080

CACTTCTGTA TTTATTGTAA GAATGATTAT AATGAAGATA CACACTRTAA CTACAAGAAA 1140

TTATAAATGT TTTTCACATC AAAAAAAAAA AA 1172

(2) INFORMATION FOR SEQ ID NO: 53:

(i) SEQUENCE CHARACTERISTICS:
(A) LENGTH: 1589 base pairs
(B) TYPE: nucleic acid
(C) STRANDEDNESS: double
(D) TOPOLOGY: linear

(xi) SEQUENCE DESCRIPTION: SEQ ID NO: 53:

CCCACGCGTC CGCCCACGCG TCCGCCCACG CGTCCGTTTC AAAGGGAGCG CACTTCCGCT 60

GCCCTTTCTT TCGCCAGCCT TACGGCCCCG AACCCTCGTG TGAAGGGTGC AGTACCTAAG 120

CCGGAGCGGG GTAGAGGCGG GCCGGCACCC CCTTCTGACC TCCAGTGCCG CCGGCCTCAA 180

GATCAGACAT GGCCCAGAAC TTGAAGGACT TGGCGGGACG GCTGCCCGCC GGGCCCCGGG 240

GCATGGGCAC GGCCCTGAAG CTGTTGCTGG GGGCCGGCGC CGTGGCCTAC GGTGTGCGCG 300

AATCTGTGTT CACCGTGGAA GGCGGGCACA GAGCCATCTT CTTCAATCGG ATCGGTGGAG 360

TGCAGCAGGA CACTATCCTG GCCGAGGGCC TTCACTTCAG GATCCCTTGG TTCCAGTACC 420

CCATTATCTA TGACATTCGG GCCAGACCTC GAAAAATCTC CTCCCCTACA GGCTCCAAAG 480

ACCTACAGAT GGTGAATATC TCCCTGCGAG TGTTGTCTCG ACCCAATGCT CAGGAGCTTC 540

CTAGCATGTA CCAGCGCCTA GGGCTGGACT ACGAGGAACG AGTGTTGCCG TCCATTGTCA 600

ACGAGGTGCT CAAGAGTGTG GTGGCCAAGT TCAATGCCTC ACAGCTGATC ACCCAGCGGG 660

CCCAGGTATC CCTGTTGATC CGCCGGGAGC TGACAGAGAG GGCCAAGGAC TTCAGCCTCA 720

TCCTGGATGA TGTGGCCATC ACAGAGCTGA GCTTTAGCCG AGAGTACACA GCTGCTGTAG 780

AAGCCAAACA AGTGGCCCAG CAGGAGGCCC AGCGGGCCMA ATTCTTGGTA GAAAAAGCAA 840

AGCAGGAACA GCGGCAGAAA ATTGTGCAGG CCGAGGGTGA GGCCGAGGCT GCCAAGATGC 900

TTGGAGAAGC ACTGAGCAAG AACCCTGGCT ACATCAAACT TCGCAAGATT CGAGCAGCCC 960

AGAATATCTC CAAGACGATC GCCACATCAC AGAATCGTAT CTATCTCACA GCTGACAACC 1020

TTGTGCTGAA CCTACAGGAT GAAAGTTTCA CCAGGGGAAG TGACAGCCTC ATCAAGGGTA 1080

AGAAATGAGC CTAGTCACCA AGAACTCCAC CCCCAGAGGA AGTGGATCTG CTTCTCCAGT 1140

TTTTGAGGAG CCAGCCAGGG GTCCAGCACA GCCCTACCCC GCCCCAGTAT CATGCGATGG 1200

TCCCCCACAC CGGTTCCCTG AACCCCTCTT GGATTAAGGA AGACTGAAGA CTAGCCCCTT 1260

TTCTGGGGAA TTACTTTCCT CCTCCCTGTG TTAACTGGGG CTGTTGGGGA CAGTGCGTGA 1320

TTTCTCAGTG ATTTCCTACA GTGTTGTTCC CTCCCTCAAG GCTGGGAGGA GATAAACACC 1380

AACCCAGGAA TTCTCAATAA ATTTTTATTA CTTAACCTGA AGTCAAGGCT TCACGTGTTC 1440

ATGAACTGGG TAACTGGCAG CAAGCATGCG CACGTTCACA TGTGCGCTCC TGGGTCTGTC 1500

TTTGTGTGTG CCAGCAGGGG GCGCAAAAGA ATCTGGCTGG GGCGGCTAAN GGGAAGCAAG 1560

GCCTGGGCTC CGAAACANGA CCCAACTGG 1589

(2) INFORMATION FOR SEQ ID NO: 54:

(i) SEQUENCE CHARACTERISTICS:
(A) LENGTH: 2074 base pairs
(B) TYPE: nucleic acid
(C) STRANDEDNESS: double
(D) TOPOLOGY: linear

(xi) SEQUENCE DESCRIPTION: SEQ ID NO: 54:

CCGCCTGACC GCCCCGGGCT TAAGGGAGCC TGGCTAGGCC GGCAGCCGGA TGGTCCCGCA 60

GCTCGGGGCC GGCCATGCTT CGCGGTCCGT GGCGCCAGCT TTGGCTCTTT YTCCTGCTGC 120
TGCTCCCGGG CGCGCCTGAG CCCCGCGGCG CCTCCAGGCC GTGGGAGGGA ACCGACGAGC 180
CGGGCTCGGC CTGGGCCTGG CCGGGCTTCC AGCGCCTGCA GGAGCAGCTC AGGGCGGCGG 240
GTGCCCTCTC CAAGCGGTAC TGGACGCTCT TCAGCTGCCA GGTGTGGCCC GACGACTGTG 300
ACGAGGACGA GGARGCAGCC ACGGGGCCCC TGGGCTGGCG CCTTCCTCTG TTGGGCCAGC 360
GGTACCTGGA CCTCCTGACC ACGTGGTACT GCAGCTTCAA AGACTGCTGC CCTAGAGGGG 420
ATTGCAGAAT CTCCAACAAC TTTACAGGCT TAGAGTGGGA CCTGAATGTG CGGCTGCATG 480
GCCAGCATTT GGTCCAGCAG CTGGTCCTAA GAACAGTGAG GGCTACTTA GAGACGCCCC 540
ACCCAGAAAA GGCCCTTGCT CTGTCGTTCC ACGGCTGGTC TGGCACAGGC AAGAACTTCG 600
TGGCACGGAT GCTGGTGGAG AACCTGTATC GGGACGGGCT GATGAGTGAC TGTGTCAGGA 660
TGTTCATCGC CACGTTCCAC TTTCCTCACC CCAAATATGT GGACCTGTAC AAGGAGCAGC 720
TGATGAGCCA GATCCGGGAG ACGCAGCAGC TCTGCCACCA GACCCTGTTC ATCTTCGATG 780
AAGCGGAGAA GCTGCACCCA GGGCTGCTGG AGGTCCTTGG GCCACACTTA GAACGCCGGG 840
CCCCTGANGG CCACAGGGCT GAGTCTCCAT GGACTATCTT TCTGTTTCTC AGTAATCTCA 900
GGGGCGATAT AATCAATGAG GTGGTCCTAA AGTTGCTCAA GGCTGGATGG TCCCGGGAAG 960
AAATTACGAT GGAACACCTG GAGCCCCACC TCCAGGCGGA GATTGTGGAG ACCATAGACA 1020
ATGGCTTTGG CCACAGCCGT CTTGTGAAGG AAAACCTGAT TGACTACTTC ATCCCCTTCC 1080
TGCCTTTGGA GTACCGTCAC GTGAGGCTGT GTGCACGGGA TGCCTTCCTG AGCCAGGAGC 1140
TCCTGTATAA AGAAGAGACA CTGGATGAAA TAGCCCAGAT GATGGTGTAT GTCCCCAAGG 1200
AGGAACAACT CTTTTCTTCC CAGGGCTGCA AGTCTATTTC CCAGAGGATT AACTACTTCC 1260
TGTCATGAAG GCTAGAGGAA GACTTCCTGG AACTGCCTTT CTTCCACTAA CAGGACCCTG 1320
GGACCTGTAG GAGCACCCCG TTTGGGACTG TGAGGTGTTT GAGGGTGTGG ACTGGCATCC 1380
AGCAGCCACT AACAAACACA CAACTGGTGT GTAAAAGGCA GGCCTTACAT TAGAAGCCAA 1440
GCCAATCCTT TTTCTTTTTT TTGGAGGTCC CACCGAGATA GATAGGAACT TGGATTGCTG 1500
AATTCAAAAA CAGAGCCCAT TCTTAAGATC ACTTGGTGCC TTAAAGACAC GCATTCCAAA 1560
GTGGAATGTG GTTGAAGAAA GTGGGCCAGG TGGTTGAAGA AAGCCATGTG GGAGCTCAGC 1620
AAATCCCAAG GGCTTATTAT GACACTCCAG ATGGTCTCCT TAGCATCTCA GCTCTTCTGC 1680
AAGGAAGAGC TTGGGTGTTA GGCCTCAGAG GCTGTAGGGT CCTTGGGTTA CAGAGCCGGG 1740
GAGAACGAAG TTCTGTGACC CAGGGGTGGA GAATACACTC TAGGTTTGCG GGCTGGTGGG 1800
CTTTCAAATT GGTACTTCCA GAGGAAAGCC AAGCTGCTTC TGTTGTGAGC GAATCAGCCA 1860

AGAGCCTGAG GCTGAAGGGA AAAGTACACA GAGGAAGATA TTTTACAAAC CAGGTCAGTG 1920

TAGGCCAAGA CTTATGGTCT ACAGATTTTG GCGGGGGAGG GGGGACCTTT TCAAAGACAA 1980

TAGGGGGTCT TGACATGTTT GTTGTATGTA AAGATGATAA GATTAAAATT TTTGATTTTC 2040

CTAAAAAAAA AAAAAAAAAA AAAAAAAAAA TTNC 2074

(2) INFORMATION FOR SEQ ID NO: 55:

(i) SEQUENCE CHARACTERISTICS:
(A) LENGTH: 1483 base pairs
(B) TYPE: nucleic acid
(C) STRANDEDNESS: double
(D) TOPOLOGY: linear

(xi) SEQUENCE DESCRIPTION: SEQ ID NO: 55:

GAATTCGSCA CGMGCGTGGA GGCGCCACGT CCCTTGCGGC GGCGGGAGAG AAATCGCTTG 60

GACTTCGGGG CGGCCTCGGA CGGCCATGGC CTTTACCCTG TACTCACTGC TGCAGGCASC 120

CCTGCTCTGC GTCAACGCCA TCGCAGTGCT GCACGAGGAG CGATTCCTCA AGAACATTGG 180

CTGGGGAACA GACCAGGGAA TTGGTGGATT TGGAGAAGAG CCGGGAATTA AATCACAGCT 240

AATGAACCTT ATTCGATCTG TAAGAACCGT GATGAGAGTG CCATTGATAA TAGTAAACTC 300

AATTGCAATT GTGTTACTTT TATTATTTGG ATGAATATCA GTGGAGAAAA TGGAGACTCA 360

GAAGAGGACA TGCCAGTAGA AGTTATTACT TTGGTCATTA TTGGAATATT TATATCTTAG 420

CTGGCTGACC TTGCACTTGT CAAAAATGTA AAGCTGAAAA TAAAACCAGG GTTTCTATTT 480

ATCTGTTTTT TTTTTTAATG TTGCACTTGT AGTTTCATTA CAAAAGATCA GATCATGAAA 540

GGCAGTAACT CTCCAGGACT GGAATATCTG ATTGCTCAGT GTTAATAGTA GTTCATGCTG 600

TGGTGAGATT GTTAAAAGGG TGCAAGACTG TTGCTTCTCT TTTTTTAGAT ATTTTTCTAT 660

CTCTCACTTC TCAGGGATGA AATTCTTTTT CAAAGTTTTG AAGTTCCTTG CAACTTAGCC 720

ATGATGTGAG TGGTTATCCC TAGATAAAAT TAAAAGGATT TTTAAAAAGT AATTACTGCA 780

CATAAAATGA TAAATAGGTA ATTTGAATAA TTTTATTTTA AGCTCCTTGG TTAATTATTT 840

TGTCTATTGT CTCAGCTATA AATTCAAATT TATACATACT ATTGAGTATT AATATTCTCT 900

GATTTCAGGG AGAATTCTGT CAGTCACATG ATGATTATGT TTTTNTTTAA CATTCTTTCC 960

ATGCACTTGT TATTTTATTA ATTTGCCTGA ATGATGAGAC CAGACCAGTG TCTACAGATT 1020

TTCATTGTCA GAAAAATCTA TAAGTCTGCC CTTTTTACAA TGATGGATTT AAAAAAAACA 1080

ACAGCGTAAA TATTAGCCCA CAAGAGCAGT CCTAAACAAT CACAATTACA CTGTACTACC 1140

CAAGAAGACT GTTTATTGTG AAGCATTTAC CTTTCAAAAA ATCATTACAT TTCTATTTCT 1200

TGGTGGAGCA GCACATTGTG GAGTGTGATT CTTAATTCTT CATTGAGTTT GTCAATAGGA 1260

CATTGATGCT GGATAGGTTG TCTTTTGTTT TTATGTCTCA GACCATCTTG TGAGATTGTT 1320

TGCCTATCTC ATAATACAGT TTTATGCAGA AAGGTTGAAA CTATGTAAAT GGTTTTTATG 1380

GAAATTATCA GTTACAATAT TTTAAAGGTG TAGAATGGCA TCTTTGTTTA TAGGAGAACA 1440

TTTGTAAATA AAGTTAAATT TCTAAGTCAA AAAAAAAAAA AAA 1483

(2) INFORMATION FOR SEQ ID NO: 56:

(i) SEQUENCE CHARACTERISTICS:
(A) LENGTH: 1123 base pairs
(B) TYPE: nucleic acid
(C) STRANDEDNESS: double
(D) TOPOLOGY: linear

(xi) SEQUENCE DESCRIPTION: SEQ ID NO: 56:

CAAAAATAAT AATAGTCATC ACATTTGTAT AGCACTGGGT CATTTTTCCC AAGACCATTT 60

AGTTACTTGA CCTCAGCTGT TGTCCAGCTT CCAGTCTTGG GGTAATGGCA GCTTAATAAT 120

CTGAAAATTG CCAAGAGAAA GATGTGGAAG GATGAAATGG AGGCAACATG AATTTCTGTC 180

ACCTTGTCAT ATGTTCTCAT TTCCAKGCCT TGNGAGCAAG AGAGTTAGGT ATATCTTCTG 240

TAACTCAGAC AATTTTCTTC CTCTTTGCAG AATGGCCCCT AGGAATCAAG GTAGCTTTTC 300

TTTTGGAAAC TTCATGCTGT TTTTAGTGTT GATAGAAAGG AGGTATCTGC CATTTCTGTC 360

ACCTATTTTA TTTTGTTGTA GCACCCATAA TAGATCAGCT GTCACAGCCA CAAATCTCTG 420

AGGAGACTGG AATCATTCCC AGATAAATCA GAAAGTCAGA ATCACTTTAT GGTTATAGTC 480

CTGGCTTCTT GAGAGCTTGT CTGGAGGTTG TAGCAGGGGA GCACAGCTAG TCATATACCC 540

TWGACTARSG ACCGGTCTWC CTCTATTGGG GATGGTTGTC CTCTTCTACT GAGCTTGCAG 600

CTTTGGGAGG GACGCACATG GAGTGGTGAG GGAGGAAGGG GACACCCGCC TAGCCAGCCA 660

GATCAGCTGA ATCAACCCTG GCAATCAATG GGGTGACAGA TGTTGCAGCC AGATCGCCCT 720

CACATCCAGT CCTACCTTCT TGGTAACAAA ACAATTGGTT TTGCTGGTCT AGAAACTGTA 780

GGGCTAGACA TGTATTATAG GACTGGCTTA GGGAGAGTTA CTTTATATTA GCACTCATGT 840

TTTCACTCAT TTATTTCTTG TAGCTCATTA AAAGAAAAAC CATAATTGAG CATCTACTAT 900

ATGCCATGCA TTGTGCTGAG TATCCATGAT GCTCAGGTGA ACGGGACATG GTCCTGTAAA 960

AAGTGTAAAG TCTGCTGGGA AAGTTAGTGC TCAAAAGTGT AACTAAATAC TTGAGGCAAG 1020

TGCTTTACTA GGGAATAAAC TAAATATCAA GAGAACAAAG ATAAGCAATT CCTTCACGAT 1080

GTTTTACATG GTAAATCCAT ACAATTTTAA AAAAAAAAAA AAA 1123

(2) INFORMATION FOR SEQ ID NO: 57:

(i) SEQUENCE CHARACTERISTICS:
(A) LENGTH: 1239 base pairs
(B) TYPE: nucleic acid
(C) STRANDEDNESS: double
(D) TOPOLOGY: linear

(xi) SEQUENCE DESCRIPTION: SEQ ID NO: 57:

GTATTGATAC GAATTTTGAC TACATTTCTG ATGGTGTGTT TTGCTGGTTT TAACTTAAAA 60

GAAAAGATAT TTATTTCTTT TGCATGGCTT CCAAAGGCCA CAGTTCAGGC TGCAATAGGA 120

TCTGTGGCTT TGGACACAGC AAGGTSACAT GGAGAGAAAC AATTAGAAGA CTATGGAATG 180

GATGTGTTGA CAGTGGCATT TTTGTCCATC CTCATCACAG CCCCAATTGG AAGTCTGCTT 240

ATTGGTTTAC TGGGCCCCAG GCTTCTGCAG AAAGTTGAAC ATCAAAATAA AGATGAAGAA 300

GTTCAAGGAG AGACTTCTGT GCAAGTTTAG AGGTGAAAAG AGAGAGTGCT GAACATAATG 360

TTTAGAAAGC TGCTACTTTT TTCAAGATGC ATATTGAAAT ATGTNAWGTT TAAGCTTAAA 420

ATGTAATAGA ACCAAAAGTG TAGCTGTTTC TTTAAACAGC ATTTTTAGCC CTNGCTCTTT 480

CCATGTGGGT GGTAATGATC TATATCACCA ACCTKAATCT CTCTGCCTTT TTTTTCAAAC 540

ACCCCTTCAT CATCCATCTT AATTTGCATA AGGACATATC TACTTTAATG TACTACCACA 600

GTTTACAGTT AATGTGGGAA AGACCAGCTT CAGTATCCTC TTCAGCTAGG ATTGCCCTAA 660

CTTTTAACTT TCACAGTTTC CTGATTCATA TTTGCCCAGG CTCTGATGCC TTGAATTGGT 720

TTTGGCTCTC TTTTTTGGAT CTGTTTTTGT TGTTAAACAT CATAATGCAG TCTCTCATTA 780

ATTTTTACCA TCATTTACCC TGATAATCTG CCTCTTCTCC ATTTCTCCTT CCCTTACTAC 840

CTTTCTTTGA ATTACTGTAA CTGATTGGTC CCACCAAAAT TTTAAAGTAC ATGAAGTATC 900

TTCATTGGTT CATCCTCTTG CCCCCTCCAG ATGTCAAAAA ACTTTATCCT GCCCCCTAGC 960

TGACCACCCA GGTTCCTTTA TTTCAGTGGC CCATGTGAGT CTACCTTCCC CTAAGGAGTG 1020

CCCTAATCCA GCCCTTTTTT TGTTTCTTAT GACCCATATC TTTAGGCTCT TCCCATTTCT 1080

AGGTGGGAGA TAGGTAAGTT TCAAATCTAT GCCAGTCTTA TGAATATTAC ATTAGGGTAA 1140

TGTGCTATAA TGAAGAAATA AAAAATACAG TGCTTAAAAG AAAATAAAAT TCTATTTCTG 1200

TCTAAAAAAA AAAAAAAAAA CCNNGGGGGG GGCCCCGGT 1239

(2) INFORMATION FOR SEQ ID NO: 58:

(i) SEQUENCE CHARACTERISTICS:
(A) LENGTH: 803 base pairs
(B) TYPE: nucleic acid
(C) STRANDEDNESS: double
(D) TOPOLOGY: linear

(xi) SEQUENCE DESCRIPTION: SEQ ID NO: 58:

GGCAGAGGTC AATCCAGGAC TACAAACACC TGTGCCAAGA CCTGAGCTTC TGCCAGGACC 60

TGTCATCCTC CCTCCATTCG GACAGCTCCT ACCCACCGGA TGCGGGCCTG TYTGACGACG 120

AGGAGCCTCC CGATGCCAGC CTGCCTCCTG ACCCGCCACC CCTTACTGTG CCCCAGACGC 180

ACAATGCCCG TGACCAGTGG CTGCAGGATG CCTTCCACAT CAGCCTCTGA AGGGCTGGGG 240

GGCAGGGGGC ATGCACCCAT GCAAAAGGCT CAGAAACTCC CCCTCCGGCA AGCCCTCAGA 300

CTTCGGAGCC TGCGCCTTCC CCCCTACCGC CTCACCTCAC AGGAGGGCCA GGCATGTATT 360

CCTCAGAGGC GAAACTGCCA AACTCTTTCT CCTGTCTTGG GTTGGCTGGC ACTGGGGCGG 420

GCATCTAGGG TACAGCCTCT GCTCATGGCA CTGGGCCTCC AGTTCTTCCA CATGTGTGCA 480

CCCCCAGCTT GGCCAACCCT CAGCCTTGCG GTGGGGCCCG AAGCATCTTC CCTTCCGCTT 540

GGCGTCTCTG GGATTGGGAT GAGTGCCTGG CTCCCATCTC CTCCTCACCT TTTGTTGCTA 600

TCGGCAGCTG CTGGCTCAGG GGCATCCCAM CTCCGGGCTC TGGGTTCCTC TGCCCTGGAA 660

GGGCTCCAGG ACCCGTCCCA ATAACCACCC ACGGCCAGKA RGCCAAGGCC CCGTGCTGGA 720

TATTTAAATT TAGGGGCCGG TCTCCAGGGC GCGTAGATAA ATAAATACAC TCAGCGTCAA 780

AAAAAAAAAA ARAAAAAAAA ATT 803

(2) INFORMATION FOR SEQ ID NO: 59:

(i) SEQUENCE CHARACTERISTICS:
(A) LENGTH: 995 base pairs
(B) TYPE: nucleic acid
(C) STRANDEDNESS: double
(D) TOPOLOGY: linear

(xi) SEQUENCE DESCRIPTION: SEQ ID NO: 59:

GATTTCNGCA CGAGGNAACA GCTTTATTCT TGGTTATTCC TAATGTCCAC CTAGTCCTCT 60

TTWACTTTYC TTGGTAGGGT TAGGGTGGCA TGGGGAAATG GGACGGTATC ATTTTGTCTT 120

TTTAACTTTT TTTTTTTCCA CCTACAGCAG CTGTTTTTAC CCTGTGGTCA GTCAGGTACT 180

ATATTTAGTT TGCAGTTGCA CTGCTGATCG ACCCTTGATG GCCCCAGTTG GAAGTTGTTT 240

GGGGGGAAGG AAYTAGGAGA GGCCAGGSCC TCCATTTAAA CCATGTCTGT AATGTCTCCT 300

TGGAAAGAAA AAAAGATACT GTTCCAGTCA TGGTTTCCTG GTAGTTGACG TTTAAAATGG 360

GCCTCATTTA AAAATTTCAA TAATTCAGGC TAATTTTTTC CCTTTATATG GTAACTCCAC 420

CAAGTTTGTC TAAATGTATG ATTTTTATCA TGATTAAGTT TTTAYTTCCA CATCATGTGA 480

CAACTGGCCT GGGATGGGAT ATAAGCTCAG AACACAAAGT CATTCACCTC TTAAAAAAAT 540

AATTCTATCT GTGGCGGGTT ATGTTATTTT TGTTCAAAGA GGACACAATA TGATGCAGAA 600

TACACCATTG AAGGATTTTT TGGTTTGGCA AGTTCTTATT TTTTTAAATG GCTGTAAAAC 660

CTAGCAGTGT TTCTGAAATT GCATACCTTA CCTGATGTTC AGAGATCCGA TTTACTTCTT 720

GATTTCCCAG CAAGTGATTT TGAAAACATT TAATCTAATC ATTCCCCCCA CCGTCTGTTC 780

AAATCAAAGG AAGTGGCATC CAGCACTAAT TTTCATGCAT TTATGAAAGG ATGCCTGAGG 840

ACCCTTAAGT ATAATTCAAA ATTTTGTTTA ATGTGTGTTC CTTGATGAAG TTCTTTAGGA 900

GTCGTAGAAC GAACTGATTG CCCACTGATC ATCAAATGCA AGTTATGAAC ATTTAATAAA 960

AATTTAAAAC CAAAAAAAAA AAAAAAAAAA CTCGA 995

(2) INFORMATION FOR SEQ ID NO: 60:

(i) SEQUENCE CHARACTERISTICS:
(A) LENGTH: 966 base pairs
(B) TYPE: nucleic acid
(C) STRANDEDNESS: double
(D) TOPOLOGY: linear

(xi) SEQUENCE DESCRIPTION: SEQ ID NO: 60:

GACAGTACGG TCCGAATTCC CGGGTCGACC CACGCGTCCG GGAGAGGACA TGCAGTGGGC 60

ACAGAAAGTT CAATGGAACA GATGCCACTG TGGGCACCAA GACTGTAATG ACTCTGTGTG 120

GTAGGTAGTT TTAAAGGACT GCATGCCTTG GAAATGATTC TTCACTTGGA GAACATACTT 180

GCCTCTAGAT ATGTTTGTCA CTCTAAGCAT CCTGAATATA ACAATAGAGA AAGATAAGTC 240

AACCAACAGA TTTAGGGATG TGTTTCTTCA GCACATTTTG GTCATTTTGA TGCCAAGTTT 300

GACATACTGT TTAATTGGGC AGCACCTTTG CTCCTTTACC AGGTATGTAT CACTTTGTTA 360

CTCCAGGTGC CATTCTTGGT GATGACAGAA TGTTTATCAC TATCGTTGTT AGCAAGAGGA 420

AGCTTTCAAT ATAGGAACTT AACATCTTCC CATGAGTATA AATGAATTTA AGACATTTGA 480

ATCAAAACTT CAGTAGAGGG AGGTTTTAGA ATTCATAAAA CTGGTTTAAG GAAATTCTTT 540

TTACTTTTCC CAAGGTTAAT CTTTTTAAAT ATCTCTAGAC ATCAAATACT TTCTGTATGT 600

ATTAGCTGTG TCTGTCTATG ATGCAAGTAA CTCTCCTCCT ATTTGGGGGA TAGTTCAGAG 660

AGGTAGGAGC ATTATCTCCC ATTTTTCTGG TGACTTCTTG GAGTATAGAA TTCACCATTT 720

TATCCGTAAG TCTTCAAAGG ATTATGGTGG ACTAGAACTT ACATAGTGCA AAATAGTCTT 780

CTATTTTTAA TAGGAACTTA GAAAAAACTT AGAATTATAT ATAGAGTTGT TTCCTTTAGA 840

AACCAGAGCT ATTTATTTGT ATTTAAAGCA CTGTTTATTA TTTGTACTGA TTCTTATCCC 900

TCTGTGTGAA TAAATGTAAG ACGGTGAAAA AAAAAAAAAA AAAAAAAAAA AAAAAAAAAA 960

ACTCGA 966

(2) INFORMATION FOR SEQ ID NO: 61:

(i) SEQUENCE CHARACTERISTICS:
(A) LENGTH: 262 base pairs
(B) TYPE: nucleic acid
(C) STRANDEDNESS: double
(D) TOPOLOGY: linear

(xi) SEQUENCE DESCRIPTION: SEQ ID NO: 61:

TTGCAGGTAT ACATCCAGAT GCACAGAATG TCCATTTGTC CCTTATTGGT GATGCTAATT 60

TTGATCACTT GGGTAAGATG TCCAGTTTCT CCAGTGTATC GTTATTGTTT TTCCTTTTGC 120

AATTAGTGGG TAATTTGTGA GGAGAAACTT TGAGACCTTG TTTGACAATT CTGTTCCTCC 180

ATCAAATCTA CCCCTCCCTA GGTTTAGCAT CCTTTGACAA TCCTTGTTCT GAATAAATTT 240

TTAACTAAGA TGTTTNCCCA AN 262

(2) INFORMATION FOR SEQ ID NO: 62:

(i) SEQUENCE CHARACTERISTICS:
(A) LENGTH: 753 base pairs
(B) TYPE: nucleic acid
(C) STRANDEDNESS: double
(D) TOPOLOGY: linear

(xi) SEQUENCE DESCRIPTION: SEQ ID NO: 62:

GGCACAGGTT CTTTTGCCAG TCATGACAGA ACCATGCAAG ATATTGTTTA CAAATTGGTA 60

CCAGGCCTCC AAGAAGGTGA GTGTCTGACT GTCTTGCTGA TCCCTGAGGT CCCAGCCTGG 120

CCTCTGCAGC CCCTGCTCTC CTGGAAGTTT GGTTCTCGGA TGGGAGGCCC CTTTCCTTTT 180

GGCCGAATCA CCGTCTTCTC ATCCCTGCTC TCAGCCCAAC TTCATCTCCT TGGCTGGTCT 240

CTTCTTTCGT CTAAGATGCG TAKACATCTT TTTACCCCTT ATGTGTATTC ATTCAGCAAG 300

TATGGATCGC ATGTTTAGCA CATGGGAMCC CCAGGGNTCA ACGCAGCTCC TGCCCCTCCC 360

AGGACCCTGC CTTSTTCCTG GGCCCCACCT CCTGTCCCAG GCCTGCCTCC CCTCATCCCA 420

CAGCGCCAGC TTCCCCACAA CAGAGGAGCA GCACGTTGGC ATAGCGGGTA GCTGGTGTTT 480

CTAGAAAAAC TTCACCATAA AGTCAAATTT CATTTAGAAT TAAAAGAAAT ACCAAGTAGT 540

ACAAATACCC TGAAAGTGGA AATCGGTTGC TTGGGGATCG CTCAGCTGAA AGCTCCCCCA 600

GCTCCCGACA CTCTCACGGT GGTTGGCCCT CCGCTGGCGA ACCGGCAANG AAGCCCAAGG 660

AAGGGGGCCA GGTTCAGCGC CCAGGTTGGG CTTGTCCCTG GTTATTCCTG CTCCATCCAN 720

AACCTTTCCA AAAGGCAGAA TAGAAAAACN TGA 753

(2) INFORMATION FOR SEQ ID NO: 63:

(i) SEQUENCE CHARACTERISTICS:
(A) LENGTH: 739 base pairs
(B) TYPE: nucleic acid
(C) STRANDEDNESS: double
(D) TOPOLOGY: linear

(xi) SEQUENCE DESCRIPTION: SEQ ID NO: 63:

ACAATACATG CATCATATCT TTTGACTTTG AAGGATATCT CATGTCAAAG GAATCAAGTT 60

ATGATTTATA GAGGATTCAG CTGGAATACC TTGTGGGTGC TGGCTGAGGG TGGCAAAACG 120

CCTACCGAGA CATGAAGGTT TTAGCCACTA GTTTTGTCCT TGGGAGCCTG GGGTTGGCCT 180

TCTACCTGCC TTTGGTGGTG ACTACACCTA AAACACTGGC CATCCCTGAN GAAGCTGCAA 240

GAAGCTGTGG GGAAAGTTAT CATCAATGCC ACAACCTGTA CTGTCACCTG TGGCCTTGGC 300

TATAAGGAGG AGACCGTCTG TGAGGTGGGC CCTGATGGAG TGAGAAGGAA ATGTCAGACT 360

CGGCGCTTAG AATGTCTGAC CAACTGGATC TGTGGGATGC TCCATTTCAC CATTCTCATT 420

GGCAAGGAAT TTGAGCTTAG CTGTCTGAGT TCAGACATCT TGGAGTTTGG ACAGGAAGCT 480

TTCCGGTTCA CCTGKAKACT TGCTCGAGGT GTCATCTCCA CTGACGATGA GGTCTTCAAA 540

CCCTTTCAAG CCAACTCCCA CTTTGTGAAG TTTAAATATG CTCAGGAGTA TGACTCTGGG 600

ACATATCGCT GTGATGTGCA GCTGGTAAAA AACTTGAGAC TCGTCAAGAG GCTCTATTTT 660

GGGTTGAGGG TCCTTCCTCC TAACTTGGTG AATCTGAATT TCCATCAGTC ACTTACTGAG 720

GATCAGGACT AATAGAGAA 739

(2) INFORMATION FOR SEQ ID NO: 64:

(i) SEQUENCE CHARACTERISTICS:

(A) LENGTH: 476 base pairs
(B) TYPE: nucleic acid
(C) STRANDEDNESS: double
(D) TOPOLOGY: linear

(xi) SEQUENCE DESCRIPTION: SEQ ID NO: 64:

GAATTCGGCA CGAGAGGACA TGGATTATGG GTACTACTCA GCAGGCCAGT TTTTACTCCA 60

CCTCTTTCTA GCTGACTTGA CACAAGCAAC AACCCAACAG AAAACCAATA CTTCTGAGAA 120

TGGCTGCAAG TTTGTTTGTG CTGTCTTTTG AGGTAAGAAA TCAAGGCTGA GCTCTTCTTT 180

CTCCTAATTC TCAGGAAGGA GGAAGGCAGA TGTGAGAACA CTGATTGGGT CTGAGTGTAC 240

TGGGCAGCAT CACTGTTAAA AGGTCAGCAC ACAGATGCAA GCTCACTTGT CTGCTTNCTT 300

TCATGTGACT GAAGTGGTTA AGAARGTTGT NCAACTCCCC CCTGCACCCC CCTCACCACC 360

GCAGTAAGGG AGAGACAGGG CCAAACCTGC AGCTTCGGTA GAAGAGGCCA AGGCAGGTGT 420

CCAAGGCCAG ATCAGCAGTC AGCCAGGGCA AATGGGCTCA CTCTGGTTAC ATGACC 476

(2) INFORMATION FOR SEQ ID NO: 65:

(i) SEQUENCE CHARACTERISTICS:
(A) LENGTH: 754 base pairs
(B) TYPE: nucleic acid
(C) STRANDEDNESS: double
(D) TOPOLOGY: linear

(xi) SEQUENCE DESCRIPTION: SEQ ID NO: 65:

AATTCGGCAC GAGACCAATT GTACTTTTAT TATATCAGGC TGATTCACTG TTTCTAATGC 60

AATGAACTTG ACACAGATTT TAAATTTTTY CTCAATCTGT CCCATTGTGT AGACAAATTA 120

ATTCAAAGTT CTTTTTCTTC CTTCTCTTTT TCATCTAAGC CTGTGCTTAT GAGTAGAAAA 180

AGAGAAGAGG CTACCTTGAA ATGCCTCGGG CCCAAACTCA GAAGGCTCTG CACTCAACTG 240

AGCCTCCCTT CCTACTAAGA ATGGAATAGT GTTGCTTATA GGGGTGTTGG TCCAAGTATC 300

AGCTGTGGAT GATTAATTCC CAGGGCTGCT ATCACCTAAG GTAACTTCAG TAATCTTATG 360

TGTTTGGAAA GGAGGATGAG GATTATTTTT CAAATACATA ATTTTGTTTT ATTTTGAAAC 420

AATCTCACAC CTACAGAAAA GTTGCAATTA TAATACAAAG AGCTTCCCCC TCGCCTGAAC 480

TGTTTGATAG TAAGTTTGCC AAACTGATAT ACCCACGATC CCCAAATGCT TCAGTGTTAT 540

TTCCTCCCAG CCAAGGACAT TCTCCCTGCA TAACCCACAA TACAACCCAT AAAAGTCAGG 600

AAAATTTAAC ACCCAGTTCC ATTTTTGAAC CCATCCTGAA ATTCCAGGTG TTCATTCCAT 660

GTTTTTGGCC AGTTGGTNCC TTTGGTATGT TCCCTCCCNT AGCCCAAAAA AAAAAAAAAA 720

AAACNCCAAG GGGGGGGGCC CCGGTCCCCA ATCC 754

(2) INFORMATION FOR SEQ ID NO: 66:

(i) SEQUENCE CHARACTERISTICS:
(A) LENGTH: 1890 base pairs
(B) TYPE: nucleic acid
(C) STRANDEDNESS: double
(D) TOPOLOGY: linear

(xi) SEQUENCE DESCRIPTION: SEQ ID NO: 66:

GGCAGAGRAA AAACAAAATG GGTAATGCAT TCGAGGTGAC AGGGTTAATG TTGGCATTAC 60

TTTGTTATGT TGTTGATGGG CAGAAACCCA AGGKGGGGTT TTKTTGAGCA TAAACACAAG 120

AAGCAATTAT TTGTGGCACT AGACTTAACC CAAAGGACAG ACCCCTACAT GTATATAGTA 180

GAGAAATCCT GTCTTTTAGC ACTATCTCAC AGGGGAAGCT GAGGAATCAC ATTATCTTTA 240

ATATAAATAA ATGAAATGCN AGCACTGTAT AATTTATATC CTTAAGCAAC TGGATTCAMC 300

GTACCACTAA TGGCCTGGTC ATGTTTTAAA CATTACCCCA AAACAGCCTA ACTGTTCTGT 360

GACTCAGTGT CTCTGTGGAA TCCTATTTAG TAGCACCATG GTCTCTAAAT GTTTTGATTA 420

CACATCAGTA TTAGGAAAAC ATGTTTGAAG CATTGTCTAA GTCTGTTTGT GCTGATGTAA 480

CAGAATACCA TAGACTGGGK AGTTTATAAA GAGAGAAATT ATTGGCTTAC AGTTGTGGAG 540

GCTGGAAAGT CTAGTATCAG CGTACTGGGA TTTGGCAAGG GCCTTCTTGG TGCATGATAG 600

TATGGTGGAA GGTATCACAC GGCAGGCAGA AAGGCAGAGA GAGAACAAAA GGGGGCGAAC 660

CCACTCCCTT GATGAGAACC TAAATACCTC TTAAAAGTCC TAACTCTCAA TGCTGTTTAC 720

AATGGCAACC AAATTTAAAC AAGAGTTTTG TAGGGAACAA ACACTCAATC AAAACCATAG 780

CAAGTATGTA CCATGACTGT ATGTGTATTT ATAAAATACA TTCATATATT TCTACAGCAA 840

TATATATGAG GTACATTTAA GCATGTAAAA ATAGGAATTT TTAAAAATAG GACAGTTGTA 900

ATAATTTCTT TGTACATTCC ACTTTGGAGA CTGTTTTTAT ATGGRGCTTG TTTTATCACC 960

AAAAGGCATT TTAATTTTGC ACACTTTAGA WTTCTTACAA TGTGTAATTG ACTGCTAGTT 1020

GCTGAACAAA GGACAGATAA AGTGTTTCCT GCACCTGAGC AGCCTAAAGG TGAGTGTAAT 1080

ACAGATGCAC AAGTGACTGG TTGATAATGG AATGAGACCC CTTATAAGAA AGACATACAG 1140

AGCACGGCAG AGGAGCAAGA ACMACACAGA GGCAATGACA TTTGAGCTAG GCCTCTTATA 1200

TCTGTAGATG AACATTTGAT GGTAGGTAGT AGGGAAGATG GAACTAAGAA TATTTGAGCT 1260

ACTTAATATA TGCCAGGCAG CATGCTGAGT GCTTGTGTTC ATTTAATTCT CAAGACAGCC 1320

ATAAGCGGCA ATACAGGTAT TGGGCCTATT ATTCTAAATC CCATTTTATA AGAGAGTTAG 1380

GATTAGATTC AGTTCCATCT TTCTACAAAA CCTGGCACTG TCATTCCAGG CAAAGGGAGT 1440

ACAATCCATT TTTCTCTTAA GAGGTTGATT TTGCCAATGA GACAGAATGA ATCTCTACAG 1500

CTTGTTAAGT TTCWACCCGT CTTTGGGTGA CTGAAAAATT CAAATGTAAA GATGTGGCAA 1560

AATTGGTTCT CTAAGGATTT TAAGTACAGC CAAATGATAT GTCACAAGTT TTTTCCTAAA 1620

TATCCAACCA TTTAGTCTTT CATAAGCTTT TAATTCCACT AGCCTCACTT TCTGAGATTG 1680

TTGATGTTTT CTTGTTCTAA CCTGAAATTT TCTTTGTTTG ATGTTAACAG GAGTATAATG 1740

AAGGAGTAAC CATTTTTATT TTATGATAGT CTATCAATAG ACTTTTTTTA ACCTTCTTTA 1800

AGCTAGGTGT GTTTGTCCTT TATTAAAGTC AGTTTGACCC AGCCTGTACA ACATTGCAAG 1860

ACCTTAACTT TAATAAAAAA AAAAAAAAAA 1890

(2) INFORMATION FOR SEQ ID NO: 67:

(i) SEQUENCE CHARACTERISTICS:
(A) LENGTH: 1614 base pairs
(B) TYPE: nucleic acid
(C) STRANDEDNESS: double
(D) TOPOLOGY: linear

(xi) SEQUENCE DESCRIPTION: SEQ ID NO: 67:

AAATAAGACN TCTTTGAGCA GCGATTGCTG GATCATTGAT CTGTTTGAGG AATGTCTGAC 60

CTGGGCCTRA RAGCTGGAGA AGGTGCAGAT TCAAAGTRAG CGGCTCCTRA GGAGAGCCCC 120

AAGSTGCTCG CCTTCTCCGT GGCTTCCGCA GCTACCGTCT GCACGGTGAG AGGGCACGGG 180

CACACGGTTC GGGCTGGCGT GCAGTCTCCC AGCCAGCCAC GCTCTGCTCA GGCCTGGAAG 240

TGAAAGCCGC CTCCTTCCCG TTATGCCCCC CATACAGGAG CCTCGGTTTT TCAGCAAAAC 300

GCGGCCAGTC CCCTTCTCCA CTGCTGCCTC CCAGCAGAGG GCCCCAGGAT CTCCAAGGTC 360

CCAGCTATGG CTTTGGACAA CGTGGCTTCG GCCCCTGGGG TTGCAGAGCT TGCATTGGGT 420

TTACCTCGGT CTCATTCATT CATGGAGCCA AGGGTGGGGT TTCACCTGCG AACATCAGAC 480

TGACTTGCTG GCGTCAAGAG CAGTTGACTC ACTGATGAAG GCCCTGGTGA GGAGAAAGCA 540

CTCTGTTCTT CGCCTACTCT GTAATCGTTT TGTCATAATG AGCCATGAAA AAAGTAATGA 600

ACTTGTGCTG TTAATCGTCA CTGTAATGAG AAGTCTTACG TACAACATAG CTGTGGTGGC 660

TGCGTGGTTT AATGGCTGCA TTAGATAGGA TCCTCACATC CCATTCAGAA CCAAAACTGA 720

TACAGTGAAA CAATTAAGGT GAGCAAATAG TTTTAACTTT TCTTTTTTTT TTTAAGTTTC 780

ATTCTTCCTA GAATATTTTT CTAACAATTT TTATTTCAGC TTTAAAGATG GGTCATATAG 840

CCAAACGGGC CATATAATCC AACATTGTTG AGATGTCTTA GGACATCTAA GGCAAAACTG 900

GCACATTTGT TCTGCAGACT ATTGCAGGAA TGTTTTTTCC TAGCATTTCT ATATTATCTG 960

TCCATTCTGA GGAACCAGTG AATGTCCTAT AAATGCACCT CCTGTCAAAA CCATGCCTGA 1020

GAGGTCCCGG CTGGGAGTGA CAGGGTGCTT NCTTAGATTC TATTGGTCCT TCTCTCATTC 1080

TCCGAACTTA CTCCTTTTTA TGGGTAAGTC AACTAGGTYY ACAGTCCCTT ATTTTTAATG 1140

CCTAAGTTTT GACAGCAGGN AAGAAAACAA TTTTTTAAAA ATTCTCATTA CATAGACGCA 1200

CAAGAATATG TCACATAAAG AAAATGTGTT TAGAATACTG GTTTTCTATT TACGCATGAT 1260

ATTTTCCTAA GTAAAATTGC CAAGTGGACT TGGAAGTCCA GAAAGGAAAA TAATTTAAAT 1320

TAATGCTGGT GATCTTAACA ATATTTTGTA AAATGATGCT TCCCCCTTCT CCATGGTGTA 1380

GTCAATTTTG TACAATTAGG TATCTGACTT TACAAGTTTG TTATCCTTTC TAATTTTTAC 1440

TGAACTGAAA GCACAAAGAA GACTACACAG AAAATCTGGA AACAGTTGCA GGTGTTGGGA 1500

GGAAGATGAA ATCGAGCTGT CTTTTAACTT TCGTATGTGT TTTATCAGAA TTTGCTGGAC 1560

TATGCTAGCA AGGACTTTGT TTACNATCAA ATTGTACTAG TGTCTGCAGG GTTT 1614

(2) INFORMATION FOR SEQ ID NO: 68:

(i) SEQUENCE CHARACTERISTICS:
(A) LENGTH: 596 base pairs
(B) TYPE: nucleic acid
(C) STRANDEDNESS: double
(D) TOPOLOGY: linear

(xi) SEQUENCE DESCRIPTION: SEQ ID NO: 68:

CTTTTCACCC TTAGAGACAG GGTTTCACTT TTTTGCCTTC TTAATGGAGA TATTCAGTTT 60

TCTTTTTTTC ATTTAAACAA AGAAAAAAAA TGTATCTACT CTACCTTCCC TCTGCTCTCC 120

TCCCTCCCTA TCCTACTTGC CCATATGAGC ACGGCTCCCC ATGGCCACAT ACTCCTGCAA 180

AGCTTTTATG CTGCTTCGCT TTTCTCTAAA CAGATCTGAT ATTGCTGCTC CTGTGGTTTT 240

CTCAAAATTA ACTTTGCCGT GGTTTTTAAA AAGGAATCAA AATGCATTGT TGCATTAAGC 300

TTTTTCAATA AAGGAAAATT ACGGAAGGAA AATAGGCAAC ACCAGCAAAT TATATGTGGA 360

CAGGTTCTAA ACTCTATATA TACATATATA TATATATATC TATATATCTA TATACGTAAT 420

CATCTAGTTC TGTCATCTTA CTGAAAGGAA TAACACTTCT AAAGATCACC ATTTCTGAGA 480

AGTTCTTGGA AATCTTTATG TCTAAGTGAT TGTATTAGAT CAGCAATAAT GACTATGTAA 540

TCTCAAAAAA CAAATAAAAT ATTCTTAACA TGGAAAAAAA AAAAAAAAAA ACTCGA 596

(2) INFORMATION FOR SEQ ID NO: 69:

(i) SEQUENCE CHARACTERISTICS:
(A) LENGTH: 1524 base pairs
(B) TYPE: nucleic acid
(C) STRANDEDNESS: double
(D) TOPOLOGY: linear

(xi) SEQUENCE DESCRIPTION: SEQ ID NO: 69:

ATCCGGAATT CCCGGGTGTG TTCGACCCGT CCGGGACTTT GCACAGCACC TTCCAGCCCA 60

ACATTTCCCA GGGAAAACTT CAGATGTGGG TGGATGTTTT CCCCAAGAGT TTGGGGCCAC 120

CAGGCCCTCC TTTCAACATC ACACCCCGGA AAGCCAAGAA ATACTACCTG CGTGTGATCA 180

TCTGGAACAC CAAGGACGTT ATCTTGGACG AGAAAAGCAT CACAGGAGAG GAAATGAGTG 240

ACATCTACGT CAAAGGCTGG ATTCCTGGCA ATGAAGAAAA CAAACAGAAA ACAGATGTCC 300

ATTACAGATC TTTGGATGGT GAAGGGAATT TTAACTGGCG ATTTGTTTTC CCGTTTGACT 360

ACCTTCCAGC CGAACAACTC TGTATCGTTG CGAAAAAAGA GCATTTCTGG AGTATTGACC 420

AAACGGAATT TCGAATCCCA CCCAGGCTGA TCATTCAGAT ATGGGACAAT GACAAGTTTT 480

CTCTGGATGA CTACTTGGGT TTCCTAGAAC TTGACTTGCG TCACACGATC ATTCCTGCAA 540

AATCACCAGA GAAATGCAGG TTGGACATGA TTCCGGACCT CAAAGCCATG AACCCCCTTA 600

AAGCCAAGAC AGCCTCCCTC TTTGAGCAGA AGTCCATGAA AGGATGGTGG CCATGCTACG 660

CAGAGAAAGA TGGCGCCCGC GTAATGGCTG GGAAAGTGGA GATGACATTG GAAATCCTCA 720

ACGAGAAGGA GGCCGACGAG AGGCCAGCCG GGAAGGGGCG GGACGAACCC AACATGAACC 780

CCAAGCTGGA CTTACCAAAT CGACCAGAAA CCTCCTTCCT CTGGTTCACC AACCCATGCA 840

AGACCATGAA GTTCATCGTG TGGCGCCGCT TTAAGTGGGT CATCATCGGC TTGCTGTTCC 900

TGCTTATCCT GCTGCTCTTC GTGGCCGTGC TCCTCTACTC TTTGCCGAAC TATTTGTCAA 960

TGAAGATTGT AAAGCCAAAT GTGTAACAAA GGCAAAGGCT TCATTTCAAG AGTCATCCAG 1020

CAATGAGAGA ATCCTGCCTC TGTAGACCAA CATCCAGTGT GATTTTGTGT CTGAGACCAC 1080

ACCCCAGTAG CAGGTTACGC CATGTCACCG AGCCCCATTG ATTCCCAGAG GGTCTTAGTC 1140

CTGGAAAGTC AGGCCAACAA GCAACGTTTG CATCATGTTA TCTCTTAAGT ATTAAAAGTT 1200

TTATTTTCTA AAGTTTAAAT CATGTTTTTC AAAATATTTT TCAAGGTGGC TGGTTCCATT 1260

TAAAAATCAT CTTTTTATAT GTGTCTTCGG TTCTAGACTT CAGCTTTTGG AAATTGCTAA 1320

ATAGAATTCA AAAATCTCTG CATCCTGAGG TGATATACTT CATATTTGTA ATCAACTGAA 1380

AGAGCTGTGC ATTATAAAAT CAGTTAGAAT AGTTAGAACA ATTCTTATTT ATGCCCACAA 1440

CCATTGCTAT ATTTTGTATG GATGTCATAA AAGTCTATTT AACCTCTGTA ATGAAACTAA 1500

ATAAAAATGT TTCACCTTTA AAAN 1524

(2) INFORMATION FOR SEQ ID NO: 70:

(i) SEQUENCE CHARACTERISTICS:
(A) LENGTH: 819 base pairs
(B) TYPE: nucleic acid
(C) STRANDEDNESS: double
(D) TOPOLOGY: linear

(xi) SEQUENCE DESCRIPTION: SEQ ID NO: 70:

GGCACGAGGG AGAGGGACGG GGAGGGGGCG AGGGGCGGAG GCCGAGGGGG CAGGGGNTGG 60

GCGGCGGCCA GTGTTTACAG ATGAGCTTTA ACTGCCGCCT CAGGCGTGGA GACGGAGACC 120

CCGCAGCCCG GCGGCGCCTC AGCCCTTCAA CGACAGTATT GAGTGGTCAG GTTACAATAA 180

ACCGGAGAGA AAAGGTCCGC TTGCACTTTT TTTAGTTTTC TTATTTTTAG ACACCCCTCC 240

CCTCCAGGGT GATCTTTAAA AAAGCAAAAC AAAAAACACG ACTTTTCCAG CGCTCAGCGT 300

TTTTTCCTTT CGTCCGAAGC CGTTTTCTGA TTTGACTTTT CTCGCCGGCC GGTCTCAGGC 360

CCACAGACGT TCCAGAGGAG GAGGGTGACA TTTTTACTCC CTTTTTGGGG CTAACCATTT 420

ATGCTTTTGT ACATCAACCG TGCGCGGCCG GAGGGGGCAG GGGGGCGGGG GCGAGGGGCG 480

TTCCAATCAA ATTTCTAATT TCTGTTAATT ATTAATCCCC KTTTTACTGC GGTTTCTGTT 540

GTCATTTTTA AAATTTTTTT AATTTTTTTT TTTTTTTTAC TTTTACTTTT TACCTCTTGT 600

GTATATGTAG GGAATTTATA GGGAAATATG TACTTTATGG AATAAATTTT AAGAACTAAA 660

ATATATTTTA TTTTAAATAA AGTAATGGAC CTTTAATCTT ACACAGCTAA ATTACTGATT 720

ATATATTTSC TGAGCTGATT TAAGGGTTAA AAAAATTGTA TCAAGAGTTT TATTTTTTGA 780

CTTCAAAGCC TTCTTAATAA AGCCTCTTTT CTACATGTG 819

(2) INFORMATION FOR SEQ ID NO: 71:

(i) SEQUENCE CHARACTERISTICS:
(A) LENGTH: 1442 base pairs
(B) TYPE: nucleic acid
(C) STRANDEDNESS: double
(D) TOPOLOGY: linear

(xi) SEQUENCE DESCRIPTION: SEQ ID NO: 71:

AATTGCTTGG CATGAGTTTA CTTTAATGGC TGTTTCTGAG TTTGATCCCT CTCCGGAACC 60

AACCSCTCTG ATGTGTCCTG TTCCAGCAGG AAGAGACAGA CCTGGAGGTT CTGTACTTGT 120

GATTTCTGGT TGTGGATCCT GAGAACAAGA AGTACTGGGA TCCTAAAGTT CTGACATTTG 180

CAAAGCAGAT TAATGACCTA CCACATTCCA GATCATTTGG TGAYYWTGTG TTGTGCGTGT 240

GGGTGTGTGT GTGTGTGTGC CAAATTCAAG GTGGTCCCAG CCTTTCTAGT CTTCTCTAAC 300

CTTTCTTCTC ARAARTCGCA CCTGTTCTGT CTTTCTAGGA TATAATTTTT TTTCTATTAG 360

CCTGGGTAAC ACCCCAACCA ATAAAGTTTG CAATATCCAA GCCTCCTAAT TTCTCTACTT 420

ATTAGCTTAT ATTAAGCTTC AGCATGAGCA AGCCTAAAAA CTCGCCATTA TCTGGAAAAG 480

TTCTATTTCA CAGGCTTTAA TCTCTCCTAG AGTAGTTAGC ACTCTTTTGT GGCTTTGTGT 540

TCCTGTACTA GCTTGAATTC CACAGTCTGA CGTTAATAAT TAGCTCCTTA ACACGTCCAT 600

CCTCTCTTGA TGTCCTGCTC TCTATTTTTC CTTCTTTCTT CCAAGTTGGG ATAAATTCAG 660

CTTCTTATTT TCCTGCTCCA GAMCTTGGTT GTGGAGAAAG ATAGAAAAAG TTCCATACAG 720

GGGACTCTGT GATCCTGCTA ACATCATTAT TTACCTAAGC TCTTTAGACT CCAGTGAAAG 780

CTTCTGATTT AATGTCATGT CCCTACTTTA TGCCACATGT CCCATACCAT TTTCTTTGTT 840

TTATGCAATT TATTTCCACT ATCTGATCCC ATTCCACCCA CATGACTTTG AGTGGAAAAC 900

TTCATCTCTT CATTGCTGAG TAAACAAACT TCAGGATGAA CAAGCCCTGT CCACTATTTT 960

CCCTTTTACT KTAAARKYCT GGAATTTWWA TGATCTACGT TTTTTTCCTC TGTTTTTATT 1020

CTTCACTCCA TATCAACTTA CTTGGGGATC TACACCTTCA TTCATYCTTT TCATTCTGTC 1080

GGCACCTGGC TATGGAGTTT ACATTTCTCA TCATATTTAC TCCTCATAAT AATCCTGTGA 1140

GGTATATACC ACTCTGAGTC TTGTATAAGA GAAAAAGAAA CTGAGATAGG GATAACTCAA 1200

AGGGATAATT CATTTGCTGG AGCTACCAAC TAGCTACTAA CCATGCTAGA ATGGACAGAG 1260

ATGACATTCA TGCCAAAGAC CATGTTGACT TGCTATCTCT ACATTTGCTC TAAGTTTAGA 1320

AAAAAAAAAT CCCTTCAATT TATCCTCCAA CAGTCTTCTT AGAACCTTAC CATGGATGCC 1380

TTGTWTAACA CATTTCACCT TTCTGGTAAA AAAAAAAAAA AAAAAAAAAA AAAAAAACTC 1440

GA 1442

(2) INFORMATION FOR SEQ ID NO: 72:

(i) SEQUENCE CHARACTERISTICS:
(A) LENGTH: 1223 base pairs
(B) TYPE: nucleic acid
(C) STRANDEDNESS: double
(D) TOPOLOGY: linear

(xi) SEQUENCE DESCRIPTION: SEQ ID NO: 72:

AACCTGAGGA GGCTGTCATG ATAGGAGATG ATTGCAGGGA TGATGTTGGT GGGGCTCAAG 60

ATGTCGGCAT GCTGGGCATC TTAGTAAAGA CTGGGAAATA TCGAGCATCA GATGAAGAAA 120

AAATTAATCC ACCTCCTTAC TTAACTTGTG AGAGTTTCCC TCATGCTGTG GACCACATTC 180

TGCAGCACCT ATTGTGAAGC AATGTGTGCA TCTGAAGCAA CTTGAAATGC AGCTTCTTAT 240

TGTCTGGAAT GAATCCCTTA CCAACTCAGT GCCAGCATCG GTAGACACCA GTCAGTGCTG 300

ATCGCTTTTT AACCCTCTTT TGTTGTGCAT TAATTAGAAA GAAAGGTATT GAATTGCGGC 360

TAGCCAGTAA GCCTTGCTAA TCTCTTTTAT TTTGTAACTG AAGATGAGAC CCAAAGAAAG 420

GGAAAGCTGA GATTTTGTGC CATTCCTTTT AAAATATTCA TCAGGTTAGG TGGGGCTGTG 480

GGGGAAAAGC TACTACAGGG AAGAGTGTTC TCTGCTGTCT CTTCACTGGA AAACAGGGAG 540

GGGGGATTTC AGACTGTGAA GAAAGTTGAA TGGTGGTTTT TAAATTATAA AGTAATGTAT 600

TAAAAGGTGC ATTAGGCTGT AGTTCTAATA TTGAGTTCAA CTGTGAAATC CATCAGATGT 660

GCCAAATGGA GAAGACAGAA AGCAACAAAG TGAATTGTTC TTTAGCCCAA GTGGTACAGT 720

GAATTTGCTT TAACAGATGT TGAAAACTAA ATTTTCTACT GTATTCCCAG CACGGGTGAC 780

TTCTTTTTCT CTTCATTAGC CAGAGATGAC TAATTTAAAT TTAGAACCAG ATTTTAATTT 840

AAATTAATAT TTCCATTAAT AACCTATTCA TTGCAGATAC CTATTATACT GTGTAACAGT 900

TGTTTTGGAA ATTTTATGTA AAATTAAAAC TATCAGTATT TTACAGATGT TTTAATTAGA 960

CATGTTATTA ACAGGAACAG TGCAGAAACT AGAATCAAGC CTTATAATAT CTTATAGACC 1020

ATGCATTTTG AAGTTAGTGT CCACTARGGT CCTATTAACT GTACATTGCA AGATTCATTA 1080

TTTTGCCTCT GACACTAWGG GAAAATTTTT AGAAGCCAAT GGGACAGATT CCAGCCTTTA 1140

AGCACTGGGT ACTACAGCCG TAAAAGGAAA TCCCGCCTGG TAGCCAGGGA TATNCCTCCC 1200

CAGGTTAAAN CCCCCCAAAT NAA 1223

(2) INFORMATION FOR SEQ ID NO: 73:

(i) SEQUENCE CHARACTERISTICS:
(A) LENGTH: 1814 base pairs
(B) TYPE: nucleic acid
(C) STRANDEDNESS: double
(D) TOPOLOGY: linear

(xi) SEQUENCE DESCRIPTION: SEQ ID NO: 73:

CAAGCTTTGT ACTTAGATCT TTTACTTAGA TCTGCTTTTT GTCTTATTCT TTTTAGTGGA 60

TGTTTCCAAG GATTGTCTTC AGTCATGGCC TTGGGATTAA AGTGCTTCCG CATGGTCCAC 120

CCTACCTTTC GCAATTATCT TGCAGCCTCT ATCAGACCCG TTTCAGAAGT TACACTGAAG 180

ACAGTGCATG AAAGACAACA TGGCCATAGG CAATACATGG CCTATTCAGC TGTACCAGTC 240

CGCCATTTTG CTACCAAGAA AGCCAAAGCC AAAGGGAAAG GACAGTCCCA AACCAGAGTG 300

AATATTAATG CTGCCTTGGT TGAGGATATA ATCAACTTGG AAGAGGTGAA TGAAGAAATG 360

AAGTCTGTGA TAGAAGCTCT CAAGGATAAT TTCAATAAGA CTCTCAATAT AAGGACCTCA 420

CCAGGATCCC TTGACAAGAT TGCTGTGGTA ACTGCTGACG GGAAGCTTGC TTTAAACCAG 480

ATTAGCCAGA TCTCCATGAA GTCGCCACAG CTGATTTTGG TGAATATGGC CAGCTTCCCA 540

GAGTGTACAG CTGCAGCTAT CAAGGCTATA AGAGAAAGTG GAATGAATCT GAACCCAGAA 600

GTGGAAGGGA CGCTAATTCG GGTACCCATT CCCCAAGTAA CCAGAGAGCA CAGAGAAATG 660

CTGGTGAAAC TGGCCAAACA GAACACCAAC AAGGCCAAAG ACTCTTTACG GAAGGTTCGC 720

ACCAACTCAA TGAACAAGCT GAAGAAATCC AAGGATACAG TCTCAGAGGA CACCATTAGG 780

CTAATAGAGA AACAGATCAG CCAAATGGCC GATGACACAG TGGCAGAACT GGACAGGCAT 840

CTGGCAGTGA AGACCAAAGA ACTCCTTGGA TGAAAGTCCA CTGGGGCCAG CAATACTCCA 900

GAGCCCAGTT TCTGCTGGAT CCCATGGGTG GCACATTGGG ACTTCTCTCC CTCCCCCATC 960

TACACAGAAG ACTGTCACCA TGCTGACAGA AGCCTGTCCT TGTAAGGCCC AGCCTTCCAG 1020

GGGAACACTC AGACATGTTC ATTCTCTTCC TGCTTCTGCT CTGGGCCGGT GGGTGGCTCT 1080

CAGAAAWTAC TTGCTGCTGG CAAAAGGCCT GTACTCAGGC ATTTGCTTTG ACTTGATGTT 1140

GCCAAGGGAC TGAGGCCATT GGCAGGCTTA GTACCACCTG CTCCTCATCT TAGGAGTCTC 1200

CTTTTCAAAT AATTAGGCTC TGTTCCCATT TTAAAACTCT GATATTGGCC TTCACCTGTG 1260

ACTGGACACT TTACTAGAGG CCCATTTTCA CTAAACAATA AAATCTAAAT AAATTGGAAG 1320

GAATAACAAC CACAAAGGAA AGAATAGAGT TGGTCTGGAT TGATGATCAC TGAGGATCTG 1380

TATGTGAGGC ACCCATAACA GTAGTTTTGC CTGTGAGTCG TCTTCACACA TGCTGTTTTC 1440

TCTGCCTGGC TCTCTCTTCC CCTCCTTACC TGGCCAGTCC TGTTTATCAT CAGGCCTTGT 1500

CTTGGATATC ACGTCCTCTG GGAAGTCTTC TTTTCCCCTC TAACCTAGGA CCCTCATTAC 1560

CGGCTCTCAT AGCACAGTCT ACTGCTTTGT ACGAATTCTA AGTATTCTTG TTGCACTTAA 1620

TTAGCCTGTA TATCCTCAGA ACTTTGTGTA ATGCCTGGAG CATAGTAGGC AGTCATATGT 1680

TGTATCGTGA ATAAATTGCA CATAGTAGCT ACCCAGCAAA TGCTGACTTC TTTTCTTTCT 1740

AGTCTTAACA CTCCCTTTCT AATNCATTTC CACTNTTGTA NTGTTCTCAA CATTACTTGG 1800

TAGTGACAAA CTTT 1814

(2) INFORMATION FOR SEQ ID NO: 74:

(i) SEQUENCE CHARACTERISTICS:
(A) LENGTH: 4712 base pairs
(B) TYPE: nucleic acid
(C) STRANDEDNESS: double
(D) TOPOLOGY: linear

(xi) SEQUENCE DESCRIPTION: SEQ ID NO: 74:

CATGGTACGC CTGCAGGTAC CGGTCCGGAA TTCCCGGGTC GACCCACGCG TCCGCCCAYG 60

CGTCCGGCGG CTCCGAGCCA GGGGCTATTG CAAAGCCAGG GTGCGCTACC GGACGGAGAG 120

GGGAGAGCCC TGAGCAGAGT GAGCAACATC GCAGCCAAGG CGGAGGCCGA AGAGGGGCGC 180

CAGGCACCAA TCTCCGCGTT GCCTCAGCCC CGGAGGCGCC CCAGAGCGCT TCTTGTCCCA 240

GCAGAGCCAC TCTGCMTGCG CCTGCCTCTC AGTGTMTCCA ACTTTGCGCT GGAAGAAAAA 300

CTTCCCGCGC GCCGGCAGAA CTGCAGCGCC TCCTCTTAGT GACTCCGGGA GCTTCGGCTG 360

TAGCCKGCTM TGCGCGCCCT TCCAACGAAT AATAGAAATT GTTAATTTTA ACAATCCAGA 420

GCAGGCCAAC GAGGCTKTGC TCTCCCGACC CGAACTAAAG CTCCCTCGCT CCGTGCGCTG 480

CTACGAGCGG TGTCTCCTGG GGCTCCAATG CAGCGAGCTG TGCCCGAGGG GTTCGGAAGG 540

CGCAAGCTGG GCAGCGACAT GGGGAACGCG GAGCGGGCTC CGGGGTCTCG GAGCTTTGGG 600

CCCGTACCCA CGCTGCTGCT GCTCSCCGCG GCGCTACTGS CCGTGTCGGA CGCACTCGGG 660

CGCCCCTCCG AGGAGGACGA GGAGCTAGTG GTGCCGGAGC TGGAGCGCGC CCCGGGACAC 720

GGGACCACGC GCCTCCGCCT GCACGCCTTT GACCAGCAGC TGGATCTGGA GCTGCGGCCC 780

GACAGCAGCT TTTTGGCGCC CGGCTTCACG CTCCAGAACG TGGGGCGCAA ATCCGGGTCC 840

GAGACGCCGC TTCCGGAAAC CGACCTGGCG CACTGCTTCT ACTCCGGCAC CGTGAATGGC 900

GATCCCAGCT CGGCTGCCGC CCTCAGCCTC TGCGAGGGCG TGCGCGGCGC CTTCTACCTG 960

CTGGGGGAGG CGTATTTCAT CCAGCCGCTG CCCGCCGCCA GCGAGCGCCT CKCCACCGCC 1020

GCCCCAGGGG AGAAGCCGCC GGCACCACTA CAGTTCCACC TCCTGCGGCG GAATCGGCAG 1080

GGCGACGTAG GCGGCACGTG CGGGGTCGTG GACGACGAGC CCCGGCCGAC TGGGAAAGCG 1140

GAGACCGAAG ACGAGGACGA AGGGACTGAG GGCGAGGACG AAGGGCCTCA GTGGTCGCCG 1200

CAGGACCCGG CACTGCAAGG CGTAGGACAG CCCACAGGAA CTGGAAGCAT AAGAAAGAAG 1260

CGATTTGTGT CCAGTCACCG CTATGTGGAA ACCATGCTTG TGGCAGACCA GTCGATGGCA 1320

GAATTCCACG GCAGTGGTCT AAAGCATTAC CTTCTCACGT TGTTTTCGGT GGCAGCCAGA 1380

TTGTWCAAAC ACCCCAGSAT TCGTAATTCA GTTAGCCTGG TGGTGGTGAA GATCTTGGTC 1440

ATCCACGATG AACAGAAGGG GCCGGAAGTG ACCTCCAATG CTGCCCTCAC TCTGCGGAAC 1500

TTTTGCAACT GGCAGAAGCA GCACAACCCA CCCAGTGACC GGGATGCAGA GCACTATGAC 1560
ACAGCAATTC TTTTCACCAG ACAGGACTTG TGTGGGTCCC AGACATGTGA TACTCTTGGG 1620
ATGGCTGATG TTGGAACTGT GTGTGATCCG AGCAGAAGCT GCTCCGTCAT AGAAGATGAT 1680
GGTTTACAAG CTGCCTTCAC CACAGCCCAT GAATTAGGCC ACGTGTTTAA CATGCCACAT 1740
GATGATGCAA AGCAGTGTGC CAGCCTTAAT GGTGTGAACC AGGATTCCCA CATGATGGCG 1800
TCAATGCTTT CCAACCTGGA CCACAGCCAG CCTTGGTCTC CTTGCAGTGC CTACATGATT 1860
ACATCATTTC TGGATAATGG TCATGGGGAA TGTTTGATGG ACAAGCCTCA GAATCCCATA 1920
CAGCTCCCAG GCGATCTCCC TGGCACCTCG TACGATGCCA ACCGGCAGTG CCAGTTTACA 1980
TTTGGGGAGG ACTCCAAACA CTGCCCTGAT GCAGCCAGCA CATGTAGCAC CTTGTGGTGT 2040
ACCGGCACCT CTGGTGGGGT GCTGGTGTGT CAAACCAAAC ACTTCCCGTG GGCGGATGGC 2100
ACCAGCTGTG GAGAAGGGAA ATGGTGTATC AACGGCAAGT GTGTGMACAA AACCGACAGA 2160
AAGCATTTTG ATACGCCTTT TCATGGAAGC TGGGGAATGT GGGGGCCTTG GGGAGACTGT 2220
TCGAGAACGT GCGGTGGAGG AGTCCAGTAC ACGATGAGGG AATGTGACAA CCCAGTCCCA 2280
AAGAATGGAG GGAAGTACTG TGAAGGCAAA CGAGTGCGCT ACAGATCCTG TAACCTTGAG 2340
GACTGTCCAG ACAATAATGG AAAAACCTTT AGAGAGGAAC AATGTGAAGC ACACAACGAG 2400
TTTTCAAAAG CTTCCTTTGG GAGTGGGCCT GCGGTGGAAT GGATTCCCAA GTACGCTGGC 2460
GTCTCACCAA AGGACAGGTG CAAGCTCATC TGCCAAGCCA AAGGCATTGG CTACTTCTTC 2520
GTTTTGCAGC CCAAGGTTGT AGATGGTACT CCATGTAGCC CAGATTCCAC CTCTGTCTGT 2580
GTGCAAGGAC AGTGTGTAAA AGCTGGTTGT GATCGCATCA TAGACTCCAA AAAGAAGTTT 2640
GATAAATGTG GTGTTTGCGG GGGAAATGGA TCTACTTGTA AAAAAATATC AGGATCAGTT 2700
ACTAGTGCAA AACCTGGATA TCATGATATC ATCACAATTC CAACTGGAGC CACCAACATC 2760
GAAGTGAAAC AGCGGAACCA GAGGGGATCC AGGAACAATG GCAGCTTTCT TGCCATCAAA 2820
GCTGCTGATG GCACATATAT TCTTAATGGT GACTACACTT TGTCCACCTT AGAGCAAGAC 2880
ATTATGTACA AAGGTGTTGT CTTGAGGTAC AGCGGCTCCT CTGCGGCATT GGAAAGAATT 2940
CGCAGCTTTA GCCCTCTCAA AGAGCCCTTG ACCATCCAGG TTCTTACTGT GGGCAATGCC 3000
CTTCGACCTA AAATTAAATA CACCTACTTC GTAAAGAAGA AGAAGGAATC TTTCAATGCT 3060
ATCCCCACTT TTTCAGCATG GGTCATTGAA GAGTGGGGCG AATGTTCTAA GTCATGTGAA 3120
TTGGGTTGGC AGAGAAGACT GGTAGAATGC CGAGACATTA ATGGACAGCC TGCTTCCGAG 3180
TGTGCAAAGG AAGTGAAGCC AGCCAGCACC AGACCTTGTG CAGACCATCC CTGCCCCCAG 3240
TGGCAGCTGG GGGAGTGGTC ATCATGTTCT AAGACCTGTG GGAAGGGTTA CAAAAAAAGA 3300

AGCTTGAAGT GTCTGTCCCA TGATGGAGGG GTGTTATCTC ATGAGAGCTG TGATCCTTTA 3360

AAGAAACCTA AACATTTCAT AGACTTTTGC ACAATGGCAG AATGCAGTTA AGTGGTTTAA 3420

GTGGTGTTAG CTTTGAGGGC AAGGCAAAGT GAGGAAGGGC TGGTGCAGGG AAAGCAAGAA 3480

GGCTGGAGGG ATCCAGCGTA TCTTGCCAGT AACCAGTGAG GTGTATCAGT AAGGTGGGAT 3540

TATGGGGGTA GATAGAAAAG GAGTTGAATC ATCAGAGTAA ACTGCCAGTT GCAAATTTGA 3600

TAGGATAGTT AGTGAGGATT ATTAACCTCT GAGCAGTGAT ATAGCATAAT AAAGCCCCGG 3660

GCATTATTAT TATTATTTCT TTTGTTACAT CTATTACAAG TTTAGAAAAA ACAAAGCAAT 3720

TGTCAAAAAA AGTTAGAACT ATTACAACCC CTGTTTCCTG GTACTTATCA AATACTTAGT 3780

ATCATGGGGG TTGGGAAATG AAAAGTAGGA GAAAAGTGAG ATTTTACTAA GACCTGTTTT 3840

ACTTTACCTC ACTAACAATG GGGGGAGAAA GGAGTACAAA TAGGATCTTT GACCAGCACT 3900

GTTTATGGCT GCTATGGTTT CAGAGAATGT TTATACATTA TTTCTACCGA GAATTAAAAC 3960

TTCAGATTGT TCAACATGAG AGAAAGGCTC AGCAACGTGA AATAACGCAA ATGGCTTCCT 4020

CTTTCCTTTT TTGGACCATC TCAGTCTTTA TTTGTGTAAT TCATTTTGAG GAAAAAACAA 4080

CTCCATGTAT TTATTCAAGT GCATTAAAGT CTACAATGGA AAAAAAGCAG TGAAGCATTA 4140

GATGCTGGTA AAAGCTAGAG GAGACACAAT GAGCTTAGTA CCTCCAACTT CCTTTCTTTC 4200

CTACCATGTA ACCCTGCTTT GGGAATATGG ATGTAAAGAA GTAACTTGTG TCTCATGAAA 4260

ATCAGTACAA TCACACAAGG AGGATGAAAC GCCGGAACAA AAATGAGGTG TGTAGAACAG 4320

GGTCCCACAG GTTTGGGGAC ATTGAGATCA CTTGTCTTGT GGTGGGGAGG CTGCTGAGGG 4380

GTAGCAGGTC CATCTCCAGC AGCTGGTCCA ACAGTCGTAT CCTGGTGAAT GTCTGTTCAG 4440

CTCTTCTGTG AGAATATGAT TTTTTCCATA TGTATATAGT AAAATATGTT ACTATAAATT 4500

ACATGTACTT TATAAGTATT GGTTTGGGTG TTCCTTCCAA GAAGGACTAT AGTTAGTAAT 4560

AAATGCCTAT AATAACATAT TTATTTTTAT ACATTTATTT CTAATGAAAA AAACTTTTAA 4620

ATTATATCGC TTTTGTGGAA GTGCATATAA AATAGAGTAT TTATACAATA TATGTTACTA 4680

GAAATAAAAG AACACTTTTG GAAAAAAAAA AA 4712

(2) INFORMATION FOR SEQ ID NO: 75:

(i) SEQUENCE CHARACTERISTICS:
(A) LENGTH: 1885 base pairs
(B) TYPE: nucleic acid
(C) STRANDEDNESS: double
(D) TOPOLOGY: linear

(xi) SEQUENCE DESCRIPTION: SEQ ID NO: 75:

ATGCCARGAA GACTGATGGA GCAGGCTTGC AATATTAAAG TNCCAACCAA GAAGCTGAAG 60

AAATWTGAGA AAGAATATCC AGACAATGCG AGAGAGTCAG CTGCAACAGG AAGACCCAAT 120

GGATAGATAC AAGTTTGTAT ATTTGTAGGT AACTCCAGCT GTTGCATTTA TACTGGGAAT 180

CTTCATAAGA AGCTGAGAGA AAGAGAGGGG AAAAAGAAAG TGGCTTTCTA CTTTCAAAAA 240

TGAAACAAAA AGGAAAAATG GCAAAGTACT GTTTTAGCTG TGCATGTCAT ATCCACAAAG 300

ACTTTTAGCA GGTGAACTGT TCCAAGACTG ACACAAGGAT GTTTCAAACT TGCCTCTGTC 360

TGTAGAAAAT GTTAAAAATA CCAACTCACT TGGAAGGAAA AATAAAAATC ACAAAGGTAT 420

ATTGAGCACA GTAGTGGTGT TTGTTGCAAC ATTTATTTCC ACAAATGAAT TTATGAACAA 480

CAGTGATATT TGACTTAAAG TATGAAGTTT CAGAATCAAA ATAATTTCAT TTTAATACGT 540

TCNGTTAATT GTGAATCTCT TCMATGGTAA TTAGCAACAC TGTTCCCAGG ATGCAAAGTT 600

GGGAAACACT TATTTCCAAC TTATTTTTTT CCAAGTAAAA TATTATCTCT CTTCAACATG 660

CTTTAACTTT TCAGACTCAC ACAGATACGT WACAGCTCCC TTCTCCCTCC ATATCAATAC 720

ACTAAGATAA AAGAATACTG TATTTTCAGC ACTGAGCAGC AGTGCCAAAA TCTCCTGCCA 780

AGAAATGGAC TGTGTGGCAT TATTAATTAA ATCACCCACA TTGGGATGAC TTCCACTTTT 840

GTAACTAGAG TTATCTTTAT GTGGTCAGAG CTGGACATAG GCAGCATAGT CACACAGAAC 900

ATCTTATCTC TGTKGCKGAA TKGAATAGCA TGGGATGTGT GCAGAGGAAC ATGGKGGGAG 960

TATGTAGGTT TKGTAGTCAG ACAGACCKGA ACTCAAATCT TGYTCATTTT TTAGAGCACA 1020

GGATTTGGAY TCCAAATTGA GGGTTTTAAT CCCCATGCCA CCATTCAGCA TCTTCGACTA 1080

GTTATTGAAC CTYTTCCTCA TSKATAAAAG ATATAGTGTT TCTGATTCCT TGATGGATTG 1140

TTACAAGGAT GAGGGATGCT GTATGTTAAG GACTCAGCTC ATAGTTGTGT TCAATAAATG 1200

GCTGTTATTT TATGAAGCCT ACTACTACAG ATTATGCAAT TATTACTAGA ATAATGCCAC 1260

CTTATGTGGG TCTTCCCCTC TAGTCCCTTA TTGATTGTTC TTATTTCTCT CAAGTATTGC 1320

CAACCAATAA TCTCCCCTTG CTTATAGAAG TGGTTCAAGA TCTGATTATA AAATCCCACA 1380

TACTTCTATA GCAGATAACT ATTAACAGAT AATGTTTGRA CTAATTTCAC CACCAACATT 1440

CCCCCTCAAT AAAACCAGCT TTTAATGTAA ATCACATAGC ATACTGCTTT AGAAAGGCTT 1500

GAAGGTAGTA ATTATAAACT ATTATTAAGC ATCCAAAATG AAGGTCTCCT TTTGCTAATA 1560

TCATTCAGAT TTTCTTATTA CTACAATTAT TATGAATAAA TTCTGTGAAG AGTGCTTTAA 1620

AATAAGAGAG AAATGGRAGA CCAAACTTGT ACATTTAAAA TCAGGCTGGA ATTGAACTTG 1680

TTATTGTGTC TTAAATCCTT TTTTGTGCCA AAGCAGGTAT GTATACATTA ATAGTAAGAT 1740

GTACATTATT TTTAAAGTAC TTATMACATG TAAGATTATC AATATGTATA GTTTTTATTG 1800

AGAGATCAAA GTAGGATTAA ACTTCTTGTT TTGAAAGCAG GCATTACTTT TTAAAAAAAA 1860

AAAAAAAAAA AAAAAAAAAA AAAAA 1885

(2) INFORMATION FOR SEQ ID NO: 76:

(i) SEQUENCE CHARACTERISTICS:
(A) LENGTH: 890 base pairs
(B) TYPE: nucleic acid
(C) STRANDEDNESS: double
(D) TOPOLOGY: linear

(xi) SEQUENCE DESCRIPTION: SEQ ID NO: 76:

TTCAAACTAG CAAAAAATGT ATGAAACTAT GAAGCTCGAT GCGTGTRATC ATCAGCAGAG 60

GCCGACGCTG CAGGCAGGGC CAAAGCTTCT GACCCTGGCC CCCAGGGAGG AACCCAGAGG 120

CCAGTCAGGG AGGGGCAGCG AGCTCACGGC CAGGCAGCGC CACAGCACTG GCGACCCTCA 180

GGGAGAACAG GCACTACCCA GGGCTGGATG CGTAACGGGC CCCCCGGCCA CACCCCACCG 240

CCCATCAGAG CCGCAGCTCC TGAGAACGCA TCCGGATGCN AGGCCAAAGT CAGCCATGGC 300

ACAAACATTT GTGCATCAAG GTCCTGTTGC TCTGCAACAA CTCACCACAA ACAGAAGGGT 360

GGAAACCTCC ATGTCATCGG ACGGCCACGG SCAGAATCCA ACGCCATCTC CCTGGGCTGA 420

TGTCTGTGCA AGCAGGGCTG ATGCCGTAGC TTTTCCGGCT TCTGGAARCT GCCACAGCCC 480

CTGGCTCATG GSACCATCCT CACATCCTCT GAATCCACAT TCTCCTCTGA ATCTCCCGCC 540

TCCCTCTTTC CACTGTAAGG ACCCTGTGAT GACACTGCAC CCTCAGACCC TGGTAACCCA 600

GGGTCATCTT TCCACCTCAG GGCGTCTGAC TTAAGCCTGC CTGGAGGGTC CCTGTGGTCA 660

CATTCATGGG TTCCAGGCTT CAGACACGGC CACTTTGTGG GATCATTACT CTGCCTACCA 720

CACCATGTGG CCCTGTGTGT GTTTTCAGGG GGCATTTGCG CYTATATGCA AATAATACAT 780

ATATGAATAA ACGTGTGAAT GGTGGTCACG TAGGAGARGG CATCTGTATG GGGCCACACC 840

TGTAAAAAAA AAAAAAAAAA AAAAAAAAAA AAAAAAAAAA AAAAAAAAAA 890

(2) INFORMATION FOR SEQ ID NO: 77:

(i) SEQUENCE CHARACTERISTICS:
(A) LENGTH: 1657 base pairs
(B) TYPE: nucleic acid
(C) STRANDEDNESS: double
(D) TOPOLOGY: linear

(xi) SEQUENCE DESCRIPTION: SEQ ID NO: 77:

AGAACGGCCT TCCCCACATC TTCCAGCACC TGCGCGCCTG AATCCGTCCC ACCCAGGCCC 60

AGACGCAGGC TTCTTCTCGG GTCTTGGTCC TGCATCCTCT CTCTCCCAGA GCCTCCGTTA 120

GGGGTGGGAA AGGACTTTGC CATAGGTCGC TGAGGCCACC ATCTGCTCTC TTACTGGCCA 180

AGGGCGTAAA AAGATAGTCY TCCCATTAGC TAGAGAGCAA ACCCCAGAAA GCCTATTGGC 240

TGCGCCGTCC GCGGGCCTTG GTCCGNTTTG AAGGCGGGCT GCGGCTGCGA GAGGAGGGCG 300

GGCGGGAGGC TAGCTGTTGT CGTGGTTGCT CGGAGGCACG TGTGCAGTCC CGGAAGCGGC 360

GAGGGGAAAC TGCTCCGCGC GCGCCGCGGG AGGAGGAACC GCCCGGTCCT TTAGGGTCCG 420

GGCCCGGCCG GGCATGGATT CAATGCCTGA GCCCGCGTCC CGCTGTCTTC TGCTTCTTCC 480

CTTGCTGCTG CTGCTGCTGC TGCTGCTGCC GGCCCCGGAG CTGGGCCCGA GCCAGGCCGG 540

AGCTGAGGAG AACGACTGGG TTCGCCTGCC CAGCAAATGC GAAGGGACTT GCGGTTAATC 600

GAAGTCACTG AGAACCATTT GCAAGAGGCT CCTGGATTAT AGCCTGCACA AGGAGAGGAC 660

CGGCAGCAAT CGATTTGCCA AGGGCATGTC AGAGACCTTT GAGACATTAC ACAACCTGGT 720

ACACAAAGGG GTCAAGGTGG TGATGGACAT CCCCTATGAG CTGTGGAACG AGACTTCTGC 780

AGAGGTGGCT GACCTCAAGA AGCAGTGTGA TGTGCTGGTG GAAGAGTTTG AGGAGGTGAT 840

CGAGGACTGG TACAGRAACC ACCAGGAGGA AGACCTGACT GAATTCCTCT GCGCCAACCA 900

CGTGCTGAAG GGAAAAGACA CCAGTTGCCT GGCAGAGCAG TGGTCCGGCA AGAAGGGAGA 960

CACAGCTGCC CTGGGAGGGA AGAAGTCCAA GAAGAAGAGC AKCAGGGCCA AGGCAGCAGG 1020

CGGCAGGAGT AGCAGCAGCA AACAAAGGAA GGAGCTGGGT GGCCTTGAGG GAGACCCCAG 1080

CCCCGAGGAG GATGAGGGCA TCCAGAAGGC ATCCCCTCTC ACACACAGCC CCCCTGATGA 1140

GCTCTGAGCC CACCCAGCAT CCTCTGTCCT GAGACCCCTG ATTTTGAAGC TGAGGAGTCA 1200

GGGGCATGGC TCTGGCAGGC CGGGATGGCC CCGCAGCCTT CAGCCCCTCC TTGCCTTGGC 1260

TGTGCCCTCT TCTGCCAAGG AAAGACACAA GCCCCAGGAA GAACTCAGAG CCGTCATGGG 1320

TAGCCCACGC CGTCCTTTCC CCTCCCCAAG TGTTTCTCTC CTGACCCAGG GTTCAGGCAG 1380

GCCTTGTGGT TTCAGGACTG CAAGGACTCC AGTGTGAACT CAGGAGGGGC AGGTGTCAGA 1440

ACTGGGCACC AGGACTGGAG CCCCCTCCGG AGACCAAACT CACCATCCCT CAGTCCTCCC 1500

CAACAGGGTA CTAGGACTGC AGCCCCCTGT AGCTCCTCTC TGCTTACCCC TCCTGTGGAC 1560

ACCTTGCACT CTGCCTGGCC CTTCCCAGAG CCCAAAGAGT AAAAATGTTC TGGTTCTGAW 1620

RAAAAAAAAA AAAAAAAAAA CCCCGGGGGG GGCCCGT 1657

(2) INFORMATION FOR SEQ ID NO: 78:

(i) SEQUENCE CHARACTERISTICS:
(A) LENGTH: 2015 base pairs
(B) TYPE: nucleic acid
(C) STRANDEDNESS: double
(D) TOPOLOGY: linear

(xi) SEQUENCE DESCRIPTION: SEQ ID NO: 78:

GGCCGGGCTG AGAGAAGAGC TTGCGGGGTT TGCGGTTGAT GGCCCCGACT GAAGGGCTGG 60

AGGCGGTGTA TGCCGCTGTT CTTGCTGTCG CTCCCGACAC CTCCGTCCGC TTCTGGTCAT 120

GAGAGGAGAC AGAGGCCTGA AGCAAAGACA TCTGGGTCAG AGAAAAAGTA TTTAAGGGCC 180

ATGCAAGCCA ATCGTAGCCA ACTGCACAGT CCTCCAGGAA CTGGAAGCAG TGAGGATGCC 240

TCAACCCCTC AGTGTGTCCA CACAAGATTG ACAGGAGAGG GTTCTTGCCC TCATTCTGGA 300

GATGTTCATA TCCAGATAAA CTCCATACCT AAAGAATGTG CAGAAAATGC AAGCTCCAGA 360

AATATAAGGT CAGGTGTCCA TAGCTGTGCC CATGGATGTG TACACAGTCG CTTACGGGGT 420

CACTCCCACA GTGAAGCAAG GCTGACTGAT GATACTGCCG CAGAATCTGG AGATCATGGT 480

AGTAGCTCCT TCTCAGAATT CCGCTATCTC TTCAAGTGGC TGCAAAAAAG TCTTCCATAT 540

ATTTTGATTC TGAGCGTCAA ACTTGTTATG CAGCATATAA CAGGAATTTC TCTTGGAATT 600

GGGCTGCTAA CAACTTTTAT GTATGCAAAC AAAAGCATTG TAAATCAGGT TTTTCTAAGA 660

GAAAGGTCCT CAAAGATTCA GTGTGCTTGG TTACTGGTAT TCTTAGCAGG ATCTTCTGTT 720

CTTTTATATT ACACCTTTCA TTCTCAGTCA CTTTATTACA GCTTAATTTT TTTAAATCCT 780

ACTTTGGACC ATTTGAGCTT CTGGGAAGTA TTTKGGATTG TTGGAATNAC AGACTTCATT 840

CTGAAATTCT TTTTCATGGG CTTAAAATGC CTTATTTTAT TGGTGCCTTC TTTCATCATG 900

CCTTTTAAAT CTAAGGGTTA CTGGTATATG CTTTTAGAAG AATTGTGTCA ATACTACCGA 960

ACTTTTGTTC CCATACCAGT TTGGTTTCGC TACCTTATAA GCTATGGGGA RTTTGGTMAC 1020

GTAACTAGAT GGARTCTTGG GATACTGCTG GCTTTACTCT ACCTCATATT AAAACTTTTG 1080

GAATTTTTTG GGCATCTGAG AACTTTCAGA CAGGTTTTAC GAATATTTTT TACACMACCM 1140

AGTTATGGAG TGGCTGCCAG CAAGAGACAG TGTTCAGATG TGGATGATAT TTGTTCAATA 1200

TGTCAAGCTG AATTTCAGAA GCCAATTCTT CTCATTTGTC AGCATATATT TTGTGAAGAG 1260

TGCATGACCT TATGGTTTAA CAGAGAGAAA ACATGTCCAC TCTGCAGAAC TGTGATTTCA 1320

GACCATATAA ACAAATGGAA GGATGGAGCC ACTTCATCAC ACCTTCAAAT ATATTAAGTT 1380

GTATAAACTA TCAAGGCCAC AAAATACTAA TGTCATTTGG TCATAATGAC TACTGATAAG 1440

GCATCAGAAT GGATTTTCAG GGCTACCAGA AAAATGTTTC CAGATGGTTT TAGAATGTAG 1500

GACTTATGAT CCAATTCACC AAAAGATTAA ATGAAACCAC CCTGTGTTTT AAAATATATA 1560

TAATGTTCAA CCTAATGTAT ATGCAACATT TATTCTATTC TAATTATTTG ACAGGTAACT 1620

GCAGTGTTAA ATTGTAAATG TGTTTTCTTT ATGTTACCAA AACAGCAATT TGAAATTAGA 1680

ACTAGTGGTT TTAGAGAACT CAGGTATTCT TTCCTGACAT TGTTTTCAGA ATAAAGAATA 1740

TTTTTCATAA TATTTTAAGA TACATACTAT CTAAAAGTAG AATTTTGTTC AGCATTGACT 1800

TTTATAATTC CCATCCTAAA AATTCTTAAT ATTTTCATAA AATTTGTATT TTTAAATGAA 1860

AATTCTAAAT GTTGTATTTT ATCAGTAACA TTTTCTAAGT GAAGATTAAT TTACTGAGGA 1920

TGATACATTA TAGTATTGTA TTATTCTCTG TAGTAAGATT AGTAATAAGT GAAAATAAAT 1980

G[?]TTTAAATT CAAAAAAAAA AAAAAANTNA CTCGA 2015

(2) INFORMATION FOR SEQ ID NO: 79:

(i) SEQUENCE CHARACTERISTICS:
(A) LENGTH: 1213 base pairs
(B) TYPE: nucleic acid
(C) STRANDEDNESS: double
(D) TOPOLOGY: linear

(xi) SEQUENCE DESCRIPTION: SEQ ID NO: 79:

AGCCTAGTTA CAGATTGCAC TGCGTCAGAC TGTTCCACAC CCAGAAGACG TCAGGTGACT 60

TCAGTCCTGC TGCAGTTGTG CAGCAGAGGA GACTGCAGAC TTCGGTTGAG GAAACGGGTA 120

TTTCATGTCT CAGGGAGTAG GTTTGTGCAG TTACAGCTTT TCTGTTGGTA TGCATAATTA 180

ATAATTGGAG CTGCAAASCA GATCGTGACA AGAGATGGAC GGTCAGAAGA AAAATTGGAA 240

GGACAAGGTT GTTGACCTCC TGTACTGGAG AGACATTAAG AAGACTGGAG TGGTGTTTGG 300

TGCCAGCCTA TTCCTGCTGC TTTCATTGAC AGTATTCAGC ATTGTGAGCG TAACAGCCTA 360

CATTGCCTTG GCCCTGCTCT CTGTGACCAT CAGCTTTAGG ATATACAAGG GTGTGATCCA 420

AGCTATCCAG AAATCAGATG AAGGCCACCC ATTCAGGGCA TATCTGGAAT CTGAAGTTGC 480

TATATCTGAG GAGTTGGTTC AGAAGTACAG TAATTCTGCT CTTGGTCATG TGAACTGCAC 540

GATAAAGGAA CTCAGGCGCC TCTTCTTAGT TGATGATTTA GTTGATTCTC TGAAGTTTGC 600

AGTGTTGATG TGGGTATTTA CCTATGTTGG TGCCTTGTTT AATGGTCTGA CACTACTGAT 660

TTTGGCTCTC ATTTCACTCT TCAGTGTTCC TGTTATTTAT GAACGGCATC AGGCACAGAT 720

AGATCATTAT CTAGGACTTG CAAATAAGAA TGTTAAAGAT GCTATGGCTA AAATCCAAGC 780

AAAAATCCCT GGATTGAAGC GCAAAGCTGA ATGAAAACGC CCAAAATAAT TAGTAGGAGT 840

TCATCTTTAA AGGGGATATT CATTTGATTA TACGGGGGAG GGTCAGGGAA GAACGAACCT 900

TGACGTTGCA GTGCAGTTTC ACAGATCGTT GTTAGATCTT TATTTTTAGC CATGCACTGT 960

TGTGAGGAAA AATTACCTGT CTTGACTGCC ATGTGTTCAT CATCTTAAGT ATTGTAAGCT 1020

GCTATGTATG GATTTAAACC GTAATCATAT CTTTTTCCTA TCTGAGGCAC TGGTGGAATA 1080

AAAAACCTGT ATATTTTACT TTGTTGCAGA TAGTCTTGCC GCATCTTGGC AAGTTGCAGA 1140

GATGGTGGAG CTAGAAAAAA AAAAAAAAAA ANCTYGAGAC TAGCGGCACG AGGGGGGGCC 1200

CGTACCCAAN ACG 1213

(2) INFORMATION FOR SEQ ID NO: 80:

(i) SEQUENCE CHARACTERISTICS:
(A) LENGTH: 1391 base pairs
(B) TYPE: nucleic acid
(C) STRANDEDNESS: double
(D) TOPOLOGY: linear

(xi) SEQUENCE DESCRIPTION: SEQ ID NO: 80:

GCAGAGGCCG ACTGCTGAAG GTGGTTTGCG TCGACATGGC GGTTACCCTG AGTCTCTTGC 60

TGGGCGGGCG CGTTTGCGCG CCGTCACTCG CTGTGGGTTC GCGACCCGGG GGGTGGCGGG 120

CCCAGGCCCT ATTGGCCGGG AGCCGGACCC CGATTCCGAC TGGGAGCCGG AGGAACGGGA 180

GCTGCAGGAG GTGGAGAGCA CCCTGAAACG ACAGAAACAA GCAATCCGAT TCCAGAAAAT 240

TCGGAGGCAA ATGGAGGCGC CTGGTGCCCC GCCCAGGACC CTGACGTGGG AAGCCATGGA 300

GCAGATACGG TATTTACATG AGGAATTTCC AGAGTCCTGG TCAGTTCCCA GGTTGGCTGA 360

AGGCTTTGAT GTCAGCACTG ATGTGATCCG AAGAGTTTTA AAAAGCAAGT TTTTACCCAC 420

ATTGGAGCAG AAGCTGAAGC AGGATCAAAA AGTCCTTAAG AAAGCTGGGC TTGCCCACTC 480

GCTGCAGCAC CTCCGGGGCT CTGGAAATAC CTCAAAGCTG CTCCCTGCAG GCCACTCTGT 540

ATCAGGCTCT TTGCTTATGC CAGGGCATGA AGCCTCATCT AAAGACCCAA ATCACAGCAC 600

AGCTTTGAAA GTGATAGAGT CAGACACTCA CAGGACAAAT ACACCAAGGA GAAGGAAGGG 660

AAGAAATAAA GAAATCCAGG ACCTGGAGGA GAGCTTTGTG CCTGTTGCTG CACCCCTAGG 720

TCATCCAAGA GAGCTGCAGA AGTACTCCAG TGATTCTGAG AGCCCCAGAG GAACTGGCAG 780

TGGTGCGTTG CCAAGTGGTC AGAAGCTGGA GGAGTTGAAG GCAGAGGAGC CAGATAACTT 840

CAGCAGCAAA GTAGTGCAGA GGGGCCGAGA GTTCTTTGAC AGCAACGGGA ACTTCCTGTA 900

CAGAATTTGA GTCGGGGCTT GGCTTATGGA GATGCCTCGT GAAACACAGC TGGGCAAGTA 960

TTAATGTATA TGGAACAGCC TGGATTTCTG CATATGGATA AGCCACCTTG GAATAGGAAG 1020

AGGTGTTGAG CCTGGACTGT GGGAGGAAAG AGCTGCGTGG ATAGATTCAA ACTTCCTGTG 1080

GTAGTGCTCC CAGTCTGACC TCTGTAGACC TTCAGTACTC ACTCTTCTTG CTTAGGCTCT 1140

CTGTGTGTTG AAAGCCATCC CGTGTTGCAT GTGTTGTTAC AATTTTCTGT GATACTTGCA 1200

ATTTATGTTT GAGAAGAAGT GAAAAGTTTG CCTTCTGACC TCATTTCCTT CTTGATCAGT 1260

GAACACTAAC ATTTTGGGGA CAACTTAGTC AATTGGTTTT CCTTACAACA AAATAAAGTA 1320

AAATGTAGCA AAAAAAAAAA AAAAAAAACN CGGGGGGGGC CCGTCCCATT GCCCAAAAGG 1380

GGGCCGAATA A 1391

(2) INFORMATION FOR SEQ ID NO: 81:

(i) SEQUENCE CHARACTERISTICS:
(A) LENGTH: 1008 base pairs
(B) TYPE: nucleic acid
(C) STRANDEDNESS: double
(D) TOPOLOGY: linear

(xi) SEQUENCE DESCRIPTION: SEQ ID NO: 81:

TGACATCGCC CTCATGAAGC TGCAGTTCCC ACTCACTTTC TCAGGCACAG TCAGGCCCAT 60

CTGTCTGCCC TTCTTTGATG AGGAGCTCAC TCCAGCCACC CCACTCTGGA TCATTGGATG 120

GGGCTTTACG AAGCAGAATG GAGGGAAGAT GTCTGACATA CTGCTGCAGG CGTCAGTCCA 180

GGTCATTGAC AGCACACGGT GMAATGCAGA CGATGCGTAC CAGGGGGAAG TCACCGAGAA 240

GATGATGTGT GCAGGCATCC CGGAAGGGGG TGTGGACACC TGCCAGGGTG ACAGTGGTGG 300

GCCCCTGATG TACCAATCTG ACCAGTGGCA TGTGGTGGGC ATCGTTAGCT GGGGCTATGG 360

CTGCGGGGGC CCGAGCACCC CAGGAGTATA CACCAAGGTC TCAGCCTATC TCAACTGGAT 420

CTACAATGTC TGGAAGGCTG AGCTGTAATG CTGCTGCCCC TTTGCAGTGC TGGGAGCCGC 480

TTCCTTCCTG CCCTGCCCAC CTGGGGATYC CCCAAAGTCA GACACAGAGC AAGAGTCCCC 540

TTGGGTACAM CCCTYTGCCC ACAGCCTCAG CATTTCTTGG AGCAGCAAAG GGCCTCAATT 600

CCTATAAGAG ACCCTCGCAG CCCAGAGGCG CCCAGAGGAA GTCAGCAGCC CTAGCTCGGC 660

CACACTTGGT GCTCCCAGCA TCCCAGGGAG AGACACAGCC CACTGAACAA GGTCTCAGGG 720

GTATTGCTAA GCCAAGAAGG AACTTTCCCA CACTACTGAA TGGAAGCAGG CTGTCTTGTA 780

AAAGCCCAGA TCACTGTGGG CTGGAGAGGA GAAGGAAAGG GTCTGCGCCA GCCCTGTCCG 840

TCTTCACCCA TCCCCAAGCC TACTAGAGCA AGAAACCAGT TGTAATATAA AATGCACTGC 900

CCTACTGTTG GTATGACTAC CGTTACCTAC TGTTGTCATT GTTATTACAG CTATGGCCAC 960

TATTATTAAA GAGCTGTGTA ACATCAAAAA AAAAAAAAAA AAACTCGA 1008

(2) INFORMATION FOR SEQ ID NO: 82:

(i) SEQUENCE CHARACTERISTICS:
(A) LENGTH: 1261 base pairs
(B) TYPE: nucleic acid
(C) STRANDEDNESS: double
(D) TOPOLOGY: linear

(xi) SEQUENCE DESCRIPTION: SEQ ID NO: 82:

GTTTTCAAAC TCATTTCTAA GCCAAATAGT TTAGATAAAT ATTTACCCTT ATATTTGGGG 60

G[?]AATTCAGG CTCACCATTT GCCGAGGCAA GCCCATCAAC AGTCTAGAGG CATATTCTGT 120

GTCATTCCTT CCCGTCTCCT TCATAGAATA CTACTTTTTC CTTTTGTCTC CTGGCCATTC 180

TCCATCATCT GCTGATTATT GCTAACCACA GGATGCTGGC AAAGCTTACA GTGATAGGCA 240

CATGTGTTCA GTGATGTCCA ATACACTCTT ATCACAGTGG TTATTGCTTC TTACTCTTTT 300

CAAATGCATT ATTCTACCCC TCAACCTAYA TCCAATCATT AGAACTATAC CTGACTGGAG 360

CCCAGAACTT GGGACCAATA CTTAATTCAA ATAGCAGGGG CTTGCTCACA AACATTAAGC 420

CCAAMAAGAA GCACAGCACT TTKGAAAAGT CAAATAGGSC TTTGGTAGCT CTGTACATTT 480

NGCAATTTAC ATTGTTATTA AGTTTATAGC ACTAATAACA CTTCAGTCGT GAATCTACAG 540

TCTCAATATG ATAAGTCTTA GAACATGTTC TAGAAATAGT GGTACCTTGC TGCTATTATA 600

CTTAGTAACT TATACCCCAA TATAATAATA AGTATTAAAT ACAGATTGTG TATGCATTCT 660

TTGTGTGTAT ATGCCAACTG TACTACTTAA CCTCACTGAT GAGCAATTAG AAAAATACAC 720

AAATTGTCAT AGTGAAAATA AGTCTTGGTC AATTCAGATG ATACGTGAAC CTGATAAATG 780

CTCTAATAGA TATGCTATTT TGTCCTGTAT TGCTTGTTTT ACAGTATGGT GCATGTTGTT 840

TGCTAAGTAA AATGATAATA ATAATAAAGT ATACCCAATT TTAAGGTTAG AATTAAAATT 900

TTGCACATAT GCTTCTTGAT ATTCTGAAAT GTATTCTGTG GSTTMATTAT CTTATTCATA 960

CACATTKMGC TWGGCTTTTT ACCCCTAGGA AATAACTGTC CAAGTATATA TCTCGTCTTC 1020

TTTCTTGTAA CTTTGATTAA ACTGCTTACT TCAACTTACA ACATTGTAAA GCCAGAATAC 1080

CTCATTTTAA CAGTGAAAAA AAATATTATG ACCTGATGTG TTCTCTTGTA TTTGATTTGA 1140

ACTACCTAAA TAGGCTTAAC TGTAATAATA AATATACAAT TTTGGCAAAA AAAAAAAAAA 1200

AAAAAAAAAA AAAAAAAAAA AAAAAAAAAA AAAAAAAAAA AAAAAAAAAA AAAGGGCGGC 1260

C 1261

(2) INFORMATION FOR SEQ ID NO: 83:

(i) SEQUENCE CHARACTERISTICS:
(A) LENGTH: 1045 base pairs
(B) TYPE: nucleic acid
(C) STRANDEDNESS: double
(D) TOPOLOGY: linear

(xi) SEQUENCE DESCRIPTION: SEQ ID NO: 83:

TCGAGTTTTT TTTTTTTTTT TTTTAAGCAA CAGTTTATTG AGACGGAAAA AATATGATCC 60

AGCAAAGGCG AGGAGGCGAG CCGGGCCCCG AGCCAGCTGG TGTCATTGTC ACTGGCTCCC 120

AAACCTGACT CCTGTGGACG TGTCTGTACC CCAAACACAG CTGCCCACCC CAGCCCTGGC 180

ACAGAGCCCT TCTGAAAGAA AGAAAAAAGA AGAAAGACGC GGCACCTGAC GCCAGCGGGT 240

AAAAGCAGGG CCCCAGAGGC ATTTATTGAA AACACAGCAT CCAAAACACG ACATCTAGGC 300

CAGGCGCGAT GGTTACAGTG ATGAGAGGGT CACTAGACAA TTATCCACAA TTCTACGACA 360

TGAGACAGAG ACTCAGCAAC AGTCACAGAC AGAAGGGTCA TGTGTTCCTT CCTGGGCAGG 420

GCTGAATGTG GCAGGTGCGG CGTGGAGGCT GCGTCCTGGC GGTTTGCTCC CAGGCAAGGG 480

GTACGGGGGG CCGGCTTGGC TGGGTGGGGA CCTCAAGTCT GAGGGTGAGG ATGGCTGAAT 540

CTACCTCGCT TATGTCTCAG GGACGGTCAC CCATACCTAG GATGACCCCA GCCAGACCCT 600

AGAAGGTCTG ATGGCCATCC CAAGTNCCCC CGCGAGGAGA AGAGTTCCCT GGCAGGGGTG 660

ACACATTCCC GGTCAACAAG CCACAACACA GTGGTGCCTG CACTCTCTCA GCTGTTGCCA 720

CAACACTTGG TGCTGGAATT TTCTCCACGT AGTGAAACTT TTAAGGGACA CATGAATAAT 780

TTAAAAAGTC ACACAAAACT CTACGAAAGG CAGGAATCCT CACTCTGCTG AGAGCTACCT 840

CCTGAGATGT CGCTTCCGGA CCCCGGCAGA GGGCAGGAGC GACATCAGCT CGGCAGGAGG 900

ATCCTNGCCA GCGCGAGGGC TGGCTCTGGT TATTATAAAT AATCTAATTT AAATACGCAC 960

ATACACACAG ATGTCCTGCT TCTACCNAAC GCCAAGAAAA GCAGACATTA GCATCACACT 1020

GTCAACACTT CCTCGAGAAC NGAAG 1045

(2) INFORMATION FOR SEQ ID NO: 84:

(i) SEQUENCE CHARACTERISTICS:
(A) LENGTH: 2877 base pairs
(B) TYPE: nucleic acid
(C) STRANDEDNESS: double
(D) TOPOLOGY: linear

(xi) SEQUENCE DESCRIPTION: SEQ ID NO: 84:

GAATTCGGCA CGAGACAAGA TGGCAGTCAA CAGCTTCCCA AAAGATAGGG ATTACAGAAG 60
AGAGGTGATC ACAGACATGA AAAGATGCGA GACGCCGGAG ATCCTTCACC ACCAAATAAA 120
ATGTTGCGGA GATCTGATAG TCCTGAAAAC AAATACAGTG ACAGCACAGG TCACAGTAAG 180
GCCAAAAATG TGCATACTCA CAGAGTTAGA GAGAGGGATG GTGGGACCAG TTACTCTCCA 240
CAAGAAAATT CACACAACCA CAGTGCTCTT CATAGTTCAA ATTCACATTC TTCTAATCCA 300
AGCAATAACC CAAGCAAAAC TTCAGATGCA CCTTATGATT CTGCAGATGA CTGGTCTGAG 360
CATATTAGCT CTTCTGGGAA AAAGTACTAC TACAATTGTC GAACAGAAGT TTCACAATGG 420
GAAAAACCAA AAGAGTGGCT TGAAAGAGAA CAGAGACAAA AAGAAGCAAA CAAGATGGCA 480
GTCAACAGCT TCCCAAAAGA TAGGGATTAC AGAAGAGAGG TGATGCAAGC AACAGCCACT 540
AGTGGGTTTG CCAGTGGAAT GGAAGACAAG CATTCCAGTG ATGCCAGTAG TTTGCTCCCA 600
CAGAATATTT TGTCTCAAAC AAGCAGACAC AATGACAGAG ACTACAGACT GCCAAGAGCA 660
GAGACTCACA GTAGTTCTAC GCCAGTACAG CACCCCATCA AACCAGTGGT TCATCCAACT 720
GCTACCCCAA GCACTGTTCC TTCTAGTCCA TTTACGCTAC AGTCTGATCA CCAGCCAAAG 780
AAATCATTTG ATGCTAATGG AGCATCTACT TTATCAAAAC TGCCTACACC CACATCTTCT 840
GTCCCTGCAC AGAAAACAGA AAGAAAAGAA TCTACATCAG GAGACAAACC CGTATCACAT 900
TCTTGCACAA CTCCTTCCAC GTCTTCTGCC TCTGGACTGA ACCCCACATC TGCACCTCCA 960
ACATCTGCTT CAGCGGTCCC TGTTTCTCCT GTTCCACAGT CGCCAATACC TCCCTTACTT 1020
CAGGACCCAA ATCTTCTTAG ACAATTGCTT CCTGCTTTGC AAGCCACGCT GCAGCTTAAT 1080
AATTCTAATG TGGACATATC TAAAATAAAT GAAGTTCTTA CAGCAGCTGT GACACAAGCC 1140
TCACTGCAGT CTATAATTCA TAAGTTTCTT ACTGCTGGAC CATCTGCTTT CAACATAACG 1200
TCTCTGATTT CTCAAGCTGC TCAGCTCTCT ACACAAGCCC AGCCATCTAA TCAGTCTCCG 1260
ATGTCTTTAA CATCTGATGC GTCATCCCCA AGATCATATG TTTCTCCAAG AATAAGCACA 1320
CCTCAAACTA ACACAGTCCC TATCAAACCT TTGATCAGTA CTCCTCCTGT TTCATCACAG 1380
CCAAAGGTTA GTACTCCAGT AGTTAAGCAA GGACCAGTGT CACAGTCAGC CACACAGCAG 1440
CCTGTAACTG CTGACAAGCM GCAAGGTCAT GAACCTGTCT CTCCTCGAAG TCTTCAGCGC 1500
TCAAGTAGCC AGAGAAGTCC ATCACCTGGT CCCAATCATA CTTCTAATAG TAGTAATGCA 1560
TCAAATGCAA CAGTTGTACC ACAGAATTCT TCTGCCCGAT CCACGTGTTC ATTAACGCCT 1620
GCACTAGCAG CACACTTCAG TGAAAATCTC ATAAAACACG TTCAAGGATG GCCTGCAGAT 1680
CATGCAGAGA AGCAGGCATC AAGATTACGC GAAGAAGCGC ATAACATGGG AACTATTCAC 1740
ATGTCCGAAA TTTGTACTGA ATTAAAAAAT TTAAGATCTT TAGTCCGAGT ATGTGAAATT 1800

CAAGCAACTT TGCGAGAGCA AAGGGATACT ATTTTTGAGA CAACAAATTA AGGAACTTGA 1860

AAAGCTAAAA AATCAGAATT CCTTCATGGT GTGAAGATGT GAATAATTGC ACATGGTTTT 1920

GAGAACAGGA ACTGTAAATC TGTTGCCCAA TCTTAACATT TTTGAGCTGC ATTTAAGTAG 1980

ACTTTGGACC GTTAAGCTGG GCAAAGGAAA TGACAAGGGG ACGGGGTCTG TGAGAGTCAA 2040

TTCAGGGGAA AGATACAAGA TTGATTTGTA AAACCCTTGA AATGTAGATT TCTTGTAGAT 2100

GTATCCTTCA CGTTGTAAAT ATGTTTTGTA GAGTGAAGCC ATGGGAAGCC ATGTGTAACA 2160

GAGCTTAGAC ATCCAAAACT AATCAATGCT GAGGTGGCTA AATACCTAGC CTTTTACATG 2220

TAAACCTGTC TGCAAAATTA GCTTTTTTAA AAAAAAAAAA AAAAAAATTG GGGGGGTTAA 2280

TTTATCATTC AGAAATCTTG CATTTTCAAA AATTCAGTGC AAGCGCCAGG CGATTTGTGT 2340

CTAAGGATAC GATTTTGAAC CATATGGGCA GTGTACAAAA TATGAAACAA CTGTTTCCAC 2400

ACTTGCACCT GATCAAGAGC AGTGCTTCTC CATTTGTTTT GCAGAGAAAT GTTTTTCATT 2460

TCCCGTGTGT TTCCATTTCC TTCTGAAATT CTGATTTTAT CCATTTTTTT AAGGCTCCTC 2520

TTTATCTCCT TTCTTAAGGC ACTGTTGCTA TGGCACTTTT CTATAACCTT TTCATTCCTG 2580

TGTACAGTAG CTTAAAATTG CAGTGATTGA GCATAACCTA CTTGTTTGTA TAAATTATTG 2640

AAATCCATTT GCACCCTGTA AGAATGGACT TAAAAGTACT GCTGGACAGG CATGTGTGCT 2700

CAAAGTACAT TGATTGCTCA AATATAAGGA AATGGCCCAA TGAACGTGGT TGTGGGAGGG 2760

GAAAGAGGAA ACAGAGCTAG TCAGATGTGA ATTGTATCTG TTGTAATAAA CATGTTAAAA 2820

CAAAAAAAAA AAAAAAAGGG CGGCGGCTCG CGATCCTAGA ACTAGCGGAC GCGTGGG 2877

(2) INFORMATION FOR SEQ ID NO: 85:

(i) SEQUENCE CHARACTERISTICS:
(A) LENGTH: 1367 base pairs
(B) TYPE: nucleic acid
(C) STRANDEDNESS: double
(D) TOPOLOGY: linear

(xi) SEQUENCE DESCRIPTION: SEQ ID NO: 85:

AATCATGAGC CTCCAGAAGA GACAGATGGC CCACCAGGAG CTGTTGCTCT GGTTGCCTTC 60

CTGCAGGCCT TGGAGAAGGA GGTCGCCATA ATCGTTGACC AGAGAGCCTG GNAACTTGCA 120

CCARAAGATT GTTGAAGATG CTGTTGAGCA AGGTGTTCTG AAGACGCAGA TCCCGATATT 180

AACTTACCAA GGTGGATCAG TGGAAGCTGC TCAGGCATTC CTGTGCAAAA ATGGGGACCC 240

GCAGACACCT AGATTTGACC ACCTGGTGGC CATAGAGCGT GCCGGAAGAG CTGCTGATGG 300

CAATTACTAC AATGCAAGGA AGATGAACAT CAAGCACTTG GTTGACCCCA TTGACGATCT 360

TTTTCTTGCT GCGAAGAAGA TTCCTGGAAT CTCATCAACT GGAGTCGGTG ATGGAGGCAA 420

CGAGCTTGGG ATGGGTAAAG TCAAGGAGGC TGTGAGGAGG CACATACGGC ACGGGGATGT 480

CATCGCCTGC GACGTGGAGG CTGACTTTGC CGTCATTGCT GGTGTTTCTA ACTGGGGAGG 540

CTATGCCCTG GCCTGCGCAC TCTACATCCT GTACTCATGT GCTGTCCACA GTCAGTACCT 600

GAGGAAAGCA GTCGGACCCT CCAGGGCACC TGGAGATCAG GCCTGGACTC AGGCCCTCCC 660

GTCGGTCATT AAGGAAGAAA AAATGCTGGG CATCTTGGTG CAGCACAAAG TCCGGAGTGG 720

CGTCTCGGGC ATCGTGGGCA TGGARGTGGA TGGGCTGCCC TTCCACAACA MCCACGCCGA 780

GATGATCCAG AAGCTGGTGG ACGTCACCAC GGCACAGGTG TAACCGTCCA TGTTCCGTGT 840

GAGCAGAGTC CCTACCAACG GGCAGGTCTG CATCCGGGGA GAATGCAGCT GCTTCTGGCG 900

ACAATCCTGC TAGTAAACAC TGGTCTTCGG TGAGCAACGA ACACTCGCCT GGCCTGGGAA 960

ACTGCATGCC CACTTTCTGG GAGGGGTTAG TGCAGGTGCC GTGGACAAAG GACAACATTT 1020

CTCTGGGGCT TTTTAACTTT TATTCCTAAG ACTCTAAAGG CGTTGATTTC AACCCTCCTT 1080

CACTCTGGCT TCTTCAGGCA ACCCACGTGG TCTCCTGTGA GAATCTTCTC GACAGTTACT 1140

TATGGGGACA CTTGTGAACA ATTAACTGCC AGGCAGAGCA TGAGAACAAA CATTCCCAGG 1200

CCATGTAGGA TAGGATACTC CAGACTCCAG TCATCCTCCC CCATCCATGG TTTCTGTTAC 1260

TCATGGTTTC AGTTACTCAT AGCCAACTGC AGACCGAAAA TACTAAATGA AAAATTTCAG 1320

AAATAAACAA CTCTTAAGTT TTAAAAAAAA AAAAAAWWAA ACTCGTA 1367

(2) INFORMATION FOR SEQ ID NO: 86:

(i) SEQUENCE CHARACTERISTICS:
(A) LENGTH: 1009 base pairs
(B) TYPE: nucleic acid
(C) STRANDEDNESS: double
(D) TOPOLOGY: linear

(xi) SEQUENCE DESCRIPTION: SEQ ID NO: 86:

GAATTCGGCA CGAGCTCGTG CCGAATTCTC GTGCCGAACT GAAACGTATC AAGAAATACC 60

TGGGCTTGAA GAATATTCAC CTGAAATATA CCAAGAAACA TCCCAGCTTG AAGAATATTC 120

ACCTGAAATA TACCAAGAAA CACCGGGGCC TGAAGACCTC TCTACTGAGA CATATAAAAA 180

TAAGGATGTG CCTAAAGAAT GCTTTCCAGA ACCACACCAA GAAACAGGTG GGCCCCAAGG 240

CCAGGATCCT AAAGCACACC AGGAAGATGC TAAAGATGCT TATACTTTTC CTCAAGAAAT 300

GAAAGAAAAA CCCAAAGAAG AGCCAGGAAT ACCAGCAATT CTGAATGAGA GTCATCCAGA 360

AAATGATGTC TATAGTTATG TTTTGTTTTA ACAATGCTCA ACCATAAAGT TGTGGTCCAA 420

TGGAACATAC AGCTTAATAG TTTATGCGTG ATTTTCTCAA AATATTGTAA AACTTTTGAC 480

AATGCTCATT AATATTATTT TTTCTATTTG TAGACCATAT CTGAAAGAAA TAACATTTTT 540

TAAGGCTCTA CCACATAGAC AATATCATGC TAGAATGTGT GTGTGTGTGT GTGTGTGTGT 600

GTGTGTATGT ATGTATAGGT CGGGGAGAGG ATAGTGGTGG GAACAGACAA ATAAGGAAGC 660

GGGGAGGACT GGATAATTGG TTTTCCCCCC TAAGAACATT TATTTACGTC TTAAGAGCAG 720

ATAAGTGACT AAGACTGAAC ACATACATTT TGTGGAGTAT ATAGTTTTCT TGTAAATGCT 780

GTTCAATTAT TAATGTAACA GTAGCATCAA AATTTTATTC AGGCTTTAGT TGACTCTTTT 840

GGTCAGTTTT AACAATTCTC CTTAAAAGAT ATTTTGGAGT GATGAATGTA GTTTACTTTT 900

GTATTTGAAT TTTGATTTTC TATTTTTATT TTTTAAATAT TGTATTTGTG CACAATGTAC 960

ATTAAATCAT TATTACATGC TTAAAAAAAA AAAAAAAAAA AAAACTCGA 1009

(2) INFORMATION FOR SEQ ID NO: 87:

(i) SEQUENCE CHARACTERISTICS:
(A) LENGTH: 1367 base pairs
(B) TYPE: nucleic acid
(C) STRANDEDNESS: double
(D) TOPOLOGY: linear

(xi) SEQUENCE DESCRIPTION: SEQ ID NO: 87:

AATTCCAAAA CAAGGTAAAA GGAACCAGAA AAGAAAAAAA ATGTAAATAA AGTTATAAAA 60

ATAAAGAATT TTTTCAAGGT TAAAAAGCTG AAAAAGAAAT AATTTTATAT AAGAAAGAAT 120

TTTATATGGT AAATTTAGTC CTAAAATAAA ATAACTGGTT GTTTAACAAG GAGGGATGTT 180

CAGGACAAAC CAGAAAGTCC AAGCATGTCA TGAACATTGG TGTAAGTCAT GATAAGATTT 240

TATATATATA TATACACACA CACACACACA CCCCAAAAGC TTTTATATAA TCAAGTTGTC 300

MTATTATTAT TAAGTTTTGG TTTGCTTAGG GAAGAAAGAR CTAATTTTTA AAAAATCAAG 360

GTTATTACAT CCATGTATCT TCCTGTGTAT GCTTTTAAAG TCCTTGTAAC ATTGAGTTAC 420

AGGGCTTTAA CTCCTGTGTC TGAAAAATCA CAAACACTGA TGACAATCAA AGCCTCATCT 480

TAAGGCCCCG TAGAAGATGC CAATCAAAAT AAACTGCATT CCTGAGGCAC TAGGCAAGAA 540

ATTAAAGCTA TTCAACTCCT CAAGGCCCAG GGACTATTGC GGAAGAGGTG GGCGCGTAAG 600

ATTGTAAGGG CCGATTTTGA AAGATCCAGT AAGTTCAGTT TCTCTATGAA CTAATCATTC 660

AAGTCAAAGG CACACTGATG CAAAATCAGT ATATGGACCC CTGTGTCTGA TTAGCAAGGT 720

TTTCTTGAAG CATTAACCAA CTCCTTCATA AAGGTTATAA AAGGCTTATG GRAGTTATAT 780

TTTATAATCA AGATTAAATC TTATAGTTTG TTTACAAAAT TTTGAAAATC AAATGTGATT 840

GGCTTCAGGC TGTTTTTATT AGGGCTTCTT GTTTAGAAAG TTAAGTCACC TCTCTCAAAG 900

AATGAAGGTT TTTGCTTTTT TTGAAATCCT TGAATTATCA CTTGGRTTAA ATAAATGACT 960

TTACGATGAC CTGTAATTTT ATTTTGTAAT GTCAAGTGTT TTAAACCTTT TGTATTTGAC 1020

AAGCTTTCCA AAATCAAATT ATAAATTATG TATTTTTCTA ACCTAATTAA TCCTTTAAGA 1080

TCTTAGTTTC CCTAAAGTCC TAAAATGACA TAATTTGGCT TATTTGGTAT AAAAATTATA 1140

TAGGAAGCAT TGTCAAATGT GAAATGGTGT TTGGTTTTCT TTGGGCTGTA TTTGTATAAA 1200

TATGTTATTG GTGTATGTTC CAAAATTATG TGAAACTCCT ATAATTCTAA TATAACTTAG 1260

TGTACATTAT CAGTAATAAT CATAATTGTT ATATTAAAAT TATTGTGTGC CACAGAGGTA 1320

AAAAAAAAGG AATTCGATAT CAAGCTTATC GATACCGTCG ACCTCGA 1367

(2) INFORMATION FOR SEQ ID NO: 88:

(i) SEQUENCE CHARACTERISTICS:
(A) LENGTH: 1088 base pairs
(B) TYPE: nucleic acid
(C) STRANDEDNESS: double
(D) TOPOLOGY: linear

(xi) SEQUENCE DESCRIPTION: SEQ ID NO: 88:

GAATTCGGCA CGAGTGAAAT TTTGTCGATT TCAAAAATGG AAAATACATA ATATGCCAGG 60

CACTTCCTGG GCAATACAGA TACCTGCAGT AATGGAGTGA GCACCAGCAT CTTCCCTGAT 120

GGCGTGTGCA GTGAGGTGAC TCGTCTGTAG TGTCCTCAAG GTCACGTAGA GAGCATACAG 180

TAAATACTTG TTGACTCTTT CAAACTTAAG TTAATGATAC AGTCAGGACT GATAGCCATT 240

TTGTTGTCTT TCTTGAAAGT TTACGTGGAA GGCAGACCTT GTGTATGCTT TTCAAAGGGG 300

CTCMTTTAGC GCACTTGGCG CTTAAGAATT TGAGATCAGT AAGTGTGATG GTCCTAATCT 360

TTTTTTAAAA GTATTGGAAG TTTGAACYCM CCTGATGGGG TTGGTTTTTT TTTTTTTTTT 420

TTCCAAAAAA ATAATCATTC AAAATAATCG GTTAACATTT TCAATAAGAG CATTACATAC 480

AAGGAGTTAG GGAACAAAGA GTTTTAAAAT CTGGCTCTTT TTATCTCTAC TTAGGGCGTG 540

CATCTTCTCT TCTTACCCCA ACATATACTG ACTTTTTAGG ACCTCCTTTA GGGAGATCTC 600

AATATCCCGA ATTTTTCTGT GTGGAGAGGG GAAGGAATAT GTCTTTTTTT GCTTTGGTCA 660

GAGTGGATAC ATTTTATAGT TTGTTTTTTC AAAGACGGGT CTTCTGAGTC ASTTCTTTCA 720

CTGCTGCCGT AAAGAAACTG TATAAAGGTG ATTGAGCAGT GAAGGCATGG ATAAAAGGGG 780

AAATATTCAG CAGTTCTGAA CGTGCATGTC ATCAAATATA AAGGAGTGAG AACTTGATGT 840

ATAAGAAAAA ATGGAAGTTA AAAAAAAWAA AAATCCAAGA ATGGGCTGCT TGTTGCAGTA 900

GTGAACTCCT CGCTGGAGGT ACTAGAGCGG AGTCTGTCTC AAGGATGCTA TTGGAAGCAC 960

CCCAGCTGTG GGTGGAAAAC TGCACTTTCT GAGCCTAGTC TTTTATAGCC TGGRGTTTTT 1020

GATGCTGATG CTTTTACTAC TTGTTCTTAG ACTWTTTTGC CATACGCTGC TCTGTTTTCT 1080

CACCTCCA 1088

(2) INFORMATION FOR SEQ ID NO: 89:

(i) SEQUENCE CHARACTERISTICS:
(A) LENGTH: 1861 base pairs
(B) TYPE: nucleic acid
(C) STRANDEDNESS: double
(D) TOPOLOGY: linear

(xi) SEQUENCE DESCRIPTION: SEQ ID NO: 89:

TCTCTGCCCC TCATCTTGGT AATTAGCCAG CCTCAGATAC TTCTGTGGGC CCTGAAGTGG 60

ACTCTCAAGG TCAGACCAAG GTTGCTGATC TCAGTCCCAC TGTCTTCAGC CAGCTGAAGC 120

TGTGGGGCTG GGCTGGCAGC TTTATTGTCA TCTTGCTTCA CCATTTTTTT TTCTCTCTCT 180

TTTCATTCTA TTTTAAGTTT AGACCAAAAA AATACAGAGT CATCCCCTAC CCCCACCCCT 240

CTAGAGACCC TCCAGCTAAA AACAGAGCCT GAGTTCAGGG ACCCAAGTGG TGAGCGGCGT 300

CTTTTGGGGG TGAGGGAGCT TGGGTAGATG AGGCTCCTGG CTGAGCCCTC CCTGTGGTGA 360

TCCCAGCCTA AGATGGCCCC TCTTCCCTCC TGGTGGGAGA CAGAGGACTG GACCCTGGGT 420

CTCAGGTTCC AGCAAGTCAG GCTAGGGACC TGGGGGGAGG AGACCCATGG ACTTCACCCA 480

TACTCAGTGA GGGGGCTCCT GCCGTCCTGA CGCCACCCCG CCCCATCAGC ACTTAAGCCA 540

CATGACACAA AGTCTGTACC GCACGGGAAA TGTTCACGCG CCTGGGCCGT GTGCATGGCC 600

TCCCGGGCTG TGGGGCAGCC GCATCTGTGA GGTGACYCGT GAAAGTAGGT GATTCCYTTG 660

CAGAACTTCA GGGACTGGGA GCAGAGGCCC CTCACTCAAC GACGTTTGTG CGACATAGTA 720

TTGTATCCAC CTTAGTATTG TATCGAGCCT TTTCTGTGTT TTAATGAGAA AGCAGAACAC 780

TAGTTTCCTA TTTAAGACTT TAAGGGTTTG TGGGGCGGGG CGGGATTAAC ACAACATTTG 840

GCTTTGTTTT CTTTTTCCTT TGATTTCCAC ATCAGGTGTG TGCGAGTGTG TGTGTGTGGA 900

GATGTTAAGA GCCTCACAAG GAAACTGGGT TATTGGAGGC CAAGGCGGCT TACAGTTCTC 960

TGCGTTCGTC ACTTAATTCC TGAATGTTTC AGAGAAACAG GAATCAGAAA ATAGCAGATA 1020

TCATGTAGGA AAGAGAGGAT AAACAAAGAA AAAAGAAAAA AAAATAAGCT CATACCCAAA 1080

TTCACAAAGC CTATTTTTTA AACCAAAGCA CATTTTGAAT GAGTATGGAA CCTCCATGGG 1140

CTCAGAAAAA AGATGCTAAT ATATTTATCT CATTGTTTAC ATAAGCTTTT ACAGTTTCAG 1200

ACCTCAGCAG CTGTAAGGCC AGTCCAGGGA ACCCTCCCCT GCTGCTGGAA ACCCTTCTGA 1260

GTTGGCCCTG GAGTGGCTCA SGGGCAGAGA AGGGTAGCCC TGGGGCTGGG GGAGGGATTG 1320

GAAGCCTCCC TGGAGTCACC TGAGCCCTCG TCCCCATTCC CAGGGCCCCT CCAAGCCCAG 1380

CTGGCACCAA ARAGCTTGGG CCCGTSCTGA CCAGCCCCCA AGGCCCTCTG GCCGGACCAT 1440

GCTGGTCCTG ACCAGCTAGC CTACGCGGGG ATGGCCGTCA GTTCTGGCCA CAGGACCCGA 1500

GTCTGGGCTT GGGTCCCCCT GCTGCTCTGC CCGTGACCCT TGGGGATGGG TTGATGCGAG 1560

GGTCCCACTC AAGCCAAAAA GCCGGGACCT TTGCGCAGCT CTGTCGACTC TGGTGGGTCC 1620

CCACTCCTGG GGCCCCCTAA CCCCACCCCA GGCAGCGGAA GGGGCTGACT GGGTCTGGTC 1680

CTTACCAACA TAGACGGTGC AAACACTCTT AACAGTGTTG TTTTTGTATC AATATGTTTG 1740

TGCAGTGATG AATGTATTTA TTTCTCAGAC TTGGGGCGAG TGAGCGGGTG GCAGGCCGGC 1800

TCCGCCACTG CAATGCTCCC GCCGGACCGA GCCCCAGCAA GGGCTCCTCC AGGATTGCAA 1860

A 1861

(2) INFORMATION FOR SEQ ID NO: 90:

(i) SEQUENCE CHARACTERISTICS:
(A) LENGTH: 1259 base pairs
(B) TYPE: nucleic acid
(C) STRANDEDNESS: double
(D) TOPOLOGY: linear

(xi) SEQUENCE DESCRIPTION: SEQ ID NO: 90:

AATTCGGCAC GAGCTCGTGG AGAGATTGAA GATGGCGGCT TCTCAGGCGG TGGAGGAAAT 60

GCGGACCGCG TGGTTCTGGG GGAGTTTGGG GTTCGCAATG TCCATACTAC TGACTTTCCC 120

GGTAACTATT CCGGTTATGA TGATGCCTGG GACCAGGACC GCTTCGAGAA GAATTTCCGT 180

GTGGATGTAG TACACATGGA TGAAAACTCA CTGGAGTTTG ACATGGTGGG AATTGACGCA 240

GCCATTGCCA ATGCTTTTCG ACGAATTCTG CTAGCTGAGG TGCCAACTAT GGCTGTGGAG 300

AAGGTCCTGG TGTACAATAA TACATCCATT GTTCAGGATG AGATTCTTGC TCACCGTCTG 360

GGGCTCATTC CCATTCATGC TGATCCCCGT CTTTTTGAGT ATCGGAACCA AGGAGATGAA 420

GAAGGCACAG AGATAGATAC TCTACAGTTT CGTCTCCAGG TCAGATGCAC TCGGAACCCC 480

CATGCTGCTA AAGATTCCTC TGACCCCAAC GAACTGTACG TGAACCACAA AGGCTGATCT 540

MTTTCCAGAG GGCACTATCC GACCAGTGCA TGATGATATC CTCATCGCTC AGCTGCGGCC 600

TGGCCAAGAA ATTGACCTGC TCATGCACTG TGTCAAGGGC ATTGGCAAAG ATCATGCCAA 660

GTTTTCACCA GTGGCAACAG CCAGTTACAG GYTCCTGCCA GACATCACCC TGCTTGAGCC 720

CGTGGAAGGG GAGGCAGCTG AGGAGTTGAG CAGGTGYTTC TCAMCTGGTG TTATTGAGGT 780

GCAGGAAGTC CAAGGTAAAA AGGTGGCCAG AGTTGCCAAC CCCCGGCTGG ATACCTTCAG 840

CAGAGAAATC TTCCGGAATG AGAAGCTAAA GAAGGTTGTG AGGCTTGCCC GGGTTCGAGA 900

TCATTATATC TTCTCTGTTG AGTCAACGGG GGTGTTGCCA CCAGATGTGC TGGTGAGTGA 960

AGCCATCAAA GTACTGATGG GGAAGTGCCG GCGCTTCTTG GATGAACTAG ATGCGGTTCA 1020

GATGGACTGA GCTTGGATGC TTCTGAGGCA AGCTGAAGCT TTGGGTTCTG ACTGACCCAC 1080

CCTACAGGAC TGCTGAACAG AGAGCCCAGT GTGACTAGGG ATCCTGAGTT TTCTGGGACA 1140

ATTCCAGCTT TAATCAATAC ATTTTGTTAA ATGTGCCATA AAATGAGACT TTTTACGCCT 1200

TTATAAGGCC TTAGATGTAA ATAAACTCAC CCAAACAAAA AAAAAAAAAA AAAACTCGA 1259

(2) INFORMATION FOR SEQ ID NO: 91:

(i) SEQUENCE CHARACTERISTICS:
(A) LENGTH: 1566 base pairs
(B) TYPE: nucleic acid
(C) STRANDEDNESS: double
(D) TOPOLOGY: linear

(xi) SEQUENCE DESCRIPTION: SEQ ID NO: 91:

CTAGAAGAGC AAGCCCGCCA GNANTGATGA AAACTGATTT TCCTGGAGAC CTTGGCAGTC 60

AGCGACAAGC TATTCCAACA ACTAAGAGAT CAGGACTCCA GTAGCAGTGA GTTCTGCACC 120

TTCTGGTGAC AGTGAGGGTG ATGAAGAGGA GACGACACAA GATGAAGTCT CTTCCCACAC 180

ATCAGAGGAA GATGGAGGGG TGGTCAAAGT GGAGAAAGAG TTAGAAAATA CAGAACAGCC 240

TGTTGGTGGG AACGAAGKGT TAGAGCACGA GGTCACAGGG AATTTGAATT CTGACCCCTT 300

GCTTGAACTC TGCCAGTGTC CCCTCTGCCA GCTAGACTGC GGGACCGGGA GCAGTTGATT 360

GCTCACGTGT ACCAGCACAC TGCAGCAGTG GTGAGCGCCA AGAGCTACAT GTGTCCTGTC 420

TGTGGCCGGG CCCTTAGCTC CCCGGGGTCA TTGGGTCGCC ACCTCTTAAT CCACTCGGAG 480

GACCAGCGAT CTAACTGTGC TGTGTGTGGA GCCCGGTTCA CCAGCCATGC CACTTTTAAC 540

AGTGAGAAAC TTCCTGAAGT ACTAAATATG GAATCCCTAC CCACAGTCCA CAATGAGGGT 600

CCCTCCAGTG CTGAGGGGAA GGATATTGCC TTTAGTCCTC CAGTGTACCC TGCTGGAATT 660

CTGCTTGTGT GCAACAACTG TGCTGCCTAC CGTAAAMTGC TGGAAGCCCA GACTCCCAGT 720

GTASGCAAGT GGGCTCTACG TCGACAGAAT GAGCCTTTGG AAGTACGGCT GCAGCGGCTG 780

GAACGAGAGC GCACGGCCAA GAAGAGCCGG CGGGACAATG AGACCCCCGA GGAGCGGGAG 840

GTGAGGCGCA TGAGGGACCG TGAAGCCAAG CGCTTGCAGC GCATGCAGGA GACAGACGAG 900

CAGCGGGCAC GCCGGCTGCA GCGGGATCGG GAGGCCATGA GGCTGAAGCG GGCCAATGAA 960

ACCCCGGAAA AGCGGCAGGC CCGGCTCATC CGAGAGCGAG AGGCCAAGCG GCTCAAGAGG 1020

AGGCTGGAGA AAATGGACAT GATGTTGCGA GCTCAGTTTG GCCAGGACCC TTCTGCCATG 1080

GCAGCCTTAG CAGCTGAAAT GAACTTCTTC CAGCTGCCTG TAAGTGGGGT GGAGTTGGAC 1140

ARCCAGCTTC TGGGCAAGAT GGCCTTTGAA GAGCAGAACA GCAGYTYTCT GCACTGAACC 1200

ACACCCTCCT GCCTGCCCTC CTTCCCACCT ACCTACCCAC CCACCCACAC CCACAGCCAC 1260

GAGGACCAGT GCTGCTGCCA CCCACGAGGC CCTGTCCTTG CTGCCAGAGG CAGGCCTGGG 1320

TTTATTGCAG GTGGACCTGA GCAGCCCTTG CATATGGGAA CAGGATGATG GGGTCAGGAG 1380

GGACCTGGCT CAAGGCAGCT CTGGACAAGG GAGCAGGCAG TCCAGAGAAC TGGCCTCCCC 1440

AGCCCACTGC CACAGGCTGT GCTTCTAGGA CTGTGGGCCC CTGTGTGGCC CATGAAGTTG 1500

TGAAGTCAAA TAAATTAATT TTATCTTTAA AAAAAAAAAA AAAAAAYYGG GGGGTTTTTT 1560

TGGGGG 1566

(2) INFORMATION FOR SEQ ID NO: 92:

(i) SEQUENCE CHARACTERISTICS:
(A) LENGTH: 1593 base pairs
(B) TYPE: nucleic acid
(C) STRANDEDNESS: double
(D) TOPOLOGY: linear

(xi) SEQUENCE DESCRIPTION: SEQ ID NO: 92:

GGCACGAGCC TCGGCCTCGG TGGCGGTGGT GGACACGTCG AGCCGGGTAG AAGTGGAGGG 60

GCCGTTCGAA GAGTCGTGAG GGGGTGACGG GTTAAGATTC GGAGAGAGAG GTGCTAGTGG 120

CTGGACTTGA CCTGGAAAGA ATCTTCTGCT GACTCTCAAC TTTTCCTGGA AAAAATGGAT 180

CATTCCCACC ATATGGGGAT GAGCTATATG GACTCCAACA GTACCATGCA ACCTTCTCAC 240

CATCACCCAA CCACTTCAGC CTCACACTCC CATGGTGGAG GAGACAGCAG CATGATGATG 300

ATGCCTATGA CCTTCTACTT TGGCTTTAAG AATGTGGAAC TACTGTTTTC CGGTTTGGTG 360

ATCAATACAG CTGGAGAAAT GGCTGGAGCT TTTGTGGCAG TGTTTTTACT AGCAATGTTC 420

TATGAAGGAC TCAAGATAGC CCGAGAGAGC CTGCTGCGTA AGTCACAAGT CAGCATTCGC 480

TACAATTCCA TGCCTGTCCC AGGACCAAAT GGAACCATCC TTATGGAGAC ACACAAAACT 540

GTTGGGCAAC AGATGCTGAG CTTTCCTCAC CTCCTGCAAA CAGTGCTGCA CATCATCCAG 600

GTGGTCATAA GCTACTTCCT CATGCTCATC TTCATGACCT ACAACGGGTA CCTCTGCATT 660

GCAKKAGCAG CAGGGGCCGG TACAGGATAC TTCCTCTTCA GCTGGAAGAA GGCAGTGGTA 720

GTGGATATCA CAGAGCATTG CCATTGACAT CAAACTCTAT GGCGTGGCCT TATCGATTGC 780

AGTGGGAAGT TGTTGAAGAC TTGAAGACGT GATTCCTGCT CCAATCATCC CTTCTTGCTC 840

CTCTTTGKGC ACGTACACAC ACACACACAC ACACACACAC ACACACCCGT GYTCAAACAG 900

AGGTTTAGTT TACAGTCTCT GAACTAAAGT AGTAACCTCC CAAATTGTTT TTTCTAATAA 960

GCTGAGATTC CCATTTCTCT TAAGGAGAAG CCACCCATGA GATGTCTTTT CCTTCTCCAT 1020

CATCTTAGAG CCAAGTTATA TGTTCTTGTC TAATCCATGT AGCTTTTTGT TCAATGACTT 1080

GATCATCTGC TTCCTTTTTG AATTTTTAAC AGATAGTAAG TAAATTTGGT GGTTTTTTCC 1140

CCTGGGTCAG TGATGGAAAG GGGTTAACTT CAGCCAGGAT TGATGGCAGC TGAGGGAAAT 1200

TCTTGCCCAA CTAAACCCAG AACTCAAACT TAACATTAGA AAATAAGGTC CAGGGCCGGA 1260

CACAGTGGCC CAAGCAAGTA ATCCCAGCAC TTTGGGGGGC CAAGGCAGGC TGGATCACCT 1320

GAGGACAGGA GTTCGAGACC AGTCTGGCCA ACATGGGGAA ACCCCGTCTC TACTAAAAAT 1380

ACATAAATTA GCCGGGCATG GTGGTGGGCG CCTGTAATCC CAGCTACTCA GAAGGCTGAG 1440

GCAGGAGAAT CACTTGAACA TAGGAGGCGG AGGTTGCAGT GAGCCAAGAT GGCGCCATTG 1500

CACTCCAGCC TGGGTGACAA GNGTGAAACT CCATCTCATA AAAAAAAAAA AAAATANTCG 1560

AGGGGGGGCC CGGACCCAAA ACGCCGGAAA GTG 1593

(2) INFORMATION FOR SEQ ID NO: 93:

(i) SEQUENCE CHARACTERISTICS:
(A) LENGTH: 970 base pairs
(B) TYPE: nucleic acid
(C) STRANDEDNESS: double
(D) TOPOLOGY: linear

(xi) SEQUENCE DESCRIPTION: SEQ ID NO: 93:

CTCGTGCCGA ATTCGGCACG AGGTGCCCAG GCTCTCAGGG CAGAGGGTCC AGTGTGATCA 60

CTTTGCATGG CCTCTCTCCC CTCCTGAGCT TGTGCCAGGG CCCCAGGGCT GACCTGGAGA 120

GGAAAAWGGC AGAGGGTGAA GATGGGGTGT CTGGTTTGGG GACCATCCTG GCCCCCCTTG 180

TCACTGTTGG CATCTCTTCT GCACAGTGGC ATTGCTGGGA GGTGCTTACT GTGCCTATTC 240

AAGGGGCTGG CAGCCGCAGC CTCACTGCAG ATCAGGGACT TGGCTTCCCG GTTGACCACA 300

GGTCCAAGAA CCTGCAGGGT CCAGCCTCCC CCCCATCCCC AGTCTTCCCC ACCCTGGCCC 360

GGCCCTCCAG GTGCAGAAAC ATGCAGGCCC CTCTCCAGGA CTGTGGGAGG AGTGTGTCCC 420

TCAGACTGGC CTGTGTCCTG GCTCCTCTTA CCACCTCTTC CAGAGGTTGT CACCTGCAGC 480

TGCCCCAGGA TAAAGGCAAG GCCAGAGAGG ACTCCTGAAC TCCTGTGTGC CTGGGGTGGC 540

AGGGGCAAAC ATAGCCAACT GGTGGCCTGA GCGGGGCCAT GGTGARGACA CCCTTGGTGG 600

CTTGTCCCAC ATCAAGCTGG GARGTGACAC TGAGGATGCA TTAGTCTGCA GCGTATGATA 660

AAAACGGCAT TTCAGGCCAG GCGTGGTGGC TCATGCCTGT CACCCCAGCA CCTTGGGAGG 720

CCGAGGTGGG CAGATCACAT GAGGTCAGGA CTTTGAGACC AGCCTGGCCA ACATGGTGAA 780

AACTCATCTG TACTAAAAAA ACAAAAATTA TGTGGGTTGG TGGTGTGTGC CTGTAATCCC 840

AGCTACTTGG GAGGCTGAGG CAGGAGAATC ACTTGAACCT GGGAGGCGGA GGCTACAACG 900

AGCCGAGATT GCACCACTGC ACTCCAGCCT GATCCGTCTC AAAAAAAAAA AAAAAAAAAA 960

AAAAACTCGA 970

(2) INFORMATION FOR SEQ ID NO: 94:

(i) SEQUENCE CHARACTERISTICS:
(A) LENGTH: 934 base pairs
(B) TYPE: nucleic acid
(C) STRANDEDNESS: double
(D) TOPOLOGY: linear

(xi) SEQUENCE DESCRIPTION: SEQ ID NO: 94:

TCTCTCTCTC TCTCTCTCTC TCTGCTGTAA AGAACTCCCA AAACTCAAAT GTATCAGGAA 60

ATGTAAAGGT TAAGTCTGAC TACAAGAAGG CCAAAATTGC ACCAGCTTCC TAAGTGAAGA 120

ATAATAGAAT AAAACATATA GAGGGCAGAA ATAAAATGAG GTGTATCTGG AGAATTTCAT 180

GATGAGCATT TAGATTTAGC AATGCCCAAT GTCATGCTGA CACTGTTTGT CATGACCTTG 240

TCTTCAGCTA GTAATTTGGG GTTGTACTTT TTTAAATTTA ATTTTGAATG TTCTTGCATG 300

TTTGGTACCT CTCTCCTCAC TGCTAAAGAT AAATTGTTTA TCTGTATAAC ATAACTACAC 360

CAATGTCATT TATTGTATAC GCTAGTACAC AAATGTGTTT TTTTATTAAG TAATGAARTA 420

TTTGCTGTGA AAAATGTATT ATTTGTGCCA CCGTTTATAT CTGTGTTCAT TTTCTGTGTG 480

TATATGCGTG TGTATTCGAA TCTCAATTTT TCTTTTACTC TAGTTTAGAT TAAGACATAT 540

TTAGATGAAA TTTTAAAAAT AACATTGGAA ATAGGAGGCT AAGTTTTGTT SAGTCTCATT 600

CCCTTGGGGG GAAATTGCTT TTGCCATTTT ATTTTCATGT ACAATAACCT AAAAAGGATC 660

TCCTACTGAC TTCCTTCCTA ATTATTATTG TTTTACACGA AAGAAAGGAA ATACGTTTTC 720

AATTGAGTTG TTTGAAATCA TTCACTTTGT GTAGATTTCC CAGACTGATG TTTCATTGTA 780

AGAATATTAC ATTATAGACA GGTTGGCCAT TTCACAAGCA ACTAATCCAT AGTTTTGGAA 840

GCCCGCTTTA AGAGACCTGA ATATCTTTGT TTTTAATAAA ATACTTAGAG TTTAAAAAAA 900

AAAAAAAAAA AAAAAAAAAA AAAAAAAAGG TAAA 934

(2) INFORMATION FOR SEQ ID NO: 95:

(i) SEQUENCE CHARACTERISTICS:
(A) LENGTH: 1392 base pairs
(B) TYPE: nucleic acid
(C) STRANDEDNESS: double
(D) TOPOLOGY: linear

(xi) SEQUENCE DESCRIPTION: SEQ ID NO: 95:

CAGCTCAGCT CTGCGCTGCT GCACGCCAAC CACACACTCA GCACCATTGA CCACCTGGTG 60

TTGGAGACGG TGGAGAGGCT GGGCGAGGCG GTGAGGACAG AGCTGACCAC CCTGGAGGAG 120

GTGCTCGANC CGCGCACGGA GCTGGTGGNT GCCGCCCGAG GGGCTCGACG GCAGGCGGAG 180

GCTGCGGCCC AGCAGCTGCA GGGGCTGGCC TTCTGGCAGG GAGTGCSCCT GAGCCCCCTG 240

CAGGTGGCTG AAAATGTGTC CTTTGTGGAG GAGTACAGGT GGCTGGCCTA YGTCCTCCTG 300

CTGCTCCTGG AGCTGCTGGT CTGCCTCTTC ACCCTCCTNG GCCTGGCGAA CAGAGCAAGT 360

GGCTGGTGAT CGTGATGACA GTCATGAGTC TCCTGGTTCT CGTCCTGAGC TGGGGCTCCA 420

TGGGCCTGGA GGCAGCCACG GCCGTGGGCC TCAGTGACTT CTGCTCCAAT CCAGACCCTT 480

ATGTTCTGAA CCTGACCCAG GAGGAGACAG GGCTCAGCTC AGACATCCTG AGCTATTATC 540

TCCTCTGCAA CCGGGCCGTC TCCAACCCCT TCCAACAGAG GCTGACTCTG TCCCAGCGAG 600

CTCTGGCCAA CATCCACTCC CAGCTGCTGG GCCTGGAGCG AGAAGCTGTG CCTCAGTTCC 660

CTTCAGCGCA GAAGCCTCTG CTGTCCTTGG AGGAGACTCT GAATGTGACA GAAGGAAATT 720

TCCACCAGTT GGTGGCACTG CTACACTGCC GCAGCCTGCA CAAGGACTAT GGTGCAGCCC 780

TGCGGGGCCT GTGCGAARAC GSCCTGGAAG GCCTGCTCTT CCTGCTGCTC TTCTCCCTGC 840

TGTCTGCAGG AGCGCTGGCC ASTGCCCTMT GCAKCCTGCC CCGAGCSTGG GCCCTCTTCC 900

CACCCAGGAA TCCAAGCGCT TTGTGCAGTG GCAGTCGTCT ATCTGAGCCC CTCCTCCCGG 960

CTGGACTGGA GCCTGGCTCC CCTCTTCGTT CCTTCCCTGG CTGCCGGAGA GACCCCACTA 1020

ACCCAGCCTG CCTGGGCTCT GACCACTAAC ACTCTTGGCC ATGGACAGCC TGCACAGGAC 1080

CGCCTCCCTG CTCTTGGCCA CTGTGCTCCC ATTTCTGTCC TTGGCCTTGG GAGTAGCTGA 1140

GGGGGCAGAC TAGGGAGTAG GGCTGGCAGG GGAGGGGGCA GACAGCCTCG CCTCGCACCC 1200

TTCATCCCTG GCTGCCGGTC CCATCCTTGG AGGGACTAAG CTGGGGGTGG GACATGAGTC 1260

CCCCTGCTGC CCCTGCCACA TCCCAGTGGG CTCTGACCCC CTGATCTCAA CTCGTGGCAC 1320

TAACTTGGAA AAGGGTTGAT TTAAAATAAA AGGGAAGACT ATTTTACAAA AAAAAAAAAA 1380

AAAAAAACTC GA 1392

(2) INFORMATION FOR SEQ ID NO: 96:

(i) SEQUENCE CHARACTERISTICS:
(A) LENGTH: 1963 base pairs
(B) TYPE: nucleic acid
(C) STRANDEDNESS: double
(D) TOPOLOGY: linear

(xi) SEQUENCE DESCRIPTION: SEQ ID NO: 96:

GGTANCTGCA GTACGGTCCG ATTCCCGGGT CGACCCACGC GTCCGGAGAA ATGCAAATTA 60

AAACAGTAAA GTGTCATTTT CACTTCCTGG ATTGGCAAAG GGTTTTATGT ATTTTACTGA 120

CAGTGCTCAA CATTAGCAGT AAACAACAAA TGGTGAGTAA ATATGAGCTT CGGAACCTCA 180

GGGAAATGAT CTCCTTATTT CAACCTGCAG ATTCCTTCCT ACAACCAGTG TAGAGCAGAG 240

TACCAGGACG GGCCATTGAG CACCCTGGTG TTGAGATCAA GTGGCCTCTA GTCAGAGTTG 300

GGTCAGGGCC ACTGTGAGTG GGCTGCCCCC AACATGAGTC AGCTGTCTAG GACTAGTTTA 360

TCTCTGCTTC TCACTTTACT GGTATTATGG GGCAGCTCCT GCTGTCTTCC AATTTGGTGT 420

CTTCCAAATC GGCACCGTCT TTTAAAGTTG AGTTTCTTGT TATTCTCACC TGATATACCT 480

TATTTATCCC ACACCCACCC CAATAACATA TCGTGCTCAG TGTTATCTTT GAGACAACAC 540

TTGAATTTTA CTCAGCCTGG AGCGCTCTTC ACATGTCTTG TCCAGATCCA GTTCGGACTC 600

ATTCTTCAGC CGTGCATCAG TAAATGGGGG CTAGGTTAAA CTGTGGTGAC AAACAACCTC 660

CAAATTTCAG TGGCTCAAAA ATCTTCTTCC TCATTTATWT ACATTTCATC ATGGGTCAGG 720

TGAGAGGTAG CTCTGTGCTG TGTCATCCTA ACACAGGAAT CCAGACGGAA GGAGGGACAA 780

TCAATAAGAT CCCCATTGCT ATAGAAAAGA RAAAAAAGTA TGCGGAATAR CACTCYGTTT 840

CYTGGAGAWT YCTCCTGAAA AAGTCACATG TTATTTCTTC TCACCTCCAT TGGCAAAAAA 900

AAAGTCATGT GGCCATGTGA AAATGTAAGT AGGCGGGATG GAACAGTCAG AATGCATTCA 960

TAAAATATGA ACTGAAAATA TCTGGAGAAC AKCACCTATG ACTACCACGA ATGCCAACAT 1020

GCATCCCTAA CAACCCAGTG CTGTCACCCT CCAAACTTTT TATGTCTTGC AAAGTATTAG 1080

AACTTCTTAT CTGAAGCCAT ACCACTCAGA GGGAANGCAA AATACATATT GACATCTCCT 1140

TTAGGATGTC CTTAGAGAAT TCAAGGAAAA GAAGTTAAAT AATTTTAAAG TGCTTTTGGG 1200

TACAGCTATT TAGCACTAGA GGGTAAGATT AGACATAGAT TGTAAAGATA ATNATAGGGT 1260

TAGGGATAGG ATTAGGATCT GGGTCAGAGT CAGGSCCAGA AGTATGGTTA GAGGTGGGGT 1320

CATGGTCAGG GTSGAGATCA AAGTCAGGGT CAAAGTAAGG GTCAGAATTA GGGACCCAGG 1380

ATAGGGATCA GGATTTAGGT TCAGTGTCAA AGTCTTGGGA CAAGGTTAGG GTTAGAATTA 1440

GAACCAGAGC TTTGTTCTCC TCAGGACCCA CCCGAGGGTG GGTCACCATG GCTTTGGAGC 1500

GCCTGGTAGT GTGGTGTGTC CACAGKGAAG ACCAGAGTTT CATTGTCCTT AAGACTGACY 1560

TGGGGAGATG TGGCTGTAGS CCATTGAGGA AGGTGAGGCA ACAGCTTCCT GTCTGCTYCC 1620

CCGTGTGCTG AGGAGGGAGT TCTGCCATGG GCTTTACTTT CACATGTTAT ATTCCACAAG 1680

TCTTGTTTTA CAAAAGCATC CCTTCCTTGA GGCTTCGGCT GCTCATCGCT GCTCATCATM 1740

ATAGCGTGCC ATAACATATA GTAAGATTTG GGTTTGTTTC TGGGGAGATA TCTTGGTATA 1800

GAGAAAGGAG AAATGCTTAG AGCCACCATC AGGACAGTTG GGATGAAAGT TGGGTATAGG 1860

CAGAGGCTGG AGGAAACATG TGCATCCCCT GTAAACACTT TTATTCATGT TTTAATTACT 1920

CATTTTTCTT ACAGTGTTAA ATTAGTAAAG ATAGTATTGA AAA 1963

(2) INFORMATION FOR SEQ ID NO: 97:

(i) SEQUENCE CHARACTERISTICS:
(A) LENGTH: 1052 base pairs
(B) TYPE: nucleic acid
(C) STRANDEDNESS: double
(D) TOPOLOGY: linear

(xi) SEQUENCE DESCRIPTION: SEQ ID NO: 97:

TCATTAACTT CAGACAACAT CATAAAGCAA TGATAGCTCT TTTCTTTGTG ACCACAAYCT 60

TAACTTGAGC TTTGCTGGGT GTTTTGCACA TAACAATGAG GGACTATTAG ACATAACATA 120

ATTTTCATAG GTCATTGCCC TGTCAATGAT AGAGAAGATA ATTGCMAGAK AGTTWATTTC 180

TGGTGTGTGT ATATGTGCAC AAATGTGCAG GGCCTCTACT TTGCAACTGG AATTTATAGA 240

CTAATGATAA AATATATCCC TTTAAATATA CAAATGACAA TTGACTTCAA ACTTTCCCAA 300

GCCCACATAG AAATTCCCTG AAAACATATA AAATATTGAG TTCTTCAACC TCAGCACTAT 360

TGACATTTTG GACCARATAG TTCTGTWTGT KAAAGGCKGT CTTTGCACTG TAGAATGTTT 420

AGCAATATTC CAGGCCTCTA TCCACCTGAT ACCGGGCCTG TATCCCCCTG ATACTGGTAG 480

TTCTTTTTTC CCCCATCACA AATTGTGACA ACCCAGAAAT ATCTCCTTAT ACCTTTCCAG 540

AATGTTTTCC CTGGGGGACA AAAAGCACTC CCATTGAAAA ATCCACTGGT CCCAAATGGT 600

TAAAAATTGG TTCCCTTCCC ATTCCTTTTA CCAGGTTTGG GGCCAAGCCC CCTTCCCTTA 660

ATTTCCCTCC CGAAATGAAC TGAAACCCAA CTGTWACTCT TAATGAAATA TTGAAGGKTT 720

GAAGCTTTAA AAAAAAAAAA AAAAKTACAG CTTGGCTGGG TGCAGTGGCT CAAGCCTGTA 780

ATCCTAGCAC TTTCGGAGGC CAAGGTGGGC AGATTGCCTG AGCTCAGGAG TTCGACACCA 840

GCGTGGGCAA CATGGTGAAA CTCTGTCTCT ACTAAAATAC AAAAAGTTAA CCTGGCATGG 900

TGGCAGGTGC CTGTAGTCCC AGCTACTAGG GAGGCTGAGG CAGGAGAATT GCTTGAACCC 960

AGGAGGCAGA GGTTGCAGTG AGCCAAGATT GCCACTGCAC TCCAGCCTGG GCAACATAGC 1020

AAGACTCTGT CAAAAAAAAA AAAAAAACTC GA 1052

(2) INFORMATION FOR SEQ ID NO: 98:

(i) SEQUENCE CHARACTERISTICS:
(A) LENGTH: 929 base pairs
(B) TYPE: nucleic acid
(C) STRANDEDNESS: double
(D) TOPOLOGY: linear

(xi) SEQUENCE DESCRIPTION: SEQ ID NO: 98:

ATCCATCACA GCCTTTCTAT CTAGGCCACA CTATAAAATC TGGAGACCTT GAATATGTGG 60

GTATGGAAGG AGGAATTGTC TTAAGTGTAG AATCAATGAA AAGACTTAAC AGCCTTCTCA 120

ATATCCCAGA AAAGTGTCCT GAACAGGGAG GGATGATTTG GAAGATATCT GAAGATAAAC 180

AGCTAGCAGT TTGCCTGAAA TATGCTGGAG TATTTGCAGA AAATGCAGAA GATGCTGATG 240

GAAAAGATGT ATTTAATACC AAATCTGTTG GGCTTTCTAT TAAAGAGGCA ATGACTTATC 300

ACCCCAACCA GGTAGTAGAA GGCTGTTGTT CAGATATGGC TGTTACTTTT AATGGACTGA 360

CTCCAAATCA GATGCATGTG ATGATGTATG GGGTATACCG CCTTAGGGCA TTTGGGCATA 420

TTTTCAATGA TGCATTGGTT TTCTTACCTC CAAATGGTTC TGACAATGAC TGAGAAGTGG 480

TAGAAAAGCG TGAATATGAT CTTTGTATAG GACGTGTGTT GTCATTATTT GTAGTAGTAA 540

CTACATATCC AATACAGCTG TATGTTTCTT TTTCTTTTCT AATTTGGTGG CACTGGTATA 600

ACCACACATT AAAGTCAGTA GTACATTTTT AAATGAGGGT GGTTTTTTTC TTTAAAACAC 660

ATGAACATTG TAAATGTGTT GGAAAGAAGT GTTTTAAGAA TAATAATTTT GCAAATAAAC 720

TATTAATAAA TATTATATGT GATAAATTCT AAATTATGAA CATTAGAAAT CTGTGGGGCA 780

CATATTTTTG CTGATTGGTT AAAAAATTTT AACAGGTCTT TAGCGTTCTA AGATATGCAA 840

ATGATATCTC TAGTTGTGAA TTTGTGATTA AAGTAAAACT TTTAGCTGTG TGTTCCCTTT 900

ACTTCTGATA CTGATTTATG TTNTAACCG 929

(2) INFORMATION FOR SEQ ID NO: 99:

(i) SEQUENCE CHARACTERISTICS:
(A) LENGTH: 359 base pairs
(B) TYPE: nucleic acid
(C) STRANDEDNESS: double
(D) TOPOLOGY: linear

(xi) SEQUENCE DESCRIPTION: SEQ ID NO: 99:

ATNGGANTCC CCCCNGGCTG CAGGAAATTC CCCGGGCTGC ATGTCTAGTT CCAGTCTGCA 60

CTGGAAAGAA TTCAAATATG CACCTGGCTC CCTTCACTAT TTTGCCCTAT CCTTTGTGCT 120

CATTCTTACT GAAATCTGTC TTGTCAGCTC AGGAATGGGA TTCCCCCAGG AAGGAAAGCA 180

CTTTTCTGTT CTGGGAAGCC CAGACTGTTC ACTTTGGGGC AGGGACGAAC ATGTGCCTCG 240

TGAATTTGCT TGAAAACAGT CACCATCTTC TACCCCCATC ACTGTATAGT GAAAAACCTG 300

ATTAAAGTGG TATCTGAGAA CCAWAAAAAA AAAAAAAAAA ANCTCGAGGG GGGGCCCGG 359

(2) INFORMATION FOR SEQ ID NO: 100:

(i) SEQUENCE CHARACTERISTICS:
(A) LENGTH: 952 base pairs
(B) TYPE: nucleic acid
(C) STRANDEDNESS: double
(D) TOPOLOGY: linear

(xi) SEQUENCE DESCRIPTION: SEQ ID NO: 100:

GAATTCCCCG GGGGATCAGG GCAGCCGGGG AGGTGGCCAG GCCAGTGGCA GGCCTGTGGA 60

GACAATCCCT YAGGACTAGG GACAGGGCTG TGCCGGCCTG GGCCAGGGCC CACGGACCCG 120

CAGCTCAGGG CGCCTGCCCA CGTCGTCTGC CGGCGGTGCG CCGCGGGCGT CCCTCGCGTC 180

TCTTCACTGC ACATTGCAAT GCATTTGCGA TTCCCATTTC TCTGCTAGGA GCCAGCCTGG 240

GTTGGCGCTG CTCCCAGAGC CCGTGGGTCC CAAGANCTTG CGTTCCCTTT TGTTCCTGTC 300

CCGTTTATCA AGAACACGGG CCCCACCTGT TCACGTTGCC CGAAGGCCAC CCCAAGCCCA 360

ASCCTGCGGG GGCGTTCCCM MAYTGCCYTG RAATGCCCGG CTTNAAGTTY TTGCGCAACG 420

CMAGGAATTC AGTGTGGGGA CGGCCCCTGC CGGATTAGGC YTAGCCCTGG CCCAGGTGGT 480

GAGCGGTTTG CAGTGTCCGT TCTCATCCAC CTGATGGGCC CAGATAAAGG CCCCCGCTGT 540

CCAGCCTCCC TGGACGGCCC TCGCGGTCCC TGCAGCCCAA GATGGGACTC AGACCCTGTG 600

CCCCAGAGCT CCCCTGCCGC AGAATGGGGC CCCAGCCGGC CCCGACCGGG TCCAGGAGCA 660

CTGCTCGCCT GTACATACTG TTGCCCTAGC CCACCTGGTG CCGTGGGAGC CACCCCCAGG 720

TGCNTGGCAC AGCCCCTCCC CACTCCGCCA CGCCCCCACC CACCCCGCGT GTTTCTGCCC 780

TGTGACTCCT GGAACCTGCG TCCTCCCCAA AGCCATGGGA GGGGTGTCCT CCTCAGACCA 840

TGCCCCCAGA TGATTTTTTT AAATAAAGAA ACAAATGCAC CTGCAAAAMA AAAAAAAAAA 900

AAAAAAACTC GAGGGGGGGC CCGGTACCCA ATTCGCCCTA TAGTGAGCGA TT 952

(2) INFORMATION FOR SEQ ID NO: 101:

(i) SEQUENCE CHARACTERISTICS:
(A) LENGTH: 1545 base pairs
(B) TYPE: nucleic acid
(C) STRANDEDNESS: double
(D) TOPOLOGY: linear

(xi) SEQUENCE DESCRIPTION: SEQ ID NO: 101:

GAAAGACAAA AGGAAATAGA AGAAAGGGAA AAAAGGCGTA AAGACAGACA TGAAGCAAGT 60

GGGTTTGCAA GGAGACCGAG ATCTCCAACC GGACCTAGCA CGGTGGCGCA CAAGATCATG 120

CAGAAGTACG GCTTCCGGGA GGGCCAGGGT CTGGGGAAGC ATGAGCAGGG CCTGAGCACT 180

GCCTTGTCAG TGGAGAAGAC CAGCAAGCGT GGCGGCAAGA TCATCGTGGG CGACGCCACA 240

GAGAAAGGTG TGTCCCCAGG GAAGCGTGTG ACTAGAGGGA AAGGACTGGC CCCATCCATA 300

TCAGACATGG CCAGTCTTGA TCCTCATGTG TCAGCAGGGG GACAATGAGG CGTGTGGCCA 360

GAGGGAGAGG GCTGGCCCTG CCATCACTAG AACACAGGCC GTCCTGTTCA TATGATGCAC 420

TGCCACTTCC GTTTTGTGAA ACCAGGAATC CTGAGGCTCA TCTTTATTTT TTCAGAACAG 480

ACGTAGAGAG ATGAAGGCTT GTGGAGGAAA AGATGGTGAG AGACTTGGGC AGAAAATGAG 540

TAGTCCTCAG GAAGAAATCT TGGTTATGTG TTTAGAGCAT GAAGGACAGA GCCATATAGT 600

GTGGCAGTGA ATATACCTGC TATCTCCATC TCAGAGGTCG TCTCTACTTT TCCCTTTTGC 660

CCTTTCAGTA TAGATGTGAT TTCTGATTCT CTTACAGATT GTTTGCTTTG CGAGATCTGA 720

TGTTATGTTG CAGTCTCTTG GTAAATGATG CCTAGTTGGT GTTTTATTTT CATTTAATTT 780

TTACAGTCTG TTCTGTGTTG AGGGAATTCA GGAAAGAGAC AAACATATGT TAGCATTTTA 840

ATCAGGGAAT TAAGTTTGAG TCAGCCTAGC TGAACTTCCT TTGCTAAAGA AAGAAGAAAA 900

CTTTTCTGGC AGCCCCGTTC ATGCACAGCT TAGGATACAT CACGAGCCTG ACAGATGCAT 960

CCAAGAAGTC AGATTCAAAT CCGCTGACTG AAATACTTAA GTGTCCTACT AAAGTGGTCT 1020

TACTAAGGAA CATGGTTGGT GCGGGAGAGG TGGATGAAGA CTTGGGAAGT TGAAACCAAG 1080

GAAGAATGTG NAAAAATATG GCAAAGTTGG AAAATGTGTG ATATTTGAAA TTCCTGGTGC 1140

CCCTGATGAT GAAGCAGTAC GGATATTTTT AGAATTTGAG AGAGTTGAAT CAGCAATTAA 1200

AGCGGTTGTT GACTTGAATG GGAGGTATTT TGGTGGACGG GTGGTAAAAG CATGTTTCTA 1260

CAATTTGGAC AAATTCAGGG TCTTGGATTT GGCAGAACAA GTTTGATTTT AAGAACTAGA 1320

GCACGAGTCA TCTCCGGTGA TCCTTAAATG AACTGCAGGC TGAGAAAAGA AGGAAAAAGG 1380

TCACAGCCTC CATGGCTGTT GCATACCAAG ACTCTTGGAA GGACTTCTAA GATATATGTT 1440

GATTGATCCC TTTTTTATTT TGTGGTTTTT TAATATAGTA TAAAAATCCT TTTAAAAAAA 1500

CAAMAAAAAA AAAAAAAACT CGAGGGGGGG CCCGGTACCC AATTT 1545

(2) INFORMATION FOR SEQ ID NO: 102:

(i) SEQUENCE CHARACTERISTICS:
(A) LENGTH: 1322 base pairs
(B) TYPE: nucleic acid
(C) STRANDEDNESS: double
(D) TOPOLOGY: linear

(xi) SEQUENCE DESCRIPTION: SEQ ID NO: 102:

CTTCTGGGAG CGACCGCTCC GCTCGTCTCG TTGGTTCCGG AGGTCGCTGC GGCGGTGGGA 60

AATGCTGGCG CGCGCGGCGC GNGGCACTGG GGCCCTTTTG CTGAGGGGCT CTCTACTGGC 120

TTCTGGCCGC GCTCCGCSCG CGCCTCCTCT GGATTGCCCC GAAACACCGT GGTACTGTTC 180

GTGCCGCAGC AGGAGGCCTG GGTGGTGGAG CGAATGGGCC GATTCCACCG GATCCTGGAG 240

CCTGGTTTGA ACATCCTCAT CCCTGTGTTA GACCGGATCC GATATGTGCA GAGTCTCAAG 300

GAAATTGTCA TCAACGTGCC TGAGCAGTCG GCTGTGACTC TCGACAATGT AACTCTGCAA 360

ATCGATGGAG TCCTTTACCT GCGCATCATG GACCCTTACA AGGCAAGCTA CGGTGTGGAG 420

GACCCTGAGT ATGCCGTCAC CCAGCTAGCT CAAACAACCA TGAGATCAGA GCTCGGCAAA 480

CTCTCTCTGG ACAAAGTCTT CCGGGAACGG GAGTCCCTGA ATGCCAGCAT TGTGGATGCC 540

ATCAACCAAG CTGCTGACTG CTGGGGTATC CGCTGCCTCC GTTATGAGAT CAAGGATATC 600

CATGTGCCAC CCCGGGTGAA AGAGTCTATG CAGATGCAGG TGGAGGCAGA GCGGCGGAAA 660

CGGGCCACAG TTCTAGAGTC TGAGGGGACC CGAGAGTCGG CCATCAATGT GGCAGAAGGG 720

AAGAAACAGG CCCAGATCCT GGCCTCCGAA GCAGAAAAGG CTGAACAGAT AAATCAGGCA 780

GCAGGAGAGG CCAGTGCAGT TCTGGCGAAG GCCAAGGCTA AAGCTGAAGC TATTCGAATC 840

CTGGCTGCAG CTCTGACACA ACATAATGGA GATGCAGCAG CTTCACTGAC TGTGGCCGAG 900

CAGTATGTCA GCGCGTTCTC CAAACTGGCC AAGGACTCCA ACACTATCCT ACTGCCCTCC 960

AACCCTGGCG ATGTCACCAG CATGGTGGCT CAGGCCATGG GTGTATATGG AGCCCTCACC 1020

AAAGCCCCAG TGCCAGGGAC TCCAGACTCA CTCTCCAGTG GGAGCAGCAG AGATGTCCAG 1080

GGTACAGATG CAAGTCTTGA TGAGGAACTT GATCGAGTCA AGATGAGTTA GTGGAGCTGG 1140

GCTTGGCCAG GGAGTCTGGG GACAAGGAAG CAGATTTTCC TGATTCTGGC TCTAGCTTCC 1200

CTGCCAAGAT TTTGGTTTTT ATTTTTTTAT TTGAACTTTA GTCGTGTAAT AAACTCACCA 1260

GTGGCAAACC AAAAAAAAAA AAAAAAAAAA AAAAAAAAAA AAAAAAAAAA AAAAAAAAAN 1320

NN 1322

(2) INFORMATION FOR SEQ ID NO: 103:

(i) SEQUENCE CHARACTERISTICS:
(A) LENGTH: 276 base pairs
(B) TYPE: nucleic acid
(C) STRANDEDNESS: double
(D) TOPOLOGY: linear

(xi) SEQUENCE DESCRIPTION: SEQ ID NO: 103:

NNATAGCTCA ACCATGTTCC AGGAGTGTAT TCCAATCAGC TTGTTTTTTC TTAACTGGTT 60

AAAGGAATGT TGCTCATTCA CCTGCCCCAA CTCACATATT AACAATTGTT TAACTGGGAT 120

TAGATAAAAG GAAAGCTGAC TTACAGATGA ACCAAGAGGG AGCTATTTAT GCCACAGCCC 180

CCAGCCCAGT AACTTTATGT TTCTGATCTC CTGCAAAATT TTTTTATAAA AAAAGCTTAG 240

CCAGGAACTA GTAGAAAGAA TAAAGTAAAG ATGGTG 276

(2) INFORMATION FOR SEQ ID NO: 104:

(i) SEQUENCE CHARACTERISTICS:
(A) LENGTH: 381 base pairs
(B) TYPE: nucleic acid
(C) STRANDEDNESS: double
(D) TOPOLOGY: linear

(xi) SEQUENCE DESCRIPTION: SEQ ID NO: 104:

GATTAAGGTA GAAAAGTACA GAAAACACTA AATTTTCATT GTGCTGTTTC AATGTGGCAG 60

ATTCTTTAAA ATACTTCGAC ACGCTACAAT AATTAAAGGT TTTAAGAACA TTAAGATACT 120

TAAAAAATAA AAGCCCACAA TTGAATAACA AAAATGAACT TTGTTTTATT TTTTATTGGC 180

ATTAATGTAG GTTGCCGTGG TGAAAATAGT TTGAAATACT TCACAGTAAC AGTTTTKTGC 240

AGCCCTAGAG ATTAAAAACA GCAAAGTAAA TAAGCAGGAC TCTCAACGAC TCATACTCAC 300

AGACTGTTTA ATGTWATCCT ARCACTTCSG GARGCTGARG CGGGAGGATT ACTTGAGCCT 360

AGGATTTGAG ACCAGCCTGG G 381

(2) INFORMATION FOR SEQ ID NO: 105:

(i) SEQUENCE CHARACTERISTICS:
(A) LENGTH: 638 base pairs
(B) TYPE: nucleic acid
(C) STRANDEDNESS: double
(D) TOPOLOGY: linear

(xi) SEQUENCE DESCRIPTION: SEQ ID NO: 105:

TGTGGAAAAC AGTAGGAAAG CAATGAAAGA AGCTGGTAAG GGAGGCGTCG CTGATTCCAG 60

AGAGCTAAAG CCGATGGTAG GTGGAGATGA RGARGTGGCC GCCCTCCAAG AATTTCACTT 120

TCACTTCCTC TCTCTCTCTG TCTTCACTGA CTGCACTTCT TCAGGAGAAG CTTTTGTTAT 180

CTGTATCACG CAGACATGCT GCTCTTTCTG TTTGTGTGCT TACCCATCAC TTGGATGGCA 240

GAATTCTTGT CACAACTGAG ACACCTYCTA TAAAAGTAAG CTGAAAGGAA CAGCATCCTC 300

GTCAGTGCTC GGCAGGGGCG GGTAGGGGAT GATGGTTTTT TCCCTAAGGT AAAACTGCTG 360

TTGCTCTTGT TTCCTTTTTA ACTGTCAGTG TTTGGCTTTC ATCAGACTGA ACATTTTGGT 420

GTACACTTGA ACTGACGGTT TGATTTTTAT CATTTTGGAA GGTGATCATA GCAATTCCTT 480

TCAACTTGCT AAAATTCATA CTCCCCCTTT TAAAAGTATG GTTCTGCTTA CATTGCTGTC 540

CTTTTCCCTT GGCTGACTTT TTCTTCTGTT GCCTAGGTTG TACTTTTTTN TTTTTTTTNT 600

TTTTCAGTAG CAAACAAGGC TGTTTTCATC AATACCCA 638

(2) INFORMATION FOR SEQ ID NO: 106:

(i) SEQUENCE CHARACTERISTICS:
(A) LENGTH: 2246 base pairs
(B) TYPE: nucleic acid
(C) STRANDEDNESS: double
(D) TOPOLOGY: linear

(xi) SEQUENCE DESCRIPTION: SEQ ID NO: 106:

GGCACGAGGC CGGGGGAGAG TCACGCAAAT GACTTGGAGT GTTCAGGAAA AGGAAAATGC 60

ACCACGAAGC CGTCAGAGGC AACTTTTTCC TGTACCTGTG AGGAGCAGTA CGTGGGTACT 120

TTCTGTGAAG AATACGATGC TTGCCAGAGG AAACCTTGCC AAAACAACGC GAGCTGTATT 180
GATGCAAATG AAAAGCAAGA TGGGAGCAAT TTCACCTGTG TTTGCCTTCC TGGTTATACT 240
GGAGAGCTTT GCCAGTCCAA GATTGATTAC TGCATCCTAG ACCCATGCAG AAATGGAGCA 300
ACATGCATTT CCAGTCTCAG TGGATTCACC TGCCAGTGTC CAGAAGGATA CTTCGGATCT 360
GCTTGTGAAG AAAAGGTGGA CCCCTGCGCC TCGTCTCCGT GCCAGAACAA CGGCACCTGC 420
TATGTGGACG GGGTACACTT TACCTGCAAC TGCAGCCCGG GCTTCACAGG GCCGACCTGT 480
GCCCAGCTTA TTGACTTCTG TGCCCTCAGC CCCTGTGCTC ATGGCACGTG CCGCAGCGTG 540
GGCACCAGCT ACAAATGCCT CTGTGATCCA GGTTACCATG GCCTCTACTG TGAGGAGGAA 600
TATAATGAGT GCCTCTCCGC TCCATGCCTG AATGCAGCCA CCTGCAGGGA CCTCGTTAAT 660
GGCTATGAGT GTGTGTGCCT GGCAGAATAC AAAGGAACAC ACTGTGAATT GTACAAGGAT 720
CCCTGCGCTA ACGTCAGCTG TCTGAACGGA GCCACCTGTG ACAGCGACGG CCTGAATGGC 780
ACGTGCATCT GTGCACCCGG GTTTACAGGT GAAGAGTGCG ACATTGACAT AAATGAATGT 840
GACAGTAACC CCTGCCACCA TGGTGGGAGC TGCCTGGACC AGCCCAATGG TTATAACTGC 900
CACTGCCCGC ATGGTTGGGT GGGAGCAAAC TGTGAGATCC ACCTCCAATG GAAGTCCGGG 960
CACATGGCGG AGAGCCTCAC CAACATGCCA CGGCACTCCC TCTACATCAT CATTGGAGCC 1020
CTCTGCGTGG CCTTCATCCT TATGCTGATC ATCCTGATCG TGGGGATTTG CCGCATCAGC 1080
CGCATTGAAT ACCAGGGTTC TTCCAGGCCA GCCTATGAGG AGTTCTACAA CTGCCGCAGC 1140
ATCGACAGCG AGTTCAGCAA TGCCATTGCA TCCATCCGGC ATGCCAGGTT TGGAAAGAAA 1200
TCCCGGCCTG CAATGTATGA TGTGAGCCCC ATCGCCTATG AAGATTACAG TCCTGATGAC 1260
AAACCCTTGG TCACACTGAT TAAAACTAAA GATTTGTAAT CTTTTTTTGG ATTATTTTTC 1320
AAAAAGATGA GATACTACAC TCATTTAAAT ATTTTTAAGG AAAWTAAAAA GCTTAAGAAA 1380
TTTAAAATGC TAGCTGCTCA AGRGTTTTCA GTAGAATATT TAAGAACTAA TTTTCTGCAG 1440
CTTTTAGTTT GGAAAAAATA TTTTAAAAAC AAAATTTGTG AAACCTATAG ACGATGTTTT 1500
AATGTACCTT CAGCTCTCTA AACTGTGTGC TTCTACTAGT GTGTGCTCTT TTCACTGTAG 1560
ACACTATCAC GAGACCCAGA TTAATTTCTG TGGTTGTTAC AGAATAAGTC TAATCAAGGA 1620
GAAGTTTCTG TTTGACGTTT GAGTGCCGGC TTTCTGAGTA GAGTTAGGAA AACCACGTAA 1680
CGTAGCATAT GATGTATAAT AGAGTATACC CGTTACTTAA AAAGAAGTCT GAAATGTTCG 1740
TTTTGTGGAA AAGAAACTAG TTAAATTTAC TATTCCTAAC CCGAATGAAA TTAGCCTTTG 1800
CCTTATTCTG TGCATGGGTA AGTAACTTAT TTCTGCACTG TTTTGTTGAA CTTTGTGGAA 1860
ACATTCTTTC GAGTTTGTTT TTGTCATTTT CGTAACAGTC GTCGAACTAG GCCTCAAAAA 1920

CATACGTAAC GAAAAGGCCT AGCGAGGCAA ATTCTGATTG ATTTGAATCT ATATTTTTCT 1980

TTAAAAAGTC AAGGGTTCTA TATTGTGAGT AAATTAAATT TACATTTGAG TTGTTTGTTG 2040

CTAAGAGGTA GTAAATGTAA GAGAGTACTG GTTCCTTCAG TAGTGAGTAT TTCTCATAGT 2100

GCAGCTTTAT TTATCTCCAG GATGTTTTTG TGGCTGTATT TGATTGATAT GTGCTTCTTC 2160

TGATTCTTGC TAATTTCCAA CCATATTGAA TAAATGTGAT CAAGTCAAAA AAAAAAAAAA 2220

AAAAAAAATT ACTCGGTCGC AAGGGA 2246

(2) INFORMATION FOR SEQ ID NO: 107:

(i) SEQUENCE CHARACTERISTICS:
(A) LENGTH: 1105 base pairs
(B) TYPE: nucleic acid
(C) STRANDEDNESS: double
(D) TOPOLOGY: linear

(xi) SEQUENCE DESCRIPTION: SEQ ID NO: 107:

GAATTCGGCA GAGCCCACTT AGAGGAGCTA AAATAGCTAA AGGTTACATG CTTTGCCTCA 60

AATAATAGAC TTAGTGAAGA GGGTAGAAGT AGAAATRAGG TCAGCCCCCC AGAGCAGTCT 120

GGTGGCCTTR AGCAACCAGG AAGGTAAAGC CGGTACCTCA GTTAAATCAC CAAGTTTACT 180

GGAAGTGCAT ATTTTTCATG TGCCAAATTC AGTAAGTCAT GGAGCAAATG TTTATTTTGC 240

TATGCTTTAA AAAGTTGCTT GCTTCTTGTA AGTTTTCTCA GTGGAAGGGT TCCAAGTTAT 300

GACTTAATCT ATGTTTGCAG CATTGCACTG GAAACAGGAT TTGTCTGTGA AATGGCTCTG 360

TCATTTGTGG ACCACTTCTG TAGGGAGATT GTGGATTTAG GAAGGGCAGA AGCAACAGCA 420

GATATGCCTG GTGTTTGAAT GGATGTGCCT CTYTCGGAGG CAGCAAGCAG CATACCCATA 480

TTATAAAGTT TTTGATTTTC TAACATCTGA AGACAGGCAT CCAGCCTTGC AGAACAGCCA 540

GGTGTCTGTT CTATAGACTA CAGTTCCTTG TTTCCAGAAT TACGGTAACC AAATAATACA 600

CAAGGTCACC TGATTGCACT TCCCAACAAC CTGAACAAAG AGCACCTTTG CGCTTGCTGG 660

TAGGTGCTGT ACCAGACTCT TTGTAATCTG CCTTAGKTCA GRGAAGAACA AGCCATTACC 720

AGTATGGGAG TCCATCCYTA GTCAGGGCTA GTTGCTATTA TCCCTTGAAT ACTCTGCAGG 780

CATCCCACAA GACATTTGAG ACTTCATATT TGTCAAATAA TAGAAATSTG GCTGGCCTAG 840

TGGCTCATGC CTGTAATCCT AACCCTTTGG GAGGCTGATG TGGGCAGATT GCTTGAGGCC 900

AGGAGTTTGA GACCCACCTG GGCAACACAG TGACATGTTG TCTCTACAAA AAATTTAAAA 960

ATTAACTAGG CATGGTAGTG TGCCTATAGT CCCAGCTACT CCAGAGGCTG AGGCAGGAAG 1020

ATCCCTTGAG CCCAGTAATT CAAGGCTACA GTTAGCTCTG ATCCTGCCAC TGCACTCCTG 1080

TCTTGGTAAA GGAGCTAAAC CCAGT 1105

(2) INFORMATION FOR SEQ ID NO: 108:

(i) SEQUENCE CHARACTERISTICS:
(A) LENGTH: 505 base pairs
(B) TYPE: nucleic acid
(C) STRANDEDNESS: double
(D) TOPOLOGY: linear

(xi) SEQUENCE DESCRIPTION: SEQ ID NO: 108:

ATTTCACACA GGAAACAGCT ATGACCATGA TTCCGCCAAG CNCGAAATTA ACCNTCACTA 60

AAGGGAACAA AACTGGAGCT CCACCGCGGT GGCGGCCGCT CTAGAACTAG TGGATCCCCC 120

GGGCTCAGGA ATTCGGCACG AGTTCTTCCA CATGTGTGCA CCCCCAGCTT GGCCAACCCT 180

CAGCCTTGCG GTGGGGCCCG AAGCATCTTC CCTTCCGCTT GGCGTCTCTG GGATTGGGAT 240

GAGTGCCTGG CTCCCATCTC CTCCTCACCT TTTGTTGCTA TCGGCAGCTG CTGGCTCAGG 300

GGCATCCCAC CTCCGGGCTC TGGGTTCCTC TGCCCTGGAA GGGCTCCAGG ACCCGTCCCA 360

ATAACCACCC ACGGCCAGGA GRGCCAAGGC CCCGTGCTGG ATATTTAAAT TTAGGGGCCG 420

GTCTCCAGGG CGCGTAGATA AATAAATACA CTCAGCGTCA AAAAAAAAAA AAAAAAAAAA 480

AAAAAAAAAA AAAAAAAAAA CTCGA 505

(2) INFORMATION FOR SEQ ID NO: 109:

(i) SEQUENCE CHARACTERISTICS:
(A) LENGTH: 1380 base pairs
(B) TYPE: nucleic acid
(C) STRANDEDNESS: double
(D) TOPOLOGY: linear

(xi) SEQUENCE DESCRIPTION: SEQ ID NO: 109:

AATCATGAGC CTCCAGAAGA GACAGATGGC CCACCAGGAG CTGTTGCTCT GGTTGCCTTC 60

CTGCAGGCCT TGGAGAAGGA GGTCGCCATA ATCGTTGACC AGAGAGCCTG GAACTTGCAC 120

CARAAGATTG TTGAAGATGC TGTTGAGCAA GGTGTTCTGA AGACGCAGAT CCCGATATTA 180

ACTTACCAAG GTGGATCAGT GGAAGCTGCT CAGGCATTCC TGTGCAAAAA TGGGGACCCG 240

CAGACACCTA GATTTGACCA CCTGGTGGCC ATAGAGCGTG CCGGAAGAGC TGCTGATGGC 300

AATTACTACA ATGCAAGGAA GATGAACATC AAGCACTTGG TTGACCCCAT TGACGATCTT 360

TTTCTTGCTG CGAAGAAGAT TCCTGGAATC TCATCAACTG GAGTCGGTGA TGGAGGCAAC 420

GAGCTTGGGA TGGGTAAAGT CAAGGAGGCT GTGAGGAGGC ACATACGGCA CGGGRATGTC 480

ATCGCCTGCG ACGTGGAGGC TGACTTTGCC GTCATTGCTG GTGTTTCTAA CTGGGGAGGC 540

TATGCCCTGG CCTGCGCACT CTACATCCTG TACTCATGTG CTGTCCACAG TCAGTACCTG 600

AGGAAAGCAG TCGGACCCTC CAGGGCACCT GGAGATCAGG CCTGGACTCA GGCCCTCCCG 660

TCGGTCATTA AGGAAGAAAA AATGCTGGGC ATCTTGGTGC AGCACAAAGT CCGGAGTGGC 720

GTCTCGGGCA TCGTGGGCAT GGAGGTGGAT GGGCTGCCCT TCCACAACAC CCACGCCGAG 780

ATGATCCAGA AGCTGGTGGA CGTCACCACG GCACAGGTGT AACCGTCCAT GTTCCGTGTG 840

AGCAGAGTCC CTACCAACGG GCAGGTCTGC ATCCGGGGAG AATGCAGCTG CTTCTGGCGA 900

CAATCCTGCT AGTAAACACT GGTCTTCGGT GAGCAACGAA CACTCGCCTG GCCTGGGAAA 960

CTGCATGCCC ACTTTCTGGG AGGGGTTAGT GCAGGTGCCG TGGACAAAGG ACAACATTTC 1020

TCTGGGGCTT TTTAACTTTT ATTCCTAAGA CTCTAAAGGC GTTGATTTCA ACCCTCCTTC 1080

ACTCTGGCTT CTTCAGGCAA CCCACGTGGT CTCCTGTGAG AATCTTCTCG ACAGTTACTT 1140

ATGGGGACAC TTGTGAACAA TTAACTGCCA GGCAGAGCAT GAGAACAAAC ATTCCCAGGC 1200

CATGTAGGAT AGGATACTCC AGACTCCAGT CATCCTCCCC CATCCATGGT TTCTGTTACT 1260

CATGGTTTCA GTTACTCATA GCCAACTGCA GACCGAAAAT ACTAAATGAA AAATTTCAGA 1320

AATAAACAAC TCTTAAGTTT TAAAAAAAAA AAAAAAAAAA AAAAAAAAAA GGGCGGCCGC 1380

(2) INFORMATION FOR SEQ ID NO: 110:

(i) SEQUENCE CHARACTERISTICS:
(A) LENGTH: 646 base pairs
(B) TYPE: nucleic acid
(C) STRANDEDNESS: double
(D) TOPOLOGY: linear

(xi) SEQUENCE DESCRIPTION: SEQ ID NO: 110:

CAGATGCCAG GGACTTGGNC TTCCCCCGGT TGAACCACAG GTTCCAAGAA ACCTGCAGGG 60

TCCAGCCTCC CCCCCATCCC CAGTYTTCCC CACCCTGGCC CGGCCCTCCA GGTGCAGAAA 120

CATGCAGGCC CCTCTCCAGG ACTGTGGGAG GAGTGTGTCC CTCAGACTGG CCTGTGTCCT 180

GGCTCCTCTT ACCACCTCTT CCAGAGGTTG TCACCTGCAG CTGCCCCAGG ATAAAGGCAA 240

GGCCAGARAG GACTCCTGAA CTCCTGTGTG CCTGGGGTGG CAGGGGCAAA CATAGCCAAC 300

TGGTGGCCTG AGCGGGGCCA TGGTGARGAC ACCCTTGGTG GCTTGTCCCA CATCAAGCTG 360

GGARGTGACA CTTAGGATGC ATTTTTCAAT ATTTTAGTGT TTGAATAACG GGCTAWCTTG 420

AGAAAAAAAT AATTTGAATC ACACATCACA CCAAAAATAA ATTCTAGGTG GATTTTAACA 480

CTTTCCAAAA ATTATTATTA GTTTAGAGAC AGGGTCTCAC TCCGTCGCCT AGGCTGGAGT 540

GCANGGGTAT GATCATGGTT CACTGCAACC TTAAACTCCC TGGCCTCATA TGATCCCCCC 600

GGGCTCCAGC CCCTCCAAAG TTACTGGGAA ACTACCAAAC ATGCCC 646

(2) INFORMATION FOR SEQ ID NO: 111:

(i) SEQUENCE CHARACTERISTICS:
(A) LENGTH: 32 amino acids
(B) TYPE: amino acid
(D) TOPOLOGY: linear
(xi) SEQUENCE DESCRIPTION: SEQ ID NO: 111:

Met Asp Ser Tyr Trp His Ser Arg Cys Leu Lys Cys Ser Cys Cys Gln
1               5                   10                  15

Ala Xaa Trp Ala Thr Ser Ala Arg Pro Val Thr Pro Lys Val Ala Xaa
            20                  25                  30

(2) INFORMATION FOR SEQ ID NO: 112:

(i) SEQUENCE CHARACTERISTICS:
(A) LENGTH: 36 amino acids
(B) TYPE: amino acid
(D) TOPOLOGY: linear
(xi) SEQUENCE DESCRIPTION: SEQ ID NO: 112:

Ile Tyr Ser Ser Gly Tyr Phe Gln Ile Tyr Asn Met Leu Leu Leu Thr
1               5                   10                  15

Ile Leu Ile Leu Leu Cys Asn Arg Thr Pro Glu Leu Ile Pro Gly Phe
            20                  25                  30

Tyr Ile Arg Xaa
        35

(2) INFORMATION FOR SEQ ID NO: 113:

(i) SEQUENCE CHARACTERISTICS:
(A) LENGTH: 220 amino acids
(B) TYPE: amino acid
(D) TOPOLOGY: linear
(xi) SEQUENCE DESCRIPTION: SEQ ID NO: 113:

Met Ser His Lys Leu Gly Asp Pro Gly Phe Val Val Phe Ala Thr Leu
1               5                   10                  15

Val Val Ile Val Ala Leu Ile Leu Ile Phe Val Val Gly Pro Arg His
            20                  25                  30

Gly Gln Thr Asn Ile Leu Val Tyr Ile Thr Ile Cys Ser Val Ile Gly
        35                  40                  45

Ala Phe Ser Val Ser Cys Val Lys Gly Leu Gly Ile Ala Ile Lys Glu
    50                  55                  60

Leu Phe Ala Gly Lys Pro Val Leu Arg His Pro Leu Ala Trp Ile Leu
65                  70                  75                  80

Leu Leu Ser Leu Ile Val Cys Val Ser Thr Gln Ile Asn Tyr Leu Asn
                85                  90                  95

Arg Ala Leu Asp Ile Phe Asn Thr Ser Ile Val Thr Pro Ile Tyr Tyr
            100                 105                 110

Val Phe Phe Thr Thr Ser Val Leu Thr Cys Ser Ala Ile Leu Phe Lys
        115                 120                 125

Glu Trp Gln Asp Met Pro Val Asp Asp Val Ile Gly Thr Leu Ser Gly
    130                 135                 140

Phe Phe Thr Ile Ile Val Gly Ile Phe Leu Leu His Ala Phe Lys Asp
145                 150                 155                 160

Val Ser Phe Ser Leu Ala Ser Leu Pro Val Ser Phe Arg Lys Asp Glu
                165                 170                 175

Lys Ala Met Asn Gly Asn Leu Ser Asn Met Tyr Glu Val Leu Asn Asn
            180                 185                 190

Asn Glu Glu Ser Leu Thr Cys Gly Ile Glu Gln His Thr Gly Glu Asn
        195                 200                 205

Val Ser Arg Arg Asn Gly Asn Leu Thr Ala Phe Xaa
    210                 215                 220

(2) INFORMATION FOR SEQ ID NO: 114:

(i) SEQUENCE CHARACTERISTICS:
(A) LENGTH: 32 amino acids
(B) TYPE: amino acid
(D) TOPOLOGY: linear
(xi) SEQUENCE DESCRIPTION: SEQ ID NO: 114:

Met Thr Ile Trp Glu Arg Lys Tyr Ile Trp Met Leu Gln Ile Cys Val
1               5                   10                  15

Phe Leu Glu Pro Arg Ala Lys Pro Ser Leu Gly Asp Leu Asp Trp Xaa
            20                  25                  30

(2) INFORMATION FOR SEQ ID NO: 115:

(i) SEQUENCE CHARACTERISTICS:
(A) LENGTH: 27 amino acids
(B) TYPE: amino acid
(D) TOPOLOGY: linear
(xi) SEQUENCE DESCRIPTION: SEQ ID NO: 115:

Met Leu Thr Phe Leu Leu Phe Ile Pro Val Ala Pro Thr Glu Thr Ser
1               5                   10                  15

Gln Lys Asn Arg Ser Val Phe Leu Pro Pro Xaa
            20                  25

(2) INFORMATION FOR SEQ ID NO: 116:

(i) SEQUENCE CHARACTERISTICS:
(A) LENGTH: 132 amino acids
(B) TYPE: amino acid
(D) TOPOLOGY: linear
(xi) SEQUENCE DESCRIPTION: SEQ ID NO: 116:

Met Leu Phe Val Phe Cys Cys Thr Val Phe Phe Val Cys Leu Phe Val
1               5                   10                  15

Tyr Leu Val Gly Phe Leu Glu Arg Glu Ile Trp Lys Arg Asp Ile His
            20                  25                  30

Lys Ser Tyr Thr Pro Thr Phe Pro Phe Tyr His Asp Ile Gln Glu Glu
        35                  40                  45

Thr Ser Arg Ala Lys Asn Gly Val Lys Lys Gly Ser Met Ala Gly Thr
    50                  55                  60

Ser Lys Glu Leu Arg Ala Val Ala Leu Lys Asn Tyr Phe Phe Tyr Tyr
65                  70                  75                  80

Tyr Phe Glu Ser Met Glu Val Phe His Ser Leu Gly Lys Gly Gly Lys
                85                  90                  95

Ser Ala Phe Ile Phe Ile Gln Ser Tyr Leu Ile Thr Ser Lys Thr His
            100                 105                 110

Met Leu Glu Ile Ala Phe Ala Gly Ala Lys Tyr Ile Asn Glu Gln Glu
        115                 120                 125

Tyr Ile His Xaa
    130

(2) INFORMATION FOR SEQ ID NO: 117:

(i) SEQUENCE CHARACTERISTICS:
(A) LENGTH: 65 amino acids
(B) TYPE: amino acid
(D) TOPOLOGY: linear

(xi) SEQUENCE DESCRIPTION: SEQ ID NO: 117:

Met Trp Tyr Phe Met Ser Leu Ile Ser Met Val Leu Leu Leu Ser Pro
1               5                   10                  15

Ser Cys Ser Asp Leu Leu Val Ile Ser Val Leu Asn Leu Glu Gln Arg
            20                  25                  30

Arg Gln Ser Lys Val Gly Phe Glu Pro Phe Thr Ser Pro Leu Cys Gly
        35                  40                  45

Xaa Trp His His Leu Ser Pro Asp Arg Leu Pro Gln Asp Gly Thr Phe
    50                  55                  60

Xaa
65

(2) INFORMATION FOR SEQ ID NO: 118:

(i) SEQUENCE CHARACTERISTICS:
(A) LENGTH: 9 amino acids
(B) TYPE: amino acid
(D) TOPOLOGY: linear
(xi) SEQUENCE DESCRIPTION: SEQ ID NO: 118:

Leu Leu Leu Phe Cys Ile Leu Gly Xaa
1               5

(2) INFORMATION FOR SEQ ID NO: 119:

(i) SEQUENCE CHARACTERISTICS:
(A) LENGTH: 50 amino acids
(B) TYPE: amino acid
(D) TOPOLOGY: linear
(xi) SEQUENCE DESCRIPTION: SEQ ID NO: 119:

Met Gly Val Leu Phe Val Pro Gln Glu Thr Ser Xaa Lys Val Xaa Xaa
1               5                   10                  15

Asp Ile Xaa Gly Leu Ser Gln Phe Val Met Gly Glu Lys Arg Thr Thr
            20                  25                  30

Ser Ile Arg Gly Ile Gln Ala Arg Tyr Gln Val Asp Arg Gly Leu Glu
        35                  40                  45

Tyr Cys
    50

(2) INFORMATION FOR SEQ ID NO: 120:

(i) SEQUENCE CHARACTERISTICS:
(A) LENGTH: 76 amino acids
(B) TYPE: amino acid
(D) TOPOLOGY: linear

(xi) SEQUENCE DESCRIPTION: SEQ ID NO: 120:

Met Leu Leu Leu Leu Leu Leu Leu Leu Leu Leu Leu Trp Thr Cys Gln
1               5                   10                  15

Lys Ala Leu Val Arg Arg Gln Phe Cys Leu Phe Asn Leu Ile Ala Arg
            20                  25                  30

Asn Ser Ser Leu Met Leu Gln Lys Asp Glu Lys Lys Gly Lys Lys Arg
        35                  40                  45

Asp Asn Ser Gln Ala Gln Arg Glu Lys Lys Gly Gly Gly Lys Glu Pro
    50                  55                  60

Gln Gly Asp Leu Gln Glu Arg Pro Gly Pro Gly Xaa
65                  70                  75

(2) INFORMATION FOR SEQ ID NO: 121:

(i) SEQUENCE CHARACTERISTICS:
(A) LENGTH: 27 amino acids
(B) TYPE: amino acid
(D) TOPOLOGY: linear
(xi) SEQUENCE DESCRIPTION: SEQ ID NO: 121:

Met His Asn Ala Phe Asn Leu Asn Val Leu Thr Leu Phe Leu Ser Val
1               5                   10                  15

Leu Cys Cys Thr Phe Ser Asp Ser Glu Leu Xaa
            20                  25

(2) INFORMATION FOR SEQ ID NO: 122:

(i) SEQUENCE CHARACTERISTICS:
(A) LENGTH: 24 amino acids
(B) TYPE: amino acid
(D) TOPOLOGY: linear
(xi) SEQUENCE DESCRIPTION: SEQ ID NO: 122:

Met Ser Trp Leu Phe Leu Leu Phe Ala Leu Leu Cys Lys Phe Gln His
1               5                   10                  15

Lys Leu Xaa Phe His Asn Ile Xaa
            20

(2) INFORMATION FOR SEQ ID NO: 123:

(i) SEQUENCE CHARACTERISTICS:
(A) LENGTH: 22 amino acids
(B) TYPE: amino acid
(D) TOPOLOGY: linear
(xi) SEQUENCE DESCRIPTION: SEQ ID NO: 123:

Met Leu Leu Phe Leu Thr Val Ile Asn Phe Met Ala Leu Ala Lys Met

1                   5                   10                  15

Asn Phe Cys Gly Asp Xaa
            20

(2) INFORMATION FOR SEQ ID NO: 124:

      (i) SEQUENCE CHARACTERISTICS:
            (A) LENGTH: 55 amino acids
            (B) TYPE: amino acid
            (D) TOPOLOGY: linear
      (xi) SEQUENCE DESCRIPTION: SEQ ID NO: 124:

Met Val Xaa Asn Leu Gln Val Ile Ser Ile Trp Xaa Xaa Ser Thr Thr
  1               5                  10                  15

Cys Phe Tyr Ala Cys Ile Trp Xaa Gln Gly Cys Leu Met Leu Arg Xaa
            20                  25                  30

Phe Xaa Thr Leu Asn Asn Val Thr Arg Leu Pro Ser Ser Gln Lys Pro
        35                  40                  45

Ile Lys Cys Tyr Leu Leu Xaa
    50                  55

(2) INFORMATION FOR SEQ ID NO: 125:

      (i) SEQUENCE CHARACTERISTICS:
            (A) LENGTH: 318 amino acids
            (B) TYPE: amino acid
            (D) TOPOLOGY: linear
      (xi) SEQUENCE DESCRIPTION: SEQ ID NO: 125:

Met Leu Ser Glu Ser Ser Ser Phe Leu Lys Gly Val Met Leu Gly Ser
  1               5                  10                  15

Ile Phe Cys Ala Leu Ile Thr Met Leu Gly His Ile Arg Ile Gly His
            20                  25                  30

Gly Asn Arg Met His His His Glu His His His Leu Gln Ala Pro Asn
        35                  40                  45

Lys Glu Asp Ile Leu Lys Ile Ser Glu Asp Glu Arg Met Glu Leu Ser
    50                  55                  60

Lys Ser Phe Arg Val Tyr Cys Ile Ile Leu Val Lys Pro Lys Asp Val
 65                  70                  75                  80

Ser Leu Trp Ala Ala Val Lys Glu Thr Trp Thr Lys His Cys Asp Lys
                85                  90                  95

Ala Glu Phe Phe Ser Ser Glu Asn Val Lys Val Phe Glu Ser Ile Asn
            100                 105                 110

Met Asp Thr Asn Asp Met Trp Leu Met Met Arg Lys Ala Tyr Lys Tyr
        115                 120                 125

Ala Phe Xaa Lys Tyr Arg Asp Gln Tyr Asn Trp Phe Phe Leu Ala Arg
130                 135                 140

Pro Thr Thr Phe Ala Ile Ile Glu Asn Leu Lys Tyr Phe Leu Leu Lys
145                 150                 155                 160

Lys Asp Pro Ser Gln Pro Phe Tyr Leu Gly His Thr Ile Lys Ser Gly
                165                 170                 175

Asp Leu Glu Tyr Val Gly Met Glu Gly Gly Ile Val Leu Ser Val Glu
            180                 185                 190

Ser Met Lys Arg Leu Asn Ser Leu Leu Asn Ile Pro Glu Lys Cys Pro
        195                 200                 205

Glu Gln Gly Gly Met Ile Trp Lys Ile Ser Glu Asp Lys Gln Leu Ala
    210                 215                 220

Val Cys Leu Lys Tyr Ala Gly Val Phe Ala Glu Asn Ala Glu Asp Ala
225                 230                 235                 240

Asp Gly Lys Asp Val Phe Asn Thr Lys Ser Val Gly Leu Ser Ile Lys
                245                 250                 255

Glu Ala Met Thr Tyr His Pro Asn Gln Val Val Glu Gly Cys Cys Ser
            260                 265                 270

Asp Met Ala Val Thr Phe Asn Gly Leu Thr Pro Asn Gln Met His Val
        275                 280                 285

Met Met Tyr Gly Val Tyr Arg Leu Arg Ala Phe Gly His Ile Phe Asn
    290                 295                 300

Asp Ala Leu Val Phe Leu Pro Pro Asn Gly Ser Asp Asn Asp
305                 310                 315

(2) INFORMATION FOR SEQ ID NO: 126:

(i) SEQUENCE CHARACTERISTICS:
(A) LENGTH: 59 amino acids
(B) TYPE: amino acid
(D) TOPOLOGY: linear
(xi) SEQUENCE DESCRIPTION: SEQ ID NO: 126:

Met Thr Trp Pro Pro Ser Cys Leu Val Ala Leu Leu Leu Ser Thr Val
  1               5                  10                  15

Thr Gln Lys Met Thr Pro Leu Asn Leu Met Arg Thr Thr Gly Pro Ile
             20                  25                  30

Asn Ser Phe Cys Leu Leu Pro Thr Phe Phe Phe Phe Pro Ser Tyr Leu
         35                  40                  45

Pro Ser Leu Met Pro Thr Pro Thr Asp Pro Xaa
    50                  55

(2) INFORMATION FOR SEQ ID NO: 127:

(i) SEQUENCE CHARACTERISTICS:
(A) LENGTH: 99 amino acids
(B) TYPE: amino acid
(D) TOPOLOGY: linear
(xi) SEQUENCE DESCRIPTION: SEQ ID NO: 127:

Ile Leu Phe Ser Phe Leu Ile Pro Ser Asn Leu Ser Phe Ser Pro Val
1               5                   10                  15

Ile Phe Phe Leu Cys Gly Pro Phe Lys Val Val Ile Ile Cys Thr Glu
            20                  25                  30

Leu Gln Asn Val Ser Arg Ser Pro Gln Thr Thr Leu Ala Thr Val Tyr
        35                  40                  45

Cys Asn Lys Ile Thr Ser Tyr Ile Cys Arg Asn Ser Phe Gly Val Ile
    50                  55                  60

Leu Phe Phe Pro Leu Asn Ile Tyr Asn Trp Thr Asn Ala Gly Lys Lys
65                  70                  75                  80

Lys Lys Met Val Ser Lys Lys Pro Lys Ile Lys Phe Arg Gly His Gln
                85                  90                  95

Ala Phe Xaa

(2) INFORMATION FOR SEQ ID NO: 128:

(i) SEQUENCE CHARACTERISTICS:
(A) LENGTH: 29 amino acids
(B) TYPE: amino acid
(D) TOPOLOGY: linear
(xi) SEQUENCE DESCRIPTION: SEQ ID NO: 128:

Met Ser Ile Leu Leu Leu Xaa Phe Pro Ser Ala Pro Ala Pro Val Val
1               5                   10                  15

Ser Gly Gly Leu Gln Pro Trp Leu His Ser Cys Ile Xaa
            20                  25

(2) INFORMATION FOR SEQ ID NO: 129:

(i) SEQUENCE CHARACTERISTICS:
(A) LENGTH: 22 amino acids
(B) TYPE: amino acid
(D) TOPOLOGY: linear
(xi) SEQUENCE DESCRIPTION: SEQ ID NO: 129:

Met Gly Thr Ser Leu Asn Leu Gln Ile Met Ala Leu Phe Ser Gly Gln
1               5                   10                  15

Ala Met Ala Pro Arg Xaa

            20

(2) INFORMATION FOR SEQ ID NO: 130:

(i) SEQUENCE CHARACTERISTICS:
(A) LENGTH: 112 amino acids
(B) TYPE: amino acid
(D) TOPOLOGY: linear
(xi) SEQUENCE DESCRIPTION: SEQ ID NO: 130:

Met Leu Trp Leu Pro Leu Leu Ala Ala Leu Ser Pro Ser Pro Pro Gly
1               5                   10                  15

Val Ser Ser Glu Glu Glu Gln His Trp Ser Gln Ala Glu Ala Leu Pro
            20                  25                  30

Cys Trp Asp Pro Gly Ser Glu Ser Ser Pro Arg Ile Pro Gly Cys Arg
        35                  40                  45

Glu Leu Gln Ser Cys Pro Pro Pro Thr Ala Pro Ser Ala His Thr Gln
    50                  55                  60

Ser Pro Gly Gly Leu Gly Ala Lys Ala Gly Ala Ala Leu Val Pro Phe
65                  70                  75                  80

Pro Gly Pro Ser Phe Pro Thr Ser Lys Pro Lys Lys Gly Glu Ala Gly
                85                  90                  95

Ala Pro Val Pro Gln Pro His Ser Ala Leu Thr Val Pro Ser Ser Xaa
            100                 105                 110

(2) INFORMATION FOR SEQ ID NO: 131:

(i) SEQUENCE CHARACTERISTICS:
(A) LENGTH: 114 amino acids
(B) TYPE: amino acid
(D) TOPOLOGY: linear
(xi) SEQUENCE DESCRIPTION: SEQ ID NO: 131:

Met Glu Lys Pro Leu Phe Pro Leu Val Pro Leu His Trp Phe Gly Phe
1               5                   10                  15

Gly Tyr Thr Ala Leu Val Val Ser Gly Gly Ile Val Gly Tyr Val Lys
            20                  25                  30

Thr Gly Ser Val Pro Ser Leu Ala Ala Gly Leu Leu Phe Gly Ser Leu
        35                  40                  45

Ala Gly Leu Gly Ala Tyr Gln Leu Tyr Gln Asp Pro Arg Asn Val Trp
    50                  55                  60

Gly Phe Leu Ala Ala Thr Ser Val Thr Phe Val Gly Val Met Gly Met
65                  70                  75                  80

Arg Ser Tyr Tyr Tyr Gly Lys Phe Met Pro Val Gly Leu Ile Ala Gly
                85                  90                  95

Ala Ser Leu Leu Met Ala Ala Lys Val Gly Val Arg Met Leu Met Thr
            100                 105                 110

Ser Asp

(2) INFORMATION FOR SEQ ID NO: 132:

(i) SEQUENCE CHARACTERISTICS:
(A) LENGTH: 22 amino acids
(B) TYPE: amino acid
(D) TOPOLOGY: linear
(xi) SEQUENCE DESCRIPTION: SEQ ID NO: 132:

Met Ile Thr Leu Leu Ile Trp Met Leu Ala Gly Phe Ile Ala Arg Ile
1               5                   10                  15

Xaa Val Ala Leu Gln Xaa
            20

(2) INFORMATION FOR SEQ ID NO: 133:

(i) SEQUENCE CHARACTERISTICS:
(A) LENGTH: 52 amino acids
(B) TYPE: amino acid
(D) TOPOLOGY: linear
(xi) SEQUENCE DESCRIPTION: SEQ ID NO: 133:

Met Ala Gly Val Ser Glu Ile Ser Val Cys Phe Xaa Leu Leu Ser Leu
1               5                   10                  15

Phe Ser Leu Phe Cys Ser Phe Tyr Phe Pro Lys Gln Ala Thr Pro Lys
            20                  25                  30

Arg Asp Leu Phe Val Gln Glu Ser Gly Lys Gly Lys Arg Asn Thr Glu
        35                  40                  45

Ser Trp Glu Xaa
    50

(2) INFORMATION FOR SEQ ID NO: 134:

(i) SEQUENCE CHARACTERISTICS:
(A) LENGTH: 99 amino acids
(B) TYPE: amino acid
(D) TOPOLOGY: linear
(xi) SEQUENCE DESCRIPTION: SEQ ID NO: 134:

Met Thr Ser Ala Leu Thr Gln Gly Leu Glu Arg Ile Pro Asp Gln Leu
1               5                   10                  15

Gly Tyr Leu Val Leu Ser Glu Gly Ala Val Leu Ala Ser Ser Gly Asp
            20                  25                  30

Leu Glu Asn Asp Glu Gln Ala Ala Ser Ala Ile Ser Glu Leu Val Ser
        35                  40                  45

Thr Ala Cys Gly Phe Arg Leu His Arg Gly Met Asn Val Pro Phe Lys
    50                  55                  60

Arg Leu Ser Val Val Phe Gly Glu His Thr Leu Leu Val Thr Val Ser
65                  70                  75                  80

Gly Gln Arg Val Phe Val Val Lys Arg Gln Asn Arg Gly Arg Glu Pro
                85                  90                  95

Ile Asp Val

(2) INFORMATION FOR SEQ ID NO: 135:

(i) SEQUENCE CHARACTERISTICS:
(A) LENGTH: 176 amino acids
(B) TYPE: amino acid
(D) TOPOLOGY: linear
(xi) SEQUENCE DESCRIPTION: SEQ ID NO: 135:

Met Gly Ser Ala Ala Leu Glu Ile Leu Gly Leu Val Leu Cys Leu Val
1               5                   10                  15

Gly Trp Gly Gly Leu Ile Leu Ala Cys Gly Leu Pro Met Trp Gln Val
            20                  25                  30

Thr Ala Phe Leu Asp His Asn Ile Val Thr Ala Gln Thr Thr Trp Lys
        35                  40                  45

Gly Leu Trp Met Ser Cys Val Val Gln Ser Thr Gly His Met Gln Cys
    50                  55                  60

Lys Val Tyr Asp Ser Val Leu Ala Leu Ser Thr Glu Val Gln Ala Ala
65                  70                  75                  80

Arg Ala Leu Thr Val Ser Ala Val Leu Leu Ala Phe Val Ala Leu Phe
                85                  90                  95

Val Thr Leu Ala Gly Ala Gln Cys Thr Thr Cys Val Ala Pro Gly Pro
            100                 105                 110

Ala Lys Ala Arg Val Ala Leu Thr Gly Gly Val Leu Tyr Leu Phe Cys
        115                 120                 125

Gly Leu Leu Ala Leu Val Pro Leu Cys Trp Phe Ala Asn Ile Val Val
    130                 135                 140

Arg Glu Phe Tyr Asp Pro Ser Val Pro Val Ser Gln Lys Tyr Glu Leu
145                 150                 155                 160

Gly Ala Xaa Cys Thr Ser Ala Gly Arg Pro Pro Arg Cys Ser Trp Xaa

                165                 170                 175

(2) INFORMATION FOR SEQ ID NO: 136:

(i) SEQUENCE CHARACTERISTICS:
(A) LENGTH: 187 amino acids
(B) TYPE: amino acid
(D) TOPOLOGY: linear
(xi) SEQUENCE DESCRIPTION: SEQ ID NO: 136:

Met Val Leu Leu Trp Val Val Thr Cys Pro Ala Thr Met Leu Thr Glu
1               5                   10                  15

Pro Gln Asn Pro His Leu Ile Gly Phe Val Ala Tyr Ser Gly Pro Ser
            20                  25                  30

His Thr Thr Gln Pro His Lys Tyr Trp Leu Leu Leu Asp Gly Gln Ala
        35                  40                  45

Asp Pro Ala Ala Ala Glu Gly Pro Val Lys Arg Lys Ala Ala Ser Val
    50                  55                  60

Val Trp Trp Pro Gln Ala Leu Arg His Leu Ser Leu Leu Val His Cys
65                  70                  75                  80

Trp Glu Glu Ser Tyr Glu Met Asn Ile Gly Cys Gln Ser Leu Trp Ala
                85                  90                  95

Gly Gly Leu Ala Ser Ser Gly Asn Gly Trp Asp Leu Gly Val Ala Phe
            100                 105                 110

Arg Arg Asp Thr Cys Met Ser Ser Ser Ser Leu His Trp Lys Glu Phe
        115                 120                 125

Lys Tyr Ala Pro Gly Ser Leu His Tyr Phe Ala Leu Ser Phe Val Leu
    130                 135                 140

Ile Leu Thr Glu Ile Cys Leu Val Ser Ser Gly Met Gly Phe Pro Gln
145                 150                 155                 160

Glu Gly Lys His Phe Ser Val Leu Gly Ser Pro Asp Cys Ser Leu Trp
                165                 170                 175

Gly Arg Asp Glu His Val Pro Arg Glu Phe Ala
            180                 185

(2) INFORMATION FOR SEQ ID NO: 137:

(i) SEQUENCE CHARACTERISTICS:
(A) LENGTH: 288 amino acids
(B) TYPE: amino acid
(D) TOPOLOGY: linear
(xi) SEQUENCE DESCRIPTION: SEQ ID NO: 137:

Met Pro Ala His Arg Phe Val Leu Ala Val Gly Ser Ala Val Phe Asn
1               5                   10                  15

Ala Met Phe Asn Gly Gly Met Ala Thr Thr Ser Thr Glu Ile Glu Leu
            20                  25                  30

Pro Asp Val Glu Pro Ala Ala Phe Leu Ala Leu Leu Lys Phe Leu Tyr
        35                  40                  45

Ser Asp Glu Val Gln Ile Gly Pro Glu Thr Val Met Thr Thr Xaa Tyr
    50                  55                  60

Thr Ala Lys Lys Tyr Ala Val Pro Ala Leu Glu Ala His Cys Val Glu
65                  70                  75                  80

Phe Leu Lys Lys Asn Leu Arg Ala Asp Asn Ala Phe Met Leu Leu Thr
                85                  90                  95

Gln Ala Arg Leu Phe Asp Glu Pro Gln Leu Ala Ser Leu Cys Leu Glu
            100                 105                 110

Asn Ile Asp Lys Asn Thr Ala Asp Ala Ile Thr Ala Glu Gly Phe Thr
        115                 120                 125

Asp Ile Asp Leu Asp Thr Leu Val Ala Val Leu Glu Arg Asp Thr Leu
    130                 135                 140

Gly Ile Arg Glu Val Arg Leu Phe Asn Ala Val Val Arg Trp Ser Glu
145                 150                 155                 160

Ala Glu Cys Gln Arg Gln Gln Leu Gln Val Thr Pro Glu Asn Arg Arg
                165                 170                 175

Lys Val Leu Gly Lys Ala Leu Gly Leu Ile Arg Phe Pro Leu Met Thr
            180                 185                 190

Ile Glu Glu Phe Ala Ala Gly Pro Ala Gln Ser Gly Ile Leu Val Asp
        195                 200                 205

Arg Glu Val Val Ser Leu Phe Cys Thr Ser Pro Ser Thr Pro Ser His
    210                 215                 220

Glu Trp Ser Ser Leu Thr Gly Pro Ala Ala Ala Cys Val Gly Arg Ser
225                 230                 235                 240

Ala Ala Ser Thr Ala Ser Ser Arg Trp Arg Val Ala Gly Ala Thr Xaa
                245                 250                 255

Gly Pro Val Thr Ala Ser Gly Ser Gln Ser Thr Ser Ala Ser Ser Trp
            260                 265                 270

Trp Asp Leu Gly Cys Met Asp Pro Ser Thr Gly Pro Pro Thr Thr Lys
        275                 280                 285

(2) INFORMATION FOR SEQ ID NO: 138:

(i) SEQUENCE CHARACTERISTICS:
(A) LENGTH: 114 amino acids
(B) TYPE: amino acid
(D) TOPOLOGY: linear
(xi) SEQUENCE DESCRIPTION: SEQ ID NO: 138:

Met Pro Arg Cys Arg Trp Leu Ser Leu Ile Leu Leu Thr Ile Pro Leu
1               5                   10                  15

Ala Leu Val Ala Arg Lys Asp Pro Lys Lys Asn Glu Thr Gly Val Leu
            20                  25                  30

Arg Lys Leu Lys Pro Val Asn Ala Phe Xaa Cys Gln Arg Gly Ser Ser
        35                  40                  45

Val Xaa Gly Phe Ala Met Gln Glu Tyr Asn Lys Glu Ser Glu Asp Lys
    50                  55                  60

Tyr Val Phe Leu Val Val Lys Thr Leu Gln Ala Gln Leu Gln Val Thr
65                  70                  75                  80

Asn Leu Leu Glu Tyr Leu Ile Asp Val Glu Ile Ala Arg Ser Asp Cys
                85                  90                  95

Arg Lys Pro Leu Ser Thr Asn Glu Ile Ala Pro Phe Lys Xaa Thr Pro
            100                 105                 110

Ser Xaa

(2) INFORMATION FOR SEQ ID NO: 139:

(i) SEQUENCE CHARACTERISTICS:
(A) LENGTH: 120 amino acids
(B) TYPE: amino acid
(D) TOPOLOGY: linear
(xi) SEQUENCE DESCRIPTION: SEQ ID NO: 139:

Met Ser Pro His Pro Thr Ala Leu Leu Gly Leu Val Leu Cys Leu Ala
1               5                   10                  15

Gln Thr Ile His Thr Gln Glu Glu Asp Leu Pro Arg Pro Ser Ile Ser
            20                  25                  30

Ala Glu Pro Gly Thr Val Ile Pro Leu Gly Ser His Val Thr Phe Val
        35                  40                  45

Cys Arg Gly Pro Val Gly Val Gln Thr Phe Arg Leu Glu Arg Glu Ser
    50                  55                  60

Arg Ser Thr Tyr Asn Asp Thr Glu Asp Val Ser Gln Ala Ser Pro Ser
65                  70                  75                  80

Glu Ser Glu Ala Arg Phe Arg Ile Asp Ser Val Ser Glu Gly Asn Ala
                85                  90                  95

Gly Pro Tyr Arg Cys Ile Tyr Tyr Lys Pro Pro Lys Trp Ser Glu Gln
            100                 105                 110

Ser Asp Tyr Trp Ser Cys Trp Xaa
        115                 120

(2) INFORMATION FOR SEQ ID NO: 140:

    (i) SEQUENCE CHARACTERISTICS:
        (A) LENGTH: 438 amino acids
        (B) TYPE: amino acid
        (D) TOPOLOGY: linear
    (xi) SEQUENCE DESCRIPTION: SEQ ID NO: 140:

Met Asn Thr Pro Asn Gly Asn Ser Leu Ser Ala Ala Glu Leu Thr Cys
  1               5                  10                  15

Gly Met Ile Met Cys Leu Ala Arg Gln Ile Pro Gln Ala Thr Ala Ser
            20                  25                  30

Met Lys Asp Gly Lys Trp Glu Arg Lys Lys Phe Met Gly Thr Glu Leu
        35                  40                  45

Asn Gly Lys Thr Leu Gly Ile Leu Gly Leu Gly Arg Ile Gly Arg Glu
    50                  55                  60

Val Ala Thr Arg Met Gln Ser Phe Gly Met Lys Thr Ile Gly Tyr Asp
 65                  70                  75                  80

Pro Ile Ile Ser Pro Glu Val Ser Ala Ser Phe Gly Val Gln Gln Leu
                 85                  90                  95

Pro Leu Glu Glu Ile Trp Pro Leu Cys Asp Phe Ile Thr Val His Thr
            100                 105                 110

Pro Leu Leu Pro Ser Thr Thr Gly Leu Leu Asn Asp Asn Thr Phe Ala
        115                 120                 125

Gln Cys Lys Lys Gly Val Arg Val Val Asn Cys Ala Arg Gly Gly Ile
    130                 135                 140

Val Asp Glu Gly Ala Leu Leu Arg Ala Leu Gln Ser Gly Gln Cys Ala
145                 150                 155                 160

Gly Ala Ala Leu Asp Val Phe Thr Glu Glu Pro Pro Arg Asp Arg Ala
                165                 170                 175

Leu Val Asp His Glu Asn Val Ile Ser Cys Pro His Leu Gly Ala Ser
            180                 185                 190

Thr Lys Glu Ala Gln Ser Arg Cys Gly Glu Glu Ile Ala Val Gln Phe
        195                 200                 205

Val Asp Met Val Lys Gly Lys Ser Leu Thr Gly Val Val Asn Ala Gln
    210                 215                 220

Ala Leu Thr Ser Ala Phe Ser Pro His Thr Lys Pro Trp Ile Gly Leu
225                 230                 235                 240

Ala Glu Ala Leu Gly Thr Leu Met Arg Ala Trp Ala Gly Ser Pro Lys
                245                 250                 255

Gly Thr Ile Gln Val Ile Thr Gln Gly Thr Ser Leu Lys Asn Ala Gly
            260                 265                 270

Asn Cys Leu Ser Pro Ala Val Ile Val Gly Leu Leu Lys Glu Ala Ser
        275                 280                 285

Lys Gln Ala Asp Val Asn Leu Val Asn Ala Lys Leu Leu Val Lys Glu
    290                 295                 300

Ala Gly Leu Asn Val Thr Thr Ser His Ser Pro Ala Ala Pro Gly Glu
305                 310                 315                 320

Gln Gly Phe Gly Glu Cys Leu Leu Ala Val Ala Leu Ala Gly Ala Pro
                325                 330                 335

Tyr Gln Ala Val Gly Leu Val Gln Gly Thr Thr Pro Val Leu Gln Gly
            340                 345                 350

Leu Asn Gly Ala Val Phe Arg Pro Glu Val Pro Leu Arg Arg Asp Leu
        355                 360                 365

Pro Leu Leu Leu Phe Arg Thr Gln Thr Ser Asp Pro Ala Met Leu Pro
    370                 375                 380

Thr Met Ile Gly Leu Leu Ala Glu Ala Gly Val Arg Leu Leu Ser Tyr
385                 390                 395                 400

Gln Thr Ser Leu Val Ser Asp Gly Glu Thr Trp His Val Met Gly Ile
                405                 410                 415

Ser Ser Leu Leu Pro Ser Leu Glu Ala Trp Lys Gln His Val Thr Glu
            420                 425                 430

Ala Phe Gln Phe His Phe
        435

(2) INFORMATION FOR SEQ ID NO: 141:

(i) SEQUENCE CHARACTERISTICS:
(A) LENGTH: 164 amino acids
(B) TYPE: amino acid
(D) TOPOLOGY: linear
(xi) SEQUENCE DESCRIPTION: SEQ ID NO: 141:

Met Ser Arg Pro Thr His Thr Pro Leu Ser Pro Ala Thr Ile Ser Pro
1               5                   10                  15

Thr Ile Thr Val Ala Val Phe Phe Ala Val Phe Val Ala Ala Ala Ala
            20                  25                  30

Ala Thr Ala Val Val Ala Val Ala Ala Ala Thr Thr Ser Ser Gly Arg
        35                  40                  45

Arg Thr Xaa Asp Lys Ser Pro Ile Ala Thr Gln Ser Ser Val Thr His

50                  55                  60

Ile Ala Ala Lys Arg Cys His Asn Tyr Thr Glu Cys Leu Ser Leu Ile
65                  70                  75                  80

Arg Xaa Thr Arg Ile Pro Thr Trp Xaa Xaa Xaa Thr Thr Cys Pro Ser
                85                  90                  95

Arg Ile Pro Ser Thr His Val Ala Ala Gly Ala Gly Phe Ile Arg Glu
            100                 105                 110

Arg Ala Cys Leu Gln Cys Gly Ala Val Gly Pro Pro Gly Cys Ile Leu
        115                 120                 125

Ala Ser Leu Pro Pro Pro Ser Leu Tyr Leu Ser Pro Glu Leu Arg Cys
    130                 135                 140

Met Pro Lys Arg Val Glu Ala Arg Ser Glu Leu Arg Leu Cys Pro Pro
145                 150                 155                 160

Gly Val Xaa Xaa

(2) INFORMATION FOR SEQ ID NO: 142:

(i) SEQUENCE CHARACTERISTICS:
(A) LENGTH: 73 amino acids
(B) TYPE: amino acid
(D) TOPOLOGY: linear
(xi) SEQUENCE DESCRIPTION: SEQ ID NO: 142:

Met Gln Arg Trp Val Cys Ile Leu Glu Phe Lys Glu Asn Leu Phe Gln
1               5                   10                  15

Ile Pro Ser Ser Leu Val Ala Leu Leu Asn Thr Leu Phe Leu Asp Ile
            20                  25                  30

Leu His Pro Gln Asn Ser Leu Ser Pro His Gly Ser Phe Ser Leu Ser
        35                  40                  45

Ser Leu Ser Phe Pro Pro Leu Pro Val Ser Ser Leu Gln Pro Phe Leu
    50                  55                  60

Phe Leu Arg Ser Leu Leu Cys Arg Xaa
65                  70

(2) INFORMATION FOR SEQ ID NO: 143:

(i) SEQUENCE CHARACTERISTICS:
(A) LENGTH: 123 amino acids
(B) TYPE: amino acid
(D) TOPOLOGY: linear
(xi) SEQUENCE DESCRIPTION: SEQ ID NO: 143:

Phe Gly Thr Arg Phe Leu Ala Asn Leu Leu Leu Glu Glu Asp Asn Lys
1               5                   10                  15

Phe Cys Ala Asp Cys Gln Ser Lys Gly Pro Arg Trp Ala Ser Trp Asn
            20                  25                  30

Ile Gly Val Phe Ile Cys Ile Arg Cys Ala Xaa Ile His Arg Asn Leu
        35                  40                  45

Gly Val His Ile Ser Arg Val Lys Ser Val Asn Leu Asp Gln Trp Thr
    50                  55                  60

Gln Val Gln Ile Gln Cys Met Gln Xaa Met Gly Asn Gly Lys Ala Asn
65                  70                  75                  80

Arg Leu Tyr Glu Ala Tyr Leu Pro Glu Thr Phe Arg Arg Pro Gln Ile
                85                  90                  95

Asp Pro Ala Val Glu Gly Phe Ile Arg Asp Xaa Tyr Glu Lys Lys Lys
            100                 105                 110

Tyr Met Asp Arg Ser Leu Gly His Gln Cys Leu
        115                 120

(2) INFORMATION FOR SEQ ID NO: 144:

    (i) SEQUENCE CHARACTERISTICS:
        (A) LENGTH: 138 amino acids
        (B) TYPE: amino acid
        (D) TOPOLOGY: linear
    (xi) SEQUENCE DESCRIPTION: SEQ ID NO: 144:

Met Ser Leu Tyr Asp Asp Leu Gly Val Glu Thr Ser Asp Ser Lys Thr
1               5                   10                  15

Glu Gly Trp Ser Lys Asn Phe Lys Leu Leu Gln Ser Gln Leu Gln Val
            20                  25                  30

Lys Lys Ala Ala Leu Thr Gln Ala Lys Ser Gln Arg Thr Lys Gln Ser
        35                  40                  45

Thr Val Leu Ala Pro Val Ile Asp Leu Lys Arg Gly Gly Ser Ser Asp
    50                  55                  60

Asp Arg Gln Ile Val Asp Thr Pro Pro His Val Ala Ala Gly Leu Lys
65                  70                  75                  80

Asp Pro Val Pro Ser Gly Phe Ser Ala Gly Glu Val Leu Ile Pro Leu
                85                  90                  95

Ala Asp Glu Tyr Asp Pro Met Phe Pro Asn Asp Tyr Glu Lys Val Val
            100                 105                 110

Lys Arg Ala Lys Arg Gly Thr Thr Glu Thr Ala Gly Val Xaa Lys Thr
        115                 120                 125

Lys Gly Asn Arg Arg Lys Gly Lys Lys Ala
    130                 135

(2) INFORMATION FOR SEQ ID NO: 145:

(i) SEQUENCE CHARACTERISTICS:
(A) LENGTH: 356 amino acids
(B) TYPE: amino acid
(D) TOPOLOGY: linear
(xi) SEQUENCE DESCRIPTION: SEQ ID NO: 145:

Met Leu Ala Arg Ala Ala Arg Gly Thr Gly Ala Leu Leu Leu Arg Gly
1               5                   10                  15

Ser Leu Leu Ala Ser Gly Arg Ala Pro Arg Arg Ala Ser Ser Gly Leu
            20                  25                  30

Pro Arg Asn Thr Val Val Leu Phe Val Pro Gln Gln Glu Ala Trp Val
        35                  40                  45

Val Glu Arg Met Gly Arg Phe His Arg Ile Leu Glu Pro Gly Leu Asn
    50                  55                  60

Ile Leu Ile Pro Val Leu Asp Arg Ile Arg Tyr Val Gln Ser Leu Lys
65                  70                  75                  80

Glu Ile Val Ile Asn Val Pro Glu Gln Ser Ala Val Thr Leu Asp Asn
                85                  90                  95

Val Thr Leu Gln Ile Asp Gly Val Leu Tyr Leu Arg Ile Met Asp Pro
            100                 105                 110

Tyr Lys Ala Ser Tyr Gly Val Glu Asp Pro Glu Tyr Ala Val Thr Gln
        115                 120                 125

Leu Ala Gln Thr Thr Met Arg Ser Glu Leu Gly Lys Leu Ser Leu Asp
    130                 135                 140

Lys Val Phe Arg Glu Arg Glu Ser Leu Asn Ala Ser Ile Val Asp Ala
145                 150                 155                 160

Ile Asn Gln Ala Ala Asp Cys Trp Gly Ile Arg Cys Leu Arg Tyr Glu
                165                 170                 175

Ile Lys Asp Ile His Val Pro Pro Arg Val Lys Glu Ser Met Gln Met
            180                 185                 190

Gln Val Glu Ala Glu Arg Arg Lys Arg Ala Thr Val Leu Glu Ser Glu
        195                 200                 205

Gly Thr Arg Glu Ser Ala Ile Asn Val Ala Glu Gly Lys Lys Gln Ala
    210                 215                 220

Gln Ile Leu Ala Ser Glu Ala Glu Lys Ala Glu Gln Ile Asn Gln Ala
225                 230                 235                 240

Ala Gly Glu Ala Ser Ala Val Leu Ala Lys Ala Lys Ala Lys Ala Glu
                245                 250                 255

Ala Ile Arg Ile Leu Ala Ala Ala Leu Thr Gln His Asn Gly Asp Ala
            260                 265                 270

Ala Ala Ser Leu Thr Val Ala Glu Gln Tyr Val Ser Ala Phe Ser Lys
        275                 280                 285

Leu Ala Lys Asp Ser Asn Thr Ile Leu Leu Pro Ser Asn Pro Gly Asp
    290                 295                 300

Val Thr Ser Met Val Ala Gln Ala Met Gly Val Tyr Gly Ala Leu Thr
305                 310                 315                 320

Lys Ala Pro Val Pro Gly Thr Pro Asp Ser Leu Ser Ser Gly Ser Ser
                325                 330                 335

Arg Asp Val Gln Gly Thr Asp Ala Ser Leu Asp Glu Glu Leu Asp Arg
            340                 345                 350

Val Lys Met Ser
        355

(2) INFORMATION FOR SEQ ID NO: 146:

(i) SEQUENCE CHARACTERISTICS:
(A) LENGTH: 40 amino acids
(B) TYPE: amino acid
(D) TOPOLOGY: linear
(xi) SEQUENCE DESCRIPTION: SEQ ID NO: 146:

Met Tyr Ile Leu Leu Phe Trp Gly Gly Xaa Phe His Arg Cys Leu Ser
  1               5                  10                  15

Xaa Leu Phe Asp Pro Glu Leu Xaa Ser Xaa Pro Gly Ile Ser Xaa Phe
            20                  25                  30

Thr Val Xaa Leu Gln Met Thr Xaa
        35                  40

(2) INFORMATION FOR SEQ ID NO: 147:

(i) SEQUENCE CHARACTERISTICS:
(A) LENGTH: 71 amino acids
(B) TYPE: amino acid
(D) TOPOLOGY: linear
(xi) SEQUENCE DESCRIPTION: SEQ ID NO: 147:

Met Pro Ser Pro Lys Tyr Cys Met His Thr Asn Asp Val Gln Ser Val
  1               5                  10                  15

Glu Tyr Asn Gly Asp Thr Leu Phe Gln Lys Leu Ser Ser Ser Xaa Leu
            20                  25                  30

Ser Phe Lys Ser Ile His Ile Tyr Pro Asn Glu Xaa Lys Thr Cys Xaa
        35                  40                  45

Xaa Ile Phe Ile Ser Lys Val Tyr Met Ile Ser Lys Thr Trp Lys Xaa
    50                  55                  60

Pro Arg Phe Thr Ser Xaa Gly

65                    70

(2) INFORMATION FOR SEQ ID NO: 148:

(i) SEQUENCE CHARACTERISTICS:
(A) LENGTH: 33 amino acids
(B) TYPE: amino acid
(D) TOPOLOGY: linear
(xi) SEQUENCE DESCRIPTION: SEQ ID NO: 148:

Met Asn Phe Val Leu Phe Phe Ile Gly Ile Asn Val Gly Cys Arg Gly
1               5                   10                  15

Glu Asn Ser Leu Lys Tyr Phe Thr Val Thr Val Leu Cys Ser Pro Arg
            20                  25                  30

Asp

(2) INFORMATION FOR SEQ ID NO: 149:

(i) SEQUENCE CHARACTERISTICS:
(A) LENGTH: 78 amino acids
(B) TYPE: amino acid
(D) TOPOLOGY: linear
(xi) SEQUENCE DESCRIPTION: SEQ ID NO: 149:

Met Lys Glu Ala Gly Lys Gly Gly Val Ala Asp Ser Arg Glu Leu Lys
1               5                   10                  15

Pro Met Val Gly Gly Asp Glu Glu Val Ala Ala Leu Gln Glu Phe His
            20                  25                  30

Phe His Phe Leu Ser Leu Ser Val Phe Thr Asp Cys Thr Ser Ser Gly
        35                  40                  45

Glu Ala Phe Val Ile Cys Ile Thr Gln Thr Cys Cys Ser Phe Cys Leu
    50                  55                  60

Cys Ala Tyr Pro Ser Leu Gly Trp Gln Asn Ser Cys His Asn
65                  70                  75

(2) INFORMATION FOR SEQ ID NO: 150:

(i) SEQUENCE CHARACTERISTICS:
(A) LENGTH: 32 amino acids
(B) TYPE: amino acid
(D) TOPOLOGY: linear
(xi) SEQUENCE DESCRIPTION: SEQ ID NO: 150:

Met Phe Ser Ser Lys Ser Leu Leu Val Leu Pro Phe Cys Phe Arg Ser
1               5                   10                  15

Ala Ala His Leu Glu Leu Ser Val Trp Cys Val Cys Gly Val Arg Xaa

            20                  25                  30

(2) INFORMATION FOR SEQ ID NO: 151:

(i) SEQUENCE CHARACTERISTICS:
(A) LENGTH: 464 amino acids
(B) TYPE: amino acid
(D) TOPOLOGY: linear
(xi) SEQUENCE DESCRIPTION: SEQ ID NO: 151:

Met Leu Ala Leu Gly Asn Asn His Phe Ile Gly Phe Val Asn Asp Ser
1               5                   10                  15

Val Thr Lys Ser Ile Val Ala Leu Arg Leu Thr Leu Val Val Lys Val
            20                  25                  30

Ser Thr Xaa Pro Gly Glu Ser His Ala Asn Asp Leu Glu Cys Ser Gly
        35                  40                  45

Lys Gly Lys Cys Thr Thr Lys Pro Ser Glu Ala Thr Phe Ser Cys Thr
    50                  55                  60

Cys Glu Glu Gln Tyr Val Gly Thr Phe Cys Glu Glu Tyr Asp Ala Cys
65                  70                  75                  80

Gln Arg Lys Pro Cys Gln Asn Asn Ala Ser Cys Ile Asp Ala Asn Glu
                85                  90                  95

Lys Gln Asp Gly Ser Asn Phe Thr Cys Val Cys Leu Pro Gly Tyr Thr
            100                 105                 110

Gly Glu Leu Cys Gln Ser Lys Ile Asp Tyr Cys Ile Leu Asp Pro Cys
        115                 120                 125

Arg Asn Gly Ala Thr Cys Ile Ser Ser Leu Ser Gly Phe Thr Cys Gln
    130                 135                 140

Cys Pro Glu Gly Tyr Phe Gly Ser Ala Cys Glu Glu Lys Val Asp Pro
145                 150                 155                 160

Cys Ala Ser Ser Pro Cys Gln Asn Asn Gly Thr Cys Tyr Val Asp Gly
                165                 170                 175

Val His Phe Thr Cys Asn Cys Ser Pro Gly Phe Thr Gly Pro Thr Cys
            180                 185                 190

Ala Gln Leu Ile Asp Phe Cys Ala Leu Ser Pro Cys Ala His Gly Thr
        195                 200                 205

Cys Arg Ser Val Gly Thr Ser Tyr Lys Cys Leu Cys Asp Pro Gly Tyr
    210                 215                 220

His Gly Leu Tyr Cys Glu Glu Glu Tyr Asn Glu Cys Leu Ser Ala Pro
225                 230                 235                 240

Cys Leu Asn Ala Ala Thr Cys Arg Asp Leu Val Asn Gly Tyr Glu Cys
                245                 250                 255

Val Cys Leu Ala Glu Tyr Lys Gly Thr His Cys Glu Leu Tyr Lys Asp
            260                 265                 270

Pro Cys Ala Asn Val Ser Cys Leu Asn Gly Ala Thr Cys Asp Ser Asp
        275                 280                 285

Gly Leu Asn Gly Thr Cys Ile Cys Ala Pro Gly Phe Thr Gly Glu Glu
    290                 295                 300

Cys Asp Ile Asp Ile Asn Glu Cys Asp Ser Asn Pro Cys His His Gly
305                 310                 315                 320

Gly Ser Cys Leu Asp Gln Pro Asn Gly Tyr Asn Xaa His Cys Pro His
                325                 330                 335

Gly Trp Val Gly Ala Asn Cys Glu Ile His Leu Gln Trp Lys Ser Gly
            340                 345                 350

His Met Ala Glu Ser Leu Thr Asn Met Pro Arg His Ser Leu Tyr Ile
        355                 360                 365

Ile Ile Gly Ala Leu Cys Val Ala Phe Ile Leu Met Leu Ile Ile Leu
    370                 375                 380

Ile Val Gly Ile Cys Arg Ile Ser Arg Ile Glu Tyr Gln Gly Ser Ser
385                 390                 395                 400

Arg Pro Ala Tyr Xaa Glu Phe Tyr Asn Cys Arg Ser Ile Asp Ser Glu
                405                 410                 415

Phe Ser Asn Ala Ile Ala Ser Ile Arg His Ala Arg Phe Gly Lys Lys
            420                 425                 430

Ser Arg Pro Ala Met Tyr Asp Val Ser Pro Ile Ala Tyr Glu Asp Tyr
        435                 440                 445

Ser Pro Asp Asp Lys Pro Leu Val Thr Leu Ile Lys Thr Lys Asp Leu
    450                 455                 460

(2) INFORMATION FOR SEQ ID NO: 152:

(i) SEQUENCE CHARACTERISTICS:
(A) LENGTH: 151 amino acids
(B) TYPE: amino acid
(D) TOPOLOGY: linear
(xi) SEQUENCE DESCRIPTION: SEQ ID NO: 152:

Met His His Gln Met Thr Arg Thr Thr Leu Met Thr Lys Gln His Glu
1               5                   10                  15

Leu Gly Gly Leu Leu Ala Leu Val Gln Asn Cys Gln Ser Glu Met Asn
            20                  25                  30

Ile Lys Asp Ser Arg Ala Val Gly Leu Ser Val Lys Arg Leu Cys Ile
        35                  40                  45

Ser Phe Val Asp Glu Phe Cys Glu Arg Thr Glu Arg Pro Leu Tyr Leu
    50                  55                  60

Ala Gln Gly Leu Phe Met Lys Arg Glu Thr Tyr Trp Glu Val Gln Asp
65                  70                  75                  80

Ser Gly Ile Ser Pro Leu Leu Leu Leu Leu Ser Thr Ala Leu Asp Cys
                85                  90                  95

Ser Pro Glu Ala Glu Thr Arg Gln Ser Pro Gly Gly Arg Lys Met Leu
            100                 105                 110

Gln Glu Pro Thr Leu Ser Met Ser Leu Gln Ile Leu Thr Gly Phe Leu
        115                 120                 125

Trp Val Gln Leu Trp Asn Trp Glu Thr Phe Leu Arg Ile Arg Thr His
    130                 135                 140

Ser Thr Asp Ala Ser Cys Pro
145                 150

(2) INFORMATION FOR SEQ ID NO: 153:

    (i) SEQUENCE CHARACTERISTICS:
        (A) LENGTH: 299 amino acids
        (B) TYPE: amino acid
        (D) TOPOLOGY: linear
    (xi) SEQUENCE DESCRIPTION: SEQ ID NO: 153:

Met Ala Gln Asn Leu Lys Asp Leu Ala Gly Arg Leu Pro Ala Gly Pro
1               5                   10                  15

Arg Gly Met Gly Thr Ala Leu Lys Leu Leu Leu Gly Ala Gly Ala Val
            20                  25                  30

Ala Tyr Gly Val Arg Glu Ser Val Phe Thr Val Glu Gly Gly His Arg
        35                  40                  45

Ala Ile Phe Phe Asn Arg Ile Gly Gly Val Gln Gln Asp Thr Ile Leu
    50                  55                  60

Ala Glu Gly Leu His Phe Arg Ile Pro Trp Phe Gln Tyr Pro Ile Ile
65                  70                  75                  80

Tyr Asp Ile Arg Ala Arg Pro Arg Lys Ile Ser Ser Pro Thr Gly Ser
                85                  90                  95

Lys Asp Leu Gln Met Val Asn Ile Ser Leu Arg Val Leu Ser Arg Pro
            100                 105                 110

Asn Ala Gln Glu Leu Pro Ser Met Tyr Gln Arg Leu Gly Leu Asp Tyr
        115                 120                 125

Glu Glu Arg Val Leu Pro Ser Ile Val Asn Glu Val Leu Lys Ser Val

130                 135                 140

Val Ala Lys Phe Asn Ala Ser Gln Leu Ile Thr Gln Arg Ala Gln Val
145                 150                 155                 160

Ser Leu Leu Ile Arg Arg Glu Leu Thr Glu Arg Ala Lys Asp Phe Ser
                165                 170                 175

Leu Ile Leu Asp Asp Val Ala Ile Thr Glu Leu Ser Phe Ser Arg Glu
            180                 185                 190

Tyr Thr Ala Ala Val Glu Ala Lys Gln Val Ala Gln Gln Glu Ala Gln
        195                 200                 205

Arg Ala Xaa Phe Leu Val Glu Lys Ala Lys Gln Glu Gln Arg Gln Lys
    210                 215                 220

Ile Val Gln Ala Glu Gly Glu Ala Glu Ala Ala Lys Met Leu Gly Glu
225                 230                 235                 240

Ala Leu Ser Lys Asn Pro Gly Tyr Ile Lys Leu Arg Lys Ile Arg Ala
                245                 250                 255

Ala Gln Asn Ile Ser Lys Thr Ile Ala Thr Ser Gln Asn Arg Ile Tyr
            260                 265                 270

Leu Thr Ala Asp Asn Leu Val Leu Asn Leu Gln Asp Glu Ser Phe Thr
        275                 280                 285

Arg Gly Ser Asp Ser Leu Ile Lys Gly Lys Lys
    290                 295

(2) INFORMATION FOR SEQ ID NO: 154:

        (i) SEQUENCE CHARACTERISTICS:
            (A) LENGTH: 398 amino acids
            (B) TYPE: amino acid
            (D) TOPOLOGY: linear
        (xi) SEQUENCE DESCRIPTION: SEQ ID NO: 154:

Met Leu Arg Gly Pro Trp Arg Gln Leu Trp Leu Phe Xaa Leu Leu Leu
  1               5                  10                  15

Leu Pro Gly Ala Pro Glu Pro Arg Gly Ala Ser Arg Pro Trp Glu Gly
             20                  25                  30

Thr Asp Glu Pro Gly Ser Ala Trp Ala Trp Pro Gly Phe Gln Arg Leu
         35                  40                  45

Gln Glu Gln Leu Arg Ala Ala Gly Ala Leu Ser Lys Arg Tyr Trp Thr
     50                  55                  60

Leu Phe Ser Cys Gln Val Trp Pro Asp Asp Cys Asp Glu Asp Glu Glu
 65                  70                  75                  80

Ala Ala Thr Gly Pro Leu Gly Trp Arg Leu Pro Leu Leu Gly Gln Arg
                 85                  90                  95

Tyr Leu Asp Leu Leu Thr Thr Trp Tyr Cys Ser Phe Lys Asp Cys Cys
            100                 105                 110

Pro Arg Gly Asp Cys Arg Ile Ser Asn Asn Phe Thr Gly Leu Glu Trp
        115                 120                 125

Asp Leu Asn Val Arg Leu His Gly Gln His Leu Val Gln Gln Leu Val
    130                 135                 140

Leu Arg Thr Val Arg Gly Tyr Leu Glu Thr Pro Gln Pro Glu Lys Ala
145                 150                 155                 160

Leu Ala Leu Ser Phe His Gly Trp Ser Gly Thr Gly Lys Asn Phe Val
                165                 170                 175

Ala Arg Met Leu Val Glu Asn Leu Tyr Arg Asp Gly Leu Met Ser Asp
            180                 185                 190

Cys Val Arg Met Phe Ile Ala Thr Phe His Phe Pro His Pro Lys Tyr
        195                 200                 205

Val Asp Leu Tyr Lys Glu Gln Leu Met Ser Gln Ile Arg Glu Thr Gln
    210                 215                 220

Gln Leu Cys His Gln Thr Leu Phe Ile Phe Asp Glu Ala Glu Lys Leu
225                 230                 235                 240

His Pro Gly Leu Leu Glu Val Leu Gly Pro His Leu Glu Arg Arg Ala
                245                 250                 255

Pro Xaa Gly His Arg Ala Glu Ser Pro Trp Thr Ile Phe Leu Phe Leu
            260                 265                 270

Ser Asn Leu Arg Gly Asp Ile Ile Asn Glu Val Val Leu Lys Leu Leu
        275                 280                 285

Lys Ala Gly Trp Ser Arg Glu Glu Ile Thr Met Glu His Leu Glu Pro
    290                 295                 300

His Leu Gln Ala Glu Ile Val Glu Thr Ile Asp Asn Gly Phe Gly His
305                 310                 315                 320

Ser Arg Leu Val Lys Glu Asn Leu Ile Asp Tyr Phe Ile Pro Phe Leu
                325                 330                 335

Pro Leu Glu Tyr Arg His Val Arg Leu Cys Ala Arg Asp Ala Phe Leu
            340                 345                 350

Ser Gln Glu Leu Leu Tyr Lys Glu Glu Thr Leu Asp Glu Ile Ala Gln
        355                 360                 365

Met Met Val Tyr Val Pro Lys Glu Glu Gln Leu Phe Ser Ser Gln Gly
    370                 375                 380

Cys Lys Ser Ile Ser Gln Arg Ile Asn Tyr Phe Leu Ser Xaa
385                 390                 395

(2) INFORMATION FOR SEQ ID NO: 155:

(i) SEQUENCE CHARACTERISTICS:
(A) LENGTH: 83 amino acids
(B) TYPE: amino acid
(D) TOPOLOGY: linear
(xi) SEQUENCE DESCRIPTION: SEQ ID NO: 155:

Met Ala Phe Thr Leu Tyr Ser Leu Leu Gln Ala Xaa Leu Leu Cys Val
1               5                   10                  15

Asn Ala Ile Ala Val Leu His Glu Glu Arg Phe Leu Lys Asn Ile Gly
            20                  25                  30

Trp Gly Thr Asp Gln Gly Ile Gly Gly Phe Gly Glu Glu Pro Gly Ile
        35                  40                  45

Lys Ser Gln Leu Met Asn Leu Ile Arg Ser Val Arg Thr Val Met Arg
    50                  55                  60

Val Pro Leu Ile Ile Val Asn Ser Ile Ala Ile Val Leu Leu Leu Leu
65                  70                  75                  80

Phe Gly Xaa

(2) INFORMATION FOR SEQ ID NO: 156:

(i) SEQUENCE CHARACTERISTICS:
(A) LENGTH: 50 amino acids
(B) TYPE: amino acid
(D) TOPOLOGY: linear
(xi) SEQUENCE DESCRIPTION: SEQ ID NO: 156:

Met Ala Pro Arg Asn Gln Gly Ser Phe Ser Phe Gly Asn Phe Met Leu
1               5                   10                  15

Phe Leu Val Leu Ile Glu Arg Arg Tyr Leu Pro Phe Leu Ser Pro Ile
            20                  25                  30

Leu Phe Cys Cys Ser Thr His Asn Arg Ser Ala Val Thr Ala Thr Asn
        35                  40                  45

Leu Xaa
    50

(2) INFORMATION FOR SEQ ID NO: 157:

(i) SEQUENCE CHARACTERISTICS:
(A) LENGTH: 51 amino acids
(B) TYPE: amino acid
(D) TOPOLOGY: linear
(xi) SEQUENCE DESCRIPTION: SEQ ID NO: 157:

Met Asp Val Leu Thr Val Ala Phe Leu Ser Ile Leu Ile Thr Ala Pro
1               5                   10                  15

Ile Gly Ser Leu Leu Ile Gly Leu Leu Gly Pro Arg Leu Leu Gln Lys
            20                  25                  30

Val Glu His Gln Asn Lys Asp Glu Glu Val Gln Gly Glu Thr Ser Val
        35                  40                  45

Gln Val Xaa
    50

(2) INFORMATION FOR SEQ ID NO: 158:

      (i) SEQUENCE CHARACTERISTICS:
            (A) LENGTH: 17 amino acids
            (B) TYPE: amino acid
            (D) TOPOLOGY: linear
      (xi) SEQUENCE DESCRIPTION: SEQ ID NO: 158:

Pro Asn Ser Phe Ser Cys Leu Gly Leu Ala Gly Thr Gly Ala Gly Ile
  1               5                  10                  15

Xaa

(2) INFORMATION FOR SEQ ID NO: 159:

      (i) SEQUENCE CHARACTERISTICS:
            (A) LENGTH: 53 amino acids
            (B) TYPE: amino acid
            (D) TOPOLOGY: linear
      (xi) SEQUENCE DESCRIPTION: SEQ ID NO: 159:

Met Gly Arg Tyr His Phe Val Phe Leu Thr Phe Phe Phe Ser Thr Tyr
  1               5                  10                  15

Ser Ser Cys Phe Tyr Pro Val Val Ser Gln Val Leu Tyr Leu Val Cys
            20                  25                  30

Ser Cys Thr Ala Asp Arg Pro Leu Met Ala Pro Val Gly Ser Cys Leu
        35                  40                  45

Gly Gly Arg Asn Xaa
    50

(2) INFORMATION FOR SEQ ID NO: 160:

      (i) SEQUENCE CHARACTERISTICS:
            (A) LENGTH: 64 amino acids
            (B) TYPE: amino acid
            (D) TOPOLOGY: linear
      (xi) SEQUENCE DESCRIPTION: SEQ ID NO: 160:

Met Phe Val Thr Leu Ser Ile Leu Asn Ile Thr Ile Glu Lys Asp Lys
  1               5                  10                  15

Ser Thr Asn Arg Phe Arg Asp Val Phe Leu Gln His Ile Leu Val Ile
            20                  25                  30

Leu Met Pro Ser Leu Thr Tyr Cys Leu Ile Gly Gln His Leu Cys Ser
        35                  40                  45

Phe Thr Arg Tyr Val Ser Leu Cys Tyr Ser Arg Cys His Ser Trp Xaa
    50                  55                  60

(2) INFORMATION FOR SEQ ID NO: 161:

(i) SEQUENCE CHARACTERISTICS:
(A) LENGTH: 33 amino acids
(B) TYPE: amino acid
(D) TOPOLOGY: linear
(xi) SEQUENCE DESCRIPTION: SEQ ID NO: 161:

Met Ser Ile Cys Pro Leu Leu Val Met Leu Ile Leu Ile Thr Trp Val
 1               5                  10                  15

Arg Cys Pro Val Ser Pro Val Tyr Arg Tyr Cys Phe Ser Phe Cys Asn
            20                  25                  30

Xaa

(2) INFORMATION FOR SEQ ID NO: 162:

(i) SEQUENCE CHARACTERISTICS:
(A) LENGTH: 95 amino acids
(B) TYPE: amino acid
(D) TOPOLOGY: linear
(xi) SEQUENCE DESCRIPTION: SEQ ID NO: 162:

Met Gln Asp Ile Val Tyr Lys Leu Val Pro Gly Leu Gln Glu Gly Glu
 1               5                  10                  15

Cys Leu Thr Val Leu Leu Ile Pro Glu Val Pro Ala Trp Pro Leu Gln
            20                  25                  30

Pro Leu Leu Ser Trp Lys Phe Gly Ser Arg Met Gly Gly Pro Phe Pro
        35                  40                  45

Phe Gly Arg Ile Thr Val Phe Ser Ser Leu Leu Ser Ala Gln Leu His
    50                  55                  60

Leu Leu Gly Trp Ser Leu Leu Ser Ser Lys Met Arg Xaa His Leu Phe
65                  70                  75                  80

Thr Pro Tyr Val Tyr Ser Phe Ser Lys Tyr Gly Ser His Val Xaa
                85                  90                  95

(2) INFORMATION FOR SEQ ID NO: 163:

(i) SEQUENCE CHARACTERISTICS:
(A) LENGTH: 58 amino acids
(B) TYPE: amino acid
(D) TOPOLOGY: linear
(xi) SEQUENCE DESCRIPTION: SEQ ID NO: 163:

Met Lys Val Leu Ala Thr Ser Phe Val Leu Gly Ser Leu Gly Leu Ala
1               5                   10                  15

Phe Tyr Leu Pro Leu Val Val Thr Thr Pro Lys Thr Leu Ala Ile Pro
            20                  25                  30

Xaa Glu Ala Ala Arg Ser Cys Gly Glu Ser Tyr His Gln Cys His Asn
        35                  40                  45

Leu Tyr Cys His Leu Trp Pro Trp Leu Xaa
    50                  55

(2) INFORMATION FOR SEQ ID NO: 164:

(i) SEQUENCE CHARACTERISTICS:
(A) LENGTH: 44 amino acids
(B) TYPE: amino acid
(D) TOPOLOGY: linear
(xi) SEQUENCE DESCRIPTION: SEQ ID NO: 164:

Met Asp Tyr Gly Tyr Tyr Ser Ala Gly Gln Phe Leu Leu His Leu Phe
1               5                   10                  15

Leu Ala Asp Leu Thr Gln Ala Thr Thr Gln Gln Lys Thr Asn Thr Ser
            20                  25                  30

Glu Asn Gly Cys Lys Phe Val Cys Ala Val Phe Xaa
        35                  40

(2) INFORMATION FOR SEQ ID NO: 165:

(i) SEQUENCE CHARACTERISTICS:
(A) LENGTH: 18 amino acids
(B) TYPE: amino acid
(D) TOPOLOGY: linear
(xi) SEQUENCE DESCRIPTION: SEQ ID NO: 165:

Gly Ile Val Leu Leu Ile Gly Val Leu Val Gln Val Ser Ala Val Asp
1               5                   10                  15

Asp Xaa

(2) INFORMATION FOR SEQ ID NO: 166:

(i) SEQUENCE CHARACTERISTICS:
(A) LENGTH: 30 amino acids
(B) TYPE: amino acid
(D) TOPOLOGY: linear
(xi) SEQUENCE DESCRIPTION: SEQ ID NO: 166:

Met Gly Asn Ala Phe Glu Val Thr Gly Leu Met Leu Ala Leu Leu Cys
1               5                   10                  15

Tyr Val Val Asp Gly Gln Lys Pro Lys Xaa Gly Phe Xaa Xaa
            20                  25                  30

(2) INFORMATION FOR SEQ ID NO: 167:

(i) SEQUENCE CHARACTERISTICS:
(A) LENGTH: 37 amino acids
(B) TYPE: amino acid
(D) TOPOLOGY: linear
(xi) SEQUENCE DESCRIPTION: SEQ ID NO: 167:

Met Ser His Glu Lys Ser Asn Glu Leu Val Leu Leu Ile Val Thr Val
1               5                   10                  15

Met Arg Ser Leu Thr Tyr Asn Ile Ala Val Val Ala Ala Trp Phe Asn
            20                  25                  30

Gly Cys Ile Arg Xaa
        35

(2) INFORMATION FOR SEQ ID NO: 168:

(i) SEQUENCE CHARACTERISTICS:
(A) LENGTH: 40 amino acids
(B) TYPE: amino acid
(D) TOPOLOGY: linear
(xi) SEQUENCE DESCRIPTION: SEQ ID NO: 168:

Met Tyr Leu Leu Tyr Leu Pro Ser Ala Leu Leu Pro Pro Tyr Pro Thr
1               5                   10                  15

Cys Pro Tyr Glu His Gly Ser Pro Trp Pro His Thr Pro Ala Lys Leu
            20                  25                  30

Leu Cys Cys Phe Ala Phe Leu Xaa
        35                  40

(2) INFORMATION FOR SEQ ID NO: 169:

(i) SEQUENCE CHARACTERISTICS:
(A) LENGTH: 47 amino acids
(B) TYPE: amino acid
(D) TOPOLOGY: linear
(xi) SEQUENCE DESCRIPTION: SEQ ID NO: 169:

Met Lys Phe Ile Val Trp Arg Arg Phe Lys Trp Val Ile Ile Gly Leu
1               5                   10                  15

Leu Phe Leu Leu Ile Leu Leu Leu Phe Val Ala Val Leu Leu Tyr Ser
            20                  25                  30

Leu Pro Asn Tyr Leu Ser Met Lys Ile Val Lys Pro Asn Val Xaa
        35                  40                  45

(2) INFORMATION FOR SEQ ID NO: 170:

(i) SEQUENCE CHARACTERISTICS:
(A) LENGTH: 34 amino acids
(B) TYPE: amino acid
(D) TOPOLOGY: linear
(xi) SEQUENCE DESCRIPTION: SEQ ID NO: 170:

Ile Glu Trp Ser Gly Tyr Asn Lys Pro Glu Arg Lys Gly Pro Leu Ala
1               5                   10                  15

Leu Phe Leu Val Phe Leu Phe Leu Asp Thr Pro Pro Leu Gln Gly Asp
            20                  25                  30

Leu Xaa

(2) INFORMATION FOR SEQ ID NO: 171:

(i) SEQUENCE CHARACTERISTICS:
(A) LENGTH: 5 amino acids
(B) TYPE: amino acid
(D) TOPOLOGY: linear
(xi) SEQUENCE DESCRIPTION: SEQ ID NO: 171:

Met Ser Leu Leu Xaa
1               5

(2) INFORMATION FOR SEQ ID NO: 172:

(i) SEQUENCE CHARACTERISTICS:
(A) LENGTH: 25 amino acids
(B) TYPE: amino acid
(D) TOPOLOGY: linear
(xi) SEQUENCE DESCRIPTION: SEQ ID NO: 172:

Met Gln Leu Leu Ile Val Trp Asn Glu Ser Leu Thr Asn Ser Val Pro
1               5                   10                  15

Ala Ser Val Asp Thr Ser Gln Cys Xaa
            20                  25

(2) INFORMATION FOR SEQ ID NO: 173:

(i) SEQUENCE CHARACTERISTICS:
(A) LENGTH: 262 amino acids
(B) TYPE: amino acid
(D) TOPOLOGY: linear
(xi) SEQUENCE DESCRIPTION: SEQ ID NO: 173:

Met Ala Leu Gly Leu Lys Cys Phe Arg Met Val His Pro Thr Phe Arg
1               5                   10                  15

Asn Tyr Leu Ala Ala Ser Ile Arg Pro Val Ser Glu Val Thr Leu Lys
            20                  25                  30

Thr Val His Glu Arg Gln His Gly His Arg Gln Tyr Met Ala Tyr Ser
        35                  40                  45

Ala Val Pro Val Arg His Phe Ala Thr Lys Lys Ala Lys Ala Lys Gly
    50                  55                  60

Lys Gly Gln Ser Gln Thr Arg Val Asn Ile Asn Ala Ala Leu Val Glu
65                  70                  75                  80

Asp Ile Ile Asn Leu Glu Glu Val Asn Glu Glu Met Lys Ser Val Ile
                85                  90                  95

Glu Ala Leu Lys Asp Asn Phe Asn Lys Thr Leu Asn Ile Arg Thr Ser
            100                 105                 110

Pro Gly Ser Leu Asp Lys Ile Ala Val Val Thr Ala Asp Gly Lys Leu
        115                 120                 125

Ala Leu Asn Gln Ile Ser Gln Ile Ser Met Lys Ser Pro Gln Leu Ile
    130                 135                 140

Leu Val Asn Met Ala Ser Phe Pro Glu Cys Thr Ala Ala Ala Ile Lys
145                 150                 155                 160

Ala Ile Arg Glu Ser Gly Met Asn Leu Asn Pro Glu Val Glu Gly Thr
                165                 170                 175

Leu Ile Arg Val Pro Ile Pro Gln Val Thr Arg Glu His Arg Glu Met
            180                 185                 190

Leu Val Lys Leu Ala Lys Gln Asn Thr Asn Lys Ala Lys Asp Ser Leu
        195                 200                 205

Arg Lys Val Arg Thr Asn Ser Met Asn Lys Leu Lys Lys Ser Lys Asp
    210                 215                 220

Thr Val Ser Glu Asp Thr Ile Arg Leu Ile Glu Lys Gln Ile Ser Gln
225                 230                 235                 240

Met Ala Asp Asp Thr Val Ala Glu Leu Asp Arg His Leu Ala Val Lys
                245                 250                 255

Thr Lys Glu Leu Leu Gly
            260

(2) INFORMATION FOR SEQ ID NO: 174:

(i) SEQUENCE CHARACTERISTICS:
(A) LENGTH: 967 amino acids
(B) TYPE: amino acid
(D) TOPOLOGY: linear
(xi) SEQUENCE DESCRIPTION: SEQ ID NO: 174:

Met Gln Arg Ala Val Pro Glu Gly Phe Gly Arg Arg Lys Leu Gly Ser
1               5                   10                  15

Asp Met Gly Asn Ala Glu Arg Ala Pro Gly Ser Arg Ser Phe Gly Pro
            20                  25                  30

Val Pro Thr Leu Leu Leu Leu Xaa Ala Ala Leu Leu Xaa Val Ser Asp
        35                  40                  45

Ala Leu Gly Arg Pro Ser Glu Glu Asp Glu Glu Leu Val Val Pro Glu
    50                  55                  60

Leu Glu Arg Ala Pro Gly His Gly Thr Thr Arg Leu Arg Leu His Ala
65                  70                  75                  80

Phe Asp Gln Gln Leu Asp Leu Glu Leu Arg Pro Asp Ser Ser Phe Leu
                85                  90                  95

Ala Pro Gly Phe Thr Leu Gln Asn Val Gly Arg Lys Ser Gly Ser Glu
            100                 105                 110

Thr Pro Leu Pro Glu Thr Asp Leu Ala His Cys Phe Tyr Ser Gly Thr
        115                 120                 125

Val Asn Gly Asp Pro Ser Ser Ala Ala Ala Leu Ser Leu Cys Glu Gly
    130                 135                 140

Val Arg Gly Ala Phe Tyr Leu Leu Gly Glu Ala Tyr Phe Ile Gln Pro
145                 150                 155                 160

Leu Pro Ala Ala Ser Glu Arg Leu Xaa Thr Ala Ala Pro Gly Glu Lys
                165                 170                 175

Pro Pro Ala Pro Leu Gln Phe His Leu Leu Arg Arg Asn Arg Gln Gly
            180                 185                 190

Asp Val Gly Gly Thr Cys Gly Val Val Asp Asp Glu Pro Arg Pro Thr
        195                 200                 205

Gly Lys Ala Glu Thr Glu Asp Glu Asp Glu Gly Thr Glu Gly Glu Asp
    210                 215                 220

Glu Gly Pro Gln Trp Ser Pro Gln Asp Pro Ala Leu Gln Gly Val Gly
225                 230                 235                 240

Gln Pro Thr Gly Thr Gly Ser Ile Arg Lys Lys Arg Phe Val Ser Ser
                245                 250                 255

His Arg Tyr Val Glu Thr Met Leu Val Ala Asp Gln Ser Met Ala Glu
            260                 265                 270

Phe His Gly Ser Gly Leu Lys His Tyr Leu Leu Thr Leu Phe Ser Val

        275                 280                 285

Ala Ala Arg Leu Xaa Lys His Pro Xaa Ile Arg Asn Ser Val Ser Leu
    290                 295                 300

Val Val Val Lys Ile Leu Val Ile His Asp Glu Gln Lys Gly Pro Glu
305                 310                 315                 320

Val Thr Ser Asn Ala Ala Leu Thr Leu Arg Asn Phe Cys Asn Trp Gln
                325                 330                 335

Lys Gln His Asn Pro Pro Ser Asp Arg Asp Ala Glu His Tyr Asp Thr
            340                 345                 350

Ala Ile Leu Phe Thr Arg Gln Asp Leu Cys Gly Ser Gln Thr Cys Asp
        355                 360                 365

Thr Leu Gly Met Ala Asp Val Gly Thr Val Cys Asp Pro Ser Arg Ser
    370                 375                 380

Cys Ser Val Ile Glu Asp Asp Gly Leu Gln Ala Ala Phe Thr Thr Ala
385                 390                 395                 400

His Glu Leu Gly His Val Phe Asn Met Pro His Asp Asp Ala Lys Gln
                405                 410                 415

Cys Ala Ser Leu Asn Gly Val Asn Gln Asp Ser His Met Met Ala Ser
            420                 425                 430

Met Leu Ser Asn Leu Asp His Ser Gln Pro Trp Ser Pro Cys Ser Ala
        435                 440                 445

Tyr Met Ile Thr Ser Phe Leu Asp Asn Gly His Gly Glu Cys Leu Met
    450                 455                 460

Asp Lys Pro Gln Asn Pro Ile Gln Leu Pro Gly Asp Leu Pro Gly Thr
465                 470                 475                 480

Ser Tyr Asp Ala Asn Arg Gln Cys Gln Phe Thr Phe Gly Glu Asp Ser
                485                 490                 495

Lys His Cys Pro Asp Ala Ala Ser Thr Cys Ser Thr Leu Trp Cys Thr
            500                 505                 510

Gly Thr Ser Gly Gly Val Leu Val Cys Gln Thr Lys His Phe Pro Trp
        515                 520                 525

Ala Asp Gly Thr Ser Cys Gly Glu Gly Lys Trp Cys Ile Asn Gly Lys
    530                 535                 540

Cys Val Xaa Lys Thr Asp Arg Lys His Phe Asp Thr Pro Phe His Gly
545                 550                 555                 560

Ser Trp Gly Met Trp Gly Pro Trp Gly Asp Cys Ser Arg Thr Cys Gly
                565                 570                 575

Gly Gly Val Gln Tyr Thr Met Arg Glu Cys Asp Asn Pro Val Pro Lys
            580                 585                 590

Asn Gly Gly Lys Tyr Cys Glu Gly Lys Arg Val Arg Tyr Arg Ser Cys

595                 600                 605

Asn Leu Glu Asp Cys Pro Asp Asn Asn Gly Lys Thr Phe Arg Glu Glu
    610                 615                 620

Gln Cys Glu Ala His Asn Glu Phe Ser Lys Ala Ser Phe Gly Ser Gly
625                 630                 635                 640

Pro Ala Val Glu Trp Ile Pro Lys Tyr Ala Gly Val Ser Pro Lys Asp
                645                 650                 655

Arg Cys Lys Leu Ile Cys Gln Ala Lys Gly Ile Gly Tyr Phe Phe Val
            660                 665                 670

Leu Gln Pro Lys Val Val Asp Gly Thr Pro Cys Ser Pro Asp Ser Thr
        675                 680                 685

Ser Val Cys Val Gln Gly Gln Cys Val Lys Ala Gly Cys Asp Arg Ile
    690                 695                 700

Ile Asp Ser Lys Lys Lys Phe Asp Lys Cys Gly Val Cys Gly Gly Asn
705                 710                 715                 720

Gly Ser Thr Cys Lys Lys Ile Ser Gly Ser Val Thr Ser Ala Lys Pro
                725                 730                 735

Gly Tyr His Asp Ile Ile Thr Ile Pro Thr Gly Ala Thr Asn Ile Glu
            740                 745                 750

Val Lys Gln Arg Asn Gln Arg Gly Ser Arg Asn Asn Gly Ser Phe Leu
        755                 760                 765

Ala Ile Lys Ala Ala Asp Gly Thr Tyr Ile Leu Asn Gly Asp Tyr Thr
    770                 775                 780

Leu Ser Thr Leu Glu Gln Asp Ile Met Tyr Lys Gly Val Val Leu Arg
785                 790                 795                 800

Tyr Ser Gly Ser Ser Ala Ala Leu Glu Arg Ile Arg Ser Phe Ser Pro
                805                 810                 815

Leu Lys Glu Pro Leu Thr Ile Gln Val Leu Thr Val Gly Asn Ala Leu
            820                 825                 830

Arg Pro Lys Ile Lys Tyr Thr Tyr Phe Val Lys Lys Lys Lys Glu Ser
        835                 840                 845

Phe Asn Ala Ile Pro Thr Phe Ser Ala Trp Val Ile Glu Glu Trp Gly
    850                 855                 860

Glu Cys Ser Lys Ser Cys Glu Leu Gly Trp Gln Arg Arg Leu Val Glu
865                 870                 875                 880

Cys Arg Asp Ile Asn Gly Gln Pro Ala Ser Glu Cys Ala Lys Glu Val
                885                 890                 895

Lys Pro Ala Ser Thr Arg Pro Cys Ala Asp His Pro Cys Pro Gln Trp
            900                 905                 910

Gln Leu Gly Glu Trp Ser Ser Cys Ser Lys Thr Cys Gly Lys Gly Tyr

915                    920                    925

Lys Lys Arg Ser Leu Lys Cys Leu Ser His Asp Gly Gly Val Leu Ser
    930                    935                    940

His Glu Ser Cys Asp Pro Leu Lys Lys Pro Lys His Phe Ile Asp Phe
945                    950                    955                    960

Cys Thr Met Ala Glu Cys Ser
                965

(2) INFORMATION FOR SEQ ID NO: 175:

(i) SEQUENCE CHARACTERISTICS:
(A) LENGTH: 19 amino acids
(B) TYPE: amino acid
(D) TOPOLOGY: linear
(xi) SEQUENCE DESCRIPTION: SEQ ID NO: 175:

Met Leu Lys Ile Pro Thr His Leu Glu Gly Lys Ile Lys Ile Thr Lys
  1               5                   10                  15

Val Tyr Xaa

(2) INFORMATION FOR SEQ ID NO: 176:

(i) SEQUENCE CHARACTERISTICS:
(A) LENGTH: 205 amino acids
(B) TYPE: amino acid
(D) TOPOLOGY: linear
(xi) SEQUENCE DESCRIPTION: SEQ ID NO: 176:

Met Tyr Glu Thr Met Lys Leu Asp Ala Cys Xaa His Gln Gln Arg Pro
  1               5                   10                  15

Thr Leu Gln Ala Gly Pro Lys Leu Leu Thr Leu Ala Pro Arg Glu Glu
             20                  25                  30

Pro Arg Gly Gln Ser Gly Arg Gly Ser Glu Leu Thr Ala Arg Gln Arg
         35                  40                  45

His Ser Thr Gly Asp Pro Gln Gly Glu Gln Ala Leu Pro Arg Ala Gly
     50                  55                  60

Cys Val Thr Gly Pro Pro Ala Thr Pro His Arg Pro Ser Glu Pro Gln
 65                  70                  75                  80

Leu Leu Arg Thr His Pro Asp Ala Arg Pro Lys Ser Ala Met Ala Gln
                 85                  90                  95

Thr Phe Val His Gln Gly Pro Val Ala Leu Gln Gln Leu Thr Thr Asn
            100                 105                 110

Arg Arg Val Glu Thr Ser Met Ser Ser Asp Gly His Gly Gln Asn Pro
        115                 120                 125

Thr Pro Ser Pro Trp Ala Asp Val Cys Ala Ser Arg Ala Asp Ala Val
    130                 135                 140

Ala Phe Pro Ala Ser Gly Xaa Cys His Ser Pro Trp Leu Met Xaa Pro
145                 150                 155                 160

Ser Ser His Pro Leu Asn Pro His Ser Pro Leu Asn Leu Pro Pro Pro
                165                 170                 175

Ser Phe His Cys Lys Asp Pro Val Met Thr Leu His Pro Gln Thr Leu
            180                 185                 190

Val Thr Gln Gly His Leu Ser Thr Ser Gly Arg Leu Thr
        195                 200                 205

(2) INFORMATION FOR SEQ ID NO: 177:

        (i) SEQUENCE CHARACTERISTICS:
                (A) LENGTH: 54 amino acids
                (B) TYPE: amino acid
                (D) TOPOLOGY: linear
        (xi) SEQUENCE DESCRIPTION: SEQ ID NO: 177:

Met Asp Ser Met Pro Glu Pro Ala Ser Arg Cys Leu Leu Leu Leu Pro
  1               5                  10                  15

Leu Leu Leu Leu Leu Leu Leu Leu Leu Pro Ala Pro Glu Leu Gly Pro
            20                  25                  30

Ser Gln Ala Gly Ala Glu Glu Asn Asp Trp Val Arg Leu Pro Ser Lys
        35                  40                  45

Cys Glu Gly Thr Cys Gly
    50

(2) INFORMATION FOR SEQ ID NO: 178:

        (i) SEQUENCE CHARACTERISTICS:
                (A) LENGTH: 436 amino acids
                (B) TYPE: amino acid
                (D) TOPOLOGY: linear
        (xi) SEQUENCE DESCRIPTION: SEQ ID NO: 178:

Met Pro Leu Phe Leu Leu Ser Leu Pro Thr Pro Pro Ser Ala Ser Gly
  1               5                  10                  15

His Glu Arg Arg Gln Arg Pro Glu Ala Lys Thr Ser Gly Ser Glu Lys
            20                  25                  30

Lys Tyr Leu Arg Ala Met Gln Ala Asn Arg Ser Gln Leu His Ser Pro
        35                  40                  45

Pro Gly Thr Gly Ser Ser Glu Asp Ala Ser Thr Pro Gln Cys Val His
    50                  55                  60

Thr Arg Leu Thr Gly Glu Gly Ser Cys Pro His Ser Gly Asp Val His
65                  70                  75                  80

Ile Gln Ile Asn Ser Ile Pro Lys Glu Cys Ala Glu Asn Ala Ser Ser
                85                  90                  95

Arg Asn Ile Arg Ser Gly Val His Ser Cys Ala His Gly Cys Val His
            100                 105                 110

Ser Arg Leu Arg Gly His Ser His Ser Glu Ala Arg Leu Thr Asp Asp
        115                 120                 125

Thr Ala Ala Glu Ser Gly Asp His Gly Ser Ser Ser Phe Ser Glu Phe
    130                 135                 140

Arg Tyr Leu Phe Lys Trp Leu Gln Lys Ser Leu Pro Tyr Ile Leu Ile
145                 150                 155                 160

Leu Ser Val Lys Leu Val Met Gln His Ile Thr Gly Ile Ser Leu Gly
                165                 170                 175

Ile Gly Leu Leu Thr Thr Phe Met Tyr Ala Asn Lys Ser Ile Val Asn
            180                 185                 190

Gln Val Phe Leu Arg Glu Arg Ser Ser Lys Ile Gln Cys Ala Trp Leu
        195                 200                 205

Leu Val Phe Leu Ala Gly Ser Ser Val Leu Leu Tyr Tyr Thr Phe His
    210                 215                 220

Ser Gln Ser Leu Tyr Tyr Ser Leu Ile Phe Leu Asn Pro Thr Leu Asp
225                 230                 235                 240

His Leu Ser Phe Trp Glu Val Phe Xaa Ile Val Gly Xaa Thr Asp Phe
                245                 250                 255

Ile Leu Lys Phe Phe Phe Met Gly Leu Lys Cys Leu Ile Leu Leu Val
            260                 265                 270

Pro Ser Phe Ile Met Pro Phe Lys Ser Lys Gly Tyr Trp Tyr Met Leu
        275                 280                 285

Leu Glu Glu Leu Cys Gln Tyr Tyr Arg Thr Phe Val Pro Ile Pro Val
    290                 295                 300

Trp Phe Arg Tyr Leu Ile Ser Tyr Gly Glu Phe Gly Xaa Val Thr Arg
305                 310                 315                 320

Trp Xaa Leu Gly Ile Leu Leu Ala Leu Leu Tyr Leu Ile Leu Lys Leu
                325                 330                 335

Leu Glu Phe Phe Gly His Leu Arg Thr Phe Arg Gln Val Leu Arg Ile
            340                 345                 350

Phe Phe Thr Xaa Pro Ser Tyr Gly Val Ala Ala Ser Lys Arg Gln Cys
        355                 360                 365

Ser Asp Val Asp Asp Ile Cys Ser Ile Cys Gln Ala Glu Phe Gln Lys
    370                 375                 380

Pro Ile Leu Leu Ile Cys Gln His Ile Phe Cys Glu Glu Cys Met Thr
385                 390                 395                 400

Leu Trp Phe Asn Arg Glu Lys Thr Cys Pro Leu Cys Arg Thr Val Ile
                405                 410                 415

Ser Asp His Ile Asn Lys Trp Lys Asp Gly Ala Thr Ser Ser His Leu
            420                 425                 430

Gln Ile Tyr Xaa
        435

(2) INFORMATION FOR SEQ ID NO: 179:

(i) SEQUENCE CHARACTERISTICS:
(A) LENGTH: 175 amino acids
(B) TYPE: amino acid
(D) TOPOLOGY: linear
(xi) SEQUENCE DESCRIPTION: SEQ ID NO: 179:

Val Val Phe Gly Ala Ser Leu Phe Leu Leu Leu Ser Leu Thr Val Phe
1               5                   10                  15

Ser Ile Val Ser Val Thr Ala Tyr Ile Ala Leu Ala Leu Leu Ser Val
            20                  25                  30

Thr Ile Ser Phe Arg Ile Tyr Lys Gly Val Ile Gln Ala Ile Gln Lys
        35                  40                  45

Ser Asp Glu Gly His Pro Phe Arg Ala Tyr Leu Glu Ser Glu Val Ala
    50                  55                  60

Ile Ser Glu Glu Leu Val Gln Lys Tyr Ser Asn Ser Ala Leu Gly His
65                  70                  75                  80

Val Asn Cys Thr Ile Lys Glu Leu Arg Arg Leu Phe Leu Val Asp Asp
                85                  90                  95

Leu Val Asp Ser Leu Lys Phe Ala Val Leu Met Trp Val Phe Thr Tyr
            100                 105                 110

Val Gly Ala Leu Phe Asn Gly Leu Thr Leu Leu Ile Leu Ala Leu Ile
        115                 120                 125

Ser Leu Phe Ser Val Pro Val Ile Tyr Glu Arg His Gln Ala Gln Ile
    130                 135                 140

Asp His Tyr Leu Gly Leu Ala Asn Lys Asn Val Lys Asp Ala Met Ala
145                 150                 155                 160

Lys Ile Gln Ala Lys Ile Pro Gly Leu Lys Arg Lys Ala Glu Xaa
                165                 170                 175

(2) INFORMATION FOR SEQ ID NO: 180:

(i) SEQUENCE CHARACTERISTICS:

(A) LENGTH: 219 amino acids
(B) TYPE: amino acid
(D) TOPOLOGY: linear
(xi) SEQUENCE DESCRIPTION: SEQ ID NO: 180:

Met Glu Ala Pro Gly Ala Pro Pro Arg Thr Leu Thr Trp Glu Ala Met
1               5                   10                  15

Glu Gln Ile Arg Tyr Leu His Glu Glu Phe Pro Glu Ser Trp Ser Val
            20                  25                  30

Pro Arg Leu Ala Glu Gly Phe Asp Val Ser Thr Asp Val Ile Arg Arg
        35                  40                  45

Val Leu Lys Ser Lys Phe Leu Pro Thr Leu Glu Gln Lys Leu Lys Gln
    50                  55                  60

Asp Gln Lys Val Leu Lys Lys Ala Gly Leu Ala His Ser Leu Gln His
65                  70                  75                  80

Leu Arg Gly Ser Gly Asn Thr Ser Lys Leu Leu Pro Ala Gly His Ser
                85                  90                  95

Val Ser Gly Ser Leu Leu Met Pro Gly His Glu Ala Ser Ser Lys Asp
            100                 105                 110

Pro Asn His Ser Thr Ala Leu Lys Val Ile Glu Ser Asp Thr His Arg
        115                 120                 125

Thr Asn Thr Pro Arg Arg Arg Lys Gly Arg Asn Lys Glu Ile Gln Asp
    130                 135                 140

Leu Glu Glu Ser Phe Val Pro Val Ala Ala Pro Leu Gly His Pro Arg
145                 150                 155                 160

Glu Leu Gln Lys Tyr Ser Ser Asp Ser Glu Ser Pro Arg Gly Thr Gly
                165                 170                 175

Ser Gly Ala Leu Pro Ser Gly Gln Lys Leu Glu Glu Leu Lys Ala Glu
            180                 185                 190

Glu Pro Asp Asn Phe Ser Ser Lys Val Val Gln Arg Gly Arg Glu Phe
        195                 200                 205

Phe Asp Ser Asn Gly Asn Phe Leu Tyr Arg Ile
    210                 215

(2) INFORMATION FOR SEQ ID NO: 181:

(i) SEQUENCE CHARACTERISTICS:
(A) LENGTH: 6 amino acids
(B) TYPE: amino acid
(D) TOPOLOGY: linear
(xi) SEQUENCE DESCRIPTION: SEQ ID NO: 181:

Trp Lys Ala Glu Leu Xaa
1               5

(2) INFORMATION FOR SEQ ID NO: 182:

(i) SEQUENCE CHARACTERISTICS:
(A) LENGTH: 44 amino acids
(B) TYPE: amino acid
(D) TOPOLOGY: linear
(xi) SEQUENCE DESCRIPTION: SEQ ID NO: 182:

Met Ser Asn Thr Leu Leu Ser Gln Trp Leu Leu Leu Leu Thr Leu Phe
1               5                   10                  15

Lys Cys Ile Ile Leu Pro Leu Asn Leu Xaa Pro Ile Ile Arg Thr Ile
            20                  25                  30

Pro Asp Trp Ser Pro Glu Leu Gly Thr Asn Thr Xaa
        35                  40

(2) INFORMATION FOR SEQ ID NO: 183:

(i) SEQUENCE CHARACTERISTICS:
(A) LENGTH: 59 amino acids
(B) TYPE: amino acid
(D) TOPOLOGY: linear
(xi) SEQUENCE DESCRIPTION: SEQ ID NO: 183:

Met Trp Gln Val Arg Arg Gly Gly Cys Val Leu Ala Val Cys Ser Gln
1               5                   10                  15

Ala Arg Gly Thr Gly Gly Arg Leu Gly Trp Val Gly Thr Ser Ser Leu
            20                  25                  30

Arg Val Arg Met Ala Glu Ser Thr Ser Leu Met Ser Gln Gly Arg Ser
        35                  40                  45

Pro Ile Pro Arg Met Thr Pro Ala Arg Pro Xaa
    50                  55

(2) INFORMATION FOR SEQ ID NO: 184:

(i) SEQUENCE CHARACTERISTICS:
(A) LENGTH: 588 amino acids
(B) TYPE: amino acid
(D) TOPOLOGY: linear
(xi) SEQUENCE DESCRIPTION: SEQ ID NO: 184:

Met Arg Asp Ala Gly Asp Pro Ser Pro Pro Asn Lys Met Leu Arg Arg
1               5                   10                  15

Ser Asp Ser Pro Glu Asn Lys Tyr Ser Asp Ser Thr Gly His Ser Lys
            20                  25                  30

Ala Lys Asn Val His Thr His Arg Val Arg Glu Arg Asp Gly Gly Thr
        35                  40                  45

Ser Tyr Ser Pro Gln Glu Asn Ser His Asn His Ser Ala Leu His Ser
    50                  55                  60

Ser Asn Ser His Ser Ser Asn Pro Ser Asn Asn Pro Ser Lys Thr Ser
65                  70                  75                  80

Asp Ala Pro Tyr Asp Ser Ala Asp Asp Trp Ser Glu His Ile Ser Ser
                85                  90                  95

Ser Gly Lys Lys Tyr Tyr Tyr Asn Cys Arg Thr Glu Val Ser Gln Trp
            100                 105                 110

Glu Lys Pro Lys Glu Trp Leu Glu Arg Glu Gln Arg Gln Lys Glu Ala
        115                 120                 125

Asn Lys Met Ala Val Asn Ser Phe Pro Lys Asp Arg Asp Tyr Arg Arg
    130                 135                 140

Glu Val Met Gln Ala Thr Ala Thr Ser Gly Phe Ala Ser Gly Met Glu
145                 150                 155                 160

Asp Lys His Ser Ser Asp Ala Ser Ser Leu Leu Pro Gln Asn Ile Leu
                165                 170                 175

Ser Gln Thr Ser Arg His Asn Asp Arg Asp Tyr Arg Leu Pro Arg Ala
            180                 185                 190

Glu Thr His Ser Ser Ser Thr Pro Val Gln His Pro Ile Lys Pro Val
        195                 200                 205

Val His Pro Thr Ala Thr Pro Ser Thr Val Pro Ser Ser Pro Phe Thr
    210                 215                 220

Leu Gln Ser Asp His Gln Pro Lys Lys Ser Phe Asp Ala Asn Gly Ala
225                 230                 235                 240

Ser Thr Leu Ser Lys Leu Pro Thr Pro Thr Ser Ser Val Pro Ala Gln
                245                 250                 255

Lys Thr Glu Arg Lys Glu Ser Thr Ser Gly Asp Lys Pro Val Ser His
            260                 265                 270

Ser Cys Thr Thr Pro Ser Thr Ser Ser Ala Ser Gly Leu Asn Pro Thr
        275                 280                 285

Ser Ala Pro Pro Thr Ser Ala Ser Ala Val Pro Val Ser Pro Val Pro
    290                 295                 300

Gln Ser Pro Ile Pro Pro Leu Leu Gln Asp Pro Asn Leu Leu Arg Gln
305                 310                 315                 320

Leu Leu Pro Ala Leu Gln Ala Thr Leu Gln Leu Asn Asn Ser Asn Val
                325                 330                 335

Asp Ile Ser Lys Ile Asn Glu Val Leu Thr Ala Ala Val Thr Gln Ala
            340                 345                 350

Ser Leu Gln Ser Ile Ile His Lys Phe Leu Thr Ala Gly Pro Ser Ala
        355                 360                 365

Phe Asn Ile Thr Ser Leu Ile Ser Gln Ala Ala Gln Leu Ser Thr Gln
    370                 375                 380

Ala Gln Pro Ser Asn Gln Ser Pro Met Ser Leu Thr Ser Asp Ala Ser
385                 390                 395                 400

Ser Pro Arg Ser Tyr Val Ser Pro Arg Ile Ser Thr Pro Gln Thr Asn
                405                 410                 415

Thr Val Pro Ile Lys Pro Leu Ile Ser Thr Pro Pro Val Ser Ser Gln
            420                 425                 430

Pro Lys Val Ser Thr Pro Val Val Lys Gln Gly Pro Val Ser Gln Ser
        435                 440                 445

Ala Thr Gln Gln Pro Val Thr Ala Asp Lys Xaa Gln Gly His Glu Pro
    450                 455                 460

Val Ser Pro Arg Ser Leu Gln Arg Ser Ser Ser Gln Arg Ser Pro Ser
465                 470                 475                 480

Pro Gly Pro Asn His Thr Ser Asn Ser Ser Asn Ala Ser Asn Ala Thr
                485                 490                 495

Val Val Pro Gln Asn Ser Ser Ala Arg Ser Thr Cys Ser Leu Thr Pro
            500                 505                 510

Ala Leu Ala Ala His Phe Ser Glu Asn Leu Ile Lys His Val Gln Gly
        515                 520                 525

Trp Pro Ala Asp His Ala Glu Lys Gln Ala Ser Arg Leu Arg Glu Glu
    530                 535                 540

Ala His Asn Met Gly Thr Ile His Met Ser Glu Ile Cys Thr Glu Leu
545                 550                 555                 560

Lys Asn Leu Arg Ser Leu Val Arg Val Cys Glu Ile Gln Ala Thr Leu
                565                 570                 575

Arg Glu Gln Arg Asp Thr Ile Phe Glu Thr Thr Asn
            580                 585

(2) INFORMATION FOR SEQ ID NO: 185:

(i) SEQUENCE CHARACTERISTICS:
(A) LENGTH: 166 amino acids
(B) TYPE: amino acid
(D) TOPOLOGY: linear
(xi) SEQUENCE DESCRIPTION: SEQ ID NO: 185:

Met Asn Ile Lys His Leu Val Asp Pro Ile Asp Asp Leu Phe Leu Ala
1               5                   10                  15

Ala Lys Lys Ile Pro Gly Ile Ser Ser Thr Gly Val Gly Asp Gly Gly
            20                  25                  30

Asn Glu Leu Gly Met Gly Lys Val Lys Glu Ala Val Arg Arg His Ile
        35                  40                  45

Arg His Gly Asp Val Ile Ala Cys Asp Val Glu Ala Asp Phe Ala Val
    50                  55                  60

Ile Ala Gly Val Ser Asn Trp Gly Gly Tyr Ala Leu Ala Cys Ala Leu
65                  70                  75                  80

Tyr Ile Leu Tyr Ser Cys Ala Val His Ser Gln Tyr Leu Arg Lys Ala
                85                  90                  95

Val Gly Pro Ser Arg Ala Pro Gly Asp Gln Ala Trp Thr Gln Ala Leu
            100                 105                 110

Pro Ser Val Ile Lys Glu Glu Lys Met Leu Gly Ile Leu Val Gln His
        115                 120                 125

Lys Val Arg Ser Gly Val Ser Gly Ile Val Gly Met Glu Val Asp Gly
    130                 135                 140

Leu Pro Phe His Asn Xaa His Ala Glu Met Ile Gln Lys Leu Val Asp
145                 150                 155                 160

Val Thr Thr Ala Gln Val
                165

(2) INFORMATION FOR SEQ ID NO: 186:

(i) SEQUENCE CHARACTERISTICS:
(A) LENGTH: 9 amino acids
(B) TYPE: amino acid
(D) TOPOLOGY: linear
(xi) SEQUENCE DESCRIPTION: SEQ ID NO: 186:

Met Leu Ile Leu Phe Leu Lys Lys Xaa
1               5

(2) INFORMATION FOR SEQ ID NO: 187:

(i) SEQUENCE CHARACTERISTICS:
(A) LENGTH: 20 amino acids
(B) TYPE: amino acid
(D) TOPOLOGY: linear
(xi) SEQUENCE DESCRIPTION: SEQ ID NO: 187:

Thr His Thr His Thr His Pro Lys Ser Phe Tyr Ile Ile Lys Leu Ser
1               5                   10                  15

Tyr Tyr Tyr Xaa
            20

(2) INFORMATION FOR SEQ ID NO: 188:

(i) SEQUENCE CHARACTERISTICS:
(A) LENGTH: 32 amino acids

(B) TYPE: amino acid
(D) TOPOLOGY: linear
(xi) SEQUENCE DESCRIPTION: SEQ ID NO: 188:

Met Ile Gln Ser Gly Leu Ile Ala Ile Leu Leu Ser Phe Leu Lys Val
1               5                   10                  15

Tyr Val Glu Gly Arg Pro Cys Val Cys Phe Ser Lys Gly Leu Xaa Xaa
            20                  25                  30

(2) INFORMATION FOR SEQ ID NO: 189:

(i) SEQUENCE CHARACTERISTICS:
(A) LENGTH: 19 amino acids
(B) TYPE: amino acid
(D) TOPOLOGY: linear
(xi) SEQUENCE DESCRIPTION: SEQ ID NO: 189:

Tyr Ile Tyr Leu Ile Val Tyr Ile Ser Phe Tyr Ser Phe Arg Pro Gln
1               5                   10                  15

Gln Leu Xaa

(2) INFORMATION FOR SEQ ID NO: 190:

(i) SEQUENCE CHARACTERISTICS:
(A) LENGTH: 33 amino acids
(B) TYPE: amino acid
(D) TOPOLOGY: linear
(xi) SEQUENCE DESCRIPTION: SEQ ID NO: 190:

Met Arg Phe Leu Leu Thr Val Trp Gly Ser Phe Pro Phe Met Leu Ile
1               5                   10                  15

Pro Val Phe Leu Ser Ile Gly Thr Lys Glu Met Lys Lys Ala Gln Arg
            20                  25                  30

Xaa

(2) INFORMATION FOR SEQ ID NO: 191:

(i) SEQUENCE CHARACTERISTICS:
(A) LENGTH: 84 amino acids
(B) TYPE: amino acid
(D) TOPOLOGY: linear
(xi) SEQUENCE DESCRIPTION: SEQ ID NO: 191:

Met Arg Val Pro Pro Val Leu Arg Gly Arg Ile Leu Pro Leu Val Leu
1               5                   10                  15

Gln Cys Thr Leu Leu Glu Phe Cys Leu Cys Ala Thr Thr Val Leu Pro
            20                  25                  30

Thr Val Xaa Cys Trp Lys Pro Arg Leu Pro Val Xaa Ala Ser Gly Leu
        35                  40                  45

Tyr Val Asp Arg Met Ser Leu Trp Lys Tyr Gly Cys Ser Gly Trp Asn
    50                  55                  60

Glu Ser Ala Arg Pro Arg Arg Ala Gly Gly Thr Met Arg Pro Pro Arg
65                  70                  75                  80

Ser Gly Arg Xaa

(2) INFORMATION FOR SEQ ID NO: 192:

(i) SEQUENCE CHARACTERISTICS:
(A) LENGTH: 123 amino acids
(B) TYPE: amino acid
(D) TOPOLOGY: linear
(xi) SEQUENCE DESCRIPTION: SEQ ID NO: 192:

Met Ala Gly Ala Phe Val Ala Val Phe Leu Leu Ala Met Phe Tyr Glu
1               5                   10                  15

Gly Leu Lys Ile Ala Arg Glu Ser Leu Leu Arg Lys Ser Gln Val Ser
            20                  25                  30

Ile Arg Tyr Asn Ser Met Pro Val Pro Gly Pro Asn Gly Thr Ile Leu
        35                  40                  45

Met Glu Thr His Lys Thr Val Gly Gln Gln Met Leu Ser Phe Pro His
    50                  55                  60

Leu Leu Gln Thr Val Leu His Ile Ile Gln Val Val Ile Ser Tyr Phe
65                  70                  75                  80

Leu Met Leu Ile Phe Met Thr Tyr Asn Gly Tyr Leu Cys Ile Ala Xaa
                85                  90                  95

Ala Ala Gly Ala Gly Thr Gly Tyr Phe Leu Phe Ser Trp Lys Lys Ala
            100                 105                 110

Val Val Val Asp Ile Thr Glu His Cys His Xaa
        115                 120

(2) INFORMATION FOR SEQ ID NO: 193:

(i) SEQUENCE CHARACTERISTICS:
(A) LENGTH: 143 amino acids
(B) TYPE: amino acid
(D) TOPOLOGY: linear
(xi) SEQUENCE DESCRIPTION: SEQ ID NO: 193:

Met Gly Cys Leu Val Trp Gly Pro Ser Trp Pro Pro Leu Ser Leu Leu
1               5                   10                  15

Ala Ser Leu Leu His Ser Gly Ile Ala Gly Arg Cys Leu Leu Cys Leu
            20                  25                  30

Phe Lys Gly Leu Ala Ala Ala Ala Ser Leu Gln Ile Arg Asp Leu Ala
        35                  40                  45

Ser Arg Leu Thr Thr Gly Pro Arg Thr Cys Arg Val Gln Pro Pro Pro
    50                  55                  60

His Pro Gln Ser Ser Pro Pro Trp Pro Gly Pro Pro Gly Ala Glu Thr
65                  70                  75                  80

Cys Arg Pro Leu Ser Arg Thr Val Gly Gly Val Cys Pro Ser Asp Trp
                85                  90                  95

Pro Val Ser Trp Leu Leu Leu Pro Pro Leu Pro Glu Val Val Thr Cys
            100                 105                 110

Ser Cys Pro Arg Ile Lys Ala Arg Pro Glu Arg Thr Pro Glu Leu Leu
        115                 120                 125

Cys Ala Trp Gly Gly Arg Gly Lys His Ser Gln Leu Val Ala Xaa
    130                 135                 140

(2) INFORMATION FOR SEQ ID NO: 194:

(i) SEQUENCE CHARACTERISTICS:
(A) LENGTH: 51 amino acids
(B) TYPE: amino acid
(D) TOPOLOGY: linear
(xi) SEQUENCE DESCRIPTION: SEQ ID NO: 194:

Met Pro Asn Val Met Leu Thr Leu Phe Val Met Thr Leu Ser Ser Ala
1               5                   10                  15

Ser Asn Leu Gly Leu Tyr Phe Phe Lys Phe Asn Phe Glu Cys Ser Cys
            20                  25                  30

Met Phe Gly Thr Ser Leu Leu Thr Ala Lys Asp Lys Leu Phe Ile Cys
        35                  40                  45

Ile Thr Xaa
    50

(2) INFORMATION FOR SEQ ID NO: 195:

(i) SEQUENCE CHARACTERISTICS:
(A) LENGTH: 222 amino acids
(B) TYPE: amino acid
(D) TOPOLOGY: linear
(xi) SEQUENCE DESCRIPTION: SEQ ID NO: 195:

Met Ser Leu Leu Val Leu Val Leu Ser Trp Gly Ser Met Gly Leu Glu

1                   5                   10                  15

Ala Ala Thr Ala Val Gly Leu Ser Asp Phe Cys Ser Asn Pro Asp Pro
                20                  25                  30

Tyr Val Leu Asn Leu Thr Gln Glu Glu Thr Gly Leu Ser Ser Asp Ile
            35                  40                  45

Leu Ser Tyr Tyr Leu Leu Cys Asn Arg Ala Val Ser Asn Pro Phe Gln
        50                  55                  60

Gln Arg Leu Thr Leu Ser Gln Arg Ala Leu Ala Asn Ile His Ser Gln
65                  70                  75                  80

Leu Leu Gly Leu Glu Arg Glu Ala Val Pro Gln Phe Pro Ser Ala Gln
                85                  90                  95

Lys Pro Leu Leu Ser Leu Glu Glu Thr Leu Asn Val Thr Glu Gly Asn
            100                 105                 110

Phe His Gln Leu Val Ala Leu Leu His Cys Arg Ser Leu His Lys Asp
        115                 120                 125

Tyr Gly Ala Ala Leu Arg Gly Leu Cys Glu Xaa Xaa Leu Glu Gly Leu
    130                 135                 140

Leu Phe Leu Leu Leu Phe Ser Leu Leu Ser Ala Gly Ala Leu Ala Xaa
145                 150                 155                 160

Ala Leu Cys Xaa Leu Pro Arg Ala Trp Ala Leu Phe Pro Pro Arg Asn
                165                 170                 175

Pro Ser Ala Leu Cys Ser Gly Ser Arg Leu Ser Glu Pro Leu Leu Pro
            180                 185                 190

Ala Gly Leu Glu Pro Gly Ser Pro Leu Arg Ser Phe Pro Gly Cys Arg
        195                 200                 205

Arg Asp Pro Thr Asn Pro Ala Cys Leu Gly Ser Asp His Xaa
    210                 215                 220

(2) INFORMATION FOR SEQ ID NO: 196:

(i) SEQUENCE CHARACTERISTICS:
(A) LENGTH: 102 amino acids
(B) TYPE: amino acid
(D) TOPOLOGY: linear
(xi) SEQUENCE DESCRIPTION: SEQ ID NO: 196:

Met Ser Gln Leu Ser Arg Thr Ser Leu Ser Leu Leu Leu Thr Leu Leu
1               5                   10                  15

Val Leu Trp Gly Ser Ser Cys Cys Leu Pro Ile Trp Cys Leu Pro Asn
            20                  25                  30

Arg His Arg Leu Leu Lys Leu Ser Phe Leu Leu Phe Ser Pro Asp Ile
        35                  40                  45

Pro Tyr Leu Ser His Thr His Pro Asn Asn Ile Ser Cys Ser Val Leu
    50                  55                  60

Ser Leu Arg Gln His Leu Asn Phe Thr Gln Pro Gly Ala Leu Phe Thr
65                  70                  75                  80

Cys Leu Val Gln Ile Gln Phe Gly Leu Ile Leu Gln Pro Cys Ile Ser
                85                  90                  95

Lys Trp Gly Leu Gly Xaa
            100

(2) INFORMATION FOR SEQ ID NO: 197:

(i) SEQUENCE CHARACTERISTICS:
(A) LENGTH: 13 amino acids
(B) TYPE: amino acid
(D) TOPOLOGY: linear
(xi) SEQUENCE DESCRIPTION: SEQ ID NO: 197:

Met Ile Ala Leu Phe Phe Val Thr Thr Xaa Leu Thr Xaa
1               5                   10

(2) INFORMATION FOR SEQ ID NO: 198:

(i) SEQUENCE CHARACTERISTICS:
(A) LENGTH: 61 amino acids
(B) TYPE: amino acid
(D) TOPOLOGY: linear
(xi) SEQUENCE DESCRIPTION: SEQ ID NO: 198:

Met Thr Tyr His Pro Asn Gln Val Val Glu Gly Cys Cys Ser Asp Met
1               5                   10                  15

Ala Val Thr Phe Asn Gly Leu Thr Pro Asn Gln Met His Val Met Met
            20                  25                  30

Tyr Gly Val Tyr Arg Leu Arg Ala Phe Gly His Ile Phe Asn Asp Ala
        35                  40                  45

Leu Val Phe Leu Pro Pro Asn Gly Ser Asp Asn Asp Xaa
    50                  55                  60

(2) INFORMATION FOR SEQ ID NO: 199:

(i) SEQUENCE CHARACTERISTICS:
(A) LENGTH: 71 amino acids
(B) TYPE: amino acid
(D) TOPOLOGY: linear
(xi) SEQUENCE DESCRIPTION: SEQ ID NO: 199:

Met Ser Ser Ser Ser Leu His Trp Lys Glu Phe Lys Tyr Ala Pro Gly
1               5                   10                  15

Ser Leu His Tyr Phe Ala Leu Ser Phe Val Leu Ile Leu Thr Glu Ile
            20                  25                  30

Cys Leu Val Ser Ser Gly Met Gly Phe Pro Gln Glu Gly Lys His Phe
        35                  40                  45

Ser Val Leu Gly Ser Pro Asp Cys Ser Leu Trp Gly Arg Asp Glu His
    50                  55                  60

Val Pro Arg Glu Phe Ala Xaa
65                  70

(2) INFORMATION FOR SEQ ID NO: 200:

(i) SEQUENCE CHARACTERISTICS:
(A) LENGTH: 10 amino acids
(B) TYPE: amino acid
(D) TOPOLOGY: linear
(xi) SEQUENCE DESCRIPTION: SEQ ID NO: 200:

Met His Leu Arg Phe Pro Phe Leu Cys Xaa
1               5                   10

(2) INFORMATION FOR SEQ ID NO: 201:

(i) SEQUENCE CHARACTERISTICS:
(A) LENGTH: 50 amino acids
(B) TYPE: amino acid
(D) TOPOLOGY: linear
(xi) SEQUENCE DESCRIPTION: SEQ ID NO: 201:

Met Arg Arg Val Ala Arg Gly Arg Gly Leu Ala Leu Pro Ser Leu Glu
1               5                   10                  15

His Arg Pro Ser Cys Ser Tyr Asp Ala Leu Pro Leu Pro Phe Cys Glu
            20                  25                  30

Thr Arg Asn Pro Glu Ala His Leu Tyr Phe Phe Arg Thr Asp Val Glu
        35                  40                  45

Arg Xaa
    50

(2) INFORMATION FOR SEQ ID NO: 202:

(i) SEQUENCE CHARACTERISTICS:
(A) LENGTH: 13 amino acids
(B) TYPE: amino acid
(D) TOPOLOGY: linear
(xi) SEQUENCE DESCRIPTION: SEQ ID NO: 202:

Ala Lys Ile Leu Val Phe Ile Phe Leu Phe Glu Leu Xaa
1               5                   10

(2) INFORMATION FOR SEQ ID NO: 203:

(i) SEQUENCE CHARACTERISTICS:
(A) LENGTH: 38 amino acids
(B) TYPE: amino acid
(D) TOPOLOGY: linear
(xi) SEQUENCE DESCRIPTION: SEQ ID NO: 203:

Met Phe Gln Glu Cys Ile Pro Ile Ser Leu Phe Phe Leu Asn Trp Leu
1               5                   10                  15

Lys Glu Cys Cys Ser Phe Thr Cys Pro Asn Ser His Ile Asn Asn Cys
            20                  25                  30

Leu Thr Gly Ile Arg Xaa
        35

(2) INFORMATION FOR SEQ ID NO: 204:

(i) SEQUENCE CHARACTERISTICS:
(A) LENGTH: 34 amino acids
(B) TYPE: amino acid
(D) TOPOLOGY: linear
(xi) SEQUENCE DESCRIPTION: SEQ ID NO: 204:

Met Asn Phe Val Leu Phe Phe Ile Gly Ile Asn Val Gly Cys Arg Gly
1               5                   10                  15

Glu Asn Ser Leu Lys Tyr Phe Thr Val Thr Val Xaa Cys Ser Pro Arg
            20                  25                  30

Asp Xaa

(2) INFORMATION FOR SEQ ID NO: 205:

(i) SEQUENCE CHARACTERISTICS:
(A) LENGTH: 26 amino acids
(B) TYPE: amino acid
(D) TOPOLOGY: linear
(xi) SEQUENCE DESCRIPTION: SEQ ID NO: 205:

Met Leu Leu Phe Leu Phe Val Cys Leu Pro Ile Thr Trp Met Ala Glu
1               5                   10                  15

Phe Leu Ser Gln Leu Arg His Leu Leu Xaa
            20                  25

(2) INFORMATION FOR SEQ ID NO: 206:

(i) SEQUENCE CHARACTERISTICS:
(A) LENGTH: 105 amino acids

(B) TYPE: amino acid
(D) TOPOLOGY: linear
(xi) SEQUENCE DESCRIPTION: SEQ ID NO: 206:

Met Pro Arg His Ser Leu Tyr Ile Ile Ile Gly Ala Leu Cys Val Ala
1               5                   10                  15

Phe Ile Leu Met Leu Ile Ile Leu Ile Val Gly Ile Cys Arg Ile Ser
            20                  25                  30

Arg Ile Glu Tyr Gln Gly Ser Ser Arg Pro Ala Tyr Glu Glu Phe Tyr
        35                  40                  45

Asn Cys Arg Ser Ile Asp Ser Glu Phe Ser Asn Ala Ile Ala Ser Ile
    50                  55                  60

Arg His Ala Arg Phe Gly Lys Lys Ser Arg Pro Ala Met Tyr Asp Val
65                  70                  75                  80

Ser Pro Ile Ala Tyr Glu Asp Tyr Ser Pro Asp Asp Lys Pro Leu Val
                85                  90                  95

Thr Leu Ile Lys Thr Lys Asp Leu Xaa
            100                 105

(2) INFORMATION FOR SEQ ID NO: 207:

(i) SEQUENCE CHARACTERISTICS:
(A) LENGTH: 64 amino acids
(B) TYPE: amino acid
(D) TOPOLOGY: linear
(xi) SEQUENCE DESCRIPTION: SEQ ID NO: 207:

Leu Lys Ser Cys Leu Leu Leu Val Ser Phe Leu Ser Gly Arg Val Pro
1               5                   10                  15

Ser Tyr Asp Leu Ile Tyr Val Cys Ser Ile Ala Leu Glu Thr Gly Phe
            20                  25                  30

Val Cys Glu Met Ala Leu Ser Phe Val Asp His Phe Cys Arg Glu Ile
        35                  40                  45

Val Asp Leu Gly Arg Ala Glu Ala Thr Ala Asp Met Pro Gly Val Xaa
    50                  55                  60

(2) INFORMATION FOR SEQ ID NO: 208:

(i) SEQUENCE CHARACTERISTICS:
(A) LENGTH: 42 amino acids
(B) TYPE: amino acid
(D) TOPOLOGY: linear
(xi) SEQUENCE DESCRIPTION: SEQ ID NO: 208:

Met Ser Ala Trp Leu Pro Ser Pro Pro His Leu Leu Leu Leu Ser Ala
1               5                   10                  15

Ala Ala Gly Ser Gly Ala Ser His Leu Arg Ala Leu Gly Ser Ser Ala
            20                  25                  30

Leu Glu Gly Leu Gln Asp Pro Ser Gln Xaa
        35                  40

(2) INFORMATION FOR SEQ ID NO: 209:

(i) SEQUENCE CHARACTERISTICS:
(A) LENGTH: 42 amino acids
(B) TYPE: amino acid
(D) TOPOLOGY: linear
(xi) SEQUENCE DESCRIPTION: SEQ ID NO: 209:

Met Ser Ser Pro Ala Thr Trp Arg Leu Thr Leu Pro Ser Leu Leu Val
1               5                   10                  15

Phe Leu Thr Gly Glu Ala Met Pro Trp Pro Ala His Ser Thr Ser Cys
            20                  25                  30

Thr His Val Leu Ser Thr Val Ser Thr Xaa
        35                  40

(2) INFORMATION FOR SEQ ID NO: 210:

(i) SEQUENCE CHARACTERISTICS:
(A) LENGTH: 46 amino acids
(B) TYPE: amino acid
(D) TOPOLOGY: linear
(xi) SEQUENCE DESCRIPTION: SEQ ID NO: 210:

Met Gln Ala Pro Leu Gln Asp Cys Gly Arg Ser Val Ser Leu Arg Leu
1               5                   10                  15

Ala Cys Val Leu Ala Pro Leu Thr Thr Ser Ser Arg Gly Cys His Leu
            20                  25                  30

Gln Leu Pro Gln Asp Lys Gly Lys Ala Arg Xaa Asp Ser Xaa
        35                  40                  45

(2) INFORMATION FOR SEQ ID NO: 211:

(i) SEQUENCE CHARACTERISTICS:
(A) LENGTH: 266 amino acids
(B) TYPE: amino acid
(D) TOPOLOGY: linear
(xi) SEQUENCE DESCRIPTION: SEQ ID NO: 211:

Met Asn Gly Ser His Lys Asp Pro Leu Leu Pro Phe Pro Ala Ser Ala
1               5                   10                  15

Arg Thr Pro Ser Leu Pro Pro Ala Pro Pro Ala Gln Ala Pro Leu Pro
            20                  25                  30

Trp Lys Pro Ser Gly Phe Ala Arg Ile Ser Pro Pro Pro Pro Leu Ala
        35                  40                  45

Ile Leu Gln Tyr Arg Gly Lys Ala Asp His Gly Glu Ser Gly Gln Gln
    50                  55                  60

Leu Ala Ala Ala Pro Gly Asp Gly Arg Leu Pro Leu Leu Glu Ala Val
65                  70                  75                  80

Arg Arg Leu Arg Gly Gln Asp Cys Gly Pro Leu Ser Ala Leu Cys His
                85                  90                  95

Gly Gln Leu Leu Ala Gln Pro Val Pro Gln Val Leu Leu Leu Pro Gly
            100                 105                 110

Ala Xaa Gly Asp Ile Gly Thr Ser Cys Tyr Thr Lys Ser Gly Met Ile
        115                 120                 125

Leu Cys Arg Asn Asp Tyr Ile Arg Leu Phe Gly Asn Ser Gly Ala Cys
    130                 135                 140

Ser Ala Cys Gly Gln Ser Ile Pro Ala Ser Glu Leu Val Met Arg Ala
145                 150                 155                 160

Gln Gly Asn Val Tyr His Leu Lys Cys Phe Thr Cys Ser Thr Cys Arg
                165                 170                 175

Asn Arg Leu Val Pro Gly Asp Arg Phe His Tyr Ile Asn Gly Ser Leu
            180                 185                 190

Phe Cys Glu His Asp Arg Pro Thr Ala Leu Ile Asn Gly His Leu Asn
        195                 200                 205

Ser Leu Gln Ser Asn Pro Leu Leu Pro Asp Gln Lys Val Cys Lys Val
    210                 215                 220

Arg Val Met Gln Asn Ala Cys Leu His Leu Arg Phe Val His His Arg
225                 230                 235                 240

Trp Ile Pro Cys Xaa Phe Ser Arg Gln Val Thr Phe Val Ala Ser Thr
                245                 250                 255

Ser Ala Ser Ser Met Pro Leu His Leu Leu
            260                 265

(2) INFORMATION FOR SEQ ID NO: 212:

(i) SEQUENCE CHARACTERISTICS:
(A) LENGTH: 94 amino acids
(B) TYPE: amino acid
(D) TOPOLOGY: linear
(xi) SEQUENCE DESCRIPTION: SEQ ID NO: 212:

Met Ala Arg Thr Arg Thr Pro Ser Ser Pro Phe Leu Leu Leu Arg Glu
1               5                   10                  15

Leu Pro Pro Ser Leu Gln Leu Arg Gln Pro Arg Arg Pro Phe Pro Gly
            20                  25                  30

Ser Arg Ala Ala Ser Leu Ala Phe His Arg Arg Arg Leu Ser Gln Tyr
        35                  40                  45

Cys Asn Ile Gly Glu Lys Gln Thr Met Val Asn Pro Gly Ser Ser Ser
    50                  55                  60

Gln Pro Pro Pro Val Thr Ala Gly Ser Leu Ser Trp Lys Arg Cys Ala
65                  70                  75                  80

Gly Cys Gly Gly Lys Ile Ala Asp Arg Phe Leu Leu Tyr Ala
                85                  90

(2) INFORMATION FOR SEQ ID NO: 213:

        (i) SEQUENCE CHARACTERISTICS:
                (A) LENGTH: 24 amino acids
                (B) TYPE: amino acid
                (D) TOPOLOGY: linear
        (xi) SEQUENCE DESCRIPTION: SEQ ID NO: 213:

Leu Phe Gly Asn Ser Gly Ala Cys Ser Ala Cys Gly Gln Ser Ile Pro
  1               5                  10                  15

Ala Ser Glu Leu Val Met Arg Ala
            20

(2) INFORMATION FOR SEQ ID NO: 214:

        (i) SEQUENCE CHARACTERISTICS:
                (A) LENGTH: 19 amino acids
                (B) TYPE: amino acid
                (D) TOPOLOGY: linear
        (xi) SEQUENCE DESCRIPTION: SEQ ID NO: 214:

His Asp Arg Pro Thr Ala Leu Ile Asn Gly His Leu Asn Ser Leu Gln
  1               5                  10                  15

Ser Asn Pro

(2) INFORMATION FOR SEQ ID NO: 215:

        (i) SEQUENCE CHARACTERISTICS:
                (A) LENGTH: 12 amino acids
                (B) TYPE: amino acid
                (D) TOPOLOGY: linear
        (xi) SEQUENCE DESCRIPTION: SEQ ID NO: 215:

Leu Val Pro Gly Asp Arg Phe His Tyr Ile Asn Gly
  1               5                  10

(2) INFORMATION FOR SEQ ID NO: 216:

(i) SEQUENCE CHARACTERISTICS:
(A) LENGTH: 81 amino acids
(B) TYPE: amino acid
(D) TOPOLOGY: linear
(xi) SEQUENCE DESCRIPTION: SEQ ID NO: 216:

Met Lys Tyr Met Gly Gly Cys Ala Lys Val Met Cys Lys Tyr Tyr Val
1               5                   10                  15

Ile Leu Tyr Gln Gly Leu Glu Tyr Pro Leu Leu Xaa Ser Gly Asp Pro
            20                  25                  30

Glu Thr Ser Pro Pro Trp Ile Leu Arg Ala Asp Cys Ile Val Leu Ser
        35                  40                  45

Ser Arg Asn Phe His Ser Asn Xaa Gly Arg Leu Thr Ile Asn Lys Ile
    50                  55                  60

Tyr Val Ile Gly Gly Gly Lys Tyr Arg Gly Glu Val Thr Asn Gly Ala
65                  70                  75                  80

Lys

(2) INFORMATION FOR SEQ ID NO: 217:

(i) SEQUENCE CHARACTERISTICS:
(A) LENGTH: 41 amino acids
(B) TYPE: amino acid
(D) TOPOLOGY: linear
(xi) SEQUENCE DESCRIPTION: SEQ ID NO: 217:

(2) INFORMATION FOR SEQ ID NO: 218:

(i) SEQUENCE CHARACTERISTICS:
(A) LENGTH: 35 amino acids
(B) TYPE: amino acid
(D) TOPOLOGY: linear
(xi) SEQUENCE DESCRIPTION: SEQ ID NO: 218:

Met Val Leu Leu Leu Leu Thr Val Ala Ser Tyr Thr Val Phe Trp Met
1               5                   10                  15

Ile Gly Asp Val Leu Asp Ile Leu Phe Leu Trp Asn Phe Glu Tyr Thr
            20                  25                  30

Thr Leu Tyr
        35

(2) INFORMATION FOR SEQ ID NO: 219:

        (i) SEQUENCE CHARACTERISTICS:
                (A) LENGTH: 38 amino acids
                (B) TYPE: amino acid
                (D) TOPOLOGY: linear
        (xi) SEQUENCE DESCRIPTION: SEQ ID NO: 219:

Met Glu Leu Tyr Asn Ser Leu Cys Pro Ile Cys Tyr Phe Ser Thr Val
  1               5                  10                  15

Leu Thr Thr Thr Tyr Tyr Ile Tyr Phe Val Tyr Ser Gln Ser Ser Xaa
            20                  25                  30

Ile Arg Met Lys Val Pro
        35

(2) INFORMATION FOR SEQ ID NO: 220:

        (i) SEQUENCE CHARACTERISTICS:
                (A) LENGTH: 45 amino acids
                (B) TYPE: amino acid
                (D) TOPOLOGY: linear
        (xi) SEQUENCE DESCRIPTION: SEQ ID NO: 220:

Met Gln Ile Val Ile Val Leu Tyr Cys Val Arg Asn Lys Asp Lys Lys
  1               5                  10                  15

Lys Val Cys Thr Cys Ser Val Gln Thr Gln Phe Phe Phe Pro Ile Phe
            20                  25                  30

Pro Ile Leu Gly Cys Leu Asn Gly Cys Arg Thr Gln Glu
        35                  40                  45

(2) INFORMATION FOR SEQ ID NO: 221:

        (i) SEQUENCE CHARACTERISTICS:
                (A) LENGTH: 28 amino acids
                (B) TYPE: amino acid
                (D) TOPOLOGY: linear
        (xi) SEQUENCE DESCRIPTION: SEQ ID NO: 221:

Met Lys Tyr Met Gly Gly Cys Ala Lys Val Met Cys Lys Tyr Tyr Val
  1               5                  10                  15

Ile Leu Tyr Gln Gly Leu Glu Tyr Pro Leu Leu Xaa
            20                  25

(2) INFORMATION FOR SEQ ID NO: 222:

(i) SEQUENCE CHARACTERISTICS:
(A) LENGTH: 35 amino acids
(B) TYPE: amino acid
(D) TOPOLOGY: linear
(xi) SEQUENCE DESCRIPTION: SEQ ID NO: 222:

Leu Glu Tyr Pro Leu Leu Xaa Ser Gly Asp Pro Glu Thr Ser Pro Pro
1               5                   10                  15

Trp Ile Leu Arg Ala Asp Cys Ile Val Leu Ser Ser Arg Asn Phe His
            20                  25                  30

Ser Asn Xaa
        35

(2) INFORMATION FOR SEQ ID NO: 223:

(i) SEQUENCE CHARACTERISTICS:
(A) LENGTH: 32 amino acids
(B) TYPE: amino acid
(D) TOPOLOGY: linear
(xi) SEQUENCE DESCRIPTION: SEQ ID NO: 223:

Arg Asn Phe His Ser Asn Xaa Gly Arg Leu Thr Ile Asn Lys Ile Tyr
1               5                   10                  15

Val Ile Gly Gly Gly Lys Tyr Arg Gly Glu Val Thr Asn Gly Ala Lys
            20                  25                  30

(2) INFORMATION FOR SEQ ID NO: 224:

(i) SEQUENCE CHARACTERISTICS:
(A) LENGTH: 145 amino acids
(B) TYPE: amino acid
(D) TOPOLOGY: linear
(xi) SEQUENCE DESCRIPTION: SEQ ID NO: 224:

Val Thr Asn Glu Met Ser Gln Gly Arg Gly Lys Tyr Asp Phe Tyr Ile
1               5                   10                  15

Gly Leu Gly Leu Ala Met Ser Ser Ser Ile Phe Ile Gly Gly Ser Phe
            20                  25                  30

Ile Leu Lys Lys Lys Gly Leu Leu Arg Leu Ala Arg Lys Gly Ser Met
        35                  40                  45

Arg Ala Gly Gln Gly Gly His Ala Tyr Leu Lys Glu Trp Leu Trp Trp
    50                  55                  60

Ala Gly Leu Leu Ser Met Gly Ala Gly Glu Val Ala Asn Phe Ala Ala
65                  70                  75                  80

Tyr Ala Phe Ala Pro Ala Thr Leu Val Thr Pro Leu Gly Ala Leu Ser
                85                  90                  95

Val Leu Val Ser Ala Ile Leu Ser Ser Tyr Phe Leu Asn Glu Arg Leu
            100                 105                 110

Asn Leu His Gly Lys Ile Gly Cys Leu Leu Ser Ile Leu Gly Ser Thr
        115                 120                 125

Val Met Val Ile His Ala Pro Lys Glu Glu Glu Ile Glu Thr Leu Asn
    130                 135                 140

Glu
145

(2) INFORMATION FOR SEQ ID NO: 225:

(i) SEQUENCE CHARACTERISTICS:
(A) LENGTH: 78 amino acids
(B) TYPE: amino acid
(D) TOPOLOGY: linear
(xi) SEQUENCE DESCRIPTION: SEQ ID NO: 225:

Val Thr Asn Glu Met Ser Gln Gly Arg Gly Lys Tyr Asp Phe Tyr Ile
1               5                   10                  15

Gly Leu Gly Leu Ala Met Ser Ser Ser Ile Phe Ile Gly Gly Ser Phe
            20                  25                  30

Ile Leu Lys Lys Lys Gly Leu Leu Arg Leu Ala Arg Lys Gly Ser Met
        35                  40                  45

Arg Ala Gly Gln Gly Gly His Ala Tyr Leu Lys Glu Trp Leu Trp Trp
    50                  55                  60

Ala Gly Leu Leu Ser Met Gly Ala Gly Glu Val Ala Asn Phe
65                  70                  75

(2) INFORMATION FOR SEQ ID NO: 226:

(i) SEQUENCE CHARACTERISTICS:
(A) LENGTH: 30 amino acids
(B) TYPE: amino acid
(D) TOPOLOGY: linear
(xi) SEQUENCE DESCRIPTION: SEQ ID NO: 226:

Asn Phe Ala Ala Tyr Ala Phe Ala Pro Ala Thr Leu Val Thr Pro Leu
1               5                   10                  15

Gly Ala Leu Ser Val Leu Val Ser Ala Ile Leu Ser Ser Tyr
            20                  25                  30

(2) INFORMATION FOR SEQ ID NO: 227:

(i) SEQUENCE CHARACTERISTICS:
(A) LENGTH: 36 amino acids
(B) TYPE: amino acid
(D) TOPOLOGY: linear
(xi) SEQUENCE DESCRIPTION: SEQ ID NO: 227:

Glu Arg Leu Asn Leu His Gly Lys Ile Gly Cys Leu Leu Ser Ile Leu
1               5                   10                  15

Gly Ser Thr Val Met Val Ile His Ala Pro Lys Glu Glu Glu Ile Glu
            20                  25                  30

Thr Leu Asn Glu
        35

(2) INFORMATION FOR SEQ ID NO: 228:

(i) SEQUENCE CHARACTERISTICS:
(A) LENGTH: 31 amino acids
(B) TYPE: amino acid
(D) TOPOLOGY: linear
(xi) SEQUENCE DESCRIPTION: SEQ ID NO: 228:

Arg Phe Lys Thr Leu Met Thr Asn Lys Ser Glu Gln Asp Gly Asp Ser
1               5                   10                  15

Ser Lys Thr Ile Glu Ile Ser Asp Met Lys Tyr His Ile Phe Gln
            20                  25                  30

(2) INFORMATION FOR SEQ ID NO: 229:

(i) SEQUENCE CHARACTERISTICS:
(A) LENGTH: 20 amino acids
(B) TYPE: amino acid
(D) TOPOLOGY: linear
(xi) SEQUENCE DESCRIPTION: SEQ ID NO: 229:

Leu Val Glu Gly Lys Leu Phe Tyr Ala His Lys Val Leu Leu Val Thr
1               5                   10                  15

Xaa Ser Asn Arg
            20

(2) INFORMATION FOR SEQ ID NO: 230:

(i) SEQUENCE CHARACTERISTICS:
(A) LENGTH: 87 base pairs
(B) TYPE: nucleic acid
(C) STRANDEDNESS: double
(D) TOPOLOGY: linear

(xi) SEQUENCE DESCRIPTION: SEQ ID NO: 230:

CCTTAAAAGC TGACATTTTA TAATTGTGTT GTATAGCAGC AACTATATCC TTCCAAAAAT

CAAATGTTTT TTGACCATTG TTCAGTT

(2) INFORMATION FOR SEQ ID NO: 231:

(i) SEQUENCE CHARACTERISTICS:
(A) LENGTH: 38 base pairs
(B) TYPE: nucleic acid
(C) STRANDEDNESS: double
(D) TOPOLOGY: linear

(xi) SEQUENCE DESCRIPTION: SEQ ID NO: 231:

CCTTAAAAGC TGACATTTTA TAATTGTGTT GTATAGCA

(2) INFORMATION FOR SEQ ID NO: 232:

(i) SEQUENCE CHARACTERISTICS:
(A) LENGTH: 38 base pairs
(B) TYPE: nucleic acid
(C) STRANDEDNESS: double
(D) TOPOLOGY: linear

(xi) SEQUENCE DESCRIPTION: SEQ ID NO: 232:

CTTCCAAAAA TCAAATGTTT TTTGACCATT GTTCAGTT

(2) INFORMATION FOR SEQ ID NO: 233:

(i) SEQUENCE CHARACTERISTICS:
(A) LENGTH: 455 amino acids
(B) TYPE: amino acid
(D) TOPOLOGY: linear
(xi) SEQUENCE DESCRIPTION: SEQ ID NO: 233:

Met Ala Gln His Phe Ser Leu Ala Ala Cys Asp Val Val Gly Phe Asp
1               5                   10                  15

Leu Asp His Thr Leu Cys Arg Tyr Asn Leu Pro Glu Ser Ala Pro Leu
            20                  25                  30

Ile Tyr Asn Ser Phe Ala Gln Phe Leu Val Lys Glu Lys Gly Tyr Asp
        35                  40                  45

Lys Glu Leu Leu Asn Val Thr Pro Glu Asp Trp Asp Phe Cys Cys Lys
    50                  55                  60

Gly Leu Ala Leu Asp Leu Glu Asp Gly Asn Phe Leu Lys Leu Ala Asn
65                  70                  75                  80

Asn Gly Thr Val Leu Arg Ala Ser His Gly Thr Lys Met Met Thr Pro
                85                  90                  95

Glu Val Leu Ala Glu Ala Tyr Gly Lys Lys Glu Trp Lys His Phe Leu
            100                 105                 110

Ser Asp Thr Gly Met Ala Cys Arg Ser Gly Lys Tyr Tyr Phe Tyr Asp
        115                 120                 125

Asn Tyr Phe Asp Leu Pro Gly Ala Leu Leu Cys Ala Arg Val Val Asp
    130                 135                 140

Tyr Leu Thr Lys Leu Asn Asn Gly Gln Lys Thr Phe Asp Phe Trp Lys
145                 150                 155                 160

Asp Ile Val Ala Ala Ile Gln His Asn Tyr Lys Met Ser Ala Phe Lys
                165                 170                 175

Glu Asn Cys Gly Ile Tyr Phe Pro Glu Ile Lys Arg Asp Pro Gly Arg
            180                 185                 190

Tyr Leu His Ser Cys Pro Glu Ser Val Lys Lys Trp Leu Arg Gln Leu
        195                 200                 205

Lys Asn Ala Gly Lys Ile Leu Leu Leu Ile Thr Ser Ser His Ser Asp
    210                 215                 220

Tyr Cys Arg Leu Leu Cys Glu Tyr Ile Leu Gly Asn Asp Phe Thr Asp
225                 230                 235                 240

Leu Phe Asp Ile Val Ile Thr Asn Ala Leu Lys Pro Gly Phe Phe Ser
                245                 250                 255

His Leu Pro Ser Gln Arg Pro Phe Arg Thr Leu Glu Asn Asp Glu Glu
            260                 265                 270

Gln Glu Ala Leu Pro Ser Leu Asp Lys Pro Gly Trp Tyr Ser Gln Gly
        275                 280                 285

Asn Ala Val His Leu Tyr Glu Leu Leu Lys Lys Met Thr Gly Lys Pro
    290                 295                 300

Glu Pro Lys Val Val Tyr Phe Gly Asp Ser Met His Ser Asp Ile Phe
305                 310                 315                 320

Pro Ala Arg His Tyr Ser Asn Trp Glu Thr Val Leu Ile Leu Glu Glu
                325                 330                 335

Leu Arg Gly Asp Glu Gly Thr Arg Ser Gln Arg Pro Glu Glu Ser Glu
            340                 345                 350

Pro Leu Glu Lys Lys Gly Lys Tyr Glu Gly Pro Lys Ala Lys Pro Leu
        355                 360                 365

Asn Thr Ser Ser Lys Lys Trp Gly Ser Phe Phe Ile Asp Ser Val Leu
    370                 375                 380

Gly Leu Glu Asn Thr Glu Asp Ser Leu Val Tyr Thr Trp Ser Cys Lys
385                 390                 395                 400

Arg Ile Ser Thr Tyr Ser Thr Ile Ala Ile Pro Ser Ile Glu Ala Ile
                405                 410                 415

Ala Glu Leu Pro Leu Asp Tyr Lys Phe Thr Arg Phe Ser Ser Ser Asn
            420                 425                 430

Ser Lys Thr Ala Gly Tyr Tyr Pro Asn Pro Pro Leu Val Leu Ser Ser
        435                 440                 445

Asp Glu Thr Leu Ile Ser Lys
    450                 455

(2) INFORMATION FOR SEQ ID NO: 234:

(i) SEQUENCE CHARACTERISTICS:
(A) LENGTH: 27 amino acids
(B) TYPE: amino acid
(D) TOPOLOGY: linear
(xi) SEQUENCE DESCRIPTION: SEQ ID NO: 234:

Thr Ser Ser His Ser Asp Tyr Cys Arg Leu Leu Cys Glu Tyr Ile Leu
  1               5                  10                  15

Gly Asn Asp Phe Thr Asp Leu Phe Asp Ile Val
             20                  25

(2) INFORMATION FOR SEQ ID NO: 235:

(i) SEQUENCE CHARACTERISTICS:
(A) LENGTH: 327 amino acids
(B) TYPE: amino acid
(D) TOPOLOGY: linear
(xi) SEQUENCE DESCRIPTION: SEQ ID NO: 235:

Met Lys Thr Lys Asn Ile Pro Glu Ala His Gln Asp Ala Phe Lys Thr
  1               5                  10                  15

Gly Phe Ala Glu Gly Phe Leu Lys Ala Gln Ala Leu Thr Gln Lys Thr
             20                  25                  30

Asn Asp Ser Leu Arg Arg Thr Arg Leu Ile Leu Phe Val Leu Leu Leu
         35                  40                  45

Phe Gly Ile Tyr Gly Leu Leu Lys Asn Pro Phe Leu Ser Val Arg Phe
     50                  55                  60

Arg Thr Thr Thr Gly Leu Asp Ser Ala Val Asp Pro Val Gln Met Lys
 65                  70                  75                  80

Asn Val Thr Phe Glu His Val Lys Gly Val Glu Glu Ala Lys Gln Glu
                 85                  90                  95

Leu Gln Glu Val Val Glu Phe Leu Lys Asn Pro Gln Lys Phe Thr Ile

            100                 105                 110

Leu Gly Gly Lys Leu Pro Lys Gly Ile Leu Leu Val Gly Pro Pro Gly
        115                 120                 125

Thr Gly Lys Thr Leu Leu Ala Arg Ala Val Ala Gly Glu Ala Asp Val
    130                 135                 140

Pro Phe Tyr Tyr Ala Ser Gly Ser Glu Phe Asp Glu Met Phe Val Gly
145                 150                 155                 160

Val Gly Ala Ser Arg Ile Arg Asn Leu Phe Arg Glu Ala Lys Ala Asn
                165                 170                 175

Ala Pro Cys Val Ile Phe Ile Asp Glu Leu Asp Ser Val Gly Gly Lys
            180                 185                 190

Arg Ile Glu Ser Pro Met His Pro Tyr Ser Arg Gln Thr Ile Asn Gln
        195                 200                 205

Leu Leu Ala Glu Met Asp Gly Phe Lys Pro Asn Glu Gly Val Ile Ile
    210                 215                 220

Ile Gly Ala Thr Asn Phe Pro Glu Ala Leu Asp Asn Ala Leu Ile Arg
225                 230                 235                 240

Pro Gly Arg Phe Asp Met Gln Val Thr Val Pro Arg Pro Asp Val Lys
                245                 250                 255

Gly Arg Thr Glu Ile Leu Lys Trp Tyr Leu Asn Lys Ile Lys Phe Asp
            260                 265                 270

Xaa Ser Val Asp Pro Glu Ile Ile Ala Arg Gly Thr Val Gly Phe Ser
        275                 280                 285

Gly Ala Glu Leu Glu Asn Leu Val Asn Gln Ala Ala Leu Lys Ala Ala
    290                 295                 300

Val Asp Gly Lys Glu Met Val Thr Met Lys Glu Leu Gly Val Phe Gln
305                 310                 315                 320

Arg Gln Asn Ser Asn Gly Ala
                325

(2) INFORMATION FOR SEQ ID NO: 236:

(i) SEQUENCE CHARACTERISTICS:
(A) LENGTH: 21 amino acids
(B) TYPE: amino acid
(D) TOPOLOGY: linear
(xi) SEQUENCE DESCRIPTION: SEQ ID NO: 236:

Met Lys Thr Lys Asn Ile Pro Glu Ala His Gln Asp Ala Phe Lys Thr
1               5                   10                  15

Gly Phe Ala Glu Gly
            20

(2) INFORMATION FOR SEQ ID NO: 237:

(i) SEQUENCE CHARACTERISTICS:
(A) LENGTH: 23 amino acids
(B) TYPE: amino acid
(D) TOPOLOGY: linear
(xi) SEQUENCE DESCRIPTION: SEQ ID NO: 237:

Pro Val Gln Met Lys Asn Val Thr Phe Glu His Val Lys Gly Val Glu
1               5                   10                  15

Glu Ala Lys Gln Glu Leu Gln
            20

(2) INFORMATION FOR SEQ ID NO: 238:

(i) SEQUENCE CHARACTERISTICS:
(A) LENGTH: 23 amino acids
(B) TYPE: amino acid
(D) TOPOLOGY: linear
(xi) SEQUENCE DESCRIPTION: SEQ ID NO: 238:

Ser Arg Gln Thr Ile Asn Gln Leu Leu Ala Glu Met Asp Gly Phe Lys
1               5                   10                  15

Pro Asn Glu Gly Val Ile Ile
            20

(2) INFORMATION FOR SEQ ID NO: 239:

(i) SEQUENCE CHARACTERISTICS:
(A) LENGTH: 24 amino acids
(B) TYPE: amino acid
(D) TOPOLOGY: linear
(xi) SEQUENCE DESCRIPTION: SEQ ID NO: 239:

Phe Ser Gly Ala Glu Leu Glu Asn Leu Val Asn Gln Ala Ala Leu Lys
1               5                   10                  15

Ala Ala Val Asp Gly Lys Glu Met
            20

(2) INFORMATION FOR SEQ ID NO: 240:

(i) SEQUENCE CHARACTERISTICS:
(A) LENGTH: 192 amino acids
(B) TYPE: amino acid
(D) TOPOLOGY: linear
(xi) SEQUENCE DESCRIPTION: SEQ ID NO: 240:

Leu Pro Met Trp Gln Val Thr Ala Phe Leu Asp His Asn Ile Val Thr
1               5                   10                  15

Ala Gln Thr Thr Trp Lys Gly Leu Trp Met Ser Cys Val Val Gln Ser
            20                  25                  30

Thr Gly His Met Gln Cys Lys Val Tyr Asp Ser Val Leu Ala Leu Ser
        35                  40                  45

Thr Glu Val Gln Ala Ala Arg Ala Leu Thr Val Ser Ala Val Leu Leu
    50                  55                  60

Ala Phe Val Ala Leu Phe Val Thr Leu Ala Gly Ala Gln Cys Thr Thr
65                  70                  75                  80

Cys Val Ala Pro Gly Pro Ala Lys Ala Arg Val Ala Leu Thr Gly Gly
                85                  90                  95

Val Leu Tyr Leu Phe Cys Gly Leu Leu Ala Leu Val Pro Leu Cys Trp
            100                 105                 110

Phe Ala Asn Ile Val Val Arg Glu Phe Tyr Asp Pro Ser Val Pro Val
        115                 120                 125

Ser Gln Lys Tyr Glu Leu Gly Ala Xaa Leu Tyr Ile Gly Trp Ala Ala
    130                 135                 140

Thr Ala Leu Leu Met Val Gly Gly Cys Leu Leu Cys Cys Gly Ala Trp
145                 150                 155                 160

Val Cys Thr Gly Arg Pro Asp Leu Ser Phe Pro Val Lys Tyr Ser Ala
                165                 170                 175

Pro Arg Arg Pro Thr Ala Thr Gly Asp Tyr Asp Lys Lys Asn Tyr Val
            180                 185                 190

(2) INFORMATION FOR SEQ ID NO: 241:

(i) SEQUENCE CHARACTERISTICS:
(A) LENGTH: 24 amino acids
(B) TYPE: amino acid
(D) TOPOLOGY: linear
(xi) SEQUENCE DESCRIPTION: SEQ ID NO: 241:

Leu His Tyr Phe Ala Leu Ser Phe Val Leu Ile Leu Thr Glu Ile Cys
1               5                   10                  15

Leu Val Ser Ser Gly Met Gly Phe
            20

(2) INFORMATION FOR SEQ ID NO: 242:

(i) SEQUENCE CHARACTERISTICS:
(A) LENGTH: 31 amino acids
(B) TYPE: amino acid

(D) TOPOLOGY: linear
(xi) SEQUENCE DESCRIPTION: SEQ ID NO: 242:

Gln Leu Arg Asn Gly Ile Pro Pro Gly Arg Lys Ala Leu Phe Cys Ser
1               5                   10                  15

Gly Lys Pro Arg Leu Phe Thr Leu Gly Gln Gly Arg Thr Cys Ala
            20                  25                  30

(2) INFORMATION FOR SEQ ID NO: 243:

(i) SEQUENCE CHARACTERISTICS:
(A) LENGTH: 39 amino acids
(B) TYPE: amino acid
(D) TOPOLOGY: linear
(xi) SEQUENCE DESCRIPTION: SEQ ID NO: 243:

Trp Ser Gly Leu Trp Val Thr Thr Trp Asn Gly Ser Ser Gly Glu Arg
1               5                   10                  15

Thr Pro Ser Pro Trp Arg Arg Lys Arg Ala Ser Gln Ser Ala Gly Arg
            20                  25                  30

Ile Ala Ser Trp Met Ser Phe
        35

(2) INFORMATION FOR SEQ ID NO: 244:

(i) SEQUENCE CHARACTERISTICS:
(A) LENGTH: 14 amino acids
(B) TYPE: amino acid
(D) TOPOLOGY: linear
(xi) SEQUENCE DESCRIPTION: SEQ ID NO: 244:

Glu Tyr Asn Lys Glu Ser Glu Asp Lys Tyr Val Phe Leu Val
1               5                   10

(2) INFORMATION FOR SEQ ID NO: 245:

(i) SEQUENCE CHARACTERISTICS:
(A) LENGTH: 14 amino acids
(B) TYPE: amino acid
(D) TOPOLOGY: linear
(xi) SEQUENCE DESCRIPTION: SEQ ID NO: 245:

Ile Asp Val Glu Ile Ala Arg Ser Asp Cys Arg Lys Pro Leu
1               5                   10

(2) INFORMATION FOR SEQ ID NO: 246:

(i) SEQUENCE CHARACTERISTICS:
(A) LENGTH: 142 amino acids

(B) TYPE: amino acid
(D) TOPOLOGY: linear
(xi) SEQUENCE DESCRIPTION: SEQ ID NO: 246:

Met Pro Arg Cys Arg Trp Leu Ser Leu Ile Leu Leu Thr Ile Pro Leu
1               5                   10                  15

Ala Leu Val Ala Arg Lys Asp Pro Lys Lys Asn Glu Thr Gly Val Leu
            20                  25                  30

Arg Lys Leu Lys Pro Val Asn Ala Ser Asn Ala Asn Val Lys Gln Cys
        35                  40                  45

Leu Trp Phe Ala Met Gln Glu Tyr Asn Lys Glu Ser Glu Asp Lys Tyr
    50                  55                  60

Val Phe Leu Val Val Lys Thr Leu Gln Ala Gln Leu Gln Val Thr Asn
65                  70                  75                  80

Leu Leu Glu Tyr Leu Ile Asp Val Glu Ile Ala Arg Ser Asp Cys Arg
                85                  90                  95

Lys Pro Leu Ser Thr Asn Glu Ile Cys Ala Ile Gln Glu Asn Ser Lys
            100                 105                 110

Leu Lys Arg Lys Leu Ser Cys Ser Phe Leu Val Gly Ala Leu Pro Trp
        115                 120                 125

Asn Gly Glu Phe Thr Val Met Glu Lys Lys Cys Glu Asp Ala
    130                 135                 140

(2) INFORMATION FOR SEQ ID NO: 247:

(i) SEQUENCE CHARACTERISTICS:
(A) LENGTH: 92 amino acids
(B) TYPE: amino acid
(D) TOPOLOGY: linear
(xi) SEQUENCE DESCRIPTION: SEQ ID NO: 247:

Cys Leu Trp Phe Ala Met Gln Glu Tyr Asn Lys Glu Ser Glu Asp Lys
1               5                   10                  15

Tyr Val Phe Leu Val Val Lys Thr Leu Gln Ala Gln Leu Gln Val Thr
            20                  25                  30

Asn Leu Leu Glu Tyr Leu Ile Asp Val Glu Ile Ala Arg Ser Asp Cys
        35                  40                  45

Arg Lys Pro Leu Ser Thr Asn Glu Ile Cys Ala Ile Gln Glu Asn Ser
    50                  55                  60

Lys Leu Lys Arg Lys Leu Ser Cys Ser Phe Leu Val Gly Ala Leu Pro
65                  70                  75                  80

Trp Asn Gly Glu Phe Thr Val Met Glu Lys Lys Cys
                85                  90

(2) INFORMATION FOR SEQ ID NO: 248:

(i) SEQUENCE CHARACTERISTICS:
(A) LENGTH: 123 amino acids
(B) TYPE: amino acid
(D) TOPOLOGY: linear
(xi) SEQUENCE DESCRIPTION: SEQ ID NO: 248:

Ala Arg Lys Asp Pro Lys Lys Asn Glu Thr Gly Val Leu Arg Lys Leu
1               5                   10                  15

Lys Pro Val Asn Ala Ser Asn Ala Asn Val Lys Gln Cys Leu Trp Phe
            20                  25                  30

Ala Met Gln Glu Tyr Asn Lys Glu Ser Glu Asp Lys Tyr Val Phe Leu
        35                  40                  45

Val Val Lys Thr Leu Gln Ala Gln Leu Gln Val Thr Asn Leu Leu Glu
    50                  55                  60

Tyr Leu Ile Asp Val Glu Ile Ala Arg Ser Asp Cys Arg Lys Pro Leu
65                  70                  75                  80

Ser Thr Asn Glu Ile Cys Ala Ile Gln Glu Asn Ser Lys Leu Lys Arg
                85                  90                  95

Lys Leu Ser Cys Ser Phe Leu Val Gly Ala Leu Pro Trp Asn Gly Glu
            100                 105                 110

Phe Thr Val Met Glu Lys Lys Cys Glu Asp Ala
        115                 120

(2) INFORMATION FOR SEQ ID NO: 249:

(i) SEQUENCE CHARACTERISTICS:
(A) LENGTH: 44 amino acids
(B) TYPE: amino acid
(D) TOPOLOGY: linear
(xi) SEQUENCE DESCRIPTION: SEQ ID NO: 249:

Asp Ser Pro Asp Thr Glu Pro Gly Ser Ser Ala Gly Pro Thr Gln Arg
1               5                   10                  15

Pro Ser Asp Asn Ser His Asn Glu His Ala Pro Ala Ser Gln Gly Leu
            20                  25                  30

Lys Ala Glu His Leu Tyr Ile Leu Ile Gly Val Ser
        35                  40

(2) INFORMATION FOR SEQ ID NO: 250:

(i) SEQUENCE CHARACTERISTICS:
(A) LENGTH: 101 amino acids
(B) TYPE: amino acid
(D) TOPOLOGY: linear

(xi) SEQUENCE DESCRIPTION: SEQ ID NO: 250:

His Arg Gln Asn Gln Ile Lys Gln Gly Pro Pro Arg Ser Lys Asp Glu
1               5                   10                  15

Glu Gln Lys Pro Gln Gln Arg Pro Asp Leu Ala Val Asp Val Leu Glu
            20                  25                  30

Arg Thr Ala Asp Lys Ala Thr Val Asn Gly Leu Pro Glu Lys Asp Arg
        35                  40                  45

Glu Thr Asp Thr Ser Ala Leu Ala Ala Gly Ser Ser Gln Glu Val Thr
    50                  55                  60

Tyr Ala Gln Leu Asp His Trp Ala Leu Thr Gln Arg Thr Ala Arg Ala
65                  70                  75                  80

Val Ser Pro Gln Ser Thr Lys Pro Met Ala Glu Ser Ile Thr Tyr Ala
                85                  90                  95

Ala Val Ala Arg His
            100

(2) INFORMATION FOR SEQ ID NO: 251:

(i) SEQUENCE CHARACTERISTICS:
(A) LENGTH: 115 amino acids
(B) TYPE: amino acid
(D) TOPOLOGY: linear
(xi) SEQUENCE DESCRIPTION: SEQ ID NO: 251:

Met Ser Pro His Pro Thr Ala Leu Leu Gly Leu Val Leu Cys Leu Ala
1               5                   10                  15

Gln Thr Ile His Thr Gln Glu Glu Asp Leu Pro Arg Pro Ser Ile Ser
            20                  25                  30

Ala Glu Pro Gly Thr Val Ile Pro Leu Gly Ser His Val Thr Phe Val
        35                  40                  45

Cys Arg Gly Pro Val Gly Val Gln Thr Phe Arg Leu Glu Arg Glu Ser
    50                  55                  60

Arg Ser Thr Tyr Asn Asp Thr Glu Asp Val Ser Gln Ala Ser Pro Ser
65                  70                  75                  80

Glu Ser Glu Ala Arg Phe Arg Ile Asp Ser Val Ser Glu Gly Asn Ala
                85                  90                  95

Gly Pro Tyr Arg Cys Ile Tyr Tyr Lys Pro Pro Lys Trp Ser Glu Gln
            100                 105                 110

Ser Asp Tyr
        115

(2) INFORMATION FOR SEQ ID NO: 252:

(i) SEQUENCE CHARACTERISTICS:
(A) LENGTH: 18 amino acids
(B) TYPE: amino acid
(D) TOPOLOGY: linear
(xi) SEQUENCE DESCRIPTION: SEQ ID NO: 252:

Thr Ala Leu Leu Gly Leu Val Leu Cys Leu Ala Gln Thr Ile His Thr
1               5                   10                  15

Gln Glu

(2) INFORMATION FOR SEQ ID NO: 253:

(i) SEQUENCE CHARACTERISTICS:
(A) LENGTH: 14 amino acids
(B) TYPE: amino acid
(D) TOPOLOGY: linear
(xi) SEQUENCE DESCRIPTION: SEQ ID NO: 253:

Leu Pro Arg Pro Ser Ile Ser Ala Glu Pro Gly Thr Val Ile
1               5                   10

(2) INFORMATION FOR SEQ ID NO: 254:

(i) SEQUENCE CHARACTERISTICS:
(A) LENGTH: 15 amino acids
(B) TYPE: amino acid
(D) TOPOLOGY: linear
(xi) SEQUENCE DESCRIPTION: SEQ ID NO: 254:

Cys Arg Gly Pro Val Gly Val Gln Thr Phe Arg Leu Glu Arg Glu
1               5                   10                  15

(2) INFORMATION FOR SEQ ID NO: 255:

(i) SEQUENCE CHARACTERISTICS:
(A) LENGTH: 31 amino acids
(B) TYPE: amino acid
(D) TOPOLOGY: linear
(xi) SEQUENCE DESCRIPTION: SEQ ID NO: 255:

Val Leu Glu Arg Thr Ala Asp Lys Ala Thr Val Asn Gly Leu Pro Glu
1               5                   10                  15

Lys Asp Arg Glu Thr Asp Thr Ser Ala Leu Ala Ala Gly Ser Ser
            20                  25                  30

(2) INFORMATION FOR SEQ ID NO: 256:

(i) SEQUENCE CHARACTERISTICS:

(A) LENGTH: 438 amino acids
(B) TYPE: amino acid
(D) TOPOLOGY: linear
(xi) SEQUENCE DESCRIPTION: SEQ ID NO: 256:

Met Asn Thr Pro Asn Gly Asn Ser Leu Ser Ala Ala Glu Leu Thr Cys
1               5                   10                  15

Gly Met Ile Met Cys Leu Ala Arg Gln Ile Pro Gln Ala Thr Ala Ser
            20                  25                  30

Met Lys Asp Gly Lys Trp Glu Arg Lys Lys Phe Met Gly Thr Glu Leu
        35                  40                  45

Asn Gly Lys Thr Leu Gly Ile Leu Gly Leu Gly Arg Ile Gly Arg Glu
    50                  55                  60

Val Ala Thr Arg Met Gln Ser Phe Gly Met Lys Thr Ile Gly Tyr Asp
65                  70                  75                  80

Pro Ile Ile Ser Pro Glu Val Ser Ala Ser Phe Gly Val Gln Gln Leu
                85                  90                  95

Pro Leu Glu Glu Ile Trp Pro Leu Cys Asp Phe Ile Thr Val His Thr
            100                 105                 110

Pro Leu Leu Pro Ser Thr Thr Gly Leu Leu Asn Asp Asn Thr Phe Ala
        115                 120                 125

Gln Cys Lys Lys Gly Val Arg Val Val Asn Cys Ala Arg Gly Gly Ile
    130                 135                 140

Val Asp Glu Gly Ala Leu Leu Arg Ala Leu Gln Ser Gly Gln Cys Ala
145                 150                 155                 160

Gly Ala Ala Leu Asp Val Phe Thr Glu Glu Pro Pro Arg Asp Arg Ala
                165                 170                 175

Leu Val Asp His Glu Asn Val Ile Ser Cys Pro His Leu Gly Ala Ser
            180                 185                 190

Thr Lys Glu Ala Gln Ser Arg Cys Gly Glu Glu Ile Ala Val Gln Phe
        195                 200                 205

Val Asp Met Val Lys Gly Lys Ser Leu Thr Gly Val Val Asn Ala Gln
    210                 215                 220

Ala Leu Thr Ser Ala Phe Ser Pro His Thr Lys Pro Trp Ile Gly Leu
225                 230                 235                 240

Ala Glu Ala Leu Gly Thr Leu Met Arg Ala Trp Ala Gly Ser Pro Lys
                245                 250                 255

Gly Thr Ile Gln Val Ile Thr Gln Gly Thr Ser Leu Lys Asn Ala Gly
            260                 265                 270

Asn Cys Leu Ser Pro Ala Val Ile Val Gly Leu Leu Lys Glu Ala Ser
        275                 280                 285

Lys Gln Ala Asp Val Asn Leu Val Asn Ala Lys Leu Leu Val Lys Glu

290                 295                 300

Ala Gly Leu Asn Val Thr Thr Ser His Ser Pro Ala Ala Pro Gly Glu
305                 310                 315                 320

Gln Gly Phe Gly Glu Cys Leu Leu Ala Val Ala Leu Ala Gly Ala Pro
                325                 330                 335

Tyr Gln Ala Val Gly Leu Val Gln Gly Thr Thr Pro Val Leu Gln Gly
            340                 345                 350

Leu Asn Gly Ala Val Phe Arg Pro Glu Val Pro Leu Arg Arg Asp Leu
        355                 360                 365

Pro Leu Leu Leu Phe Arg Thr Gln Thr Ser Asp Pro Ala Met Leu Pro
    370                 375                 380

Thr Met Ile Gly Leu Leu Ala Glu Ala Gly Val Arg Leu Leu Ser Tyr
385                 390                 395                 400

Gln Thr Ser Leu Val Ser Asp Gly Glu Thr Trp His Val Met Gly Ile
                405                 410                 415

Ser Ser Leu Leu Pro Ser Leu Glu Ala Trp Lys Gln His Val Thr Glu
            420                 425                 430

Ala Phe Gln Phe His Phe
        435

(2) INFORMATION FOR SEQ ID NO: 257:

        (i) SEQUENCE CHARACTERISTICS:
                (A) LENGTH: 24 amino acids
                (B) TYPE: amino acid
                (D) TOPOLOGY: linear
        (xi) SEQUENCE DESCRIPTION: SEQ ID NO: 257:

Met Ala Phe Ala Asn Leu Arg Lys Val Leu Ile Ser Asp Ser Leu Asp
  1               5                  10                  15

Pro Cys Cys Arg Lys Ile Leu Gln
            20

(2) INFORMATION FOR SEQ ID NO: 258:

        (i) SEQUENCE CHARACTERISTICS:
                (A) LENGTH: 18 amino acids
                (B) TYPE: amino acid
                (D) TOPOLOGY: linear
        (xi) SEQUENCE DESCRIPTION: SEQ ID NO: 258:

Gly Gly Leu Gln Val Val Glu Lys Gln Asn Leu Ser Lys Glu Glu Leu
  1               5                  10                  15

Ile Ala

(2) INFORMATION FOR SEQ ID NO: 259:

(i) SEQUENCE CHARACTERISTICS:
(A) LENGTH: 29 amino acids
(B) TYPE: amino acid
(D) TOPOLOGY: linear
(xi) SEQUENCE DESCRIPTION: SEQ ID NO: 259:

Met Cys Leu Ala Arg Gln Ile Pro Gln Ala Thr Ala Ser Met Lys Asp
1               5                   10                  15

Gly Lys Trp Glu Arg Lys Lys Phe Met Gly Thr Glu Leu
            20                  25

(2) INFORMATION FOR SEQ ID NO: 260:

(i) SEQUENCE CHARACTERISTICS:
(A) LENGTH: 29 amino acids
(B) TYPE: amino acid
(D) TOPOLOGY: linear
(xi) SEQUENCE DESCRIPTION: SEQ ID NO: 260:

Ala Leu Thr Ser Ala Phe Ser Pro His Thr Lys Pro Trp Ile Gly Leu
1               5                   10                  15

Ala Glu Ala Leu Gly Thr Leu Met Arg Ala Trp Ala Gly
            20                  25

(2) INFORMATION FOR SEQ ID NO: 261:

(i) SEQUENCE CHARACTERISTICS:
(A) LENGTH: 36 amino acids
(B) TYPE: amino acid
(D) TOPOLOGY: linear
(xi) SEQUENCE DESCRIPTION: SEQ ID NO: 261:

Glu Val Pro Leu Arg Arg Asp Leu Pro Leu Leu Leu Phe Arg Thr Gln
1               5                   10                  15

Thr Ser Asp Pro Ala Met Leu Pro Thr Met Ile Gly Leu Leu Ala Glu
            20                  25                  30

Ala Gly Val Arg
        35

(2) INFORMATION FOR SEQ ID NO: 262:

(i) SEQUENCE CHARACTERISTICS:
(A) LENGTH: 109 amino acids
(B) TYPE: amino acid
(D) TOPOLOGY: linear

(xi) SEQUENCE DESCRIPTION: SEQ ID NO: 262:

Phe Gly Thr Arg Phe Leu Ala Asn Leu Leu Leu Glu Glu Asp Asn Lys
1               5                   10                  15

Phe Cys Ala Asp Cys Gln Ser Lys Gly Pro Arg Trp Ala Ser Trp Asn
            20                  25                  30

Ile Gly Val Phe Ile Cys Ile Arg Cys Ala Xaa Ile His Arg Asn Leu
        35                  40                  45

Gly Val His Ile Ser Arg Val Lys Ser Val Asn Leu Asp Gln Trp Thr
    50                  55                  60

Gln Val Gln Ile Gln Cys Met Gln Xaa Met Gly Asn Gly Lys Ala Asn
65                  70                  75                  80

Arg Leu Tyr Glu Ala Tyr Leu Pro Glu Thr Phe Arg Arg Pro Gln Ile
                85                  90                  95

Asp Pro Ala Val Glu Gly Phe Ile Arg Asp Xaa Tyr Glu
            100                 105

(2) INFORMATION FOR SEQ ID NO: 263:

(i) SEQUENCE CHARACTERISTICS:
(A) LENGTH: 21 amino acids
(B) TYPE: amino acid
(D) TOPOLOGY: linear
(xi) SEQUENCE DESCRIPTION: SEQ ID NO: 263:

Glu Glu Asp Asn Lys Phe Cys Ala Asp Cys Gln Ser Lys Gly Pro Arg
1               5                   10                  15

Trp Ala Ser Trp Asn
            20

(2) INFORMATION FOR SEQ ID NO: 264:

(i) SEQUENCE CHARACTERISTICS:
(A) LENGTH: 20 amino acids
(B) TYPE: amino acid
(D) TOPOLOGY: linear
(xi) SEQUENCE DESCRIPTION: SEQ ID NO: 264:

Gly Val Phe Ile Cys Ile Arg Cys Ala Xaa Ile His Arg Asn Leu Gly
1               5                   10                  15

Val His Ile Ser
            20

(2) INFORMATION FOR SEQ ID NO: 265:

(i) SEQUENCE CHARACTERISTICS:

(A) LENGTH: 23 amino acids
(B) TYPE: amino acid
(D) TOPOLOGY: linear
(xi) SEQUENCE DESCRIPTION: SEQ ID NO: 265:

Ser Val Asn Leu Asp Gln Trp Thr Gln Val Gln Ile Gln Cys Met Gln
1               5                   10                  15

Xaa Met Gly Asn Gly Lys Ala
            20

(2) INFORMATION FOR SEQ ID NO: 266:

(i) SEQUENCE CHARACTERISTICS:
(A) LENGTH: 245 amino acids
(B) TYPE: amino acid
(D) TOPOLOGY: linear
(xi) SEQUENCE DESCRIPTION: SEQ ID NO: 266:

Met Asp Leu Leu Gly Leu Asp Ala Pro Val Ala Cys Ser Ile Ala Asn
1               5                   10                  15

Ser Lys Thr Ser Asn Thr Leu Glu Lys Asp Leu Asp Leu Leu Ala Ser
            20                  25                  30

Val Pro Ser Pro Ser Ser Ser Gly Ser Arg Lys Val Val Gly Ser Met
        35                  40                  45

Pro Thr Ala Gly Ser Ala Gly Ser Val Pro Glu Asn Leu Asn Leu Phe
    50                  55                  60

Pro Glu Pro Gly Ser Lys Ser Glu Glu Ile Gly Lys Lys Gln Leu Ser
65                  70                  75                  80

Lys Asp Ser Ile Leu Ser Leu Tyr Gly Ser Gln Thr Xaa Gln Met Pro
                85                  90                  95

Thr Gln Ala Met Phe Met Ala Pro Ala Gln Met Ala Tyr Pro Thr Ala
            100                 105                 110

Tyr Pro Ser Phe Pro Gly Val Thr Pro Pro Asn Ser Ile Met Gly Ser
        115                 120                 125

Met Met Pro Pro Pro Val Gly Met Val Ala Gln Pro Gly Ala Ser Gly
    130                 135                 140

Met Val Ala Pro Met Ala Met Pro Ala Gly Tyr Met Gly Gly Met Gln
145                 150                 155                 160

Ala Ser Met Met Gly Val Pro Asn Gly Met Met Thr Thr Gln Gln Ala
                165                 170                 175

Gly Tyr Met Ala Gly Met Ala Ala Met Pro Gln Thr Val Tyr Gly Val
            180                 185                 190

Gln Pro Ala Gln Gln Leu Gln Trp Asn Leu Thr Gln Met Thr Gln Gln
        195                 200                 205

Met Ala Gly Met Asn Phe Tyr Gly Ala Asn Gly Met Met Asn Tyr Gly
    210                 215                 220

Gln Ser Met Ser Gly Gly Asn Gly Gln Ala Ala Asn Gln Thr Leu Ser
225                 230                 235                 240

Pro Gln Met Trp Lys
                245

(2) INFORMATION FOR SEQ ID NO: 267:

(i) SEQUENCE CHARACTERISTICS:
(A) LENGTH: 315 amino acids
(B) TYPE: amino acid
(D) TOPOLOGY: linear
(xi) SEQUENCE DESCRIPTION: SEQ ID NO: 267:

Met Asp Leu Leu Gly Leu Asp Ala Pro Val Ala Cys Ser Ile Ala Asn
1               5                   10                  15

Ser Lys Thr Ser Asn Thr Leu Glu Lys Asp Leu Asp Leu Leu Ala Ser
            20                  25                  30

Val Pro Ser Pro Ser Ser Ser Gly Ser Arg Lys Val Val Gly Ser Met
        35                  40                  45

Pro Thr Ala Gly Ser Ala Gly Ser Val Pro Glu Asn Leu Asn Leu Phe
    50                  55                  60

Pro Glu Pro Gly Ser Lys Ser Glu Glu Ile Gly Lys Lys Gln Leu Ser
65                  70                  75                  80

Lys Asp Ser Ile Leu Ser Leu Tyr Gly Ser Gln Thr Xaa Gln Met Pro
                85                  90                  95

Thr Gln Ala Met Phe Met Ala Pro Ala Gln Met Ala Tyr Pro Thr Ala
            100                 105                 110

Tyr Pro Ser Phe Pro Gly Val Thr Pro Pro Asn Ser Ile Met Gly Ser
        115                 120                 125

Met Met Pro Pro Pro Val Gly Met Val Ala Gln Pro Gly Ala Ser Gly
    130                 135                 140

Met Val Ala Pro Met Ala Met Pro Ala Gly Tyr Met Gly Gly Met Gln
145                 150                 155                 160

Ala Ser Met Met Gly Val Pro Asn Gly Met Met Thr Thr Gln Gln Ala
                165                 170                 175

Gly Tyr Met Ala Gly Met Ala Ala Met Pro Gln Thr Val Tyr Gly Val
            180                 185                 190

Gln Pro Ala Gln Gln Leu Gln Trp Asn Leu Thr Gln Met Thr Gln Gln
        195                 200                 205

Met Ala Gly Met Asn Phe Tyr Gly Ala Asn Gly Met Met Asn Tyr Gly
    210                 215                 220

Gln Ser Met Ser Gly Gly Asn Gly Gln Ala Ala Asn Gln Thr Leu Ser
225                 230                 235                 240

Pro Gln Met Trp Lys Phe Gly Thr Arg Phe Leu Ala Asn Leu Leu Leu
                245                 250                 255

Glu Glu Asp Asn Lys Phe Cys Ala Asp Cys Gln Ser Lys Gly Pro Arg
            260                 265                 270

Trp Ala Ser Trp Asn Ile Gly Val Phe Ile Cys Ile Arg Cys Ala Xaa
        275                 280                 285

Ile His Arg Asn Leu Gly Val His Ile Ser Arg Val Lys Ser Val Asn
    290                 295                 300

Leu Asp Gln Trp Thr Gln Val Gln Ile Gln Cys
305                 310                 315

(2) INFORMATION FOR SEQ ID NO: 268:

        (i) SEQUENCE CHARACTERISTICS:
                (A) LENGTH: 39 amino acids
                (B) TYPE: amino acid
                (D) TOPOLOGY: linear
        (xi) SEQUENCE DESCRIPTION: SEQ ID NO: 268:

Met Gln Xaa Met Gly Asn Gly Lys Ala Asn Arg Leu Tyr Glu Ala Tyr
  1               5                  10                  15

Leu Pro Glu Thr Phe Arg Arg Pro Gln Ile Asp Pro Ala Val Glu Gly
            20                  25                  30

Phe Ile Arg Asp Xaa Tyr Glu
        35

(2) INFORMATION FOR SEQ ID NO: 269:

        (i) SEQUENCE CHARACTERISTICS:
                (A) LENGTH: 67 amino acids
                (B) TYPE: amino acid
                (D) TOPOLOGY: linear
        (xi) SEQUENCE DESCRIPTION: SEQ ID NO: 269:

Lys Tyr Gly Lys Val Gly Lys Cys Val Ile Phe Glu Ile Pro Gly Ala
  1               5                  10                  15

Pro Asp Asp Glu Ala Val Arg Ile Phe Leu Glu Phe Glu Arg Val Glu
            20                  25                  30

Ser Ala Ile Lys Ala Val Val Asp Leu Asn Gly Arg Tyr Phe Gly Gly
        35                  40                  45

Arg Val Val Lys Ala Cys Phe Tyr Asn Leu Asp Lys Phe Arg Val Leu
    50                  55                  60

Asp Leu Ala
65

(2) INFORMATION FOR SEQ ID NO: 270:

(i) SEQUENCE CHARACTERISTICS:
(A) LENGTH: 12 amino acids
(B) TYPE: amino acid
(D) TOPOLOGY: linear
(xi) SEQUENCE DESCRIPTION: SEQ ID NO: 270:

Lys Ala Val Asp Leu Gly Arg Tyr Phe Gly Gly Arg
 1               5                   10

(2) INFORMATION FOR SEQ ID NO: 271:

(i) SEQUENCE CHARACTERISTICS:
(A) LENGTH: 9 amino acids
(B) TYPE: amino acid
(D) TOPOLOGY: linear
(xi) SEQUENCE DESCRIPTION: SEQ ID NO: 271:

Glu Ala Val Arg Ile Phe Phe Arg Glu
 1               5

(2) INFORMATION FOR SEQ ID NO: 272:

(i) SEQUENCE CHARACTERISTICS:
(A) LENGTH: 306 amino acids
(B) TYPE: amino acid
(D) TOPOLOGY: linear
(xi) SEQUENCE DESCRIPTION: SEQ ID NO: 272:

Arg Met Gly Arg Phe His Arg Ile Leu Glu Pro Gly Leu Asn Ile Leu
 1               5                   10                  15

Ile Pro Val Leu Asp Arg Ile Arg Tyr Val Gln Ser Leu Lys Glu Ile
            20                  25                  30

Val Ile Asn Val Pro Glu Gln Ser Ala Val Thr Leu Asp Asn Val Thr
        35                  40                  45

Leu Gln Ile Asp Gly Val Leu Tyr Leu Arg Ile Met Asp Pro Tyr Lys
    50                  55                  60

Ala Ser Tyr Gly Val Glu Asp Pro Glu Tyr Ala Val Thr Gln Leu Ala
65                  70                  75                  80

Gln Thr Thr Met Arg Ser Glu Leu Gly Lys Leu Ser Leu Asp Lys Val
                85                  90                  95

Phe Arg Glu Arg Glu Ser Leu Asn Ala Ser Ile Val Asp Ala Ile Asn
            100                 105                 110

Gln Ala Ala Asp Cys Trp Gly Ile Arg Cys Leu Arg Tyr Glu Ile Lys
        115                 120                 125

Asp Ile His Val Pro Pro Arg Val Lys Glu Ser Met Gln Met Gln Val
    130                 135                 140

Glu Ala Glu Arg Arg Lys Arg Ala Thr Val Leu Glu Ser Glu Gly Thr
145                 150                 155                 160

Arg Glu Ser Ala Ile Asn Val Ala Glu Gly Lys Lys Gln Ala Gln Ile
                165                 170                 175

Leu Ala Ser Glu Ala Glu Lys Ala Glu Gln Ile Asn Gln Ala Ala Gly
            180                 185                 190

Glu Ala Ser Ala Val Leu Ala Lys Ala Lys Ala Lys Ala Glu Ala Ile
        195                 200                 205

Arg Ile Leu Ala Ala Ala Leu Thr Gln His Asn Gly Asp Ala Ala Ala
    210                 215                 220

Ser Leu Thr Val Ala Glu Gln Tyr Val Ser Ala Phe Ser Lys Leu Ala
225                 230                 235                 240

Lys Asp Ser Asn Thr Ile Leu Leu Pro Ser Asn Pro Gly Asp Val Thr
                245                 250                 255

Ser Met Val Ala Gln Ala Met Gly Val Tyr Gly Ala Leu Thr Lys Ala
            260                 265                 270

Pro Val Pro Gly Thr Pro Asp Ser Leu Ser Ser Gly Ser Ser Arg Asp
        275                 280                 285

Val Gln Gly Thr Asp Ala Ser Leu Asp Glu Glu Leu Asp Arg Val Lys
    290                 295                 300

Met Ser
305

(2) INFORMATION FOR SEQ ID NO: 273:

        (i) SEQUENCE CHARACTERISTICS:
                (A) LENGTH: 26 amino acids
                (B) TYPE: amino acid
                (D) TOPOLOGY: linear
        (xi) SEQUENCE DESCRIPTION: SEQ ID NO: 273:

Ala Ser Tyr Gly Val Glu Asp Pro Glu Tyr Ala Val Thr Gln Leu Ala
  1               5                  10                  15

Gln Thr Thr Met Arg Ser Glu Leu Gly Lys
            20                  25

(2) INFORMATION FOR SEQ ID NO: 274:

        (i) SEQUENCE CHARACTERISTICS:

(A) LENGTH: 27 amino acids
(B) TYPE: amino acid
(D) TOPOLOGY: linear
(xi) SEQUENCE DESCRIPTION: SEQ ID NO: 274:

Met Gln Met Gln Val Glu Ala Glu Arg Arg Lys Arg Ala Thr Val Leu
1               5                   10                  15

Glu Ser Glu Gly Thr Arg Glu Ser Ala Ile Asn
            20                  25

(2) INFORMATION FOR SEQ ID NO: 275:

(i) SEQUENCE CHARACTERISTICS:
(A) LENGTH: 26 amino acids
(B) TYPE: amino acid
(D) TOPOLOGY: linear
(xi) SEQUENCE DESCRIPTION: SEQ ID NO: 275:

Leu Thr Val Ala Glu Gln Tyr Val Ser Ala Phe Ser Lys Leu Ala Lys
1               5                   10                  15

Asp Ser Asn Thr Ile Leu Leu Pro Ser Asn
            20                  25

(2) INFORMATION FOR SEQ ID NO: 276:

(i) SEQUENCE CHARACTERISTICS:
(A) LENGTH: 70 amino acids
(B) TYPE: amino acid
(D) TOPOLOGY: linear
(xi) SEQUENCE DESCRIPTION: SEQ ID NO: 276:

Leu Leu Gly Ala Thr Ala Pro Leu Val Ser Leu Val Pro Glu Val Ala
1               5                   10                  15

Ala Ala Val Gly Asn Ala Gly Ala Arg Gly Ala Xaa His Trp Gly Pro
            20                  25                  30

Phe Ala Glu Gly Leu Ser Thr Gly Phe Trp Pro Arg Ser Ala Arg Ala
        35                  40                  45

Ser Ser Gly Leu Pro Arg Asn Thr Val Val Leu Phe Val Pro Gln Gln
    50                  55                  60

Glu Ala Trp Val Val Glu
65                  70

(2) INFORMATION FOR SEQ ID NO: 277:

(i) SEQUENCE CHARACTERISTICS:
(A) LENGTH: 46 amino acids
(B) TYPE: amino acid
(D) TOPOLOGY: linear

(xi) SEQUENCE DESCRIPTION: SEQ ID NO: 277:

Arg Met Trp Arg Asn Gly Thr His Phe Trp Glu Cys Lys Ile Val Gln
1               5                   10                  15

Pro Leu Trp Lys Thr Val Trp Trp Phe Pro Arg Lys Leu Ser Ile Glu
            20                  25                  30

Leu Pro Glu Asn Leu Ala Ile Leu Ile Gly Thr Tyr Phe Lys
        35                  40                  45

(2) INFORMATION FOR SEQ ID NO: 278:

(i) SEQUENCE CHARACTERISTICS:
(A) LENGTH: 33 amino acids
(B) TYPE: amino acid
(D) TOPOLOGY: linear
(xi) SEQUENCE DESCRIPTION: SEQ ID NO: 278:

Leu Lys Arg His Phe Pro Lys Glu Ala Asn Lys His Val Lys Arg Cys
1               5                   10                  15

Ser Thr Ser Leu Asp Ile Arg Glu Ile Gln Ile Lys Ile Lys Met Arg
            20                  25                  30

Tyr

(2) INFORMATION FOR SEQ ID NO: 279:

(i) SEQUENCE CHARACTERISTICS:
(A) LENGTH: 328 amino acids
(B) TYPE: amino acid
(D) TOPOLOGY: linear
(xi) SEQUENCE DESCRIPTION: SEQ ID NO: 279:

Gly Thr Arg Pro Gly Glu Ser His Ala Asn Asp Leu Glu Cys Ser Gly
1               5                   10                  15

Lys Gly Lys Cys Thr Thr Lys Pro Ser Glu Ala Thr Phe Ser Cys Thr
            20                  25                  30

Cys Glu Glu Gln Tyr Val Gly Thr Phe Cys Glu Glu Tyr Asp Ala Cys
        35                  40                  45

Gln Arg Lys Pro Cys Gln Asn Asn Ala Ser Cys Ile Asp Ala Asn Glu
    50                  55                  60

Lys Gln Asp Gly Ser Asn Phe Thr Cys Val Cys Leu Pro Gly Tyr Thr
65                  70                  75                  80

Gly Glu Leu Cys Gln Ser Lys Ile Asp Tyr Cys Ile Leu Asp Pro Cys
                85                  90                  95

Arg Asn Gly Ala Thr Cys Ile Ser Ser Leu Ser Gly Phe Thr Cys Gln
            100                 105                 110

Cys Pro Glu Gly Tyr Phe Gly Ser Ala Cys Glu Glu Lys Val Asp Pro
        115                 120                 125

Cys Ala Ser Ser Pro Cys Gln Asn Asn Gly Thr Cys Tyr Val Asp Gly
    130                 135                 140

Val His Phe Thr Cys Asn Cys Ser Pro Gly Phe Thr Gly Pro Thr Cys
145                 150                 155                 160

Ala Gln Leu Ile Asp Phe Cys Ala Leu Ser Pro Cys Ala His Gly Thr
                165                 170                 175

Cys Arg Ser Val Gly Thr Ser Tyr Lys Cys Leu Cys Asp Pro Gly Tyr
            180                 185                 190

His Gly Leu Tyr Cys Glu Glu Glu Tyr Asn Glu Cys Leu Ser Ala Pro
        195                 200                 205

Cys Leu Asn Ala Ala Thr Cys Arg Asp Leu Val Asn Gly Tyr Glu Cys
    210                 215                 220

Val Cys Leu Ala Glu Tyr Lys Gly Thr His Cys Glu Leu Tyr Lys Asp
225                 230                 235                 240

Pro Cys Ala Asn Val Ser Cys Leu Asn Gly Ala Thr Cys Asp Ser Asp
                245                 250                 255

Gly Leu Asn Gly Thr Cys Ile Cys Ala Pro Gly Phe Thr Gly Glu Glu
            260                 265                 270

Cys Asp Ile Asp Ile Asn Glu Cys Asp Ser Asn Pro Cys His His Gly
        275                 280                 285

Gly Ser Cys Leu Asp Gln Pro Asn Gly Tyr Asn Cys His Cys Pro His
    290                 295                 300

Gly Trp Val Gly Ala Asn Cys Glu Ile His Leu Gln Trp Lys Ser Gly
305                 310                 315                 320

His Met Ala Glu Ser Leu Thr Asn
                325

(2) INFORMATION FOR SEQ ID NO: 280:

(i) SEQUENCE CHARACTERISTICS:
(A) LENGTH: 25 amino acids
(B) TYPE: amino acid
(D) TOPOLOGY: linear
(xi) SEQUENCE DESCRIPTION: SEQ ID NO: 280:

Gly Lys Cys Thr Thr Lys Pro Ser Glu Ala Thr Phe Ser Cys Thr Cys
1               5                   10                  15

Glu Glu Gln Tyr Val Gly Thr Phe Cys
            20                  25

(2) INFORMATION FOR SEQ ID NO: 281:

(i) SEQUENCE CHARACTERISTICS:
(A) LENGTH: 22 amino acids
(B) TYPE: amino acid
(D) TOPOLOGY: linear
(xi) SEQUENCE DESCRIPTION: SEQ ID NO: 281:

Cys Ala His Gly Thr Cys Arg Ser Val Gly Thr Ser Tyr Lys Cys Leu
1               5                   10                  15

Cys Asp Pro Gly Tyr His
            20

(2) INFORMATION FOR SEQ ID NO: 282:

(i) SEQUENCE CHARACTERISTICS:
(A) LENGTH: 33 amino acids
(B) TYPE: amino acid
(D) TOPOLOGY: linear
(xi) SEQUENCE DESCRIPTION: SEQ ID NO: 282:

Cys Ala Asn Val Ser Cys Leu Asn Gly Ala Thr Cys Asp Ser Asp Gly
1               5                   10                  15

Leu Asn Gly Thr Cys Ile Cys Ala Pro Gly Phe Thr Gly Glu Glu Cys
            20                  25                  30

Asp

(2) INFORMATION FOR SEQ ID NO: 283:

(i) SEQUENCE CHARACTERISTICS:
(A) LENGTH: 299 amino acids
(B) TYPE: amino acid
(D) TOPOLOGY: linear
(xi) SEQUENCE DESCRIPTION: SEQ ID NO: 283:

Met Ala Gln Asn Leu Lys Asp Leu Ala Gly Arg Leu Pro Ala Gly Pro
1               5                   10                  15

Arg Gly Met Gly Thr Ala Leu Lys Leu Leu Leu Gly Ala Gly Ala Val
            20                  25                  30

Ala Tyr Gly Val Arg Glu Ser Val Phe Thr Val Glu Gly Gly His Arg
        35                  40                  45

Ala Ile Phe Phe Asn Arg Ile Gly Gly Val Gln Gln Asp Thr Ile Leu
    50                  55                  60

Ala Glu Gly Leu His Phe Arg Ile Pro Trp Phe Gln Tyr Pro Ile Ile
65                  70                  75                  80

Tyr Asp Ile Arg Ala Arg Pro Arg Lys Ile Ser Ser Pro Thr Gly Ser

85                    90                    95

Lys Asp Leu Gln Met Val Asn Ile Ser Leu Arg Val Leu Ser Arg Pro
            100                 105                 110

Asn Ala Gln Glu Leu Pro Ser Met Tyr Gln Arg Leu Gly Leu Asp Tyr
        115                 120                 125

Glu Glu Arg Val Leu Pro Ser Ile Val Asn Glu Val Leu Lys Ser Val
    130                 135                 140

Val Ala Lys Phe Asn Ala Ser Gln Leu Ile Thr Gln Arg Ala Gln Val
145                 150                 155                 160

Ser Leu Leu Ile Arg Arg Glu Leu Thr Glu Arg Ala Lys Asp Phe Ser
                165                 170                 175

Leu Ile Leu Asp Asp Val Ala Ile Thr Glu Leu Ser Phe Ser Arg Glu
            180                 185                 190

Tyr Thr Ala Ala Val Glu Ala Lys Gln Val Ala Gln Gln Glu Ala Gln
        195                 200                 205

Arg Ala Gln Phe Leu Val Glu Lys Ala Lys Gln Glu Gln Arg Gln Lys
    210                 215                 220

Ile Val Gln Ala Glu Gly Glu Ala Glu Ala Ala Lys Met Leu Gly Glu
225                 230                 235                 240

Ala Leu Ser Lys Asn Pro Gly Tyr Ile Lys Leu Arg Lys Ile Arg Ala
                245                 250                 255

Ala Gln Asn Ile Ser Lys Thr Ile Ala Thr Ser Gln Asn Arg Ile Tyr
            260                 265                 270

Leu Thr Ala Asp Asn Leu Val Leu Asn Leu Gln Asp Glu Ser Phe Thr
        275                 280                 285

Arg Gly Ser Asp Ser Leu Ile Lys Gly Lys Lys
    290                 295

(2) INFORMATION FOR SEQ ID NO: 284:

(i) SEQUENCE CHARACTERISTICS:
(A) LENGTH: 18 amino acids
(B) TYPE: amino acid
(D) TOPOLOGY: linear
(xi) SEQUENCE DESCRIPTION: SEQ ID NO: 284:

Lys Ala Leu Ala Leu Ser Phe His Gly Trp Ser Gly Thr Gly Lys Asn
  1               5                  10                  15

Phe Val

(2) INFORMATION FOR SEQ ID NO: 285:

(i) SEQUENCE CHARACTERISTICS:
(A) LENGTH: 22 amino acids
(B) TYPE: amino acid
(D) TOPOLOGY: linear
(xi) SEQUENCE DESCRIPTION: SEQ ID NO: 285:

Asn Leu Ile Asp Tyr Phe Ile Pro Phe Leu Pro Leu Glu Tyr Arg His
1               5                   10                  15

Val Arg Leu Cys Ala Arg
            20

(2) INFORMATION FOR SEQ ID NO: 286:

(i) SEQUENCE CHARACTERISTICS:
(A) LENGTH: 20 amino acids
(B) TYPE: amino acid
(D) TOPOLOGY: linear
(xi) SEQUENCE DESCRIPTION: SEQ ID NO: 286:

Asn Leu Ile Asp Tyr Phe Ile Pro Phe Leu Pro Leu Glu Tyr Arg His
1               5                   10                  15

Val Arg Leu Cys
            20

(2) INFORMATION FOR SEQ ID NO: 287:

(i) SEQUENCE CHARACTERISTICS:
(A) LENGTH: 26 amino acids
(B) TYPE: amino acid
(D) TOPOLOGY: linear
(xi) SEQUENCE DESCRIPTION: SEQ ID NO: 287:

Cys His Gln Thr Leu Phe Ile Phe Asp Glu Ala Glu Lys Leu His Pro
1               5                   10                  15

Gly Leu Leu Glu Val Leu Gly Pro His Leu
            20                  25

(2) INFORMATION FOR SEQ ID NO: 288:

(i) SEQUENCE CHARACTERISTICS:
(A) LENGTH: 21 amino acids
(B) TYPE: amino acid
(D) TOPOLOGY: linear
(xi) SEQUENCE DESCRIPTION: SEQ ID NO: 288:

Pro Glu Lys Ala Leu Ala Leu Ser Phe His Gly Trp Ser Gly Thr Gly
1               5                   10                  15

Lys Asn Phe Val Ala
            20

(2) INFORMATION FOR SEQ ID NO: 289:

(i) SEQUENCE CHARACTERISTICS:
(A) LENGTH: 23 amino acids
(B) TYPE: amino acid
(D) TOPOLOGY: linear
(xi) SEQUENCE DESCRIPTION: SEQ ID NO: 289:

Asn Leu Lys Glu Lys Ile Phe Ile Ser Phe Ala Trp Leu Pro Lys Ala
1               5                   10                  15

Thr Val Gln Ala Ala Ile Gly
            20

(2) INFORMATION FOR SEQ ID NO: 290:

(i) SEQUENCE CHARACTERISTICS:
(A) LENGTH: 17 amino acids
(B) TYPE: amino acid
(D) TOPOLOGY: linear
(xi) SEQUENCE DESCRIPTION: SEQ ID NO: 290:

Trp Leu Pro Lys Ala Thr Val Gln Ala Ala Ile Gly Ser Val Ala Leu
1               5                   10                  15

Asp

(2) INFORMATION FOR SEQ ID NO: 291:

(i) SEQUENCE CHARACTERISTICS:
(A) LENGTH: 18 amino acids
(B) TYPE: amino acid
(D) TOPOLOGY: linear
(xi) SEQUENCE DESCRIPTION: SEQ ID NO: 291:

His Asp Arg Thr Met Gln Asp Ile Val Tyr Lys Leu Val Pro Gly Leu
1               5                   10                  15

Gln Glu

(2) INFORMATION FOR SEQ ID NO: 292:

(i) SEQUENCE CHARACTERISTICS:
(A) LENGTH: 23 amino acids
(B) TYPE: amino acid
(D) TOPOLOGY: linear
(xi) SEQUENCE DESCRIPTION: SEQ ID NO: 292:

Phe Ala Ser His Asp Arg Thr Met Gln Asp Ile Val Tyr Lys Leu Val

1                 5                   10                  15

Pro Gly Leu Gln Glu Gly Glu
            20

(2) INFORMATION FOR SEQ ID NO: 293:

      (i) SEQUENCE CHARACTERISTICS:
            (A) LENGTH: 17 amino acids
            (B) TYPE: amino acid
            (D) TOPOLOGY: linear
      (xi) SEQUENCE DESCRIPTION: SEQ ID NO: 293:

Leu Val Leu Ser Leu Gly Ala Trp Gly Trp Pro Ser Thr Cys Leu Trp
  1               5                   10                  15

Trp

(2) INFORMATION FOR SEQ ID NO: 294:

      (i) SEQUENCE CHARACTERISTICS:
            (A) LENGTH: 15 amino acids
            (B) TYPE: amino acid
            (D) TOPOLOGY: linear
      (xi) SEQUENCE DESCRIPTION: SEQ ID NO: 294:

Gln Gly Lys Leu Gln Met Trp Val Asp Val Phe Pro Lys Ser Leu
  1               5                   10                  15

(2) INFORMATION FOR SEQ ID NO: 295:

      (i) SEQUENCE CHARACTERISTICS:
            (A) LENGTH: 16 amino acids
            (B) TYPE: amino acid
            (D) TOPOLOGY: linear
      (xi) SEQUENCE DESCRIPTION: SEQ ID NO: 295:

Pro Pro Phe Asn Ile Thr Pro Arg Lys Ala Lys Lys Tyr Tyr Leu Arg
  1               5                   10                  15

(2) INFORMATION FOR SEQ ID NO: 296:

      (i) SEQUENCE CHARACTERISTICS:
            (A) LENGTH: 19 amino acids
            (B) TYPE: amino acid
            (D) TOPOLOGY: linear
      (xi) SEQUENCE DESCRIPTION: SEQ ID NO: 296:

Lys Thr Asp Val His Tyr Arg Ser Leu Asp Gly Glu Gly Asn Phe Asn
1               5                   10                  15

Trp Arg Phe

(2) INFORMATION FOR SEQ ID NO: 297:

(i) SEQUENCE CHARACTERISTICS:
(A) LENGTH: 26 amino acids
(B) TYPE: amino acid
(D) TOPOLOGY: linear
(xi) SEQUENCE DESCRIPTION: SEQ ID NO: 297:

Pro Arg Leu Ile Ile Gln Ile Trp Asp Asn Asp Lys Phe Ser Leu Asp
1               5                   10                  15

Asp Tyr Leu Gly Phe Leu Glu Leu Asp Leu
            20                  25

(2) INFORMATION FOR SEQ ID NO: 298:

(i) SEQUENCE CHARACTERISTICS:
(A) LENGTH: 15 amino acids
(B) TYPE: amino acid
(D) TOPOLOGY: linear
(xi) SEQUENCE DESCRIPTION: SEQ ID NO: 298:

Ala Val Met Ile Gly Asp Asp Cys Arg Asp Asp Val Gly Gly Ala
1               5                   10                  15

(2) INFORMATION FOR SEQ ID NO: 299:

(i) SEQUENCE CHARACTERISTICS:
(A) LENGTH: 17 amino acids
(B) TYPE: amino acid
(D) TOPOLOGY: linear
(xi) SEQUENCE DESCRIPTION: SEQ ID NO: 299:

Ile Leu Val Lys Thr Gly Lys Tyr Arg Ala Ser Asp Glu Glu Lys Ile
1               5                   10                  15

Asn

(2) INFORMATION FOR SEQ ID NO: 300:

(i) SEQUENCE CHARACTERISTICS:
(A) LENGTH: 277 amino acids
(B) TYPE: amino acid
(D) TOPOLOGY: linear
(xi) SEQUENCE DESCRIPTION: SEQ ID NO: 300:

Met Asp Ser Met Pro Glu Pro Ala Ser Arg Cys Leu Leu Leu Leu Pro
1               5                   10                  15

Leu Leu Leu Leu Leu Leu Leu Leu Leu Pro Ala Pro Glu Leu Gly Pro
            20                  25                  30

Ser Gln Ala Gly Ala Glu Glu Asn Asp Trp Val Arg Leu Pro Ser Lys
        35                  40                  45

Cys Glu Val Cys Lys Tyr Val Ala Val Glu Leu Lys Lys Pro Leu Arg
    50                  55                  60

Lys Arg Gln Asp Thr Glu Val Ile Gly Thr Val Tyr Gly Ile Leu Asp
65                  70                  75                  80

Gln Lys Ala Ser Gly Val Lys Tyr Thr Lys Ser Asp Leu Arg Leu Ile
                85                  90                  95

Glu Val Thr Glu Thr Ile Cys Lys Arg Leu Leu Asp Tyr Ser Leu His
            100                 105                 110

Lys Glu Arg Thr Gly Ser Xaa Arg Phe Ala Lys Gly Met Ser Glu Thr
        115                 120                 125

Phe Glu Thr Leu His Xaa Leu Val His Lys Gly Val Lys Val Val Met
    130                 135                 140

Asp Ile Pro Tyr Glu Leu Trp Asn Glu Thr Ser Ala Glu Val Ala Asp
145                 150                 155                 160

Leu Lys Lys Gln Cys Asp Val Leu Val Glu Glu Phe Glu Glu Val Ile
                165                 170                 175

Glu Asp Trp Tyr Arg Asn His Gln Glu Glu Asp Leu Thr Glu Phe Leu
            180                 185                 190

Cys Ala Asn His Val Leu Lys Gly Lys Asp Thr Ser Cys Leu Ala Glu
        195                 200                 205

Gln Trp Ser Gly Lys Lys Gly Asp Thr Ala Ala Leu Gly Gly Lys Lys
    210                 215                 220

Ser Lys Lys Lys Ser Ile Arg Ala Lys Ala Ala Gly Gly Arg Ser Ser
225                 230                 235                 240

Ser Ser Lys Gln Arg Lys Glu Leu Gly Gly Leu Glu Gly Asp Pro Ser
                245                 250                 255

Pro Glu Glu Asp Glu Gly Ile Gln Lys Ala Ser Pro Leu Thr His Ser
            260                 265                 270

Pro Pro Asp Glu Leu
        275

(2) INFORMATION FOR SEQ ID NO: 301:

(i) SEQUENCE CHARACTERISTICS:

(A) LENGTH: 199 amino acids
(B) TYPE: amino acid
(D) TOPOLOGY: linear
(xi) SEQUENCE DESCRIPTION: SEQ ID NO: 301:

Met Asp Gly Gln Lys Lys Asn Trp Lys Asp Lys Val Val Asp Leu Leu
1               5                   10                  15

Tyr Trp Arg Asp Ile Lys Lys Thr Gly Val Val Phe Gly Ala Ser Leu
            20                  25                  30

Phe Leu Leu Leu Ser Leu Thr Val Phe Ser Ile Val Ser Val Thr Ala
        35                  40                  45

Tyr Ile Ala Leu Ala Leu Leu Ser Val Thr Ile Ser Phe Arg Ile Tyr
    50                  55                  60

Lys Gly Val Ile Gln Ala Ile Gln Lys Ser Asp Glu Gly His Pro Phe
65                  70                  75                  80

Arg Ala Tyr Leu Glu Ser Glu Val Ala Ile Ser Glu Glu Leu Val Gln
                85                  90                  95

Lys Tyr Ser Asn Ser Ala Leu Gly His Val Asn Cys Thr Ile Lys Glu
            100                 105                 110

Leu Arg Arg Leu Phe Leu Val Asp Asp Leu Val Asp Ser Leu Lys Phe
        115                 120                 125

Ala Val Leu Met Trp Val Phe Thr Tyr Val Gly Ala Leu Phe Asn Gly
    130                 135                 140

Leu Thr Leu Leu Ile Leu Ala Leu Ile Ser Leu Phe Ser Val Pro Val
145                 150                 155                 160

Ile Tyr Glu Arg His Gln Ala Gln Ile Asp His Tyr Leu Gly Leu Ala
                165                 170                 175

Asn Lys Asn Val Lys Asp Ala Met Ala Lys Ile Gln Ala Lys Ile Pro
            180                 185                 190

Gly Leu Lys Arg Lys Ala Glu
        195

(2) INFORMATION FOR SEQ ID NO: 302:

(i) SEQUENCE CHARACTERISTICS:
(A) LENGTH: 15 amino acids
(B) TYPE: amino acid
(D) TOPOLOGY: linear
(xi) SEQUENCE DESCRIPTION: SEQ ID NO: 302:

Met Ala Val Thr Leu Ser Leu Leu Leu Gly Gly Arg Val Cys Ala
1               5                   10                  15

(2) INFORMATION FOR SEQ ID NO: 303:

(i) SEQUENCE CHARACTERISTICS:
(A) LENGTH: 41 amino acids
(B) TYPE: amino acid
(D) TOPOLOGY: linear
(xi) SEQUENCE DESCRIPTION: SEQ ID NO: 303:

Pro Ser Leu Ala Val Gly Ser Arg Pro Gly Gly Trp Arg Ala Gln Ala
1               5                   10                  15

Leu Leu Ala Gly Ser Arg Thr Pro Ile Pro Thr Gly Ser Arg Arg Asn
            20                  25                  30

Gly Ser Cys Arg Arg Trp Arg Ala Pro
        35                  40

(2) INFORMATION FOR SEQ ID NO: 304:

(i) SEQUENCE CHARACTERISTICS:
(A) LENGTH: 56 amino acids
(B) TYPE: amino acid
(D) TOPOLOGY: linear
(xi) SEQUENCE DESCRIPTION: SEQ ID NO: 304:

Met Ala Val Thr Leu Ser Leu Leu Leu Gly Gly Arg Val Cys Ala Pro
1               5                   10                  15

Ser Leu Ala Val Gly Ser Arg Pro Gly Gly Trp Arg Ala Gln Ala Leu
            20                  25                  30

Leu Ala Gly Ser Arg Thr Pro Ile Pro Thr Gly Ser Arg Arg Asn Gly
        35                  40                  45

Ser Cys Arg Arg Trp Arg Ala Pro
    50                  55

(2) INFORMATION FOR SEQ ID NO: 305:

(i) SEQUENCE CHARACTERISTICS:
(A) LENGTH: 481 base pairs
(B) TYPE: nucleic acid
(C) STRANDEDNESS: double
(D) TOPOLOGY: linear

(xi) SEQUENCE DESCRIPTION: SEQ ID NO: 305:

GATGTTACAC AGCTCTTTAA TAATAGTGGC CATAGCTGTA ATAACAATGA CAACAGTAGG 60

TAACGGTAGT CATACCAACA GTAGGGCAGT GCATTTTATA TTACAACTGG TTTCTTGCTC 120

TAGTAGGCTT GGGGATGGGT GAAGACGGAC AGGGCTGGCG CAGACCCTTT CCTTCTCCTC 180

TCCAGCCCAC AGTGATCTGG GCTTTTACAA GACAGCCTGC TTCCATTCAG TAGTGTGGGA 240

AAGTTCCTTC TTGGCTTAGC AATACCCCTG AGACCTTGTT CAGTGGGCTG TGTCTCTCCC 300

TGGGATGCTG GGAGCACCAA GTGTGGCCGA GCTAGGGCTG CTGACTTCCT CTGGGCGCCT 360

CTGGGCTGCG AGGGTCTCTT ATAGGAATTG AGGCCCTTTG CTGCTCCAAG AAATGCTGAG 420

GCTGTGGGCA RAGGGKTGTA CCCAAGGGGA CTCTTGCTCT GTGTCTGACT TTGGGGRATC 480

C 481

(2) INFORMATION FOR SEQ ID NO: 306:

(i) SEQUENCE CHARACTERISTICS:
(A) LENGTH: 58 base pairs
(B) TYPE: nucleic acid
(C) STRANDEDNESS: double
(D) TOPOLOGY: linear

(xi) SEQUENCE DESCRIPTION: SEQ ID NO: 306:

CACAGCTCTT TAATAATAGT GGCCATAGCT GTAATAACAA TGACAACAGT AGGTAACG 58

(2) INFORMATION FOR SEQ ID NO: 307:

(i) SEQUENCE CHARACTERISTICS:
(A) LENGTH: 59 base pairs
(B) TYPE: nucleic acid
(C) STRANDEDNESS: double
(D) TOPOLOGY: linear

(xi) SEQUENCE DESCRIPTION: SEQ ID NO: 307:

TGTGTCTCTC CCTGGGATGC TGGGAGCACC AAGTGTGGCC GAGCTAGGGC TGCTGACTT 59

(2) INFORMATION FOR SEQ ID NO: 308:

(i) SEQUENCE CHARACTERISTICS:
(A) LENGTH: 85 base pairs
(B) TYPE: nucleic acid
(C) STRANDEDNESS: double
(D) TOPOLOGY: linear

(xi) SEQUENCE DESCRIPTION: SEQ ID NO: 308:

GCGAGGGTCT CTTATAGGAA TTGAGGCCCT TTGCTGCTCC AAGAAATGCT GAGGCTGTGG 60

GCARAGGGKT GTACCCAAGG GGACT 85

(2) INFORMATION FOR SEQ ID NO: 309:

(i) SEQUENCE CHARACTERISTICS:
(A) LENGTH: 34 amino acids
(B) TYPE: amino acid
(D) TOPOLOGY: linear
(xi) SEQUENCE DESCRIPTION: SEQ ID NO: 309:

Met Val Gly Pro Val Thr Leu His Lys Lys Ile His Thr Thr Thr Val
1               5                   10                  15

Leu Phe Ile Val Gln Ile His Ile Leu Leu Ile Gln Ala Ile Thr Gln
            20                  25                  30

Ala Lys

(2) INFORMATION FOR SEQ ID NO: 310:

(i) SEQUENCE CHARACTERISTICS:
(A) LENGTH: 67 amino acids
(B) TYPE: amino acid
(D) TOPOLOGY: linear
(xi) SEQUENCE DESCRIPTION: SEQ ID NO: 310:

Leu Gln Met His Leu Met Ile Leu Gln Met Thr Gly Leu Ser Ile Leu
1               5                   10                  15

Ala Leu Leu Gly Lys Ser Thr Thr Thr Ile Val Glu Gln Lys Phe His
            20                  25                  30

Asn Gly Lys Asn Gln Lys Ser Gly Leu Lys Glu Asn Arg Asp Lys Lys
        35                  40                  45

Lys Gln Thr Arg Trp Gln Ser Thr Ala Ser Gln Lys Ile Gly Ile Thr
    50                  55                  60

Glu Glu Arg
65

(2) INFORMATION FOR SEQ ID NO: 311:

(i) SEQUENCE CHARACTERISTICS:
(A) LENGTH: 101 amino acids
(B) TYPE: amino acid
(D) TOPOLOGY: linear
(xi) SEQUENCE DESCRIPTION: SEQ ID NO: 311:

Met Val Gly Pro Val Thr Leu His Lys Lys Ile His Thr Thr Thr Val
1               5                   10                  15

Leu Phe Ile Val Gln Ile His Ile Leu Leu Ile Gln Ala Ile Thr Gln
            20                  25                  30

Ala Lys Leu Gln Met His Leu Met Ile Leu Gln Met Thr Gly Leu Ser
        35                  40                  45

Ile Leu Ala Leu Leu Gly Lys Ser Thr Thr Thr Ile Val Glu Gln Lys

50                  55                  60

Phe His Asn Gly Lys Asn Gln Lys Ser Gly Leu Lys Glu Asn Arg Asp
65                  70                  75                  80

Lys Lys Lys Gln Thr Arg Trp Gln Ser Thr Ala Ser Gln Lys Ile Gly
                85                  90                  95

Ile Thr Glu Glu Arg
            100

(2) INFORMATION FOR SEQ ID NO: 312:

(i) SEQUENCE CHARACTERISTICS:
(A) LENGTH: 74 amino acids
(B) TYPE: amino acid
(D) TOPOLOGY: linear
(xi) SEQUENCE DESCRIPTION: SEQ ID NO: 312:

Met Gln Thr Cys Pro Leu Val Gly Thr Leu Leu Thr Arg Asn Met Asp
1               5                   10                  15

Gly Tyr Thr Cys Ala Val Val Thr Ser Thr Ser Phe Trp Ile Ile Ser
            20                  25                  30

Ala Trp Xaa Leu Trp Lys Gly Ser Pro Ser Thr Ser Met Pro Thr Met
        35                  40                  45

Pro Glu Thr Pro Leu Arg Thr Leu Cys Cys Thr Lys Met Pro Ser Ile
    50                  55                  60

Phe Ser Ser Leu Met Thr Asp Gly Arg Ala
65                  70

(2) INFORMATION FOR SEQ ID NO: 313:

(i) SEQUENCE CHARACTERISTICS:
(A) LENGTH: 78 amino acids
(B) TYPE: amino acid
(D) TOPOLOGY: linear
(xi) SEQUENCE DESCRIPTION: SEQ ID NO: 313:

Met Thr Leu Ile Gln Asn Cys Trp Tyr Ser Trp Leu Phe Phe Gly Phe
1               5                   10                  15

Phe Phe His Phe Leu Arg Lys Ser Ile Ser Ile Phe Ser Ile Phe Leu
            20                  25                  30

Val Cys Phe Arg Ile Leu Ala Leu Gly Pro Thr Cys Phe Leu Val Trp
        35                  40                  45

Phe Trp Lys Ala Phe Phe Arg His Ile Leu Ile Phe Ile Cys Leu Ser
    50                  55                  60

Arg Glu Val Phe Arg Pro Arg Cys Phe Leu Val Tyr Phe Arg
65                  70                  75

(2) INFORMATION FOR SEQ ID NO: 314:

(i) SEQUENCE CHARACTERISTICS:
(A) LENGTH: 71 amino acids
(B) TYPE: amino acid
(D) TOPOLOGY: linear
(xi) SEQUENCE DESCRIPTION: SEQ ID NO: 314:

Met Gly Thr Arg Ala Gln Val Thr Pro Gly Arg Leu Pro Ile Pro Pro
1               5                   10                  15

Pro Ala Pro Gly Leu Pro Phe Ser Ala Xaa Glu Pro Leu Gln Gly Gln
            20                  25                  30

Leu Arg Arg Val Ser Ser Ser Arg Gly Gly Phe Pro Gly Leu Ala Leu
        35                  40                  45

Gln Leu Leu Arg Ser Glu Thr Val Lys Ala Tyr Val Asn Asn Glu Ile
    50                  55                  60

Asn Ile Leu Ala Ser Phe Phe
65                  70

(2) INFORMATION FOR SEQ ID NO: 315:

(i) SEQUENCE CHARACTERISTICS:
(A) LENGTH: 40 amino acids
(B) TYPE: amino acid
(D) TOPOLOGY: linear
(xi) SEQUENCE DESCRIPTION: SEQ ID NO: 315:

Met Leu Val Arg Thr Arg Pro Ser Gln Pro Leu Pro Leu Pro Gly Val
1               5                   10                  15

Gly Leu Gly Gly Pro Arg Ser Gly Asp Pro Pro Glu Ser Thr Glu Leu
            20                  25                  30

Arg Lys Gly Pro Gly Phe Leu Ala
        35                  40

(2) INFORMATION FOR SEQ ID NO: 316:

(i) SEQUENCE CHARACTERISTICS:
(A) LENGTH: 262 amino acids
(B) TYPE: amino acid
(D) TOPOLOGY: linear
(xi) SEQUENCE DESCRIPTION: SEQ ID NO: 316:

Met Cys Pro Val Cys Gly Arg Ala Leu Ser Ser Pro Gly Ser Leu Gly
1               5                   10                  15

Arg His Leu Leu Ile His Ser Glu Asp Gln Arg Ser Asn Cys Ala Val
            20                  25                  30

Cys Gly Ala Arg Phe Thr Ser His Ala Thr Phe Asn Ser Glu Lys Leu
        35                  40                  45

Pro Glu Val Leu Asn Met Glu Ser Leu Pro Thr Val His Asn Glu Gly
    50                  55                  60

Pro Ser Ser Ala Glu Gly Lys Asp Ile Ala Phe Ser Pro Pro Val Tyr
65                  70                  75                  80

Pro Ala Gly Ile Leu Leu Val Cys Asn Asn Cys Ala Ala Tyr Arg Lys
                85                  90                  95

Xaa Leu Glu Ala Gln Thr Pro Ser Val Xaa Lys Trp Ala Leu Arg Arg
            100                 105                 110

Gln Asn Glu Pro Leu Glu Val Arg Leu Gln Arg Leu Glu Arg Glu Arg
        115                 120                 125

Thr Ala Lys Lys Ser Arg Arg Asp Asn Glu Thr Pro Glu Glu Arg Glu
    130                 135                 140

Val Arg Arg Met Arg Asp Arg Glu Ala Lys Arg Leu Gln Arg Met Gln
145                 150                 155                 160

Glu Thr Asp Glu Gln Arg Ala Arg Arg Leu Gln Arg Asp Arg Glu Ala
                165                 170                 175

Met Arg Leu Lys Arg Ala Asn Glu Thr Pro Glu Lys Arg Gln Ala Arg
            180                 185                 190

Leu Ile Arg Glu Arg Glu Ala Lys Arg Leu Lys Arg Arg Leu Glu Lys
        195                 200                 205

Met Asp Met Met Leu Arg Ala Gln Phe Gly Gln Asp Pro Ser Ala Met
    210                 215                 220

Ala Ala Leu Ala Ala Glu Met Asn Phe Phe Gln Leu Pro Val Ser Gly
225                 230                 235                 240

Val Glu Leu Asp Xaa Gln Leu Leu Gly Lys Met Ala Phe Glu Glu Gln
                245                 250                 255

Asn Ser Ser Xaa Leu His
            260

(2) INFORMATION FOR SEQ ID NO: 317:

        (i) SEQUENCE CHARACTERISTICS:
                (A) LENGTH: 190 amino acids
                (B) TYPE: amino acid
                (D) TOPOLOGY: linear
        (xi) SEQUENCE DESCRIPTION: SEQ ID NO: 317:

Met Asp His Ser His His Met Gly Met Ser Tyr Met Asp Ser Asn Ser
1               5                   10                  15

Thr Met Gln Pro Ser His His His Pro Thr Thr Ser Ala Ser His Ser

20                  25                  30

His Gly Gly Gly Asp Ser Ser Met Met Met Met Pro Met Thr Phe Tyr
        35                  40                  45

Phe Gly Phe Lys Asn Val Glu Leu Leu Phe Ser Gly Leu Val Ile Asn
    50                  55                  60

Thr Ala Gly Glu Met Ala Gly Ala Phe Val Ala Val Phe Leu Leu Ala
65                  70                  75                  80

Met Phe Tyr Glu Gly Leu Lys Ile Ala Arg Glu Ser Leu Leu Arg Lys
                85                  90                  95

Ser Gln Val Ser Ile Arg Tyr Asn Ser Met Pro Val Pro Gly Pro Asn
            100                 105                 110

Gly Thr Ile Leu Met Glu Thr His Lys Thr Val Gly Gln Gln Met Leu
        115                 120                 125

Ser Phe Pro His Leu Leu Gln Thr Val Leu His Ile Ile Gln Val Val
    130                 135                 140

Ile Ser Tyr Phe Leu Met Leu Ile Phe Met Thr Tyr Asn Gly Tyr Leu
145                 150                 155                 160

Cys Ile Ala Xaa Ala Ala Gly Ala Gly Thr Gly Tyr Phe Leu Phe Ser
                165                 170                 175

Trp Lys Lys Ala Val Val Val Asp Ile Thr Glu His Cys His
            180                 185                 190

(2) INFORMATION FOR SEQ ID NO: 318:

    (i) SEQUENCE CHARACTERISTICS:
        (A) LENGTH: 123 amino acids
        (B) TYPE: amino acid
        (D) TOPOLOGY: linear
    (xi) SEQUENCE DESCRIPTION: SEQ ID NO: 318:

Met Val Gln Pro Cys Gly Ala Cys Ala Lys Thr Xaa Trp Lys Ala Cys
1               5                   10                  15

Ser Ser Cys Cys Ser Ser Pro Cys Cys Leu Gln Glu Arg Trp Pro Xaa
            20                  25                  30

Pro Xaa Ala Xaa Cys Pro Glu Xaa Gly Pro Ser Ser His Pro Gly Ile
        35                  40                  45

Gln Ala Leu Cys Ala Val Ala Val Val Tyr Leu Ser Pro Ser Ser Arg
    50                  55                  60

Leu Asp Trp Ser Leu Ala Pro Leu Phe Val Pro Ser Leu Ala Ala Gly
65                  70                  75                  80

Glu Thr Pro Leu Thr Gln Pro Ala Trp Ala Leu Thr Thr Asn Thr Leu
                85                  90                  95

Gly His Gly Gln Pro Ala Gln Asp Arg Leu Pro Ala Leu Gly His Cys
            100                 105                 110

Ala Pro Ile Ser Val Leu Gly Leu Gly Ser Ser
        115                 120

## Description

### Field of the Invention

This invention relates to newly identified polynucleotides and the polypeptides encoded by these polynucleotides, uses of such polynucleotides, and polypeptides, and their production.

### Background of the Invention

Unlike bacterium, which exist as a single compartment surrounded by a membrane, human cells and other eucaryotes are subdivided by membranes into many functionally distinct compartments. Each membrane-bounded compartment, or organelle, contains different proteins essential for the function of the organelle. The cell uses "sorting signals," which are amino acid motifs located within the protein, to target proteins to particular cellular organelles.

One type of sorting signal, called a signal sequence, a signal peptide, or a leader sequence, directs a class of proteins to an organelle called the endoplasmic reticulum (ER). The ER separates the membrane-bounded proteins from all other types of proteins. Once localized to the ER, both groups of proteins can be further directed to another organelle called the Golgi apparatus. Here, the Golgi distributes the proteins to vesicles, including secretory vesicles, the cell membrane, lysosomes, and the other organelles.

Proteins targeted to the ER by a signal sequence can be released into the extracellular space as a secreted protein. For example, vesicles containing secreted proteins can fuse with the cell membrane and release their contents into the extracellular space - a process called exocytosis. Exocytosis can occur constitutively or after receipt of a triggering signal. In the latter case, the proteins are stored in secretory vesicles (or secretory granules) until exocytosis is triggered. Similarly, proteins residing on the cell membrane can also be secreted into the extracellular space by proteolytic cleavage of a "linker" holding the protein to the membrane.

Despite the great progress made in recent years, only a small number of genes encoding human secreted proteins have been identified. These secreted proteins include the commercially valuable human insulin, interferon, Factor VIII, human growth hormone, tissue plasminogen activator, and erythropoeitin. Thus, in light of the pervasive role of secreted proteins in human physiology, a need exists for identifying and characterizing novel human secreted proteins and the genes that encode them. This knowledge will allow one to detect, to treat, and to prevent medical disorders by using secreted proteins or the genes that encode them.

### Summary of the Invention

The present invention relates to novel polynucleotides and the encoded polypeptides. Moreover, the present invention relates to vectors, host cells, antibodies, and recombinant methods for producing the polypeptides and polynucleotides. Also provided are diagnostic methods for detecting disorders related to the polypeptides, and therapeutic methods for treating such disorders. The invention further relates to screening methods for identifying binding partners of the polypeptides.

### Detailed Description

### Definitions

The following definitions are provided to facilitate understanding of certain terms used throughout this specification.

In the present invention, "isolated" refers to material removed from its original environment (e.g., the natural environment if it is naturally occurring), and thus is altered "by the hand of man" from its natural state. For example, an isolated polynucleotide could be part of a vector or a composition of matter, or could be contained within a cell, and still be "isolated" because that vector, composition of matter, or particular cell is not the original environment of the polynucleotide.

In the present invention, a "secreted" protein refers to those proteins capable of being directed to the ER, secretory vesicles, or the extracellular space as a result of a signal sequence, as well as those proteins released into the extracellular space without necessarily containing a signal sequence. If the secreted protein is released into the extracellular space, the secreted protein can undergo extracellular processing to produce a "mature" protein. Release into the extracellular space can occur by many mechanisms, including exocytosis and proteolytic cleavage.

As used herein, a "polynucleotide" refers to a molecule having a nucleic acid sequence contained in SEQ ID NO:X or the cDNA contained within the clone deposited with the ATCC. For example, the polynucleotide can contain the nucleotide sequence of the full length cDNA sequence, including the 5' and 3' untranslated sequences, the coding region, with or without the signal sequence, the secreted protein coding region, as well as fragments, epitopes, domains, and variants of the nucleic acid sequence. Moreover, as used herein, a "polypeptide" refers to a molecule having the translated amino acid sequence generated from the polynucleotide as broadly defined.

In the present invention, the full length sequence identified as SEQ ID NO:X was often generated by overlapping sequences contained in multiple clones (contig analysis). A representative clone containing all or most of the sequence for SEQ ID NO:X was deposited with the American Type Culture Collection ("ATCC"). As shown in Table 1, each clone is identified by a cDNA Clone ID (Identifier) and the ATCC Deposit Number. The ATCC is located at 10801 University Boulevard, Manassas, Virginia 20110-2209, USA. The ATCC deposit was made pursuant to the terms of the Budapest Treaty on the international recognition of the deposit of microorganisms for purposes of patent procedure.

A "polynucleotide" of the present invention also includes those polynucleotides capable of hybridizing, under stringent hybridization conditions, to sequences contained in SEQ ID NO:X, the complement thereof, or the cDNA within the clone deposited with the ATCC. "Stringent hybridization conditions" refers to an overnight incubation at 42° C in a solution comprising 50% formamide, 5x SSC (750 mM NaCl, 75 mM sodium citrate), 50 mM sodium phosphate (pH 7.6), 5x Denhardt's solution, 10% dextran sulfate, and 20 µg/ml denatured, sheared salmon sperm DNA, followed by washing the filters in 0.1x SSC at about 65°C.

Also contemplated are nucleic acid molecules that hybridize to the polynucleotides of the present invention at lower stringency hybridization conditions. Changes in the stringency of hybridization and signal detection are primarily accomplished through the manipulation of formamide concentration (lower percentages of formamide result in lowered stringency); salt conditions, or temperature. For example, lower stringency conditions include an overnight incubation at 37°C in a solution comprising 6X SSPE (20X SSPE = 3M NaCl; 0.2M NaH₂PO₄; 0.02M EDTA, pH 7.4), 0.5% SDS, 30% formamide, 100 ug/ml salmon sperm blocking DNA; followed by washes at 50°C with 1XSSPE, 0.1% SDS. In addition, to achieve even lower stringency, washes performed following stringent hybridization can be done at higher salt concentrations (e.g. 5X SSC).

Note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility.

Of course, a polynucleotide which hybridizes only to polyA+ sequences (such as any 3' terminal polyA+ tract of a cDNA shown in the sequence listing), or to a complementary stretch of T (or U) residues, would not be included in the definition of "polynucleotide," since such a polynucleotide would hybridize to any nucleic acid molecule containing a poly (A) stretch or the complement thereof (e.g., practically any double-stranded cDNA clone).

The polynucleotide of the present invention can be composed of any polyribonucleotide or polydeoxribonucleotide, which may be unmodified RNA or DNA or modified RNA or DNA. For example, polynucleotides can be composed of single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, the polynucleotide can be composed of triple-stranded regions comprising RNA or DNA or both RNA and DNA. A polynucleotide may also contain one or more modified bases or DNA or RNA backbones modified for stability or for other reasons. "Modified" bases include, for example, tritylated bases and unusual bases such as inosine. A variety of modifications can be made to DNA and RNA; thus, "polynucleotide" embraces chemically, enzymatically, or metabolically modified forms.

The polypeptide of the present invention can be composed of amino acids joined to each other by peptide bonds or modified peptide bonds, i.e., peptide isosteres, and may contain amino acids other than the 20 gene-encoded amino acids. The polypeptides may be modified by either natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched , for example, as a result of ubiquitination, and they may be cyclic, with or without branching. Cyclic, branched, and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination. (See, for instance, PROTEINS - STRUCTURE AND MOLECULAR PROPERTIES, 2nd Ed., T. E. Creighton, W. H. Freeman and Company, New York (1993); POSTTRANSLATIONAL COVALENT MODIFICATION OF PROTEINS, B. C. Johnson, Ed., Academic Press, New York, pgs. 1-12 (1983); Seifter et al., Meth Enzymol 182:626-646 (1990); Rattan et al., Ann NY Acad Sci 663:48-62 (1992).)

"SEQ ID NO:X" refers to a polynucleotide sequence while "SEQ ID NO:Y" refers to a polypeptide sequence, both sequences identified by an integer specified in Table 1.

"A polypeptide having biological activity" refers to polypeptides exhibiting activity similar, but not necessarily identical to, an activity of a polypeptide of the present invention, including mature forms, as measured in a particular biological assay, with or without dose dependency. In the case where dose dependency does exist, it need not be identical to that of the polypeptide, but rather substantially similar to the dose-dependence in a given activity as compared to the polypeptide of the present invention (i.e., the candidate polypeptide will exhibit greater activity or not more than about 25-fold less and, preferably, not more than about tenfold less activity, and most preferably, not more than about three-fold less activity relative to the polypeptide of the present invention.)

### Polynucleotides and Polypeptides of the Invention

### FEATURES OF PROTEIN ENCODED BY GENE NO: 1

The translation product of this gene shares sequence homology with LIM-homeobox domain proteins, such as T-cell translocation protein, which are thought to be important in development and leukemogenesis. In addition, translation product of this gene shares homology with the human breast tumor autoantigen (See Accession No. gil1914877). In one embodiment the polypeptides of the invention comprise the sequence:
MNGSHKDPLLPFPASARTPSLPPAPPAQAPLPWKPSGFARISPPPPLAILQYRG KADHGESGQQLAAAPGDGRLPLLEAVRRLRGQDCGPLSALCHGQLLAQPVPQ VLLLPGAXGDIGTSCYTKSGMILCRNDYIRLFGNSGACSACGQSIPASELVMRA QGNVYHLKCFTCSTCRNRLVPGDRFHYINGSLFCEHDRPTALINGHLNSLQSN PLLPDQKVCKVRVMQNACLHLRFVHHRWIPCXFSRQVTFVASTSASSMPLHLL (SEQ ID NO:211); MARTRTPSSPFLLLRELPPSLQLRQPRRPFPGSRAASLAFHRR RLSQYCNIGEKQTMVNPGSSSQPPPVTAGSLSWKRCAGCGGKIADRFLLYA (SEQ ID NO:212); LFGNSGACSACGQSIPASELVMRA (SEQ ID NO:213); HDRPTALINGHLNSLQSNP (SEQ ID NO:214); and/or LVPGDRFHYING (SEQ ID NO:215 ). Polynucleotide fragments encoding these polypeptide fragments are also encompassed by the invention.

This gene is expressed primarily in fetal brain, osteosarcoma, IL-1/TNF treated synovial, and estradiol treated endometrial stromal cells, and to a lesser extent in chondrosarcoma, smooth muscle and number of other tissues.

Therefore, polynucleotides and polypeptides of the invention are useful as reagents for differential identification of the tissue(s) or cell type(s) present in a biological sample and for diagnosis of diseases and conditions which include, but are not limited to, developmental defects or leukemia. Similarly, polypeptides and antibodies directed to these polypeptides are useful in providing immunological probes for differential identification of the tissue(s) or cell type(s). For a number of disorders of the above tissues or cells, particularly of the hematopoietic system and immune system, expression of this gene at significantly higher or lower levels may be routinely detected in certain tissues and cell types (e.g., brain and other tissue of the nervous system, bone cells, synovial tissue, endometrial tissue and other reproductive tissue, cartilage cells, smooth muscle, and blood cells and cells and tissue of the immune system, and cancerous and wounded tissues) or bodily fluids (e.g., serum, plasma, urine, synovial fluid or spinal fluid) or another tissue or cell sample or another tissue or cell sample or another tissue or cell sample taken from an individual having such a disorder, relative to the standard gene expression level, i.e., the expression level in healthy tissue or bodily fluid or bodily fluid or bodily fluid from an individual not having the disorder. Preferred epitopes include those comprising a sequence shown in SEQ ID NO. 111 as residues: Met-1 to Cys-9.

The tissue distribution and homology to the LIM-homeodomain containing proteins, such as T-cell translocation factor, indicates that polynucleotides and polypeptides corresponding to this gene are useful for diagnosis and intervention of leukemia and other developmental defects. Because of the importance of the LIM-homeodomain proteins in development and their correlation to number of leukemic diseases, the molecule can be either used as a diagnostic or prognostic indicator for leukemia progression or a therapeutic target. In addition, polynucleotides and polypeptides corresponding to this gene are useful for the detection/treatment of neurodegenerative disease states and behavioral disorders such as Alzheimer's Disease, Parkinson's Disease, Huntington's Disease, schizophrenia, mania, dementia, paranoia, obsessive compulsive disorder, panic disorder, and autism. In addition, the gene or gene product may also play a role in the treatment and/or detection of developmental disorders associated with the developing embryo, sexually-linked disorders, or disorders of the cardiovascular system. Furthermore, homology to the breast autoantigen may suggest this gene is useful in the detection, prevention, and or treatment of breast cancer and/or other proliferative disorders.

### FEATURES OF PROTEIN ENCODED BY GENE NO: 2

Translation product of gene has homology to a highly conserved member of the human calpain family of proteases, Calpain large subunit 1 gene (See Accession No.T32454). Calpains are thought to play a defining role in protein regulation, particularly during development. One embodiment for this gene is the polypeptide fragments comprising the following amino acid sequence:
MKYMGGCAKVMCKYYVILYQGLEYPLLXSGDPETSPPWILRADCIVLSSRNFH SNXGRLTINKIYVIGGGKYRGEVTNGAK (SEQ ID NO:216); MGQSELYSSILRNLGVLFLVYTRGGFLLSPLLHGTLTCAHS (SEQ ID NO:217); MVLLLLTVASYTVFWMIGDVLDILFLWNFEYTTLY (SEQ ID NO:218); MELYNSLCPICYFSTVLTTTYYIYFVYSQSSXIRMKVP (SEQ ID NO:219); MQIVIVLYCVRNKDKKKVCTCSVQTQFFFPIFPILGCLNGCRTQE (SEQ ID NO:220); MKYMGGCAKVMCKYYVILYQGLEYPLLX (SEQ ID NO:221); LEYPLLXSGDPET SPPWILRADCIVLSSRNFHSNX (SEQ ID NO:222); and/or RNFHSNXGRLTINKIY VIGGGKYRGEVTNGAK (SEQ ID NO:223). An additional embodiment is the polynucleotide fragments encoding these polypeptide fragments.

This gene is expressed primarily in caudate nucleus, dermatofibrosarcoma protuberance and apoptotic T-cells, and to alesser extent in eosinophils, brain and smooth muscle.

Therefore, polynucleotides and polypeptides of the invention are useful as reagents for differential identification of the tissue(s) or cell type(s) present in a biological sample and for diagnosis of diseases and conditions which include, but are not limited to, neurodegenerative diseases or immune disorders. Similarly, polypeptides and antibodies directed to these polypeptides are useful in providing immunological probes for differential identification of the tissue(s) or cell type(s). For a number of disorders of the above tissues or cells, particularly of the nervous system or immune system, expression of this gene at significantly higher or lower levels may be routinely detected in certain tissues and cell types (e.g., skin, T-cells and other blood cells and cells and tissue of the immune system, brain and other tissue of the nervous system, and smooth muscle, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution in caudate nucleus and apoptic T-cells indicates that polynucleotides and polypeptides corresponding to this gene are useful for detection or intervention of neurodegenerative diseases and behavioral disorders such as Alzheimer's Disease, Parkinson's Disease, Huntington's disease, schizophrenia, mania, dementia, paranoia, obsessive compulsive disorder, panic disorder or immune disorders, because the elevated level of the molecule in cells undergoing cell death may be the cause or consequence of these degenerative conditions. In addition, the gene or gene product may also play a role in the treatment and/or detection of developmental disorders associated with the developing embryo, or disorders of the cardiovascular system.

### FEATURES OF PROTEIN ENCODED BY GENE NO: 3

This gene maps to chromosome 15, and therefore, may be used as a marker in linkage analysis for chromosome 15. One embodiment for this gene is the polypeptide fragments comprising the following amino acid sequence: VTNEMSQGRGKYDFY IGLGLAMSSSIFIGGSFILKKKGLLRLARKGSMRAGQGGHAYLKEWLWWAGL LSMGAGEVANFAAYAFAPATLVTPLGALSVLVSAILSSYFLNERLNLHGKIGCL LSILGSTVMVIHAPKEEEIETLNE (SEQ ID NO:224); VTNEMSQGRGKYDFYIGLGLAMSSSIFIGGSFILKKKGLLRLARKGSMRAGQG GHAYLKEWLWWAGLLSMGAGEVANF (SEQ ID NO:225); NFAAYAFAPATLVTPLGALSVLVSAILSSY (SEQ ID NO:226); and/or ERLNLHGKIGCLLSILGSTVMVIHAPKEEEIETLNE (SEQ ID NO:227). An additional embodiment is the polynucleotide fragments encoding these polypeptide fragments

This gene is expressed primarily in colon carcinoma cell line, and to a lesser extent in aorta endothelial cells, T-cells, human erythroleukemia cells (HEL), and stromal cells (TF274).

Therefore, polynucleotides and polypeptides of the invention are useful as reagents for differential identification of the tissue(s) or cell type(s) present in a biological sample and for diagnosis of diseases and conditions which include, but are not limited to, colon carcinoma. Similarly, polypeptides and antibodies directed to these polypeptides are useful in providing immunological probes for differential identification of the tissue(s) or cell type(s). For a number of disorders of the above tissues or cells, particularly of colon carcinoma tissues, expression of this gene at significantly higher or lower levels may be routinely detected in certain tissues and cell types (e.g., colon, aorta and other vascular tissue, T-cells and other cells and tissue of the immune system, and stromal cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 113 as residues: Asn-191 to Ser-196, Asn-208 to Gly-214.

The tissue distribution in colon carcinoma indicates that polynucleotides and polypeptides corresponding to this gene are useful for detection and intervention of colon carcinoma and/or other tumors. Additionally the significant presence in T-cell populations may indicate the involvement of the function of the gene product in cancer immunosurveillance. Furthermore, the tissue distribution indicates that polynucleotides and polypeptides corresponding to this gene are useful for the diagnosis and treatment of cancer and other proliferative disorders, in general. The expression in hematopoietic cells and tissues indicates that this protein may play a role in the proliferation, differentiation, and/or survival of hematopoietic cell lineages. Thus, this gene may be useful in the treatment of lymphoproliferative disorders, and in the maintenance and differentiation of various hematopoietic lineages from early hematopoietic stem and committed progenitor cells.

### FEATURES OF PROTEIN ENCODED BY GENE NO: 4

This gene is expressed primarily in ovary.

Therefore, polynucleotides and polypeptides of the invention are useful as reagents for differential identification of the tissue(s) or cell type(s) present in a biological sample and for diagnosis of diseases and conditions which include, but are not limited to, reproductive or endocrine disorders. Similarly, polypeptides and antibodies directed to these polypeptides are useful in providing immunological probes for differential identification of the tissue(s) or cell type(s). For a number of disorders of the above tissues or cells, particularly of the reproductive or endocrine systems, expression of this gene at significantly higher or lower levels may be routinely detected in certain tissues and cell types (e.g., ovary and other reproductive tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 114 as residues: Pro-20 to Ser-25.

The tissue distribution in ovary indicates that polynucleotides and polypeptides corresponding to this gene are useful for assessing reproductive dysfunction or endocrine disorders, because factors secreted by ovary may be involved in reproductive processes, and in cases have global hormonal effects.

### FEATURES OF PROTEIN ENCODED BY GENE NO: 5

This gene is expressed primarily in tissues in the central nervous system, including pineal gland, frontal cortex, and dura mater, and to a lesser extent in bladder, lung, T-cells and liver.

Therefore, polynucleotides and polypeptides of the invention are useful as reagents for differential identification of the tissue(s) or cell type(s) present in a biological sample and for diagnosis of diseases and conditions which include, but are not limited to, neurodegenerative diseases, endocrine disorders, and immune disorders. Similarly, polypeptides and antibodies directed to these polypeptides are useful in providing immunological probes for differential identification of the tissue(s) or cell type(s). For a number of disorders of the above tissues or cells, particularly of the nervous and endocrine systems, expression of this gene at significantly higher or lower levels may be routinely detected in certain tissues and cell types (e.g., tissue of the nervous system, bladder, lung, liver, and T-cells and other cells and tissues of the immune system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 115 as residues: Glu-14 to Arg-20.

The primary tissue distribution in the central nerve system indicates that polynucleotides and polypeptides corresponding to this gene are useful for the detection and intervention of neurodegenerative diseases or endocrinedisorders, because extracellular proteins in these tissues may function as a neurotrophic factor, a matrix protein for tissue integrity, a neuroguidance factor or as a hormone.

### FEATURES OF PROTEIN ENCODED BY GENE NO: 6

This gene is expressed primarily in spleen, resting T-cells, colorectal tumor and pancreatic carcinoma, and to a lesser extent in number of tissues including prostate, synovial hypoxia, osteosarcoma, ulcerative colitis, myeloid progenitor cells, lung and placenta.

Therefore, polynucleotides and polypeptides of the invention are useful as reagents for differential identification of the tissue(s) or cell type(s) present in a biological sample and for diagnosis of diseases and conditions which include, but are not limited to, inflammation, immunosurveillance of cancers, and immune and gastrointestinal disorders. Similarly, polypeptides and antibodies directed to these polypeptides are useful in providing immunological probes for differential identification of the tissue(s) or cell type(s). For a number of disorders of the above tissues or cells, particularly in carcinogenesis or the immune system, expression of this gene at significantly higher or lower levels may be routinely detected in certain tissues and cell types (e.g., prostate, synovial tissue, bone cells, colon, myeloid progenitor cells, lung, cells and tissue of the immune system, cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 116 as residues: Arg-29 to Pro-37, Gln-46 to Val-56.

The primary tissue distribution in lymphatic tissues such as T-cells and spleen, as well as tumors and ulcerative tissues indicates that the protein product of this gene may be involved in the immune response to or immunosurveillance of carcinogenesis and/or inflammatory conditions.

### FEATURES OF PROTEIN ENCODED BY GENE NO: 7

The translation product of this gene shares very weak sequence homology with voltage dependent sodium channel protein and Bowman-Birk proteinassse inhibitor which is thought to be important in membrane signaling or extracellular signaling cascades. One embodiment for this gene is the polypeptide fragments comprising the following amino acid sequence: RFKTLMTNKSEQDGDSSKTIEISDMKYHIFQ (SEQ ID NO:228); and/or LVEGKLFYAHKVLLVTXSNR (SEQ ID NO:229) (See Accession No.gnIlPIDld1020763 (AB000216)). An additional embodiment is the polynucleotide fragments encoding these polypeptide fragments.

This gene is expressed primarily in prostate cancer.

Therefore, polynucleotides and polypeptides of the invention are useful as reagents for differential identification of the tissue(s) or cell type(s) present in a biological sample and for diagnosis of diseases and conditions which include, but are not limited to, prostate cancer. Similarly, polypeptides and antibodies directed to these polypeptides are useful in providing immunological probes for differential identification of the tissue(s) or cell type(s). For a number of disorders of the above tissues or cells, particularly of prostate cancer tissue, expression of this gene at significantly higher or lower levels may be routinely detected in certain tissues and cell types (e.g., prostate and cancerous and wounded tissues) or bodily fluids (e.g., serum, plasma; urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 117 as residues: Glu-30 to Ser-35.

The tissue distribution in the prostate cancer and homology to sodium channel or proteinase inhibitor suggest that polynucleotides and polypeptides corresponding to this gene are useful for the intervention of cancer progression, because the gene product may be involved in multidrug resistance by altering the drug kinetics by serving the function as a channel transporter. Alternatively, the proteinase inhibitor like function may facilitate tumor metastasis. By targeting these functions, either through vaccine or small molecules, therapeutics may be rationally designed to slow the cancer progression.

### FEATURES OF PROTEIN ENCODED BY GENE NO: 8

This gene is expressed primarily in ovary and to a lesser extent in the adrenal gland.

Therefore, polynucleotides and polypeptides of the invention are useful as reagents for differential identification of the tissue(s) or cell type(s) present in a biological sample and for diagnosis of diseases and conditions which include, but are not limited to, female infertility and endocrine disorders. Similarly, polypeptides and antibodies directed to these polypeptides are useful in providing immunological probes for differential identification of the tissue(s) or cell type(s). For a number of disorders of the above tissues or cells, particularly of the female reproductive system and the endocrine system, expression of this gene at significantly higher or lower levels may be routinely detected in certain tissues and cell types (e.g., ovary and other reproductive tissue, and adrenal gland, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution of this gene in ovary and adrenal gland indicates that polynucleotides and polypeptides corresponding to this gene are useful for treatment/diagnosis of female infertility, endocrine disorders, ovarian function, amenorrhea, ovarian cancer and metabolic disorders.

### FEATURES OF PROTEIN ENCODED BY GENE NO: 9

This gene is expressed only in prostate cancer.

Therefore, polynucleotides and polypeptides of the invention are useful as reagents for differential identification of the tissue(s) or cell type(s) present in a biological sample and for diagnosis of diseases and conditions which include, but are not limited to, prostate disorders including cancer. Similarly, polypeptides and antibodies directed to these polypeptides are useful in providing immunological probes for differential identification of the tissue(s) or cell type(s). For a number of disorders of the above tissues or cells, particularly of the endocrine and male reproductive system, expression of this gene at significantly higher or lower levels may be routinely detected in certain tissues and cell types (e.g., prostrate and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution of this gene only in prostate cancerous tissue, indicates that polynucleotides and polypeptides corresponding to this gene are useful for the treatment/diagnosis of male infertility, metabolic disorders, and prostate disorders including benign prostate hyperplasia and prostate cancer.

### FEATURES OF PROTEIN ENCODED BY GENE NO: 10

This gene is expressed primarily in placenta and to a lesser extent in ovary.

Therefore, polynucleotides and polypeptides of the invention are useful as reagents for differential identification of the tissue(s) or cell type(s) present in a biological sample and for diagnosis of diseases and conditions which include, but are not limited to, female infertility, pregnancy disorders, and ovarian cancer. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels may be routinely detected in certain tissues and cell types (e.g., placenta, and ovary and other reproductive tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 120 as residues: Gln-39 to Gly-73.

The tissue distribution of this gene in placenta and ovary indicates that polynucleotides and polypeptides corresponding to this gene are useful for treatment/diagnosis of female infertility, endocrine disorders, fetal deficiencies, ovarian failure, amenorrhea, and ovarian cancer.

### FEATURES OF PROTEIN ENCODED BY GENE NO: 11

Gene shares homology with the gene for the Human 3' apolipoprotein B SAR element gene Rh32 (See Accession No. T31530).

This gene is expressed primarily in prostate and in the pancreas.

Therefore, polynucleotides and polypeptides of the invention are useful as reagents for differential identification of the tissue(s) or cell type(s) present in a biological sample and for diagnosis of diseases and conditions which include, but are not limited to, prostate and pancreatic disorders. Similarly, polypeptides and antibodies directed to these polypeptides are useful in providing immunological probes for differential identification of the tissue(s) or cell type(s). For a number of disorders of the above tissues or cells, particularly of the endocrine system, expression of this gene at significantly higher or lower levels may be routinely detected in certain tissues and cell types (e.g., prostate and pancreas, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution of this gene in prostate and pancrease, indicates that polynucleotides and polypeptides corresponding to this gene are useful for treatment/diagnosis of male infertility, prostate disorders including benign prostate hyperplasia, prostate cancer, pancreatic cancer, type I and type II diabetes and hypoglycemia. Homology to a known human apolipoprotein may suggest this gene is useful for the detection, prevention, or treatment of various metabolic disorders, particularly those secondary to lipoprotein disorders such as atherosclerosis, coronary heart disease, stroke, and hyperlipidemias.

### FEATURES OF PROTEIN ENCODED BY GENE NO: 12

Gene has homology to conserved Beta-casein, an abundant milk protein (See Accession No.Q37894).

This gene is expressed primarily in stomach.

Therefore, polynucleotides and polypeptides of the invention are useful as reagents for differential identification of the tissue(s) or cell type(s) present in a biological sample and for diagnosis of diseases and conditions which include, but are not limited to, disorders of the digestive tract and/or mammary glands. Similarly, polypeptides and antibodies directed to these polypeptides are useful in providing immunological probes for differential identification of the tissue(s) or cell type(s). For a number of disorders of the above tissues or cells, particularly of the digestive system and breast, expression of this gene at significantly higher or lower levels may be routinely detected in certain tissues and cell types (e.g., mammary tissue, and stomach and other gastrointestinal tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution of this gene indicates a role in the treatment/diagnosis of digestive disorders including stomach cancer and ulceration. Furthermore, the homology to conserved beta-casein may indicate this gene as having utility in the diagnosis and prevention of mammary gland disorders.

### FEATURES OF PROTEIN ENCODED BY GENE NO: 13

This gene is expressed in brain and lung.

Therefore, polynucleotides and polypeptides of the invention are useful as reagents for differential identification of the tissue(s) or cell type(s) present in a biological sample and for diagnosis of diseases and conditions which include, but are not limited to, neurodegenerative disease states, behavioral abnormalities and pulmonary disorders. Similarly, polypeptides and antibodies directed to these polypeptides are useful in providing immunological probes for differential identification of the tissue(s) or cell type(s). For a number of disorders of the above tissues or cells, particularly of the immune, nervous, and pulmonary systems, expression of this gene at significantly higher or lower levels may be routinely detected in certain tissues and cell types (e.g., brain and other tissue of the nervous system, and lung, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the detection/treatment of neurodegenerative disease states and behavioral disorders such as Alzheimer's Disease, Parkinson's Disease, Huntington's Disease, schizophrenia, mania, dementia, paranoia, obsessive compulsive disorder and panic disorder. In addition it could be used in the detection and treatment of pulmonary disease states such as lung lymphoma or sarcoma formation, pulmonary edema and embolism, bronchitis and cystic fibrosis.

### FEATURES OF PROTEIN ENCODED BY GENE NO: 14

This gene is expressed exclusively in T-cells.

Therefore, polynucleotides and polypeptides of the invention are useful as reagents for differential identification of the tissue(s) or cell type(s) present in a biological sample and for diagnosis of diseases and conditions which include, but are not limited to, immune disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., T-cells and other cells and tissue of the immune system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment/detection of immune disorders such as arthritis, asthma, immune, deficiency diseases such as AIDS, and leukemia. Additionally, the expression in hematopoietic cells and tissues indicates that this protein may play a role in the proliferation, differentiation, and/or survival of hematopoietic cell lineages. Thus, this gene may be useful in the treatment of lymphoproliferative disorders, and in the maintenance and differentiation of various hematopoietic lineages from early hematopoietic stem and committed progenitor cells.

### FEATURES OF PROTEIN ENCODED BY GENE NO: 15

This gene is expressed primarily in T-cells.

Therefore, polynucleotides and polypeptides of the invention are useful as reagents for differential identification of the tissue(s) or cell type(s) present in a biological sample and for diagnosis of diseases and conditions which include, but are not limited to, immune disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., T-cells and other cells and tissue of the immune system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 125 as residues: Ala-46 to Asp-51.

The tissue distribution indicates that polynucleotides and polypeptides corresponding to this gene are useful for the diagnosis and treatment of immune disorders including: leukemias, lymphomas, auto-immunities, immunodeficiencies (e.g. AIDS), immuno-suppressive conditions (transplantation) and hematopoeitic disorders.

### FEATURES OF PROTEIN ENCODED BY GENE NO: 16

This gene is expressed primarily in endometrial tumors.

Therefore, polynucleotides and polypeptides of the invention are useful as reagents for differential identification of the tissue(s) or cell type(s) present in a biological sample and for diagnosis of diseases and conditions which include, but are not limited to, cancer, particularly endometrial. Similarly, polypeptides and antibodies directed to these polypeptides are useful in providing immunological probes for differential identification of the tissue(s) or cell type(s). For a number of disorders of the above tissues or cells, particularly of the female reproductive system, expression of this gene at significantly higher or lower levels may be routinely detected in certain tissues and cell types (e.g., endometrial cells and other reproductive cells or tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the treatment and diagnosis of ovarian and other endometrial cancers, as well as reproductive disfunction, prenatal disorders or fetal deficiencies.

### FEATURES OF PROTEIN ENCODED BY GENE NO: 17

This gene is expressed primarily in a variety of osteoclastic cells: osteoclastoma stromal cells, osteosarcoma, chondrosarcoma and stromal cell culture. To a lesser extent, it is also seen in a variety of fetal and embryonic cell and tissue types.

Therefore, polynucleotides and polypeptides of the invention are useful as reagents for differential identification of the tissue(s) or cell type(s) present in a biological sample and for diagnosis of diseases and conditions which include. but are not limited to, bone cancer. Similarly, polypeptides and antibodies directed to these polypeptides are useful in providing immunological probes for differential identification of the tissue(s) or cell type(s). For a number of disorders of the above tissues or cells, particularly of the skeletal and developmental systems, expression of this gene at significantly higher or lower levels may be routinely detected in certain tissues and cell types (e.g., bone cells, cartilage, and stomal cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 127 as residues: Gln-34 to Gln-41, Asn-76 to Lys-82, Ser-85 to Lys-91.

The tissue distribution indicates that polynucleotides and polypeptides corresponding to this gene are useful for treatment and detection of a variety disorders and conditions affecting bone and the skeletal system, including: osteoperosis, fracture, osteosarcoma, osteoclastoma, chondrosarcoma, ossification and osteonecrosis, arthritis, tendonitis, chrondomalacia and inflammation.

### FEATURES OF PROTEIN ENCODED BY GENE NO: 18

This gene is expressed primarily in smooth muscle.

Therefore, polynucleotides and polypeptides of the invention are useful as reagents for differential identification of the tissue(s) or cell type(s) present in a biological sample and for diagnosis of diseases and conditions which include, but are not limited to, cardiovascular disorders including lymphatic system disorders. Similarly, polypeptides and antibodies directed to these polypeptides are useful in providing immunological probes for differential identification of the tissue(s) or cell type(s). For a number of disorders of the above tissues or cells, particularly of the cardiovascular and lymphatic systems, expression of this gene at significantly higher or lower levels may be routinely detected in certain tissues and cell types (e.g., smooth muscles, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of conditions and pathologies of the cardiovascular system: heart disease, restenosis, atherosclerosis, stoke, angina, thrombosis, and wound healing.

### FEATURES OF PROTEIN ENCODED BY GENE NO: 19

The translation product of this gene shares sequence homology with 5'-nucleotidase (See Accession No. 2668557) as well as the gene for alpha-1 collagen type X (See Accession No. gblX67348lMMCOL10A ). One embodiment for this gene is the polypeptide fragments comprising the following amino acid sequence:
MAQHFSLAACDVVGFDLDHTLCRYNLPESAPLIYNSFAQFLVKEKGYDKELLN VTPEDWDFCCKGLALDLEDGNFLKLANNGTVLRASHGTKMMTPEVLAEAYG KKEWKHFLSDTGMACRSGKYYFYDNYFDLPGALLCARVVDYLTKLNNGQKT FDFWKDIVAAIQHNYKMSAFKENCGIYFPEIKRDPGRYLHSCPESVKKWLRQL KNAGKILLLITSSHSDYCRLLCEYILGNDFTDLFDIVITNALKPGFFSHLPSQRPF RTLENDEEQEALPSLDKPGWYSQGNAVHLYELLKKMTGKPEPKVVYFGDSMH SDIFPARHYSNWETVLILEELRGDEGTRSQRPEESEPLEKKGKYEGPKAKPLNT SSKKWGSFFIDSVLGLENTEDSLVYTWSCKRISTYSTIAIPSIEAIAELPLDYKFT RFSSSNSKTAGYYPNPPLVLSSDETLISK (SEQ ID NO:233); and/or TSSHSDYCRLLCEYILGNDFTDLFDIV (SEQ ID NO:234). An additional embodiment is the polynucleotide fragments encoding these polypeptide fragments. Additionally, another embodiment for this gene is the polynucleotide fragments comprising the following sequence:
CCTTAAAAGCTGACATTTTATAATTGTGTTGTATAGCAGCAACTATATCCTTC CAAAAATCAAATGTTTTTTGACCATTGTTCAGTT (SEQ ID NO:230); CCTTAAAAGCT GACATTTTATAATTGTGTTGTATAGCA (SEQ ID NO:231); and/or CTTCCAAAAA TCAAATGTTTTTTGACCATTGTTCAGTT (SEQ ID NO:232). An additional embodiment is the polypeptide fragments encoded by these polynucleotide fragments. This gene maps to chromosome 6, and therefore, may be used as a marker in linkage analysis for chromosome 6.

This gene is expressed primarily in prostate and smooth muscle.

Therefore, polynucleotides and polypeptides of the invention are useful as reagents for differential identification of the tissue(s) or cell type(s) present in a biological sample and for diagnosis of diseases and conditions which include, but are not limited to, prostate cancer and cardiovascular disorders. Similarly, polypeptides and antibodies directed to these polypeptides are useful in providing immunological probes for differential identification of the tissue(s) or cell type(s). For a number of disorders of the above tissues or cells, particularly of the prostate and cardiovascular system, expression of this gene at significantly higher or lower levels may be routinely detected in certain tissues and cell types (e.g., prostate, and smooth muscle, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the treatment and diagnosis of prostate cancer and other disorders. In addition the expression in smooth muscle would suggest a role for this gene product in the treatment and diagnosis of cardiovascular disorders such as hypertension, restenosis, atherosclerosis, stoke, angina, thrombosis, and other aspects of heart disease and respiration.

### FEATURES OF PROTEIN ENCODED BY GENE NO: 20

This gene is expressed primarily in endometrial tissue and to a lesser extent in synovium.

Therefore, polynucleotides and polypeptides of the invention are useful as reagents for differential identification of the tissue(s) or cell type(s) present in a biological sample and for diagnosis of diseases and conditions which include, but are not limited to, endometrial cancer and arthritis. Similarly, polypeptides and antibodies directed to these polypeptides are useful in providing immunological probes for differential identification of the tissue(s) or cell type(s). For a number of disorders of the above tissues or cells, particularly of the reproductive and skeletal systems, expression of this gene at significantly higher or lower levels may be routinely detected in certain tissues and cell types (e.g., endometrial tissue and other reproductive tissue, and synovial tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 130 as residues: Ser-19 to His-24, Pro-36 to Arg-43, Ala-61 to Gly-67, Pro-86 to Ala-95.

The tissue distribution indicates that polynucleotides and polypeptides corresponding to this gene are useful for the diagnosis and treatment of endometrial cancers, as well as reproductive and developmental disorders (fetal deficiencies and other pre-natal conditions). In addition the expression of this gene product in synovium would suggest a role in the detection and treatment of disorders and conditions affecting the skeletal system, in particular the connective tissues (e.g. arthritis, trauma, tendonitis, chrondomalacia and inflammation).

### FEATURES OF PROTEIN ENCODED BY GENE NO: 21

This gene maps to chromosome 6, and therefore, may be used as a marker in linkage analysis for chromosome 6.

This gene is expressed primarily in keratinocytes, fetal tissue (especially fetal brain) and leukocytic cell types and tissues (e.g. B-cell, macrophages, Jurkat T-Cell, T cell helper cells, spleen, thymus and lymphoma).

Therefore, polynucleotides and polypeptides of the invention are useful as reagents for differential identification of the tissue(s) or cell type(s) present in a biological sample and for diagnosis of diseases and conditions which include, but are not limited to, integument and immune systems, as well as developmental disorders. Similarly, polypeptides and antibodies directed to these polypeptides are useful in providing immunological probes for differential identification of the tissue(s) or cell type(s). For a number of disorders of the above tissues or cells, particularly of the skin, immune and central nervous systems, expression of this gene at significantly higher or lower levels may be routinely detected in certain tissues and cell types (e.g., keratinocytes, brain and other tissue of the nervous system, differentiating tissue, leukocytes and other cells and tissue of the immune system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of immune disorders including: leukemias, lymphomas, auto-immunities, immunodeficiencies (e.g. AIDS), immuno-suppressive conditions (transplantation) and hematopoeitic disorders. Expression in keratinocytes would suggest a role for the gene product in the diagnosis treatment of skin disorders such as cancers (melanomas), eczema, psoriasis, wound healing and grafts. In addition the expression in fetal brain might implicate this gene product in the detection and treatment of developmental and neurodegenerative diseases of the brain and nervous system: behavioral or nervous system disorders, such as depression, schizophrenia, Alzheimer's disease, Parkinson's disease, Huntington's disease, mania, dementia, paranoia, addictive behavior and sleep disorders.

### FEATURES OF PROTEIN ENCODED BY GENE NO: 22

Translation product of this gene shares significant homology with the conserved YME1 PROTEIN from *Saccharomyces cerevisiae,* which is a putative ATP-dependent protease thought to regulate the assembly of key respiratory chains within the mitochondria (See Accession No. P32795). Preferred polypeptide fragments comprise the following amino acid sequence:
MKTKNIPEAHQDAFKTGFAEGFLKAQALTQKTNDSLRRTRLILFVLLLFGIYGL LKNPFLSVRFRTTTGLDSAVDPVQMKNVTFEHVKGVEEAKQELQEVVEFLKNP QKFTILGGKLPKGILLVGPPGTGKTLLARAVAGEADVPFYYASGSEFDEMFVG VGASRIRNLFREAKANAPCVIFIDELDSVGGKRIESPMHPYSRQTINQLLAEMD GFKPNEGVIIIGATNFPEALDNALIRPGRFDMQVTVPRPDVKGRTEILKWYLNK IKFDXSVDPEIIARGTVGFSGAELENLVNQAALKAAVDGKEMVTMKELGVFQR QNSNGA (SEQ ID NO:235); MKTKNIPEAHQDAFKTGFAEG (SEQ ID NO:236); PVQMKNVTFEHVKGVEEAKQELQ (SEQ ID NO:237);
SRQTINQLLAEMDGFKPN EGVII (SEQ ID NO:238); and/or
FSGAELENLVNQAALKAAVDGKEM (SEQ ID NO:239). Also preferred are polynucleotide fragments encoding these polypeptide fragments.

This gene is expressed primarily in T-cells.

Therefore, polynucleotides and polypeptides of the invention are useful as reagents for differential identification of the tissue(s) or cell type(s) present in a biological sample and for diagnosis of diseases and conditions which include, but are not limited to, immune and hematopoeitic disorders. Similarly, polypeptides and antibodies directed to these polypeptides are useful in providing immunological probes for differential identification of the tissue(s) or cell type(s). For a number of disorders of the above tissues or cells, particularly of the immune and hematopoeitic systems, expression of this gene at significantly higher or lower levels may be routinely detected in certain tissues and cell types (e.g., T-cells and other cells and tissue of the immune system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of immune disorders including:leukemias, lymphomas, auto-immunities, immunodeficiencies (e.g. AIDS), immuno-suppressive conditions (transplantation) and hematopoeitic disorders. Furthermore, the homology of this gene indicates that it may play an important role in disorders affecting metabolism.

### FEATURES OF PROTEIN ENCODED BY GENE NO: 23

This gene is expressed primarily in human chronic synovitis.

Therefore, polynucleotides and polypeptides of the invention are useful as reagents for differential identification of the tissue(s) or cell type(s) present in a biological sample and for diagnosis of diseases and conditions which include, but are not limited to, synovial and other inflammatory disorders. Similarly, polypeptides and antibodies directed to these polypeptides are useful in providing immunological probes for differential identification of the tissue(s) or cell type(s). For a number of disorders of the above tissues or cells, particularly of the synovial tissue and immune system, expression of this gene at significantly higher or lower levels may be routinely detected in certain tissues and cell types (e.g., synovial tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein product of this gene are useful for study, diagnosis and treatment of inflammatory disorders such as chronic synovitis.

### FEATURES OF PROTEIN ENCODED BY GENE NO: 24

This gene is expressed primarily in pituitary, breast cancer, and bone marrow; and to a lesser extent in breast, prostate, uterine cancer and cerebellum.

Therefore, polynucleotides and polypeptides of the invention are useful as reagents for differential identification of the tissue(s) or cell type(s) present in a biological sample and for diagnosis of diseases and conditions which include, but are not limited to, endocrine, reproductive disorders and cancers. Similarly, polypeptides and antibodies directed to these polypeptides are useful in providing immunological probes for differential identification of the tissue(s) or cell type(s). For a number of disorders of the above tissues or cells, particularly of the reproductive, metabolic and endocrine systems, expression of this gene at significantly higher or lower levels may be routinely detected in certain tissues and cell types (e.g., pituitary, mammary tissue, bone marrow, prostate, reproductive tissue, uterus, and brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 134 as residues: Asp-32 to Gln-38, Lys-88 to Ile-97.

The tissue distribution indicates that the protein products of this gene are useful for the study, treatment and diagnosis of various endocrine disorders, reproductive diseases and disorders and cancers.

### FEATURES OF PROTEIN ENCODED BY GENE NO: 25

The translation product of this gene shares sequence homology with androgen withdrawal apoptosis protein in rat which is thought to be important in programmed cell death. Preferred polypeptides encoded by this gene comprise the following amino acid sequence:
LPMWQVTAFLDHNIVTAQTTWKGLWMSCVVQSTGHMQCKVYDSVLALSTEV QAARALTVSAVLLAFVALFVTLAGAQCTTCVAPGPAKARVALTGGVLYLFCGL LALVPLCWFANIVVREFYDPSVPVSQKYELGAXLYIGWAATALLMVGGCLLCC GAWVCTGRPDLSFPVKYSAPRRPTATGDYDKKNYV (SEQ ID NO:240). This polypeptide is expected to contain multiple transmembrane domains. The extracellular portion of the polypeptide is expected to comprise residues 1-51 of the foregoing amino acid sequence. Therefore, particularly preferred polypeptides encoded by this gene comprise residues 1-51 of the foregoing amino acid sequence. Polynucleotides encoding the foregoing polypeptides are also provided.

This gene is expressed primarily in human adult pulmonary and brain (striatum) tissue and to a lesser extent in thymus, synovium and testis.

Therefore, polynucleotides and polypeptides of the invention are useful as reagents for differential identification of the tissue(s) or cell type(s) present in a biological sample and for diagnosis of diseases and conditions which include, but are not limited to, reproductive, metabolic, and neurodegenerative disorders. Similarly, polypeptides and antibodies directed to these polypeptides are useful in providing immunological probes for differential identification of the tissue(s) or cell type(s). For a number of disorders of the above tissues or cells, particularly of the reproductive, nervous, respiratory and metabolic systems expression of this gene at significantly higher or lower levels may be routinely detected in certain tissues and cell types (e.g., thymus, synovial tissue, testis and other reproductive tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to androgen withdrawal apoptosis rat gene protein indicates that polynucleotides and polypeptides corresponding to this gene are useful for study, diagnosis and treatment of disorders in which the mechanism controlling programmed cell death is instrumental. This could include reproductive, neurodegenerative, and various metabolic disorders and diseases such as cancer.

### FEATURES OF PROTEIN ENCODED BY GENE NO: 26

The translation product of this gene shares homology with both ubiquitin and a G-protein coupled receptor TM3 consensus polypeptide (see Genbank accession Nos. gnIlPIDle331456 (AJ000657) and R50664, respectively). Preferred polypeptides encoded by this gene comprising the following amino acid sequence:
LHYFALSFVLILTEICLVSSGMGF (SEQ ID NO:241);
QLRNGIPPGRKALFCSGKPR LFTLGQGRTCA (SEQ ID NO:242); and/or
WSGLWVTTWNGSSGERTPSPWRRK RASQSAGRIASWMSF (SEQ ID NO:243).
An additional embodiment is polynucleotides encoding these polypeptides. This gene maps to chromosome 1, and therefore, may be used as a marker in linkage analysis for chromosome 1.

This gene is expressed primarily in activated T cells and to a lesser extent in CD34 depleted buffy coat.

Therefore, polynucleotides and polypeptides of the invention are useful as reagents for differential identification of the tissue(s) or cell type(s) present in a biological sample and for diagnosis of diseases and conditions which include, but are not limited to, immune and hemopoietic disorders. Similarly, polypeptides and antibodies directed to these polypeptides are useful in providing immunological probes for differential identification of the tissue(s) or cell type(s). For a number of disorders of the above tissues or cells, particularly of the hemopoietic and immune system, expression of this gene at significantly higher or lower levels may be routinely detected in certain tissues and cell types (e.g., T-cells and other blood cells and other cells and tissue of the immune system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 136 as residues: Thr-15 to His-21, Gly-30 to Lys-39, Arg-113 to Met-118, Arg-178 to Ala-187.

The tissue distribution indicates that polynucleotides and polypeptides corresponding to this gene are useful for the treatment and diagnosis of hematopoetic related disorders such as anemia, pancytopenia, leukopenia, thrombocytopenia or leukemia since stromal cells are important in the production of cells of hematopoietic lineages. The uses include bone marrow cell ex vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency etc. Furthermore, the homology to G-coupled proteins as well as to ubiquitin may implicate this gene as being important in regulation of gene expression and protein sorting - both of which are vital to development and would healing models. Therefore, the gene may provide utility in the diagnosis, prevention, and/or treatment of various developmental disorders.

### FEATURES OF PROTEIN ENCODED BY GENE NO: 27

This gene is expressed primarily in activated T cells and to a lesser extent in fetal kidney.

Therefore, polynucleotides and polypeptides of the invention are useful as reagents for differential identification of the tissue(s) or cell type(s) present in a biological sample and for diagnosis of diseases and conditions which include, but are not limited to, immune, developmental and metabolic diseases. Similarly, polypeptides and antibodies directed to these polypeptides are useful in providing immunological probes for differential identification of the tissue(s) or cell type(s). For a number of disorders of the above tissues or cells, particularly of the immune and metabolic systems, expression of this gene at significantly higher or lower levels may be routinely detected in certain tissues and cell types (e.g., T-cells and other cells and tissue of the immune system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the study and treatment of diseases and disorders of the immune, metabolic, and endocrine systems; such as renal diseases and T cell dysfunctions. Since the gene is expressed in cells of lymphoid origin, the natural gene product may be involved in immune functions. Therefore it may be also used as an agent for immunological disorders including arthritis, asthma, immune deficiency diseases such as AIDS, and leukemia.

### FEATURES OF PROTEIN ENCODED BY GENE NO: 28

The translation product of this gene shares sequence homology with Cystatin-related epididymal specific protein in mouse which is thought to be important in reproductive system function/regulation (See Genbank accession no.bbs1118813). Based on the structural similarity between these proteins, the translation product of this clone, hereinafter "Cystatin G", is expected to share biological activities with cystatin related proteins and other cysteine protease inhibitors. Such activities are known in the art and are described elsewhere herein. Preferred polypeptides encoded by this gene comprising the following amino acid sequence:
MPRCRWLSLILLTIPLALVARKDPKKNETGVLRKLKPVNASNANVKQCLWFA MQEYNKESEDKYVFLVVKTLQAQLQVTNLLEYLIDVEIARSDCRKPLSTNEICAI QENSKLKRKLSCSFLVGALPWNGEFTVMEKKCEDA (SEQ ID NO:246);
ARKDPKKNETGVLRKLKPVNASNANVKQCLWFAMQEYNKESEDKYVFLVVK TLQAQLQVTNLLEYLIDVEIARSDCRKPLSTNEICAIQENSKLKRKLSCSFLVGA LPWNGEFTVMEKKCEDA (SEQ ID NO:248);
CLWFAMQEYNKESEDKYVFLVVKTLQAQLQVTNLLEYLIDVEIARSDCRKPLST NEICAIQENSKLKRKLSCSFLVGALPWNGEFTVMEKKC (SEQ ID NO:247);
EYNKESEDKYVFLV (SEQ ID NO:244); and/or IDVEIARSDCRKPL (SEQ ID NO:245). An additional embodiment is the polynucleotide fragments encoding these polypeptide fragments. Preferred cystatin polypeptide fragments are shown to be active in the following assays: The methods used for active site titration of papain, titration of the molar enzyme inhibitory concentration in cystatin G preparations, and for determination of equilibrium constants for dissociation (Ki) of complexes between cystatin G and cysteine peptidases are described in detail in Hall et al., Biochem. J., 291:123-29 (1993) and Abrahamson, Methods Enzymol., 244:685-700 (1994), both of which are hereby incorporated herein by reference. The enzymes used for equilibrium assays are papain (EC 3.4.22.2; from Sigma, St Louis, MO) and cathepsin B (EC 3.4.22.1; from Calbiochem, La Jolla, CA). The fluorogenic substrate used was Z-Phe-Arg-NHMec (10 mM; from Bachem Feinchemikalien, Bubendorf, Switzerland) and the assay buffer was 100 mM Na-phosphate buffer (pH 6.5 and 6.0 for papain and cathepsin B, respectively), containing 1 mM dithiothreitol and 2 mM EDTA. Steady state velocities are measured and Ki values were calculated according to Henderson, Biochem J., 127:321-333 (1972), incorporated herein by reference. Corrections for substrate competition are made using Km values of 150 =B5M for cathepsins B (Barrett and Kirschke, Methods Enzymol.; 80:535-561 (1981) and 60 =B5M for papain (Hall et al., Biochem. J., 291:123-29 (1992)), both of which are hereby incorporated herein by reference.

This gene is expressed primarily in human testes.

Therefore, polynucleotides and polypeptides of the invention are useful as reagents for differential identification of the tissue(s) or cell type(s) present in a biological sample and for diagnosis of diseases and conditions which include, but are not limited to, reproductive disorders and cancer. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels may be routinely detected in certain tissues and cell types (e.g., testis and other reproductive tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 138 as residues: Arg-21 to Thr-29.

The tissue distribution and homology to cystatin-related epididymal specific protein-mouse indicates that polynucleotides and polypeptides corresponding to this gene are useful for study, diagnosis and treatment of reproductive diseases and disorders. Cysteine proteinase inhibitors of the cystatin superfamily are ubiquitous in the body and are generally tight-binding inhibitors of papain-like cysteine proteinases, such as cathepsins B, H, L, S, and K (for review, see Ref. 1). They should therefore serve a protective function to regulate the activities of such endogenous proteinases, which otherwise may cause uncontrolled proteolysis and tissue damage. Cysteine proteinase activity can normally not be measured in body fluids, but can been detected extracellularly in conditions like endotoxin-induced sepsis (2), metastasizing cancer (3), and at local inflammatory processes in rheumatoid arthritis (4), purulent bronchiectasis (5) and periodontitis (6), which indicates that a tight cystatin regulation is a necessity in the normal state. A deficiency state in which the levels of the intracellular cystatin, cystatin B, are lowered due to mutations has recently been shown to segregate with a form of progressive myoclonus epilepsy (7), which points to additional specialized functions of cystatins. Moreover, results showing that chicken cystatin inhibits polio virus replication (8), human cystatin C inhibits corona- and herpes simplex virus replication (9, 10), and human cystatin A inhibits rhabdovirus-induced apoptosis (11) in cell cultures indicates that cystatins play additional roles in the human defense system. The cystatins constitute a superfamily of evolutionary related proteins, all composed of at least one 100-120 residue domain with conserved sequence motifs (12). The previously well characterized single-domain human members of superfamily could be grouped in two protein families. The Family 1 members, cystatins (or stefins) A and B, contain approximately 100 amino acid residues, lack disulfide bridges, and are not synthesized as preproteins with signal peptides. The Family 2 cystatins (cystatins C, D, S, SN, and SA) are secreted proteins of approx. 120 amino acid residues (Mr 13,000-14,000) and have two characteristic intrachain disulfide bonds. Recently, we identified an additional human cystatin superfamily member by EST1 sequencing in epithelial cell derived cDNA libraries which we named cystatin E (13). The same cystatin was independently discovered by differential display experiments as a mRNA species downregulated in breast tumor tissue, but present in the surrounding epithelium and reported under the name cystatin M (14). Cystatin E/M is an atypical, secreted low-Mr cystatin in that it is a glycoprotein and just shows 30-35% sequence identity in alignments with the human Family 2 cystatins, which shows that additional cystatin families are yet to be identified (13). The cystatin E/M gene has been localized to chromosome 2 (15), whereas all human Family 2 cystatin genes are clustered on the short arm of chromosome 20 (16), which further stresses that cystatin E/M is just distantly related to the other secreted human low-Mr cystatins.

### FEATURES OF PROTEIN ENCODED BY GENE NO: 29

The translation product of this gene shares sequence homology with the leukocyte-associated Ig-like receptor-1, putative inhibitory receptor which is thought to be important in regulation of various physiological functions (See Accession No. gil2352941 (AF013249). Preferred polypeptides encoded by this gene comprise the following amino acid sequence:
DSPDTEPGSSAGPTQRPSDNSHNEHAPASQGLKAEHLYILIGVS (SEQ ID NO:249); HRQNQIKQGPPRSKDEEQKPQQRPDLAVDVLERTADKATVNGL PEKDRETDTSALAAGSSQEVTYAQLDHWALTQRTARAVSPQSTKPMAESITYAA VARH (SEQ ID NO:250); MSPHPTALLGLVLCLAQTIHTQEEDLPRPSISAEPGTVIPLGSHVTFVCRGPVGV QTFRLERESRSTYNDTEDVSQASPSESEARFRIDSVSEGNAGPYRCIYYKPPKW SEQSDY (SEQ ID NO:251); TALLGLVLCLAQTIHTQE (SEQ ID NO:252); LPRPSISAEPGTVI (SEQ ID NO:253); CRGPVGVQTFRLERE (SEQ ID NO:254);
and/or VLERTADKATVNGLPEKDRETDTSALAAGSS (SEQ ID NO:255). Additional embodiments of the invention include polynucleotides encoding these polypeptides.

This gene is expressed primarily in macrophages and T-cells and to a lesser extent in human fetal heart.

Therefore, polynucleotides and polypeptides of the invention are useful as reagents for differential identification of the tissue(s) or cell type(s) present in a biological sample and for diagnosis of diseases and conditions which include, but are not limited to, developmental, inflammatory, and immune disorders. Similarly, polypeptides and antibodies directed to these polypeptides are useful in providing immunological probes for differential identification of the tissue(s) or cell type(s). For a number of disorders of the above tissues or cells, particularly of the growth and inflammatory systems, expression of this gene at significantly higher or lower levels may be routinely detected in certain tissues and cell types (e.g., macrophages, T-cells and other cells and tissue of the immune system, heart, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 139 as residues: His-20 to Arg-28, Glu-61 to Val-74, Ser-78 to Ala-84, Lys-105 to Ser-117.

The tissue distribution and homology to putative inhibitory receptor indicates that polynucleotides and polypeptides corresponding to this gene are useful for the study, diagnosis and treatment of functional disorders of the developing fetal heart; including circulatory and vascular; and inflammatory disorders. In addition expression in macrophages and lymphocytes indicates a role in the treatment/detection of immune disorders including disorders such as arthritis, asthma, immune deficiency diseases such as AIDS, and leukemia.

### FEATURES OF PROTEIN ENCODED BY GENE NO: 30

The translation product of this gene shares sequence homology with erythroid cell specific transcription factor- murine which is thought to be important in normal physiological function of erythroid cells. In addition, the translation product of this gene also shares homology with the conserved 3-phosphoglycerate dehydrogenase gene which is essential component of metabolic biosynthetic pathways. Preferred polypeptides comprise the following amino acid sequence:
MNTPNGNSLSAAELTCGMIMCLARQIPQATASMKDGKWERKKFMGTELNGK TLGILGLGRIGREVATRMQSFGMKTIGYDPIISPEVSASFGVQQLPLEEIWPLCDF ITVHTPLLPSTTGLLNDNTFAQCKKGVRVVNCARGGIVDEGALLRALQSGQCA GAALDVFTEEPPRDRALVDHENVISCPHLGASTKEAQSRCGEEIAVQFVDMVK GKSLTGVVNAQALTSAFSPHTKPWIGLAEALGTLMRAWAGSPKGTIQVITQGT SLKNAGNCLSPAVIVGLLKEASKQADVNLVNAKLLVKEAGLNVTTSHSPAAPG EQGFGECLLAVALAGAPYQAVGLVQGTTPVLQGLNGAVFRPEVPLRRDLPLLL FRTQTSDPAMLPTMIGLLAEAGVRLLSYQTSLVSDGETWHVMGISSLLPSLEAW KQHVTEAFQFHF (SEQ ID NO:256); MAFANLRKVLISDSLDPCCRKILQ (SEQ ID NO:257); GGLQVVEKQNL SKEELIA (SEQ ID NO:258);
MCLARQIPQATASMKDGKWERKKFMGTEL (SEQ ID NO:259);
ALTSAFSPHTKPWIGLAEALGTLMRAWAG (SEQ ID NO:260); and/or
EVPLRRDLPLLLFRTQTSDPAMLPTMIGLLAEAGVR (SEQ ID NO:261). Also preferred are polynucleotide fragments encoding these polypeptides. This gene maps to chromosome 1, and therefore, may be used as a marker in linkage analysis for chromosome 1.

This gene is expressed primarily in IL-1 induced smooth muscle and fetal kidney and to a lesser extent in myeloid progenitor cell line and bone marrow.

Therefore, polynucleotides and polypeptides of the invention are useful as reagents for differential identification of the tissue(s) or cell type(s) present in a biological sample and for diagnosis of diseases and conditions which include, but are not limited to, immune, hemopoietic, and cardiovascular disorders. Similarly, polypeptides and antibodies directed to these polypeptides are useful in providing immunological probes for differential identification of the tissue(s) or cell type(s). For a number of disorders of the above tissues or cells, particularly of the hemopoietic and immune system, expression of this gene at significantly higher or lower levels may be routinely detected in certain tissues and cell types (e.g., smooth muscle, kidney, myeloid progenitor cells, bone, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 140 as residues: Met-1 to Asn-7, Met-33 to Lys-42, Asn-123 to Cys-130, Glu-169 to Asp-174, Ser-192 to Gly-201, Thr-266 to Asn-273, Pro-318 to Phe-323.

The tissue distribution and homology to erythroid cell specific murine transcription factor indicates that polynucleotides and polypeptides corresponding to this gene are useful for study, diagnosis and treatment of disorders and diseases involving the hemopoietic and immune systems; the maturation of progenitor cells; and the development of various smooth muscle tissues (heart, etc.). In addition, homology to a key biosynthetic protein implicates this the protein product of this gene as being important in metabolism. Therefore, the protein may show utility in the diagnosis, prevention, and/or treatment of metabolic disorders and conditions.

### FEATURES OF PROTEIN ENCODED BY GENE NO: 31

This gene is expressed primarily in human adult testes.

Therefore, polynucleotides and polypeptides of the invention are useful as reagents for differential identification of the tissue(s) or cell type(s) present in a biological sample and for diagnosis of diseases and conditions which include, but are not limited to, reproductive disorders, particularly of the male genitalia. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels may be routinely detected in certain tissues and cell types (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 141 as residues: Met-1 to Pro-8, Ser-45 to Thr-50.

The tissue distribution indicates that polynucleotides and polypeptides corresponding to this gene are useful for the study, diagnosis, treatment, and possibly prevention of various male reproductive disorders and diseases including male impotence, failed lebido and male secondary sex characteristics, infertility, and testicular cancer.

### FEATURES OF PROTEIN ENCODED BY GENE NO: 32

This gene is expressed primarily in human adult testis.

Therefore, polynucleotides and polypeptides of the invention are useful as reagents for differential identification of the tissue(s) or cell type(s) present in a biological sample and for diagnosis of diseases and conditions which include, but are not limited to, reproductive disorders and cancers of the male reproductive system. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels may be routinely detected in certain tissues and cell types (e.g., testis and other reproductive tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the study, diagnosis, treatment, and possibly prevention of various male reproductive disorders and diseases including male impotence, failed lebido and male secondary sex characteristics, infertility, and testicular cancer.

### FEATURES OF PROTEIN ENCODED BY GENE NO: 33

The translation product of this gene shares homology to the W09D10.1 protein of Caenorhabditis elegans. In addition, the gene also shares homology with the human protein hRIP, a protein known to be critical for HIV replication (See Accession Nos.gnIlPIDle1186472 and W12713). Preferred polypeptides encoded by this gene comprise the following amino acid sequence:
MDLLGLDAPVACSIANSKTSNTLEKDLDLLASVPSPSSSGSRKVVGSMPTAGSA GSVPENLNLFPEPGSKSEEIGKKQLSKDSILSLYGSQTXQMPTQAMFMAPAQM AYPTAYPSFPGVTPPNSIMGSMMPPPVGMVAQPGASGMVAPMAMPAGYMGG MQASMMGVPNGMMTTQQAGYMAGMAAMPQTVYGVQPAQQLQWNLTQMTQ QMAGMNFYGANGMMNYGQSMSGGNGQAANQTLSPQMWKFGTRFLANLLLE EDNKFCADCQSKGPRWASWNIGVFICIRCAXIHRNLGVHISRVKSVNLDQWTQ VQIQC (SEQ ID NO:267); MQXMGNGKANRLYEAYLPETFRRPQIDPAVEGFIR DXYE (SEQ ID NO:268); EEDNKFCADCQSKGPRWASWN (SEQ ID NO:263); GVFICIRCAXIHR NLGVHIS (SEQ ID NO:264); and/or SVNLDQWTQVQIQCMQX MGNGKA (SEQ ID NO:265). Polynucleotides encoding these polypeptides are also provided.

This gene is expressed primarily in lymphoid tumors.

Therefore, polynucleotides and polypeptides of the invention are useful as reagents for differential identification of the tissue(s) or cell type(s) present in a biological sample and for diagnosis of diseases and conditions which include, but are not limited to, immune and inflammatory disorders. Similarly, polypeptides and antibodies directed to these polypeptides are useful in providing immunological probes for differential identification of the tissue(s) or cell type(s). For a number of disorders of the above tissues or cells, particularly of the immune, hematopoietic and inflammatory, expression of this gene at significantly higher or lower levels may be routinely detected in certain tissues and cell types (e.g., lymphoid tissue and other tissue and cells of the immune system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 143 as residues: Cys-21 to Trp-28.

The tissue distribution indicates that the protein products of this gene are useful for study, diagnosis and treatment of various immune disorders and diseases, including self-recognition and rejection functions of the immune system, hematopoietic disorders, and inflammatory disorders. Homology to the W09D10.1 of C.elegans and the hRIP implicates this gene as playing a role as an essential receptor for host-viral interactions including, but not limited to retroviral infections such as AIDS.

### FEATURES OF PROTEIN ENCODED BY GENE NO: 34

The translation product of this gene shares homology to an Arabidopsis thaliana recombination and DNA-damage resistance/repair protein (See Accession No.gil166694). Preferred polypeptides encoded by this gene comprise the following amino acid sequence:
KYGKVGKCVIFEIPGAPDDEAVRIFLEFERVESAIKAVVDLNGRYFGGRVVKAC FYNLDKFRVLDLA (SEQ ID NO:269); KAVDLGRYFGGR (SEQ ID NO:270); and/or EAVRIFFRE (SEQ ID NO:271). Polynucleotides encoding these polypeptides are also provided.

This gene is expressed primarily in ovarian and other cancers.

Therefore, polynucleotides and polypeptides of the invention are useful as reagents for differential identification of the tissue(s) or cell type(s) present in a biological sample and for diagnosis of diseases and conditions which include, but are not limited to, cancer, particularly of the female reproductive system. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels may be routinely detected in certain tissues and cell types (e.g., ovaries and other reproductive tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 144 as residues: Thr-11 to Trp-19, Ala-40 to Gln-47, Lys-58 to Arg-66, Asp-98 to Lys-110, Arg-114 to Glu-121.

The tissue distribution in tumors of ovarian origins combined with the homology to a known DNA damage repair enzyme indicates that polynucleotides and polypeptides corresponding to this gene are useful for diagnosis and intervention of tumors. Protein, as well as, antibodies directed against the protein may show utility as a tumor marker and/or immunotherapy targets for the above listed tumors and tissues.

### FEATURES OF PROTEIN ENCODED BY GENE NO: 35

Translation product of this gene shares homology with human stomatin, intestinal surface antigens, as well as protein F30A10.5 of Caenorhabditis elegans (See Accession No.gnllPIDle276130). Preferred polypeptides encoded by this contig comprise the following amino acid sequence: RMGRFHRILEPGLNILIPVLDRIRYVQ SLKEIVINVPEQSAVTLDNVTLQIDGVLYLRIMDPYKASYGVEDPEYAVTQLAQT TMRSELGKLSLDKVFRERESLNASIVDAINQAADCWGIRCLRYEIKDIHVPPRV KESMQMQVEAERRKRATVLESEGTRESAINVAEGKKQAQILASEAEKAEQINQA AGEASAVLAKAKAKAEAIRILAAALTQHNGDAAASLTVAEQYVSAFSKLAKDS NTILLPSNPGDVTSMVAQAMGVYGALTKAPVPGTPDSLSSGSSRDVQGTDASL DEELDRVKMS (SEQ ID NO:272); ASYGVEDPEYAVTQLAQTT MRSELGK (SEQ ID NO:273); MQMQVEAERRKRATVLESEGTRESAIN (SEQ ID NO:274); LTVAEQYVSAFSKLAKDSNTILLPSN (SEQ ID NO:275), and/or LLGATAPLVSLVPEVAAAVGNAGARGAXHWGPFAEGLSTGFWPRSARASSGL PRNTVVLFVPQQEAWVVE (SEQ ID NO:276). Polynucleotides encoding these polypeptides are also provided.

This gene is expressed primarily in activated T-cells and to a lesser extent in other cell types.

Therefore, polynucleotides and polypeptides of the invention are useful as reagents for differential identification of the tissue(s) or cell type(s) present in a biological sample and for diagnosis of diseases arid conditions which include, but are not limited to, immune disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., T-cells and other cells and tissue of the immune system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 145 as residues: Arg-23 to Pro-33, Pro-184 to Ser-189, Ala-196 to Arg-201, Glu-208 to Ser-213, Glu-230 to Ile-237, Gly-326 to Leu-331, Gly-334 to Gln-340.

The tissue distribution indicates that the protein products of this gene are useful for the treatment and diagnosis of hematopoetic related disorders such as anemia, pancytopenia, leukopenia, thrombocytopenia or leukemia since stromal cells are important in the production of cells of hematopoietic lineages. The uses include bone marrow cell ex vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency etc. In addition, the homology to known intestinal antigens may suggest that the protein is important in the diagnosis, treatment, and/or prevention of gastrointestinal disorders.

### FEATURES OF PROTEIN ENCODED BY GENE NO: 36

Translation product of this gene has homology to a human estrogen receptor variant from human breast cancer. Preferred polypeptides encoded by this gene comprise the following amino acid sequence: RMWRNGTHFWECKIVQPLWK TVWWFPRKLSIELPENLAILIGTYFK (SEQ ID NO:277); and/or LKRHFPKEANK HVKRCSTSLDIREIQIKIKMRY (SEQ ID NO:278). Polynucleotides encoding these polypeptides are also provided.

This gene is expressed primarily in ulcerative colitis.

Therefore, polynucleotides and polypeptides of the invention are useful as reagents for differential identification of the tissue(s) or cell type(s) present in a biological sample and for diagnosis of diseases and conditions which include, but are not limited to, intestinal ulcers, inflammatory conditions and cancers, particular of the breast. Similarly, polypeptides and antibodies directed to these polypeptides are useful in providing immunological probes for differential identification of the tissue(s) or cell type(s). For a number of disorders of the above tissues or cells, particularly of the gastrointestinal system, expression of this gene at significantly higher or lower levels may be routinely detected in certain tissues and cell types (e.g., colon and other gastrointestinal tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution in colon and breast origins indicates that polynucleotides and polypeptides corresponding to this gene are useful for diagnosis and intervention of tumors or other conditions within these tissues, in addition to other tumors where expression has been indicated. Protein, as well as; antibodies directed against the protein may show utility as a tumor marker and/or immunotherapy targets for the above listed tumors and tissues.

### FEATURES OF PROTEIN ENCODED BY GENE NO: 37

This gene is expressed primarily in epithelial cells.

Therefore, polynucleotides and polypeptides of the invention are useful as reagents for differential identification of the tissue(s) or cell type(s) present in a biological sample and for diagnosis of diseases and conditions which include, but are not limited to, cancers and skin disorders, particularly melanoma. Similarly, polypeptides and antibodies directed to these polypeptides are useful in providing immunological probes for differential identification of the tissue(s) or cell type(s). For a number of disorders of the above tissues or cells, particularly of the skin and other epithelia, expression of this gene at significantly higher or lower levels may be routinely detected in certain tissues and cell types (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 147 as residues: Met-1 to Tyr-6.

The tissue distribution in epithelial tissue indicates that polynucleotides and polypeptides corresponding to this gene are useful for diagnosis and intervention of tumors of this tissue. Protein, as well as, antibodies directed against the protein may show utility as a tumor marker and/or immunotherapy targets for the above listed tissues.

### FEATURES OF PROTEIN ENCODED BY GENE NO: 38

This gene is expressed primarily in adult retina.

Therefore, polynucleotides and polypeptides of the invention are useful as reagents for differential identification of the tissue(s) or cell type(s) present in a biological sample and for diagnosis of diseases and conditions which include, but are not limited to, diseases of the eye. Similarly, polypeptides and antibodies directed to these polypeptides are useful in providing immunological probes for differential identification of the tissue(s) or cell type(s). For a number of disorders of the above tissues or cells, particularly of the eye, expression of this gene at significantly higher or lower levels may be routinely detected in certain tissues and cell types (e.g., epithelial cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 148 as residues: Cys-14 to Lys-21.

The tissue distribution indicates that polynucleotides and polypeptides corresponding to this gene are useful for treatment and diagnosis of disorders of the eye.

### FEATURES OF PROTEIN ENCODED BY GENE NO: 39

This gene is expressed primarily in bone marrow and fetal liver.

Therefore, polynucleotides and polypeptides of the invention are useful as reagents for differential identification of the tissue(s) or cell type(s) present in a biological sample and for diagnosis of diseases and conditions which include, but are not limited to, hemopoietic disorders. Similarly, polypeptides and antibodies directed to these polypeptides are useful in providing immunological probes for differential identification of the tissue(s) or cell type(s). For a number of disorders of the above tissues or cells, particularly of the hemopoietic system, expression of this gene at significantly higher or lower levels may be routinely detected in certain tissues and cell types (e.g., bone marrow and liver, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment and diagnosis of disorders of the hemopoietic system.

### FEATURES OF PROTEIN ENCODED BY GENE NO: 40

This gene is expressed primarily in lymph node, fetal liver and brain.

Therefore, polynucleotides and polypeptides of the invention are useful as reagents for differential identification of the tissue(s) or cell type(s) present in a biological sample and for diagnosis of diseases and conditions which include, but are not limited to, hemopoietic diseases and disorders of the CNS. Similarly, polypeptides and antibodies directed to these polypeptides are useful in providing immunological probes for differential identification of the tissue(s) or cell type(s). For a number of disorders of the above tissues or cells, particularly of the hemopoietic and CNS, expression of this gene at significantly higher or lower levels may be routinely detected in certain tissues and cell types (e.g., lymphoid tissue and other tissue of the immune system, liver, and brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein products of this gene are useful for the diagnosis and treatment of cancer and other proliferative disorders. Expression in embryonic tissue and other cellular sources marked by proliferating cells indicates that this protein may play a role in the regulation or cellular division. Additionally, the expression in hematopoietic cells and tissues indicates that this protein may play a role in the proliferation, differentiation, and/or survival of hematopoietic cell lineages. Thus, this gene may be useful in the treatment of lymphoproliferative disorders, and in the maintenance and differentiation of various hematopoietic lineages from early hematopoietic stem and committed progenitor cells. In addition, polynucleotides and polypeptides corresponding to this gene are useful for the detection/treatment of neurodegenerative disease states and behavioral disorders such as Alzheimer's Disease, Parkinson's Disease, Huntington's Disease, schizophrenia, mania, dementia, paranoia, obsessive compulsive disorder, panic disorder, and autism. In addition, the gene or gene product may also play a role in the treatment and/or detection of developmental disorders associated with the developing embryo, sexually-linked disorders, or disorders of the cardiovascular system.

### FEATURES OF PROTEIN ENCODED BY GENE NO: 41

The translation product of this gene shares sequence homology with fibropellin and epidermal growth factors which are thought to be important in growth and regeneration of epidermal cells (See Genbank Accession Nos. W11719 and gil310660). Preferred polypeptides comprise the following amino acid sequence:
GTRPGESHANDLECSGKGKCTTKPSEATFSCTCEEQYVGTFCEEYDACQRKPC QNNASCIDANEKQDGSNFTCVCLPGYTGELCQSKIDYCILDPCRNGATCISSLS GFTCQCPEGYFGSACEEKVDPCASSPCQNNGTCYVDGVHFTCNCSPGFTGPTC AQLIDFCALSPCAHGTCRSVGTSYKCLCDPGYHGLYCEEEYNECLSAPCLNAA TCRDLVNGYECVCLAEYKGTHCELYKDPCANVSCLNGATCDSDGLNGTCICA PGFTGEECDIDINECDSNPCHHGGSCLDQPNGYNCHCPHGWVGANCEIHLQW KSGHMAESLTN (SEQ ID NO:279); GKCTTKPSEATFSCTCEEQYVGTFC (SEQ ID NO:280); CAHG TCRSVGTSYKCLCDPGYH (SEQ ID NO:281); and/or
CANVSCLNGATCDSDGLNG TCICAPGFTGEECD (SEQ ID NO:282).
Polynucleotides encoding these polypeptides are also provided.

This gene is expressed primarily in brain and kidney and to a lesser extent in several other tissues and organs.

Therefore, polynucleotides and polypeptides of the invention are useful as reagents for differential identification of the tissue(s) or cell type(s) present in a biological sample and for diagnosis of diseases and conditions which include, but are not limited to, disorders of the neural and renal systems, particularly growth disorders such as cancer. Similarly, polypeptides and antibodies directed to these polypeptides are useful in providing immunological probes for differential identification of the tissue(s) or cell type(s). For a number of disorders of the above tissues or cells, particularly of the neural and renal systems, expression of this gene at significantly higher or lower levels may be routinely detected in certain tissues and cell types (e.g., brain and other tissue of the nervous system, and kidney, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to epidermal growth factor indicates that polynucleotides and polypeptides corresponding to this gene are useful for diagnosis and treatment of growth disorders especially in the neural and renal systems. In addition, polynucleotides and polypeptides corresponding to this gene are useful for the detection/treatment of neurodegenerative disease states and behavioral disorders such as Alzheimer's Disease, Parkinson's Disease, Huntington's Disease, schizophrenia, mania, dementia, paranoia, obsessive compulsive disorder, panic disorder, and autism. In addition, the gene or gene product may also play a role in the treatment and/or detection of developmental disorders associated with the developing embryo, sexually-linked disorders, or disorders of the cardiovascular system

### FEATURES OF PROTEIN ENCODED BY GENE NO: 42

This gene is expressed primarily in brain, kidney and stromal cells.

Therefore, polynucleotides and polypeptides of the invention are useful as reagents for differential identification of the tissue(s) or cell type(s) present in a biological sample and for diagnosis of diseases and conditions which include, but are not limited to, disorders of the CNS and hemopoietic system. Similarly, polypeptides and antibodies directed to these polypeptides are useful in providing immunological probes for differential identification of the tissue(s) or cell type(s). For a number of disorders of the above tissues or cells, particularly of the hemopoietic, renal and central nervous system, expression of this gene at significantly higher or lower levels may be routinely detected in certain tissues and cell types (e.g., brain and other tissue of the nervous system, kidney, and stromal cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 152 as residues: Lys-71 to Trp-76, Glu-99 to Gly-108, Arg-142 to Ser-149.

The tissue distribution indicates that polynucleotides and polypeptides corresponding to this gene are useful for the detection/treatment of neurodegenerative disease states and behavioral disorders such as Alzheimer's Disease, Parkinson's Disease, Huntington's Disease, schizophrenia, mania, dementia, paranoia, obsessive compulsive disorder, panic disorder, and autism. In addition, the gene or gene product may also play a role in the treatment and/or detection of developmental disorders associated with the developing embryo, sexually-linked disorders, or disorders of the cardiovascular system. In addition, polynucleotides and polypeptides corresponding to this gene are useful for the treatment and diagnosis of hematopoetic related disorders such as anemia, pancytopenia, leukopenia, thrombocytopenia or leukemia since stromal cells are important in the production of cells of hematopoietic lineages. The uses include bone marrow cell ex vivo culture, bone marrow transplantation, bone marrow reconstitution, radiotherapy or chemotherapy of neoplasia. The gene product is thought to be involved in lymphopoiesis, therefore, it can be used in immune disorders to modulate infection, inflammation, allergy, immunodeficiency, etc.

### FEATURES OF PROTEIN ENCODED BY GENE NO: 43

The preferred polypeptide encoded by this gene comprise the following amino acid sequence: MAQNLKDLAGRLPAGPRGMGTALKLLLGAGAVAYGVRESVFT VEGGHRAIFFNRIGGVQQDTILAEGLHFRIPWFQYPIIYDIRARPRKISSPTGSKD LQMVNISLRVLSRPNAQELPSMYQRLGLDYEERVLPSIVNEVLKSVVAKFNASQ LTTQRAQVSLLIRRELTERAKDFSLILDDVAITELSFSREYTAAVEAKQVAQQEAQ RAQFLVEKAKQEQRQKIVQAEGEAEAAKMLGEALSKNPGYIKLRKIRAAQNIS KTIATSQNRIYLTADNLVLNLQDESFTRGSDSLIKGKK (SEQ ID NO:283). The gene product above share sequence similarity with prohibitin. Thus, these polypeptides are expected to share biological activities with prohibitin. Such activities are known in the art and discussed elsewhere herein.

This gene is expressed primarily in fetal brain.

Therefore, polynucleotides and polypeptides of the invention are useful as reagents for differential identification of the tissue(s) or cell type(s) present in a biological sample and for diagnosis of diseases and conditions which include, but are not limited to, neural diseases. Similarly, polypeptides and antibodies directed to these polypeptides are useful in providing immunological probes for differential identification of the tissue(s) or cell type(s). For a number of disorders of the above tissues or cells, particularly of the nervous system, expression of this gene at significantly higher or lower levels may be routinely detected in certain tissues and cell types (e.g., brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 153 as residues: Ala-85 to Ser-91, Pro-93 to Asp-98, Glu-167 to Lys-173, Gln-205 to Ala-210.

The tissue distribution and structural similarity to prohibitin indicates that the protein products of this gene are useful for the detection/treatment of neurodegenerative disease states and behavioral disorders such as Alzheimer's Disease, Parkinson's Disease, Huntington's Disease, schizophrenia, mania, dementia, paranoia, obsessive compulsive disorder, panic disorder, and autism. In addition, the gene or gene product may also play a role in the treatment and/or detection of developmental disorders associated with the developing embryo, sexually-linked disorders, and/or disorders of the cardiovascular system.

### FEATURES OF PROTEIN ENCODED BY GENE NO: 44

The translation product of this gene shares sequence homology with the F44G4.1 gene of the c. elegans genome which has no known function (See Accession No.gnIlPIDle236516). The translation product of this gene also shares sequence homology with the human torsionA and torsionB gene products, a gene candidate for the Torsion Dystonia disease locus (See Accession Nos gil2358279 (AF007871) and gil2358281 (AF007872)). One embodiment for this gene is the polypeptide fragments comprising the following amino acid sequence: KALALSFHGWSGTGKNFV (SEQ ID NO:284); NLIDYFIPFLPLEYRHVRLCAR (SEQ ID NO:285); NLIDYFIPFLPL - EYRHVRLC (SEQ ID NO:286); CHQTLFIFDEAEKLHPGLLEVLGPHL (SEQ ID NO:287); and/or PEKALALSFHGWSGTGKNFVA (SEQ ID NO:288). An additional embodiment is the polynucleotide fragments encoding these polypeptide fragments.

This gene is expressed primarily in tonsils.

Therefore, polynucleotides and polypeptides of the invention are useful as reagents for differential identification of the tissue(s) or cell type(s) present in a biological sample and for diagnosis of diseases and conditions which include, but are not limited to, such as tonsilitis or adnoiditis. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., tonsils, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to F44G4.1 gene of the c. elegans genome indicates that polynucleotides and polypeptides corresponding to this gene are useful for the treatment and detection of conditions affecting the tonsils. The tonsils have not been thoroughly studied and the actually function of this organ is not known, but this gene could be used in determining what may trigger tonsillitis. Especially in children, where the tonsils seem to be most active. Furthermore, due to the homology of this gene, it may display potential utility in the detection, diagnosis, and/or treatment for Torsion Dystonia disease.

### FEATURES OF PROTEIN ENCODED BY GENE NO: 45

Has exact sequence homology on the nucleotide level as Human HepG2 3' region cDNA, but the function of this gene is not known.

This gene is expressed primarily in osteoclastoma stromal cells and to a lesser extent in T-cells.

Therefore, polynucleotides and polypeptides of the invention are useful as reagents for differential identification of the tissue(s) or cell type(s) present in a biological sample and for diagnosis of diseases and conditions which include, but are not limited to, leukemia and bone disorders. Similarly, polypeptides and antibodies directed to these polypeptides are useful in providing immunological probes for differential identification of the tissue(s) or cell type(s). For a number of disorders of the above tissues or cells, particularly of the haemolymphoid system, expression of this gene at significantly higher or lower levels may be routinely detected in certain tissues and cell types (e.g., bone tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of diseases such as leukemia.

### FEATURES OF PROTEIN ENCODED BY GENE NO: 46

This gene is expressed primarily in activated monocytes.

Therefore, polynucleotides and polypeptides of the invention are useful as reagents for differential identification of the tissue(s) or cell type(s) present in a biological sample and for diagnosis of diseases and conditions which include, but are not limited to, immune disorders, including leukemia and allergies. Similarly, polypeptides and antibodies directed to these polypeptides are useful in providing immunological probes for differential identification of the tissue(s) or cell type(s). For a number of disorders of the above tissues or cells, particularly of the lymphoid system, expression of this gene at significantly higher or lower levels may be routinely detected in certain tissues and cell types (e.g., hemopoietic cells, bone marrow, and spleen, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 156 as residues: Met-1 to Gly-7.

The tissue distribution indicates that polynucleotides and polypeptides corresponding to this gene are useful for the treatment in tissue repair and modeling since monocytes engage the synthesis and secretion of many cytokines which are soluble proteins that regulate highly diverse aspects of cellular biology. Monocytes are also important in the fact that their expression of Major Histocompatibility Factor II (MHCII) enable them to select and stimulate the appropriate lymphocytes to combat specific antigens in the blood. Since the gene is expressed in cells of lymphoid origin, the natural gene product may be involved in immune functions. Therefore it may be also used as an agent for immunological disorders including arthritis, asthma, immune deficiency diseases such as AIDS, and leukemia.

### FEATURES OF PROTEIN ENCODED BY GENE NO: 47

Translation product of this gene has homology to the Na+/H+-exchanging protein: Na+/H+ antiporter in Methanobacterium thermoautotrophicum as well as the Na+/H+ antiporter cdu2' in Clostridium difficile (See Accession Nos. gil2621849 (AE000854) and pirlJ53431JC5343, respectively). Thus, it is likely that this gene has similar Na+/H+ antiporter activity. One embodiment for this gene are polypeptide fragments comprising the following amino acid sequence:
NLKEKIFISFAWLPKATVQAAIG (SEQ ID NO:289) and/or WLPKATVQAAIGSVALD (SEQ ID NO:290). An additional embodiment is the polynucleotide fragments encoding these polypeptide fragments.

This gene is expressed primarily in osteoclastoma cells.

Therefore, polynucleotides and polypeptides of the invention are useful as reagents for differential identification of the tissue(s) or cell type(s) present in a biological sample and for diagnosis of diseases and conditions which include, but are not limited to, osteoporosis, leukemia. Similarly, polypeptides and antibodies directed to these polypeptides are useful in providing immunological probes for differential identification of the tissue(s) or cell type(s). For a number of disorders of the above tissues or cells, particularly of the lymphoid and skeletal systems, expression of this gene at significantly higher or lower levels may be routinely detected in certain tissues and cell types (e.g., bone cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 157 as residues: His-35 to Gln-43.

The tissue distribution predominantly in osteoclastoma cells (the site of hematopoeisis) indicates that polynucleotides and polypeptides corresponding to this gene are useful for the diagnosis and treatment of bone related diseases including osteporosis, osteopetrosis and leukemia. Furthermore, its homology to known transporter proteins may suggest the protein is useful in the diagnosis, treatment, and prevention of various developmental and metabolic disorders, particularly those based upon ion and proton transport.

### FEATURES OF PROTEIN ENCODED BY GENE NO: 48

This gene is expressed primarily in amygdala and to a lesser extent in amniotic cells.

Therefore, polynucleotides and polypeptides of the invention are useful as reagents for differential identification of the tissue(s) or cell type(s) present in a biological sample and for diagnosis of diseases and conditions which include, but are not limited to, depression and other emotional behavioral problems. Similarly, polypeptides and antibodies directed to these polypeptides are useful in providing immunological probes for differential identification of the tissue(s) or cell type(s). For a number of disorders of the above tissues or cells, particularly of the nervous system, expression of this gene at significantly higher or lower levels may be routinely detected in certain tissues and cell types (e.g., brain and tissues of the nervous system, and tissues of the reproductive system, and cancerous and wounded tissues) or bodily fluids (e.g., serum, plasma, urine, synovial fluid, spinal fluid or amniotic fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of mental problems associated with emotional behavior and neurodegenerative states such as Alzheimer's Disease, Parkinson's Disease, Huntington's Disease, schizophrenia, mania, dementia, paranoia, obsessive compulsive disorder and panic disorders, and depression. The amygdala processes sensory information and relays this to other areas of the brain including the endocrine and autonomic domains of the hypothalamus and the brain stem. In addition, expression of this protein in amniotic cells suggests that this protein would be useful in the diagnosis, prevention, and/or treatment of various developmental and/or reproductive system disorders.

### FEATURES OF PROTEIN ENCODED BY GENE NO: 49

This gene is expressed primarily in stromal cells.

Therefore, polynucleotides and polypeptides of the invention are useful as reagents for differential identification of the tissue(s) or cell type(s) present in a biological sample and for diagnosis of diseases and conditions which include, but are not limited to, leukemia and other cancers and disorders deriving from hematopoietic cells. Similarly, polypeptides and antibodies directed to these polypeptides are useful in providing immunological probes for differential identification of the tissue(s) or cell type(s). For a number of disorders of the above tissues or cells, particularly of the lymphoid system, expression of this gene at significantly higher or lower levels may be routinely detected in certain tissues and cell types (e.g., haematopoietic tissues, and cancerous and wounded tissues) or bodily fluids (e.g., serum, plasma, urine, synovial fluid, spinal fluid, or lymph fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the treatment and diagnosis of hematopoetic related disorders such as anemia, pancytopenia, leukopenia, thrombocytopenia or leukemia since stromal cells are important in the production of cells of hematopoietic lineages. The uses include bone marrow cell ex vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency etc.

### FEATURES OF PROTEIN ENCODED BY GENE NO: 50

This gene maps to chromosome 9, and therefore, may be used as a marker in linkage analysis for chromosome 9.

This gene is expressed primarily in tumors, particularly skin and adrenal gland tumors, and to a lesser extent in bone marrow stromal cells and activated T cells.

Therefore, polynucleotides and polypeptides of the invention are useful as reagents for differential identification of the tissue(s) or cell type(s) present in a biological sample and for diagnosis of diseases and conditions which include, but are not limited to, cancer, hematopoietic and immune disorders. Similarly, polypeptides and antibodies directed to these polypeptides are useful in providing immunological probes for differential identification of the tissue(s) or cell type(s). For a number of disorders of the above tissues or cells, particularly of the skin, adrenal gland, and immune system, expression of this gene at significantly higher or lower levels may be routinely detected in certain tissues and cell types (e.g., endocrine glands, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 160 as residues: Glu-13 to Arg-22, Ser-58 to Trp-63.

The tissue distribution indicates that polynucleotides and polypeptides corresponding to this gene are useful for the detection and treatment of cancer. Elevated levels of expression of this gene in a variety of tumors suggest that it may play a role in cell proliferation, the induction of angiogenesis, destruction of the basal lamina, or a variety of other physiological processes that support the growth and development of tumors and cancer. Alternatively, its expression in the hematopoietic compartment, particularly in the bone marrow stroma and by activated T cells suggest that it may represent a soluble factor capable of influencing a variety of hematopoietic lineages. Therefore, this gene product may have commercial utility in the expansion of stem cells and committed progenitors of various blood lineages, and in the differentiation and/or proliferation of blood cells.

### FEATURES OF PROTEIN ENCODED BY GENE NO: 51

This gene is expressed primarily in benign human breast tissue.

Therefore, polynucleotides and polypeptides of the invention are useful as reagents for differential identification of the tissue(s) or cell type(s) present in a biological sample and for diagnosis of diseases and conditions which include, but are not limited to, breast cancer and other female reproductive disorders. Similarly, polypeptides and antibodies directed to these polypeptides are useful in providing immunological probes for differential identification of the tissue(s) or cell type(s). For a number of disorders of the above tissues or cells, particularly of the breast and reproductive tissues, expression of this gene at significantly higher or lower levels may be routinely detected in certain tissues and cell types (e.g., breast tissue, secretory/ductile organs, and cancerous and wounded tissues) or bodily fluids (e.g., serum, plasma, urine, synovial fluid, spinal fluid or milk) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the treatment and/or diagnosis of breast cancer. Alternately, this protein may play an important role in lactation or represent a critical component secreted into the milk, which may have an important function in the immunoprotection, health, and/or nourishment of the infant upon breastfeeding. Protein; as well as, antibodies directed against the protein may show utility as a tumor marker and/or immunotherapy targets for the above listed tumors and tissues

### FEATURES OF PROTEIN ENCODED BY GENE NO: 52

Translation product of this gene has homology with the conserved human ring finger proteins (See Accession No.gnIlPIDle351238 (AJ001019)) which are thought to be important in facilitating and regulating signal transduction pathways in eukaryotic cells. One embodiment for this gene is the polypeptide fragments comprising the following amino acid sequence: HDRTMQDIVYKLVPGLQE (SEQ ID NO:291) and/or FASHDRTM QDIVYKLVPGLQEGE (SEQ ID NO:292). An additional embodiment is the polynucleotide fragments encoding these polypeptide fragments.

This gene is expressed primarily in adult whole brain.

Therefore, polynucleotides and polypeptides of the invention are useful as reagents for differential identification of the tissue(s) or cell type(s) present in a biological sample and for diagnosis of diseases and conditions which include; but are not limited to, neurodegenerative disorders; Schizophrenia; Alzheimer's; tumors of a brain or neuronal cell origin. Similarly, polypeptides and antibodies directed to these polypeptides are useful in providing immunological probes for differential identification of the tissue(s) or cell type(s). For a number of disorders of the above tissues or cells, particularly of the CNS and/or peripheral nervous system, expression of this gene at significantly higher or lower levels may be routinely detected in certain tissues and cell types (e.g., brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 162 as residues: Phe-39 to Gly-44.

The tissue distribution indicates that polynucleotides and polypeptides corresponding to this gene are useful for the detection/treatment of neurodegenerative disease states and behavioral disorders such as Alzheimer's Disease, Parkinson's Disease, Huntington's Disease, schizophrenia, mania, dementia, paranoia, obsessive compulsive disorder and panic disorder. In addition, considering the homology to the conserved ring finger proteins may suggest that the gene or gene product may also play a role in the treatment and/or detection of developmental disorders associated with the developing embryo.

### FEATURES OF PROTEIN ENCODED BY GENE NO: 53

Translation product of this gene shares homology with the human conserved Lst-1 gene product, a member of the TNF family of proteins (See Accession No.gil1127546). One embodiment for this gene is the polypeptide fragments comprising the following amino acid sequence: LVLSLGAWGWPSTCLWW (SEQ ID NO:293). An additional embodiment is the polynucleotide fragments encoding these polypeptide fragments.

This gene is expressed primarily in human 6-week old embryo.

Therefore, polynucleotides and polypeptides of the invention are useful as reagents for differential identification of the tissue(s) or cell type(s) present in a biological sample and for diagnosis of diseases and conditions which include, but are not limited to, abnormal cell proliferation; defects in terminal tissue differentiation. Similarly, polypeptides and antibodies directed to these polypeptides are useful in providing immunological probes for differential identification of the tissue(s) or cell type(s). For a number of disorders of the above tissues or cells, particularly of the embryo, expression of this gene at significantly higher or lower levels may be routinely detected in certain tissues and cell types (e.g., proliferating and differentiating tissues, and cancerous and wounded tissues) or bodily fluids (e.g., serum, plasma, urine, synovial fluid, spinal fluid or amniotic fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the treatment and/or diagnosis of fetal disorders. Alternately, expression within embryonic tissues may reflect a role for this protein in proliferating cells. In such an event, this gene product may be useful in the treatment or diagnosis of abnormal cell proliferation, such as that involved in cancer. Similarly, embryonic development also involves decisions involving cell differentiation and/or apoptosis involved in pattern formation. Thus, this protein may also be involved in apoptosis or tissue differentiation, and could again be useful in cancer therapy.

### FEATURES OF PROTEIN ENCODED BY GENE NO: 54

This gene is expressed primarily in human epithelioid sarcoma.

Therefore, polynucleotides and polypeptides of the invention are useful as reagents for differential identification of the tissue(s) or cell type(s) present in a biological sample and for diagnosis of diseases and conditions which include, but are not limited to, epithelial sarcoma; tumors of an epithelial cell origin including the underlying integument. Similarly, polypeptides and antibodies directed to these polypeptides are useful in providing immunological probes for differential identification of the tissue(s) or cell type(s). For a number of disorders of the above tissues or cells, particularly of the skin and epithelial tissue layers, expression of this gene at significantly higher or lower levels may be routinely detected in certain tissues and cell types (e.g., epithelial cells and tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 164 as residues: Met-1 to Tyr-6, Thr-24 to Cys-36.

The tissue distribution indicates that polynucleotides and polypeptides corresponding to this gene are useful for the treatment and/or diagnosis of epithelial cancer. This gene product displays enhanced expression in epithelial cell sarcoma, and thus may be involved in cell proliferation, apoptosis, or in the control of angiogenesis.

### FEATURES OF PROTEIN ENCODED BY GENE NO: 55

This gene is expressed primarily in endometrial tumors.

Therefore, polynucleotides and polypeptides of the invention are useful as reagents for differential identification of the tissue(s) or cell type(s) present in a biological sample and for diagnosis of diseases and conditions which include, but are not limited to, endometrial cancer including other cancers of the female reproductive system. Similarly, polypeptides and antibodies directed to these polypeptides are useful in providing immunological probes for differential identification of the tissue(s) or cell type(s). For a number of disorders of the above tissues or cells, particularly of the endometrium and reproductive system, expression of this gene at significantly higher or lower levels may be routinely detected in certain tissues and cell types (e.g., endrometrial tissue as well as other tissues of the female reproductive system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of cancers, particularly those of the endometrium and other reproductive organs. Protein, as well as, antibodies directed against the protein may show utility as a tumor marker and/or immunotherapy targets for the above listed tumors and tissues

### FEATURES OF PROTEIN ENCODED BY GENE NO: 56

This gene is expressed primarily in metastatic melanoma and to a lesser extent in fetal lung.

Therefore, polynucleotides and polypeptides of the invention are useful as reagents for differential identification of the tissue(s) or cell type(s) present in a biological sample and for diagnosis of diseases and conditions which include, but are not limited to, cancer of the integument system, particularly melanoma, as well as within the developing pulmonary system. Similarly, polypeptides and antibodies directed to these polypeptides are useful in providing immunological probes for differential identification of the tissue(s) or cell type(s). For a number of disorders of the above tissues or cells, particularly of the skin, expression of this gene at significantly higher or lower levels may be routinely detected in certain tissues and cell types (e.g., cells capable of forming melanin, epithelia, and lung, and cancerous and wounded tissues) or bodily fluids (e.g., serum, plasma, urine, synovial fluid, spinal fluid, or pulmonary surfactant) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 166 as residues: Asp-20 to Lys-25.

The tissue distribution indicates that polynucleotides and polypeptides corresponding to this gene are useful for diagnosis and treatment of cancer, particularly melanoma and more particularly, metastasizing melanomas. In addition, the tissue distribution also indicates that polynucleotides and polypeptides corresponding to this gene are useful for the diagnosis and treatment of cancer and other proliferative disorders. Expression in embryonic tissue and other cellular sources marked by proliferating cells indicates that this protein may play a role in the regulation or cellular division.

### FEATURES OF PROTEIN ENCODED BY GENE NO: 57

This gene is expressed primarily in T-cell lymphoma.

Therefore, polynucleotides and polypeptides of the invention are useful as reagents for differential identification of the tissue(s) or cell type(s) present in a biological sample and for diagnosis of diseases and conditions which include, but are not limited to, lymphomas and other immune derived cancers. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., T-cells and other cells and tissue of the immune system, and cancerous and wounded tissues) or bodily fluids (e.g., serum, plasma, urine, . synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 167 as residues: Met-1 to Asn-7.

The tissue distribution indicates that polynucleotides and polypeptides corresponding to this gene are useful for diagnosis and treatment of lymomas, particularly T cell lymphomas, and other cancers. In addition, the tissue distribution indicates that polynucleotides and polypeptides corresponding to this gene are useful for the diagnosis and treatment of cancer and other proliferative disorders. Additionally, the expression in hematopoietic cells and tissues indicates that this protein may play a role in the proliferation, differentiation, and/or survival of hematopoietic cell lineages. Thus, this gene may be useful in the treatment of lymphoproliferative disorders, and in the maintenance and differentiation of various hematopoietic lineages from early hematopoietic stem and committed progenitor cells.

### FEATURES OF PROTEIN ENCODED BY GENE NO: 58

This gene maps to chromosome 7, and therefore is useful in linkage analysis as a marker for chromosome 7.

This gene is expressed primarily in brain and to a lesser extent in spinal cord.

Therefore, polynucleotides and polypeptides of the invention are useful as reagents for differential identification of the tissue(s) or cell type(s) present in a biological sample and for diagnosis of diseases and conditions which include, but are not limited to, CNS and PNS diseases and disorders. Similarly, polypeptides and antibodies directed to these polypeptides are useful in providing immunological probes for differential identification of the tissue(s) or cell type(s). For a number of disorders of the above tissues or cells, particularly of the nervous system, expression of this gene at significantly higher or lower levels may be routinely detected in certain tissues and cell types (e.g., brain, spinal cord and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 168 as residues: Tyr-14 to Ala-30.

The tissue distribution indicates that polynucleotides and polypeptides corresponding to this gene are useful for the detection/treatment of neurodegenerative disease states and behavioral disorders such as Alzheimer's Disease, Parkinson's Disease, Huntington's Disease, schizophrenia, mania, dementia, paranoia, obsessive compulsive disorder, panic disorder, and autism.

### FEATURES OF PROTEIN ENCODED BY GENE NO: 59

Translation product of this gene shares homology to the conserved *C. elegans* protein FER-1 (See Accession No.gil1373333). One embodiment for this gene is the polypeptide fragments comprising the following amino acid sequence:
QGKLQMWVDVFPKSL (SEQ ID NO:294); PPFNITPRKAKKYYLR (SEQ ID NO:295); KTDVHYRSLDGEGNFNWRF (SEQ ID NO:296); and/or PRLIIQIWDNDKFSLDDY LGFLELDL (SEQ ID NO:297). An additional embodiment is the polynucleotide fragments encoding these polypeptide fragments.

This gene is expressed primarily in synovial fibroblasts and to a lesser extent in synovial hypoxia.

Therefore, polynucleotides and polypeptides of the invention are useful as reagents for differential identification of the tissue(s) or cell type(s) present in a biological sample and for diagnosis of diseases and conditions which include, but are not limited to, synovial inflammation and other diseases of the joints. Similarly, polypeptides and antibodies directed to these polypeptides are useful in providing immunological probes for differential identification of the tissue(s) or cell type(s). For a number of disorders of the above tissues or cells, particularly of the synovium, expression of this gene at significantly higher or lower levels may be routinely detected in certain tissues and cell types (e.g., synovial tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of diseases affecting the synovium of the joints, such as rheumatoid arthritis, osteoarthritis, other inflammatory conditions affecting the joints, as well as in the detection and treatment of disorders and conditions affecting the skeletal system, in particular the connective tissues (e.g. trauma, tendonitis, chrondomalacia and inflammation). Furthermore, the homology to a conserved C.elegans protein may suggest protein is important in human development and thus is beneficial in the diagnosis, prevention, and treatment of developmental disorders.

### FEATURES OF PROTEIN ENCODED BY GENE NO: 60

This gene is expressed primarily in endothelial cells and to a lesser extent in brain.

Therefore, polynucleotides and polypeptides of the invention are useful as reagents for differential identification of the tissue(s) or cell type(s) present in a biological sample and for diagnosis of diseases and conditions which include, but are not limited to, inflammation and other disorders of the integument, in addition to neurodegenerative and nervous system disorder, such as stroke. Similarly, polypeptides and antibodies directed to these polypeptides are useful in providing immunological probes for differential identification of the tissue(s) or cell type(s). For a number of disorders of the above tissues or cells, particularly of the endothelial, circulatory, and nervous systems, expression of this gene at significantly higher or lower levels may be routinely detected in certain tissues and cell types (e.g., endothelial cells, and brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 170 as residues: Ser-4 to Gly-13.

The tissue distribution indicates that polynucleotides and polypeptides corresponding to this gene are useful for diagnosis and treatment of inflammatory diseases primarily mediated through endothelial cells, such as sepsis, inflammatory bowel disease, psoriasis, and Crohn's disease, as well as for stroke. Alternatively, the tissue distribution indicates that polynucleotides and polypeptides corresponding to this gene are useful for the detection/treatment of neurodegenerative disease states and behavioral disorders such as Alzheimer's Disease, Parkinson's Disease, Huntington's Disease, schizophrenia, mania, dementia, paranoia, obsessive compulsive disorder and panic disorder. In addition, the gene or gene product may also play a role in the treatment and/or detection of developmental disorders associated with the developing embryo, or disorders of the cardiovascular system.

### FEATURES OF PROTEIN ENCODED BY GENE NO: 61

This gene is expressed primarily in fetal brain.

Therefore, polynucleotides and polypeptides of the invention are useful as reagents for differential identification of the tissue(s) or cell type(s) present in a biological sample and for diagnosis of diseases and conditions which include, but are not limited to, CNS and PNS disorders. Similarly, polypeptides and antibodies directed to these polypeptides are useful in providing immunological probes for differential identification of the tissue(s) or cell type(s). For a number of disorders of the above tissues or cells, particularly of the nervous system, expression of this gene at significantly higher or lower levels may be routinely detected in certain tissues and cell types (e.g., developing and differentiating tissues, brain and other tissue of the nervous system, and cancerous and wounded tissues) or bodily fluids (e.g., serum, plasma, urine, synovial fluid, spinal fluid, or amniotic fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of neural disorders such as Alzheimer's disease, depression, paranoia, schizophrenia, autism, and particularly developmental brain disorders.

### FEATURES OF PROTEIN ENCODED BY GENE NO: 62

Translation product of this gene shares homology with a conserved 4-nitrophenylphosphatase from *Schizosaccharomyces pombe* (See Accession No. gil1938421). One embodiment for this gene is the polypeptide fragments comprising the following amino acid sequence: AVMIGDDCRDDVGGA (SEQ ID NO:298), and/or ILVKTGKYRASDEEKIN (SEQ ID NO:299). An additional embodiment is the polynucleotide fragments encoding these polypeptide fragments. This gene maps to chromosome 18, and therefore, may be used as a marker in linkage analysis for chromosome 18.

This gene is expressed primarily in endometrial tumors and to a lesser extent in leukemia and lymphoma.

Therefore, polynucleotides and polypeptides of the invention are useful as reagents for differential identification of the tissue(s) or cell type(s) present in a biological sample and for diagnosis of diseases and conditions which include, but are not limited to, cancer, particularly of the immune and hematopoietic systems. Similarly, polypeptides and antibodies directed to these polypeptides are useful in providing immunological probes for differential identification of the tissue(s) or cell type(s). For a number of disorders of the above tissues or cells, particularly of the endometrium and white blood cells, expression of this gene at significantly higher or lower levels may be routinely detected in certain tissues and cell types (e.g., endrometrial and/or proliferating tissues, and cells and tissue of the immune system, and cancerous and wounded tissues) or bodily fluids (e.g., serum, plasma, urine, synovial fluid, spinal fluid, or lymph) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 172 as residues: Val-19 to Cys-24.

The tissue distribution indicates that polynucleotides and polypeptides corresponding to this gene are useful for detection, diagnosis , and treatment of cancers, particularly those cancers affecting endometrial tissues and the lymphatic system. In addition, the tissue distribution indicates that polynucleotides and polypeptides corresponding to this gene are useful for the treatment and diagnosis of hematopoetic related disorders such as anemia, pancytopenia, leukopenia, thrombocytopenia or leukemia since stromal cells are important in the production of cells of hematopoietic lineages. The uses include bone marrow cell ex vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency etc. Furthermore, homology to a conserved S.pombe protein may suggest protein is important in development. Therefore, protein may be beneficial in the diagnosis, prevention, and treatment of developmental disorders.

### FEATURES OF PROTEIN ENCODED BY GENE NO: 63

The translation product of this gene shares sequence homology with ribosomal releasing factor which is thought to be important in protein synthesis.

This gene is expressed primarily in pancreatic tumors, placenta, testis, ovarian cancer, adipocytes, spleen, and fetal liver and heart.

Therefore, polynucleotides and polypeptides of the invention are useful as reagents for diagnosis of a number of diseases and conditions such as immune-diseases, cardiovascular and endocrine diseases and others. Similarly, polypeptides and antibodies directed to these polypeptides are useful in providing immunological probes for differential identification of the tissue(s) or cell type(s). For a number of disorders of the above tissues or cells, particularly of the immune system, cardiovascular system, digestive system and reproductive system. expression of this gene at significantly higher or lower levels may be routinely detected in certain tissues and cell types (e.g., pancreas, testis and ovary and other reproductive tissue, adipocytes, spleen, liver, and heart, and cancerous and wounded tissues) or bodily fluids (e.g., serum, plasma, urine, synovial fluid, spinal fluid, or lymph) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 173 as residues: Glu-36 to His-41, Thr-57 to Thr-70, Glu-87 to Met-92, Lys-100 to Lys-105, Ala-197 to Ser-227.

The tissue distribution and homology to ribosomal releasing factor indicates that polynucleotides and polypeptides corresponding to this gene are useful for the treatment and diagnosis of many diseases, especially cancers and immuno-related diseases.

### FEATURES OF PROTEIN ENCODED BY GENE NO: 64

The translation product of this gene shares sequence homology with metalloprotease and also with thrombospondin, which is thought to be important in the activation of proteins and the processes of thrombopoiesis and metabolism.

This gene is expressed in many tissues, but especially in bladder, kidney, and ovary.

Therefore, polynucleotides and polypeptides of the invention are useful as reagents for differential identification of the tissue(s) or cell type(s) present in a biological sample and for diagnosis of thrombopenia, hypertension, and other blood disfunctions. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., urogenital, and reproductive tissues, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 174 as residues: Gly-8 to Leu-14, Met-18 to Phe-30.

The tissue distribution and homology to thrombospondin indicates that polynucleotides and polypeptides corresponding to this gene are useful for the treatment and diagnosis of a variety of blood-related diseases.

### FEATURES OF PROTEIN ENCODED BY GENE NO: 65

This gene is expressed primarily in tonsil, placenta, and fetal tissues.

Therefore, polynucleotides and polypeptides of the invention are useful as reagents for differential identification of the tissue(s) or cell type(s) present in a biological sample and for diagnosis of many diseases of the immune system. Similarly, polypeptides and antibodies directed to these polypeptides are useful in providing immunological probes for differential identification of the tissue(s) or cell type(s). For a number of disorders of the immune system, expression of this gene at significantly higher or lower levels may be routinely detected in certain tissues and cell types (e.g., immune and developmental tissues, and cancerous and wounded tissues) or bodily fluids (e.g., serum, plasma, urine, synovial fluid, spinal fluid, or amniotic fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of diseases of the immune system including many cancers such as lymphomas, leukemias, lymphocytomas, and the like.

### FEATURES OF PROTEIN ENCODED BY GENE NO: 66

Polypeptides encoded by this gene share reasonable homology to steroid/thyroid hormone orphan nuclear receptor and to several additional orphan nuclear receptors isolated from several different tissues.

This gene is expressed primarily in testis.

Therefore, polynucleotides and polypeptides of the invention are useful as reagents for differential identification of the tissue(s) or cell type(s) present in a biological sample and for diagnosis of testicular tumors, impotence, and other reproductive disorders. Similarly, polypeptides and antibodies directed to these polypeptides are.useful in providing immunological probes for differential identification of the tissue(s) or cell type(s). For a number of disorders of the above tissues or cells, particularly of the reproductive system, expression of this gene at significantly higher or lower levels may be routinely detected in certain tissues and cell types (e.g., male reproductive tissue, and cancerous and wounded tissues) or bodily fluids (e.g., serum, plasma, urine, synovial fluid, spinal fluid, or seminal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the treatment and diagnosis of diseases in the male reproductive system such as tumors of the testis and other reproductive disorders.

### FEATURES OF PROTEIN ENCODED BY GENE NO: 67

Polypeptides encoded by polynucleotides comprising this gene have a high degree of sequence identity with CTGF-4:

In one embodiment, the polypeptides of the invention comprise the sequence: MDSMPEPASRCLLLLPLLLLLLLLLPAPELGPSQAGAEENDWVRLPSK CEVCKYVAVELKVKPLRKRQDTEVIGTVYGILDQKASGVKYTKSDLRLIEVTET ICKRLLDYSLHKERTGSXRFAKGMSETFETLHXLVHKGVKVVMDIPYELWNE TSAEVADLKKQCDVLVEEFEEVIEDWYRNHQEEDLTEFLCANHVLKGKDTSCL AEQWSGKKGDTAALGGKKSKKKSIRAKAAGGRSSSSKQRKELGGLEGDPSP EEDEGIQKASPLTHSPPDEL(SEQ ID NO:300. Polynucleotides encoding these polypeptide sequences are also encompassed by the invention.

This gene is expressed in many tissues especially including cells in the immune system.

Therefore, polynucleotides and polypeptides of the invention are useful as reagents for differential identification of the tissue(s) or cell type(s) present in a biological sample and for the diagnosis of cancers, immunological disorders, and neural diseases (such as spinocerebellar ataxia, bipolar affective disorder, schizophrenia, and autism), and other diseases featuring anticipation, neurodegeneration, or abnormalities of neurodevelopment. Similarly, polypeptides and antibodies directed to these polypeptides are useful in providing immunological probes for differential identification of the tissue(s) or cell type(s). For a number of disorders of the above tissues or cells, particularly of the nerve system, immune system, expression of this gene at significantly higher or lower levels may be routinely detected in certain tissues and cell types (e.g., immune cells and/or tissue, and cancerous and wounded tissues) or bodily fluids (e.g., serum, plasma, urine, synovial fluid, spinal fluid, or lymph) or another tissue or cell sample taken from an individual having such a disorder, relative to the standard gene expression level, i.e., the expression level in healthy tissue or bodily fluid from an individual not having the disorder.Preferred epitopes include those comprising a sequence shown in SEQ ID NO. 177 as residues: Ser-3 to Ser-9, Gly-36 to Val-43, Leu-45 to Gly-51.

### FEATURES OF PROTEIN ENCODED BY GENE NO: 68

Polypeptides encoded by polynucleotides comprising this gene contain a zinc finger homology domain. Such motifs are believed to be important for protein interactions, particularly with regard to gene regulation.

This gene is expressed primarily in T cells and the colon and, to a lesser extent, in the testes and placenta.

Therefore, polynucleotides and polypeptides of the invention are useful as reagents for differential identification of the tissue(s) or cell type(s) present in a biological sample and for diagnosis of many immune and digestive disorders. Similarly, polypeptides and antibodies directed to these polypeptides are useful in providing immunological probes for differential identification of the tissue(s) or cell type(s). For a number of disorders of the above tissues or cells, particularly of the immune and digestive systems, expression of this gene at significantly higher or lower levels may be routinely detected in certain tissues and cell types (e.g., immune, gastrointestinal, and reproductive system tissues, and cancerous and wounded tissues) or bodily fluids (e.g., serum, plasma, urine, synovial fluid, spinal fluid, or seminal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 178 as residues: Pro-12 to Lys-33, Asn-41 to His-46, Pro-48 to Ser-58, Gly-71 to Asp-78, Ala-94 to Gly-102, Ser-133 to Ser-140, Arg-197 to Lys-202.

The expression of this gene in T-cells indicates a potential role in the treatment and detection of immune disorders such as arthritis, asthma, immune deficiency diseases (such as AIDS), and leukemia. Expression of this gene in the colon indicates a potential role in the treatment and detection of colon disorders such as ulcers and colon cancer in addition to digestive disorders in general.

### FEATURES OF PROTEIN ENCODED BY GENE NO: 69

The translation product of this gene shares sequence homology with neuroendocrine protein which is thought to be important in neuronal development and differentiation. A preferred embodiment of this gene comprises the following amino acid sequence: MDGQKKNWKDKVVDLLYWRDIKKTGVVFGASLFLLLSLTVF SIVSVTAYIALALLSVTISFRIYKGVIQAIQKSDEGHPFRAYLESEVAISEELVQKY SNSALGHVNGTIKELRRLFLVDDLVDSLKFAVLMWVFTYVGALFNGLTLLILAL ISLFSVPVIYERHQAQIDHYLGLANKNVKDAMAKIQAKIPGLKRKAE (SEQ ID NO:301). Particularly preferred are polynucleotides comprising polynucleotides encoding this polypeptide sequence.

This gene is expressed in many different tissues, but primarily in brain, and, to a lesser extent, in fetal tissue, placenta, bone marrow, and stromal cells.

Therefore, polynucleotides and polypeptides of the invention are useful as reagents for diagnosis of neurodegenerative diseases and developmental disorders. Similarly, polypeptides and antibodies directed to these polypeptides are useful in providing immunological probes for differential identification of the tissue(s) or cell type(s). For a number of disorders of the above tissues or cells, particularly of the nervous system and during development, expression of this gene at significantly higher or lower levels may be routinely detected in certain tissues and cell types (e.g., neural, developmental, and hemopoietic cells and tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 179 as residues: Gln-47 to Gly-52, Leu-169 to Glu-174.

The predominant tissue distribution in brain and homology to neuroendocrine protein indicates that polynucleotides and polypeptides corresponding to this gene are useful for the diagnosis and treatment of neurodegenerative diseases and behavioral disorders such as Alzheimer's Disease, Parkinson's Disease, Huntington's Disease, schizophrenia, mania, dementia, paranoia, obsessive-compulsive disorder and panic disorder.

### FEATURES OF PROTEIN ENCODED BY GENE NO: 70

Polypeptides encoded by polynucleotides comprising this gene share sequence identity with human hepatoma-derived growth factor (WPI 95-069304/10). As such; polynucleotides comprising this gene can be used for the recombinant production of the protein, which can be used to encourage the growth of various animal cells, and for the purification of receptors. Additional embodiments of the invention comprise the following polypeptide sequences: MAVTLSLLLGGRVCA (SEQ ID NO:302); PSLAVGSRPGGW RAQALLAGSRTPIPTGSRRNGSCRRWRAP (SEQ ID NO:303); and/or MAVTLSLLLGGRVCAPSLAVGSRPGGWRAQALLAGSRTPIPTG SRRNGSCRRWRAP (SEQ ID NO:304). Also contemplated are polynucleotides comprising polynucleotides encoding the aforementioned polypeptide sequences.

This gene is expressed primarily in brain and to a lesser extent in endotheilium, T- cell, and tumors.

Therefore, polynucleotides and polypeptides of the invention are useful as reagents for differential identification of the tissue(s) or cell type(s) present in a biological sample and for diagnosis of many neurodegenerative diseases (for example, Alzheimer's Disease, ALS, and the like) and cancers (including, but not limited to neuroblastoma, glioblastoma, Schwannoma, astrocytoma, and the like). Similarly, polypeptides and antibodies directed to these polypeptides are useful in providing immunological probes for differential identification of the tissue(s) or cell type(s). For a number of disorders of the above tissues or cells, particularly of the nervous system, expression of this gene at significantly higher or lower levels may be routinely detected in certain tissues and cell types (e.g., neural, and haematopoietic cells and tissue, and cancerous and wounded tissues) or bodily fluids (e.g., serum, plasma, urine, synovial fluid, spinal fluid or lymph) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 180 as residues: Pro-4 to Thr-10, Glu-25 to Trp-30, Leu-58 to Leu-69, Arg-82 to Thr-87, Ala-108 to His-115, Ser-124 to Glu-146, Pro-159 to Gly-176, Ser-182 to Glu-187, Leu-189 to Ser-198, Phe-208 to Asn-214.

The tissue distribution indicates that polynucleotides and polypeptides corresponding to this gene are useful for the treatment and diagnosis of many neurodegenerative diseases and cancers.

### FEATURES OF PROTEIN ENCODED BY GENE NO: 71

The translation product of this gene shares sequence homology with acrosin, trypsin, as well as trypsinogen precursor which are thought to be important in cell-cell recognition and proteinase activity for protein cleavage and degradation. Preferred polynucleotide fragments comprise the following sequence:
GATGTTACACAGCTCTTTAATAATAGTGGCCATAGCTGTAATAACAATGACA ACAGTAGGTAACGGTAGTCATACCAACAGTAGGGCAGTGCATTTTATATTAC AACTGGTTTCTTGCTCTAGTAGGCTTGGGGATGGGTGAAGACGGACAGGGC TGGCGCAGACCCTTTCCTTCTCCTCTCCAGCCCACAGTGATCTGGGCTTTTA CAGACAGCCTGCTTCCATTCAGTAGTGTGGGAAAGTTCCTTCTTGGCTTAGC AATACCCCTGAGACCTTGTTCAGTGGGCTGTGTCTCTCCCTGGGATGCTGG GAGCACCAAGTGTGGCCGAGCTAGGGCTGCTGACTTCCTCTGGGCGCCTCT GGGCTGCGAGGGTCTCTTATAGGAATTGAGGCCCTTTGCTGCTCCAAGAAA TGCGAGGCTGTGGGCARAGGGKTGTACCCAAGGGGACTCTTGCTCTGTGT CTGACTTTGGGGRATCC (SEQ ID NO:305); CACAGCTCTTTAATAATAGTGGC CATAGCTGTAATAACAATGACA ACAGTAGGTAACG (SEQ ID NO:306); TGTGTCTCTCCCTGGGATGCTGGGAGCACCAAGTGTGGCCGAGCTAGGGCT GCTGACTT (SEQ ID NO:307); GCGAGGGTCTCTTATAGGAATTGAGGCCCTT TGCTGCTCCAAGAAATGCTGAGGCTGTGGGCARAGGGKTGTACCCAAGGG GACT (SEQ ID NO:308). Also preferred are polypeptide fragments encoded by these polynucleotide fragments.

This gene is expressed primarily in cheek carcinoma and to a lesser extent in uterine and pancreatic cancers.

Therefore, polynucleotides and polypeptides of the invention are useful as reagents for differential identification of the tissue(s) or cell type(s) present in a biological sample and for diagnosis of diseases and conditions which include, but are not limited to, cheek cancers or cancers of uterine and pancreatic origins. Similarly, polypeptides and antibodies directed to these polypeptides are useful in providing immunological probes for differential identification of the tissue(s) or cell type(s). For a number of disorders of the above tissues or cells, particularly of the neoplastic tissues, expression of this gene at significantly higher or lower levels may be routinely detected in certain tissues and cell types (e.g., epithelial, endocrine, and reproductive tissues, and cancerous and wounded tissues) or bodily fluids (e.g., serum, plasma, urine, synovial fluid, spinal fluid, and saliva) or another tissue or cell sample taken from an individual having such a disorder, relative to the standard gene expression level, i.e., the expression level in healthy tissue or bodily fluid from an individual not having the disorder.

The tissue distribution and homology to acrosin and trypsin indicates that polynucleotides and polypeptides corresponding to this gene are useful for diagnosis and intervention of cancers. The homology to acrosin and trypsin may indicate the gene function in tumor metastasis or migration since in both cases cell-cell interaction and extracellular matrix degradation may be involved. The gene product can also be used as a target for cancer immunotherapy or as a diagnostic marker.

### FEATURES OF PROTEIN ENCODED BY GENE NO: 72

This gene is expressed primarily in T helper cells I, T-cells stimulated with PHA for 24 hours, and in a placenta Nb2HP cDNA library.

Therefore, polynucleotides and polypeptides of the invention are useful as reagents for differential identification of the tissue(s) or cell type(s) present in a biological sample and for diagnosis of many immunodeficiencies and disorders (especially autoimmune diseases). Similarly, polypeptides and antibodies directed to these polypeptides are useful in providing immunological probes for differential identification of the tissue(s) or cell type(s). For a number of disorders of the above tissues or cells, particularly of the immune system expression of this gene at significantly higher or lower levels may be routinely detected in certain tissues and cell types (e.g., immune, and haematopoietic cells and tissue, and cancerous and wounded tissue) or bodily fluids (e.g., serum, plasma, urine, synovial fluid, spinal fluid and lymph) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of autoimmune diseases, immunodeficiencies, and other immune system disorders.

### FEATURES OF PROTEIN ENCODED BY GENE NO: 73

This gene is expressed primarily in 7 week old early stage human, human chronic synovitis, and infant brain.

Therefore, polynucleotides and polypeptides of the invention are useful as reagents for differential identification of the tissue(s) or cell type(s) present in a biological sample and for diagnosis of chronic synovitis. Similarly, polypeptides and antibodies directed to these polypeptides are useful in providing immunological probes for differential identification of the tissue(s) or cell type(s). For a number of disorders of the above tissues or cells, particularly of the synovium, expression of this gene at significantly higher or lower levels may be routinely detected in certain tissues and cell types (e.g., developmental, differentiating, and neural tissues, and cancerous and wounded tissues) or bodily fluids (e.g., serum, plasma, urine, synovial fluid, spinal fluid, and amniotic fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 183 as residues: Ser-44 to Pro-49.

The tissue distribution indicates that polynucleotides and polypeptides corresponding to this gene are useful for the diagnosis and treatment of chronic synovitis and other disorders of the synovium.

### FEATURES OF PROTEIN ENCODED BY GENE NO: 74

Polypeptides encoded by polynucleotides comprising this gene exhibit sequence homology to a number of mucin-like extracellular or cell surface proteins. In one embodiment polypeptides of the invention comprise the following sequence:
MVGPVTLHKKIHTTTVLFIVQIHILLIQAITQAK (SEQ ID NO:309); LQMHLMILQ MTGLSILALLGKSTTTIVEQKFHNGKNQKSGLKENRDKKKQTRWQSTASQKI GITEER (SEQ ID NO:310); and/or MVGPVTLHKKIHTTTVLFIVQIHILLIQAITQ AKLQMHLMILQMTGLSILALLGKSTTTIVEQKFHNGKNQKSGLKENRDKKKQ TRWQSTASQKIGITEER (SEQ ID NO:311). Polynucleotides encoding the aforementioned polypeptides are also contemplated embodiments of the invention.

This gene is expressed primarily in ovarian cancer, endometrial tumor, B-cell lymphoma, brain-medulloblastoma, hepatocellular tumor, osteosarcoma, and T- and B-cells.

Therefore, polynucleotides and polypeptides of the invention are useful as reagents for differential identification of the tissue(s) or cell type(s) present in a biological sample and for diagnosis of diseases and conditions which include, but are not limited to, Ovarian cancer, endometrial tumor, B-cell lymphoma, brain medulloblastoma, hepatocellular tumor, and osteosarcoma. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., brain and other tissue of the nervous system, bone, T-cells, and other cells of the immune system, and B cells and other blood cells, and cancerous and wounded tissues) or bodily fluids (e.g., serum, plasma, urine, synovial fluid, spinal fluid and lymph) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 184 as residues: Met-1 to Lys-12, Leu-14 to Asn-35, Arg-42 to Asn-58, Ser-65 to Trp-90, Ser-95 to Asn-129, Phe-136 to Arg-144, Met-159 to Ala-167, Thr-179 to Tyr-187, Pro-190 to Val-201, Gln-226 to Phe-235, Pro-254 to His-272, Thr-288 to Thr-293, Thr-383 to Ser-391, Asp-398 to Tyr-405, Ile-410 to Asn-416, Ala-449 to Lys-458.

The tissue distribution indicates that polynucleotides and polypeptides corresponding to this gene are useful for the diagnosis and treatment of ovarian cancer, endometrial tumors, B-cell lymphoma, brain medulloblastoma, hepatocellular tumor, and osteosarcoma.

### FEATURES OF PROTEIN ENCODED BY GENE NO: 75

An additional preferred polypeptide sequence derived from the polynucleotide of this contig comprises the following amino acid sequence: MQTCPLVGTLLTRNMDG YTCAVVTSTSFWIISAWXLWKGSPSTSMPTMPETPLRTLCCTKMPSIFSSLMTD GRA (SEQ ID NO:312). Polynucleotides encoding these polypeptides are also provided. This polypeptide sequence has sequence homology with a *Drosophila melanogaster* male germ-line specific transcript which encodes a putative protamine molecule (see, gil608696).

This gene is expressed primarily in breast tissue and to a lesser extent in various other fetal and adult cells and tissues, especially those comprising endocrine organs.

Therefore, polynucleotides and polypeptides of the invention are useful as reagents for differential identification of the tissue(s) or cell type(s) present in a biological sample and for diagnosis of diseases and conditions which include, but are not limited to, developmental and reproductive defects. Similarly, polypeptides and antibodies directed to these polypeptides are useful in providing immunological probes for differential identification of the tissue(s) or cell type(s). For a number of disorders of the above tissues or cells, particularly of the female reproductive system, expression of this gene at significantly higher or lower levels may be routinely detected in certain tissues and cell types (e.g., breast and/or other ductile secretory tissues, and cancerous and wounded tissues) or bodily fluids (e.g., serum, plasma, urine, synovial fluid, spinal fluid, and milk) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for study and treatment of developmental, reproductive and growth and metabolic disorders.

### FEATURES OF PROTEIN ENCODED BY GENE NO: 76

In one embodiment, the polypeptides of the invention comprise the sequence: MTLIQNCWYSWLFFGFFFHFLRKSISIFSIFLVCFRILALGPTCFLVWFWKAFFR HILIFICLSREVFRPRCFLVYFR (SEQ ID NO:313). This polypeptide sequence has sequence homology with the MURF4 protein of Herpetomonas muscarum (S43288). Such RNA-editing enzymes may be useful as molecular targets in the intervention of the life cycle of trypanosomes and other protozoa. Polynucleotides encoding these polypeptides are also encompassed by the invention.

This gene is expressed primarily in fetal liver and spleen, osteosarcoma and bone marrow.

Therefore, polynucleotides and polypeptides of the invention are useful as reagents for differential identification of the tissue(s) or cell type(s) present in a biological sample and for diagnosis of liver tumors, osteosarcoma, and other cancers. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., hepatic, developmental, and differentiating tissue, bone cells, liver and spleen, and cancerous and wounded tissues) or bodily fluids (e.g., serum, plasma, urine, synovial fluid, spinal fluid, and lymph) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis of cancers such as liver tumor and osteosarcoma.

### FEATURES OF PROTEIN ENCODED BY GENE NO: 77

This gene is expressed primarily in T cell lymphoma and monocytes.

Therefore, polynucleotides and polypeptides of the invention are useful as reagents for differential identification of the tissue(s) or cell type(s) present in a biological sample and for diagnosis of T-cell lymphoma. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., immune and hematopoietic cells and tissues, and cancerous and wounded tissues) or bodily fluids (e.g., serum, plasma, urine, synovial fluid, spinal fluid, and lymph) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 187 as residues: Thr-1 to Ser-9.

The tissue distribution indicates that polynucleotides and polypeptides corresponding to this gene are useful for diagnosis and treatment of T-cell lymphoma.

### FEATURES OF PROTEIN ENCODED BY GENE NO: 78

This gene is expressed primarily in tonsils and a bone marrow cell line.

Therefore, polynucleotides and polypeptides of the invention are useful as reagents for differential identification of the tissue(s) or cell type(s) present in a biological sample and for diagnosis of immunological disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., haematopoietic and immune cells and tissues, and cancerous and wounded tissues) or bodily fluids (e.g., serum, plasma, urine, synovial fluid, spinal fluid, and lymph) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of immunological disorders.

### FEATURES OF PROTEIN ENCODED BY GENE NO: 79

In one embodiment, the polypeptides of the invention comprise the sequence: MGTRAQVTPGRLPIPPPAPGLPFSAXEPLQGQLRRVSSSRGGFPGLALQLLRSE TVKAYVNNEINILASFF (SEQ ID NO:314) and/or MLVRTRPSQPLPLPGVGLGGP RSGDPPESTELRKGPGFLA (SEQ ID NO:315). Polynucleotides encoding these polypeptides are also encompassed by the invention.

This gene is expressed primarily in brain, placenta, bone marrow, keratinocyte, fetal liver, and spleen.

Therefore, polynucleotides and polypeptides of the invention are useful as reagents for differential identification of the tissue(s) or cell type(s) present in a biological sample and for diagnosis of brain and skin related diseases. Similarly, polypeptides and antibodies directed to these polypeptides are useful in providing immunological probes for differential identification of the tissue(s) or cell type(s). For a number of disorders of the above tissues or cells, particularly of the immune and skin system, expression of this gene at significantly higher or lower levels may be routinely detected in certain tissues and cell types (e.g., neural, reproductive, and hepatic tissues, keratinocytes, and spleen, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 189 as residues: Phe-13 to Leu-18.

The tissue distribution indicates that polynucleotides and polypeptides corresponding to this gene are useful for the diagnosis and treatment of many brain and skin related diseases.

### FEATURES OF PROTEIN ENCODED BY GENE NO: 80

The translation product of this gene shares sequence homology with mouse RNA Polymerase I which is thought to be important in gene transcription process.

This gene is expressed primarily in HEL cell line and aorta endothelial cells and to a lesser extent in Jurkat T-cells.

Therefore, polynucleotides and polypeptides of the invention are useful as reagents for differential identification of the tissue(s) or cell type(s) present in a biological sample and for diagnosis and treatment of cancer and autoimmune diseases. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., endothelial, haematopoietic tissues, cardiovascular tissue, and T-cells and other cells of the immune system, and cancerous and wounded tissues) or bodily fluids (e.g., serum, plasma, urine, synovial fluid, spinal fluid, and lymph) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 190 as residues: Lys-25 to Arg-32.

The tissue distribution and homology to mouse RNA polymerase I indicates that polynucleotides and polypeptides corresponding to this gene are useful for the treatment of immune diseases and cardiovascular diseases.

### FEATURES OF PROTEIN ENCODED BY GENE NO: 81

In one embodiment, the polypeptides of the invention comprise the sequence:
MCPVCGRALSSPGSLGRHLLIHSEDQRSNCAVCGARFTSHATFNSEKLPEVLN MESLPTVHNEGPSSAEGKDIAFSPPVYPAGILLVCNNCAAYRKXLEAQTPSVX KWALRRQNEPLEVRLQRLERERTAKKSRRDNETPEEREVRRMRDREAKRLQR MQETDEQRARRLQRDREAMRLKRANETPEKRQARLIREREAKRLKRRLEKMD MMLRAQFGQDPSAMAALAAEMNFFQLPVSGVELDXQLLGKMAFEEQNSSXLH (SEQ ID NO:316). This polypeptide shares sequence homology with human trichohylin which is thought to be important in gene regulation. Polynucleotides encoding this polypeptide are also encompassed by the invention.

This gene is expressed primarily in brain tissue and to a lesser extent in apoptopic T-cell and B-cell lymphoma.

Therefore, polynucleotides and polypeptides of the invention are useful as reagents for differential identification of the tissue(s) or cell type(s) present in a biological sample and for diagnosis and treatment of growth disorders, neurodegenerative diseases, and endochrine disorders. Similarly, polypeptides and antibodies directed to these polypeptides are useful in providing immunological probes for differential identification of the tissue(s) or cell type(s). For a number of disorders of the above tissues or cells, particularly of the neural and immune systems, expression of this gene at significantly higher or lower levels may be routinely detected in certain tissues and cell types (e.g., neural tissues, T-cells, B-cells and other cells and tissue of the immune system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to DNA binding protein indicates that polynucleotides and polypeptides corresponding to this gene are useful for the diagnosis and treatment of immune and neurological diseases.

### FEATURES OF PROTEIN ENCODED BY GENE NO: 82

In one embodiment, the polypeptides of the invention comprise the sequence: MDHSHHMGMSYMDSNSTMQPSHHHPTTSASHSHGGGDSSMMMMPMTFYFG FKNVELLFSGLVINTAGEMAGAFVAVFLLAMFYEGLKIARESLLRKSQVSIRYN SMPVPGPNGTILMETHKTVGQQMLSFPHLLQTVLHIIQVVISYFLMLIFMTYNG YLCIAXAAGAGTGYFLFSWKKAVVVDITEHCH (SEQ ID NO:317). This polypeptide is thought to function in mediating the uptake of copper and other metal ions by cells. Polynucleotides encoding this polypeptide are also encompassed by the invention.

This gene is expressed primarily in osteosarcoma and to a lesser extent in T-cell and bone marrow stromal cell.

Therefore, polynucleotides and polypeptides of the invention are useful as reagents for differential identification of the tissue(s) or cell type(s) present in a biological sample and for treatment and diagnosis of osteosarcoma arid copper and other metal uptake disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., hematopoietic tissue and cancerous and wounded tissues) or bodily fluids (e.g., serum, plasma, urine, synovial fluid, spinal fluid, and lymph) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 192 as residues: Ser-24 to Ser-29.

The tissue distribution indicates that polynucleotides and polypeptides corresponding to this gene are useful for the prevention or treatment of osteosarcoma and copper or other metal uptake disorders.

### FEATURES OF PROTEIN ENCODED BY GENE NO: 83

This gene is expressed primarily in skin tumor and to a lesser extent in apoptic T-cell.

Therefore, polynucleotides and polypeptides of the invention are useful as reagents for differential identification of the tissue(s) or cell type(s) present in a biological sample and for diagnosis of diseases and conditions which include, but are not limited to, skin tumor. Similarly, polypeptides and antibodies directed to these polypeptides are useful in providing immunological probes for differential identification of the tissue(s) or cell type(s). For a number of disorders of the above tissues or cells, particularly of the skin, expression of this gene at significantly higher or lower levels may be routinely detected in certain tissues and cell types (e.g., epithelial and hematopoietic tissues, and T-cells and other tissue of the immune system, and cancerous and wounded tissues) or bodily fluids (e.g., serum, plasma, urine, synovial fluid, and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 193 as residues: Leu-51 to Gly-77, Ile-117 to Pro-125.

The tissue distribution indicates that polynucleotides and polypeptides corresponding to this gene are useful for diagnosis the treatment of skin tumor.

### FEATURES OF PROTEIN ENCODED BY GENE NO: 84

This gene is expressed primarily in testis.

Therefore, polynucleotides and polypeptides of the invention are useful as reagents for differential identification of the tissue(s) or cell type(s) present in a biological sample and for diagnosis of diseases and conditions which include, but are not limited to, infertility and endocrine disorders. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels may be routinely detected in certain tissues and cell types (e.g., reproductive tissue, and cancerous and wounded tissues) or bodily fluids (e.g., serum, plasma, urine, synovial fluid, spinal fluid, and seminal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the treatment of reproductive disease and endocrine disorders.

### FEATURES OF PROTEIN ENCODED BY GENE NO: 85

In one embodiment, the polypeptides of the invention comprise the sequence: MVQPCGACAKTXWKACSSCCSSPCCLQERWPXPXAXCPEXGPSSHPGIQALC AVAVVYLSPSSRLDWSLAPLFVPSLAAGETPLTQPAWALTTNTLGHGQPAQDR LPALGHCAPISVLGLGSS (SEQ ID NO:318). Polynucleotides encoding this polypeptide sequence are also encompassed by the invention.

This gene is expressed primarily in kidney cortex, frontal cortex, spinal cord and hippocampus.

Therefore, polynucleotides and polypeptides of the invention are useful as reagents for differential identification of the tissue(s) or cell type(s) present in a biological sample and for diagnosis of diseases and conditions which include, but are not limited to, kidney fibrosis, schizophrenia and neurological disorders. Similarly, polypeptides and antibodies directed to these polypeptides are useful in providing immunological probes for differential identification of the tissue(s) or cell type(s). For a number of disorders of the above tissues or cells, particularly of the neural system, expression of this gene at significantly higher or lower levels may be routinely detected in certain tissues and cell types (e.g., endothelial, neural and endocrine tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 195 as residues: Cys-27 to Tyr-33, Thr-38 to Gly-43, Leu-125 to Gly-130.

The tissue distribution indicates that polynucleotides and polypeptides corresponding to this gene are useful for the treatment of neurological disorders and kidney diseases.

### FEATURES OF PROTEIN ENCODED BY GENE NO: 86

This gene is expressed primarily in resting T-cell.

Therefore, polynucleotides and polypeptides of the invention are useful as reagents for differential identification of the tissue(s) or cell type(s) present in a biological sample and for diagnosis of diseases and conditions which include, but are not limited to, T-cell related diseases. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., hematopoietic and immune cells and tissues, and cancerous and wounded tissues) or bodily fluids (e.g., serum, plasma, urine, synovial fluid, spinal fluid, and lymph) or another tissue or cell sample taken from an individual having such a disorder, relative to the standard gene expression level, (i.e., the expression level in healthy tissue or bodily fluid from an individual not having the disorder). Preferred epitopes include those comprising a sequence shown in SEQ ID NO. 196 as residues: Thr-54 to Ile-59.

The tissue distribution indicates that polynucleotides and polypeptides corresponding to this gene are useful for the treatment of immune diseases.

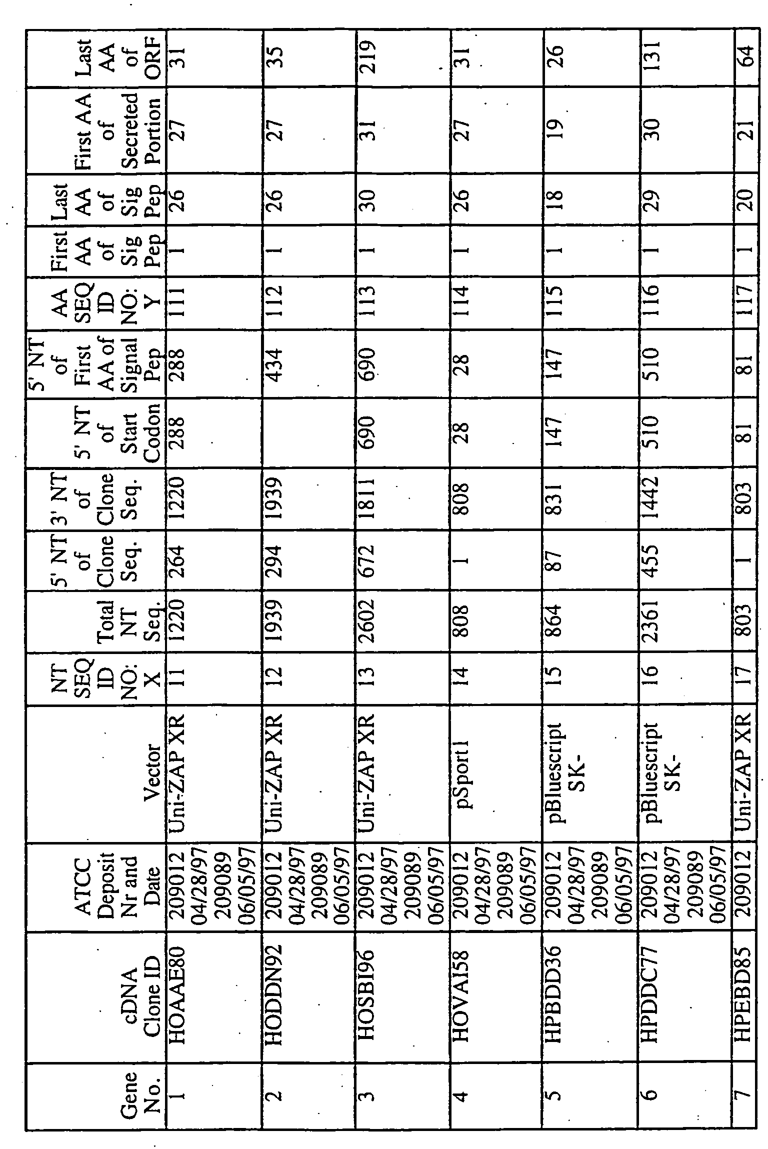

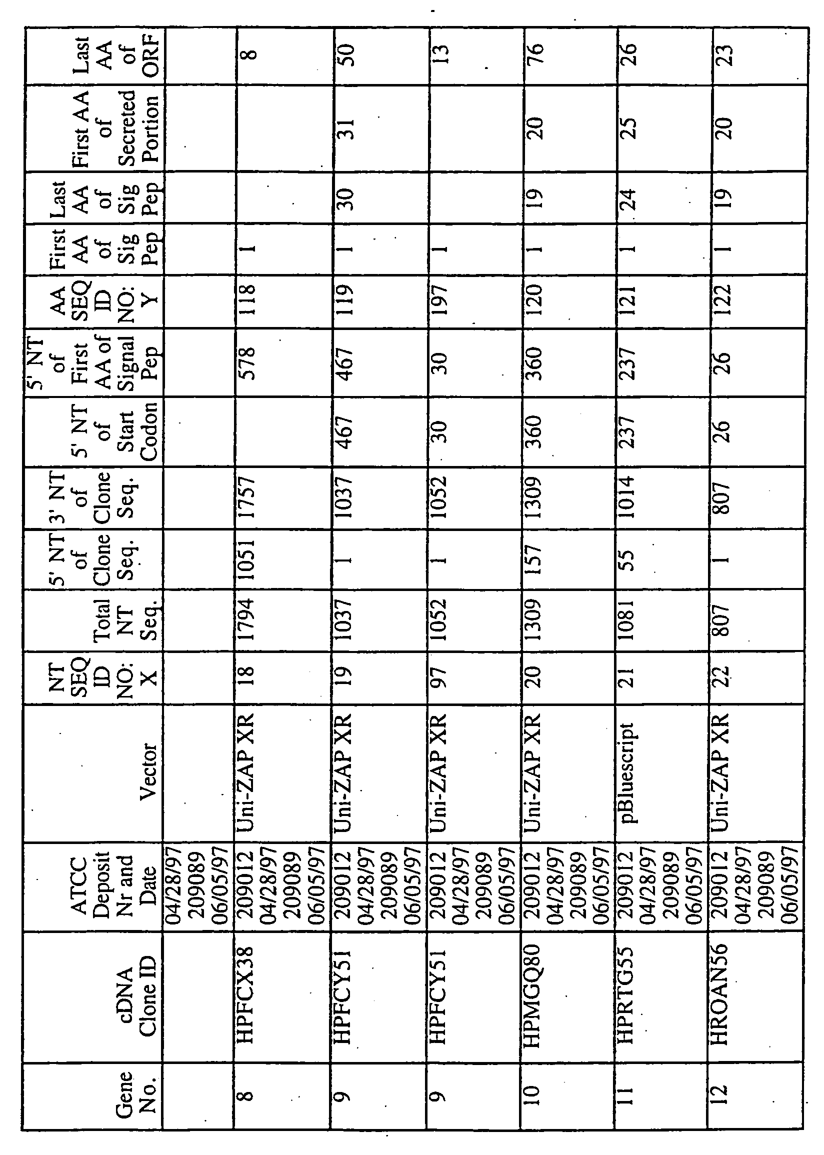

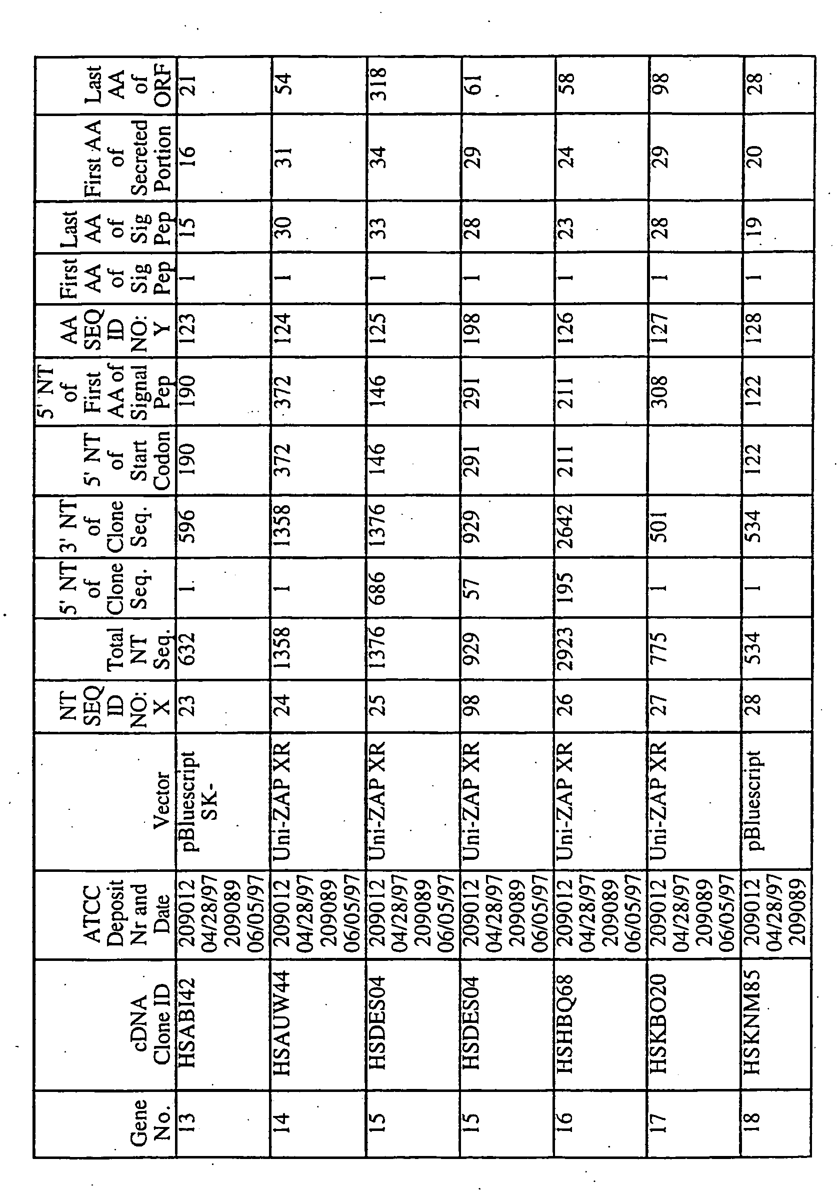

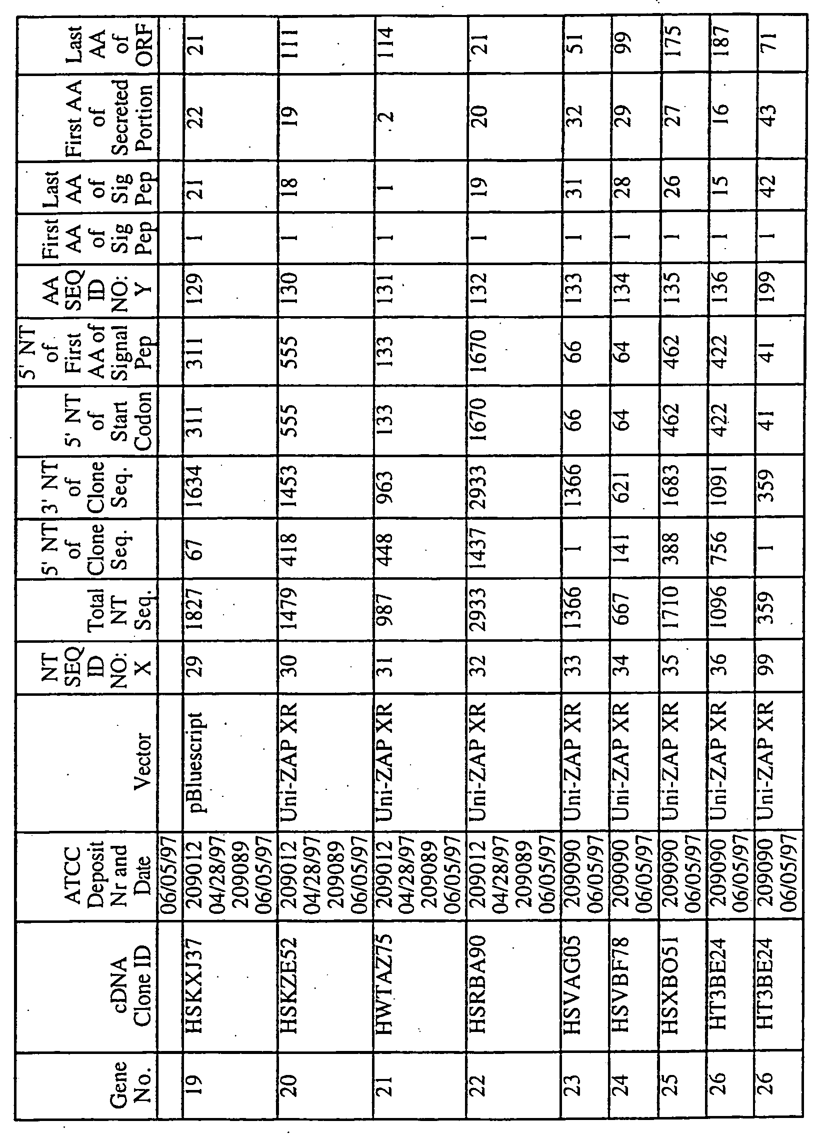

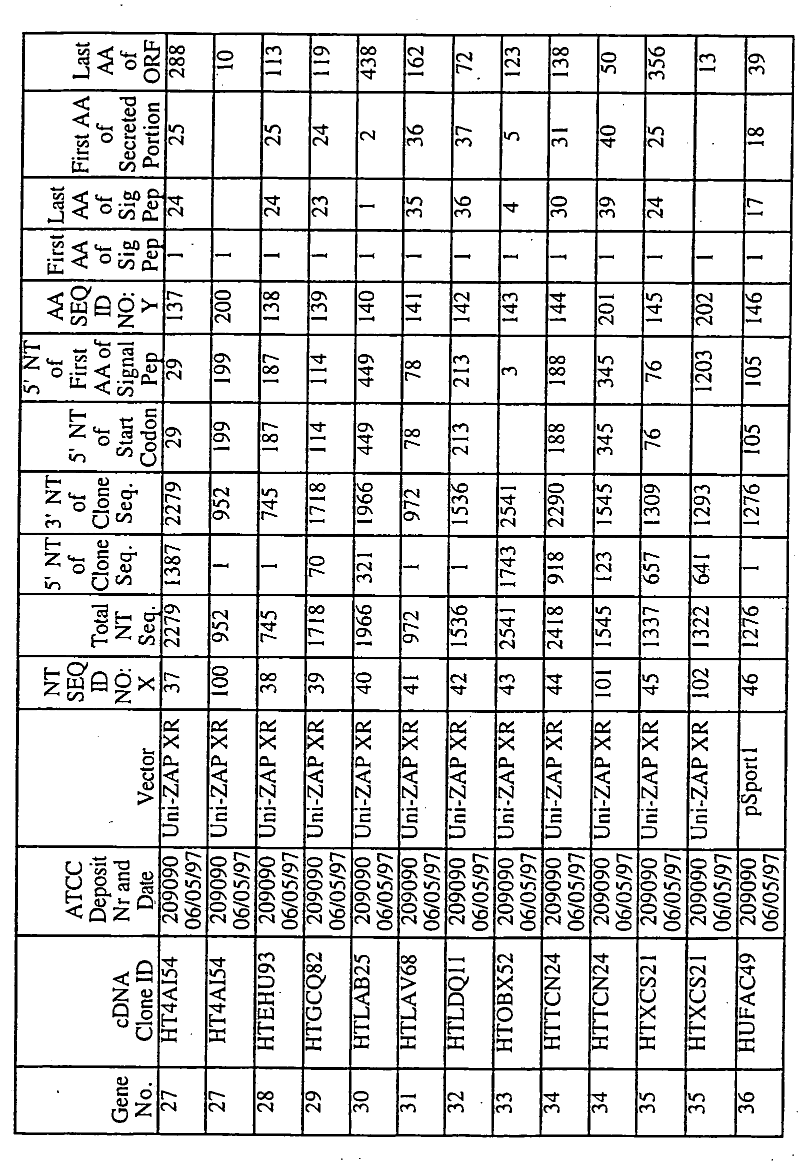

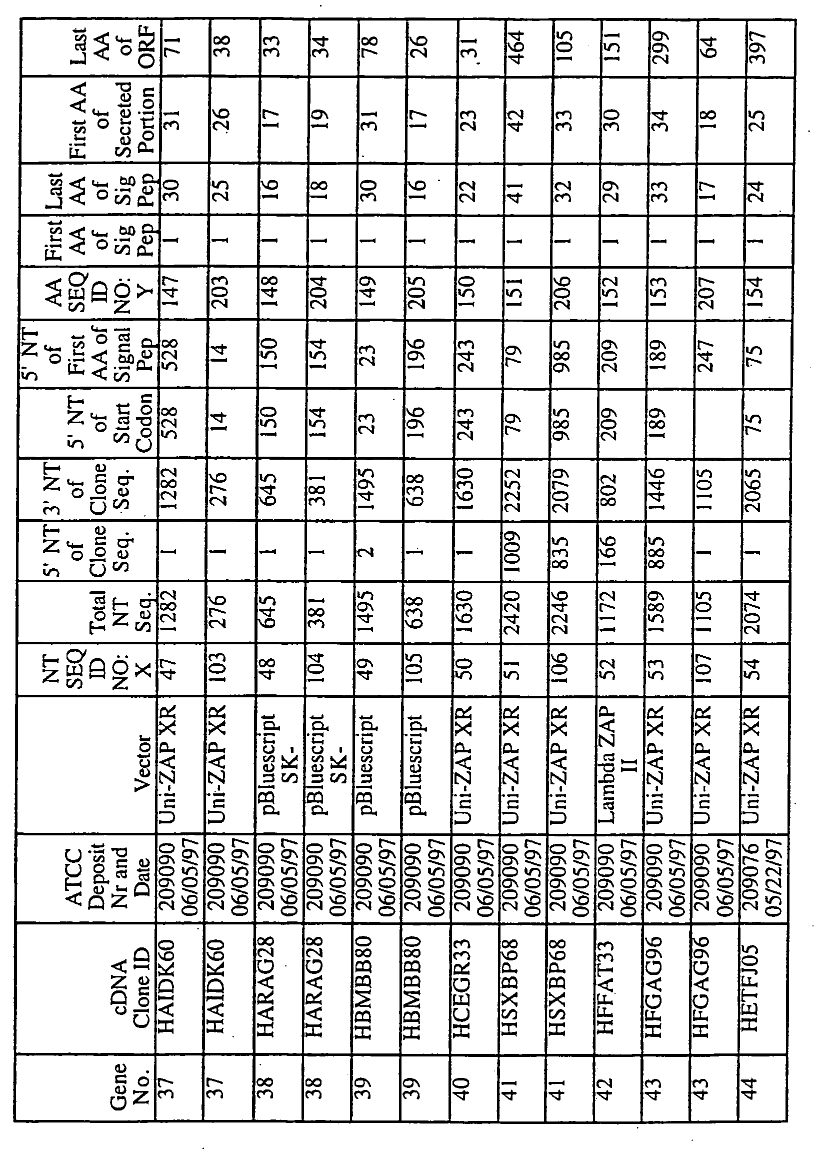

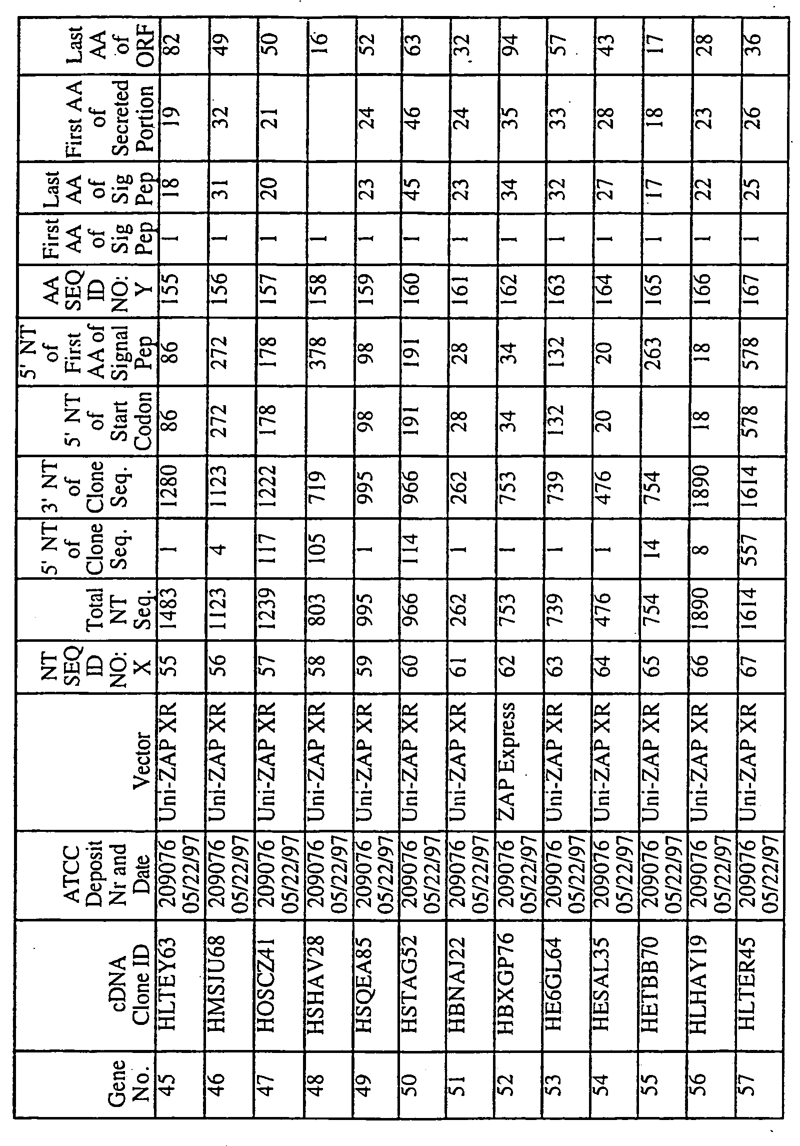

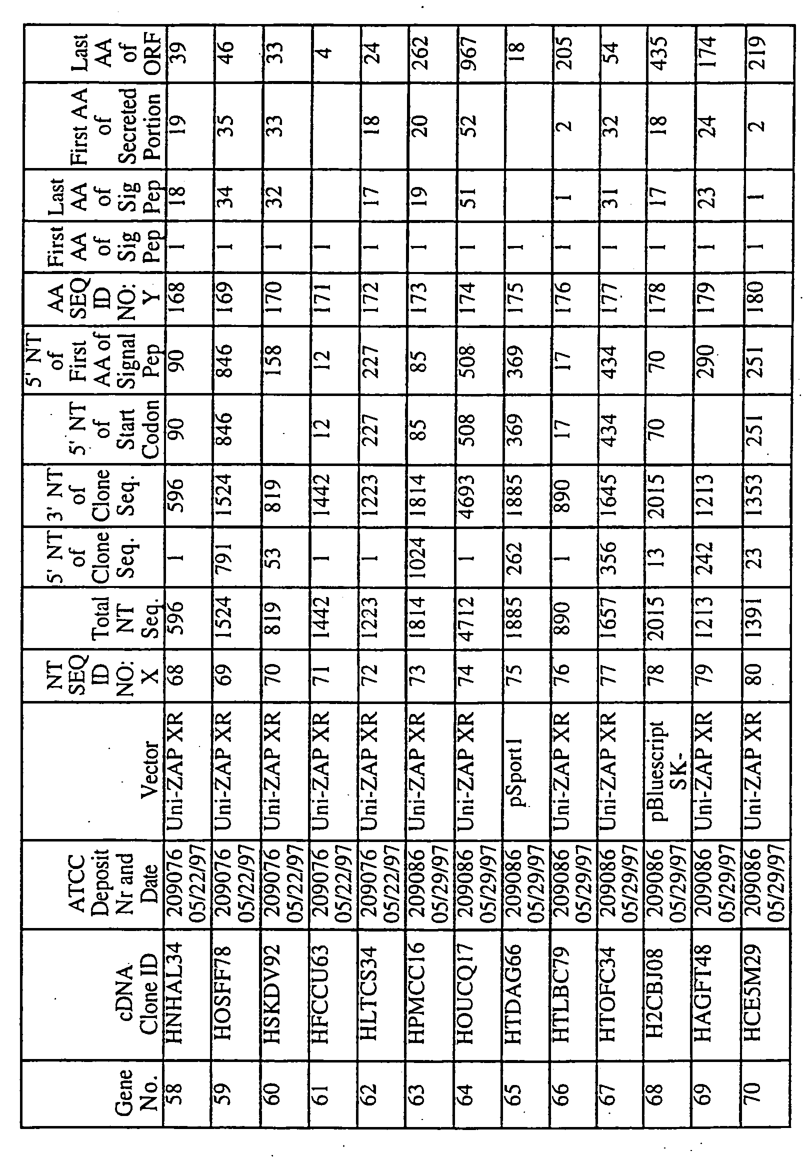

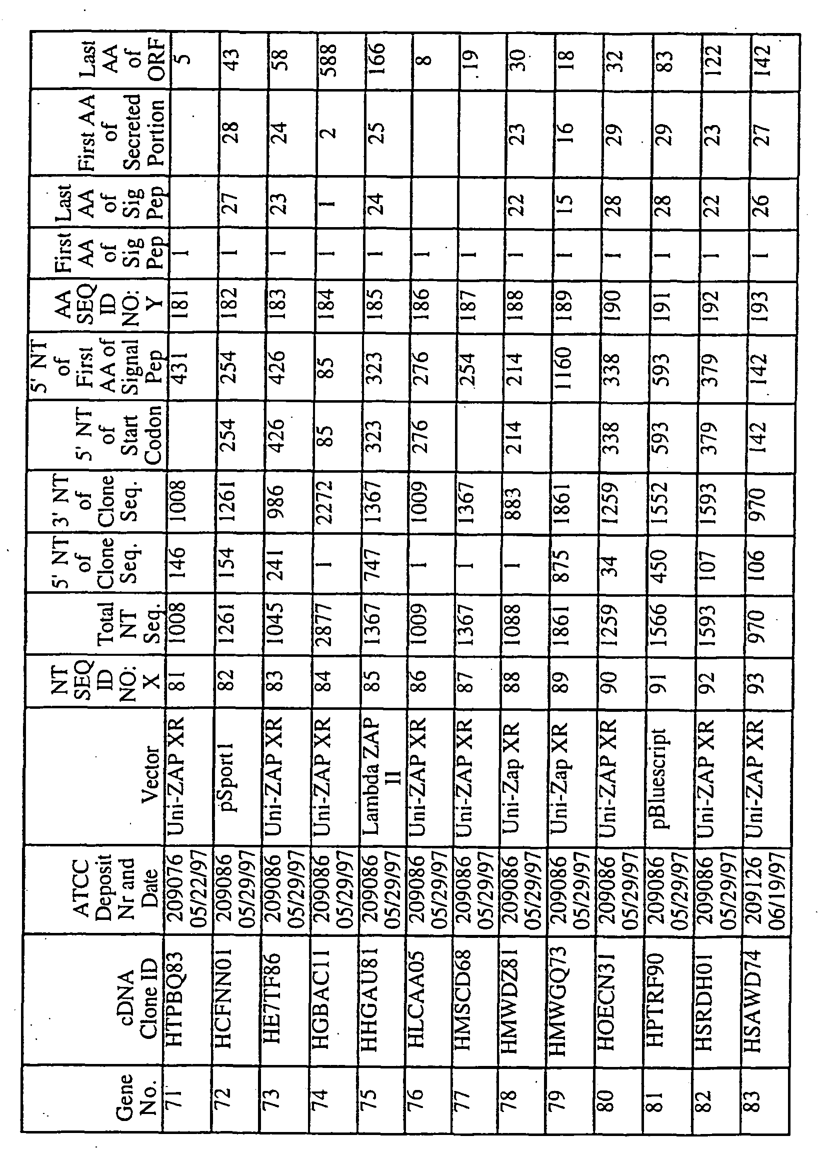

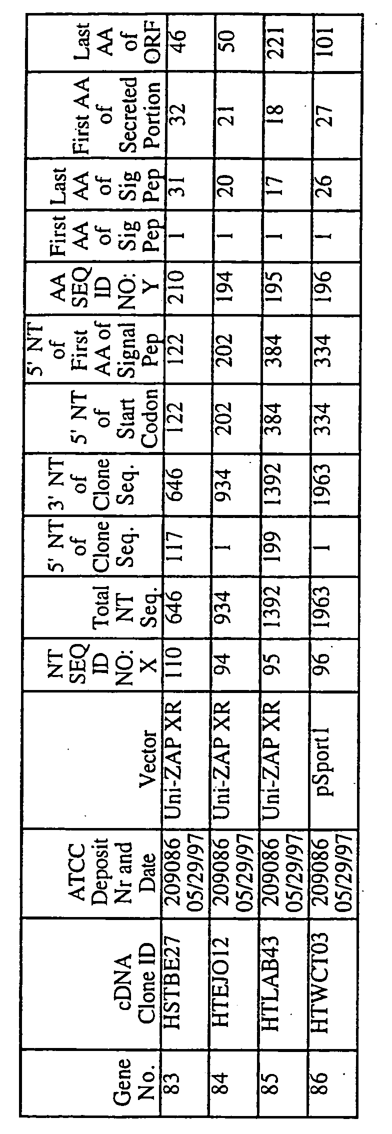

Table 1 summarizes the information corresponding to each "Gene No." described above. The nucleotide sequence identified as "NT SEQ ID NO:X" was assembled from partially homologous ("overlapping") sequences obtained from the "cDNA clone ID" identified in Table 1 and, in some cases, from additional related DNA clones. The overlapping sequences were assembled into a single contiguous sequence of high redundancy (usually three to five overlapping sequences at each nucleotide position), resulting in a final sequence identified as SEQ ID NO:X.

The cDNA Clone ID was deposited on the date and given the corresponding deposit number listed in "ATCC Deposit No:Z and Date." Some of the deposits contain multiple different clones corresponding to the same gene. "Vector" refers to the type of vector contained in the cDNA Clone ID.

"Total NT Seq." refers to the total number of nucleotides in the contig identified by "Gene No." The deposited clone may contain all or most of these sequences, reflected by the nucleotide position indicated as "5' NT of Clone Seq." and the "3' NT of Clone Seq." of SEQ ID NO:X. The nucleotide position of SEQ ID NO:X of the putative start codon (methionine) is identified as "5' NT of Start Codon." Similarly , the nucleotide position of SEQ ID NO:X of the predicted signal sequence is identified as "5' NT of First AA of Signal Pep."

The translated amino acid sequence, beginning with the methionine, is identified as "AA SEQ ID NO:Y," although other reading frames can also be easily translated using known molecular biology techniques. The polypeptides produced by these alternative open reading frames are specifically contemplated by the present invention.

The first and last amino acid position of SEQ ID NO:Y of the predicted signal peptide is identified as "First AA of Sig Pep" and "Last AA of Sig Pep." The predicted first amino acid position of SEQ ID NO:Y of the secreted portion is identified as "Predicted First AA of Secreted Portion." Finally, the amino acid position of SEQ ID NO:Y of the last amino acid in the open reading frame is identified as "Last AA of ORF."

SEQ ID NO:X and the translated SEQ ID NO:Y are sufficiently accurate and otherwise suitable for a variety of uses well known in the art and described further below. For instance, SEQ ID NO:X is useful for designing nucleic acid hybridization probes that will detect nucleic acid sequences contained in SEQ ID NO:X or the cDNA contained in the deposited clone. These probes will also hybridize to nucleic acid molecules in biological samples, thereby enabling a variety of forensic and diagnostic methods of the invention. Similarly, polypeptides identified from SEQ ID NO:Y may be used to generate antibodies which bind specifically to the secreted proteins encoded by the cDNA clones identified in Table 1.

Nevertheless, DNA sequences generated by sequencing reactions can contain sequencing errors. The errors exist as misidentified nucleotides, or as insertions or deletions of nucleotides in the generated DNA sequence. The erroneously inserted or deleted nucleotides cause frame shifts in the reading frames of the predicted amino acid sequence. In these cases, the predicted amino acid sequence diverges from the actual amino acid sequence, even though the generated DNA sequence may be greater than 99.9% identical to the actual DNA sequence (for example, one base insertion or deletion in an open reading frame of over 1000 bases).

Accordingly, for those applications requiring precision in the nucleotide sequence or the amino acid sequence, the present invention provides not only the generated nucleotide sequence identified as SEQ ID NO:X and the predicted translated amino acid sequence identified as SEQ ID NO:Y, but also a sample of plasmid DNA containing a human cDNA of the invention deposited with the ATCC, as set forth in Table 1. The nucleotide sequence of each deposited clone can readily be determined by sequencing the deposited clone in accordance with known methods. The predicted amino acid sequence can then be verified from such deposits. Moreover, the amino acid sequence of the protein encoded by a particular clone can also be directly determined by peptide sequencing or by expressing the protein in a suitable host cell containing the deposited human cDNA, collecting the protein, and determining its sequence.

The present invention also relates to the genes corresponding to SEQ ID NO:X, SEQ ID NO:Y, or the deposited clone. The corresponding gene can be isolated in accordance with known methods using the sequence information disclosed herein. Such methods include preparing probes or primers from the disclosed sequence and identifying or amplifying the corresponding gene from appropriate sources of genomic material.

Also provided in the present invention are species homologs. Species homologs may be isolated and identified by making suitable probes or primers from the sequences provided herein and screening a suitable nucleic acid source for the desired homologue.

The polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

The polypeptides may be in the form of the secreted protein, including the mature form, or may be a part of a larger protein, such as a fusion protein (see below). It is often advantageous to include an additional amino acid sequence which contains secretory or leader sequences, pro-sequences, sequences which aid in purification, such as multiple histidine residues, or an additional sequence for stability during recombinant production.

The polypeptides of the present invention are preferably provided in an isolated form, and preferably are substantially purified. A recombinantly produced version of a polypeptide, including the secreted polypeptide, can be substantially purified by the one-step method described in Smith and Johnson, Gene 67:31-40 (1988). Polypeptides of the invention also can be purified from natural or recombinant sources using antibodies of the invention raised against the secreted protein in methods which are well known in the art.

### Signal Sequences

Methods for predicting whether a protein has a signal sequence, as well as the cleavage point for that sequence, are available. For instance, the method of McGeoch, Virus Res. 3:271-286 (1985), uses the information from a short N-terminal charged region and a subsequent uncharged region of the complete (uncleaved) protein. The method of von Heinje, Nucleic Acids Res. 14:4683-4690 (1986) uses the information from the residues surrounding the cleavage site, typically residues -13 to +2, where +1 indicates the amino terminus of the secreted protein. The accuracy of predicting the cleavage points of known mammalian secretory proteins for each of these methods is in the range of 75-80%. (von Heinje, supra.) However, the two methods do not always produce the same predicted cleavage point(s) for a given protein.

In the present case, the deduced amino acid sequence of the secreted polypeptide was analyzed by a computer program called SignalP (Henrik Nielsen et al., Protein Engineering 10:1-6 (1997)), which predicts the cellular location of a protein based on the amino acid sequence. As part of this computational prediction of localization, the methods of McGeoch and von Heinje are incorporated. The analysis of the amino acid sequences of the secreted proteins described herein by this program provided the results shown in Table 1.

As one of ordinary skill would appreciate, however, cleavage sites sometimes vary from organism to organism and cannot be predicted with absolute certainty. Accordingly, the present invention provides secreted polypeptides having a sequence shown in SEQ ID NO:Y which have an N-terminus beginning within 5 residues (i.e., + or - 5 residues) of the predicted cleavage point. Similarly, it is also recognized that in some cases, cleavage of the signal sequence from a secreted protein is not entirely uniform, resulting in more than one secreted species. These polypeptides, and the polynucleotides encoding such polypeptides, are contemplated by the present invention.

Moreover, the signal sequence identified by the above analysis may not necessarily predict the naturally occurring signal sequence. For example, the naturally occurring signal sequence may be further upstream from the predicted signal sequence. However, it is likely that the predicted signal sequence will be capable of directing the secreted protein to the ER. These polypeptides, and the polynucleotides encoding such polypeptides, are contemplated by the present invention.

### Polynucleotide and Polypeptide Variants

"Variant" refers to a polynucleotide or polypeptide differing from the polynucleotide or polypeptide of the present invention, but retaining essential properties thereof. Generally, variants are overall closely similar, and, in many regions, identical to the polynucleotide or polypeptide of the present invention.

By a polynucleotide having a nucleotide sequence at least, for example, 95% "identical" to a reference nucleotide sequence of the present invention, it is intended that the nucleotide sequence of the polynucleotide is identical to the reference sequence except that the polynucleotide sequence may include up to five point mutations per each 100 nucleotides of the reference nucleotide sequence encoding the polypeptide. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. The query sequence may be an entire sequence shown inTable 1, the ORF (open reading frame), or any fragement specified as described herein.

As a practical matter, whether any particular nucleic acid molecule or polypeptide is at least 90%, 95%, 96%, 97%, 98% or 99% identical to a nucleotide sequence of the presence invention can be determined conventionally using known computer programs. A preferred method for determing the best overall match between a query sequence (a sequence of the present invention) and a subject sequence, also referred to as a global sequence alignment, can be determined using the FASTDB computer program based on the algorithm of Brutlag et al. (Comp. App. Biosci. (1990) 6:237-245). In a sequence alignment the query and subject sequences are both DNA sequences. An RNA sequence can be compared by converting U's to T's. The result of said global sequence alignment is in percent identity. Preferred parameters used in a FASTDB alignment of DNA sequences to calculate percent identiy are: Matrix=Unitary, k-tuple=4, Mismatch Penalty=1, Joining Penalty=30, Randomization Group Length=0, Cutoff Score=1, Gap Penalty=5, Gap Size Penalty 0.05, Window Size=500 or the lenght of the subject nucleotide sequence, whichever is shorter.

If the subject sequence is shorter than the query sequence because of 5' or 3' deletions, not because of internal deletions, a manual correction must be made to the results. This is becuase the FASTDB program does not account for 5' and 3' truncations of the subject sequence when calculating percent identity. For subject sequences truncated at the 5' or 3' ends, relative to the the query sequence, the percent identity is corrected by calculating the number of bases of the query sequence that are 5' and 3' of the subject sequence, which are not matched/aligned, as a percent of the total bases of the query sequence. Whether a nucleotide is matched/aligned is determined by results of the FASTDB sequence alignment. This percentage is then subtracted from the percent identity, calculated by the above FASTDB program using the specified parameters, to arrive at a final percent identity score. This corrected score is what is used for the purposes of the present invention. Only bases outside the 5' and 3' bases of the subject sequence, as displayed by the FASTDB alignment, which are not matched/aligned with the query sequence, are calculated for the purposes of manually adjusting the percent identity score.

For example, a 90 base subject sequence is aligned to a 100 base query sequence to determine percent identity. The deletions occur at the 5' end of the subject sequence and therefore, the FASTDB alignment does not show a matched/alignement of the first 10 bases at 5' end. The 10 unpaired bases represent 10% of the sequence (number of bases at the 5' and 3' ends not matched/total number of bases in the query sequence) so 10% is subtracted from the percent identity score calculated by the FASTDB program. If the remaining 90 bases were perfectly matched the final percent identity would be 90%. In another example, a 90 base subject sequence is compared with a 100 base query sequence. This time the deletions are internal deletions so that there are no bases on the 5' or 3' of the subject sequence which are not matched/aligned with the query. In this case the percent identity calculated by FASTDB is not manually corrected. Once again, only bases 5' and 3' of the subject sequence which are not matched/aligned with the query sequnce are manually corrected for. No other manual corrections are to made for the purposes of the present invention.

By a polypeptide having an amino acid sequence at least, for example, 95% "identical" to a query amino acid sequence of the present invention, it is intended that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid sequence at least 95% identical to a query amino acid sequence, up to 5% of the amino acid residues in the subject sequence may be inserted, deleted, (indels) or substituted with another amino acid. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

As a practical matter, whether any particular polypeptide is at least 90%, 95%, 96%, 97%, 98% or 99% identical to, for instance, the amino acid sequences shown in Table 1 or to the amino acid sequence encoded by deposited DNA clone can be determined conventionally using known computer programs. A preferred method for determing the best overall match between a query sequence (a sequence of the present invention) and a subject sequence, also referred to as a global sequence alignment, can be determined using the FASTDB computer program based on the algorithm of Brutlag et al. (Comp. App. Biosci. (1990) 6:237-245). In a sequence alignment the query and subject sequences are either both nucleotide sequences or both amino acid sequences. The result of said global sequence alignment is in percent identity. Preferred parameters used in a FASTDB amino acid alignment are: Matrix=PAM 0, k-tuple=2, Mismatch Penalty=1, Joining Penalty=20, Randomization Group Length=0, Cutoff Score=1, Window Size=sequence length, Gap Penalty=5, Gap Size Penalty=0.05, Window Size=500 or the length of the subject amino acid sequence, whichever is shorter.

If the subject sequence is shorter than the query sequence due to N- or C-terminal deletions, not because of internal deletions, a manual correction must be made to the results. This is becuase the FASTDB program does not account for N- and C-terminal truncations of the subject sequence when calculating global percent identity. For subject sequences truncated at the N- and C-termini, relative to the the query sequence, the percent identity is corrected by calculating the number of residues of the query sequence that are N- and C-terminal of the subject sequence, which are not matched/aligned with a corresponding subject residue, as a percent of the total bases of the query sequence. Whether a residue is matched/aligned is determined by results of the FASTDB sequence alignment. This percentage is then subtracted from the percent identity, calculated by the above FASTDB program using the specified parameters, to arrive at a final percent identity score. This final percent identity score is what is used for the purposes of the present invention. Only residues to the N- and C-termini of the subject sequence, which are not matched/aligned with the query sequence, are considered for the purposes of manually adjusting the percent identity score. That is, only query residue positions outside the farthest N- and C-terminal residues of the subject sequence.

For example, a 90 amino acid residue subject sequence is aligned with a 100 residue query sequence to determine percent identity. The deletion occurs at the N-terminus of the subject sequence and therefore, the FASTDB alignment does not show a matching/alignment of the first 10 residues at the N-terminus. The 10 unpaired residues represent 10% of the sequence (number of residues at the N- and C- termini not matched/total number of residues in the query sequence) so 10% is subtracted from the percent identity score calculated by the FASTDB program. If the remaining 90 residues were perfectly matched the final percent identity would be 90%. In another example, a 90 residue subject sequence is compared with a 100 residue query sequence. This time the deletions are internal deletions so there are no residues at the N- or C-termini of the subject sequence which are not matched/aligned with the query. In this case the percent identity calculated by FASTDB is not manually corrected. Once again, only residue positions outside the N- and C-terminal ends of the subject sequence, as displayed in the FASTDB alignment, which are not matched/aligned with the query sequnce are manually corrected for. No other manual corrections are to made for the purposes of the present invention.

The variants may contain alterations in the coding regions, non-coding regions, or both. Especially preferred are polynucleotide variants containing alterations which produce silent substitutions, additions, or deletions, but do not alter the properties or activities of the encoded polypeptide. Nucleotide variants produced by silent substitutions due to the degeneracy of the genetic code are preferred. Moreover, variants in which 5-10, 1-5, or 1-2 amino acids are substituted, deleted, or added in any combination are also preferred. Polynucleotide variants can be produced for a variety of reasons, e.g., to optimize codon expression for a particular host (change codons in the human mRNA to those preferred by a bacterial host such as E. coli).

Naturally occurring variants are called "allelic variants," and refer to one of several alternate forms of a gene occupying a given locus on a chromosome of an organism. (Genes II, Lewin, B., ed., John Wiley & Sons, New York (1985).) These allelic variants can vary at either the polynucleotide and/or polypeptide level. Alternatively, non-naturally occurring variants may be produced by mutagenesis techniques or by direct synthesis.

Using known methods of protein engineering and recombinant DNA technology, variants may be generated to improve or alter the characteristics of the polypeptides of the present invention. For instance, one or more amino acids can be deleted from the N-terminus or C-terminus of the secreted protein without substantial loss of biological function. The authors of Ron et al., J. Biol. Chem. 268: 2984-2988 (1993), reported variant KGF proteins having heparin binding activity even after deleting 3, 8, or 27 amino-terminal amino acid residues. Similarly, Interferon gamma exhibited up to ten times higher activity after deleting 8-10 amino acid residues from the carboxy terminus of this protein. (Dobeli et al., J. Biotechnology 7:199-216 (1988).)

Moreover, ample evidence demonstrates that variants often retain a biological activity similar to that of the naturally occurring protein. For example, Gayle and coworkers (J. Biol. Chem 268:22105-22111 (1993)) conducted extensive mutational analysis of human cytokine IL-1a. They used random mutagenesis to generate over 3,500 individual IL-1a mutants that averaged 2.5 amino acid changes per variant over the entire length of the molecule. Multiple mutations were examined at every possible amino acid position. The investigators found that "[m]ost of the molecule could be altered with little effect on either [binding or biological activity]." (See, Abstract.) In fact, only 23 unique amino acid sequences, out of more than 3,500 nucleotide sequences examined, produced a protein that significantly differed in activity from wild-type.

Furthermore, even if deleting one or more amino acids from the N-terminus or C-terminus of a polypeptide results in modification or loss of one or more biological functions, other biological activities may still be retained. For example, the ability of a deletion variant to induce and/or to bind antibodies which recognize the secreted form will likely be retained when less than the majority of the residues of the secreted form are removed from the N-terminus or C-terminus. Whether a particular polypeptide lacking N- or C-terminal residues of a protein retains such immunogenic activities can readily be determined by routine methods described herein and otherwise known in the art.

Thus, the invention further includes polypeptide variants which show substantial biological activity. Such variants include deletions, insertions, inversions, repeats, and substitutions selected according to general rules known in the art so as have little effect on activity. For example, guidance concerning how to make phenotypically silent amino acid substitutions is provided in Bowie, J. U. et al., Science 247:1306-1310 (1990), wherein the authors indicate that there are two main strategies for studying the tolerance of an amino acid sequence to change.

The first strategy exploits the tolerance of amino acid substitutions by natural selection during the process of evolution. By comparing amino acid sequences in different species, conserved amino acids can be identified. These conserved amino acids are likely important for protein function. In contrast, the amino acid positions where substitutions have been tolerated by natural selection indicates that these positions are not critical for protein function. Thus, positions tolerating amino acid substitution could be modified while still maintaining biological activity of the protein.

The second strategy uses genetic engineering to introduce amino acid changes at specific positions of a cloned gene to identify regions critical for protein function. For example, site directed mutagenesis or alanine-scanning mutagenesis (introduction of single alanine mutations at every residue in the molecule) can be used. (Cunningham and Wells, Science 244:1081-1085 (1989).) The resulting mutant molecules can then be tested for biological activity.

As the authors state, these two strategies have revealed that proteins are surprisingly tolerant of amino acid substitutions. The authors further indicate which amino acid changes are likely to be permissive at certain amino acid positions in the protein. For example, most buried (within the tertiary structure of the protein) amino acid residues require nonpolar side chains, whereas few features of surface side chains are generally conserved. Moreover, tolerated conservative amino acid substitutions involve replacement of the aliphatic or hydrophobic amino acids Ala, Val, Leu and Ile; replacement of the hydroxyl residues Ser and Thr; replacement of the acidic residues Asp and Glu; replacement of the amide residues Asn and Gln, replacement of the basic residues Lys, Arg, and His; replacement of the aromatic residues Phe, Tyr, and Trp, and replacement of the small-sized amino acids Ala, Ser, Thr, Met, and Gly.

Besides conservative amino acid substitution, variants of the present invention include (i) substitutions with one or more of the non-conserved amino acid residues, where the substituted amino acid residues may or may not be one encoded by the genetic code, or (ii) substitution with one or more of amino acid residues having a substituent group, or (iii) fusion of the mature polypeptide with another compound, such as a compound to increase the stability and/or solubility of the polypeptide (for example, polyethylene glycol), or (iv) fusion of the polypeptide with additional amino acids, such as an IgG Fc fusion region peptide, or leader or secretory sequence, or a sequence facilitating purification. Such variant polypeptides are deemed to be within the scope of those skilled in the art from the teachings herein.

For example, polypeptide variants containing amino acid substitutions of charged amino acids with other charged or neutral amino acids may produce proteins with improved characteristics, such as less aggregation. Aggregation of pharmaceutical formulations both reduces activity and increases clearance due to the aggregate's immunogenic activity. (Pinckard et al., Clin. Exp. Immunol. 2:331-340 (1967); Robbins et al., Diabetes 36: 838-845 (1987); Cleland et al., Crit. Rev. Therapeutic Drug Carrier Systems 10:307-377 (1993).)

### Polynucleotide and Polypeptide Fragments

In the present invention, a "polynucleotide fragment" refers to a short polynucleotide having a nucleic acid sequence contained in the deposited clone or shown in SEQ ID NO:X. The short nucleotide fragments are preferably at least about 15 nt, and more preferably at least about 20 nt, still more preferably at least about 30 nt, and even more preferably, at least about 40 nt in length. A fragment "at least 20 nt in length," for example, is intended to include 20 or more contiguous bases from the cDNA sequence contained in the deposited clone or the nucleotide sequence shown in SEQ ID NO:X. These nucleotide fragments are useful as diagnostic probes and primers as discussed herein. Of course, larger fragments (e.g., 50, 150, 500, 600, 2000 nucleotides) are preferred.

Moreover, representative examples of polynucleotide fragments of the invention, include, for example, fragments having a sequence from about nucleotide number 1-50, 51-100, 101-150, 151-200, 201-250, 251-300, 301-350, 351-400, 401-450, 451-500, 501-550, 551-600, 651-700, 701-750, 751-800, 800-850, 851-900, 901-950, 951-1000, 1001-1050, 1051-1100, 1101-1150, 1151-1200, 1201-1250, 1251-1300, 1301-1350, 1351-1400, 1401-1450, 1451-1500, 1501-1550, 1551-1600, 1601-1650, 1651-1700, 1701-1750, 1751-1800, 1801-1850, 1851-1900, 1901-1950, 1951-2000, or 2001 to the end of SEQ ID NO:X or the cDNA contained in the deposited clone. In this context "about" includes the particularly recited ranges, larger or smaller by several (5, 4, 3, 2, or 1) nucleotides, at either terminus or at both termini. Preferably, these fragments encode a polypeptide which has biological activity. More preferably, these polynucleotides can be used as probes or primers as discussed herein.

In the present invention, a "polypeptide fragment" refers to a short amino acid sequence contained in SEQ ID NO:Y or encoded by the cDNA contained in the deposited clone. Protein fragments may be "free-standing," or comprised within a larger polypeptide of which the fragment forms a part or region, most preferably as a single continuous region. Representative examples of polypeptide fragments of the invention, include, for example, fragments from about amino acid number 1-20, 21-40, 41-60, 61-80, 81-100, 102-120, 121-140, 141-160, or 161 to the end of the coding region. Moreover, polypeptide fragments can be about 20, 30, 40, 50, 60, 70, 80, 90, 100, 110, 120, 130, 140, or 150 amino acids in length. In this context "about" includes the particularly recited ranges, larger or smaller by several (5, 4, 3, 2, or 1) amino acids, at either extreme or at both extremes.

Preferred polypeptide fragments include the secreted protein as well as the mature form. Further preferred polypeptide fragments include the secreted protein or the mature form having a continuous series of deleted residues from the amino or the carboxy terminus, or both. For example, any number of amino acids, ranging from 1-60, can be deleted from the amino terminus of either the secreted polypeptide or the mature form. Similarly, any number of amino acids, ranging from 1-30, can be deleted from the carboxy terminus of the secreted protein or mature form. Furthermore, any combination of the above amino and carboxy terminus deletions are preferred. Similarly, polynucleotide fragments encoding these polypeptide fragments are also preferred.

Particularly, N-terminal deletions of the polypeptide of the present invention can be described by the general formula m-p, where p is the total number of amino acids in the polypeptide and m is an integer from 2 to (p-1), and where both of these integers (m & p) correspond to the position of the amino acid residue identified in SEQ ID NO:Y.

Moreover, C-terminal deletions of the polypeptide of the present invention can also be described by the general formula 1-n, where n is an integer from 2 to (p-1), and again where these integers (n & p) correspond to the position of the amino acid residue identified in SEQ ID NO:Y.

The invention also provides polypeptides having one or more amino acids deleted from both the amino and the carboxyl termini, which may be described generally as having residues m-n of SEQ ID NO:Y, where m and n are integers as described above.

Also preferred are polypeptide and polynucleotide fragments characterized by structural or functional domains, such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Polypeptide fragments of SEQ ID NO:Y falling within conserved domains are specifically contemplated by the present invention. Moreover, polynucleotide fragments encoding these domains are also contemplated.

Other preferred fragments are biologically active fragments. Biologically active fragments are those exhibiting activity similar, but not necessarily identical, to an activity of the polypeptide of the present invention. The biological activity of the fragments may include an improved desired activity, or a decreased undesirable activity.

### Epitopes & Antibodies

In the present invention, "epitopes" refer to polypeptide fragments having antigenic or immunogenic activity in an animal, especially in a human. A preferred embodiment of the present invention relates to a polypeptide fragment comprising an epitope, as well as the polynucleotide encoding this fragment. A region of a protein molecule to which an antibody can bind is defined as an "antigenic epitope." In contrast, an "immunogenic epitope" is defined as a part of a protein that elicits an antibody response. (See, for instance, Geysen et al., Proc. Natl. Acad. Sci. USA 81:3998- 4002 (1983).)

Fragments which function as epitopes may be produced by any conventional means. (See, e.g., Houghten, R. A., Proc. Natl. Acad. Sci. USA 82:5131-5135 (1985) further described in U.S. Patent No. 4,631,211.)

In the present invention, antigenic epitopes preferably contain a sequence of at least seven, more preferably at least nine, and most preferably between about 15 to about 30 amino acids. Antigenic epitopes are useful to raise antibodies, including monoclonal antibodies, that specifically bind the epitope. (See, for instance, Wilson et al., Cell 37:767-778 (1984); Sutcliffe, J. G. et al., Science 219:660-666 (1983).)

Similarly, immunogenic epitopes can be used to induce antibodies according to methods well known in the art. (See, for instance, Sutcliffe et al., supra; Wilson et al., supra; Chow, M. et al., Proc. Natl. Acad. Sci. USA 82:910-914; and Bittle, F. J. et al., J. Gen. Virol. 66:2347-2354 (1985).) A preferred immunogenic epitope includes the secreted protein. The immunogenic epitopes may be presented together with a carrier protein, such as an albumin, to an animal system (such as rabbit or mouse) or, if it is long enough (at least about 25 amino acids), without a carrier. However, immunogenic epitopes comprising as few as 8 to 10 amino acids have been shown to be sufficient to raise antibodies capable of binding to, at the very least, linear epitopes in a denatured polypeptide (e.g., in Western blotting.)

As used herein, the term "antibody" (Ab) or "monoclonal antibody" (Mab) is meant to include intact molecules as well as antibody fragments (such as, for example, Fab and F(ab')2 fragments) which are capable of specifically binding to protein. Fab and F(ab')2 fragments lack the Fc fragment of intact antibody, clear more rapidly from the circulation, and may have less non-specific tissue binding than an intact antibody. (Wahl et al., J. Nucl. Med. 24:316-325 (1983).) Thus, these fragments are preferred, as well as the products of a FAB or other immunoglobulin expression library. Moreover, antibodies of the present invention include chimeric, single chain, and humanized antibodies.

### Fusion Proteins

Any polypeptide of the present invention can be used to generate fusion proteins. For example, the polypeptide of the present invention, when fused to a second protein, can be used as an antigenic tag. Antibodies raised against the polypeptide of the present invention can be used to indirectly detect the second protein by binding to the polypeptide. Moreover, because secreted proteins target cellular locations based on trafficking signals, the polypeptides of the present invention can be used as targeting molecules once fused to other proteins.

Examples of domains that can be fused to polypeptides of the present invention include not only heterologous signal sequences, but also other heterologous functional regions. The fusion does not necessarily need to be direct, but may occur through linker sequences.

Moreover, fusion proteins may also be engineered to improve characteristics of the polypeptide of the present invention. For instance, a region of additional amino acids, particularly charged amino acids, may be added to the N-terminus of the polypeptide to improve stability and persistence during purification from the host cell or subsequent handling and storage. Also, peptide moieties may be added to the polypeptide to facilitate purification. Such regions may be removed prior to final preparation of the polypeptide. The addition of peptide moieties to facilitate handling of polypeptides are familiar and routine techniques in the art.

Moreover, polypeptides of the present invention, including fragments, and specifically epitopes, can be combined with parts of the constant domain of immunoglobulins (IgG), resulting in chimeric polypeptides. These fusion proteins facilitate purification and show an increased half-life in vivo. One reported example describes chimeric proteins consisting of the first two domains of the human CD4-polypeptide and various domains of the constant regions of the heavy or light chains of mammalian immunoglobulins. (EP A 394,827; Traunecker et al., Nature 331:84-86 (1988).) Fusion proteins having disulfide-linked dimeric structures (due to the IgG) can also be more efficient in binding and neutralizing other molecules, than the monomeric secreted protein or protein fragment alone. (Fountoulakis et al., J. Biochem. 270:3958-3964 (1995).)

Similarly, EP-A-O 464 533 (Canadian counterpart 2045869) discloses fusion proteins comprising various portions of constant region of immunoglobulin molecules together with another human protein or part thereof. In many cases, the Fc part in a fusion protein is beneficial in therapy and diagnosis, and thus can result in, for example, improved pharmacokinetic properties. (EP-A 0232 262.) Alternatively, deleting the Fc part after the fusion protein has been expressed, detected, and purified, would be desired. For example, the Fc portion may hinder therapy and diagnosis if the fusion protein is used as an antigen for immunizations. In drug discovery, for example, human proteins, such as hIL-5, have been fused with Fc portions for the purpose of high-throughput screening assays to identify antagonists of hIL-5. (See, D. Bennett et al., J. Molecular Recognition 8:52-58 (1995); K. Johanson et al., J. Biol. Chem. 270:9459-9471 (1995).)

Moreover, the polypeptides of the present invention can be fused to marker sequences, such as a peptide which facilitates purification of the fused polypeptide. In preferred embodiments, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz et al., Proc. Natl. Acad. Sci. USA 86:821-824 (1989), for instance, hexa-histidine provides for convenient purification of the fusion protein. Another peptide tag useful for purification, the "HA" tag, corresponds to an epitope derived from the influenza hemagglutinin protein. (Wilson et al., Cell 37:767 (1984).)

Thus, any of these above fusions can be engineered using the polynucleotides or the polypeptides of the present invention.

### Vectors, Host Cells, and Protein Production

The present invention also relates to vectors containing the polynucleotide of the present invention, host cells, and the production of polypeptides by recombinant techniques. The vector may be, for example, a phage, plasmid, viral, or retroviral vector. Retroviral vectors may be replication competent or replication defective. In the latter case, viral propagation generally will occur only in complementing host cells.

The polynucleotides may be joined to a vector containing a selectable marker for propagation in a host. Generally, a plasmid vector is introduced in a precipitate, such as a calcium phosphate precipitate, or in a complex with a charged lipid. If the vector is a virus, it may be packaged in vitro using an appropriate packaging cell line and then transduced into host cells.

The polynucleotide insert should be operatively linked to an appropriate promoter, such as the phage lambda PL promoter, the E. coli lac, trp, phoA and tac promoters, the SV40 early and late promoters and promoters of retroviral LTRs, to name a few. Other suitable promoters will be known to the skilled artisan. The expression constructs will further contain sites for transcription initiation, termination, and, in the transcribed region, a ribosome binding site for translation. The coding portion of the transcripts expressed by the constructs will preferably include a translation initiating codon at the beginning and a termination codon (UAA, UGA or UAG) appropriately positioned at the end of the polypeptide to be translated.

As indicated, the expression vectors will preferably include at least one selectable marker. Such markers include dihydrofolate reductase, G418 or neomycin resistance for eukaryotic cell culture and tetracycline, kanamycin or ampicillin resistance genes for culturing in E. coli and other bacteria. Representative examples of appropriate hosts include, but are not limited to, bacterial cells, such as E. coli, Streptomyces and Salmonella typhimurium cells; fungal cells, such as yeast cells; insect cells such as Drosophila S2 and Spodoptera Sf9 cells; animal cells such as CHO, COS, 293, and Bowes melanoma cells; and plant cells. Appropriate culture mediums and conditions for the above-described host cells are known in the art.

Among vectors preferred for use in bacteria include pQE70, pQE60 and pQE-9, available from QIAGEN, Inc.; pBluescript vectors, Phagescript vectors, pNH8A, pNH16a, pNH 18A, pNH46A, available from Stratagene Cloning Systems, Inc.; and ptrc99a, pKK223-3, pKK233-3, pDR540, pRIT5 available from Pharmacia Biotech, Inc. Among preferred eukaryotic vectors are pWLNEO, pSV2CAT, pOG44, pXT1 and pSG available from Stratagene; and pSVK3, pBPV, pMSG and pSVL available from Pharmacia. Other suitable vectors will be readily apparent to the skilled artisan.

Introduction of the construct into the host cell can be effected by calcium phosphate transfection, DEAE-dextran mediated transfection, cationic lipid-mediated transfection, electroporation, transduction, infection, or other methods. Such methods are described in many standard laboratory manuals, such as Davis et al., Basic Methods In Molecular Biology (1986). It is specifically contemplated that the polypeptides of the present invention may in fact be expressed by a host cell lacking a recombinant vector.

A polypeptide of this invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification.

Polypeptides of the present invention, and preferably the secreted form, can also be recovered from: products purified from natural sources, including bodily fluids, tissues and cells, whether directly isolated or cultured; products of chemical synthetic procedures; and products produced by recombinant techniques from a prokaryotic or eukaryotic host, including, for example, bacterial, yeast, higher plant, insect, and mammalian cells. Depending upon the host employed in a recombinant production procedure, the polypeptides of the present invention may be glycosylated or may be non-glycosylated. In addition, polypeptides of the invention may also include an initial modified methionine residue, in some cases as a result of host-mediated processes. Thus, it is well known in the art that the N-terminal methionine encoded by the translation initiation codon generally is removed with high efficiency from any protein after translation in all eukaryotic cells. While the N-terminal methionine on most proteins also is efficiently removed in most prokaryotes, for some proteins, this prokaryotic removal process is inefficient, depending on the nature of the amino acid to which the N-terminal methionine is covalently linked.

### Uses of the Polynucleotides

Each of the polynucleotides identified herein can be used in numerous ways as reagents. The following description should be considered exemplary and utilizes known techniques.

The polynucleotides of the present invention are useful for chromosome identification. There exists an ongoing need to identify new chromosome markers, since few chromosome marking reagents, based on actual sequence data (repeat polymorphisms), are presently available. Each polynucleotide of the present invention can be used as a chromosome marker.

Briefly, sequences can be mapped to chromosomes by preparing PCR primers (preferably 15-25 bp) from the sequences shown in SEQ ID NO:X. Primers can be selected using computer analysis so that primers do not span more than one predicted exon in the genomic DNA. These primers are then used for PCR screening of somatic cell hybrids containing individual human chromosomes. Only those hybrids containing the human gene corresponding to the SEQ ID NO:X will yield an amplified fragment.

Similarly, somatic hybrids provide a rapid method of PCR mapping the polynucleotides to particular chromosomes. Three or more clones can be assigned per day using a single thermal cycler. Moreover, sublocalization of the polynucleotides can be achieved with panels of specific chromosome fragments. Other gene mapping strategies that can be used include in situ hybridization, prescreening with labeled flow-sorted chromosomes, and preselection by hybridization to construct chromosome specific-cDNA libraries.

Precise chromosomal location of the polynucleotides can also be achieved using fluorescence in situ hybridization (FISH) of a metaphase chromosomal spread. This technique uses polynucleotides as short as 500 or 600 bases; however, polynucleotides 2,000-4,000 bp are preferred. For a review of this technique, see Verma et al., "Human Chromosomes: a Manual of Basic Techniques," Pergamon Press, New York (1988).

For chromosome mapping, the polynucleotides can be used individually (to mark a single chromosome or a single site on that chromosome) or in panels (for marking multiple sites and/or multiple chromosomes). Preferred polynucleotides correspond to the noncoding regions of the cDNAs because the coding sequences are more likely conserved within gene families, thus increasing the chance of cross hybridization during chromosomal mapping.

Once a polynucleotide has been mapped to a precise chromosomal location, the physical position of the polynucleotide can be used in linkage analysis. Linkage analysis establishes coinheritance between a chromosomal location and presentation of a particular disease. (Disease mapping data are found, for example, in V. McKusick, Mendelian Inheritance in Man (available on line through Johns Hopkins University Welch Medical Library).) Assuming 1 megabase mapping resolution and one gene per 20 kb, a cDNA precisely localized to a chromosomal region associated with the disease could be one of 50-500 potential causative genes.

Thus, once coinheritance is established, differences in the polynucleotide and the corresponding gene between affected and unaffected individuals can be examined. First, visible structural alterations in the chromosomes, such as deletions or translocations, are examined in chromosome spreads or by PCR. If no structural alterations exist, the presence of point mutations are ascertained. Mutations observed in some or all affected individuals, but not in normal individuals, indicates that the mutation may cause the disease. However, complete sequencing of the polypeptide and the corresponding gene from several normal individuals is required to distinguish the mutation from a polymorphism. If a new polymorphism is identified, this polymorphic polypeptide can be used for further linkage analysis.

Furthermore, increased or decreased expression of the gene in affected individuals as compared to unaffected individuals can be assessed using polynucleotides of the present invention. Any of these alterations (altered expression, chromosomal rearrangement, or mutation) can be used as a diagnostic or prognostic marker.

In addition to the foregoing, a polynucleotide can be used to control gene expression through triple helix formation or antisense DNA or RNA. Both methods rely on binding of the polynucleotide to DNA or RNA. For these techniques, preferred polynucleotides are usually 20 to 40 bases in length and complementary to either the region of the gene involved in transcription (triple helix - see Lee et al., Nucl. Acids Res. 6:3073 (1979); Cooney et al., Science 241:456 (1988); and Dervan et al., Science 251:1360 (1991)) or to the mRNA itself (antisense - Okano, J. Neurochem. 56:560 (1991); Oligodeoxy-nucleotides as Antisense Inhibitors of Gene Expression, CRC Press, Boca Raton, FL (1988).) Triple helix formation optimally results in a shut-off of RNA transcription from DNA, while antisense RNA hybridization blocks translation of an mRNA molecule into polypeptide. Both techniques are effective in model systems, and the information disclosed herein can be used to design antisense or triple helix polynucleotides in an effort to treat disease.

Polynucleotides of the present invention are also useful in gene therapy. One goal of gene therapy is to insert a normal gene into an organism having a defective gene, in an effort to correct the genetic defect. The polynucleotides disclosed in the present invention offer a means of targeting such genetic defects in a highly accurate manner. Another goal is to insert a new gene that was not present in the host genome, thereby producing a new trait in the host cell.

The polynucleotides are also useful for identifying individuals from minute biological samples. The United States military, for example, is considering the use of restriction fragment length polymorphism (RFLP) for identification of its personnel. In this technique, an individual's genomic DNA is digested with one or more restriction enzymes, and probed on a Southern blot to yield unique bands for identifying personnel. This method does not suffer from the current limitations of "Dog Tags" which can be lost, switched, or stolen, making positive identification difficult. The polynucleotides of the present invention can be used as additional DNA markers for RFLP.

The polynucleotides of the present invention can also be used as an alternative to RFLP, by determining the actual base-by-base DNA sequence of selected portions of an individual's genome. These sequences can be used to prepare PCR primers for amplifying and isolating such selected DNA, which can then be sequenced. Using this technique, individuals can be identified because each individual will have a unique set of DNA sequences. Once an unique ID database is established for an individual, positive identification of that individual, living or dead, can be made from extremely small tissue samples.

Forensic biology also benefits from using DNA-based identification techniques as disclosed herein. DNA sequences taken from very small biological samples such as tissues, e.g., hair or skin, or body fluids, e.g., blood, saliva, semen, etc., can be amplified using PCR. In one prior art technique, gene sequences amplified from polymorphic loci, such as DQa class II HLA gene, are used in forensic biology to identify individuals. (Erlich, H., PCR Technology, Freeman and Co. (1992).) Once these specific polymorphic loci are amplified, they are digested with one or more restriction enzymes, yielding an identifying set of bands on a Southern blot probed with DNA corresponding to the DQa class II HLA gene. Similarly, polynucleotides of the present invention can be used as polymorphic markers for forensic purposes.

There is also a need for reagents capable of identifying the source of a particular tissue. Such need arises, for example, in forensics when presented with tissue of unknown origin. Appropriate reagents can comprise, for example, DNA probes or primers specific to particular tissue prepared from the sequences of the present invention. Panels of such reagents can identify tissue by species and/or by organ type. In a similar fashion, these reagents can be used to screen tissue cultures for contamination.

In the very least, the polynucleotides of the present invention can be used as molecular weight markers on Southern gels, as diagnostic probes for the presence of a specific mRNA in a particular cell type, as a probe to "subtract-out" known sequences in the process of discovering novel polynucleotides, for selecting and making oligomers for attachment to a "gene chip" or other support, to raise anti-DNA antibodies using DNA immunization techniques, and as an antigen to elicit an immune response.

### Uses of the Polypeptides

Each of the polypeptides identified herein can be used in numerous ways. The following description should be considered exemplary and utilizes known techniques.

A polypeptide of the present invention can be used to assay protein levels in a biological sample using antibody-based techniques. For example, protein expression in tissues can be studied with classical immunohistological methods. (Jalkanen, M., et al., J. Cell. Biol. 101:976-985 (1985); Jalkanen, M., et al., J. Cell. Biol. 105:3087-3096 (1987).) Other antibody-based methods useful for detecting protein gene expression include immunoassays, such as the enzyme linked immunosorbent assay (ELISA) and the radioimmunoassay (RIA). Suitable antibody assay labels are known in the art and include enzyme labels, such as, glucose oxidase, and radioisotopes, such as iodine (125I, 121I), carbon (14C), sulfur (35S), tritium (3H), indium (112In), and technetium (99mTc), and fluorescent labels, such as fluorescein and rhodamine, and biotin.

In addition to assaying secreted protein levels in a biological sample, proteins can also be detected in vivo by imaging. Antibody labels or markers for in vivo imaging of protein include those detectable by X-radiography, NMR or ESR. For X-radiography, suitable labels include radioisotopes such as barium or cesium, which emit detectable radiation but are not overtly harmful to the subject. Suitable markers for NMR and ESR include those with a detectable characteristic spin, such as deuterium, which may be incorporated into the antibody by labeling of nutrients for the relevant hybridoma.

A protein-specific antibody or antibody fragment which has been labeled with an appropriate detectable imaging moiety, such as a radioisotope (for example, 131I, 112In, 99mTc), a radio-opaque substance, or a material detectable by nuclear magnetic resonance, is introduced (for example, parenterally, subcutaneously, or intraperitoneally) into the mammal. It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of 99mTc. The labeled antibody or antibody fragment will then preferentially accumulate at the location of cells which contain the specific protein. In vivo tumor imaging is described in S.W. Burchiel et al., "Immunopharmacokinetics of Radiolabeled Antibodies and Their Fragments." (Chapter 13 in Tumor Imaging: The Radiochemical Detection of Cancer, S.W. Burchiel and B. A. Rhodes, eds., Masson Publishing Inc. ( 1982).)

Thus, the invention provides a diagnostic method of a disorder, which involves (a) assaying the expression of a polypeptide of the present invention in cells or body fluid of an individual; (b) comparing the level of gene expression with a standard gene expression level, whereby an increase or decrease in the assayed polypeptide gene expression level compared to the standard expression level is indicative of a disorder.

Moreover, polypeptides of the present invention can be used to treat disease. For example, patients can be administered a polypeptide of the present invention in an effort to replace absent or decreased levels of the polypeptide (e.g., insulin), to supplement absent or decreased levels of a different polypeptide (e.g., hemoglobin S for hemoglobin B), to inhibit the activity of a polypeptide (e.g., an oncogene), to activate the activity of a polypeptide (e.g., by binding to a receptor), to reduce the activity of a membrane bound receptor by competing with it for free ligand (e.g., soluble TNF receptors used in reducing inflammation), or to bring about a desired response (e.g., blood vessel growth).

Similarly; antibodies directed to a polypeptide of the present invention can also be used to treat disease. For example, administration of an antibody directed to a polypeptide of the present invention can bind and reduce overproduction of the polypeptide. Similarly, administration of an antibody can activate the polypeptide, such as by binding to a polypeptide bound to a membrane (receptor).

At the very least, the polypeptides of the present invention can be used as molecular weight markers on SDS-PAGE gels or on molecular sieve gel filtration columns using methods well known to those of skill in the art. Polypeptides can also be used to raise antibodies, which in turn are used to measure protein expression from a recombinant cell, as a way of assessing transformation of the host cell. Moreover, the polypeptides of the present invention can be used to test the following biological activities.

### Biological Activities

The polynucleotides and polypeptides of the present invention can be used in assays to test for one or more biological activities. If these polynucleotides and polypeptides do exhibit activity in a particular assay, it is likely that these molecules may be involved in the diseases associated with the biological activity. Thus, the polynucleotides and polypeptides could be used to treat the associated disease.

### Immune Activity

A polypeptide or polynucleotide of the present invention may be useful in treating deficiencies or disorders of the immune system, by activating or inhibiting the proliferation, differentiation, or mobilization (chemotaxis) of immune cells. Immune cells develop through a process called hematopoiesis, producing myeloid (platelets, red blood cells, neutrophils, and macrophages) and lymphoid (B and T lymphocytes) cells from pluripotent stem cells. The etiology of these immune deficiencies or disorders may be genetic, somatic, such as cancer or some autoimmune disorders, acquired (e.g., by chemotherapy or toxins), or infectious. Moreover, a polynucleotide or polypeptide of the present invention can be used as a marker or detector of a particular immune system disease or disorder.

A polynucleotide or polypeptide of the present invention may be useful in treating or detecting deficiencies or disorders of hematopoietic cells. A polypeptide or polynucleotide of the present invention could be used to increase differentiation and proliferation of hematopoietic cells, including the pluripotent stem cells, in an effort to treat those disorders associated with a decrease in certain (or many) types hematopoietic cells. Examples of immunologic deficiency syndromes include, but are not limited to: blood protein disorders (e.g. agammaglobulinemia, dysgammaglobulinemia), ataxia telangiectasia, common variable immunodeficiency, Digeorge Syndrome, HIV infection, HTLV-BLV infection, leukocyte adhesion deficiency syndrome, lymphopenia, phagocyte bactericidal dysfunction, severe combined immunodeficiency (SCIDs), Wiskott-Aldrich Disorder, anemia, thrombocytopenia, or hemoglobinuria.

Moreover, a polypeptide or polynucleotide of the present invention could also be used to modulate hemostatic (the stopping of bleeding) or thrombolytic activity (clot formation). For example, by increasing hemostatic or thrombolytic activity, a polynucleotide or polypeptide of the present invention could be used to treat blood coagulation disorders (e.g., afibrinogenemia, factor deficiencies), blood platelet disorders (e.g. thrombocytopenia), or wounds resulting from trauma, surgery, or other causes. Alternatively, a polynucleotide or polypeptide of the present invention that can decrease hemostatic or thrombolytic activity could be used to inhibit or dissolve clotting. These molecules could be important in the treatment of heart attacks (infarction), strokes, or scarring.

A polynucleotide or polypeptide of the present invention may also be useful in treating or detecting autoimmune disorders. Many autoimmune disorders result from inappropriate recognition of self as foreign material by immune cells. This inappropriate recognition results in an immune response leading to the destruction of the host tissue. Therefore, the administration of a polypeptide or polynucleotide of the present invention that inhibits an immune response, particularly the proliferation, differentiation, or chemotaxis of T-cells, may be an effective therapy in preventing autoimmune disorders.

Examples of autoimmune disorders that can be treated or detected by the present invention include, but are not limited to: Addison's Disease, hemolytic anemia, antiphospholipid syndrome, rheumatoid arthritis, dermatitis, allergic encephalomyelitis, glomerulonephritis, Goodpasture's Syndrome, Graves' Disease, Multiple Sclerosis, Myasthenia Gravis, Neuritis, Ophthalmia, Bullous Pemphigoid, Pemphigus, Polyendocrinopathies, Purpura, Reiter's Disease, Stiff-Man Syndrome, Autoimmune Thyroiditis, Systemic Lupus Erythematosus, Autoimmune Pulmonary Inflammation, Guillain-Barre Syndrome, insulin dependent diabetes mellitis, and autoimmune inflammatory eye disease.

Similarly, allergic reactions and conditions, such as asthma (particularly allergic asthma) or other respiratory problems, may also be treated by a polypeptide or polynucleotide of the present invention. Moreover, these molecules can be used to treat anaphylaxis, hypersensitivity to an antigenic molecule, or blood group incompatibility.

A polynucleotide or polypeptide of the present invention may also be used to treat and/or prevent organ rejection or graft-versus-host disease (GVHD). Organ rejection occurs by host immune cell destruction of the transplanted tissue through an immune response. Similarly, an immune response is also involved in GVHD, but, in this case, the foreign transplanted immune cells destroy the host tissues. The administration of a polypeptide or polynucleotide of the present invention that inhibits an immune response, particularly the proliferation, differentiation, or chemotaxis of T-cells, may be an effective therapy in preventing organ rejection or GVHD.

Similarly, a polypeptide or polynucleotide of the present invention may also be used to modulate inflammation. For example, the polypeptide or polynucleotide may inhibit the proliferation and differentiation of cells involved in an inflammatory response. These molecules can be used to treat inflammatory conditions, both chronic and acute conditions, including inflammation associated with infection (e.g.. septic shock, sepsis, or systemic inflammatory response syndrome (SIRS)), ischemia-reperfusion injury, endotoxin lethality, arthritis, complement-mediated hyperacute rejection, nephritis, cytokine or chemokine induced lung injury, inflammatory bowel disease, Crohn's disease, or resulting from over production of cytokines (e.g., TNF or IL-1.)

### Hyperproliferative Disorders

A polypeptide or polynucleotide can be used to treat or detect hyperproliferative disorders, including neoplasms. A polypeptide or polynucleotide of the present invention may inhibit the proliferation of the disorder through direct or indirect interactions. Alternatively, a polypeptide or polynucleotide of the present invention may proliferate other cells which can inhibit the hyperproliferative disorder.

For example, by increasing an immune response, particularly increasing antigenic qualities of the hyperproliferative disorder or by proliferating, differentiating, or mobilizing T-cells, hyperproliferative disorders can be treated. This immune response may be increased by either enhancing an existing immune response, or by initiating a new immune response. Alternatively, decreasing an immune response may also be a method of treating hyperproliferative disorders, such as a chemotherapeutic agent.

Examples of hyperproliferative disorders that can be treated or detected by a polynucleotide or polypeptide of the present invention include, but are not limited to neoplasms located in the: abdomen, bone, breast, digestive system, liver, pancreas, peritoneum, endocrine glands (adrenal, parathyroid, pituitary, testicles, ovary, thymus, thyroid), eye, head and neck, nervous (central and peripheral), lymphatic system, pelvic, skin, soft tissue, spleen, thoracic, and urogenital.

Similarly, other hyperproliferative disorders can also be treated or detected by a polynucleotide or polypeptide of the present invention. Examples of such hyperproliferative disorders include, but are not limited to: hypergammaglobulinemia, lymphoproliferative disorders, paraproteinemias, purpura, sarcoidosis, Sezary Syndrome, Waldenstron's Macroglobulinemia, Gaucher's Disease, histiocytosis, and any other hyperproliferative disease, besides neoplasia, located in an organ system listed above.

### Infectious Disease

A polypeptide or polynucleotide of the present invention can be used to treat or detect infectious agents. For example, by increasing the immune response, particularly increasing the proliferation and differentiation of B and/or T cells, infectious diseases may be treated. The immune response may be increased by either enhancing an existing immune response, or by initiating a new immune response. Alternatively, the polypeptide or polynucleotide of the present invention may also directly inhibit the infectious agent, without necessarily eliciting an immune response.

Viruses are one example of an infectious agent that can cause disease or symptoms that can be treated or detected by a polynucleotide or polypeptide of the present invention. Examples of viruses, include, but are not limited to the following DNA and RNA viral families: Arbovirus, Adenoviridae, Arenaviridae, Arterivirus, Birnaviridae, Bunyaviridae, Caliciviridae, Circoviridae, Coronaviridae, Flaviviridae, Hepadnaviridae (Hepatitis), Herpesviridae (such as, Cytomegalovirus, Herpes Simplex, Herpes Zoster), Mononegavirus (e.g., Paramyxoviridae, Morbillivirus, Rhabdoviridae), Orthomyxoviridae (e.g., Influenza), Papovaviridae, Parvoviridae, Picomaviridae, Poxviridae (such as Smallpox or Vaccinia), Reoviridae (e.g., Rotavirus), Retroviridae (HTLV-I, HTLV-II, Lentivirus), and Togaviridae (e.g., Rubivirus). Viruses falling within these families can cause a variety of diseases or symptoms, including, but not limited to: arthritis, bronchiollitis, encephalitis, eye infections (e.g., conjunctivitis, keratitis), chronic fatigue syndrome, hepatitis (A, B, C, E, Chronic Active, Delta), meningitis, opportunistic infections (e.g., AIDS), pneumonia, Burkitt's Lymphoma, chickenpox , hemorrhagic fever, Measles, Mumps, Parainfluenza, Rabies, the common cold, Polio, leukemia, Rubella, sexually transmitted diseases, skin diseases (e.g., Kaposi's, warts), and viremia. A polypeptide or polynucleotide of the present invention can be used to treat or detect any of these symptoms or diseases.

Similarly, bacterial or fungal agents that can cause disease or symptoms and that can be treated or detected by a polynucleotide or polypeptide of the present invention include, but not limited to, the following Gram-Negative and Gram-positive bacterial families and fungi: Actinomycetales (e.g., Corynebacterium, Mycobacterium, Norcardia), Aspergillosis, Bacillaceae (e.g., Anthrax, Clostridium), Bacteroidaceae, Blastomycosis, Bordetella, Borrelia, Brucellosis, Candidiasis, Campylobacter, Coccidioidomycosis, Cryptococcosis, Dermatocycoses, Enterobacteriaceae (Klebsiella, Salmonella, Serratia, Yersinia), Erysipelothrix, Helicobacter, Legionellosis, Leptospirosis, Listeria, Mycoplasmatales, Neisseriaceae (e.g., Acinetobacter, Gonorrhea, Menigococcal), Pasteurellacea Infections (e.g., Actinobacillus, Heamophilus, Pasteurella), Pseudomonas, Rickettsiaceae, Chlamydiaceae, Syphilis, and Staphylococcal. These bacterial or fungal families can cause the following diseases or symptoms, including, but not limited to: bacteremia, endocarditis, eye infections (conjunctivitis, tuberculosis, uveitis), gingivitis, opportunistic infections (e.g., AIDS related infections), paronychia, prosthesis-related infections, Reiter's Disease, respiratory tract infections, such as Whooping Cough or Empyema, sepsis, Lyme Disease, Cat-Scratch Disease, Dysentery, Paratyphoid Fever, food poisoning, Typhoid, pneumonia, Gonorrhea, meningitis, Chlamydia, Syphilis, Diphtheria, Leprosy, Paratuberculosis, Tuberculosis, Lupus, Botulism, gangrene, tetanus, impetigo, Rheumatic Fever, Scarlet Fever, sexually transmitted diseases, skin diseases (e.g., cellulitis, dermatocycoses), toxemia, urinary tract infections, wound infections. A polypeptide or polynucleotide of the present invention can be used to treat or detect any of these symptoms or diseases.

Moreover, parasitic agents causing disease or symptoms that can be treated or detected by a polynucleotide or polypeptide of the present invention include, but not limited to, the following families: Amebiasis, Babesiosis, Coccidiosis, Cryptosporidiosis, Dientamoebiasis, Dourine, Ectoparasitic, Giardiasis, Helminthiasis, Leishmaniasis, Theileriasis, Toxoplasmosis, Trypanosomiasis, and Trichomonas. These parasites can cause a variety of diseases or symptoms, including, but not limited to: Scabies, Trombiculiasis, eye infections, intestinal disease (e.g., dysentery, giardiasis), liver disease, lung disease, opportunistic infections (e.g., AIDS related), Malaria, pregnancy complications, and toxoplasmosis: A polypeptide or polynucleotide of the present invention can be used to treat or detect any of these symptoms or diseases.

Preferably, treatment using a polypeptide or polynucleotide of the present invention could either be by administering an effective amount of a polypeptide to the patient, or by removing cells from the patient, supplying the cells with a polynucleotide of the present invention, and returning the engineered cells to the patient (ex vivo therapy). Moreover, the polypeptide or polynucleotide of the present invention can be used as an antigen in a vaccine to raise an immune response against infectious disease.

### Regeneration

A polynucleotide or polypeptide of the present invention can be used to differentiate, proliferate, and attract cells, leading to the regeneration of tissues. (See, Science 276:59-87 (1997).) The regeneration of tissues could be used to repair, replace, or protect tissue damaged by congenital defects, trauma (wounds, burns, incisions, or ulcers), age, disease (e.g. osteoporosis, osteocarthritis, periodontal disease, liver failure), surgery, including cosmetic plastic surgery, fibrosis, reperfusion injury, or systemic cytokine damage.

Tissues that could be regenerated using the present invention include organs (e.g., pancreas, liver, intestine, kidney, skin, endothelium), muscle (smooth, skeletal or cardiac), vascular (including vascular endothelium), nervous, hematopoietic, and skeletal (bone, cartilage, tendon, and ligament) tissue. Preferably, regeneration occurs without or decreased scarring. Regeneration also may include angiogenesis.

Moreover, a polynucleotide or polypeptide of the present invention may increase regeneration of tissues difficult to heal. For example, increased tendon/ligament regeneration would quicken recovery time after damage. A polynucleotide or polypeptide of the present invention could also be used prophylactically in an effort to avoid damage. Specific diseases that could be treated include of tendinitis, carpal tunnel syndrome, and other tendon or ligament defects. A further example of tissue regeneration of non-healing wounds includes pressure ulcers, ulcers associated with vascular insufficiency, surgical, and traumatic wounds.

Similarly, nerve and brain tissue could also be regenerated by using a polynucleotide or polypeptide of the present invention to proliferate and differentiate nerve cells. Diseases that could be treated using this method include central and peripheral nervous system diseases, neuropathies, or mechanical and traumatic disorders (e.g., spinal cord disorders, head trauma, cerebrovascular disease, and stoke). Specifically, diseases associated with peripheral nerve injuries, peripheral neuropathy (e.g., resulting from chemotherapy or other medical therapies), localized neuropathies, and central nervous system diseases (e.g., Alzheimer's disease, Parkinson's disease, Huntington's disease, amyotrophic lateral sclerosis, and Shy-Drager syndrome), could all be treated using the polynucleotide or polypeptide of the present invention.

### Chemotaxis

A polynucleotide or polypeptide of the present invention may have chemotaxis activity. A chemotaxic molecule attracts or mobilizes cells (e.g., monocytes, fibroblasts, neutrophils, T-cells, mast cells, eosinophils, epithelial and/or endothelial cells) to a particular site in the body, such as inflammation, infection, or site of hyperproliferation. The mobilized cells can then fight off and/or heal the particular trauma or abnormality.

A polynucleotide or polypeptide of the present invention may increase chemotaxic activity of particular cells. These chemotactic molecules can then be used to treat inflammation, infection, hyperproliferative disorders, or any immune system disorder by increasing the number of cells targeted to a particular location in the body. For example, chemotaxic molecules can be used to treat wounds and other trauma to tissues by attracting immune cells to the injured location. Chemotactic molecules of the present invention can also attract fibroblasts, which can be used to treat wounds.

It is also contemplated that a polynucleotide or polypeptide of the present invention may inhibit chemotactic activity. These molecules could also be used to treat disorders. Thus, a polynucleotide or polypeptide of the present invention could be used as an inhibitor of chemotaxis.

### Binding Activity

A polypeptide of the present invention may be used to screen for molecules that bind to the polypeptide or for molecules to which the polypeptide binds. The binding of the polypeptide and the molecule may activate (agonist), increase, inhibit (antagonist), or decrease activity of the polypeptide or the molecule bound. Examples of such molecules include antibodies, oligonucleotides, proteins (e.g., receptors),or small molecules.

Preferably, the molecule is closely related to the natural ligand of the polypeptide, e.g., a fragment of the ligand, or a natural substrate, a ligand, a structural or functional mimetic. (See, Coligan et al., Current Protocols in Immunology 1(2):Chapter 5 (1991).) Similarly, the molecule can be closely related to the natural receptor to which the polypeptide binds, or at least, a fragment of the receptor capable of being bound by the polypeptide (e.g., active site). In either case, the molecule can be rationally designed using known techniques.

Preferably, the screening for these molecules involves producing appropriate cells which express the polypeptide, either as a secreted protein or on the cell membrane. Preferred cells include cells from mammals, yeast, Drosophila, or *E. coli.* Cells expressing the polypeptide (or cell membrane containing the expressed polypeptide) are then preferably contacted with a test compound potentially containing the molecule to observe binding, stimulation, or inhibition of activity of either the polypeptide or the molecule.

The assay may simply test binding of a candidate compound to the polypeptide, wherein binding is detected by a label, or in an assay involving competition with a labeled competitor. Further, the assay may test whether the candidate compound results in a signal generated by binding to the polypeptide.

Alternatively, the assay can be carried out using cell-free preparations, polypeptide/molecule affixed to a solid support, chemical libraries, or natural product mixtures. The assay may also simply comprise the steps of mixing a candidate compound with a solution containing a polypeptide, measuring polypeptide/molecule activity or binding, and comparing the polypeptide/molecule activity or binding to a standard.

Preferably, an ELISA assay can measure polypeptide level or activity in a sample (e.g., biological sample) using a monoclonal or polyclonal antibody. The antibody can measure polypeptide level or activity by either binding, directly or indirectly, to the polypeptide or by competing with the polypeptide for a substrate.

All of these above assays can be used as diagnostic or prognostic markers. The molecules discovered using these assays can be used to treat disease or to bring about a particular result in a patient (e.g., blood vessel growth) by activating or inhibiting the polypeptide/molecule. Moreover, the assays can discover agents which may inhibit or enhance the production of the polypeptide from suitably manipulated cells or tissues.

Therefore, the invention includes a method of identifying compounds which bind to a polypeptide of the invention comprising the steps of: (a) incubating a candidate binding compound with a polypeptide of the invention; and (b) determining if binding has occurred. Moreover, the invention includes a method of identifying agonists/antagonists comprising the steps of: (a) incubating a candidate compound with a polypeptide of the invention; (b) assaying a biological activity , and (b) determining if a biological activity of the polypeptide has been altered.

### Other Activities

A polypeptide or polynucleotide of the present invention may also increase or decrease the differentiation or proliferation of embryonic stem cells, besides, as discussed above, hematopoietic lineage.

A polypeptide or polynucleotide of the present invention may also be used to modulate mammalian characteristics, such as body height, weight, hair color, eye color, skin, percentage of adipose tissue, pigmentation, size, and shape (e.g., cosmetic surgery). Similarly, a polypeptide or polynucleotide of the present invention may be used to modulate mammalian metabolism affecting catabolism, anabolism, processing, utilization, and storage of energy.

A polypeptide or polynucleotide of the present invention may be used to change a mammal's mental state or physical state by influencing biorhythms, caricadic rhythms, depression (including depressive disorders), tendency for violence, tolerance for pain, reproductive capabilities (preferably by Activin or Inhibin-like activity), hormonal or endocrine levels, appetite, libido, memory, stress, or other cognitive qualities.

A polypeptide or polynucleotide of the present invention may also be used as a food additive or preservative, such as to increase or decrease storage capabilities, fat content, lipid, protein, carbohydrate, vitamins, minerals, cofactors or other nutritional components.

### Other Preferred Embodiments

Other preferred embodiments of the claimed invention include an isolated nucleic acid molecule comprising a nucleotide sequence which is at least 95% identical to a sequence of at least about 50 contiguous nucleotides in the nucleotide sequence of SEQ ID NO:X wherein X is any integer as defined in Table 1.

Also preferred is a nucleic acid molecule wherein said sequence of contiguous nucleotides is included in the nucleotide sequence of SEQ ID NO:X in the range of positions beginning with the nucleotide at about the position of the 5' Nucleotide of the Clone Sequence and ending with the nucleotide at about the position of the 3' Nucleotide of the Clone Sequence as defined for SEQ ID NO:X in Table 1.

Also preferred is a nucleic acid molecule wherein said sequence of contiguous nucleotides is included in the nucleotide sequence of SEQ ID NO:X in the range of positions beginning with the nucleotide at about the position of the 5' Nucleotide of the Start Codon and ending with the nucleotide at about the position of the 3' Nucleotide of the Clone Sequence as defined for SEQ ID NO:X in Table 1.

Similarly preferred is a nucleic acid molecule wherein said sequence of contiguous nucleotides is included in the nucleotide sequence of SEQ ID NO:X in the range of positions beginning with the nucleotide at about the position of the 5' Nucleotide of the First Amino Acid of the Signal Peptide and ending with the nucleotide at about the position of the 3' Nucleotide of the Clone Sequence as defined for SEQ ID NO:X in Table 1.

Also preferred is an isolated nucleic acid molecule comprising a nucleotide sequence which is at least 95% identical to a sequence of at least about 150 contiguous nucleotides in the nucleotide sequence of SEQ ID NO:X.

Further preferred is an isolated nucleic acid molecule comprising a nucleotide sequence which is at least 95% identical to a sequence of at least about 500 contiguous nucleotides in the nucleotide sequence of SEQ ID NO:X.

A further preferred embodiment is a nucleic acid molecule comprising a nucleotide sequence which is at least 95% identical to the nucleotide sequence of SEQ ID NO:X beginning with the nucleotide at about the position of the 5' Nucleotide of the First Amino Acid of the Signal Peptide and ending with the nucleotide at about the position of the 3' Nucleotide of the Clone Sequence as defined for SEQ ID NO:X in Table 1.

A further preferred embodiment is an isolated nucleic acid molecule comprising a nucleotide sequence which is at least 95% identical to the complete nucleotide sequence of SEQ ID NO:X.

Also preferred is an isolated nucleic acid molecule which hybridizes under stringent hybridization conditions to a nucleic acid molecule, wherein said nucleic acid molecule which hybridizes does not hybridize under stringent hybridization conditions to a nucleic acid molecule having a nucleotide sequence consisting of only A residues or of only T residues.

Also preferred is a composition of matter comprising a DNA molecule which comprises a human cDNA clone identified by a cDNA Clone Identifier in Table 1, which DNA molecule is contained in the material deposited with the American Type Culture Collection and given the ATCC Deposit Number shown in Table 1 for said cDNA Clone Identifier.

Also preferred is an isolated nucleic acid molecule comprising a nucleotide sequence which is at least 95% identical to a sequence of at least 50 contiguous nucleotides in the nucleotide sequence of a human cDNA clone identified by a cDNA Clone Identifier in Table 1, which DNA molecule is contained in the deposit given the ATCC Deposit Number shown in Table 1.

Also preferred is an isolated nucleic acid molecule, wherein said sequence of at least 50 contiguous nucleotides is included in the nucleotide sequence of the complete open reading frame sequence encoded by said human cDNA clone.

Also preferred is an isolated nucleic acid molecule comprising a nucleotide sequence which is at least 95% identical to sequence of at least 150 contiguous nucleotides in the nucleotide sequence encoded by said human cDNA clone.

A further preferred embodiment is an isolated nucleic acid molecule comprising a nucleotide sequence which is at least 95% identical to sequence of at least 500 contiguous nucleotides in the nucleotide sequence encoded by said human cDNA clone.

A further preferred embodiment is an isolated nucleic acid molecule comprising a nucleotide sequence which is at least 95% identical to the complete nucleotide sequence encoded by said human cDNA clone.

A further preferred embodiment is a method for detecting in a biological sample a nucleic acid molecule comprising a nucleotide sequence which is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1; which method comprises a step of comparing a nucleotide sequence of at least one nucleic acid molecule in said sample with a sequence selected from said group and determining whether the sequence of said nucleic acid molecule in said sample is at least 95% identical to said selected sequence.

Also preferred is the above method wherein said step of comparing sequences comprises determining the extent of nucleic acid hybridization between nucleic acid molecules in said sample and a nucleic acid molecule comprising said sequence selected from said group. Similarly, also preferred is the above method wherein said step of comparing sequences is performed by comparing the nucleotide sequence determined from a nucleic acid molecule in said sample with said sequence selected from said group. The nucleic acid molecules can comprise DNA molecules or RNA molecules.

A further preferred embodiment is a method for identifying the species, tissue or cell type of a biological sample which method comprises a step of detecting nucleic acid molecules in said sample, if any, comprising a nucleotide sequence that is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1.

The method for identifying the species, tissue or cell type of a biological sample can comprise a step of detecting nucleic acid molecules comprising a nucleotide sequence in a panel of at least two nucleotide sequences, wherein at least one sequence in said panel is at least 95% identical to a sequence of at least 50 contiguous nucleotides in a sequence selected from said group.

Also preferred is a method for diagnosing in a subject a pathological condition associated with abnormal structure or expression of a gene encoding a secreted protein identified in Table 1, which method comprises a step of detecting in a biological sample obtained from said subject nucleic acid molecules, if any, comprising a nucleotide sequence that is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1.

The method for diagnosing a pathological condition can comprise a step of detecting nucleic acid molecules comprising a nucleotide sequence in a panel of at least two nucleotide sequences, wherein at least one sequence in said panel is at least 95% identical to a sequence of at least 50 contiguous nucleotides in a sequence selected from said group.

Also preferred is a composition of matter comprising isolated nucleic acid molecules wherein the nucleotide sequences of said nucleic acid molecules comprise a panel of at least two nucleotide sequences, wherein at least one sequence in said panel is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1. The nucleic acid molecules can comprise DNA molecules or RNA molecules.

Also preferred is an isolated polypeptide comprising an amino acid sequence at least 90% identical to a sequence of at least'about 10 contiguous amino acids in the amino acid sequence of SEQ ID NO:Y wherein Y is any integer as defined in Table 1.

Also preferred is a polypeptide, wherein said sequence of contiguous amino acids is included in the amino acid sequence of SEQ ID NO:Y in the range of positions beginning with the residue at about the position of the First Amino Acid of the Secreted Portion and ending with the residue at about the Last Amino Acid of the Open Reading Frame as set forth for SEQ ID NO:Y in Table 1.

Also preferred is an isolated polypeptide comprising an amino acid sequence at least 95% identical to a sequence of at least about 30 contiguous amino acids in the amino acid sequence of SEQ ID NO:Y.

Further preferred is an isolated polypeptide comprising an amino acid sequence at least 95% identical to a sequence of at least about 100 contiguous amino acids in the amino acid sequence of SEQ ID NO:Y.

Further preferred is an isolated polypeptide comprising an amino acid sequence at least 95% identical to the complete amino acid sequence of SEQ ID NO:Y.

Further preferred is an isolated polypeptide comprising an amino acid sequence at least 90% identical to a sequence of at least about 10 contiguous amino acids in the complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is a polypeptide wherein said sequence of contiguous amino acids is included in the amino acid sequence of a secreted portion of the secreted protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to a sequence of at least about 30 contiguous amino acids in the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to a sequence of at least about 100 contiguous amino acids in the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Further preferred is an isolated antibody which binds specifically to a polypeptide comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1.

Further preferred is a method for detecting in a biological sample a polypeptide comprising an amino acid sequence which is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1; which method comprises a step of comparing an amino acid sequence of at least one polypeptide molecule in said sample with a sequence selected from said group and determining whether the sequence of said polypeptide molecule in said sample is at least 90% identical to said sequence of at least 10 contiguous amino acids.

Also preferred is the above method wherein said step of comparing an amino acid sequence of at least one polypeptide molecule in said sample with a sequence selected from said group comprises determining the extent of specific binding of polypeptides in said sample to an antibody which binds specifically to a polypeptide comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1.

Also preferred is the above method wherein said step of comparing sequences is performed by comparing the amino acid sequence determined from a polypeptide molecule in said sample with said sequence selected from said group.

Also preferred is a method for identifying the species, tissue or cell type of a biological sample which method comprises a step of detecting polypeptide molecules in said sample, if any, comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is the above method for identifying the species, tissue or cell type of a biological sample, which method comprises a step of detecting polypeptide molecules comprising an amino acid sequence in a panel of at least two amino acid sequences, wherein at least one sequence in said panel is at least 90% identical to a sequence of at least 10 contiguous amino acids in a sequence selected from the above group.

Also preferred is a method for diagnosing in a subject a pathological condition associated with abnormal structure or expression of a gene encoding a secreted protein identified in Table 1, which method comprises a step of detecting in a biological sample obtained from said subject polypeptide molecules comprising an amino acid sequence in a panel of at least two amino acid sequences, wherein at least one sequence in said panel is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

In any of these methods, the step of detecting said polypeptide molecules includes using an antibody.

Also preferred is an isolated nucleic acid molecule comprising a nucleotide sequence which is at least 95% identical to a nucleotide sequence encoding a polypeptide wherein said polypeptide comprises an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is an isolated nucleic acid molecule, wherein said nucleotide sequence encoding a polypeptide has been optimized for expression of said polypeptide in a prokaryotic host.

Also preferred is an isolated nucleic acid molecule, wherein said polypeptide comprises an amino acid sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Further preferred is a method of making a recombinant vector comprising inserting any of the above isolated nucleic acid molecule into a vector. Also preferred is the recombinant vector produced by this method. Also preferred is a method of making a recombinant host cell comprising introducing the vector into a host cell, as well as the recombinant host cell produced by this method.

Also preferred is a method of making an isolated polypeptide comprising culturing this recombinant host cell under conditions such that said polypeptide is expressed and recovering said polypeptide. Also preferred is this method of making an isolated polypeptide; wherein said recombinant host cell is a eukaryotic cell and said polypeptide is a secreted portion of a human secreted protein comprising an amino acid sequence selected from the group consisting of: an amino acid sequence of SEQ ID NO:Y beginning with the residue at the position of the First Amino Acid of the Secreted Portion of SEQ ID NO:Y wherein Y is an integer set forth in Table 1 and said position of the First Amino Acid of the Secreted Portion of SEQ ID NO:Y is defined in Table 1; and an amino acid sequence of a secreted portion of a protein encoded by a human cDNA clone identified by a cDNA Clone Identifier in Table 1 and contained in the deposit with the ATCC Deposit Number shown for said cDNA clone in Table 1. The isolated polypeptide produced by this method is also preferred.

Also preferred is a method of treatment of an individual in need of an increased level of a secreted protein activity, which method comprises administering to such an individual a pharmaceutical composition comprising an amount of an isolated polypeptide, polynucleotide, or antibody of the claimed invention effective to increase the level of said protein activity in said individual.

Having generally described the invention, the same will be more readily understood by reference to the following examples, which are provided by way of illustration and are not intended as limiting.

### Examples

### Example 1: Isolation of a Selected cDNA Clone From the Deposited Sample

Each cDNA clone in a cited ATCC deposit is contained in a plasmid vector. Table 1 identifies the vectors used to construct the cDNA library from which each clone was isolated. In many cases, the vector used to construct the library is a phage vector from which a plasmid has been excised. The table immediately below correlates the related plasmid for each phage vector used in constructing the cDNA library. For example, where a particular clone is identified in Table 1 as being isolated in the vector "Lambda Zap," the corresponding deposited clone is in "pBluescript."

| Vector Used to Construct Library | Corresponding Deposited Plasmid |
|---|---|
| Lambda Zap | pBluescript (pBS) |
| Uni-Zap XR | pBluescript (pBS) |
| Zap Express | pBK |
| lafmid BA | plafmid BA |
| pSport1 | pSport1 |
| pCMVSport 2.0 | pCMVSport 2.0 |
| pCMVSport 3.0 | pCMVSport 3.0 |
| pCR® 2.1 | pCR® 2.1 |

Vectors Lambda Zap (U.S. Patent Nos. 5,128,256 and 5,286,636), Uni-Zap XR (U.S. Patent Nos. 5,128, 256 and 5,286,636), Zap Express (U.S. Patent Nos. 5,128,256 and 5,286,636), pBluescript (pBS) (Short, J. M. et al., Nucleic Acids Res. 16:7583-7600 (1988); Alting-Mees, M. A. and Short, J. M., Nucleic Acids Res. 17:9494 (1989)) and pBK (Alting-Mees, M. A. et al., Strategies 5:58-61 (1992)) are commercially available from Stratagene Cloning Systems, Inc., 11011 N. Torrey Pines Road, La Jolla, CA, 92037. pBS contains an ampicillin resistance gene and pBK contains a neomycin resistance gene. Both can be transformed into E. coli strain XL-1 Blue, also available from Stratagene. pBS comes in 4 forms SK+, SK-, KS+ and KS. The S and K refers to the orientation of the polylinker to the T7 and T3 primer sequences which flank the polylinker region ("S" is for SacI and "K" is for KpnI which are the first sites on each respective end of the linker). "+" or "-" refer to the orientation of the f1 origin of replication ("ori"), such that in one orientation, single stranded rescue initiated from the f1 ori generates sense strand DNA and in the other, antisense.

Vectors pSport1, pCMVSport 2.0 and pCMVSport 3.0, were obtained from Life Technologies, Inc., P. O. Box 6009, Gaithersburg, MD 20897. All Sport vectors contain an ampicillin resistance gene and may be transformed into E, coli strain DH10B, also available from Life Technologies. (See, for instance, Gruber, C. E., et al., Focus 15:59 (1993).) Vector lafmid BA (Bento Soares, Columbia University, NY) contains an ampicillin resistance gene and can be transformed into E. coli strain XL-1 Blue: Vector pCR®2.1, which is available from Invitrogen, 1600 Faraday Avenue, Carlsbad, CA 92008, contains an ampicillin resistance gene and may be transformed into E. coli strain DH10B, available from Life Technologies. (See, for instance, Clark, J. M., Nuc. Acids Res. 16:9677-9686 (1988) and Mead, D. et al., Bio/Technology 9: (1991).) Preferably, a polynucleotide of the present invention does not comprise the phage vector sequences identified for the particular clone in Table 1, as well as the corresponding plasmid vector sequences designated above.

The deposited material in the sample assigned the ATCC Deposit Number cited in Table 1 for any given cDNA clone also may contain one or more additional plasmids, each comprising a cDNA clone different from that given clone. Thus, deposits sharing the same ATCC Deposit Number contain at least a plasmid for each cDNA clone identified in Table 1. Typically, each ATCC deposit sample cited in Table 1 comprises a mixture of approximately equal amounts (by weight) of about 50 plasmid DNAs, each containing a different cDNA clone; but such a deposit sample may include plasmids for more or less than 50 cDNA clones, up to about 500 cDNA clones.

Two approaches can be used to isolate a particular clone from the deposited sample of plasmid DNAs cited for that clone in Table 1. First, a plasmid is directly isolated by screening the clones using a polynucleotide probe corresponding to SEQ ID NO:X.

Particularly, a specific polynucleotide with 30-40 nucleotides is synthesized using an Applied Biosystems DNA synthesizer according to the sequence reported. The oligonucleotide is labeled, for instance, with ³²P-γ-ATP using T4 polynucleotide kinase and purified according to routine methods. (E.g., Maniatis et al., Molecular Cloning: A Laboratory Manual, Cold Spring Harbor Press, Cold Spring, NY (1982).) The plasmid mixture is transformed into a suitable host, as indicated above (such as XL-1 Blue (Stratagene)) using techniques known to those of skill in the art, such as those provided by the vector supplier or in related publications or patents cited above. The transformants are plated on 1.5% agar plates (containing the appropriate selection agent, e.g., ampicillin) to a density of about 150 transformants (colonies) per plate. These plates are screened using Nylon membranes according to routine methods for bacterial colony screening (e.g., Sambrook et al., Molecular Cloning: A Laboratory Manual, 2nd Edit., (1989), Cold Spring Harbor Laboratory Press, pages 1.93 to 1.104), or other techniques known to those of skill in the art.

Alternatively, two primers of 17-20 nucleotides derived from both ends of the SEQ ID NO:X (i.e., within the region of SEQ ID NO:X bounded by the 5' NT and the 3' NT of the clone defined in Table 1) are synthesized and used to amplify the desired cDNA using the deposited cDNA plasmid as a template. The polymerase chain reaction is carried out under routine conditions; for instance, in 25 µl of reaction mixture with 0.5 ug of the above cDNA template. A convenient reaction mixture is 1.5-5 mM MgCl₂, 0.01% (w/v) gelatin, 20 µM each of dATP, dCTP, dGTP, dTTP, 25 pmol of each primer and 0.25 Unit of Taq polymerase. Thirty five cycles of PCR (denaturation at 94°C for 1 min; annealing at 55°C for 1 min; elongation at 72°C for 1 min) are performed with a Perkin-Elmer Cetus automated thermal cycler. The amplified product is analyzed by agarose gel electrophoresis and the DNA band with expected molecular weight is excised and purified. The PCR product is verified to be the selected sequence by subcloning and sequencing the DNA product.

Several methods are available for the identification of the 5' or 3' non-coding portions of a gene which may not be present in the deposited clone. These methods include but are not limited to, filter probing, clone enrichment using specific probes, and protocols similar or identical to 5' and 3' "RACE" protocols which are well known in the art. For instance, a method similar to 5' RACE is available for generating the missing 5' end of a desired full-length transcript. (Fromont-Racine et al., Nucleic Acids Res. 21(7):1683-1684 (1993).)

Briefly, a specific RNA oligonucleotide is ligated to the 5' ends of a population of RNA presumably containing full-length gene RNA transcripts. A primer set containing a primer specific to the ligated RNA oligonucleotide and a primer specific to a known sequence of the gene of interest is used to PCR amplify the 5' portion of the desired full-length gene. This amplified product may then be sequenced and used to generate the full length gene.

This above method starts with total RNA isolated from the desired source, although poly-A+ RNA can be used. The RNA preparation can then be treated with phosphatase if necessary to eliminate 5' phosphate groups on degraded or damaged RNA which may interfere with the later RNA ligase step. The phosphatase should then be inactivated and the RNA treated with tobacco acid pyrophosphatase in order to remove the cap structure present at the 5' ends of messenger RNAs. This reaction leaves a 5' phosphate group at the 5' end of the cap cleaved RNA which can then be ligated to an RNA oligonucleotide using T4 RNA ligase.

This modified RNA preparation is used as a template for first strand cDNA synthesis using a gene specific oligonucleotide. The first strand synthesis reaction is used as a template for PCR amplification of the desired 5' end using a primer specific to the ligated RNA oligonucleotide and a primer specific to the known sequence of the gene of interest. The resultant product is then sequenced and analyzed to confirm that the 5' end sequence belongs to the desired gene.

### Example 2: Isolation of Genomic Clones Corresponding to a Polynucleotide

A human genomic P1 library (Genomic Systems, Inc.) is screened by PCR using primers selected for the cDNA sequence corresponding to SEQ ID NO:X., according to the method described in Example 1. (See also, Sambrook.)

### Example 3: Tissue Distribution of Polypeptide

Tissue distribution of mRNA expression of polynucleotides of the present invention is determined using protocols for Northern blot analysis, described by, among others, Sambrook et al. For example, a cDNA probe produced by the method described in Example 1 is labeled with P³² using the rediprime™ DNA labeling system (Amersham Life Science), according to manufacturer's instructions. After labeling, the probe is purified using CHROMA SPIN-100™ column (Clontech Laboratories, Inc.), according to manufacturer's protocol number PT1200-1. The purified labeled probe is then used to examine various human tissues for mRNA expression.

Multiple Tissue Northern (MTN) blots containing various human tissues (H) or human immune system tissues (IM) (Clontech) are examined with the labeled probe using ExpressHyb™ hybridization solution (Clontech) according to manufacturer's protocol number PT1190-1. Following hybridization and washing, the blots are mounted and exposed to film at -70°C overnight, and the films developed according to standard procedures.

### Example 4: Chromosomal Mapping of the Polynucleotides

An oligonucleotide primer set is designed according to the sequence at the 5' end of SEQ ID NO:X. This primer preferably spans about 100 nucleotides. This primer set is then used in a polymerase chain reaction under the following set of conditions : 30 seconds, 95°C; 1 minute, 56°C; 1 minute, 70°C. This cycle is repeated 32 times followed by one 5 minute cycle at 70°C. Human, mouse, and hamster DNA is used as template in addition to a somatic cell hybrid panel containing individual chromosomes or chromosome fragments (Bios, Inc). The reactions is analyzed on either 8% polyacrylamide gels or 3.5 % agarose gels. Chromosome mapping is determined by the presence of an approximately 100 bp PCR fragment in the particular somatic cell hybrid.

### Example 5: Bacterial Expression of a Polypetide

A polynucleotide encoding a polypeptide of the present invention is amplified using PCR oligonucleotide primers corresponding to the 5' and 3' ends of the DNA sequence, as outlined in Example 1, to synthesize insertion fragments. The primers used to amplify the cDNA insert should preferably contain restriction sites, such as BamHI and XbaI, at the 5' end of the primers in order to clone the amplified product into the expression vector. For example, BamHI and XbaI correspond to the restriction enzyme sites on the bacterial expression vector pQE-9. (Qiagen, Inc., Chatsworth, CA). This plasmid vector encodes antibiotic resistance (Amp^{r}), a bacterial origin of replication (ori), an IPTG-regulatable promoter/operator (P/O), a ribosome binding site (RBS), a 6-histidine tag (6-His), and restriction enzyme cloning sites.

The pQE-9 vector is digested with BamHI and XbaI and the amplified fragment is ligated into the pQE-9 vector maintaining the reading frame initiated at the bacterial RBS. The ligation mixture is then used to transform the E. coli strain M15/rep4 (Qiagen, Inc.) which contains multiple copies of the plasmid pREP4, which expresses the lacI repressor and also confers kanamycin resistance (Kan^{r}). Transformants are identified by their ability to grow on LB plates and ampicillin/kanamycin resistant colonies are selected. Plasmid DNA is isolated and confirmed by restriction analysis.

Clones containing the desired constructs are grown overnight (O/N) in liquid culture in LB media supplemented with both Amp (100 ug/ml) and Kan (25 ug/ml). The O/N culture is used to inoculate a large culture at a ratio of 1:100 to 1:250. The cells are grown to an optical density 600 (O.D.⁶⁰⁰) of between 0.4 and 0.6. IPTG (Isopropyl-B-D-thiogalacto pyranoside) is then added to a final concentration of 1 mM. IPTG induces by inactivating thelacI repressor, clearing the P/O leading to increased gene expression.

Cells are grown for an extra 3 to 4 hours. Cells are then harvested by centrifugation (20 mins at 6000Xg). The cell pellet is solubilized in the chaotropic agent 6 Molar Guanidine HCl by stirring for 3-4 hours at 4°C. The cell debris is removed by centrifugation, and the supernatant containing the polypeptide is loaded onto a nickel-nitrilo-tri-acetic acid ("Ni-NTA") affinity resin column (available from QIAGEN, Inc., *supra).* Proteins with a 6 x His tag bind to the Ni-NTA resin with high affinity and can be purified in a simple one-step procedure (for details see: The QIAexpressionist (1995) QIAGEN, Inc., *supra).*

Briefly, the supernatant is loaded onto the column in 6 M guanidine-HCl, pH 8, the column is first washed with 10 volumes of 6 M guanidine-HCl, pH 8, then washed with 10 volumes of 6 M guanidine-HCl pH 6, and finally the polypeptide is eluted with 6 M guanidine-HCl, pH 5.

The purified protein is then renatured by dialyzing it against phosphate-buffered saline (PBS) or 50 mM Na-acetate, pH 6 buffer plus 200 mM NaCl. Alternatively, the protein can be successfully refolded while immobilized on the Ni-NTA column. The recommended conditions are as follows: renature using a linear 6M-1M urea gradient in 500 mM NaCl, 20% glycerol, 20 mM Tris/HCl pH 7.4, containing protease inhibitors. The renaturation should be performed over a period of 1.5 hours or more. After renaturation the proteins are eluted by the addition of 250 mM immidazole. Immidazole is removed by a final dialyzing step against PBS or 50 mM sodium acetate pH 6 buffer plus 200 mM NaCl. The purified protein is stored at 4°C or frozen at -80°C.

In addition to the above expression vector, the present invention further includes an expression vector comprising phage operator and promoter elements operatively linked to a polynucleotide of the present invention, called pHE4a. (ATCC Accession Number 209645, deposited on February 25, 1998.) This vector contains: 1) a neomycinphosphotransferase gene as a selection marker, 2) an E. coli origin of replication, 3) a T5 phage promoter sequence, 4) two lac operator sequences, 5) a Shine-Delgarno sequence, and 6) the lactose operon repressor gene (lacIq). The origin of replication (oriC) is derived from pUC19 (LTI, Gaithersburg, MD). The promoter sequence and operator sequences are made synthetically.

DNA can be inserted into the pHEa by restricting the vector with NdeI and XbaI, BamHI, XhoI, or Asp718, running the restricted product on a gel, and isolating the larger fragment (the stuffer fragment should be about 310 base pairs). The DNA insert is generated according to the PCR protocol described in Example 1, using PCR primers having restriction sites for NdeI (5' primer) and XbaI, BamHI, XhoI, or Asp718 (3' primer). The PCR insert is gel purified and restricted with compatible enzymes. The insert and vector are ligated according to standard protocols.

The engineered vector could easily be substituted in the above protocol to express protein in a bacterial system.

### Example 6: Purification of a Polypeptide from an Inclusion Body

The following alternative method can be used to purify a polypeptide expressed in *E coli* when it is present in the form of inclusion bodies. Unless otherwise specified, all of the following steps are conducted at 4-10°C.

Upon completion of the production phase of the *E. coli* fermentation, the cell culture is cooled to 4-10°C and the cells harvested by continuous centrifugation at 15,000 rpm (Heraeus Sepatech). On the basis of the expected yield of protein per unit weight of cell paste and the amount of purified protein required, an appropriate amount of cell paste, by weight, is suspended in a buffer solution containing 100 mM Tris, 50 mM EDTA, pH 7.4. The cells are dispersed to a homogeneous suspension using a high shear mixer.

The cells are then lysed by passing the solution through a microfluidizer (Microfuidics, Corp. or APV Gaulin, Inc.) twice at 4000-6000 psi. The homogenate is then mixed with NaCl solution to a final concentration of 0.5 M NaCl, followed by centrifugation at 7000 xg for 15 min. The resultant pellet is washed again using 0.5M NaCl, 100 mM Tris, 50 mM EDTA; pH 7.4.

The resulting washed inclusion bodies are solubilized with 1.5 M guanidine hydrochloride (GuHCl) for 2-4 hours. After 7000 xg centrifugation for 15 min., the pellet is discarded and the polypeptide containing supernatant is incubated at 4°C overnight to allow further GuHCl extraction.

Following high speed centrifugation (30,000xg) to remove insoluble particles, the GuHCl solubilized protein is refolded by quickly mixing the GuHCl extract with 20 volumes of buffer containing 50 mM sodium, pH 4.5, 150 mM NaCl, 2 mM EDTA by vigorous stirring. The refolded diluted protein solution is kept at 4°C without mixing for 12 hours prior to further purification steps.

To clarify the refolded polypeptide solution, a previously prepared tangential filtration unit equipped with 0.16 µm membrane filter with appropriate surface area (e.g., Filtron), equilibrated with 40 mM sodium acetate, pH 6.0 is employed. The filtered sample is loaded onto a cation exchange resin (e.g., Poros HS-50, Perseptive Biosystems). The column is washed with 40 mM sodium acetate, pH 6.0 and eluted with 250 mM, 500 mM, 1000 mM, and 1500 mM NaCl in the same buffer, in a stepwise manner. The absorbance at 280 nm of the effluent is continuously monitored. Fractions are collected and further analyzed by SDS-PAGE.

Fractions containing the polypeptide are then pooled and mixed with 4 volumes of water. The diluted sample is then loaded onto a previously prepared set of tandem columns of strong anion (Poros HQ-50, Perseptive Biosystems) and weak anion (Poros CM-20, Perseptive Biosystems) exchange resins. The columns are equilibrated with 40 mM sodium acetate, pH 6.0. Both columns are washed with 40 mM sodium acetate, pH 6.0, 200 mM NaCl. The CM-20 column is then eluted using a 10 column volume linear gradient ranging from 0.2 M NaCl, 50 mM sodium acetate, pH 6.0 to 1.0 M NaCl, 50 mM sodium acetate, pH 6.5. Fractions are collected under constant A₂₈₀ monitoring of the effluent. Fractions containing the polypeptide (determined, for instance, by 16% SDS-PAGE) are then pooled.

The resultant polypeptide should exhibit greater than 95% purity after the above refolding and purification steps. No major contaminant bands should be observed from Commassie blue stained 16% SDS-PAGE gel when 5 µg of purified protein is loaded. The purified protein can also be tested for endotoxin/LPS contamination, and typically the LPS content is less than 0.1 ng/ml according to LAL assays.

### Example 7: Cloning and Expression of a Polypeptide in a Baculovirus

### Expression System

In this example, the plasmid shuttle vector pA2 is used to insert a polynucleotide into a baculovirus to express a polypeptide. This expression vector contains the strong polyhedrin promoter of the *Autographa californica* nuclear polyhedrosis virus (AcMNPV) followed by convenient restriction sites such as BamHI, Xba I and Asp718. The polyadenylation site of the simian virus 40 ("SV40") is used for efficient polyadenylation. For easy selection of recombinant virus, the plasmid contains the beta-galactosidase gene from *E. coli* under control of a weak Drosophila promoter in the same orientation, followed by the polyadenylation signal of the polyhedrin gene. The inserted genes are flanked on both sides by viral sequences for cell-mediated homologous recombination with wild-type viral DNA to generate a viable virus that express the cloned polynucleotide.

Many other baculovirus vectors can be used in place of the vector above, such as pAc373, pVL941, and pAcIM1, as one skilled in the art would readily appreciate, as long as the construct provides appropriately located signals for transcription, translation, secretion and the like, including a signal peptide and an in-frame AUG as required. Such vectors are described, for instance, in Luckow et al., Virology 170:31-39 (1989).

Specifically, the cDNA sequence contained in the deposited clone, including the AUG initiation codon and the naturally associated leader sequence identified in Table 1, is amplified using the PCR protocol described in Example 1. If the naturally occurring signal sequence is used to produce the secreted protein, the pA2 vector does not need a second signal peptide. Alternatively, the vector can be modified (pA2 GP) to include a baculovirus leader sequence, using the standard methods described in Summers et al., "A Manual of Methods for Baculovirus Vectors and Insect Cell Culture Procedures," Texas Agricultural Experimental Station Bulletin No. 1555 (1987).

The amplified fragment is isolated from a 1% agarose gel using a commercially available kit ("Geneclean," BIO 101 Inc., La Jolla, Ca.). The fragment then is digested with appropriate restriction enzymes and again purified on a 1% agarose gel.

The plasmid is digested with the corresponding restriction enzymes and optionally, can be dephosphorylated using calf intestinal phosphatase, using routine procedures known in the art. The DNA is then isolated from a 1 % agarose gel using a commercially available kit ("Geneclean" BIO 101 Inc., La Jolla, Ca.).

The fragment and the dephosphorylated plasmid are ligated together with T4 DNA ligase. *E. coli* HB101 or other suitable *E. coli* hosts such as XL-1 Blue (Stratagene Cloning Systems, La Jolla, CA) cells are transformed with the ligation mixture and spread on culture plates. Bacteria containing the plasmid are identified by digesting DNA from individual colonies and analyzing the digestion product by gel electrophoresis. The sequence of the cloned fragment is confirmed by DNA sequencing.

Five µg of a plasmid containing the polynucleotide is co-transfected with 1.0 µg of a commercially available linearized baculovirus DNA ("BaculoGold™ baculovirus DNA", Pharmingen, San Diego, CA), using the lipofection method described by Felgner et al., Proc. Natl. Acad. Sci. USA 84:7413-7417 (1987). One µg of BaculoGold™ virus DNA and 5 µg of the plasmid are mixed in a sterile well of a microtiter plate containing 50 µl of serum-free Grace's medium (Life Technologies Inc., Gaithersburg, MD). Afterwards, 10 µl Lipofectin plus 90 µl Grace's medium are added, mixed and incubated for 15 minutes at room temperature. Then the transfection mixture is added drop-wise to Sf9 insect cells (ATCC CRL 1711) seeded in a 35 mm tissue culture plate with 1 ml Grace's medium without serum. The plate is then incubated for 5 hours at 27°C. The transfection solution is then removed from the plate and 1 ml of Grace's insect medium supplemented with 10% fetal calf serum is added. Cultivation is then continued at 27° C for four days.

After four days the supernatant is collected and a plaque assay is performed, as described by Summers and Smith, *supra.* An agarose gel with "Blue Gal" (Life Technologies Inc., Gaithersburg) is used to allow easy identification and isolation of gal-expressing clones, which produce blue-stained plaques. (A detailed description of a "plaque assay" of this type can also be found in the user's guide for insect cell culture and baculovirology distributed by Life Technologies Inc., Gaithersburg, page 9-10.) After appropriate incubation, blue stained plaques are picked with the tip of a micropipettor (e.g., Eppendorf). The agar containing the recombinant viruses is then resuspended in a microcentrifuge tube containing 200 µl of Grace's medium and the suspension containing the recombinant baculovirus is used to infect Sf9 cells seeded in 35 mm dishes. Four days later the supernatants of these culture dishes are harvested and then they are stored at 4° C.

To verify the expression of the polypeptide, Sf9 cells are grown in Grace's medium supplemented with 10% heat-inactivated FBS. The cells are infected with the recombinant baculovirus containing the polynucleotide at a multiplicity of infection ("MOI") of about 2. If radiolabeled proteins are desired, 6 hours later the medium is removed and is replaced with SF900 II medium minus methionine and cysteine (available from Life Technologies Inc., Rockville, MD). After 42 hours, 5 µCi of³⁵S-methionine and 5 µCi ³⁵S-cysteine (available from Amersham) are added. The cells are further incubated for 16 hours and then are harvested by centrifugation. The proteins in the supernatant as well as the intracellular proteins are analyzed by SDS-PAGE followed by autoradiography (if radiolabeled).

Microsequencing of the amino acid sequence of the amino terminus of purified protein may be used to determine the amino terminal sequence of the produced protein.

### Example 8: Expression of a Polypeptide in Mammalian Cells

The polypeptide of the present invention can be expressed in a mammalian cell. A typical mammalian expression vector contains a promoter element, which mediates the initiation of transcription of mRNA, a protein coding sequence, and signals required for the termination of transcription and polyadenylation of the transcript. Additional elements include enhancers, Kozak sequences and intervening sequences flanked by donor and acceptor sites for RNA splicing. Highly efficient transcription is achieved with the early and late promoters from SV40, the long terminal repeats (LTRs) from Retroviruses, e.g., RSV, HTLVI, HIVI and the early promoter of the cytomegalovirus (CMV). However, cellular elements can also be used (e.g., the human actin promoter).

Suitable expression vectors for use in practicing the present invention include, for example, vectors such as pSVL and pMSG (Pharmacia, Uppsala, Sweden), pRSVcat (ATCC 37152), pSV2dhfr (ATCC 37146), pBC12MI (ATCC 67109), pCMVSport 2.0, and pCMVSport 3.0. Mammalian host cells that could be used include, human Hela, 293, H9 and Jurkat cells, mouse NIH3T3 and C127 cells, Cos 1, Cos 7 and CV 1, quail QC1-3 cells, mouse L cells and Chinese hamster ovary (CHO) cells.

Alternatively, the polypeptide can be expressed in stable cell lines containing the polynucleotide integrated into a chromosome. The co-transfection with a selectable marker such as dhfr, gpt, neomycin, hygromycin allows the identification and isolation of the transfected cells.

The transfected gene can also be amplified to express large amounts of the encoded protein. The DHFR (dihydrofolate reductase) marker is useful in developing cell lines that carry several hundred or even several thousand copies of the gene of interest. (See, e.g., Alt, F. W., et al., J. Biol. Chem. 253:1357-1370 (1978); Hamlin, J. L. and Ma, C., Biochem. et Biophys. Acta, 1097:107-143 (1990); Page, M. J. and Sydenham, M. A., Biotechnology 9:64-68 (1991).) Another useful selection marker is the enzyme glutamine synthase (GS) (Murphy et al., Biochem J. 227:277-279 (1991); Bebbington et al., Bio/Technology 10:169-175 (1992). Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of proteins.

Derivatives of the plasmid pSV2-dhfr (ATCC Accession No. 37146), the expression vectors pC4 (ATCC Accession No. 209646) and pC6 (ATCC Accession No.209647) contain the strong promoter (LTR) of the Rous Sarcoma Virus (Cullen et al., Molecular and Cellular Biology, 438-447 (March, 1985)) plus a fragment of the CMV-enhancer (Boshart et al., Cell 41:521-530 (1985).) Multiple cloning sites, e.g., with the restriction enzyme cleavage sites BamHI, XbaI and Asp718, facilitate the cloning of the gene of interest. The vectors also contain the 3' intron, the polyadenylation and termination signal of the rat preproinsulin gene, and the mouse DHFR gene under control of the SV40 early promoter.

Specifically, the plasmid pC6, for example, is digested with appropriate restriction enzymes and then dephosphorylated using calf intestinal phosphates by procedures known in the art. The vector is then isolated from a 1% agarose gel.

A polynucleotide of the present invention is amplified according to the protocol outlined in Example 1. If the naturally occurring signal sequence is used to produce the secreted protein, the vector does not need a second signal peptide. Alternatively, if the naturally occurring signal sequence is not used, the vector can be modified to include a heterologous signal sequence. (See, e.g., WO 96/34891.)

The amplified fragment is isolated from a 1% agarose gel using a commercially available kit ("Geneclean," BIO 101 Inc., La Jolla, Ca.). The fragment then is digested with appropriate restriction enzymes and again purified on a 1% agarose gel.

The amplified fragment is then digested with the same restriction enzyme and purified on a 1% agarose gel. The isolated fragment and the dephosphorylated vector are then ligated with T4 DNA ligase. *E. coli* HB 101 or XL-1 Blue cells are then transformed and bacteria are identified that contain the fragment inserted into plasmid pC6 using, for instance, restriction enzyme analysis.

Chinese hamster ovary cells lacking an active DHFR gene is used for transfection. Five µg of the expression plasmid pC6 is cotransfected with 0.5 µg of the plasmid pSVneo using lipofectin (Felgner et al., *supra).* The plasmid pSV2-neo contains a dominant selectable marker, the *neo* gene from Tn5 encoding an enzyme that confers resistance to a group of antibiotics including G418. The cells are seeded in alpha minus MEM supplemented with 1 mg/ml G418. After 2 days, the cells are trypsinized and seeded in hybridoma cloning plates (Greiner, Germany) in alpha minus MEM supplemented with 10, 25, or 50 ng/ml of metothrezate plus 1 mg/ml G418. After about 10-14 days single clones are trypsinized and then seeded in 6-well petri dishes or 10 ml flasks using different concentrations of methotrexate (50 nM, 100 nM, 200 nM, 400 nM, 800 nM). Clones growing at the highest concentrations of methotrexate are then transferred to new 6-well plates containing even higher concentrations of methotrexate (1 µM, 2 µM, 5 µM, 10 mM, 20 mM). The same procedure is repeated until clones are obtained which grow at a concentration of 100 - 200 µM. Expression of the desired gene product is analyzed, for instance, by SDS-PAGE and Western blot or by reversed phase HPLC analysis.

### Example 9: Protein Fusions

The polypeptides of the present invention are preferably fused to other proteins. These fusion proteins can be used for a variety of applications. For example, fusion of the present polypeptides to His-tag, HA-tag, protein A, IgG domains, and maltose binding protein facilitates purification. (See Example 5; see also EP A 394,827; Traunecker, et al., Nature 331:84-86 (1988).) Similarly, fusion to IgG-1, IgG-3, and albumin increases the halflife time in vivo. Nuclear localization signals fused to the polypeptides of the present invention can target the protein to a specific subcellular localization, while covalent heterodimer or homodimers can increase or decrease the activity of a fusion protein. Fusion proteins can also create chimeric molecules having more than one function. Finally, fusion proteins can increase solubility and/or stability of the fused protein compared to the non-fused protein. All of the types of fusion proteins described above can be made by modifying the following protocol, which outlines the fusion of a polypeptide to an IgG molecule, or the protocol described in Example 5.

Briefly, the human Fc portion of the IgG molecule can be PCR amplified, using primers that span the 5' and 3' ends of the sequence described below. These primers also should have convenient restriction enzyme sites that will facilitate cloning into an expression vector, preferably a mammalian expression vector.

For example, if pC4 (Accession No. 209646) is used, the human Fc portion can be ligated into the BamHI cloning site. Note that the 3' BamHI site should be destroyed. Next, the vector containing the human Fc portion is re-restricted with BamHI, linearizing the vector, and a polynucleotide of the present invention, isolated by the PCR protocol described in Example 1, is ligated into this BamHI site. Note that the polynucleotide is cloned without a stop codon, otherwise a fusion protein will not be produced.

If the naturally occurring signal sequence is used to produce the secreted protein, pC4 does not need a second signal peptide. Alternatively, if the naturally occurring signal sequence is not used, the vector can be modified to include a heterologous signal sequence. (See, e.g., WO 96/34891.)
Human IgG Ec region :

GGGATCCGGAGCCCAAATCTTCTGACAAAACTCACACATGCCCACCGTGCC
CAGCACCTGAATTCGAGGGTGCACCGTCAGTCTTCCTCTTCCCCCCAAAACC
CAAGGACACCCTCATGATCTCCCGGACTCCTGAGGTCACATGCGTGGTGGT
GGACGTAAGCCACGAAGACCCTGAGGTCAAGTTCAACTGGTACGTGGACG
GCGTGGAGGTGCATAATGCCAAGACAAAGCCGCGGGAGGAGCAGTACAAC

AGCACGTACCGTGTGGTCAGCGTCCTCACCGTCCTGCACCAGGACTGGCTG
AATGGCAAGGAGTACAAGTGCAAGGTCTCCAACAAAGCCCTCCCAACCCCC
ATCGAGAAAACCATCTCCAAAGCCAAAGGGCAGCCCCGAGAACCACAGGT
GTACACCCTGCCCCCATCCCGGGATGAGCTGACCAAGAACCAGGTCAGCCT
GACCTGCCTGGTCAAAGGCTTCTATCCAAGCGACATCGCCGTGGAGTGGGA
GAGCAATGGGCAGCCGGAGAACAACTACAAGACCACGCCTCCCGTGCTGG
ACTCCGACGGCTCCTTCTTCCTCTACAGCAAGCTCACCGTGGACAAGAGCA
GGTGGCAGCAGGGGAACGTCTTCTCATGCTCCGTGATGCATGAGGCTCTGC
ACAACCACTACACGCAGAAGAGCCTCTCCCTGTCTCCGGGTAAATGAGTGC
GACGGCCGCGACTCTAGAGGAT (SEQ ID NO:1)

### Example 10: Production of an Antibody from a Polypeptide

The antibodies of the present invention can be prepared by a variety of methods. (See, Current Protocols, Chapter 2.) For example, cells expressing a polypeptide of the present invention is administered to an animal to induce the production of sera containing polyclonal antibodies. In a preferred method, a preparation of the secreted protein is prepared and purified to render it substantially free of natural contaminants. Such a preparation is then introduced into an animal in order to produce polyclonal antisera of greater specific activity.

In the most preferred method, the antibodies of the present invention are monoclonal antibodies (or protein binding fragments thereof). Such monoclonal antibodies can be prepared using hybridoma technology. (Köhler et al., Nature 256:495 (1975); Köhler et al., Eur. J. Immunol. 6:511 (1976); Köhler et al., Eur. J. Immunol. 6:292 (1976); Hammerling et al., in: Monoclonal Antibodies and T-Cell Hybridomas, Elsevier, N.Y., pp. 563-681 (1981).) In general, such procedures involve immunizing an animal (preferably a mouse) with polypeptide or, more preferably, with a secreted polypeptide-expressing cell. Such cells may be cultured in any suitable tissue culture medium; however, it is preferable to culture cells in Earle's modified Eagle's medium supplemented with 10% fetal bovine serum (inactivated at about 56°C), and supplemented with about 10 g/1 of nonessential amino acids, about 1,000 U/ml of penicillin, and about 100 µg/ml of streptomycin.

The splenocytes of such mice are extracted and fused with a suitable myeloma cell line. Any suitable myeloma cell line may be employed in accordance with the present invention; however, it is preferable to employ the parent myeloma cell line (SP2O), available from the ATCC. After fusion, the resulting hybridoma cells are selectively maintained in HAT medium, and then cloned by limiting dilution as described by Wands et al. (Gastroenterology 80:225-232 (1981).) The hybridoma cells obtained through such a selection are then assayed to identify clones which secrete antibodies capable of binding the polypeptide.

Alternatively, additional antibodies capable of binding to the polypeptide can be produced in a two-step procedure using anti-idiotypic antibodies. Such a method makes use of the fact that antibodies are themselves antigens, and therefore, it is possible to obtain an antibody which binds to a second antibody. In accordance with this method, protein specific antibodies are used to immunize an animal, preferably a mouse. The splenocytes of such an animal are then used to produce hybridoma cells, and the hybridoma cells are screened to identify clones which produce an antibody whose ability to bind to the protein-specific antibody can be blocked by the polypeptide. Such antibodies comprise anti-idiotypic antibodies to the protein-specific antibody and can be used to immunize an animal to induce formation of further protein-specific antibodies.

It will be appreciated that Fab and F(ab')2 and other fragments of the antibodies of the present invention may be used according to the methods disclosed herein. Such fragments are typically produced by proteolytic cleavage, using enzymes such as papain (to produce Fab fragments) or pepsin (to produce F(ab')2 fragments). Alternatively, secreted protein-binding fragments can be produced through the application of recombinant DNA technology or through synthetic chemistry.

For in vivo use of antibodies in humans, it may be preferable to use "humanized" chimeric monoclonal antibodies. Such antibodies can be produced using genetic constructs derived from hybridoma cells producing the monoclonal antibodies described above. Methods for producing chimeric antibodies are known in the art. (See, for review, Morrison, Science 229:1202 (1985); Oi et al., BioTechniques 4:214 (1986); Cabilly et al., U.S. Patent No. 4,816,567; Taniguchi et al., EP 171496; Morrison et al., EP 173494; Neuberger et al., WO 8601533; Robinson et al., WO 8702671; Boulianne et al., Nature 312:643 (1984); Neuberger et al., Nature 314:268 (1985).)

### Example 11: Production Of Secreted Protein For High-Throughput Screening Assays

The following protocol produces a supernatant containing a polypeptide to be tested. This supernatant can then be used in the Screening Assays described in Examples 13-20.

First, dilute Poly-D-Lysine (644 587 Boehringer-Mannheim) stock solution (1mg/ml in PBS) 1:20 in PBS (w/o calcium or magnesium 17-516F Biowhittaker) for a working solution of 50ug/ml. Add 200 ul of this solution to each well (24 well plates) and incubate at RT for 20 minutes. Be sure to distribute the solution over each well (note: a 12-channel pipetter may be used with tips on every other channel). Aspirate off the Poly-D-Lysine solution and rinse with 1ml PBS (Phosphate Buffered Saline). The PBS should remain in the well until just prior to plating the cells and plates may be poly-lysine coated in advance for up to two weeks.

Plate 293T cells (do not carry cells past P+20) at 2 x 10⁵ cells/well in .5ml DMEM(Dulbecco's Modified Eagle Medium)(with 4.5 G/L glucose and L-glutamine (12-604F Biowhittaker))/10% heat inactivated FBS(14-503F Biowhittaker)/1x Penstrep(17-602E Biowhittaker). Let the cells grow overnight.

The next day, mix together in a sterile solution basin: 300 ul Lipofectamine (18324-012 Gibco/BRL) and 5ml Optimem I (31985070 Gibco/BRL)/96-well plate. With a small volume multi-channel pipetter, aliquot approximately 2ug of an expression vector containing a polynucleotide insert, produced by the methods described in Examples 8 or 9, into an appropriately labeled 96-well round bottom plate. With a multi-channel pipetter, add 50ul of the Lipofectamine/Optimem I mixture to each well. Pipette up and down gently to mix. Incubate at RT 15-45 minutes. After about 20 minutes, use a multi-channel pipetter to add 150ul Optimem I to each well. As a control, one plate of vector DNA lacking an insert should be transfected with each set of transfections.

Preferably, the transfection should be performed by tag-teaming the following tasks. By tag-teaming, hands on time is cut in half, and the cells do not spend too much time on PBS. First, person A aspirates off the media from four 24-well plates of cells, and then person B rinses each well with .5-1ml PBS. Person A then aspirates off PBS rinse, and person B, using a12-channel pipetter with tips on every other channel, adds the 200ul of DNA/Lipofectamine/Optimem I complex to the odd wells first, then to the even wells, to each row on the 24-well plates. Incubate at 37°C for 6 hours.

While cells are incubating, prepare appropriate media, either 1%BSA in DMEM with 1x penstrep, or CHO-5 media ( 116.6 mg/L of CaCl2 (anhyd); 0.00130 mg/L CuSO₄-5H₂O; 0.050 mg/L of Fe(NO₃)₃-9H₂O; 0.417 mg/L of FeSO₄-7H₂O; 311.80 mg/L of Kcl; 28.64 mg/L of MgCl₂; 48.84 mg/L of MgSO₄; 6995.50 mg/L of NaCl; 2400.0 mg/L of NaHCO₃; 62.50 mg/L of NaH₂PO₄-H₂O; 71.02 mg/L of Na₂HPO4; 4320 mg/L of ZnSO₄ 7H₂O; 002 mg/L of Arachidonic Acid ; 1.022 mg/L of Cholesterol; .070 mg/L of DL-alpha-Tocopherol-Acetate; 0.0520 mg/L of Linoleic Acid; 0.010 mg/L of Linolenic Acid; 0.010 mg/L of Myristic Acid; 0.010 mg/L of Oleic. Acid; 0.010 mg/L of Palmitric Acid; 0.010 mg/L of Palmitic Acid; 100 mg/L of Pluronic F-68; 0.010 mg/L of Stearic Acid; 2.20 mg/L of Tween 80; 4551 mg/L of D-Glucose; 130.85 mg/ml of L- Alanine; 147.50 mg/ml of L-Arginine-HCL; 7.50 mg/ml of L-Asparagine-H₂O; 6.65 mg/ml of L-Aspartic Acid; 29.56 mg/ml of L-Cystine-2HCL-H₂O; 31.29 mg/ml of L-Cystine-2HCL; 7.35 mg/ml of L-Glutamic Acid; 365.0 mg/ml of L-Glutamine; 18.75 mg/ml of Glycine; 52.48 mg/ml of L-Histidine-HCL-H₂O; 106.97 mg/ml of L-Isoleucine; 111.45 mg/ml of L-Leucine; 163.75 mg/ml of L-Lysine HCL; 32.34 mg/ml of L-Methionine; 68.48 mg/ml of L-Phenylalainine; 40.0 mg/ml of L-Proline; 26.25 mg/ml of L-Serine; 101.05 mg/ml of L-Threonine; 19.22 mg/ml of L-Tryptophan; 91.79 mg/ml of L-Tryrosine-2Na-2H₂0; 99.65 mg/ml of L-Valine; 0.0035 mg/L of Biotin; 3.24 mg/L of D-Ca Pantothenate; 11.78 mg/L of Choline Chloride; 4.65 mg/L of Folic Acid; 15.60 mg/L of i-Inositol; 3.02 mg/L of Niacinamide; 3.00 mg/L of Pyridoxal HCL; 0.031 mg/L of Pyridoxine HCL; 0.319 mg/L of Riboflavin; 3.17 mg/L of Thiamine HCL; 0.365 mg/L of Thymidine; and 0.680 mg/L of Vitamin B₁₂; 25 mM of HEPES Buffer; 2.39 mg/L of Na Hypoxanthine; 0.105 mg/L of Lipoic Acid; 0.081 mg/L of Sodium Putrescine-2HCL; 55.0 mg/L of Sodium Pyruvate; 0.0067 mg/L of Sodium Selenite; 20uM of Ethanolamine; 0.122 mg/L of Ferric Citrate; 41.70 mg/L of Methyl-B-Cyclodextrin complexed with Linoleic Acid; 33.33 mg/L of Methyl-B-Cyclodextrin complexed with Oleic Acid; and 10 mg/L of Methyl-B-Cyclodextrin complexed with Retinal) with 2mm glutamine and 1x penstrep. (BSA (81-068-3 Bayer) 100gm dissolved in 1L DMEM for a 10% BSA stock solution). Filter the media and collect 50 ul for endotoxin assay in 15ml polystyrene conical.

The transfection reaction is terminated, preferably by tag-teaming, at the end of the incubation period. Person A aspirates off the transfection media, while person B adds 1.5ml appropriate media to each well. Incubate at 37°C for 45 or 72 hours depending on the media used: 1%BSA for 45 hours or CHO-5 for 72 hours.

On day four, using a 300ul multichannel pipetter, aliquot 600ul in one 1ml deep well plate and the remaining supernatant into a 2ml deep well. The supernatants from each well can then be used in the assays described in Examples 13-20.

It is specifically understood that when activity is obtained in any of the assays described below using a supernatant, the activity originates from either the polypeptide directly (e.g., as a secreted protein) or by the polypeptide inducing expression of other proteins, which are then secreted into the supernatant. Thus, the invention further provides a method of identifying the protein in the supernatant characterized by an activity in a particular assay.

### Example 12: Construction of GAS Reporter Construct

One signal transduction pathway involved in the differentiation and proliferation of cells is called the Jaks-STATs pathway. Activated proteins in the Jaks-STATs pathway bind to gamma activation site "GAS" elements or interferon-sensitive responsive element ("ISRE"), located in the promoter of many genes. The binding of a protein to these elements alter the expression of the associated gene.

GAS and ISRE elements are recognized by a class of transcription factors called Signal Transducers and Activators of Transcription, or "STATs." There are six members of the STATs family. Stat1 and Stat3 are present in many cell types. as is Stat2 (as response to IFN-alpha is widespread). Stat4 is more restricted and is not in many cell types though it has been found in T helper class I, cells after treatment with IL-12. Stat5 was originally called mammary growth factor, but has been found at higher concentrations in other cells including myeloid cells. It can be activated in tissue culture cells by many cytokines.

The STATs are activated to translocate from the cytoplasm to the nucleus upon tyrosine phosphorylation by a set of kinases known as the Janus Kinase ("Jaks") family. Jaks represent a distinct family of soluble tyrosine kinases and include Tyk2, Jak1, Jak2, and Jak3. These kinases display significant sequence similarity and are generally catalytically inactive in resting cells.

The Jaks are activated by a wide range of receptors summarized in the Table below. (Adapted from review by Schidler and Damell, Ann. Rev. Biochem. 64:621-51 (1995).) A cytokine receptor family, capable of activating Jaks, is divided into two groups: (a) Class 1 includes receptors for IL-2, IL-3, IL-4, IL-6, IL-7, IL-9, IL-11, IL-12, IL-15, Epo, PRL, GH, G-CSF, GM-CSF, LIF, CNTF, and thrombopoietin; and (b) Class 2 includes IFN-a, IFN-g, and IL-10. The Class 1 receptors share a conserved cysteine motif (a set of four conserved cysteines and one tryptophan) and a WSXWS motif (a membrane proxial region encoding Trp-Ser-Xxx-Trp-Ser (SEQ ID NO:2)).

Thus, on binding of a ligand to a receptor, Jaks are activated, which in turn activate STATs, which then translocate and bind to GAS elements. This entire process is encompassed in the Jaks-STATs signal transduction pathway.

Therefore, activation of the Jaks-STATs pathway, reflected by the binding of the GAS or the ISRE element, can be used to indicate proteins involved in the proliferation and differentiation of cells. For example, growth factors and cytokines are known to activate the Jaks-STATs pathway. (See Table below.) Thus, by using GAS elements linked to reporter molecules, activators of the Jaks-STATs pathway can be identified.

| | JAKs | | | | STATS | GAS(elements) or ISRE |
|---|---|---|---|---|---|---|
| Ligand | tyk2 | Jak1 | Jak2 | Jak3 | | |
| IFN family | | | | | | |
| IFN-a/B | + | + | - | - | 1,2,3 | ISRE |
| IFN-g | | + | + | - | 1 | GAS (IRF1>Lys6>IFP) |
| Il-10 | + | ? | ? | - | 1,3 | |
| | | | | | | |

| gp130 family | | | | | | |
|---|---|---|---|---|---|---|
| IL-6 (Pleiotrohic) | + | + | + | ? | 1,3 | GAS (IRF1>Lys6>IFP) |
| Il-11(Pleiotrohic) | ? | + | ? | ? | 1,3 | |
| OnM(Pleiotrohic) | ? | + | + | ? | 1,3 | |
| LIF(Pleiotrohic) | ? | + | + | ? | 1,3 | |
| CNTF(Pleiotrohic) | -/+ | + | + | ? | 1,3 | |
| G-CSF(Pleiotrohic) | ? | + | ? | ? | 1,3 | |
| IL-12(Pleiotrohic) | + | - | + | + | 1,3 | |
| | | | | | | |

| g-C family | | | | | | |
|---|---|---|---|---|---|---|
| IL-2 (lymphocytes) | - | + | - | + | 1,3,5 | GAS |
| IL-4 (lymph/myeloid) - | | + | - | + | 6 | GAS (IRF1 = IFP >>Ly6)(IgH) |
| IL-7 (lymphocytes) | - | + | - | + | 5 | GAS |
| IL-9 (lymphocytes) | - | + | - | + | 5 | GAS |
| IL-13 (lymphocyte) | - | + | ? | ? | 6 | GAS |
| IL-15 | ? | + | ? | + | 5 | GAS |
| | | | | | | |

| gp140 family | | | | | | |
|---|---|---|---|---|---|---|
| IL-3 (myeloid) | - | - | + | - | 5 | GAS (IRF1>IFP>>Ly6) |
| IL-5 (myeloid) | - | - | + | - | 5 | GAS |
| GM-CSF (myeloid) | - | - | + | - | 5 | GAS |
| | | | | | | |

| Growth hormone family | | | | | | |
|---|---|---|---|---|---|---|
| GH | ? | - | + | - | 5 | |
| PRL | ? | +/- | + | - | 1,3,5 | |
| EPO | ? | - | + | - | 5 | GAS(B-CAS>IRFI=IFP»Ly6) |
| | | | | | | |

| Receptor Tyrosine Kinases | | | | | | |
|---|---|---|---|---|---|---|
| EGF | ? | + | + | - | 1,3 | GAS (IRF1). |
| PDGF | ? | + | + | - - | 1,3 | |
| CSF-1 | ? | + | + | - | 1,3 | GAS (not IRF1) |

To construct a synthetic GAS containing promoter element, which is used in the Biological Assays described in Examples 13-14, a PCR based strategy is employed to generate a GAS-SV40 promoter sequence. The 5' primer contains four tandem copies of the GAS binding site found in the IRF1 promoter and previously demonstrated to bind STATs upon induction with a range of cytokines (Rothman et al., Immunity 1:457-468 (1994).), although other GAS or ISRE elements can be used instead. The 5' primer also contains 18bp of sequence complementary to the SV40 early promoter sequence and is flanked with an XhoI site. The sequence of the 5' primer is:

5':GCGCCTCGAGATTTCCCCGAAATCTAGATTTCCCCGAAATGATTTCCCCG
AAATGATTTCCCCGAAATATCTGCCATCTCAATTAG:3' (SEQ ID NO:3)

The downstream primer is complementary to the SV40 promoter and is flanked with a Hind III site: 5':GCGGCAAGCTTTTTGCAAAGCCTAGGC:3' (SEQ ID NO:4)

PCR amplification is performed using the SV40 promoter template present in the B-gal:promoter plasmid obtained from Clontech. The resulting PCR fragment is digested with XhoI/Hind III and subcloned into BLSK2-. (Stratagene.) Sequencing with forward and reverse primers confirms that the insert contains the following sequence:

5':CTCGAGATTTCCCCGAAATCTAGATTTCCCCGAAATGATTTCCCCGAAATG
ATTTCCCCGAAATATCTGCCATCTCAATTAGTCAGCAACCATAGTCCCGCCC
CTAACTCCGCCCATCCCGCCCCTAACTCCGCCCAGTTCCGCCCATTCTCCGC
CCCATGGCTGACTAATTTTTTTTATTTATGCAGAGGCCGAGGCCGCCTCGGC
CTCTGAGCTATTCCAGAAGTAGTGAGGAGGCTTTTTTGGAGGCCTAGGCTTT
TGCAAAAAGCTT:3' (SEQ ID NO:5)

With this GAS promoter element linked to the SV40 promoter, a GAS:SEAP2 reporter construct is next engineered. Here, the reporter molecule is a secreted alkaline phosphatase, or "SEAP." Clearly, however, any reporter molecule can be instead of SEAP, in this or in any of the other Examples. Well known reporter molecules that can be used instead of SEAP include chloramphenicol acetyltransferase (CAT), luciferase, alkaline phosphatase, B-galactosidase, green fluorescent protein (GFP), or any protein detectable by an antibody.

The above sequence confirmed synthetic GAS-SV40 promoter element is subcloned into the pSEAP-Promoter vector obtained from Clontech using HindIII and XhoI, effectively replacing the SV40 promoter with the amplified GAS:SV40 promoter element, to create the GAS-SEAP vector. However, this vector does not contain a neomycin resistance gene, and therefore, is not preferred for mammalian expression systems.

Thus, in order to generate mammalian stable cell lines expressing the GAS-SEAP reporter, the GAS-SEAP cassette is removed from the GAS-SEAP vector using SaII and NotI, and inserted into a backbone vector containing the neomycin resistance gene, such as pGFP-1 (Clontech), using these restriction sites in the multiple cloning site, to create the GAS-SEAP/Neo vector. Once this vector is transfected into mammalian cells, this vector can then be used as a reporter molecule for GAS binding as described in Examples 13-14.

Other constructs can be made using the above description and replacing GAS with a different promoter sequence. For example, construction of reporter molecules containing NFK-B and EGR promoter sequences are described in Examples 15 and 16. However, many other promoters can be substituted using the protocols described in these Examples. For instance, SRE, IL-2, NFAT, or Osteocalcin promoters can be substituted, alone or in combination (e.g., GAS/NF-KB/EGR, GAS/NF-KB,II 2/NFAT, or NF-KB/GAS). Similarly, other cell lines can be used to test reporter construct activity, such as HELA (epithelial), HUVEC (endothelial), Reh (B-cell), Saos-2 (osteoblast), HUVAC (aortic), or Cardiomyocyte.

### Example 13: High-Throughput Screening Assay for T-cell Activity.

The following protocol is used to assess T-cell activity by identifying factors, such as growth factors and cytokines, that may proliferate or differentiate T-cells. T-cell activity is assessed using the GAS/SEAP/Neo construct produced in Example 12. Thus, factors that increase SEAP activity indicate the ability to activate the Jaks-STATS signal transduction pathway. The T-cell used in this assay is Jurkat T-cells (ATCC Accession No. TIB-152), although Molt-3 cells (ATCC Accession No. CRL-1552) and ( Molt-4 cells (ATCC Accession No. CRL-1582) cells can also be used.

Jurkat T-cells are lymphoblastic CD4+ Thl helper cells. In order to generate stable cell lines, approximately 2 million Jurkat cells are transfected with the GAS-SEAP/neo vector using DMRIE-C (Life Technologies)(transfection procedure described below). The transfected cells are seeded to a density of approximately 20,000 cells per well and transfectants resistant to 1 mg/ml genticin selected. Resistant colonies are expanded and then tested for their response to increasing concentrations of interferon gamma. The dose response of a selected clone is demonstrated.

Specifically, the following protocol will yield sufficient cells for 75 wells containing 200 ul of cells. Thus, it is either scaled up, or performed in multiple to generate sufficient cells for multiple 96 well plates. Jurkat cells are maintained in RPMI + 10% serum with 1%Pen-Strep. Combine 2.5 mls of OPTI-MEM (Life Technologies) with 10 ug of plasmid DNA in a T25 flask. Add 2.5 ml OPTI-MEM containing 50 ul of DMRIE-C and incubate at room temperature for 15-45 mins.

During the incubation period, count cell concentration, spin down the required number of cells (10⁷ per transfection), and resuspend in OPTI-MEM to a final concentration of 10⁷ cells/ml. Then add 1ml of 1 x 10⁷ cells in OPTI-MEM to T25 flask and incubate at 37°C for 6 hrs. After the incubation, add 10 ml of RPMI + 15% serum.

The Jurkat:GAS-SEAP stable reporter lines are maintained in RPMI + 10% serum, 1 mg/ml Genticin, and 1% Pen-Strep. These cells are treated with supernatants containing a polypeptide as produced by the protocol described in Example 11.

On the day of treatment with the supernatant, the cells should be washed and resuspended in fresh RPMI + 10% serum to a density of 500,000 cells per ml. The exact number of cells required will depend on the number of supernatants being screened. For one 96 well plate, approximately 10 million cells (for 10 plates, 100 million cells) are required.

Transfer the cells to a triangular reservoir boat, in order to dispense the cells into a 96 well dish, using a 12 channel pipette. Using a 12 channel pipette, transfer 200 ul of cells into each well (therefore adding 100, 000 cells per well).

After all the plates have been seeded, 50 ul of the supernatants are transferred directly from the 96 well plate containing the supernatants into each well using a 12 channel pipette. In addition, a dose of exogenous interferon gamma (0.1, 1.0, 10 ng) is added to wells H9, H10, and H11 to serve as additional positive controls for the assay.

The 96 well dishes containing Jurkat cells treated with supernatants are placed in an incubator for 48 hrs (note: this time is variable between 48-72 hrs). 35 ul samples from each well are then transferred to an opaque 96 well plate using a 12 channel pipette. The opaque plates should be covered (using sellophene covers) and stored at-20°C until SEAP assays are performed according to Example 17. The plates containing the remaining treated cells are placed at 4°C and serve as a source of material for repeating the assay on a specific well if desired.

As a positive control, 100 Unit/ml interferon gamma can be used which is known to activate Jurkat T cells. Over 30 fold induction is typically observed in the positive control wells.

### Example 14: High-Throughput Screening Assay Identifying Myeloid Activity

The following protocol is used to assess myeloid activity by identifying factors, such as growth factors and cytokines, that may proliferate or differentiate myeloid cells. Myeloid cell activity is assessed using the GAS/SEAP/Neo construct produced in Example 12. Thus, factors that increase SEAP activity indicate the ability to activate the Jaks-STATS signal transduction pathway. The myeloid cell used in this assay is U937, a pre-monocyte cell line, although TF-1, HL60, or KG1 can be used.

To transiently transfect U937 cells with the GAS/SEAP/Neo construct produced in Example 12, a DEAE-Dextran method (Kharbanda et. al., 1994, Cell Growth & Differentiation, 5:259-265) is used. First, harvest 2x10e⁷ U937 cells and wash with PBS. The U937 cells are usually grown in RPMI 1640 medium containing 10% heat-inactivated fetal bovine serum (FBS) supplemented with 100 units/ml penicillin and 100 mg/ml streptomycin.

Next, suspend the cells in 1 ml of 20 mM Tris-HCl (pH 7.4) buffer containing 0.5 mg/ml DEAE-Dextran, 8 ug GAS-SEAP2 plasmid DNA, 140 mM NaCl, 5 mM KCl, 375 uM Na₂HPO₄.7H₂O, 1 mM MgCl₂, and 675 uM CaCl₂. Incubate at 37°C for 45 min.

Wash the cells with RPMI 1640 medium containing 10% FBS and then resuspend in 10 ml complete medium and incubate at 37°C for 36 hr.

The GAS-SEAP/U937 stable cells are obtained by growing the cells in 400 ug/ml G418. The G418-free medium is used for routine growth but every one to two months, the cells should be re-grown in 400 ug/ml G418 for couple of passages.

These cells are tested by harvesting 1x10⁸ cells (this is enough for ten 96-well plates assay) and wash with PBS. Suspend the cells in 200 ml above described growth medium, with a final density of 5x10⁵ cells/ml. Plate 200 ul cells per well in the 96-well plate (or 1x10⁵ cells/well).

Add 50 ul of the supernatant prepared by the protocol described in Example 11. Incubate at 37°C for 48 to 72 hr. As a positive control, 100 Unit/ml interferon gamma can be used which is known to activate U937 cells. Over 30 fold induction is typically observed in the positive control wells. SEAP assay the supernatant according to the protocol described in Example 17.

### Example 15: High-Throughput Screening Assay Identifying Neuronal Activity.

When cells undergo differentiation and proliferation, a group of genes are activated through many different signal transduction pathways. One of these genes, EGRI (early growth response gene 1), is induced in various tissues and cell types upon activation. The promoter of EGR1 is responsible for such induction. Using the EGR1 promoter linked to reporter molecules, activation of cells can be assessed.

Particularly, the following protocol is used to assess neuronal activity in PC12 cell lines. PC12 cells (rat phenochromocytoma cells) are known to proliferate and/or differentiate by activation with a number of mitogens, such as TPA (tetradecanoyl phorbol acetate), NGF (nerve growth factor), and EGF (epidermal growth factor). The EGR1 gene expression is activated during this treatment. Thus, by stably transfecting PC12 cells with a construct containing an EGR promoter linked to SEAP reporter, activation of PC12 cells can be assessed.

The EGR/SEAP reporter construct can be assembled by the following protocol. The EGR-1 promoter sequence (-633 to +1)(Sakamoto K et al., Oncogene 6:867-871 (1991)) can be PCR amplified from human genomic DNA using the following primers:

5' GCGCTCGAGGGATGACAGCGATAGAACCCCGG -3' (SEQ ID NO:6)

5' GCGAAGCTTCGCGACTCCCCGGATCCGCCTC-3' (SEQ ID NO:7)

Using the GAS:SEAP/Neo vector produced in Example 12, EGR1 amplified product can then be inserted into this vector. Linearize the GAS:SEAP/Neo vector using restriction enzymes XhoI/HindIII, removing the GAS/SV40 stuffer. Restrict the EGR1 amplified product with these same enzymes. Ligate the vector and the EGR1 promoter.

To prepare 96 well-plates for cell culture, two mls of a coating solution (1:30 dilution of collagen type I (Upstate Biotech Inc. Cat#08-115) in 30% ethanol (filter sterilized)) is added per one 10 cm plate or 50 ml per well of the 96-well plate, and allowed to air dry for 2 hr.

PC12 cells are routinely grown in RPMI-1640 medium (Bio Whittaker) containing 10% horse serum (JRH BIOSCIENCES, Cat. # 12449-78P), 5% heat-inactivated fetal bovine serum (FBS) supplemented with 100 units/ml penicillin and 100 ug/ml streptomycin on a precoated 10 cm tissue culture dish. One to four split is done every three to four days. Cells are removed from the plates by scraping and resuspended with pipetting up and down for more than 15 times.

Transfect the EGR/SEAP/Neo construct into PC12 using the Lipofectamine protocol described in Example 11. EGR-SEAP/PC12 stable cells are obtained by growing the cells in 300 ug/ml G418. The G418-free medium is used for routine growth but every one to two months, the cells should be re-grown in 300 ug/ml G418 for couple of passages.

To assay for neuronal activity, a 10 cm plate with cells around 70 to 80% confluent is screened by removing the old medium. Wash the cells once with PBS (Phosphate buffered saline). Then starve the cells in low serum medium (RPMI-1640 containing 1% horse serum and 0.5% FBS with antibiotics) overnight.

The next morning, remove the medium and wash the cells with PBS. Scrape off the cells from the plate, suspend the cells well in 2 ml low serum medium. Count the cell number and add more low serum medium to reach final cell density as 5x10⁵ cells/ml.

Add 200 ul of the cell suspension to each well of 96-well plate (equivalent to 1x10⁵ cells/well). Add 50 ul supernatant produced by Example 11, 37°C for 48 to 72 hr. As a positive control, a growth factor known to activate PC12 cells through EGR can be used, such as 50 ng/ul of Neuronal Growth Factor (NGF). Over fifty-fold induction of SEAP is typically seen in the positive control wells. SEAP assay the supernatant according to Example 17.

### Example 16: High-Throughput Screening Assay for T-cell Activity

NF-κB (Nuclear Factor κB) is a transcription factor activated by a wide variety of agents including the inflammatory cytokines IL-1 and TNF, CD30 and CD40, lymphotoxin-alpha and lymphotoxin-beta, by exposure to LPS or thrombin, and by expression of certain viral gene products. As a transcription factor, NF-κB regulates the expression of genes involved in immune cell activation, control of apoptosis (NF-κB appears to shield cells from apoptosis), B and T-cell development, anti-viral and antimicrobial responses, and multiple stress responses.

In non-stimulated conditions, NF- κB is retained in the cytoplasm with I-κB (Inhibitor κB). However, upon stimulation, I-κB is phosphorylated and degraded, causing NF- κB to shuttle to the nucleus, thereby activating transcription of target genes. Target genes activated by NF-κB include IL-2, IL-6, GM-CSF, ICAM-1 and class 1 MHC.

Due to its central role and ability to respond to a range of stimuli, reporter constructs utilizing the NF-κB promoter element are used to screen the supernatants produced in Example 11. Activators or inhibitors of NF-kB would be useful in treating diseases. For example, inhibitors of NF-κB could be used to treat those diseases related to the acute or chronic activation of NF-kB, such as rheumatoid arthritis.

To construct a vector containing the NF-κB promoter element, a PCR based strategy is employed. The upstream primer contains four tandem copies of the NF-κB binding site (GGGGACTTTCCC) (SEQ ID NO:8), 18 bp of sequence complementary to the 5' end of the SV40 early promoter sequence, and is flanked with an XhoI site:

5':GCGGCCTCGAGGGGACTTTCCCGGGGACTTTCCGGGGACTTTCCGGGAC
TTTCCATCCTGCCATCTCAATTAG:3' (SEQ ID NO:9)

The downstream primer is complementary to the 3' end of the SV40 promoter and is flanked with a Hind III site:

5':GCGGCAAGCTTTTTGCAAAGCCTAGGC:3' (SEQ ID NO:4)

PCR amplification is performed using the SV40 promoter template present in the pB-gal:promoter plasmid obtained from Clontech. The resulting PCR fragment is digested with XhoI and Hind III and subcloned into BLSK2-. (Stratagene) Sequencing with the T7 and T3 primers confirms the insert contains the following sequence:

5':CTCGAGGGGACTTTCCCGGGGACTTTCCGGGGACTTTCCGGGACTTTCC
ATCTGCCATCTCAATTAGTCAGCAACCATAGTCCCGCCCCTAACTCCGCCCA
TCCCGCCCCTAACTCCGCCCAGTTCCGCCCATTCTCCGCCCCATGGCTGACT
AATTTTTTTTATTTATGCAGAGGCCGAGGCCGCCTCGGCCTCTGAGCTATTC
CAGAAGTAGTGAGGAGGCTTTTTTGGAGGCCTAGGCTTTTGCAAAAAGCTT:
3' (SEQ ID NO:10)

Next, replace the SV40 minimal promoter element present in the pSEAP2-promoter plasmid (Clontech) with this NF-κB/SV40 fragment using XhoI and HindIII. However, this vector does not contain a neomycin resistance gene, and therefore, is not preferred for mammalian expression systems.

In order to generate.stable mammalian cell lines, the NF-κB/SV40/SEAP cassette is removed from the above NF-κB/SEAP vector using restriction enzymes SalI and NotI, and inserted into a vector containing neomycin resistance. Particularly, the NF-κB/SV40/SEAP cassette was inserted into pGFP-1 (Clontech), replacing the GFP gene, after restricting pGFP-1 with SalI and NotI.

Once NF-κB/SV40/SEAP/Neo vector is created, stable Jurkat T-cells are created and maintained according to the protocol described in Example 13. Similarly, the method for assaying supernatants with these stable Jurkat T-cells is also described in Example 13. As a positive control, exogenous TNF alpha (0.1,1, 10 ng) is added to wells H9, H 10, and H11, with a 5-10 fold activation typically observed.

### Example 17: Assay for SEAP Activity

As a reporter molecule for the assays described in Examples 13-16, SEAP activity is assayed using the Tropix Phospho-light Kit (Cat. BP-400) according to the following general procedure. The Tropix Phospho-light Kit supplies the Dilution, Assay, and Reaction Buffers used below.

Prime a dispenser with the 2.5x Dilution Buffer and dispense 15 µl of 2.5x dilution buffer into Optiplates containing 35 µl of a supernatant. Seal the plates with a plastic sealer and incubate at 65°C for 30 min. Separate the Optiplates to avoid uneven heating.

Cool the samples to room temperature for 15 minutes. Empty the dispenser and prime with the Assay Buffer. Add 50 µl Assay Buffer and incubate at room temperature 5 min. Empty the dispenser and prime with the Reaction Buffer (see the table below). Add 50 µl Reaction Buffer and incubate at room temperature for 20 minutes. Since the intensity of the chemiluminescent signal is time dependent, and it takes about 10 minutes to read 5 plates on luminometer, one should treat 5 plates at each time and start the second set 10 minutes later.

Read the relative light unit in the luminometer. Set H12 as blank, and print the results. An increase in chemiluminescence indicates reporter activity.

| **Reaction Buffer Formulation:** | | |
|---|---|---|
| # of plates | Rxn buffer diluent (ml) | CSPD (ml) |
| 10 | 60 | 3 |
| 11 | 65 | 3.25 |
| 12 | 70 | 3.5 |
| 13 | 75 | 3.75 |
| 14 | 80 | 4 |
| 15 | 85 | 4.25 |
| 16 | 90 | 4.5 |
| 17 | 95 | 4.75 |
| 18 | 100 | 5 |
| 19 | 105 | 5.25 |
| 20 | 110 | 5.5 |
| 21 | 115 | 5.75 |
| 22 | 120 | 6 |
| 23 | 125 | 6.25 |
| 24 | 130 | 6.5 |
| 25 | 135 | 6.75 |
| 26 | 140 | 7 |
| 27 | 145 | 7.25 |
| 28 | 150 | 7.5 |
| 29 | 155 | 7.75 |
| 30 | 160 | 8 |
| 31 | 165 | 8.25 |
| 32 | 170 | 8.5 |
| 33 | 175 | 8.75 |
| 34 | 180 | 9 |
| 35 | 185 | 9.25 |
| 36 | 190 | 9.5 |
| 37 | 195 | 9.75 |
| 38 | 200 | 10 |
| 39 | 205 | 10.25 |
| 40 | 210 | 10.5 |
| 41 | 215 | 10.75 |
| 42 | 220 | 11 |
| 43 | 225 | 11.25 |
| 44 | 230 | 11.5 |
| 45 | 235 | 11.75 |
| 46 | 240 | 12 |
| 47 | 245 | 12.25 |
| 48 | 250 | 12.5 |
| 49 | 255 | 12.75 |
| 50 | 260. | 13 |

### Example 18: High-Throughput Screening Assay Identifying Changes in Small Molecule Concentration and Membrane Permeability

Binding of a ligand to a receptor is known to alter intracellular levels of small molecules, such as calcium, potassium, sodium, and pH, as well as alter membrane potential. These alterations can be measured in an assay to identify supernatants which bind to receptors of a particular cell. Although the following protocol describes an assay for calcium, this protocol can easily be modified to detect changes in potassium, sodium, pH, membrane potential, or any other small molecule which is detectable by a fluorescent probe.

The following assay uses Fluorometric Imaging Plate Reader ("FLIPR") to measure changes in fluorescent molecules (Molecular Probes) that bind small molecules. Clearly, any fluorescent molecule detecting a small molecule can be used instead of the calcium fluorescent molecule, fluo-3, used here.

For adherent cells, seed the cells at 10,000 -20,000 cells/well in a Co-star black 96-well plate with clear bottom. The plate is incubated in a CO₂ incubator for 20 hours. The adherent cells are washed two times in Biotek washer with 200 ul of HBSS (Hank's Balanced Salt Solution) leaving 100 ul of buffer after the final wash.

A stock solution of 1 mg/ml fluo-3 is made in 10% pluronic acid DMSO. To load the cells with fluo-3, 50 ul of 12 ug/ml fluo-3 is added to each well. The plate is incubated at 37°C in a CO₂ incubator for 60 min. The plate is washed four times in the Biotek washer with HBSS leaving 100 ul of buffer.

For non-adherent cells, the cells are spun down from culture media. Cells are re-suspended to 2-5x10⁶ cells/ml with HBSS in a 50-ml conical tube. 4 ul of 1mg/ml fluo-3 solution in 10% pluronic acid DMSO is added to each ml of cell suspension. The tube is then placed in a 37°C water bath for 30-60 min. The cells are washed twice with HBSS, resuspended to 1x10⁶ cells/ml, and dispensed into a microplate, 100 ul/well. The plate is centrifuged at 1000 rpm for 5 min. The plate is then washed once in Denley CellWash with 200 ul, followed by an aspiration step to 100 ul final volume.

For a non-cell based assay, each well contains a fluorescent molecule, such as fluo-3. The supernatant is added to the well, and a change in fluorescence is detected.

To measure the fluorescence of intracellular calcium, the FLIPR is set for the following parameters: (1) System gain is 300-800 mW; (2) Exposure time is 0.4 second; (3) Camera F/stop is F/2; (4) Excitation is 488 nm; (5) Emission is 530 nm; and (6) Sample addition is 50 ul. Increased emission at 530 nm indicates an extracellular signaling event which has resulted in an increase in the intracellular Ca⁺⁺ concentration.

### Example 19: High-Throughput Screening Assay Identifying Tyrosine Kinase Activity

The Protein Tyrosine Kinases (PTK) represent a diverse group of transmembrane and cytoplasmic kinases. Within the Receptor Protein Tyrosine Kinase RPTK) group are receptors for a range of mitogenic and metabolic growth factors including the PDGF, FGF, EGF, NGF, HGF and Insulin receptor subfamilies. In addition there are a large family of RPTKs for which the corresponding ligand is unknown. Ligands for RPTKs include mainly secreted small proteins, but also membrane-bound and extracellular matrix proteins.

Activation of RPTK by ligands involves ligand-mediated receptor dimerization, resulting in transphosphorylation of the receptor subunits and activation of the cytoplasmic tyrosine kinases. The cytoplasmic tyrosine kinases include receptor associated tyrosine kinases of the src-family (e.g., src, yes, lck, lyn, fyn) and non-receptor linked and cytosolic protein tyrosine kinases, such as the Jak family, members of which mediate signal transduction triggered by the cytokine superfamily of receptors (e.g., the Interleukins, Interferons, GM-CSF, and Leptin).

Because of the wide range of known factors capable of stimulating tyrosine kinase activity, the identification of novel human secreted proteins capable of activating tyrosine kinase signal transduction pathways are of interest. Therefore, the following protocol is designed to identify those novel human secreted proteins capable of activating the tyrosine kinase signal transduction pathways.

Seed target cells (e.g., primary keratinocytes) at a density of approximately 25,000 cells per well in a 96 well Loprodyne Silent Screen Plates purchased from Nalge Nunc (Naperville, IL). The plates are sterilized with two 30 minute rinses with 100% ethanol, rinsed with water and dried overnight. Some plates are coated for 2 hr with 100 ml of cell culture grade type I collagen (50 mg/ml), gelatin (2%) or polylysine (50 mg/ml), all of which can be purchased from Sigma Chemicals (St. Louis, MO) or 10% Matrigel purchased from Becton Dickinson (Bedford,MA), or calf serum, rinsed with PBS and stored at 4°C. Cell growth on these plates is assayed by seeding 5,000 cells/well in growth medium and indirect quantitation of cell number through use of alamarBlue as described by the manufacturer Alamar Biosciences, Inc. (Sacramento, CA) after 48 hr. Falcon plate covers #3071 from Becton Dickinson (Bedford,MA) are used to cover the Loprodyne Silent Screen Plates. Falcon Microtest III cell culture plates can also be used in some proliferation experiments.

To prepare extracts, A431 cells are seeded onto the nylon membranes of Loprodyne plates (20,000/200ml/well) and cultured overnight in complete medium. Cells are quiesced by incubation in serum-free basal medium for 24 hr. After 5-20 minutes treatment with EGF (60ng/ml) or 50 ul of the supernatant produced in Example 11, the medium was removed and 100 ml of extraction buffer ((20 mM HEPES pH 7.5,0.15 M NaCl, 1% Triton X-100, 0.1% SDS, 2 mM Na3VO4, 2 mM Na4P2O7 and a cocktail of protease inhibitors (# 1836170) obtained from Boeheringer Mannheim (Indianapolis, IN) is added to each well and the plate is shaken on a rotating shaker for 5 minutes at 4°C. The plate is then placed in a vacuum transfer manifold and the extract filtered through the 0.45 mm membrane bottoms of each well using house vacuum. Extracts are collected in a 96-well catch/assay plate in the bottom of the vacuum manifold and immediately placed on ice. To obtain extracts clarified by centrifugation, the content of each well, after detergent solubilization for 5 minutes, is removed and centrifuged for 15 minutes at 4°C at 16,000 x g.

Test the filtered extracts for levels of tyrosine kinase activity. Although many methods of detecting tyrosine kinase activity are known, one method is described here.

Generally, the tyrosine kinase activity of a supernatant is evaluated by determining its ability to phosphorylate a tyrosine residue on a specific substrate (a biotinylated peptide). Biotinylated peptides that can be used for this purpose include PSK1 (corresponding to amino acids 6-20 of the cell division kinase cdc2-p34) and PSK2 (corresponding to amino acids 1-17 of gastrin). Both peptides are substrates for a range of tyrosine kinases and are available from Boehringer Mannheim.

The tyrosine kinase reaction is set up by adding the following components in order. First, add 10ul of 5uM Biotinylated Peptide, then 10ul ATP/Mg₂₊ (5mM ATP/50mM MgCl₂), then 10ul of 5x Assay Buffer (40mM imidazole hydrochloride, pH7.3, 40 mM beta-glycerophosphate, 1mM EGTA, 100mM MgCl₂, 5 mM MnCl₂, 0.5 mg/ml BSA), then 5ul of Sodium Vanadate(lmM), and then 5ul of water. Mix the components gently and preincubate the reaction mix at 30°C for 2 min. Initial the reaction by adding 10ul of the control enzyme or the filtered supernatant.

The tyrosine kinase assay reaction is then terminated by adding 10 ul of 120mm EDTA and place the reactions on ice.

Tyrosine kinase activity is determined by transferring 50 ul aliquot of reaction mixture to a microtiter plate (MTP) module and incubating at 37°C for 20 min. This allows the streptavadin coated 96 well plate to associate with the biotinylated peptide. Wash the MTP module with 300ul/well of PBS four times. Next add 75 ul of anti-phospotyrosine antibody conjugated to horse radish peroxidase(anti-P-Tyr-POD(0.5u/ml)) to each well and incubate at 37°C for one hour. Wash the well as above.

Next add 100ul of peroxidase substrate solution (Boehringer Mannheim) and incubate at room temperature for at least 5 mins (up to 30 min). Measure the absorbance of the sample at 405 nm by using ELISA reader. The level of bound peroxidase activity is quantitated using an ELISA reader and reflects the level of tyrosine kinase activity.

### Example 20: High-Throughput Screening Assay Identifying Phosphorylation Activity

As a potential alternative and/or compliment to the assay of protein tyrosine kinase activity described in Example 19, an assay which detects activation (phosphorylation) of major intracellular signal transduction intermediates can also be used. For example, as described below one particular assay can detect tyrosine phosphorylation of the Erk-1 and Erk-2 kinases. However, phosphorylation of other molecules, such as Raf, JNK,. p38 MAP, Map kinase kinase (MEK), MEK kinase, Src, Muscle specific kinase (MuSK), IRAK, Tec, and Janus, as well as any other phosphoserine, phosphotyrosine, or phosphothreonine molecule, can be detected by substituting these molecules for Erk-1 or Erk-2 in the following assay.

Specifically, assay plates are made by coating the wells of a 96-well ELISA plate with 0.1ml of protein G (1ug/ml) for 2 hr at room temp, (RT). The plates are then rinsed with PBS and blocked with 3% BSA/PBS for 1 hr at RT. The protein G plates are then treated with 2 commercial monoclonal antibodies (100ng/well) against Erk-1 and Erk-2 (1hr at RT) (Santa Cruz Biotechnology). (To detect other molecules, this step can easily be modified by substituting a monoclonal antibody detecting any of the above described molecules.) After 3-5 rinses with PBS, the plates are stored at 4°C until use.

A431 cells are seeded at 20,000/well in a 96-well Loprodyne filterplate and cultured overnight in growth medium. The cells are then starved for 48 hr in basal medium (DMEM) and then treated with EGF (6ng/well) or 50 ul of the supernatants obtained in Example 11 for 5-20 minutes. The cells are then solubilized and extracts filtered directly into the assay plate.

After incubation with the extract for 1 hr at RT, the wells are again rinsed. As a positive control, a commercial preparation of MAP kinase (10ng/well) is used in place of A431 extract. Plates are then treated with a commercial polyclonal (rabbit) antibody (1ug/ml) which specifically recognizes the phosphorylated epitope of the Erk-1 and Erk-2 kinases (1hr at RT). This antibody is biotinylated by standard procedures. The bound polyclonal antibody is then quantitated by successive incubations with Europium-streptavidin and Europium fluorescence enhancing reagent in the Wallac DELFIA instrument (time-resolved fluorescence). An increased fluorescent signal over background indicates a phosphorylation.

### Example 21: Method of Determining Alterations in a Gene Corresponding to a Polynucleotide

RNA isolated from entire families or individual patients presenting with a phenotype of interest (such as a disease) is be isolated. cDNA is then generated from these RNA samples using protocols known in the art. (See, Sambrook.) The cDNA is then used as a template for PCR, employing primers surrounding regions of interest in SEQ ID NO:X. Suggested PCR conditions consist of 35 cycles at 95°C for 30 seconds; 60-120 seconds at 52-58°C; and 60-120 seconds at 70°C, using buffer solutions described in Sidransky, D., et al., Science 252:706 (1991).

PCR products are then sequenced using primers labeled at their 5' end with T4 polynucleotide kinase, employing SequiTherm Polymerase. (Epicentre Technologies). The intron-exon borders of selected exons is also determined and genomic PCR products analyzed to confirm the results. PCR products harboring suspected mutations is then cloned and sequenced to validate the results of the direct sequencing.

PCR products is cloned into T-tailed vectors as described in Holton, T.A. and Graham, M.W., Nucleic Acids Research, 19:1156 (1991) and sequenced with T7 polymerase (United States Biochemical). Affected individuals are identified by mutations not present in unaffected individuals.

Genomic rearrangements are also observed as a method of determining alterations in a gene corresponding to a polynucleotide. Genomic clones isolated according to Example 2 are nick-translated with digoxigenindeoxy-uridine 5'-triphosphate (Boehringer Manheim), and FISH performed as described in Johnson, Cg. et al., Methods Cell Biol. 35:73-99 (1991). Hybridization with the labeled probe is carried out using a vast excess of human cot-1 DNA for specific hybridization to the corresponding genomic locus.

Chromosomes are counterstained with 4,6-diamino-2-phenylidole and propidium iodide, producing a combination of C- and R-bands. Aligned images for precise mapping are obtained using a triple-band filter set (Chroma Technology, Brattleboro, VT) in combination with a cooled charge-coupled device camera (Photometrics, Tucson, AZ) and variable excitation wavelength filters. (Johnson, Cv. et al., Genet. Anal. Tech. Appl., 8:75 (1991).) Image collection, analysis and chromosomal fractional length measurements are performed using the ISee Graphical Program System. (Inovision Corporation, Durham, NC.) Chromosome alterations of the genomic region hybridized by the probe are identified as insertions, deletions, and translocations. These alterations are used as a diagnostic marker for an associated disease.

### Example 22: Method of Detecting Abnormal Levels of a Polypeptide in a Biological Sample

A polypeptide of the present invention can be detected in a biological sample, and if an increased or decreased level of the polypeptide is detected, this polypeptide is a marker for a particular phenotype. Methods of detection are numerous, and thus, it is understood that one skilled in the art can modify the following assay to fit their particular needs.

For example, antibody-sandwich ELISAs are used to detect polypeptides in a sample, preferably a biological sample. Wells of a microtiter plate are coated with specific antibodies, at a final concentration of 0.2 to 10 ug/ml. The antibodies are either monoclonal or polyclonal and are produced by the method described in Example 10. The wells are blocked so that non-specific binding of the polypeptide to the well is reduced.

The coated wells are then incubated for > 2 hours at RT with a sample containing the polypeptide. Preferably, serial dilutions of the sample should be used to validate results. The plates are then washed three times with deionized or distilled water to remove unbounded polypeptide.

Next, 50 ul of specific antibody-alkaline phosphatase conjugate, at a concentration of 25-400 ng, is added and incubated for 2 hours at room temperature. The plates are again washed three times with deionized or distilled water to remove unbounded conjugate.

Add 75 ul of 4-methylumbelliferyl phosphate (MUP) or p-nitrophenyl phosphate (NPP) substrate solution to each well and incubate 1 hour at room temperature. Measure the reaction by a microtiter plate reader. Prepare a standard curve, using serial dilutions of a control sample, and plot polypeptide concentration on the X-axis (log scale) and fluorescence or absorbance of the Y-axis (linear scale). Interpolate the concentration of the polypeptide in the sample using the standard curve.

### Example 23: Formulating a Polypeptide

The secreted polypeptide composition will be formulated and dosed in a fashion consistent with good medical practice, taking into account the clinical condition of the individual patient (especially the side effects of treatment with the secreted polypeptide alone), the site of delivery, the method of administration, the scheduling of administration, and other factors known to practitioners. The "effective amount" for purposes herein is thus determined by such considerations.

As a general proposition, the total pharmaceutically effective amount of secreted polypeptide administered parenterally per dose will be in the range of about 1 µg/kg/day to 10 mg/kg/day of patient body weight, although, as noted above, this will be subject to therapeutic discretion. More preferably, this dose is at least 0.01 mg/kg/day, and most preferably for humans between about 0.01 and 1 mg/kg/day for the hormone. If given continuously, the secreted polypeptide is typically administered at a dose rate of about 1 µg/kg/hour to about 50 µg/kg/hour, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect.

Pharmaceutical compositions containing the secreted protein of the invention are administered orally, rectally, parenterally, intracistemally, intravaginally, intraperitoneally, topically (as by powders, ointments, gels, drops or transdermal patch), bucally, or as an oral or nasal spray. "Pharmaceutically acceptable carrier" refers to a non-toxic solid, semisolid or liquid filler, diluent, encapsulating material or formulation auxiliary of any type. The term "parenteral" as used herein refers to modes of administration which include intravenous, intramuscular, intraperitoneal, intrastemal, subcutaneous and intraarticular injection and infusion.

The secreted polypeptide is also suitably administered by sustained-release systems. Suitable examples of sustained-release compositions include semi-permeable polymer matrices in the form of shaped articles, e.g., films, or mirocapsules. Sustained-release matrices include polylactides (U.S. Pat. No. 3,773,919, EP 58,481), copolymers of L-glutamic acid and gamma-ethyl-L-glutamate (Sidman, U. et al., Biopolymers 22:547-556 (1983)), poly (2- hydroxyethyl methacrylate) (R. Langer et al., J. Biomed. Mater. Res. 15:167-277 (1981), and R. Langer, Chem. Tech. 12:98-105 (1982)), ethylene vinyl acetate (R. Langer et al.) or poly-D- (-)-3-hydroxybutyric acid (EP 133,988). Sustained-release compositions also include liposomally entrapped polypeptides. Liposomes containing the secreted polypeptide are prepared by methods known per se: DE 3,218,121; Epstein et al., Proc. Natl. Acad. Sci. USA 82:3688-3692 (1985); Hwang et al., Proc. Natl. Acad. Sci. USA 77:4030-4034 (1980); EP 52,322; EP 36,676; EP 88,046; EP 143,949; EP 142,641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal secreted polypeptide therapy.

For parenteral administration, in one embodiment, the secreted polypeptide is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation. For example, the formulation preferably does not include oxidizing agents and other compounds that are known to be deleterious to polypeptides.

Generally, the formulations are prepared by contacting the polypeptide uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes.

The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) polypeptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, manose, or dextrins; chelating agents such as EDTA; sugar alcohols such as mannitol or sorbitol; counterions such as sodium; and/or nonionic surfactants such as polysorbates, poloxamers, or PEG.

The secreted polypeptide is typically formulated in such vehicles at a concentration of about 0.1 mg/ml. to 100 mg/ml, preferably 1-10 mg/ml, at a pH of about 3 to 8. It will be understood that the use of certain of the foregoing excipients, carriers, or stabilizers will result in the formation of polypeptide salts.

Any polypeptide to be used for therapeutic administration can be sterile. Sterility is readily accomplished by filtration through sterile filtration membranes (e.g., 0.2 micron membranes). Therapeutic polypeptide compositions generally are placed into a container having a sterile access port, for example, an intravenous solution bag or vial having a stopper pierceable by a hypodermic injection needle.

Polypeptides ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous polypeptide solution, and the resulting mixture is lyophilized. The infusion solution is prepared by reconstituting the lyophilized polypeptide using bacteriostatic Water-for-Injection.

The invention also provides a pharmaceutical pack or kit comprising one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration. In addition, the polypeptides of the present invention may be employed in conjunction with other therapeutic compounds.

### Example 24: Method of Treating Decreased Levels of the Polypeptide

It will be appreciated that conditions caused by a decrease in the standard or normal expression level of a secreted protein in an individual can be treated by administering the polypeptide of the present invention, preferably in the secreted form. Thus, the invention also provides a method of treatment of an individual in need of an increased level of the polypeptide comprising administering to such an individual a pharmaceutical composition comprising an amount of the polypeptide to increase the activity level of the polypeptide in such an individual.

For example, a patient with decreased levels of a polypeptide receives a daily dose 0.1-100 ug/kg of the polypeptide for six consecutive days. Preferably, the polypeptide is in the secreted form. The exact details of the dosing scheme, based on administration and formulation, are provided in Example 23.

### Example 25: Method of Treating Increased Levels of the Polypeptide

Antisense technology is used to inhibit production of a polypeptide of the present invention. This technology is one example of a method of decreasing levels of a polypeptide, preferably a secreted form, due to a variety of etiologies, such as cancer.

For example, a patient diagnosed with abnormally increased levels of a polypeptide is administered intravenously antisense polynucleotides at 0.5, 1.0, 1.5, 2.0 and 3.0 mg/kg day for 21 days. This treatment is repeated after a 7-day rest period if the treatment was well tolerated. The formulation of the antisense polynucleotide is provided in Example 23.

### Example 26: Method of Treatment Using Gene Therapy

One method of gene therapy transplants fibroblasts, which are capable of expressing a polypeptide, onto a patient. Generally, fibroblasts are obtained from a subject by skin biopsy. The resulting tissue is placed in tissue-culture medium and separated into small pieces. Small chunks of the tissue are placed on a wet surface of a tissue culture flask, approximately ten pieces are placed in each flask. The flask is turned upside down, closed tight and left at room temperature over night. After 24 hours at room temperature, the flask is inverted and the chunks of tissue remain fixed to the bottom of the flask and fresh media (e.g., Ham's F12 media, with 10% FBS, penicillin and streptomycin) is added. The flasks are then incubated at 37°C for approximately one week.

At this time, fresh media is added and subsequently changed every several days. After an additional two weeks in culture, a monolayer of fibroblasts emerge. The monolayer is trypsinized and scaled into larger flasks.

pMV-7 (Kirschmeier, P.T. et al., DNA, 7:219-25 (1988)), flanked by the long terminal repeats of the Moloney murine sarcoma virus, is digested with EcoRI and HindIII and subsequently treated with calf intestinal phosphatase. The linear vector is fractionated on agarose gel and purified, using glass beads.

The cDNA encoding a polypeptide of the present invention can be amplified using PCR primers which correspond to the 5' and 3' end sequences respectively as set forth in Example 1. Preferably, the 5' primer contains an EcoRI site and the 3' primer includes a HindIII site. Equal quantities of the Moloney murine sarcoma virus linear backbone and the amplified EcoRI and HindIII fragment are added together, in the presence of T4 DNA ligase. The resulting mixture is maintained under conditions appropriate for ligation of the two fragments. The ligation mixture is then used to transform bacteria HB 101, which are then plated onto agar containing kanamycin for the purpose of confirming that the vector has the gene of interest properly inserted.

The amphotropic pA317 or GP+am12 packaging cells are grown in tissue culture to confluent density in Dulbecco's Modified Eagles Medium (DMEM) with 10% calf serum (CS), penicillin and streptomycin. The MSV vector containing the gene is then added to the media and the packaging cells transduced with the vector. The packaging cells now produce infectious viral particles containing the gene (the packaging cells are now referred to as producer cells).

Fresh media is added to the transduced producer cells, and subsequently, the media is harvested from a 10 cm plate of confluent producer cells. The spent media, containing the infectious viral particles, is filtered through a millipore filter to remove detached producer cells and this media is then used to infect fibroblast cells. Media is removed from a sub-confluent plate of fibroblasts and quickly replaced with the media from the producer cells. This media is removed and replaced with fresh media. If the titer of virus is high, then virtually all fibroblasts will be infected and no selection is required. If the titer is very low, then it is necessary to use a retroviral vector that has a selectable marker, such as neo or his. Once the fibroblasts have been efficiently infected, the fibroblasts are analyzed to determine whether protein is produced.

The engineered fibroblasts are then transplanted onto the host, either alone or after having been grown to confluence on cytodex 3 microcarrier beads.

### Example 27: Method of Treatment Using Gene Therapy - In Vivo

Another aspect of the present invention is using *in vivo* gene therapy methods to treat disorders, diseases and conditions. The gene therapy method relates to the introduction of naked nucleic acid (DNA, RNA, and antisense DNA or RNA) sequences into an animal to increase or decrease the expression of the polypeptide of the present invention. A polynucleotide of the present invention may be operatively linked to a promoter or any other genetic elements necessary for the expression of the encoded polypeptide by the target tissue. Such gene therapy and delivery techniques and methods are known in the art, see, for example, WO90/11092, WO98/11779; U.S. Patent NO. 5693622, 5705151, 5580859; Tabata H. et al. (1997) Cardiovasc. Res. 35(3):470-479, Chao J et al. (1997) Pharmacol. Res. 35(6):517-522, Wolff J.A. (1997) Neuromuscul. Disord. 7(5):314-318, Schwartz B. et al. (1996) Gene Ther. 3(5):405-411, Tsurumi Y. et al. (1996) Circulation 94(12):3281-3290 (incorporated herein by reference).

The polynucleotide constructs of the present invention may be delivered by any method that delivers injectable materials to the cells of an animal, such as, injection into the interstitial space of tissues (heart, muscle, skin, lung, liver, intestine and the like). These polynucleotide constructs can be delivered in a pharmaceutically acceptable liquid or aqueous carrier.

The term "naked" polynucleotide, DNA or RNA, refers to sequences that are free from any delivery vehicle that acts to assist, promote, or facilitate entry into the cell, including viral sequences, viral particles, liposome formulations, lipofectin or precipitating agents and the like. However, the polynucleotides may also be delivered in liposome formulations (such as those taught in Felgner P.L. et al. (1995) Ann. NY Acad. Sci. 772:126-139 and Abdallah B. et al. (1995) Biol. Cell 85(1):1-7) which can be prepared by methods well known to those skilled in the art.

The polynucleotide vector constructs of the present invention used in the gene therapy method are preferably constructs that will not integrate into the host genome nor will they contain sequences that allow for replication. Any strong promoter known to those skilled in the art can be used for driving the expression of DNA. Unlike other gene therapies techniques, one major advantage of introducing naked nucleic acid sequences into target cells is the transitory nature of the polynucleotide synthesis in the cells. Studies have shown that non-replicating DNA sequences can be introduced into cells to provide production of the desired polypeptide for periods of up to six months.

The polynucleotide construct of the present invention can be delivered to the interstitial space of tissues within the an animal, including of muscle, skin, brain, lung, liver; spleen, bone marrow, thymus, heart, lymph, blood, bone, cartilage, pancreas, kidney, gall bladder, stomach, intestine, testis, ovary, uterus, rectum, nervous system, eye, gland, and connective tissue. Interstitial space of the tissues comprises the intercellular fluid, mucopolysaccharide matrix among the reticular fibers of organ tissues, elastic fibers in the walls of vessels or chambers, collagen fibers of fibrous tissues, or that same matrix within connective tissue ensheathing muscle cells or in the lacunae of bone. It is similarly the space occupied by the plasma of the circulation and the lymph fluid of the lymphatic channels. Delivery to the interstitial space of muscle tissue is preferred for the reasons discussed below. They may be conveniently delivered by injection into the tissues comprising these cells. They are preferably delivered to and expressed in persistent, non-dividing cells which are differentiated, although delivery and expression may be achieved in non-differentiated or less completely differentiated cells, such as, for example, stem cells of blood or skin fibroblasts. *In vivo* muscle cells are particularly competent in their ability to take up and express polynucleotides.

For the naked polynucleotide injection, an effective dosage amount of DNA or RNA will be in the range of from about 0.05 g/kg body weight to about 50 mg/kg body weight. Preferably the dosage will be from about 0.005 mg/kg to about 20 mg/kg and more preferably from about 0.05 mg/kg to about 5 mg/kg. Of course, as the artisan of ordinary skill will appreciate, this dosage will vary according to the tissue site of injection: The appropriate and effective dosage of nucleic acid sequence can readily be determined by those of ordinary skill in the art and may depend on the condition being treated and the route of administration. The preferred route of administration is by the parenteral route of injection into the interstitial space of tissues. However, other parenteral routes may also be used, such as, inhalation of an aerosol formulation particularly for delivery to lungs or bronchial tissues, throat or mucous membranes of the nose. In addition, naked polynucleotide constructs can be delivered to arteries during angioplasty by the catheter used in the procedure.

The dose response effects of injected polynucleotide in muscle *in vivo* is determined as follows. Suitable template DNA for production of mRNA coding for the polypeptide of the present invention is prepared in accordance with a standard recombinant DNA methodology. The template DNA, which may be either circular or linear, is either used as naked DNA or complexed with liposomes. The quadriceps muscles of mice are then injected with various amounts of the template DNA.

Five to six week old female and male Balb/C mice are anesthetized by intraperitoneal injection with 0.3 ml of 2.5% Avertin. A 1.5 cm incision is made on the anterior thigh, and the quadriceps muscle is directly visualized. The template DNA is injected in 0.1 ml of carrier in a 1 cc syringe through a 27 gauge needle over one minute, approximately 0.5 cm from the distal insertion site of the muscle into the knee and about 0.2 cm deep. A suture is placed over the injection site for future localization, and the skin is closed with stainless steel clips.

After an appropriate incubation time (e.g., 7 days) muscle extracts are prepared by excising the entire quadriceps. Every fifth 15 um cross-section of the individual quadriceps muscles is histochemically stained for protein expression. A time course for protein expression may be done in a similar fashion except that quadriceps from different mice are harvested at different times. Persistence of DNA in muscle following injection may be determined by Southern blot analysis after preparing total cellular DNA and HIRT supernatants from injected and control mice. The results of the above experimentation in mice can be use to extrapolate proper dosages and other treatment parameters in humans and other animals using naked DNA of the present invention.

It will be clear that the invention may be practiced otherwise than as particularly described in the foregoing description and examples. Numerous modifications and variations of the present invention are possible in light of the above teachings and, therefore, are within the scope of the appended claims.

The entire disclosure of each document cited (including patents, patent applications, journal articles, abstracts, laboratory manuals, books, or other disclosures) in the Background of the Invention, Detailed Description, and Examples is hereby incorporated herein by reference.

## Claims

1. A nucleic acid molecule comprising a polynucleotide having a nucleotide sequence selected from the group consisting of:
(a) a polynucleotide having the entire nucleotide sequence of SEQ ID NO: 26;
(b) a polynucleotide having the entire cDNA sequence included in ATCC Deposit No. 209012 or 209089, which is hybridizable to SEQ ID NO: 26;
(c) a polynucleotide fragment of SEQ ID NO: 26;
(d) a polynucleotide fragment of the cDNA sequence included in ATCC Deposit No. 209012 or 209089, which is hybridizable to SEQ ID NO: 26;
(e) a polynucleotide encoding a polypeptide fragment of SEQ ID NO: 126;
(f) a polynucleotide encoding a polypeptide fragment encoded by the cDNA sequence included in ATCC Deposit No. 209012 or 209089, which is hybridizable to SEQ ID NO: 26;
(g) a polynucleotide encoding a polypeptide fragment of SEQ ID NO: 126 or a polypeptide fragment encoded by the cDNA sequence included in ATCC Deposit No. 209012 or 209089, having biological activity;
(h) a polynucleotide encoding a polypeptide fragment of SEQ ID NO: 126 or a polypeptide fragment encoded by the cDNA sequence included in ATCC Deposit No. 209012 or 209089, which is secreted;
(i) a polynucleotide encoding a full length polypeptide of SEQ ID NO: 126 or being encoded by the cDNA sequence included in ATCC Deposit No. 209012 or 209089;
(j) a polynucleotide encoding a polypeptide domain of SEQ ID NO: 126;
(k) a polynucleotide encoding a polypeptide domain encoded by the cDNA sequence included in ATCC Deposit No. 209012 or 209089, which is hybridizable to SEQ ID NO: 26;
(l) a polynucleotide encoding a polypeptide epitope of SEQ ID NO: 126;
(m) a polynucleotide encoding a polypeptide epitope encoded by the cDNA sequence included in ATCC Deposit No. 209012 or 209089, which is hybridizable to SEQ ID NO: 26;
(n) a polynucleotide which is at least 90% identical to a polynucleotide as defined in any one of (a) to (m);
(o) a polynucleotide encoding a polypeptide having an amino acid sequence at least 95% identical to the amino acid sequence of a polypeptide encoded by a polynucleotide of any one of (a) to (n); and
(p) a polynucleotide capable of hybridizing under stringent conditions to any one of the polynucleotides specified in (a) to (o), wherein said polynucleotide does not hybridize under stringent conditions to a nucleic acid molecule having a nucleotide sequence of only A residues or of only T residues;
or the complementary strand of polynucleotide (a) to (p).

2. The nucleic acid molecule of claim 1, wherein the nucleotide sequence comprises sequential nucleotide deletions from either the 5 prime terminus or the 3 prime terminus.

3. The nucleic acid molecule of claim 1 or 2 which is DNA or RNA.

4. A gene corresponding to the cDNA sequence of SEQ ID NO: 26 or encoding the polypeptide having the amino acid sequence of SEQ ID NO: 126.

5. A vector comprising the nucleic acid molecule of any one of claims 1 to 3.

6. A method of making a recombinant host cell comprising introducing the nucleic acid molecule of any one of claims 1 to 3, or the vector of claim 5.

7. A recombinant host cell containing the nucleic acid molecule of claims 1 to 3 or the vector of claim 5 or produced by the method of claim 6.

8. The recombinant host cell of claim 7 which expresses a polypeptide encoded by the nucleic acid molecule of any one of claims 1 to 3.

9. A method of making a polypeptide encoded by the nucleic acid molecule of any one of claims 1 to 3 comprising:
(a) culturing the recombinant host cell of claim 8 under conditions such that said polypeptide is expressed; and
(b) recovering said polypeptide.

10. A polypeptide encoded by the nucleic acid molecule of any one of claims 1 to 3 or obtainable by the method of claim 9.

11. An antibody that binds specifically to the polypeptide of claim 10.

12. An antagonist of the polypeptide of claim 10 comprising a molecule closely related to the natural ligand of said polypeptide or a polypeptide related to the natural receptor of the polypeptide of claim 10 or a fragment thereof.

13. An agonist of the polypeptide of claim 10 comprising a molecule closely related to the natural ligand of said polypeptide or a polypeptide related to the natural receptor of the polypeptide of claim 10 or a fragment thereof.

14. A method for identifying antagonists or agonists of the polypeptide of claim 10 comprising:
(a) producing cells which express said polypeptide either as a secreted protein or on the cell membrane;
(b) contacting the polypeptide produced in step (a) with a test sample potentially containing an antagonist or agonist; and
(c) identifying an antagonist or agonist by observing binding and inhibition or stimulation of activity of said polypeptide.

15. An in vitro method of diagnosing a pathological condition or a susceptibility to a pathological condition in a subject comprising:
(a) determining the presence or absence of a mutation in the nucleic acid molecule of any one of claims 1 to 3 or the gene of claim 4; and
(b) diagnosing a pathological condition or a susceptibility to a pathological condition based on the presence or absence of said mutation.

16. An in vitro method of diagnosing a pathological condition or a susceptibility to a pathological condition in a subject comprising:
(a) determining the presence or amount of expression of the polypeptide of claim 10 in a biological sample; and
(b) diagnosing a pathological condition or a susceptibility to a pathological condition based on the presence or amount of expression of the polypeptide.

17. A method for identifying a binding partner to the polypeptide of claim 10 comprising:
(a) contacting the polypeptide of claim 10 with a binding partner; and
(b) determining whether the binding partner effects an activity of the polypeptide.

18. A method of identifying an activity in a biological assay, wherein the method comprises:
(a) expressing the nucleic acid molecule of any one of claims 1 to 3 or the gene of claim 4 or the vector of claim 5 in a cell;
(b) isolating the supernatant;
(c) detecting an activity in a biological assay; and
(d) identifying the protein in the supernatant having the activity.

19. The product produced by the method of any one of claims 14, 17 and 18.

20. A pharmaceutical composition comprising the nucleic acid molecule of any one of claims 1 to 3, the polypeptide of claim 10, a DNA encoding and capable of expressing said polypeptide in vivo, the antibody of claim 11, the antagonist of claim 12, the agonist of claim 13 or the product of claim 19 and optionally a pharmaceutically acceptable carrier.

21. Use of the nucleic acid molecule of any one of claims 1 to 3, the polypeptide of claim 10, a DNA encoding and capable of expressing said polypeptide in vivo the antibody of claim 11, antagonist of claim 12, the agonist of claim 13 or the product of claim 19 for the preparation of a pharmaceutical composition for preventing, treating, or ameliorating a medical condition.

22. A diagnostic composition comprising the nucleic acid molecule of any one of claims 1 to 3 or the antibody of claim 11.

23. A method for the production of a pharmaceutical composition comprising the steps of the method of claim 14 and (d) formulating the compound identified in step (c) in a pharmaceutically acceptable form.
